# EUROPEAN PATENT APPLICATION

(11) **EP 2 581 373 A1**
(43) Date of publication of application: **17.04.2013**
(21) Application number: 11780638.0
(22) Date of filing: 11.05.2011
(51) Int. Cl.: C07D 417/14, A61K 31/427, A61K 31/4355, A61P 3/06, A61P 3/10, A61P 9/10, A61P 9/12, A61P 17/06, A61P 19/06, A61P 25/28, A61P 27/02, A61P 27/06, A61P 35/00, C07D 471/04

(54) **BENZO- OR PYRIDO-IMIDAZOLE DERIVATIVE**

(30) Priority: 20.12.2010 JP 2010294905; 11.05.2010 JP 2010129383
(71) Applicant: Fujita, Takashi, Kashiwa-shi, Chiba 277-0022 (JP); Horikoshi, Hiroyoshi, Funabashi-shi, Chiba 274-0072 (JP)
(72) Inventor: CAWTHORNE, Michael Anthony, Buckingham MK181EG (GB); FUJITA, Takashi, Kashiwa-shi, Chiba 277-0022 (JP); HORIKOSHI, Hiroyoshi, Funabashi-shi Chiba 274-0072 (JP)
(74) Representative: Wichmann, Hendrik
(86) International application number: PCT/JP2011/060837
(87) International publication number: WO 2011/142381

(57) **Abstract**

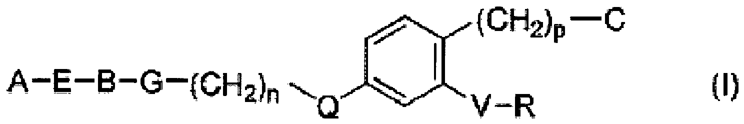

The present invention addresses the problem of finding a compound having both PPAR activation activity and angiotensin receptor antagonistic activity. The present invention is a benzo- or pyrido-imidazole derivative represented by general formula (I), a pharmaceutically acceptable salt thereof, or a ester or amide thereof (where A is biphenyl methyl-imidazolyl, biphenyl methyl-benzimidazolyl, or the like, B is divalent benzimidazolyl or the like, C is carboxyl or the like, E is divalent phenyl, naphthyl, or the like, G is a dangling bond, oxygen, or the like, Q is oxygen or sulfur, n is an integer from 1 to 6, p is an integer from 1 to 6, V is a dangling bond, oxygen, or the like, and R is hydrogen, alkyl, or the like).

## Description

### TECHNICAL FIELD

The present invention pertains to a benzo- or pyrido-imidazole derivative having excellent PPAR activation (agonist) activity and excellent angiotensin II receptor antagonistic activity, and having usefulness as a pharmaceutical.

### BACKGROUND ART

Today, PPAR activators such as thiazolidine compounds or oxazolidine compounds are known to be useful as therapeutic agents or the like for various diseases such as diabetes and hyperlipidemia (non-patent literature 1 and 2), and angiotensin II receptor antagonists such as imidazole cyclic compounds are known to be useful as therapeutic agents or the like for hypertension, heart disease, and diseases caused by hypertension (non-patent literature 3). Although compounds having excellent angiotensin II receptor antagonistic activity (for example, irbesartan and telmisartan) are known to have PPAR (γ) activation activity (non-patent literature 4 and 5), the PPAR (γ) activation activity of these compounds is a so-called selective PPAR (γ) modulator activity; that is, a partial activation (agonist) activity, not a full activation (agonist) activity. Combination drugs combining PPAR activators such as thiazolidine compounds or oxazolidine compounds and angiotensin II receptor antagonists such as imidazole cyclic compounds have also been provided in the expectation of developing excellent diabetes therapeutic agents or the like with fewer adverse drug reactions (patent literature 1 and 2, and non-patent literature 6).
No conventional compound has had both excellent PPAR activation activity (full activation (agonist) activity) and excellent angiotensin II receptor antagonistic activity.

### PRIOR ART LITERATURE

### PATENT LITERATURE

Patent literature 1: WO06/03872
Patent literature 2: WO09/088006

### NON-PATENT LITERATURE

Non-patent literature 1: Expert Opin. Investig. Drugs, vol. 12 (9), page 1489-1500 (2003)
Non-patent literature 2: J. Clinical Investig., vol. 106 (4), page 467-472 (2000).
Non-patent literature 3: J. Med. Chem., vol. 39, page 625-656 (1996)
Non-patent literature 4: Diabetes, vol. 54, page 3442-3452 (2005)
Non-patent literature 5: Diabetes Care, vol. 27 (no. 4), page 1015 (2004)
Non-patent literature 6: Hypertension, vol. 51, page 296-301 (2008)

### OUTLINE OF THE INVENTION

### PROBLEMS THAT THE INVENTION IS TO SOLVE

As a result of synthesizing a series of imidazole derivatives and studying the pharmacological activity of these derivatives, the present inventors discovered that a benzo- or pyrido-imidazole derivative having a specific structure has excellent PPAR activation activity and excellent angiotensin II receptor antagonistic activity, and so achieved the present invention.

### MEANS OF SOLVING THE PROBLEMS

The present invention relates to a benzo- or pyrido-imidazole derivative represented by general formula (I), a pharmaceutically acceptable salt thereof, or a pharmaceutically acceptable ester or amide thereof. 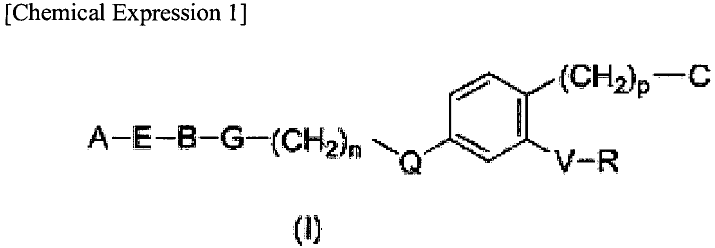 [where
A indicates the following (a)-(g):
(a) a group represented by the expression 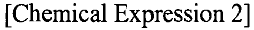 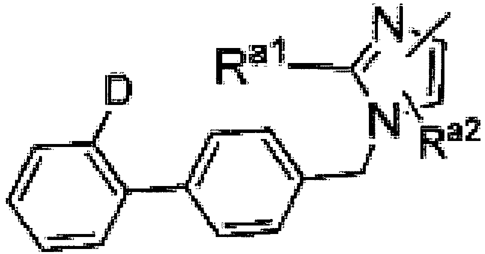 (where R^{a1} indicates the following (i)-(viii):
   (i) a C₁-C₆ alkyl, (ii) a C₁-C₆ halogenoalkyl, (iii) a C₁-C₆ alkoxy, (iv) a C₁-C₆ alkylthio, (v) a group represented by the expression -N(R^{a3})(R^{a4}) (where R^{a3} indicates (i) hydrogen, (ii) a C₁-C₆ alkyl, (iii) a C₆-C₁₀ aryl optionally having one to five substituents α to be described later, or (iv) a C₆-C₁₀ aryl-C₁-C₆ alkyl optionally having in the aryl portion one to five substituents α to be described later, and R^{a4} indicates hydrogen or a C₁-C₆ alkyl), (vi) a C₃-C₁₀ cycloalkyl, (vii) a C₃-C₁₀ cycloalkyloxy, or (viii) a C₃-C₁₀ cycloalkylthio),
      R^{a2} indicates the following (i)-(xii):
   (i) hydrogen, (ii) a halogen, (iii) formyl, (iv) a C₁-C₁₀ alkylcarbonyl or a C₂-C₆ alkenylcarbonyl, (v) carboxyl, (vi) carbamoyl, (vii) a mono- or di-C₁-C₆ alkylcarbamoyl , (viii) amino, (ix) formylamino, a C₁-C₆ alkylcarbonylamino, or a C₂-C₆ alkenylcarbonylamino, (x) a C₁-C₆ hydroxyalkyl, (xi) a formyl-C₁-C₆ alkyl, or (xii) a C₁-C₆ alkylcarbonyl-C₁-C₆ alkyl, D indicates the following (i)-(xi):
   (i) carboxyl, (ii) 5-tetrazolyl, (iii) a group represented by the expression 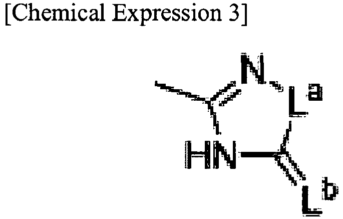 (where L^{a} and L^{b} indicate the same or different oxygen or sulfur), (iv) a group represented by the expression 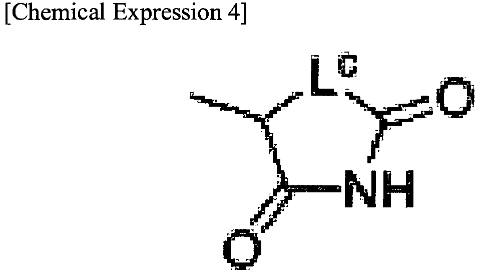 (where L^{c} indicates oxygen, sulfur, or a group represented by the expression -N(R^{a3})- (where R^{a3} is defined as described earlier), (v) a group represented by the expression CF₃SO₂-NH-, (vi) tetrazol-5-ylaminocarbonyl, (vii) a group represented by the expression 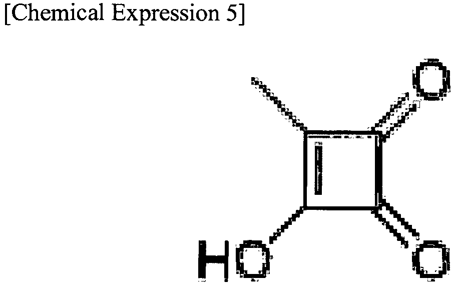
   (viii) formylaminosulfonyl, a C₁-C₆ alkylcarbonylaminosulfonyl, or a C₂-C₆ alkenylcarbonylaminosulfonyl, (ix) a C₆-C₁₀ aryl carbonylaminosulfonyl optionally having in the aryl portion one to five substituents α to be described later, (x) a C₆-C₁₀ aryl-C₁-C₆ alkylcarbonylaminosulfonyl optionally having in the aryl portion one to five substituents α to be described later, or (xi) sulfo);
(b) a group represented by the expression 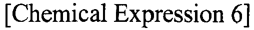 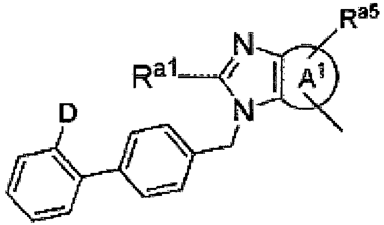 (where D and R^{a1} are defined as described earlier, the group represented by the expression 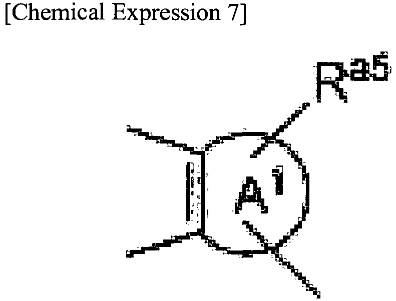 indicates a trivalent six-membered unsaturated hydrocarbon ring optionally having a substituent R^{a5}, optionally containing one to two atoms and/or groups selected from among oxygen, sulfur, the expression =N-R^{a3} (where R^{a3} is defined as described earlier), and sulfone (-S(O)₂-), and optionally having hydrogen substituted with oxo, a C₁-C₆ alkylidene, a C₁-C₆ alkylimino, hydroxyimino, or a C₁-C₆ alkoxyimino, and R^{a5} indicates (i) a group indicated by R^{a2}, (ii) a C₁-C₆ alkyl, (iii) a C₁-C₆ halogenoalkyl, (iv) a C₁-C₆ alkoxy, (v) a C₆-C₁₀ aryloxy, or (vi) a C₆-C₁₀ aryl-C₁-C₆ alkyloxy);
(c) a group represented by the expression 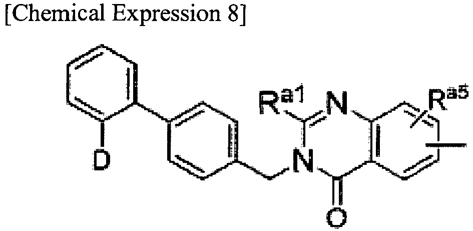 (where D, R^{a1}, and R^{a5} are defined as described earlier);
(d) a group represented by the expression 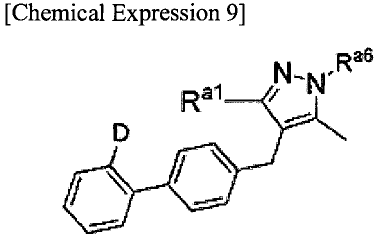 (where D and R^{a1} are defined as described earlier, and R^{a6} indicates hydrogen or a C₁-C₆ alkyl);
(e) a group represented by the expression 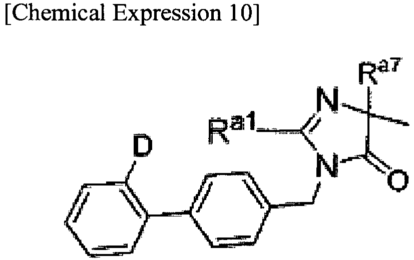 (where D and R^{a1} are defined as described earlier, and R^{a7} indicates hydrogen, a C₁-C₆ alkyl, or a C₃-C₁₀ cycloalkyl);
(f) a group represented by the expression 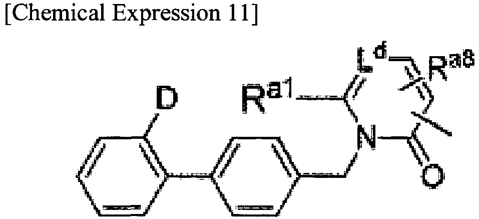 (where D and R^{a1} are defined as described earlier, R^{a8} indicates hydrogen or a C₁-C₆ alkyl, and L^{d} indicates the expression =CH- or =N-); or
(g) a group represented by the expression 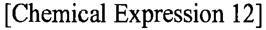 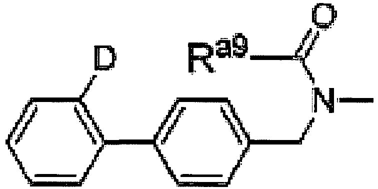 (where D is defined as described earlier, and R^{a9} indicates a C₁-C₆ alkyl, a C₁-C₆ halogenoalkyl, or a C₃-C₁₀ cycloalkyl),
   B indicates a group represented by the expression 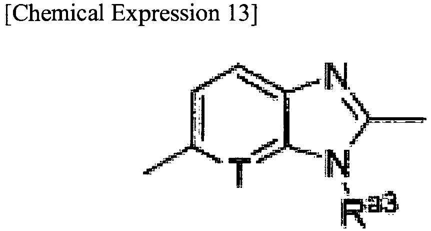 (where R^{a3} is defined as described earlier, and T indicates the expression =CH- or =N-), and B is bonded to E (or G) by a portion of an imidazole ring or a portion of a benzene or pyridine ring, C indicates the following (a)-(d):
(a) carboxyl;
(b) a group represented by the expression 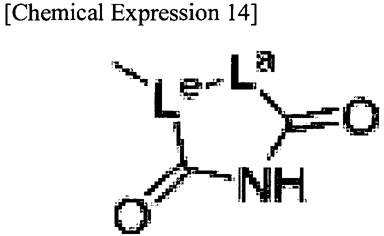 (where L^{a} is defined as described earlier, L^{e} indicates the expression =CH- or =N-, and L^{a} indicates oxygen when L^{e} indicates the expression =N-);
(c) a group represented by the expression -C(R^{c1})(COOH)-W^{c1}-R^{c2} (where R^{c1} and R^{c2} may be the same or different, and indicate the following (i)-(viii):
   (i) hydrogen, (ii) a C₁-C₆ alkyl, (iii) a C₆-C₁₀ aryl optionally having one to five substituents α to be described later, (iv) a C₆-C₁₀ aryl-C₁-C₆ alkyl optionally having in the aryl portion one to five substituents α to be described later, (v) a C₁-C₆ alkylsulfonyl, (vi) a C₁-C₆ halogenoalkylsulfonyl, (vii) a C₆-C₁₀ aryl sulfonyl optionally having one to five substituents α to be described later, or (viii) a C₆-C₁₀ aryl-C₁-C₆ alkylsulfonyl optionally having in the aryl portion one to five substituents α to be described later; provided, however, that when W^{c1} to be described later indicates oxygen or sulfur, R^{c1} and R^{c2} indicate (i) hydrogen, (ii) a C₁-C₆ alkyl, (iii) a C₆-C₁₀ aryl optionally having one to five substituents α to be described later, or (iv) a C₆-C₁₀ aryl-C₁-C₆ alkyl optionally having in the aryl portion one to five substituents α to be described later,
      W^{c1} indicates the following (i)-(iii):
   (i) oxygen, (ii) sulfur, or (iii) a group represented by the expression -N(R^{c3})- (where R^{c3} indicates the following (i)-(xiii):
   (i) hydrogen, (ii) a C₁-C₆ alkyl, (iii) a C₆-C₁₀ aryl optionally having one to five substituents α to be described later, (iv) a C₆-C₁₀ aryl-C₁-C₆ alkyl optionally having in the aryl portion one to five substituents α to be described later, (v) formyl, a C₁-C₆ alkylcarbonyl, or a C₂-C₆ alkenylcarbonyl, (vi) a C₆-C₁₀ aryl carbonyl optionally having one to five substituents α to be described later, (vii) a C₆-C₁₀ aryl-C₁-C₆ alkylcarbonyl optionally having in the aryl portion one to five substituents α to be described later, (viii) a C₃-C₁₀ cycloalkylcarbonyl, (ix) a five- to seven-membered cyclic heteroaryl carbonyl optionally having one to two substituents α to be described later and containing one to three heteroatoms selected from among a group consisting of oxygen, nitrogen, and sulfur, (x) carbamoyl optionally substituted with one to two substituents α to be described later (provided, however, that the substituents α may not be (iv) a halogen, (v) hydroxy, (vi) cyano, or (vii) nitro), (xi) a C₁-C₆ alkoxycarbonyl, (xii) a C₆-C₁₀ aryloxycarbonyl optionally having one to five substituents α to be described later, or (xiii) a C₆-C₁₀ aryl-C₁-C₆ alkoxycarbonyl optionally having one to five substituents α to be described later); or
   (d) a group represented by the expression -N(W^{c2})-CH₂COOH (where W^{c2} indicates the following (i)-(xiii):
   (i) hydrogen, (ii) a C₁-C₆ alkyl, (iii) a C₆-C₁₀ aryl optionally having one to five substituents α to be described later, (iv) a C₆-C₁₀ aryl-C₁-C₆ alkyl optionally having in the aryl portion one to five substituents α to be described later, (v) formyl, a C₁-C₆ alkylcarbonyl, or a C₂-C₆ alkenylcarbonyl, (vi) a C₆-C₁₀ aryl carbonyl optionally having one to five substituents α to be described later, (vii) a C₆-C₁₀ aryl-C₁-C₆ alkylcarbonyl optionally having in the aryl portion one to five substituents α to be described later, (viii) a C₃-C₁₀ cycloalkylcarbonyl, (ix) a five- to seven-membered cyclic heteroaryl carbonyl optionally having one to two substituents α to be described later and containing one to three heteroatoms selected from among a group consisting of oxygen, nitrogen, and sulfur, (x) carbamoyl optionally substituted with one to two substituents α to be described later (provided, however, that the substituents α may not be (iv) a halogen, (v) hydroxy, (vi) cyano, or (vii) nitro), (xi) a C₁-C₆ alkoxycarbonyl, (xii) a C₆-C₁₀ aryloxycarbonyl optionally having one to five substituents α to be described later, or (xiii) a C₆-C₁₀ aryl-C₁-C₆ alkoxycarbonyl optionally having one to five substituents α to be described later),
      E indicates the following (1) or (2):
      (1) a group represented by the expression 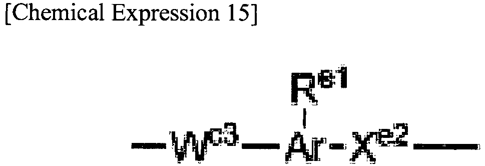 (where R^{e1} is an Ar (aryl or heteroaryl ring) substituent, of which there may be zero, one, or a same or different plurality (this plurality being the maximum number of substituents that an aryl or heteroaryl ring may have), R^{e1} indicates a substituent α to be described later, and W^{c3} indicates the following (a)-(h):
         (a) oxygen, (b) a group represented by the expression -S(O)_{q}- (where q indicates 0 or an integer from 1 to 2), (c) a group represented by the expression -N(R^{e2})- (where R^{e2} indicates the following (i)-(xiv):
            (i) hydrogen, (ii) a C₁-C₆ alkyl, (iii) a C₆-C₁₀ aryl optionally having one to five substituents α to be described later, (iv) a C₆-C₁₀ aryl-C₁-C₆ alkyl optionally having in the aryl portion one to five substituents α to be described later, (v) a C₁-C₆ alkylsulfonyl, (vi) a C₁-C₆ halogenoalkylsulfonyl, (vii) a C₆-C₁₀ aryl sulfonyl optionally having one to five substituents α to be described later, (viii) a C₆-C₁₀ aryl-C₁-C₆ alkylsulfonyl optionally having in the aryl portion one to five substituents α to be described later, (ix) formyl, a C₁-C₆ alkylcarbonyl, or a C₂-C₆ alkenylcarbonyl, (x) a C₆-C₁₀ aryl carbonyl optionally having one to five substituents α to be described later, (xi) a C₆-C₁₀ aryl-C₁-C₆ alkylcarbonyl optionally having in the aryl portion one to five substituents α to be described later, (xii) a C₃-C₁₀ cycloalkylcarbonyl, (xiii) a five- to seven-membered cyclic heteroaryl carbonyl optionally having one to two substituents α to be described later and containing one to three heteroatoms selected from among a group consisting of oxygen, nitrogen, and sulfur, or (xiv) carbamoyl optionally substituted with one to two substituents α to be described later (provided, however, that the substituents α may not be (iv) a halogen, (v) hydroxy, (vi) cyano, or (vii) nitro)), (d) a group represented by the expression -CON(R^{e2})- (where R^{e2} is defined as described earlier), (e) a group represented by the expression - N(R^{e2})-CO- (where R^{e2} is defined as described earlier), (f) a group represented by the expression -CO-, (g) a C₁-C₁₀ alkylene optionally containing one to three atoms and/or groups selected from among a group consisting of (a) to (f), or (h) a dangling bond,
               X^{e2} indicates the following (a)-(g):
               (a) oxygen, (b) sulfur, (c) a group represented by the expression -N(R^{e2})- (where R^{e2} is defined as described earlier), (d) a group represented by the expression -CO-N(R^{e2})- (where R^{e2} is defined as described earlier), (e) a C₁-C₁₀ alkylene optionally containing one to three atoms and/or groups selected from among a group consisting of oxygen, sulfur, groups represented by the expression -N(R^{e2})- (where R^{e2} is defined as described earlier), and groups represented by the formula -CO-N(R^{e2})- (where R^{e2} is defined as described earlier), (f) a group represented by the expression -W^{c4}-Phe-X^{e3}- (where W^{c4} indicates oxygen, a group represented by the expression -S(O)_{q}- (where q is defined as described earlier) or the expression -N(R^{e2})- (where R^{e2} is defined as described earlier), a C₁-C₄ alkylene optionally containing one to two atoms and/or groups selected from among a group consisting of oxygen and groups represented by the expression -S(O)_{q}- (where q is defined as described earlier) and the expression -N(R^{e2})- (where R^{e2} is defined as described earlier), or a dangling bond, Phe indicates phenylene optionally having one to four of the substituent R^{e1}, and X^{e3} indicates oxygen, a group represented by the expression -S(O)_{q}- (where q is defined as described earlier) or the expression -N(R^{e2})- (where R^{e2} is defined as described earlier), a C₁-C₄ alkylene optionally containing one to two atoms and/or groups selected from among a group consisting of oxygen and groups represented by the expression -S(O)_{q}- (where q is defined as described earlier) and the expression -N(R^{e2})- (where R^{e2} is defined as described earlier), or a dangling bond), or (g) a dangling bond, and Ar indicates phenylene, naphthylene, or a five- to ten-membered (monocyclic or annelated) heteroarylene ring containing one to five heteroatoms selected from among a group consisting of oxygen, nitrogen, and sulfur); or
      (2) a group represented by the expression -W^{c3}-W^{c3}-X^{e4}- (where the two W^{c3} may be the same or different, W^{c3} is defined as described earlier, and X^{e4} indicates the following (a)-(f):
         (a) oxygen, (b) sulfur, (c) a group represented by the expression -N(R^{e2})- (where R^{e2} is defined as described earlier), (d) a group represented by the expression -CO-N(R^{e2})- (where R^{e2} is defined as described earlier), (e) a C₁-C₁₀ alkylene optionally containing one to three atoms and/or groups selected from among a group consisting of oxygen, sulfur, groups represented by the expression -N(R^{e2})- (where R^{e2} is defined as described earlier), and groups represented by the expression -CO-N(R^{e2})- (where R^{e2} is defined as described earlier), or (f) a dangling bond); provided, however, that in W^{c3}, X^{e2}, W^{c4}, X^{e3}, X^{e4}, (a) oxygen, (b) a group represented by the expression -S(O)_{q}- (where q is defined as described earlier), and (c) a group represented by the expression -N(R^{e2})- (where R^{e2} is defined as described earlier) or a portion represented by the expression -N(R^{e2})- (where R^{e2} is defined as described earlier) are not bonded directly to each other or adjacent to -CH₂-, and groups formed by these,
            G indicates a dangling bond, oxygen, sulfur, or a group represented by the expression -N(R^{e2})-(where R^{e2} is defined as described earlier),
            Q indicates oxygen or sulfur,
            n indicates an integer from 1 to 6,
            p indicates an integer from 1 to 6,
            V indicates a dangling bond, oxygen, sulfur, or a group represented by the expression -N(R^{e2})-(where R^{e2} is defined as described earlier),
            R indicates (i) hydrogen, (ii) a C₁-C₆ alkyl, (iii) a C₆-C₁₀ aryl optionally having one to five substituents α to be described later, or (iv) a C₆-C₁₀ aryl-C₁-C₆ alkyl optionally having in the aryl portion one to five substituents α to be described later,
            the substituent α indicates the following (i)-(xxiv):
            (i) a C₁-C₆ alkyl, (ii) a C₁-C₆ halogenoalkyl, (iii) a C₁-C₆ alkoxy, (iv) a halogen, (v) hydroxy, (vi) cyano, (vii) nitro, (viii) a C₃-C₁₀ cycloalkyl, (ix) a C₆-C₁₀ aryl optionally having one to five substituents β to be described later, (x) a C₆-C₁₀ aryl-C₁-C₆ alkyl optionally having in the aryl portion one to five substituents β to be described later, (xi) formyl, a C₁-C₆ alkylcarbonyl, or a C₂-C₆ alkenylcarbonyl, (xii) a C₃-C₁₀ cycloalkylcarbonyl, (xiii) a C₆-C₁₀ arylcarbonyl optionally having one to five substituents β to be described later, (xiv) a C₆-C₁₀ aryl-C₁-C₆ alkylcarbonyl optionally having in the aryl portion one to five substituents β to be described later, (xv) a five-to seven-membered cyclic heteroaryl carbonyl optionally having one to two substituents β to be described later and containing one to three heteroatoms selected from among a group consisting of oxygen, nitrogen, and sulfur, (xvi) carbamoyl, (xvii) a C₆-C₁₀ aryl carbamoyl optionally having one to five substituents β to be described later, (xviii) amino optionally substituted with one to two substituents β to be described later (excluding, however, (ii) a halogen), (xix) a C₁-C₆ halogenoalkoxy, (xx) a C₆-C₁₀ aryloxy optionally having one to five substituents β to be described later, (xxi) a C₆-C₁₀ aryl-C₁-C₆ alkoxy optionally having in the aryl portion one to five substituents β to be described later, (xxii) a C₁-C₆ alkoxycarbonyl, (xxiii) a C₆-C₁₀ aryloxycarbonyl optionally having in the aryl portion one to five substituents β to be described later, or (xxiv) a C₆-C₁₀ aryl-C₁-C₆ alkoxycarbonyl optionally having in the aryl portion one to five substituents β to be described later,
               the substituent β indicates the following (i)-(xi):
            (i) a C₁-C₁₀ alkyl, a C₁-C₆ halogenoalkyl, or a C₁-C₆ alkoxy, (ii) a halogen, (iii) a C₆-C₁₀ aryl optionally having one to five substituents γ to be described later, (iv) a C₆-C₁₀ aryl-C₁-C₆ alkyl optionally having in the aryl portion one to five substituents γ to be described later, (v) formyl, a C₁-C₆ alkylcarbonyl, or a C₂-C₆ alkenylcarbonyl, (vi) a C₆-C₁₀ aryl carbonyl optionally having one to five substituents γ to be described later, (vii) a C₆-C₁₀ aryl-C₁-C₆ alkylcarbonyl optionally having in the aryl portion one to five substituents γ to be described later, (viii) a C₃-C₁₀ cycloalkylcarbonyl, (ix) a five- to seven-membered cyclic heteroaryl carbonyl optionally having one to two substituents γ to be described later and containing one to three heteroatoms selected from among a group consisting of oxygen, nitrogen, and sulfur, (x) carbamoyl, or (xi) a C₆-C₁₀ aryl carbamoyl optionally having one to five substituents γ to be described later, and
               the substituent γ indicates (i) a C₁-C₆ alkyl or a C₂-C₆ alkoxy, (ii) a C₁-C₆ halogenoalkyl, (iii) a halogen, or (iv) hydroxy.
The present invention also relates to a pharmaceutical composition containing an active ingredient of a benzo- or pyrido-imidazole derivative represented by general formula (I), a pharmaceutically acceptable salt thereof, or a pharmaceutically acceptable ester or amide thereof (especially a pharmaceutical composition having both PPAR activity (especially γ activity) and angiotensin receptor antagonistic activity), use of a benzo- or pyrido-imidazole derivative represented by general formula (I), a pharmaceutically acceptable salt thereof, or a pharmaceutically acceptable ester or amide thereof as a pharmaceutical composition containing an active ingredient of a benzo- or pyrido-imidazole derivative represented by general formula (I), a pharmaceutically acceptable salt thereof, or a pharmaceutically acceptable ester or amide thereof (especially a pharmaceutical composition having both PPAR activity (especially γ activity) and angiotensin receptor antagonistic activity) for producing a pharmaceutical composition containing an active ingredient of a benzo- or pyrido-imidazole derivative represented by general formula (I), a pharmaceutically acceptable salt thereof, or a pharmaceutically acceptable ester or amide thereof (especially a pharmaceutical composition having both PPAR activity (especially γ activity) and angiotensin receptor antagonistic activity), and a method of prevention or treatment of diabetes or diabetes complications comprising administering an effective dose of a benzo- or pyrido-imidazole derivative represented by general formula (I), a pharmaceutically acceptable salt thereof, or a pharmaceutically acceptable ester or amide thereof to a warm-blooded animal (especially a human).

### EFFECTS OF THE INVENTION

The benzo- or pyrido-imidazole derivative represented by general formula (I), pharmaceutically acceptable salt thereof, or pharmaceutically acceptable ester or amide thereof of the present invention has a specific chemical formula, and consequently has both excellent PPAR activation activity and excellent angiotensin II receptor antagonistic activity. Some compounds (especially benzyl thiazolidine derivatives) have both selective PPAR γ activation activity and a body weight gain inhibiting effect, a body fat increase inhibiting effect, and the like.

### BEST MODE FOR CARRYING OUT THE INVENTION

In the present specification,
the "C₁-C₆ alkyl" in R^{a1}, R^{c1}, W^{c2}, R, the substituent α, and the like or the "C₁-C₆ alkyl" portion in, for example, the "C₁-C₆ halogenoalkyl" in R^{a1}, the substituent α, or the like, the "C₁-C₆ alkoxy" in R^{a1}, R^{a5}, the substituent α, or the like, the "C₆-C₁₀ aryl-C₁-C₆ alkyl optionally having one to five substituents α" of R^{a3} and the like, the "C₁-C₆ alkylcarbonylamino" of R^{a2} and the like, the "mono- or di-C₁-C₆ alkylcarbamoyl " of R^{a2} and the like, and the "C₁-C₆ hydroxyalkyl" of R^{a2} and the like is a straight-chain or branched-chain alkyl with one to six carbons; for example, methyl, ethyl, propyl, isopropyl, butyl, isobutyl, s-butyl, t-butyl, pentyl, s-pentyl, isopentyl, 2-methylbutyl, neopentyl, 1-ethylpropyl, hexyl, 4-methylpentyl (isohexyl), 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, or 2-ethylbutyl; advantageously a C₁-C₅ alkyl; more advantageously a C₁-C₄ alkyl; even more advantageously methyl, ethyl, propyl, isopropyl, or butyl; and optimally methyl, ethyl, propyl, or butyl.
The "C₁-C₁₀ alkyl" in the substituent β is, for example, the "C₁-C₆ alkyl" described earlier, heptyl, isoheptyl, octyl, isooctyl, nonyl, or decyl; advantageously a C₁-C₆ alkyl (especially a C₁-C₅ alkyl); more advantageously a C₁-C₄ alkyl; even more advantageously methyl, ethyl, propyl, isopropyl, or butyl; and optimally methyl, ethyl, propyl, or butyl.

The "C₆-C₁₀ aryl optionally having one to five substituents α" of R^{a3}, W^{c2}, R, and the like or the "C₆-C₁₀ aryl optionally having one to five substituents α" portion in, for example, the "C₆-C₁₀ aryl-C₁-C₆ alkyl optionally having one to five substituents α" of R^{a3}, W^{c1}, W^{c2}, R, and the like and the "C₆-C₁₀ arylsulfonyl optionally having one to five substituents α" of R^{c1} and the like is an aromatic hydrocarbon with six to ten carbons, optionally having one to five substituents α; for example, phenyl, indenyl, or 1- or 2-naphthyl optionally having one to five substituents α; advantageously phenyl or 1- or 2-naphthenyl optionally having one to three substituents α-1 to α-5 to be described later; and optimally phenyl having the substituent α-5 to be described later.
The "C₃-C₁₀ cycloalkyl" of R^{a1}, substituent α, and the like or the "C₃-C₁₀ cycloalkyl" portion in, for example, the "C₃-C₁₀ cycloalkylcarbonyl" of R^{c3}, W^{c2}, substituent α, and the like is an optionally condensed three- to ten-member saturated cyclic hydrocarbon; for example, cyclopropyl, cyclobutyl, cyclopentyl, cyclohexyl, cycloheptyl, norbornyl, or adamantyl; advantageously cyclopropyl, cyclobutyl, cyclopentyl, cyclohexyl, or adamantyl; more advantageously cyclopropyl, cyclobutyl, cyclopentyl, or cyclohexyl; and optimally cyclopropyl, cyclobutyl, or cyclohexyl.
The "halogen" of R^{a2}, substituent α, and the like or the "halogen" portion in, for example, the "C₁-C₆ halogen alkyl" and the like of R^{a1} and the like is, for example, fluorine, chlorine, bromine, or iodine; advantageously fluorine, chlorine, or bromine; and more advantageously fluorine or chlorine.
The "C₁-C₁₀ alkyl" portion in the "C₁-C₁₀ alkylcarbonyl" of R^{a2} is a straight-chain or branched-chain alkyl with one to ten carbons; for example, the "C₁-C₆ alkyl" described earlier, heptyl, octyl, nonyl, or decyl; advantageously a C₁-C₆ alkyl; more advantageously a C₁-C₅ alkyl; even more advantageously a C₁-C₄ alkyl; and optimally methyl, ethyl, propyl, or isopropyl.
The "C₂-C₆ alkenylcarbonyl" of R^{a2}, W^{c2}, R^{e1}, substituent α, and the like or the "C₂-C₆ alkenylcarbonyl" portion in, for example, the "C₂-C₆ alkenylcarbonylamino" of R^{a2} and the like is an unsaturated hydrocarbon-carbonyl with two to six carbons; for example, acryloyl, methacryloyl, crotonoyl, 2-pentenoyl, 2-hexenoyl, or 2-heptenoyl; advantageously a C₂-C₄ alkenylcarbonyl; more advantageously acryloyl or crotonoyl; and optimally acryloyl.

The "trivalent six-membered unsaturated hydrocarbon ring optionally having a substituent R^{a5} and optionally containing one to two atoms and/or groups selected from among oxygen, sulfur, nitrogen, and sulfone (-S(O)₂-), in which the hydrogen is optionally substituted with oxo, C₁-C₆ alkylidene, C₁-C₆ alkylimino, hydroxyimino, or C₁-C₆ alkoxyimino" contained in A¹ may be, for example, a trivalent phenyl, cyclohexenyl, pyranyl, thiopyranyl, dihydropyranyl, dihydrothiopyranyl, 4-oxodihydrothiobyranyl, 4-methylidenedihydrothiopyranyl, 4-methyliminodihydrothiopyranyl, 4-methoxyiminodihydropyranyl, 4-hydroxyiminodihydrothiopyranyl, 1,1,4-trioxodihydrothiopyranyl, pyridyl, pyridazinyl, pyrimidinyl, pyridinyl, dihydropyridyl, 2-oxodihydropyridyl, 4-oxodihydropyridyl, 2-methylidenedihydropyridyl, 2-methyliminodihydropyridyl, dihydropyridazinyl, 3-oxodihydropyridazinyl, dihydropyrimidinyl, 4-oxodihydropyrimidinyl, dihydropyrazinyl, tetrahydropyridyl, 2-oxotetrahydropyridyl, 4-oxotetrahydropyridyl, tetrahydropyridazinyl, 3-oxotetrahydropyridazinyl, 3,6-dioxotetrahydropyridazinyl, tetrahydropyrimidyl, 2-oxotetrahydropyrimidinyl, 2,6-dioxotetrahydropyrimidyl, or tetrahydropyridinyl that is condensed with an imidazole ring and optionally has a substituent R^{a5}; advantageously a trivalent phenyl, thiopyranyl, dihydropyranyl, dihydrothiopyranyl, 4-oxodihydrothiopyranyl, 4-methyliminodihydrothiopyranyl, 4-methoxyiminodihydrothiopyranyl, 4-hydroxyiminodihydrothiopyranyl, 1,1,4-trioxodihydrothiopyranyl, pyridyl, pyridazinyl, pyrimidinyl, pyrazinyl, dihydropyridyl, 2-oxodihydropyridyl, 4-oxodihydropyridyl, dihydropyridazinyl, 3-oxodihydropyridazinyl, dihydropyrimidinyl, 4-oxodihydropyrimidinyl, 2-oxotetrahydropyridyl, 4-oxotetrahydropyridyl, tetrahydropyridazinyl, 3-oxotetrahydropyridazinyl, 3,6-dioxotetrahydropyridazinyl, tetrahydropyrimidinyl, 2-oxotetrahydropyrimidinyl, or 2,6-dioxotetrahydropyrimidinyl that optionally has a substituent R^{a5}; more advantageously a trivalent phenyl or pyridyl that optionally has one to three substituents selected from among a group consisting of C₁-C₄ alkyl, C₁-C₄ alkoxy, fluorine, chlorine, bromine, halogeno-C₁-C₄ alkyl, and carboxy, or a dihydrothiopyranyl, 4-oxodihydrothiopyranyl, 4-methoxyiminodihydrothiopyranyl, 4-hydroxyiminodihydrothiopyranyl, 1,1,4-trioxodihydrothiopyranyl, dihydropyridyl, 2-oxodihydropyridyl, 4-oxodihydropyridyl, 3,6-dioxotetrahydropyridazinyl, or 2,6-dioxotetrahydropyrimidinyl that is optionally substituted with a C₁-C₃ alkyl; even more advantageously a trivalent phenyl or pyridyl, 4-oxodihydrothiopyranyl, 4-oxodihydropyridyl, 3,6-dioxotetrahydropyridazinyl, or 2,6-dioxotetrahydropyrimidinyl that optionally has methyl, ethyl, propyl, isopropyl, methoxy, ethoxy, fluorine, chlorine, fluoromethyl, trifluoromethyl, pentafluoroethyl, or carboxy; and optimally methylphenyl, ethylphenyl, isopropylphenyl, methoxyphenyl, chlorophenyl, trifluorophenyl, fluorophenyl, 4-methylpyridyl, 4-oxodihydrothiopyranyl, 4-oxodihydropyridyl, or 2,6-dioxotetrahydropyrimidinyl.
The "five- to seven-membered cyclic heteroaryl carbonyl optionally having one to two substituents α and containing one to three heteroatoms selected from among a group consisting of oxygen, nitrogen, and sulfur" of R^{c3}, W^{c2}, and the like or the "five- to seven-membered cyclic heteroaryl carbonyl optionally having one to two substituents β and containing one to three heteroatoms selected from among a group consisting of oxygen, nitrogen, and sulfur" of the substituent α may be, for example, a furylcarbonyl, thienylcarbonyl, pyrrolylcarbonyl, pyrazolylcarbonyl, imidazolylcarbonyl, oxazolylcarbonyl, isooxazolylcarbonyl, thiazolylcarbonyl, isothiazolylcarbonyl, 1,2,3-oxadiazolylcarbonyl, triazolylcarbonyl, thiadiazolylcarbonyl, pyranylcarbonyl, nicotinoyl, isonicotinoyl, picolinyl, pyridazinylcarbonyl, pyrimidinylcarbonyl, pyrazinylcarbonyl, or azepinylcarbonyl having this substituent; advantageously a furylcarbonyl, thienylcarbonyl, pyrrolylcarbonyl, pyrazolylcarbonyl, imidazolylcarbonyl, oxazolylcarbonyl, isooxazolylcarbonyl, thiazolylcarbonyl, isothiazolylcarbonyl, pyranylcarbonyl, nicotinoyl, isonicotinoyl, picolinyl, pyridazinylcarbonyl, pyrimidinylcarbonyl, or pyrazinylcarbonyl optionally having one to two substituents α-1 to α-5, substituents β-1 to β-5, or substituents γ-1 to γ-3 to be described later; more advantageously a furylcarbonyl, thienylcarbonyl, pyrrolylcarbonyl, imidazolylcarbonyl, oxazolylcarbonyl, isooxazolylcarbonyl, thiazolylcarbonyl, isothiazolylcarbonyl, nicotinoyl, or isonicotinyl optionally having one to two substituents selected from among a group consisting of C₁-C₄ alkyl, C₁-C₄ alkoxy, fluorine, chlorine, bromine, and C₁-C₃ halogenoalkyl; even more advantageously a furylcarbonyl, thienylcarbonyl, oxazolylcarbonyl, isooxazolylcarbonyl, thiazolylcarbonyl, isothiazolylcarbonyl, nicotinoyl, or isonicotinyl optionally having one to two substituents selected from among a group consisting of methyl, ethyl, methoxy, ethoxy, fluorine, chlorine, and trifluoromethyl; even more advantageously a furylcarbonyl, thienylcarbonyl, nicotinoyl, or isonicotinyl optionally having one to two substituents selected from among a group consisting of methyl, methoxy, fluorine, chlorine, and trifluoromethyl; and optimally 2-methylfurylcarbonyl, 2,5-dimethylfurylcarbonyl, 2-chloronicotinoyl, 4-chloronicotinoyl, 6-chloronicotinoyl, 2-chloro-6-methylnicotinoyl, 2-chloro-6-trifluoromethylnicotinoyl, 2-fluoronicotinoyl, 2-methoxynicotinoyl, 3,5-dichloroisonicotinoyl, 2-fluoroisonicotinoyl, or 2-methoxyisonicotinoyl.

The "C₁-C₁₀ alkylene optionally containing one to three atoms and/or groups selected from among a group consisting of (a) oxygen, (b) groups represented by the expression -S(O)_{q}-(where q indicates 0 or an integer from 1 to 2), (c) groups represented by the expression - N(R^{e2})- (where R^{e2} is defined as described earlier), (d) groups represented by the expression - CO-N(R^{e2})- (where R^{e2} is defined as described earlier), (e) groups represented by the expression -N(R^{e2})-CO- (where R^{e2} is defined as described earlier), and (f) groups represented by the expression -CO-," of W^{c3} may be, for example, a group represented by the expression -CH₂-, -(CH₂)₂-, -CH(CH₃)-, - (CH₂) ₃-, -CH (CH₃)-CH₂-, -CH(C₂H₅)-, -C(CH₃)₂-, -(CH₂)₄-, -(CH₂)₂-CH(CH₃)-, -(CH₂)₅-, - (CH₂)₆-, -(CH₂)₇-, -(CH₂)₈-, -(CH₂)₉-, -(C H₂)₁₀-, -CH₂-O-, -CH₂-S-, -CH₂-SO-, -CH₂-SO₂-, -CH₂-NH-, -CH₂-N(CH₃)-, -CH₂-N(C₂H ₅)-, -CH₂-N(C₃H₇)-, -CH₂-N(i-C₃H₇)-, -CH₂ -N (phenyl)-, -CH₂-N (4-methylphenyl), -CH₂-N (benzyl)-, -CH₂-N (methylsulfonyl), -CH₂-N (trifluoromethylsulfonyl)-, -CH₂-N (benzenesulfonyl)-, -CH₂-N (benzylsulfonyl)-, -CH₂-N (formyl)-, -CH₂-N (acetyl)-, -CH₂-N acryloyl)-, -CH₂-N (benzoyl)-, -CH₂-N (benzylcarbonyl)-, -CH₂-N (cyclopropylcarbonyl)-, -CH₂-N (furylcarbonyl)-, -CH₂-N (thienylcarbonyl)-, -CH₂-N (carbamoyl)-, -CH₂-N (methylcarbamoyl)-, -CH₂-CONH-, - CH₂-CON(CH₃)-, -CH₂-CON(C₂H₅)-, -CH₂-N (C₂H₅)CO-, -CH₂-CON(acetyl)-, -CH₂-NHCO-, -CH₂-N(CH₃)CO-, -CH₂-N(C₂H₅)CO-, -CH₂-N (acetyl)CO-, -CH₂-CO-, -OCH₂-, -SCH₂-, -NHCH₂-, -N(CH₃)-CH₂-, -CONH-CH₂ -, -CON(CH₃)-CH₂-, -NHCO-CH₂-, -N(CH₃)C O-CH₂-, -CO-CH₂-, -(CH₂)₂-O-, -(CH₂)₂-S-, -(CH₂)₂-(NH-, -(CH₂)₂-N(CH₃)-, -(CH₂)₂-CONH-, -(CH₂)₂-CON(CH₃)-, - (CH₂)₂-NHCO-, -(CH₂)₂-N(CH₃)CO-, -(CH₂)₂-CO-, -O-(CH ₂)₂-, -S-(CH₂)₂-, -NH-(CH₂)₂-, -N(CH₃)-( CH₂)₂-, -CONH-(CH₂)₂-, -CON(CH₃)-(CH₂)₂ -, -NHCO-(CH₂)₂-, -N(CH₃)CO-(CH₂)₂-, -CO -(CH₂)₂-, -CH(CH₃)-O-, -CH(CH₃)-S-, -CH (CH₃)-NH-, -CH(CH₃)-N(CH₃)-, -CH(CH₃)-CONH-, -CH(CH₃)-CON(CH₃)-, -CH(CH₃)-NH CO-, -CH(CH₃)-N(CH₃)CO-, -CH(CH₃)-CO-, -O-CH(CH₃)-, -S-CH(CH₃)-, -NH-CH(CH₃)-, -N(CH₃)-CH(CH₃)-, -CONH-CH(CH₃)-, -CO N(CH₃)-CH(CH₃)-, -NHCO-CH(CH₃)-, -N(CH ₃)CO-CH(CH₃)-, -CO-CH(CH₃)-, - (CH₂)₃-O-, -(CH₂)₃-S-, -(CH₂)₃-NH-, -(CH₂)₃-N(CH₃ )-, -(CH₂)₃-CONH-, -(CH₂)₃-CON(CH₃)-, -( CH₂)₃-NHCO-, -(CH₂)₃-N(CH₃)CO-, -(CH₂)₃ -CO-, **-**O**-**(CH₂)₃-, -S-(CH₂)₃-, -NH-(CH₂)₃ -, -N(CH₃)-(CH₂)₃-, -CONH-(CH₂)₃-, -CON( CH₃)-(CH₂)₃-, -NHCO-(CH₂)₃-, -N(CH₃)CO-(CH₂)₃-, -CO-(CH₂)₃-, -CH(CH₃)CH₂-O-, -C H(CH₃)CH₂-S-, -CH(CH₃)CH₂-NH-, -CH(CH₃) CH₂-N(CH₃)-, -CH(CH₃)CH₂-CONH-, -CH(CH₃ )CH₂-CON(CH₃)-, -CH(CH₃)CH₂-NHCO-, -CH (CH₃)CH₂-N(CH₃)CO-, -CH(CH₃)CH₂-CO-, -O -CH(CH₃)CH₂-, -S-CH(CH₃)CH₂-, -NH-CH(C H₃)CH₂-, -N(CH₃)-CH(CH₃)CH₂-, -CONH-CH( CH₃)CH₂-, -CON(CH₃)-CH(CH₃)CH₂-, -NHCO-CH(CH₃)CH₂-, -N(CH₃)CO-CH(CH₃)CH₂-, -CO -CH(CH₃)CH₂-, -CH₂-O-(CH₂)₂-, -CH₂-S- (C H₂)₂-, -CH₂-O-CH₂-, -CH₂-S-CH₂-, -CH₂-NH -(CH₂)₂-, -(CH₂)₂-O-CH₂-, -(CH₂)₂-S-CH₂-, -C(CH₃)₂-O-CH₂-, -CH(CH₃)-S-CH₂-, -C(C H₃)₂-S-(CH₂)₂-O-, -(CH₂)₂-NH-CH₂-, -(CH₂ )₂-O-(CH₂)₂-, -(CH₂)₂-S-(CH₂)₂-, -(CH₂)₂ -NH-(CH₂)₂-, -CH-₂-O-(CH₂)₃-, -CH₂-S-(CH ₂)₃-, -CH₂-NH-(CH₂)₃-, -(CH₂)₂-O-(CH₂)₃-, -(CH₂)₂-S-(CH₂)₃-, -(CH₂)₂-(NH-(CH₂)₃-, -(CH₂)₃-O-(CH₂)₂-, -(CH₂)₃-S-(CH₂)₂-, -( CH₂)₃-NH-(CH₂)₂-, -O-(CH₂)₂-O-, -O-(CH₂ )₂-S-, -O-(CH₂)₂-NH-, -S-(CH₂)₂-O-, -S-( CH₂)₂-S-, -S-(CH₂)₂-NH-, -NH-(CH₂)₂-O-, -N(CH₃)-(CH₂)₂-O-, -NH-(CH₂)₂-S-, -NH-( CH₂)₂-NH-, -CONH-(CH₂)₂-O-, -CONH-(CH₂) ₂-S-, -CONH-(CH₂)₂-NH-, -CON(CH₃)-(CH₂) ₂-O-, -CON(CH₃)-(CH₂)₂-S-, -CON(CH₃)-(C H₂)₂-NH-, -CON(CH₃)-(CH₂)₂-N(CH₃)-, -CH ₂-O-(CH₂)₂-O-, -CH₂-O-(CH₂)₂-S-, -CH₂-O -(CH₂)₂-NH-, -CH₂-S-(CH₂)₂-O-, -CH₂-S-( CH₂)₂-S-, -CH₂-S-(CH₂)₂-NH-, -CH₂-NH-(C H₂)₂-O-, -CH₂-NH-(CH₂)₂-S-, -CH₂-NH-(CH ₂)₂-NH-, -CH₂-S(O)-(CH₂ )₂-O-, -CH₂-S(O)₂-(CH₂)₂-O-, -CH₂-O-(CH ₂)₃-O-, -CH₂-O-(CH₂)₃-S-, -CH₂-O-(CH₂)₃-NH-, -CH₂-S-(CH₂)₃-O-, -CH₂-S-(CH₂)₃-S-, -CH₂-S-(CH₂)₃-NH-, -CH₂-NH-(CH₂)₃-O-, -CH₂-NH-(CH₂)₃-S-, -CH₂-NH-(CH₂)₃-NH-, -CH₂-S-CH₂-, -NHCO-CH₂-S-, -CH₂-S-(CH₂) ₂-NHCO-CH₂-S-, -NHCO-CH₂-S-, or -CH(CH₃)-S-(CH₂)₂-O-;
advantageously a group represented by the expression
-CH₂-, -(CH ₂)₂-, -CH(CH₃)-, -(CH₂)₃-, -CH(CH₃)-CH₂-, -CH₂-O-, -CH₂-S-, -CH₂-SO-, -CH₂-SO₂-, - CH₂-NH-, -CH₂-N(CH₃)-, -CH₂-N(phenyl)-, -CH2-N (benzyl)-, -CH₂-N(methylsulfonyl)-, -CH₂-N(trifluoromethylsulfonyl)-, -CH₂-N (benzylsulfonyl)-, -CH₂-N(formyl)-, -CH₂-N(acetyl)-, -CH₂-N(benzoyl)-, -CH₂-CONH-, -CH₂-CON(CH₃)-, -CH₂-CON(C₂H₅)-, -N(CH₃)-CH₂-, -CONH-CH ₂-, -CON(CH₃)-CH₂-, -(CH₂)₂-O-, -CH(CH₃) -O-, -CH(CH₃)-S-, -CH(CH₃)-NH-, -CH(CH₃ ) -N(CH₃)-, -CH₂-O-CH₂-, -CH₂-S-CH₂-, -CH ₂-O-(CH₂)₂-, -CH₂-S-(CH₂)₂-, -CH₂-NH-(C H₂)₂-, -(CH₂)₂-O-CH₂-, -(CH₂)₂-S-CH₂-, -C (CH₃)₂-O-CH₂-, -CH(CH₃)-S-CH₂-, -C(CH₃) ₂-S-(CH₂)₂-O-, -(CH₂)₂-NH-CH₂-, -(CH₂)₂-O-(CH₂)₂-, -(CH₂)₂-S-(CH₂)₂-, -N(CH₃)-( CH₂)₂-O-, -CONH-(CH₂)₂-O-, -CONH-(CH₂)₂ -S-, -CONH-(CH₂)₂-NH-, -CON(CH₃)-(CH₂)₂ -O-, -CON(CH₃)-(CH₂)₂-S-, -CON(CH₃)-(C H₂)₂-NH-, -CON(CH₃)-(CH₂)₂-N(CH₃)-, -CH ₂-O-(CH₂)₂-O-, -CH₂-O-(CH₂)₂-S-, -CH₂-O -(CH₂)₂-NH-, -CH₂-S-(CH₂)₂-O-, -CH₂-S-( CH₂)₂-S-, -CH₂-S-(CH₂)₂-NH-, -CH₂-NH-(C H₂)₂-O-, -CH₂-NH-(CH₂)₂-S-, -CH₂-NH-(CH ₂)₂-NH-, -CH₂-S(O)-(CH₂)₂-O-, -CH₂-S(O) ₂-(CH₂)₂-O-, -CH₂-S-CH₂-, -NHCO-CH₂-S-, -CH₂-S-(CH₂)₂-NHCO-CH₂-S-, -S-CH₂-, -NH CO-CH₂-S-, or -CH(CH₃)-S-(CH₂)₂-O-;
more advantageously a group represented by the expression
-CH₂-, -(CH₂)₂-, -CH(CH₃)-, - CH₂-O-, -CH₂-S-, -CH₂-SO-, -CH₂-SO₂-, -CH ₂-NH-, -CH₂-N(CH₃)-, -CH₂-N phenyl,)-, -CH₂ -N(benzyl)-, -CH₂-N(methylsulfonyl)-, -CH₂-N(trifluoromethylsulfonyl)-, -CH₂-N (benzylsulfonyl)-, -CH₂-N(formyl)-, -CH₂-N(acetyl)-, -CH₂-N(benzoyl)-, -CH₂-CONH-, -CH₂-CON(CH₃)-, -C ONH-CH₂-, -(CH₂)₂-O-, -CH(CH₃)-S-, -CH₂-O-CH₂-, -CH₂-S-CH₂-, -C(CH₃)₂-O-CH₂-, -C H (CH₃)-S-CH₂-, -C(CH₃)₂-S-(CH₂)₂-O-, -N (CH₃)-(CH₂)₂-O-, -CH₂-O-(CH₂)₂-O-, -CH₂ -O-(CH₂)₂-S-, -CH₂-O-(CH₂)₂-NH-, -CH₂-S - (CH₂)₂-O-, -CH₂-S-(CH₂)₂-S-, -CH₂-S-(C H₂)₂-NH-, -CH₂-NH-(CH₂)₂-O-, -CH₂-NH-(C H₂)₂-S-, -CH₂-NH-(CH₂)₂-NH-, -CH₂-S(O)-(CH₂)₂-O-, -CH₂-S(O)₂-(CH₂)₂-O-, -CH₂-S -CH₂-, -CH₂-S-(CH₂)₂-NHCO-CH₂-S-, -S-C H₂-, or -CH(CH₃)-S-(CH₂)₂-O-; and optimally a group represented by the expression
-CH₂-, -CH₂-O-, -CH₂-S-, -CH₂-SO-, - CH₂-SO₂-, -CH₂-NH-, -CH₂-N(CH₃)-, -CH₂-C ONH-, -CH₂-CON(CH₃)-, -CONH-CH₂-, -(CH₂ )₂-O-, -CH(CH₃)-S-, -N(CH₃)-(CH₂)₂-O-, - CH(CH₃)-S-CH₂-, -CH₂-O-(CH₂)₂-O-, -CH₂-O-(CH₂)₂-S-, -CH₂-S-(CH₂)₂-O-, -CH₂-S-( CH₂)₂-S-, -CH₂-S-(CH₂)₂-NH-, -CH₂-S(O)-(CH₂)₂-O-, -CH₂-S(O)₂-(CH₂)₂-O-, -CH₂-S -CH₂-, -CH₂-S-(CH₂)₂-NHCO-CH₂-S-, or -S-CH₂-.The "C₁-C₁₀ alkylene optionally containing one to five atoms and/or groups selected from among a group consisting of oxygen, groups represented by the expression -S(O)_{q}-, groups represented by the expression -N(R^{e2})-, and groups represented by the expression -N(R^{e2})-CO-(where q and R^{e2} are defined as in "D-1(2)")" in "D-1(2)" of "E" to be described later may be, for example, a group in which a C₁-C₁₀ alkylene having a group represented by the expression - CO- has been deleted from the "C₁-C₁₀ alkylene optionally containing one to three atoms and/or groups selected from among a group consisting of (a) oxygen, (b) groups represented by the expression -S(O)_{q}- (where q indicates an integer from 0 to 2), (c) groups represented by the expression -N(R^{e2})- (where R^{e2} is defined as described earlier), (d) groups represented by the expression -CO-N(R^{e2})- (where R^{e2} is defined as described earlier), (e) groups represented by the expression -N(R^{e2})-CO- (where R^{e2} is defined as described earlier), and (f) groups represented by the expression -CO-" of W^{c3} described earlier, or a group represented by the expression -O-( CH₂)₂-O- (CH₂)₂-O-(CH₂)₂-O-(CH₂)₂-O-(CH ₂)₂-, -S-(CH₂)₂-O-(CH₂)₂-O-(CH₂)₂-O-(CH ₂)₂-O-(CH₂)₂-, -NH-(CH₂)₂-O-(CH₂)₂-O-(C H₂)₂-O-(CH₂)₂-O-(CH₂)₂-, -N(CH₃)-(CH₂)₂ -O-(CH₂)₂-O-(CH₂)₂-O-(CH₂)₂-O-(CH₂)₂-, -S-(CH₂)₂-S-(CH₂)₂-S-(CH₂)₂-S-(CH₂)₂-S -(CH₂)₂-, -O-(CH₂)₂-S-(CH₂)₂-S-(CH₂)₂-S -(CH₂)₂-S-(CH₂)₂-, -NH-(CH₂)₂-S-(CH₂)₂-S-(CH₂)₂-S-(CH₂)₂-S-(CH₂)₂-, -N(CH₃)-( CH₂)₂-S-(CH₂)₂-S-(CH₂)₂-S-(CH₂)₂-S-(CH ₂)₂-, -O-(CH₂)₂-NHCO-(CH₂)₂-O-(CH₂)₂-O-(CH₂)₂-O-(CH₂)₂-, -S-(CH₂)₂-NHCO-(CH₂)₂ -O-(CH₂)₂-O-(CH₂)₂-O-(CH₂)₂-, -NH-(CH₂ )₂-NHCO-(CH₂)₂-O-(CH₂)₂-O-(CH₂)₂-O-(C H₂)₂-, -N(CH₃)-(CH₂)₂-NHCO-(CH₂)₂-O-(C H₂)₂-O-(CH₂)₂-O-(CH₂)₂-, -S-(CH₂)₂-NHCO -(CH₂)₂-S-(CH₂)₂-S-(CH₂)₂-S-(CH₂)₂-, -O -(CH₂)₂-NHCO-(CH₂)₂-S-(CH₂)₂-S-(CH₂)₂-S-(CH₂)₂-, -NH-(CH₂)₂-NHCO-(CH₂)₂-S-(C H₂)₂-S-(CH₂)₂-S-(CH₂)₂-, -N(CH₃)-(CH₂)₂ -NHCO-(CH₂)₂-S-(CH₂)₂-S-(CH₂)₂-S-(CH₂ )₂-, -O-(CH₂)₂-N(CH₃)CO-(CH₂)₂-O-(CH₂)₂ -O-(CH₂)₂-O-(CH₂)₂-, -S-(CH₂)₂-N(CH₃)C O-(CH₂)₂-O-(CH₂)₂-O-(CH₂)₂-O-(CH₂)₂-, - NH-(CH₂)₂-N(CH₃)CO-(CH₂)₂-O-(CH₂)₂-O-(CH₂)₂-O-(CH₂)₂-, -N(CH₃)-(CH₂)₂-N(CH₃) CO-(CH₂)₂-O-(CH₂)₂-O-(CH₂)₂-O-(CH₂)₂-, -S-(CH₂)₂-N(CH₃)CON-(CH₂)₂-S-(CH₂)₂-S-(CH₂)₂-S-(CH₂)₂-, -O-(CH₂)₂-N(CH₃)CO-( CH₂)₂-S-(CH₂)₂-S-(CH₂)₂-S-(CH₂)₂-, -NH-(CH₂)₂-N(CH₃)CO-(CH₂)₂-S-(CH₂)₂-S-(CH₂ )₂-S-(CH₂)₂-, -N(CH₃)-(CH₂)₂-N(CH₃)CO-(CH₂)₂-S-(CH₂)₂-S-(CH₂)₂-S-(CH₂)₂-, -O-(CH₂)₂-CONH-(CH₂)₂-O-(CH₂)₂-O-(CH₂)₂-O -(CH₂)₂-, -S-(CH₂)₂-CONH-(CH₂)₂-O-(CH₂ )₂-O-(CH₂)₂-O-(CH₂)₂-, -NH-(CH₂)₂-CONH -(CH₂)₂-O-(CH₂)₂-O-(CH₂)₂-O-(CH₂)₂-, -N (CH₃)-(CH₂)₂-CONH-(CH₂)₂-O-(CH₂)₂-O-( CH₂)₂-O-(CH₂)₂-, -S-(CH₂)₂-CONH-(CH₂)₂-S-(CH₂)₂-S-(CH₂)₂-S-(CH₂)₂-, -O-(CH₂)₂-CONH-(CH₂)₂-S-(CH₂)₂-S-(CH₂)₂-S-(CH₂)₂ -, -NH-(CH₂)₂-CONH-(CH₂)₂-S-(CH₂)₂-S-( CH₂)₂-S-(CH₂)₂-, -N(CH₃)-(CH₂)₂-CONH-( CH₂)₂-S(CH₂)₂-S-(CH₂)₂-S-(CH₂)₂-, -O-( CH₂)₂-O-(CH₂)₂-O-(CH₂)₂-O-(CH₂)₂-O-, -S -(CH₂)₂-O-(CH₂)₂-O-(CH₂)₂-O-(CH₂)₂-O-, -NH-(CH₂)₂-O-(CH₂)₂-O-(CH₂)₂-O-(CH₂)₂-O-, -N(CH₃)-(CH₂)₂-O-(CH₂)₂-O-(CH₂)₂-O -(CH₂)₂-O-, -S-(CH₂)₂-S-(CH₂)₂-S-(CH₂)₂ -S-(CH₂)₂-S-, -O-(CH₂)₂-S-(CH₂)₂-S-(CH ₂)₂-S-(CH₂)₂-S-, -NH-(CH₂)₂-S-(CH₂)₂-S-(CH₂)₂-S-(CH₂)₂-S-, -N(CH₃)-(CH₂)₂-S-( CH₂)₂-S-(CH₂)₂-S-(CH₂)₂-S-, -O-(CH₂)₂-N HCO-(CH₂)₂-O-(CH₂)₂-O-(CH₂)₂-O-, -S-(C H₂)₂-NHCO-(CH₂)₂-O-(CH₂)₂-O-(CH₂)₂-O-, -NH-(CH₂)₂-NHCO-(CH₂)₂-O-(CH₂)₂-O-(CH ₂)₂-O-, -N(CH₃)-(CH₂)₂-NHCO-(CH₂)₂-O-( CH₂ ) ₂-O-(CH₂)₂-O-, -S-(CH₂)₂-NHCO-(CH₂)₂-S-(CH₂)₂-S(CH₂)₂-S-, -O-(CH₂)₂-NHCO-( CH₂)₂-S-(CH₂)₂-S-(CH₂)₂-S-, -NH-(CH₂)₂-NHCO-(CH₂)₂-S-(CH₂)₂-S-(CH₂)₂-S-, -N(C H₃)-(CH₂)₂-NHCO-(CH₂)₂-S-(CH₂)₂-S-(CH₂ ) ₂-S-, -O-(CH₂)₂-N(CH₃)CO-(CH₂)₂-O-(CH ₂)₂-O-(CH₂)₂-O-, -S-(CH₂)₂-N(CH₃)CO-(C H₂)₂-O-(CH₂)₂-O-(CH₂)₂-O-, -NH-(CH₂)₂-N (CH₃)CO-(CH₂)₂-O-(CH₂)₂-O-(CH₂)₂-O-, - N (CH₃)-(CH₂)₂-N(CH₃)CO-(CH₂)₂-O-(CH₂)₂ -O-(CH₂)₂-O-, -S-(CH₂)₂-N(CH₃)CO-(CH₂) ₂-S-(CH₂)₂-S-(CH₂)₂-S-, -O-(CH₂)₂-N(CH₃ ) CO-(CH₂)₂-S-(CH₂)₂-S-(CH₂)₂-S-, -NH-( CH₂)₂-N(CH₃)CO-(CH₂)₂-S-(CH₂)₂-S-(CH₂) ₂-S-, -N(CH₃)-(CH₂)₂-N(CH₃)CO-(CH₂)₂-S -(CH₂)₂-S-(CH₂)₂-S-, -O-(CH₂)₂-CONH-(C H₂)₂-O-(CH₂)₂-O-(CH₂)₂-O-, -S-(CH₂)₂-CO NH-(CH₂)₂-O-(CH₂)₂-O-CH₂)₂-O-, -NH-(CH ₂)₂-CONH-(CH₂)₂-O-(CH₂)₂-O-(CH₂)₂-O-, - N(CH₃)-(CH₂)₂-CONH-(CH₂)₂-O-(CH₂)₂-O-(CH₂)₂-O-, -S-(CH₂)₂-CONH-(CH₂)₂-S-(CH ₂)₂-S-(CH₂)₂-S-, -O-(CH₂)₂-CONH-(CH₂)₂-S-(CH₂)₂-S-(CH₂)₂-S-, -NH-(CH₂)₂-CONH-(CH₂)₂-S-(CH₂)₂-S-(CH₂)₂-S-, -N(CH₃)-( CH₂)₂-CONH-(CH₂)₂-S-(CH₂)₂-S-(CH₂)₂-S-, -CH₂-O-(CH₂)₂-O-(CH₂)₂-O-(CH₂)₂-O-(C H₂)₂-O-, -CH₂-S-(CH₂)₂-O-(CH₂)₂-O-(CH₂) ₂-O-(CH₂)₂-O-, -CH₂-NH-(CH₂)₂-O-(CH₂)₂-O-(CH₂)₂-O-(CH₂)₂-O-, -CH₂-N(CH₃)-(CH₂ )₂-O-(CH₂)₂-O-(CH₂)₂-O-(CH₂)₂-O-, -CH₂-S-(CH₂)₂-S-(CH₂)₂-S-(CH₂)₂-S-(CH₂)₂-S-, -CH₂-O-(CH₂)₂-S-(CH₂)₂-S-(CH₂)₂-S-(C H₂)₂-S-, -CH₂-NH-(CH₂)₂-S-(CH₂)₂-S-(CH₂ ) ₂-S-(CH₂)₂-S-, -CH₂-N(CH₃)-(CH₂)₂-S-( CH₂) ₂-S- (CH₂) ₂-S- (CH₂) ₂-S-, -CH₂-O- (CH₂ ) ₂-NHCO- (CH₂) ₂-O- (CH₂) ₂-O- (CH₂) ₂-O-, - CH₂-S- (CH₂) ₂-NHCO- (CH₂) ₂-O- (CH₂) ₂-O- ( CH₂) ₂-O-, -CH₂-NH- (CH₂) ₂-NHCO- (CH₂) ₂-O **-** (CH₂) ₂-O (CH₂) ₂-O-, -CH₂-N (CH₃) - (CH₂) ₂ -NHCO- (CH₂) ₂-O- (CH₂) ₂-O- (CH₂) ₂-O-, -CH ₂-S- (CH₂) ₂-NHCO- (CH₂) ₂-S- (CH₂) ₂-S- (CH₂ ) ₂-S-, -CH₂-O- (CH₂) ₂-NHCO- (CH₂) ₂-S- (CH ₂) ₂-S- (CH₂) ₂-S-, -CH₂-NH- (CH₂) ₂-NHCO- ( CH₂) ₂-S- (CH₂) ₂-S- (CH₂) ₂-S-, -CH₂-N (CH₃) - (CH₂) ₂-NHCO- (CH₂) ₂-S- (CH₂) ₂-S- (CH₂) ₂-S-, -CH₂-O- (CH₂) ₂-N (CH₃) CO- (CH₂) ₂-O- (C H₂) ₂-O- (CH₂) ₂-O-, -CH₂-S- (CH₂) ₂-N (CH₃) C O- (CH₂) ₂-O- (CH₂) ₂-O- (CH₂) ₂-O-, -CH₂-NH - (CH₂) ₂-N (CH₃) CO- (CH₂) ₂-O- (CH₂) ₂-O- (C H₂) ₂-O-, -CH₂-N (CH₃) - (CH₂) ₂-N (CH₃) CO- ( CH₂) ₂-O- (CH₂) ₂-O- (CH₂) ₂-O-, -CH₂-S- (CH₂ ) ₂-N (CH₃) CO- (CH₂) ₂-S- (CH₂) ₂-S- (CH₂) ₂-S -, -CH₂-O- (CH₂) ₂-N (CH₃) CO- (CH₂) ₂-S- (CH ₂) ₂-S- (CH₂) ₂-S-. -CH₂- NH- (CH₂) ₂-N (CH₃) C O- (CH₂) ₂-S- (CH₂) ₂-S- (CH₂) ₂-S-, -CH₂-N ( CH₃) - (CH₂) ₂-N (CH₃) CO- (CH₂) ₂-S- (CH₂) ₂-S - (CH₂) ₂-S-, -CH₂-O- (CH₂) ₂-CONH- (CH₂) ₂-O- (CH₂) ₂-O- (CH₂) ₂-O-, -CH₂-S- (CH₂) ₂-CO NH- (CH₂) ₂-O- (CH₂) ₂-O-CH₂) ₂-O-, -CH₂-NH - (CH₂) ₂-CONH- (CH₂) ₂-O- (CH₂) ₂-O- (CH₂) ₂-O-, -CH₂-N (CH₃) - (CH₂) ₂-CONH- (CH₂) ₂-O- ( CH₂) ₂-O- (CH₂) ₂-O-, -CH₂-S- (CH₂) ₂-CONH-(CH₂)₂-S- (CH₃) ₂-S- (CH₂)₂-S-, -CH₂-O- (C H₂) ₂-CONH- (CH₂) ₂-S- (CH₂) ₂-S- (CH₂) ₂ -S-, -CH₂-NH- (CH₂) ₂-CONH- (CH₂) ₂-S- (CH₂) ₂-S - (CH₂) ₂-S-or-CH₂-N (CH₃) - (CH₂) ₂-CONH - (CH₂) ₂-S- (CH₂) ₂-S- (CH ₂) ₂- S-.Specific groups of the "C₁-C₄ alkylene optionally containing one to three atoms and/or groups selected from among a group consisting of oxygen, groups represented by the expression -S(O)_{q}-, groups represented by the expression -N(R^{e2})-, groups represented by the expression -CO- N(R^{e2})-, and groups represented by the expression -N(R^{e2})-CO- (where q and R^{e2} are defined as in "D-2(2)")" in "D-2(2)" are the groups indicated earlier as "advantageous," and specific groups of the "C₁-C₄ alkylene optionally containing one to three atoms and/or groups selected from among a group consisting of oxygen, groups represented by the expression -S(O)_{q}-, groups represented by the expression -N(R^{e2})-, groups represented by the expression -CO- N(R^{e2})-, and groups represented by the expression -N(R^{e2})-CO- (where q and R^{e2} are defined as in "D-3(2)")" in "D-3(2)" are the groups indicated earlier as "more advantageous."

The "C₁-C₁₀ alkylene optionally containing one to three atoms and/or groups selected from among a group consisting of oxygen, sulfur, and groups represented by the expression - N(R^{e2})- (where R^{e2} is defined as described earlier) and the expression -CO-N(R^{e2})- (where R^{e2} is defined as described earlier)" (advantageously, C₁-C₁₀ alkylene containing one to three of these atoms and/or groups) of X^{e2} and X^{e4} are, for example, a group in which a C₁-C₁₀ alkylene containing a group represented by the expression -SO-, a group represented by the expression - S(O) ₂-, a group represented by the expression -N(R^{e2})-CO- (where R^{e2} is defined as described earlier), and/or the expression -CO- has been deleted from the "C₁-C₁₀ alkylene optionally containing one to three atoms and/or groups selected from among a group consisting of (a) oxygen, (b) groups represented by the expression -S(O)_{q}- (where q indicates an integer from 0 to 2), (c) groups represented by the expression -N(R^{e2})- (where R^{e2} is defined as described earlier), (d) groups represented by the expression -CO-N(R^{e2})- (where R^{e2} is defined as described earlier), (e) groups represented by the expression -N(R^{e2})-CO- (where R^{e2} is defined as described earlier), and (f) groups represented by the expression -CO-" of W^{c3} described earlier, or a group represented by the expression -CH₂-, - (CH₂) ₂-, -CH (CH₃) -, - (CH₂) ₄-, -CH₂-O-, -CH₂-S-, -CH₂-NH-, -CH₂-N (CH ₃) -. -CH₂-N (C₂H₅) -, -CH₂-N (C₃H₇) -, -CH₂-N (i-C₃H₇) -, -CH₂-N(phenyl)-, -CH₂-N(4-methylphenyl)-, -CH₂-N(benzyl)-, -OCH₂-, -SCH₂-, - NHCH₃-. -N (CH₃) -CH₂-, - (CH₂) ₂-O-, - (CH₂) ₂-S-, - (CH₂) ₂-NH-, - (CH₂) ₂-N (CH₃) -, -O- ( CH₂) ₂-, -S- (CH₂) ₂-. -NH- (CH₂) ₂-, -N (CH₃) - (CH₂) ₂-, -CH (CH₃) -O-, -CH (CH₃) -S-, -CH (CH₃) -NH-, -CH (CH₃) -N (CH₃) -, -O-CH (CH₃ ) -, -S-CH (CH₃) -, -NH-CH (CH₃) -, -N (CH₃) - CH (CH₃) -, - (CH₂) ₃-O-, - (CH₂) ₃-S, - (CH₂) ₃-NH-, - (CH₂) ₃-N (CH₃) -, -O- (CH₂) ₃-. -S- ( CH₂) ₃-, NH- (CH₂) ₃-, -N (CH₃) - (CH₂) ₃-. -C H (CH₃) CH₂-O-, -CH (CH₃) CH₂-S-, -CH (CH₃) C H₂-NH-, -CH (CH₃) CH₂-N (CH₃) -, -O-CH (CH₃) CH₂-, -S-CH (CH₃) CH₂-, -NH-CH (CH₃) CH₂-, - N (CH₃) -CH (CH₃) CH₂-, -CH₂-O- (CH₂) ₂-, -CH ₂-S- (CH₂) ₂-, -CH₂-NH- (CH₂) ₂-, - (CH₂) ₂-O-CH₂-, - (CH₂) ₂-S-CH₂-, - (CH₂) ₂-NH-CH₂-, - (CH₂) ₂-O- (CH₂) ₂-, - (CH₂) ₂-S- (CH₂) ₂-, - (C H₂) ₂-NH- (CH₂) ₂-, -CH₂-O- (CH₂) ₃-, -CH₂-S-(CH₂) ₃-, -CH₂-NH- (CH₂) ₃-, - (CH₂) ₂-O- (CH₂ ) ₃-, - (CH₂) ₂-S- (CH₂) ₃-, - (CH₂) ₂-NH- (CH₂) ₃-. - (CH₂) ₃-O- (CH₂) ₂-, - (CH₂) ₃-S- (CH₂) ₂-, - (CH₂) ₃-NH- (CH₂) ₂-, -O- (CH₂) ₂-O-, -O- ( CH₂) ₂-S-, -O- (CH₂) ₂-NH-, -S- (CH₂) ₂-O-, - S- (CH₂) ₂-S-, -S- (CH₂) ₂-NH-, -NH- (CH₂) ₂-O-, -N (CH₃) - (CH₂) ₂-O-, -NH- (CH₂) ₂-S-, -N H- (CH₂) ₂-NH-, -CH₂-O- (CH₂) ₂-O-, -CH₂-O-(CH₂) ₂-S-, -CH₂-O- (CH₂) ₂-NH-, -CH₂-S- (C H₂) ₂-O-, -CH₂-S- (CH₂) ₂-S-, -CH₂-S- (CH₂) ₂ -NH-, -CH₂-NH- (CH₂) ₂-O-, -CH₂-NH- (CH₂) ₂ -S-, -CH₂-NH- (CH₂) ₂-NH-, -CH₂-O- (CH₂) ₃-O-, -CH₂-O- (CH₂) ₃-S-. -CH₂-O- (CH₂) ₃-NH-, -CH₂-S- (CH₂) ₃-O-, -CH₂-S- (CH₂) ₃ -S-, -C H₂-S- (CH₂) ₃-NH-, -CH₂-NH- (CH₂) ₃-O-, -CH ₂-NH- (CH₂) ₃-S-, or -CH₂-NH-(CH₂) ₃-NH- more advantageously a group represented by the expression -CH₂-O-, CH₂-S-, CH₂-NH -, -CH₂-N (CH₃) -, -OCH₂-, -SCH₂-, -NHCH₂-, -N (CH₃) -CH₂-, - (CH₂) ₂-O-, - (CH₂) ₂-S-, - ( CH₂) ₂-NH-, - (CH₂) ₂-N (CH₃) -, -O- (CH₂) ₂-, -S- (CH₂) ₂-, -NH- (CH₂) ₂-, -N (CH₃) - (CH₂) ₂ -, -CH (CH₃) -O-, -CH (CH₃) -S-, -CH (CH₃) -N H-, -CH (CH₃) -N (CH₃) -, -O-CH= (CH₃) -, -S-C H (CH₃) -, -NH-CH (CH₃) -, -N (CH₃) -CH (CH₃) -, - (CH₂) ₃-O-, -O-CH (CH₃) CH₂-, - (CH₂) ₂-S -CH₂-, - (CH₂) ₂-O- (CH₂) ₂-, - (CH₂) ₂-S- (CH₂ ) ₂-, -CH₂-O- (CH₂) ₃-, -O- (CH₂) ₂-O-, -O- (C H₂) ₂-S-, -O- (CH₂) ₂-NH-, -S- (CH₂) ₂-O-, -S - (CH₂) ₂-S-, -S- (CH₂) ₂-NH-. -NH- (CH₂) ₂-O -, -N (CH₃) - (CH₂) ₂-O-, -NH- (CH₂) ₂-S-, NH - (CH₂) ₂-NH-, -CH₂-O- (CH₂) ₂-O-, -CH₂-O- ( CH₂) ₂-S , -CH₂-O- (CH₂) ₂-NH-, -CH₂-S- (CH ₂) ₂-S-, -CH₂-S- (CH₂) ₂-NH, -CH₂-NH- (CH₂ ) ₂-O-, -CH₂-NH- (CH₂) ₂-S-, -CH₂-NH- (CH₂) ₂-NH-, -CH₂-O- (CH₂) ₃-O-, -CH₂-O- (CH₂) ₃-S-, -CH₂-O- (CH₂) ₃-NH-, -CH₂-S- (CH₂) ₃-O-, -CH₂-S- (CH₂) ₃-S-, -CH₂-S- (CH₂) ₃-NH-, - CH₂-NH- (CH₂) ₃-O-, -CH₂-NH- (CH₂) ₃-S-, or -CH₂-NH-(CH₂)₃-NH- ; even more advantageously a group represented by the expression CH₂-O-, -CH₂-S-, -CH₂-NH-, -CH₂-N (CH₃) -, -OCH₂-, -SCH₂-, -NHCH₂-, -N (CH₃) -CH₂-, - ( CH₂) ₂-O-, -O- (CH₂) ₂-O-, -O- (CH₂) ₂-S-, -O- (CH₂) ₂-NH-, -S- (CH₂) ₂-O-, -S- (C H₂) ₂-S-, -S- (CH₂) ₂-NH-, -NH- (CH₂) ₂-O-, - N (CH₃) - (CH₂) ₂-O-, -NH- (CH₂) ₂-S-, -CH₂-O - (CH₂) ₂-O-, -CH₂-O- (CH₂) ₂-S-, -CH₂-S- (C H₂) ₂-S-, -CH₂-NH- (CH₂) ₂-O-, -CH₂-O- (CH₂ ) ₃-O-, -CH₂-O- (CH₂) ₃-S-, -CH₂-S- (CH₂) ₃-O-, -CH₂-S- (CH₂) ₃-S-, -CH₂-S- (CH₂) ₃-NH-, -CH₂-NH- (CH₂) ₃-O-, -CH₂-NH- (CH₂) ₃-S-, or -CH₂-NH- (CH₂) ₃-NH-; and optimally a group represented by the expression - CH₂-O-, -CH₂-S-, -CH₂-NH-, -CH₂-N (CH₃) -, -OCH₂-, -SCH₂-, -NHCH₂-, -O- (CH₂) ₂-O-, -O - (CH₂) ₂-S-, -O- (CH₂) ₂-NH-, -S- (CH₂) ₂-O-, -S- (CH₂) ₂-S-, -N (CH₃) - (CH₂) ₂-O-, -CH₂-O- (CH₂) ₂-O-, -CH₂-O- (CH₂) ₃-O-, -CH₂-O- ( CH₂) ₃-S-, -CH₂-S- (CH₂) ₃-O-. or -CH₂-S- (C H₂) ₃-S-.
Specific groups of the "C₁-C₆ alkylene optionally containing one to three atoms and/or groups selected from among a group consisting of oxygen, sulfur, and groups represented by the expression -N(R^{e2})- and the expression -CO-N(R^{e2})- (where R^{e2} is defined as in "D-1")" (advantageously, C₁-C₆ alkylene containing one to three of these atoms and/or groups) in "D-1" are the atoms and/or groups indicated earlier as "more advantageous," specific groups of the "C₁-C₄ alkylene optionally containing one to three atoms and/or groups selected from among a group consisting of oxygen, sulfur, groups represented by the expression -N(R^{e2})- (where R^{e2} is defined as in "D-2" (advantageously, C₁-C₄ alkylene containing one to three of these atoms and/or groups) in "D-2" are the atoms and/or groups indicates earlier as "more advantageous," and specific groups of the "C₁-C₄ alkylene optionally containing one to two atoms and/or groups selected from among a group consisting of oxygen, sulfur, and groups represented by the expression -N(R^{e2})- and the expression -CO-N(R^{e2})- (where R^{e2} is hydrogen, methyl, benzyl, or acetyl)" (advantageously, C₁-C₄ alkylene containing one to two of these atoms and/or groups) in "D-3" are the atoms and/or groups indicated earlier as "more advantageous."

The "C₁-C₄ alkylene optionally containing one to two atoms and/or groups selected from among a group consisting of oxygen, groups represented by the expression -S(Oq)- (where q indicates an integer from 0 to 2), and groups represented by the expression -N(R^{e2})- (where R^{e2} is defined as described earlier)" (advantageously, C₁-C₄ alkylene containing one to two of these atoms and/or groups) of W^{c4} and X^{e3} are, for example, groups represented by the expressions -CH₂-, - (CH₂) 2-, -CH (CH₃) -, - (CH₂) ₃-, -CH (CH₃) -CH₂-, -CH (C₂H₅) -, -C (CH₃) ₂-, - (CH₂ ) ₄-, - (CH₂) ₂ - CH (CH₃) -, -CH₂-O-, -CH₂-S-, -CH₂-SO-, -CH₂-SO₂-, -CH₂-NH-, -CH₂-N (CH ₃) -, -CH₂-N (C₂H₅) -, -OCH₂-, -SCH₂-, -SOCH ₂-, -SO₂CH₂-, -NHCH₂-, -N (CH₃) -CH₂-, - (CH ₂) ₂-O-, - (CH₂) ₂-S-, - (CH₂) ₂-NH-, - (CH₂) ₂-N (CH₃) -, -O- (CH₂) ₂-, -S- (CH₂) ₂-, -NH- (CH ₂) ₂-, -N (CH₃) - (CH₂) ₂-, - (CH₂) ₃-O-, - (CH₂) ₃-S-, - (CH₂) ₃-NH-, - (CH₂) ₃-N (CH₃) -, -O- ( CH₂) ₃ -S- (CH₂) ₃-, -NH- (CH₂) ₃ -N (CH₃) (CH₂) ₃-, -CH (CH₃) CH₂-O-, -CH (CH₃) CH₂-S -, -CH (CH₃) CH₂-NH-, -CH (CH₃) CH₂-N (CH₃) - , -O-CH (CH₃) CH₂-, -S-CH (CH₃) CH₂-, -NH-C H (CH₃) CH₂-, -N (CH₃) -CH (CH₃) CH₂-, -CH₂-O - (CH₂) ₂-, -CH₂-S- (CH₂) ₂-, -CH₂-NH- (CH₂) ₂ -, - (CH₂) ₂-O-CH₂-, - (CH₂) ₂-S-CH₂-, - (CH₂ ) 2 -NH-CH₂-, or - (CH₂) ₂-N (CH₃)-CH₂-_{;} advantageously a group represented by the expression -CH₂-, - (CH₂) ₂-, -CH (CH₃) -, - (C H₂)₃-, -CH(CH₃)-CH₂-, -(CH₂)₄-, -CH₂-O-, -CH₂-S-, -CH₂-SO-, -CH₂-SO₂-, -CH₂-NH-, - CH₂-NCH₃-, -OCH₂-, -SCH₂-, -SOCH₂-, -SO ₂CH₂-, -(CH₂)₂-O-, -(CH₂)₂-S-, -(CH₂)₂-NH -, -(CH₂)₂-N(CH₃)-, -O-(CH₂)₂-, -(CH₂)₃-O-, -O-(CH₂)₃-, -CH(CH₃)CH₂-O-, -CH(CH₃) CH₂-S-, -CH(CH₃)CH₂-N(CH₃)-, -O-CH(CH₃) CH₂-, -S-CH(CH₃)CH₂-, -CH₂-O-(CH₂)₂-, -C H₂-S-(CH₂)₂-, -(CH₂)₂-O-CH₂-, -(CH₂)₂-S-CH₂-, -(CH₂)₂-NH-CH₂-, or -(CH₂)₂-N(CH₃)-CH₂-; even more advantageously a group represented by the expression -CH₂-O-, -CH₂-S-, -CH₂-SO-, -CH₂-SO₂-, -CH₂-NH-, -CH₂-N(CH ₃)-, -OCH₂-, -CH₂-, -SOCH₂-, -SO₂CH₂-, -( CH₂) ₂-O-, -(CH₂)₂-S-, -(CH₂)₂-NH-, -(CH₂ )₂-N(CH₃)-, -O-(CH₂)₂-, -CH₂-O-(CH₂)₂-, -CH₂-S-(CH₂)₂-, -(CH₂)₂-O-CH₂-, -(CH₂)₂-S-CH₂-, or -(CH₂)₂-NH-CH₂-; and optimally a group represented by the expression -CH₂-O-, -CH₂-S-, -CH₂-SO-, -CH₂-SO₂-, -CH₂-NH-, -CH₂-N(CH₃)-, -OCH₂-, -SCH₂-, -SOCH₂-, -SO₂CH₂-, -(CH₂)₂-O-CH₂-, or -(CH₂)₂-S-CH₂-.
Specific groups of the "C₁-C₃ alkylene optionally containing one to two atoms and/or groups selected from among a group consisting of oxygen and groups represented by the expression -S(O)_{q}- and groups represented by the expression -N(R^{e2})- (where q and R^{e2} are defined as in "D-1(1)")" (advantageously, C₁-C₃ alkylene containing one to two of these atoms and/or groups) in "D-1(1)" to be described later are the atoms and/or groups indicated earlier as "advantageous," and specific groups of the "C₁-C₄ alkylene optionally containing one to two atoms and/or groups selected from among a group consisting of oxygen and groups represented by the expression -S(O)_{q}- and groups represented by the expression -N(R^{e2})- (where q and R^{e2} are defined as in "D-2(1)")" (advantageously, C₁-C₄ alkylene containing one to two of these atoms and/or groups) in "D-2(1)" to be described later are the atoms and/or groups indicated earlier as "more advantageous."

The "five- to ten-membered (monocyclic or annelated) heteroarylene ring containing one to five heteroatoms selected from among a group consisting of oxygen, nitrogen, and sulfur" of Ar may be, for example, a divalent pyrrole, furan, thiophen, pyrazole, imidazole, 1,2,4-triazole, 1,2,3-triazole, tetrazole, isoxazole, oxazole, 1,3,4-oxadiazole, thiazole, isothiazole, 1,3,4-thiazole, 1,2,4-thiadiazole, pyridine, pyridazine, pyrimidine, pyrazine, 1,3,5-triazine, 1,2,4-triazine, indole, benzofuran, benzo[B]thiophen, benzimidazole, benzotriazole, benzisoxazole, benzoxazole, benzisothiazole, benzothiazole, oxazolo[4,5-B]pyridine, benzofurazan, s-triazolo[1,5-A]pyrimidine, s-triazolo[4,3-A]pyrimidine, pyrazolo[4,5-C]pyridine, pyrazolo[3,4-B]pyridine, 1,2,3-benzothiadiazole, pyrazolo-3,4-D]pyrimidine, quinoline, isoquinoline, quinoxaline, pyrido[2,3-B]pyrazine, pyridoimidazole, quinazoline, phthalazine, cinnoline, or naphthyridine cyclic group; advantageously a pyrrole, furan, thiophen, pyrazole, imidazole, 1,2,4-triazole, tetrazole, isoxazole, oxazole, 1,3,4-oxadiazole, thiazole, isothiazole, 1,3,4-thiazole, pyridine, pyridazine, pyrimidine, pyrazine, indole, benzofuran, benzo[B]thiophen, benzimidazole, benzotriazole, benzisoxazole, benzoxazole, benzisothiazole, benzothiazole, oxazolo[4,5-B]pyridine, benzofurazan, pyrazolo[4,5-C]pyridine, pyrazolo[3,4-B]pyridine, 1,2,3-benzothiadiazole, pyrazolo-3,4-D]pyrimidine, quinoline, isoquinoline, quinoxaline, pyrido[2,3-B]pyrazine, pyridoimidazole, quinazoline, phthalazine, cinnoline, or naphthyridine cyclic group; more advantageously a furan, thiophen, imidazole, tetrazole, oxazole, thiazole, pyridine, pyridazine, pyrimidine, pyrazinebenzofuran, benzo[B]thiophen, benzimidazole, benzoxazole, benzothiazole, quinoline, isoquinoline, or pyridoimidazole cyclic group; even more advantageously a 2,5-tetrazole, 5,2-tetrazole, 2,5-oxazole, 2,5-thiazole, 2,5-pyridine, 3,5-pyridine, 3,6-pyridine, 1,5-benzimidazole, 2,6-benzoxazole, 2,6-benzimidazole, 6,2-benzimidazole, 4,7-benzthiazole, or 2,7-pyridoimidazole cyclic group; and optimally a 2,5-tetrazole, 5,2-tetrazole, 2,5-pyridine, 3,5-pyridine, 3,6-pyridine, 1,5-benzimidazole, 2,6-benzoxazole, 2,6-benzimidazole, 6,2-benzimidazole, or 2,7-pyridoimidazole cyclic group.

Specific examples of the "C₁-C₆ halogenoalkyl" in R^{a1} and the like are fluoromethyl, difluoromethyl, trifluoromethyl, chloromethyl, dichloromethyl, trichloromethyl, bromomethyl, iodomethyl, 2-fluoroethyl, 2,2,2-trifluoroethyl, 2-chloroethyl, 2,2,2-trichloroethyl, 2-bromoethyl, 2,2-dibromoethyl, 2-iodoethyl, 3-chloropropyl, 4-fluorobutyl, or 6-iodohexyl; advantageously fluoromethyl, difluoromethyl, trifluoromethyl, dichloromethyl, 2-fluoroethyl, 2,2,2-trifluoroethyl, 2-chloroethyl, 2,2,2-trichloroethyl, 3-chloropropyl, 4-fluorobutyl, or 6-iodohexyl; more advantageously fluoromethyl, difluoromethyl, trifluoromethyl, 2-fluoroethyl, 2,2,2-trifluoroethyl, 3-chloropropyl, or 4-fluorobutyl; and optimally trifluoromethyl.

Specific examples of the "C₆-C₁₀ aryl-C₁-C₆ alkyl optionally having one to five substituents α" of R^{a3} and the like are benzyl, naphthylmethyl, indenylmethyl, 1-phenethyl, 2-phenethyl, 1-naphthylethyl, 2-naphthylethyl, 1-phenylpropyl, 2-phenylpropyl, 3-phenylpropyl, 1-naphtylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 5-phenylpentyl, 5-naphthylpentyl, 6-phenylhexyl, or 6-naphthylhexyl; advantageously benzyl, naphthylmethyl, indenylmethyl, 1-phenethyl, 2-phenethyl, 1-naphthylethyl, 2-naphthylethyl, 3-phenylpropyl, 3-naphthylpropyl, 3-phenylbutyl, 4-phenylbutyl, 4-naphthylbutyl, 5-phenylpentyl, 5-naphthylpentyl, 6-phenylhexyl, or 6-naphthylhexyl; more advantageously benzyl, naphthylmethyl, 2-phenethyl, 2-naphthylethyl, 3-phenylpropyl, 4-phenylbutyl, 4-naphthylbutyl, 5-phenylpentyl, or 6-phenylhexyl; and optimally benzyl.

The "carbamoyl optionally substituted with one to two substituents α to be described later (provided, however, that the substituents α may not be (iv) a halogen, (v) hydroxy, (vi) cyano, or (vii) nitro) of substituent α" of R^{c3} and the like is advantageously a monoalkylcarbamoyl, dialkylcarbomoyl, monoarylcarbamoyl, diarylcarbamoyl, monoarylalkylcarbamoyl, N,N-diarylalkylcarbamoyl, N-alkyl-N-arylcarbamoyl, N-alkyl-N-arylalkylcarbamoyl, N-aryl-N-arylalkylcarbamoyl, cycloalkylcarbamoyl, dicycloalkylcarbamoyl, N-alkyl-N-cycloalkylcarbamoyl, N-aryl-N-cycloalkylcarbamoyl, N-arylalkyl-N-cycloalkylcarbamoyl, formylcarbamoyl, alkylcarbonylcarbamoyl, alkenylcarbonylcarbamoyl, arylcarbonylcarbamoyl, arylalkylcarbamoyl, carbamoyl substituted with a five- to seven-membered cyclic heteroaryl carbonyl optionally having one to two substituents β to be described later and containing one to three heteroatoms selected from among a group consisting of oxygen, nitrogen, and sulfur, or a di-substituted carbamoyl in which the five- to seven-membered heteroaryl carbonyl and the alkyl, aryl, arylalkyl, or cycloalkyl described earlier are substituted with nitrogen; more advantageously, methylcarbamoyl, ethylcarbamoyl, propylcarbamoyl, butylcarbamoyl, hexylcarbamoyl, N,N-dimethylcarbamoyl, N-methyl-N-ethylcarbamoyl, N-methyl-N-propylcarbamoyl, N-methyl-N-isopropylcarbamoyl, N-methyl-N-butylcarbamoyl, N-ethyl-N-hexylcarbamoyl, N,N-diethylcarbamoyl, N,N-dipropylcarbamoyl, N-phenyl-N-methylcarbamoyl, N-ethyl-N-phenylcarbamoyl, N-phenyl-N-propylcarbamoyl, N-butyl-N-phenylcarbamoyl, N-pentyl-N-phenylcarbamoyl, N-benzyl-N-methylcarbamoyl, N-1-naphthylmethyl-N-methylcarbamoyl, N-benzyl-N-ethylcarbamoyl" N-benzyl-N-propylcarbamoyl, N-benzyl-N-butylcarbamoyl, N-benzyl-N-hexylcarbamoyl, cyclopropylcarbamoyl, cyclobutylcarbamoyl, cyclopentylcarbamoyl, cyclohexylcarbamoyl, cyclooctylcarbamoyl, N-methyl-N-cyclopropylcarbamoyl, N-methyl-N-cyclobutylcarbamoyl, N-methyl-N-cyclopentylcarbamoyl, N-methyl-N-cyclohexylcarbamoyl, N-methyl-N-cyclooctylcarbamoyl, N-ethyl-N-cyclopropylcarbamoyl, N-ethyl-N-cyclopentylcarbamoyl, N-cyclopropyl-N-phenylcarbamoyl, N-cyclobutyl-N-phenylcarbamoyl, N-cyclopentyl-N-phenylcarbamoyl, N-cyclohexyl-N-phenylcarbamoyl, N-cyclooctyl-N-phenylcarbamoyl, N-benzyl-N-cyclopropylcarbamoyl, N-benzyl-N-cyclobutylcarbamoyl, N-benzyl-N-cyclopentylcarbamoyl, N-benzyl-N-cyclohexylcarbamoyl, N-benzyl-N-cyclooctylcarbamoyl, N,N-dicyclopropylcarbamoyl, N,N-dicyclopentylcarbamoyl, N,N-dicyclohexylcarbamoyl, N-cyclobutyl-N-cyclopentylcarbamoyl, N-cyclohexyl-N-cyclopentylcarbamoyl, N-cyclopentyl-N-cyclohexylcarbamoyl, N-cyclopentyl-N-cyclooctylcarbamoyl, phenylcarbamoyl, 1-naphthylcarbamoyl,2-naphthylcarbamoyl, 4-methylphenylcarbamoyl, 4-chloro-3-methylphenylcarbamoyl, 4-fluorophenylcarbamoyl, 2,4-difluorophenylcarbamoyl, 2-chloro-4-fluorophenylcarbamoyl, 1-chloro-4-naphthylcarbamoyl, 4-biphenylcarbamoyl, 4-benzylphenylcarbamoyl, 3-acetylphenylcarbamoyl, 4-benzoylphenylcarbamoyl, 4-nicotinoylphenylcarbamoyl, 4-phenylcarbamoylphenylcarbamoyl, N,N-diphenylcarbamoyl, benzylcarbamoyl, naphthylmethylcarbamoyl, 2-phenethylcarbamoyl, 2-naphthylethylcarbamoyl, 3-phenylpropylcarbamoyl, 4-phenylbutylcarbamoyl, 5-phenylpentylcarbamoyl, 6-phenylhexylcarbamoyl, 2-carbamoylbenzylcarbamoyl, 4-t-butylbenzylcarbamoyl, 2-bromobenzylcarbamoyl, 3-chlorobenzylcarbamoyl, 2,6-difluorobenzylcarbamoyl, N,N-dibenzylcarbamoyl, N-benzyl-N-naphthylmethylcarbamoyl, N-benzyl-N-2-phenytylcarbamoyl, acetylcarbamoyl, propionylcarbamoyl, N-methyl-N-acetylcarbamoyl, N-ethyl-N-propionylcarbamoyl, N-phenyl-N-acetylcarbamoyl, N-benzyl-N-butylcarbamoyl, benzoylcarbamoyl, naphthoylcarbamoyl, 4-fluorobenzoylcarbamoyl, N-methyl-N-benzoylcarbamoyl, N-methyl-N-naphthoylcarbamoyl, N-propyl-N-benzoylcarbamoyl, N-benzoyl-N-phenylcarbamoyl, 3-phenylpropionylcarbamoyl, N-5-pentanoyl-N-methylcarbamoyl, N-methyl-N-3-phenylpropionylcarbamoyl, nicotinoylcarbamoyl, isonicotinoylcarbamoyl, picolinoylcarbamoyl, N-methyl-N-nicotinoylcarbamoyl, N-methyl-N-isonicotinoylcarbamoyl, N-butyl-N-picolinoylcarbamoyl, N-nicotinoyl-N-phenylcarbamoyl, N-isonicotinoyl-N-phenylcarbamoyl, N-phenyl-N-picolinoylcarbamoyl, N-benzyl-N-nicotinoylcarbamoyl, N-benzyl-N-isonicotinoylcarbamoyl, or N-benzyl-N-picolinoylcarbamoyl; more advantageously methylcarbamoyl, ethylcarbamoyl, propylcarbamoyl, butylcarbamoyl, N,N-dimethylcarbamoyl, N-methyl-N-ethylcarbamoyl, N-methyl-N-propylcarbamoyl, N-methyl-N-isopropylcarbamoyl, N-methyl-N-butylcarbamoyl, N,N-dipropylcarbamoyl, N-phenyl-N-methylcarbamoyl, N-ethyl-N-phenylcarbamoyl, N-benzyl-N-methylcarbamoyl, N-1-naphthylmethyl-N-methylcarbamoyl, cyclopropylcarbamoyl, cyclopentylcarbamoyl, cyclohexylcarbamoyl, N-methyl-N-cyclopropylcarbamoyl, N-methyl-N-cyclohexylcarbamoyl, N-cyclopropyl-N-phenylcarbamoyl, N-cyclopentyl-N-phenylcarbamoyl, N-benzyl-N-cyclopropylcarbamoyl, N-benzyl-N-cyclohexylcarbamoyl, N,N-dicyclohexylcarbamoyl, phenylcarbamoyl, 2-naphthylcarbamoyl, 4-methylphenylcarbamoyl, 4-chloro-3-methylphenylcarbamoyl, 4-fluorophenylcarbamoyl, 2-chloro-4-fluorophenylcarbamoyl, 1-chloro-4-naphthylcarbamoyl, 4-benzylphenylcarbamoyl, 3-acetylphenylcarbamoyl, 4-nicotinoylphenylcarbamoyl, N,N-diphenylcarbamoyl, benzylcarbamoyl, naphthylmethylcarbamoyl, 2-phenethylcarbamoyl, 4-t-butylbenzylcarbamoyl, 3-chlorobenzylcarbamoyl, 2,6-difluorobenzylcarbamoyl, N,N-dibenzylcarbamoyl, nicotinoylcarbamoyl, isonicotinoylcarbamoyl, picolinoylcarbamoyl, N-methyl-N-nicotinoylcarbamoyl, N-methyl-N-isonicotinoylcarbamoyl, N-butyl-N-picolinoylcarbamoyl, N-nicotinoyl-N-phenylcarbamoyl, N-phenyl-N-picolinoylcarbamoyl, or N-benzyl-N-nicotinoylcarbamoyl; and optimally methylcarbamoyl, ethylcarbamoyl, butylcarbamoyl, N,N-dimethylcarbamoyl, N-methyl-N-ethylcarbamoyl, N-methyl-N-isopropylcarbamoyl, N-phenyl-N-methylcarbamoyl, N-benzyl-N-methylcarbamoyl, cyclopropylcarbamoyl, cyclohexylcarbamoyl, N-methyl-N-cyclohexylcarbamoyl, N-cyclopentyl-N-phenylcarbamoyl, phenylcarbamoyl, 2-naphthylcarbamoyl, 4-chloro-3-methylphenylcarbamoyl, 4-fluorophenylcarbamoyl, 2-chloro-4-fluorophenylcarbamoyl, 1-chloro-4-naphthylcarbamoyl, benzylcarbamoyl, naphthylmethylcarbamoyl, 2-phenethylcarbamoyl, N,N-dibenzylcarbamoyl, nicotinoylcarbamoyl, or N-methyl-N-nicotinoylcarbamoyl.

The substituent α is advantageously (i) a C₁-C₄ alkyl, (ii) a C₁-C₄ halogenoalkyl, (iii) a C₁-C₄ alkoxy, (iv) a halogen, (v) hydroxy, (vi) cyano, (vii) nitro, (viii) a C₃-C₆ cycloalkyl, (ix) a phenyl or naphthyl optionally having one to five substituents β to be described later, (x) a phenyl- or naphthyl-C₁-C₄ alkyl optionally having in the aryl portion one to five substituents β to be described later, (xi) formyl or a C₁-C₄ alkylcarbonyl or C₂-C₄ alkenylcarbonyl, (xii) a C₃-C₆ cycloalkylcarbonyl, (xiii) phenylcarbonyl or naphthylcarbonyl optionally having one to five substituents β to be described later, (xiv) a phenyl- or naphthyl-C₁-C₄ alkyl optionally having in the aryl portion one to five substituents β to be described later, (xv) furylcarbonyl, thienylcarbonyl, pyrrolylcarbonyl, pyrazolylcarbonyl, imidazolylcarbonyl, oxazolylcarbonyl, isoxazolylcarbonyl, thiazolylcarbonyl, isothiazolylcarbonyl, 1,2,3-oxadiazolylcarbonyl, triazolylcarbonyl, thiadiazolylcarbonyl, pyranylcarbonyl, nicotinoyl, isonicotinoyl, picolinyl, pyridozinylcarbonyl, pyrimidinylcarbonyl, pyrazinylcarbonyl, or azepinylcarbonyl optionally having one to two substituents β to be described later, (xvi) carbamoyl, (xvii) phenylcarbamoyl or naphthylcarbamoyl optionally having one to five substituents β to be described later, (xviii) amino optionally substituted with one to two substituents β to be described later (excluding, however, (ii) a halogen), (xix) a C₁-C₄ halogenoalkoxy, (xx) phenoxy or naphthyloxy optionally having one to five substituents β to be described later, (xxi) a phenyl- or naphthyl-C₁-C₆ alkoxy optionally having in the aryl portion one to five substituents β to be described later, (xxii) a C₁-C₄ alkoxycarbonyl, (xxiii) phenoxycarbonyl or naphthyloxycarbonyl optionally having in the aryl portion one to five substituents β to be described later, or (xxiv) a phenyl- or naphthyl-C₁-C₄ alkoxycarbonyl optionally having in the aryl portion one to five substituents β to be described later (substituent α-1).
More advantageously, the substituent α is (i) a C₁-C₄ alkyl, (ii) fluoromethyl, difluoromethyl, trifluoromethyl, dichloromethyl, trichloromethyl, 2-fluoroethyl, 2,2,2-trifluoroethyl, 2-chloroethyl, 2,2,2-trichloroethyl, 2-bromoethyl, or 2,2-dibromoethyl, (iii) a C₁-C₄ alkoxy, (iv) fluorine, chlorine, or bromine, (v) hydroxy, (vi) cyano, (vii) nitro, (viii) a C₃ or C₆ cycloalkyl, (ix) a phenyl optionally having one to three substituents nl-2 to be described later, (x) a phenyl-C₁-C₂ alkyl optionally having in the aryl portion one to three substituents β-2 to be described later, (xi) formyl, a C₁-C₂ alkylcarbonyl, acryloyl, or crotonoyl, (xii) a C₃ or C₆ cycloalkylcarbonyl, (xiii) benzoyl optionally having one to three substituents β-2 to be described later, (xiv) a phenyl-C₁-C₂ alkyl optionally having in the aryl portion one to three substituents β-2 to be described later, (xv) furylcarbonyl, thienylcarbonyl, pyrrolylcarbonyl, oxazolylcarbonyl, isoxazolylcarbonyl, thiazolylcarbonyl, isothiazolylcarbonyl, nicotinoyl, or isonicotinoyl optionally having one to two substituents β-1 to be described later, (xvi) carbamoyl, (xvii) phenylcarbamoyl optionally having one to three substituents β-1 to be described later, (xviii) amino optionally substituted with one to two substituents β-2 to be described later (excluding, however, (ii) a halogen), (xix) fluoromethoxy, difluoromethoxy, trifluoromethoxy, dichloroethoxy, 2-fluoroethoxy, 2,2,2-trifluoroethoxy, 2-chloroethoxy, 2,2,2-trichloroethoxy, 2-bromoethoxy, or 2,2-dibromoethoxy, (xx) phenoxy optionally having the substituent β-2 to be described later, (xxi) a phenyl-C₁-C₂ alkoxy optionally having in the aryl portion one to three substituents β-2 to be described later, (xxii) a C₁-C₂ alkoxycarbonyl, (xxiii) phenoxycarbamoyl optionally having in the aryl portion one to three substituents β-2 to be described later, or (xxiv) a phenyl-C₁-C₂ alkoxycarbonyl optionally having in the aryl portion one to three substituents β-2 to be described later (substituent α-2).
Even more advantageously, the substituent α is (i) a C₁-C₃ alkyl, (ii) fluoromethyl, trifluoromethyl, trichloromethyl, 2-fluoroethyl, 2,2,2-trifluoroethyl, or 2-chloroethyl, (iii) a C₁-C₃ alkoxy, (iv) fluorine or chlorine, (v) hydroxy, (viii) cyclopropyl, (ix) a phenyl optionally having the substituent β-4 to be described later, (x) benzyl optionally having the substituent β-4 to be described later, (xi) formyl or acetyl, (xii) cyclopropylcarbonyl, (xiii) benzoyl optionally having the substituent β-4 to be described later, (xiv) benzylcarbonyl optionally having the substituent β-4 to be described later, (xv) furylcarbonyl, thienylcarbonyl, or nicotinoyl optionally having the substituent β-4 to be described later, (xvi) carbamoyl, (xvii) phenylcarbamoyl optionally having the substituent β-4 to be described later, (xviii) amino optionally substituted with one to two substituents β-4 to be described later (excluding, however, (ii) a halogen), (xix) fluoromethoxy, trifluoromethoxy, or 2,2,2-trifluoroethoxy, (xx) phenoxy optionally having the substituent β-4 to be described later, (xxi) benzyloxy optionally having the substituents β-4 to be described later, (xxii) methoxycarbonyl, (xxiii) phenoxycarbamoyl optionally having the substituent β-4 to be described later, or (xxiv) benzyloxycarbonyl optionally having the substituent β-4 to be described later (substituent α-3).
Even more advantageously, the substituent α is methyl, ethyl, fluoromethyl, trifluoromethyl, methoxy, ethoxy, fluorine, chlorine, cyclopropyl, phenyl, benzyl, formyl, acetyl, benzoyl, carbamoyl, amino, trifluoromethoxy, phenoxyl, or methoxycarbonyl (substituent α-4).
Optimally, the substituent α is methyl, trifluoromethyl, methoxy, fluorine, or chlorine (substituent α-5).

The substituent β is advantageously (i) a C₁-C₄ alkyl, a C₁-C₄ halogenoalkyl, or a C₁-C₄ alkoxy, (ii) a halogen, (iii) phenyl or naphthyl optionally having one to three substituents γ-1 to be described later, (iv) a phenyl- or naphthyl-C₁-C₄ alkyl optionally having in the aryl portion one to three substituents γ-1 to be described later, (v) formyl or a C₁-C₄ alkylcarbonyl or C₂-C₄ alkenylcarbonyl, (vi) benzoyl or naphthylcarbonyl optionally having one to three substituents γ-1 to be described later, (vii) a phenyl- or naphthyl-C₁-C₄ alkylcarbonyl optionally having in the aryl portion one to three substituents γ-1 to be described later, (viii) a C₃-C₆ cycloalkylcarbonyl, (ix) furylcarbonyl, thienylcarbonyl, pyrrolylcarbonyl, pyrazolylcarbonyl, imidazolylcarbonyl, oxazolylcarbonyl, isoxazolylcarbonyl, thiazolylcarbonyl, isothiazolylcarbonyl, 1,2,3-oxadiazolylcarbonyl, triazolylcarbonyl, thiadiazolylcarbonayl, pyranylcarbonyl, nicotinoyl, isonicotinoyl, picolinyl, pyridozinylcarbonyl, pyrimidinylcarbonyl, pyrazinylcarbonyl, or azepinylcarbonyl optionally having one to two substituents γ to be described later, (x) carbamoyl, or (xi) phenylcarbamoyl or naphthylcarbamoyl optionally having one to three substituents γ-1 to be described later (group β-1).
More advantageously, the substituent β is (i) a C₁-C₄ alkyl, fluoromethyl, trifluoromethyl, trichloromethyl, 2-fluoroethyl, 2,2,2-trifluoroethyl, 2-chloroethyl, or a C₁-C₃ alkoxy, (ii) fluorine, chlorine, or bromine, (iii) phenyl optionally having the substituent γ-2 to be described later, (iv) a phenyl-C₁-C₂ alkyl optionally having one to three substituents γ-2 to be described later, (v) formyl, a C₁-C₂ alkylcarbonyl, acryloyl, or crotonoyl, (vi) benzoyl optionally having one to three substituents γ-2 to be described later, (vii) a phenyl-C₁-C₂ alkylcarbonyl optionally having one to three substituents γ-2 to be described later, (viii) a C₃-C₆ cycloalkylcarbonyl, (ix) furylcarbonyl, thienylcarbonyl, pyrrolylcarbonyl, oxazolylcarbonyl, isoxazolylcarbonyl, thiazolylcarbonyl, isothiazolylcarbonyl, nicotinoyl, or isonicotinoyl optionally having the substituent γ-2 to be described later, (x) carbamoyl, or (xi) phenylcarbamoyl optionally having one to three substituents γ -2 to be described later (group β-2).
Even more advantageously, the substituent β is (i) methyl, ethyl, propyl, trifluoromethyl, or methoxy, (ii) fluorine or chlorine, (iii) phenyl optionally having the substituent γ-3 to be described later, (iv) benzyl optionally having the substituent γ-3 to be described later, (v) formyl or a C₁-C₂ alkylcarbonyl, (vi) benzoyl optionally having the substituents γ-3 to be described later, (vii) benzylcarbonyl optionally having the substituent γ-3 to be described later, (viii) cyclopropylcarbonyl or cyclohexylcarbonyl, (ix) furylcarbonyl, thienylcarbonyl, nicotinoyl, or isonicotinoyl optionally having the substituent γ-3 to be described later, (x) carbamoyl, or (xi) phenylcarbamoyl optionally having the substituent γ -3 to be described later (group β-3).
Even more advantageously, the substituent β is methyl, ethyl, fluorine, chlorine, phenyl, benzyl, formyl, acetyl, benzoyl, benzylcarbonyl, cyclopropylcarbonyl, furylcarbonyl, thienylcarbonyl, nicotinoyl, carbamoyl, or phenylcarbamoyl (group β-4).
Optimally, the substituent β is methyl, ethyl, fluorine, or chlorine (group β-5).

The substituent γ is advantageously (i) a C₁-C₄ alkyl or a C₁-C₄ alkoxy, (ii) a C₁-C₄ halogenoalkyl, (iii) a halogen, or (iv) hydroxy (substituent γ-1); more advantageously methyl, ethyl, methoxy, ethoxy, fluoromethyl, difluoromethyl, trifluoromethyl, chloromethyl, dichloromethyl, trichloromethyl, bromomethyl, iodomethyl, 2-fluoroethyl, 2,2,2-trifluoroethyl, 2-chloroethyl, 2,2,2-trichloroethyl, 2-bromoethyl, 2-iodoethyl, fluorine, chlorine, bromine, or hydroxy (substituent γ-2); even more advantageously methyl, ethyl, fluoromethyl, trifluoromethyl, 2-fluoroethyl, 2,2,2-trifluoroethyl, 2-chloroethyl, fluorine, chlorine, or hydroxy (substituent γ-3); and optimally methyl or trifluoromethyl (substituent γ-4).

The benzo- or pyrido-imidazole derivative (I) of the present invention has an acidic group (for example, carboxyl, tetrazole, or thiazolidine-2,4-dione) and/or a basic group (for example, imidazole or benzimidazole) in the molecule, and can be made a pharmaceutically acceptable salt by reacting with an acid and/or a base following a conventional method. Examples of such a salt are an alkali metal salt such as a sodium salt, a potassium salt, or a lithium salt; an alkali earth metal salt such as a calcium salt or a magnesium salt; a metal salt such as an aluminum salt, an iron salt, a zinc salt, a copper salt, a nickel salt, or a cobalt salt; an amine salt such as an ammonium salt, a t-octylamine salt, a dibenzylamine salt, a morpholine salt, a glucosamine salt, a phenyl glycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-N-phenethylamine salt, a piperazine salt, a tetramethylammonium salt, or a tris(hydroxymethyl)aminomethane salt; a halogenated hydrocarbon salt such as a hydrofluoric acid salt, a hydrochloric acid salt, a hydrogen bromide salt, or a hydrogen iodide salt; a nitric acid salt; a perchloric acid salt; a sulfuric acid salt; a phosphoric acid salt; a sulfonic acid salt such as a methanesulfonic acid salt, a trifluoromethanesulfonic acid salt, an ethanesulfonic acid salt, a benzenesulfonic acid salt, or a p-toluenesulfonic acid salt; a carboxylic acid salt such as an acetic acid salt, a malic acid salt, a fumaric acid salt, a succinic acid salt, an ascorbic acid salt, a tartaric acid salt, an oxalic acid salt, or a maleic acid salt; or an amino acid salt such as an ornithine acid salt, a glutamic acid salt, or an asparaginic acid salt; advantageously an alkali metal salt, an alkali earth metal salt, an amine salt, a halogenated hydrocarbon salt, a nitric acid salt, a sulfuric salt, a phosphorus acid salt, a sulfonic acid salt, a carboxylic acid salt, or an amino acid salt; more advantageously a sodium salt, potassium salt, or lithium salt; a calcium salt or a magnesium salt; an ammonium salt, a morpholine salt, a glucosamine salt, a phenylglycine alkyl ester salt, an ethylenediamine salt, an N-methylglucamine salt, a guanidine salt, a chloroprocaine salt, a procaine salt, a tetramethylammonium salt, a hydrochloric acid salt, or a hydrogen bromide salt; a nitric acid salt; a sulfuric acid salt; a phosphoric acid salt; a methanesulfonic acid salt or a p-toluenesulfonic acid salt; an acetic acid salt, a malic acid salt, a fumaric acid salt, a succinic acid salt, an ascorbic acid salt, a tartaric acid salt, an oxalic acid salt, or a maleic acid salt; or an ornithine acid salt, a glutamic acid salt, or an asparaginic acid salt.

In the case that the benzo- or pyrido-imidazole derivative (I) of the present invention has a carboxyl, a pharmaceutically acceptable salt or amide can be formed by following a conventional method (a condensation reaction between a carboxylic acid and an alcohol to be described later)to react compound (I) or an active derivative thereof (for example, an acid chloride) with a corresponding alcohol (for example, a C₁-C₆ alcohol) or an active derivative thereof (for example, a 5-methyl-2-oxo-1,3-dioxolen-4-yl)methyl form, a (pivaloyloxy)methyl form, a phthalidyl form, or an [(isopropoxycarbonyl)oxy]methyl form) and ammonia or a corresponding amine (for example, a mono- or di-C₁-C₆ alkylamine); to react an ester of compound (I) with a corresponding alcohol (for example, a C₁-C₆ alcohol) (ester exchange reaction) or ammonia or a corresponding amine (for example, a mono- or di-C₁-C₆ alkylamine) (amidation); or to react an alkali metal salt of compound (I) with a corresponding halide (for example, a C₁-C₆ alkyl chloride or bromide, (5-methyl-2-oxo-1,3-dioxylen-4-yl)methyl chloride or bromide, (pivaloyloxy)methyl chloride or bromide, phthalidyl chloride or bromide, or [(isopropoxycarbonyl)oxy]methyl chloride or bromide).
Examples of such an ester are a C₁-C₆ alkyl ester such as methyl ester, ethyl ester, propyl ester, isopropyl ester, butyl ester, s-butyl ester, t-butyl ester, pentyl ester, or hexyl ester; a C₃-C₆ cycloalkyl ester such as cyclopentyl ester or cyclohexyl ester; a C₆-C₁₀ cycloalkyl ester such as cyclopentyl ester or cyclohexyl ester; a C₆-C₁₀ aryl ester such as phenyl ester or naphthyl ester; a C₆-C₁₀ aryl-C₁-C₆ alkyl ester such as benzyl ester, phenethyl ester, α-methylbenzyl ester, 3-phenylpropyl ester, 4-phenylbutyl ester, 6-phenylhexyl ester, diphenyl methyl ester, or triphenyl methyl ester; or an ester that is hydrolyzed in the body, such as (5-methyl-2-oxo-1,3-dioxolen-4-yl)methyl ester, (pivaloyloxy)methyl ester, phthalidyl ester, [(isopropoxycarbonyl)oxy]methyl ester, [(cyclohexyloxycarbonyl)oxy]ethyl ester, or 1-[(cyclohexyloxycarbonyl)oxy]ethyl ester; advantageously methyl ester, ethyl ester, propyl ester, isopropyl ester, or butyl ester; cyclopentyl ester or cyclohexyl ester; phenyl ester; benzyl ester, phenethyl ester, 4-phenylbutyl ester, 6-phenylhexyl ester, or diphenyl methyl ester; (5-methyl-2-oxo-1,3-dioxolen-4-yl)methyl ester, (pivaloyloxy)methyl ester, phthalidyl ester, [(isopropoxycarbonyl)oxy]methyl ester, [(cyclohexyloxycarbonyl)oxy]ethyl ester, or 1-[(cyclohexyloxycarbonyl)oxy]ethyl ester; more advantageously methyl ester, ethyl ester, propyl ester, butyl ester, cyclohexyl ester, benzyl ester, phenethyl ester, 4-phenylbutyl ester, (5-methyl-2-oxo-1,3-dioxolen-4-yl)methyl ester, (pivaloyloxy)methyl ester, phthalidyl ester, [(isopropoxycarbonyl)oxy]methyl ester, [(cyclohexyloxycarbonyl)oxy]ethyl ester, or 1-[(cyclohexyloxycarbonyl)oxy]ethyl ester; even more advantageously methyl ester, ethyl ester, cyclohexyl ester, phenethyl ester, 4-phenylbutyl ester, (5-methyl-2-oxo-1,3-dioxolen-4-yl)methyl ester, (pivaloyloxy)methyl ester, [(isopropoxycarbonyl)oxy]methyl ester, [(cyclohexyloxycarbonyl)oxy]ethyl ester, or 1-[(cyclohexyloxycarbonyl)oxy]ethyl ester; and optimally methyl ester, ethyl ester, phenethyl ester, (5-methyl-2-oxo-1,3-dioxolen-4-yl)methyl ester, [(cyclohexyloxycarbonyl)oxy]ethyl ester, or 1-[(cyclohexyloxycarbonyl)oxy]ethyl ester.
Examples of such an amide are amide (-CONH₂); a mono-C₁-C₆ alkyl amide or mono-C₃-C₆ cycloalkyl amide such as N-methylamide, N-ethylamide, N-propylamide, N-isopropylamide, N-butylamide, N-s-butylamide, N-t-butylamide, N-pentylamide, N-hexylamide, N-cyclopropylamide, N-cyclopentylamide, or N-cyclohexylamide; or a di-C₁-C₆ alkylamide, an N-C₁-C₆ alkyl-N-C₃-C₆ cycloalkylamide, or a di-C₃-C₆ cycloalkyl amide such as N,N-dimethylamide, N,N-diethylamide, N,N-dipropylamide, N,N-diisopropylamide, N-methyl-N-ethylamide, N-methyl-N-propylamide, N-methyl-N-butylamide, N-ethyl-N-propylamide, N-ethyl-N-butylamide, N-butyl-N-cyclopentylamide, N-ethyl-N-cyclopropylamide, or N,N-dicyclohexylamide; advantageously amide (-CONH₂); N-methylamide, N-ethylamide, N-propylamide, N-isopropylamide, N-butylamide, N-t-butylamide, N-hexylamide, N-cyclopentylamide, or N-cyclohexylamide; N,N-dimethylamide, N,N-diethylamide, N,N-dipropylamide, N-methyl-N-ethylamide, N-methyl-N-propylamide, N-methyl-N-butylamide, or N-ethyl-N-cyclopropylamide; more advantageously amide (-CONH₂), N-methylamide, N-ethylamide, N-butylamide, N-hexylamide, N-cyclohexylamide, N,N-dimethylamide, N,N-diethylamide, N-methyl-N-ethylamide, or N-methyl-N-butylamide; and more advantageously amide (-CONH₂), N-methylamide, N-ethylamide, N-butylamide, N-cyclohexylamide, N,N-dimethylamide, N,N-diethylamide, or N-methyl-N-butylamide.

Compound (I) of the present invention may absorb moisture and organic solvents such as alcohols and have absorbed water or become a hydrate or an organic solvate due to being left in the atmosphere or recrystallizing. Therefore, the present invention includes hydrates or organic solvates that have taken on these forms.
In the case that a compound exhibits different crystal forms due to recrystallizing, the present invention includes all such crystal forms and amorphous forms.
The present invention also includes all compounds or so-called prodrugs that have been converted to compound (I) of the present invention or a pharmaceutically acceptable salt thereof by metabolizing in the body.
Compound (I) of the present invention may have optical isomers. Specifically, in the case that compound (I) has asymmetric carbon or sulfoxide, there may be R or S stereoisomers. In the case that the compound has an unsaturated double bond, there may be a cis or trans geometrical isomer. The present invention includes compounds in all of these forms and at any desired proportion. Such stereoisomers may be formed by synthesizing compound (I) using an optically resolved ingredient compound, or by optically resolving compound (I) using any conventional optical resolution or separation method.

The following are preferred compounds of compound (I) of the present invention.
A is
   (A-1) advantageously
      (a) a group represented by general formula (a-1) 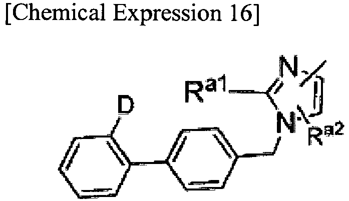 (where R^{a1} is (i) a C₂-C₅ alkyl, (ii) a C₂-C₅ alkyl substituted with fluorine or chlorine, (iii) a C₁-C₅ alkoxy, (iv) a C₂-C₅ alkylthio, (v) a group represented by the expression -N(R^{a3})(R^{a4}) (where R^{a3} is (i) hydrogen, (ii) a C₁-C₅ alkyl, (iii) phenyl optionally having one to two substituents selected from among a group consisting of methyl, ethyl, methoxy, ethoxy, fluorine, chlorine, and trifluoromethyl, or (iv) benzyl optionally having in the phenyl portion one to two substituents selected from among a group consisting of methyl, ethyl, methoxy, ethoxy, fluorine, chlorine, and trifluoromethyl, and R^{a4} is hydrogen or a C₁-C₄ alkyl), (vi) a C₃-C₆ cycloalkyl, (vii) a C₃-C₆ cycloalkyloxy, or (viii) a C₃-C₆ cycloalkylthio,
         R^{a2} is (i) hydrogen, (ii) a halogen, (iii) formyl, (iv) a C₁-C₆ alkylcarbonyl or a C₂-C₃ alkenylcarbonyl, (v) carboxyl or a C₁-C₆ alkoxycarbonyl, (vi) carbamoyl, (vii) a mono- or di-C₁-C₆ alkylcarbamoyl , (viii) amino, (ix) formylamino, a C₁-C₄ alkylcarbonylamino, or a C₂-C₃ alkenylcarbonylamino, (x) a C₁-C₄ hydroxyalkyl, (xi) a formyl-C₁-C₄ alkyl, or (xii) a C₁-C₄ alkylcarbonyl-C₁-C₄ alkyl, and
         D is (i) carboxyl, (ii) 5-tetrazolyl, (iii) 5-oxo-1,2,4-oxadiazolin-3-yl or 5-thioxo-1,2,4-oxadiazolin-3-yl, (iv) 2,4-dioxooxazolidin-5-yl or 2,4-dioxothizolidin-5-yl, (v) a group represented by the expression CF₃SO₂-NH-, (vi) tetrazol-5-ylaminocarbonyl, (vii) 2-hydroxy-3,4-dioxo-1-cyclobutenyl, (viii) acetylaminosulfonyl or propionylaminosulfonyl, (ix) benzoylaminosulfonyl optionally having in the phenyl portion one to two substituents selected from among a group consisting of methyl, ethyl, methoxy, ethoxy, fluorine, chlorine, and trifluoromethyl, or (x) phenylacetylaminosulfonyl optionally having in the phenyl portion one to two substituents selected from among a group consisting of methyl, ethyl, methoxy, ethoxy, fluorine, chlorine, and trifluoromethyl),

(b) a group represented by general formula (b-1) 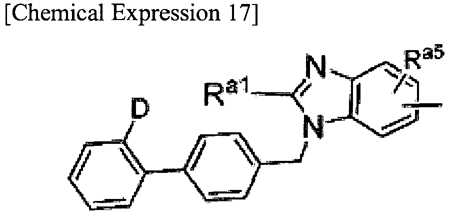 the expression (b-2) 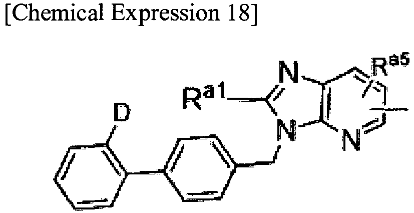 the expression (b-3) 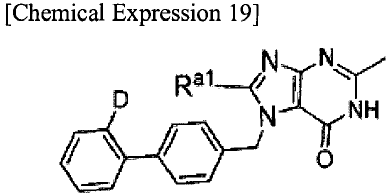 the expression (b-4) 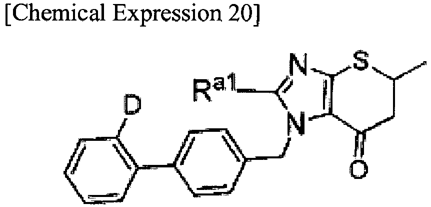 the expression (b-5)

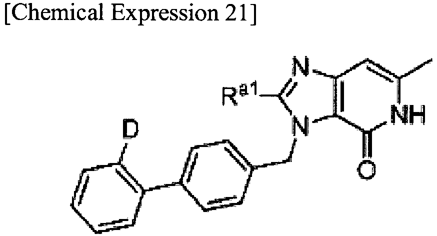

the expression (b-6) 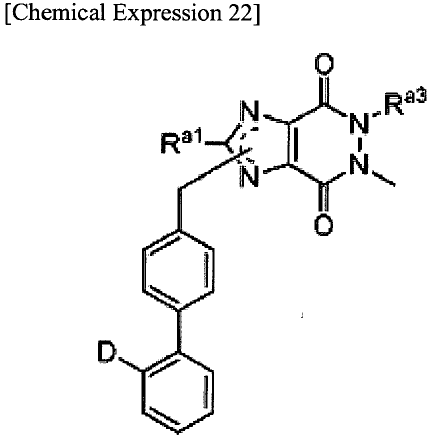 or the expression (b-7) 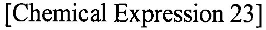 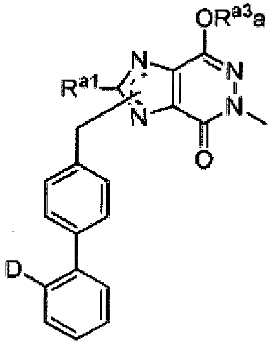 (where D and R^{a1} are defined as in the expression (a-1), and R^{a5} is (i) hydrogen, (ii) a halogen, (iii) formyl, (iv) a C₁-C₆ alkylcarbonyl or C₂-C₃ alkenylcarbonyl, (v) carboxyl or a C₁-C₆ alkoxycarbonyl, (vi) carbamoyl, (vii) a mono- or di-C₁-C₆ alkylcarbamoyl, (viii) amino, (ix) formylamino, a C₁-C₄ alkylcarbonylamino, or a C₂-C₃ alkenylcarbonylamino, (x) a C₁-C₄ hydroxyalkyl, (xi) a formyl-C₁-C₄ alkyl, (xii) a C₁-C₄ alkylcarbonyl-C₁-C₄ alkyl, (xiii) a C₁-C₄ alkyl or hexyl, (xiv) a C₁-C₄ halogenoalkyl, or (xv) a C₁-C₄ alkoxy, the bond embraced by the dotted line is a single bond or a double bond, R^{a3} is (i) hydrogen, (ii) a C₁-C₄ alkyl, or (iv) benzyl, and R^{a3}a is (ii) a C₁-C₄ alkyl or (iv) benzyl),
(c) a group represented by general formula (c-1) 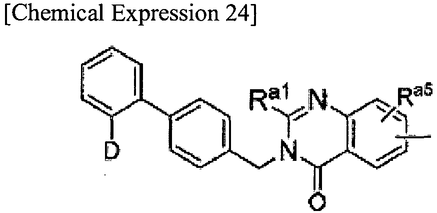 (where D is carboxyl or 5-tetrazolyl, R^{a1} is propyl, butyl, cyclopropyl, or ethoxy, and R^{a5} is hydrogen, chlorine, carboxyl, carbamoyl, methyl, ethyl, propyl, i-propyl, trifluoromethyl, or methoxy),

(d) a group represented by general formula (d-1) 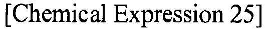 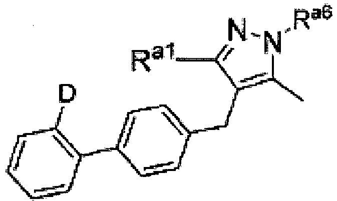 (where D is carboxyl or 5-tetrazolyl, R^{a1} is propyl, butyl, cyclopropyl, or ethoxy, and R^{a6} is methyl or ethyl),
(e) a group represented by general formula (e-1) 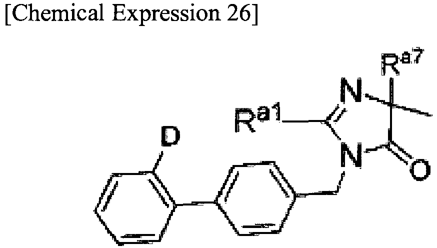 (where D and R^{a1} are defined as in the expression (a-1), and R^{a7} is hydrogen, methyl, ethyl, cyclopropyl, or cyclohexyl),
(f) a group represented by general formula (f-1) 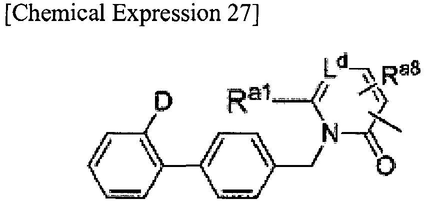 (where D and R^{a1} are defined as in the expression (a-1), R^{a8} is hydrogen, methyl, or ethyl, and L^{d} is a group represented by the expression =CH- or =N-), or

(g) a group represented by general formula (g-1) 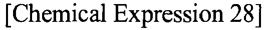 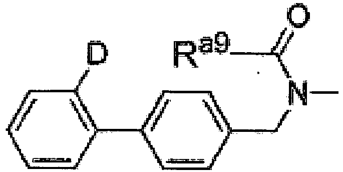 (where D is defined as in the expression (a-1), and R^{a9} is a C₁-C₄ alkyl, a C₁-C₄ halogenoalkyl, or a C₃-C₆ cycloalkyl),

(A-2) more advantageously,
   (a) a group represented by general formula (a-1)
      (where R^{a1} is (i) a C₃-C₅ alkyl, (ii) a C₃-C₄ alkyl substituted with fluorine, (iii) a C₂-C₄ alkoxy, (iv) a C₂-C₄ alkylthio, (v) a group represented by the expression -N(R^{a3})(R^{a4}) (where R^{a3} is (i) hydrogen or (ii) a C₂-C₄ alkyl, and R^{a4} is hydrogen), (vi) a C₃-C₄ cycloalkyl, (vii) cyclopropyloxy, or (viii) cyclopropylthio,
      R^{a2} is (i) hydrogen, (ii) a halogen, (iii) formyl, (iv) a C₁-C₃ alkylcarbonyl, (v) carboxyl or a C₁-C₄ alkoxycarbonyl, (vi) carbamoyl, (vii) a mono- or di-C₁-C₃ alkylcarbamoyl , (ix) formylamino or acetylamino, (x) a C₁-C₃ hydroxyalkyl, (xi) a formyl-C₁-C₂ alkyl, or (xii) a C₁-C₂ alkylcarbonyl-C₁-C₂ alkyl, and
      D is (i) carboxyl, (ii) 5-tetrazolyl, (iii) 5-oxo-1,2,4-oxadiazolin-3-yl, (iv) 2,4-dioxooxazolidin-5-yl, (v) a group represented by the expression CF₃SO₂-NH-, (vi) tetrazol-5-ylaminocarbonyl, or (viii) acetylaminosulfonyl),
   (b) a group represented by the expressions (b-1) to (b-7)
      (where D and R^{a1} are defined as in the expression (a-1) of (A-2), R^{a5} is (i) hydrogen, (ii) a halogen, (iii) formyl, (iv) a C₁-C₃ alkylcarbonyl, (v) carboxyl or a C₁-C₄ alkoxycarbonyl, (vi) carbamoyl, (vii) a mono- or di-C₁-C₄ alkylcarbamoyl, (viii) amino, (ix) formylamino or acetylamino, (x) a C₁-C₂ hydroxyalkyl, (xi) a formyl-C₁-C₂ alkyl, (xii) a C₂-C₄ alkylcarbonyl-C₁-C₂ alkyl, (xiii) a C₁-C₄ alkyl or hexyl, (xiv) a C₁-C₃ alkyl substituted with fluorine or chlorine, or (xv) a C₁-C₃ alkoxy, the bond embraced by the dotted line is a single bond or a double bond, R^{a3} is (i) hydrogen or (ii) a C₁-C₄ alkyl, and R^{a3}a is (ii) a C₁-C₄ alkyl),
   (e) a group represented by general formula (e-1)
      (where D and R^{a1} are defined as in the expression (a-1) of (A-2), and R^{a7} is cyclohexyl),
   (f) a group represented by general formula (f-1)
      (where D and R^{a1} are defined as in the expression (a-1) of (A-2), R^{a8} is methyl, and L^{d} is a group represented by the expression =N-), or
   (g) a group represented by general formula (g-1)
      (where D is defined as in the expression (a-1) of (A-2), and R^{a9} is a C₂-C₄ alkyl, a C₃-C₄ alkyl substituted with fluorine, or a C₃-C₆ cycloalkyl),

(A-3) even more advantageously,
   (a) a group represented by general formula (a-1)
      (where R^{a1} is (i) propyl, butyl, i-butyl, or pentyl, (iii) ethoxy or propoxy, (iv) ethylthio or propylthio, (v) propylamino or butylamino, (vi) cyclopropyl, or (vii) cyclopropyloxy, R^{a2} is (ii) chlorine or bromine, (iii) formyl, (iv) acetyl or propionyl, (v) carboxyl or methoxy, ethoxy, propoxy, i-propoxy, or butoxycarbonyl, (vi) carbamoyl, (vii) N-methylcarbamoyl, N-ethylcarbamoyl, or N,N-dimethylcarbamoyl, or (x) 1-hydroxyethyl or 1-hydroxy-1-methylethyl, and D is (i) carboxyl, (ii) 5-tetrazolyl, (iii) 5-oxo-1,2,4-oxadiazolin-3-yl, (v) a group represented by the expression CF₃SO₂-NH-, or (vi) tetrazol-5-ylaminocarbonyl), or (b) a group represented by the expressions (b-1), (b-2), (b-6), and (b-7)
      (where D and R^{a1} are defined as in the expression (a-1) of (A-3), R^{a5} is (i) hydrogen, (ii) fluorine or chlorine, (iii) formyl, (iv) acetyl, (v) carboxyl or a C₁-C₃ alkoxycarbonyl, (vi) carbamoyl, (vii) N-methyl, N-ethyl, N-i-propyl, N,N-dimethyl, or N-methyl-N-ethylcarbamoyl, (xiii) methyl, ethyl, propyl, i-propyl, butyl, t-butyl, or hexyl, (xiv) fluoromethyl or trifluoromethyl, or (xv) methoxy, ethoxy, propoxy, or i-propoxy, the bond embraced by the dotted line is a single bond or a double bond, R^{a3} is (i) hydrogen or (ii) methyl, ethyl, or i-propyl, and R^{a3}a is (ii) methyl or ethyl),

(A-4) even more advantageously,
   (a) a group represented by general formula (a-1)
      (where R^{a1} is (i) propyl or butyl, (iii) ethoxy, (iv) ethylthio or propylthio, (v) propylamino, or (vi) cyclopropyl, R^{a2} is (ii) chlorine, (iii) formyl, (iv) acetyl or propionyl, (v) carboxyl or methoxy, ethoxy, propoxy, i-propoxy, or butoxycarbonyl, (vi) carbamoyl, (vii) N-methylcarbamoyl, N-ethylcarbamoyl, or N,N-dimethylcarbamoyl, or (x) 1-hydroxyethyl, and D is (i) carboxyl, (ii) 5-tetrazolyl, or (iii) 5-oxo-1,2,4-oxadiazolin-3-yl), or
   (b) a group represented by general formula (b-1)
      (where D and R^{a1} are defined as in the expression (a-1) of (A-4), and R^{a5} is (i) hydrogen, (ii) chlorine, (iii) formyl, (iv) acetyl, (v) carboxyl, methoxycarbonyl, or ethoxycarbonyl, (vi) carbamoyl, (vii) N-methyl or N,N-dimethylcarbamoyl, (xiii) methyl, ethyl, propyl, i-propyl, butyl, or t-butyl, (xiv) trifluoromethyl, or (xv) methoxy), and
(A-5) optimally
   (a) a group represented by general formula (a-1)
      (where R^{a1} is (i) propyl or butyl, (iii) ethoxy, or (vi) cyclopropyl, R^{a2} is (ii) chlorine, (iii) formyl, (iv) acetyl or propionyl, (v) carboxyl or methoxy, ethoxy, propoxy, or i-propoxycarbonyl, (vi) carbamoyl, or (vii) N-methyl, N-ethyl, or N,N-dimethylcarbamoyl, and D is (i) carboxyl or (ii) 5-tetrazolyl), or
   (b) a group represented by general formula (b-1)
      (where D and R^{a1} are defined as in the expression (a-1) of (A-5), and R^{a5} is (i) hydrogen, (ii) chlorine, (v) carboxyl, (vi) carbamoyl, (xiii) methyl, ethyl, propyl, or i-propyl, (xiv) trifluoromethyl, or (xv) methoxy);

B is
(B-1) advantageously a group represented by general formula (bb-1) 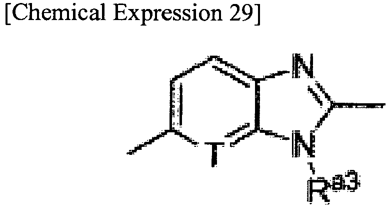 (where R^{a3} is (i) hydrogen, (ii) a C₁-C₅ alkyl, (iii) phenyl optionally having one to two substituents selected from among a group consisting of methyl, ethyl, methoxy, ethoxy, fluorine, chlorine, and trifluoromethyl, or (iv) benzyl or phenethyl optionally having in the phenyl portion one to five substituents selected from among a group consisting of C₁-C₃ alkyls, C₁-C₃ alkoxys, fluorine, chlorine, and C₁-C₃ alkyls substituted with fluorine or chlorine, T is the expression - CH= or -N=, and B is bonded to E (or G) by a portion of an imidazole ring or a portion of a benzene or pyridine ring),
(B-2) more advantageously a group represented by general formula (bb-1)
   (where R^{a3} is (i) hydrogen, (ii) a C₁-C₄ alkyl, or (iv) benzyl optionally having in the phenyl portion one to two substituents selected from among a group consisting of methyl, ethyl, ethoxy, methoxy, fluorine, chlorine, and trifluoromethyl, T is the expression -CH= or -N=, and B is bonded to E (or G) by a portion of an imidazole ring or a portion of a benzene or pyridine ring), (B-3) even more advantageously a group represented by general formula (bb-1)
   (where R^{a3} is (i) hydrogen, (ii) methyl, ethyl, propyl, i-propyl, or butyl, or (iv) benzyl optionally substituted in the phenyl portion with methyl, methoxy, fluorine, chlorine, or trifluoromethyl, T is the expression -CH= or -N=, and B is bonded to E by a portion of a benzene or pyridine ring and to G by a portion of an imidazole ring), and
(B-4) optimally a group represented by general formula (bb-1)
   (where R^{a3} is (ii) methyl or ethyl, T is the expression -CH=, and B is bonded to E by a portion of a benzene or pyridine ring and to G by a portion of an imidazole ring);

C is
(C-1) advantageously
   (a) carboxyl,
   (b) thiazolidine-2,4-dion-5-yl, oxazolidine-2,4-dion-5-yl, or 1,2,4-oxadiazolidine-3,5-dion-2-yl,
   (c) a group represented by the expression -C(R^{c1})(COOH)-W^{c1}-R^{c2} (where R^{c1} is (i) hydrogen, (ii) a C₁-C₄ alkyl, (iii) phenyl or naphthyl optionally having one to two substituents selected from among a group consisting of methyl, ethyl, methoxy, ethoxy, fluorine, chlorine, and trifluoromethyl, (iv) a phenyl-C₁-C₂ alkyl optionally having in the phenyl portion one to two substituents selected from among a group consisting of methyl, ethyl, methoxy, ethoxy, fluorine, chlorine, and trifluoromethyl, (v) a C₁-C₂ alkylsulfonyl, (vi) a C₁-C₂ alkylsulfonyl substituted with fluorine or chlorine, (vii) phenyl- or naphthyl-sulfonyl optionally having one to two substituents selected from among a group consisting of methyl, ethyl, methoxy, ethoxy, fluorine, chlorine, and trifluoromethyl, or (viii) a phenyl- or naphthyl-C₁-C₂ alkylsulfonyl optionally having in the phenyl or naphthyl portion one to two substituents selected from among a group consisting of methyl, ethyl, methoxy, ethoxy, fluorine, chlorine, and trifluoromethyl, R^{c2} is (i) hydrogen, (ii) a C₁-C₄ alkyl, (iii) phenyl or naphthyl optionally having one to three substituents selected from among a group consisting of C₁-C₄ alkyls, C₁-C₄ alkoxys, halogens, C₁-C₂ halogenoalkyls, formyl, C₁-C₂ alkylcarbonyls, and benzoyl optionally having one to two substituents selected from among a group consisting of methyl, ethyl, methoxy, ethoxy, fluorine, chlorine, and trifluoromethyl, (iv) a phenyl- or naphthyl-C₁-C₄ alkyl optionally having in the phenyl or naphthyl portion one to three substituents selected from among a group consisting of C₁-C₄ alkyls, C₁-C₄ alkoxys, halogens, and C₁-C₂ halogenoalkyls, (v) a C₁-C₄ alkylsulfonyl, (vi) a C₁-C₂ alkylsulfonyl substituted with fluorine or chlorine, (vii) phenyl- or naphthyl-sulfonyl optionally having one to three substituents selected from among a group consisting of C₁-C₄ alkyls, C₁-C₄ alkoxys, halogens, and C₁-C₂ halogenoalkyls, or (viii) a phenyl- or naphthyl-C₁-C₂ alkylsulfonyl optionally having in the phenyl or naphthyl portion one to three substituents selected from among a group consisting of C₁-C₄ alkyls, C₁-C₄ alkoxys, halogens, and C₁-C₂ halogenoalkyls, W^{c1} is (i) oxygen, (ii) sulfur, or (iii) a group represented by the expression =N(R^{c3}) (where R^{c3} is (i) hydrogen, (ii) a C₁-C₄ alkyl, (iii) phenyl or naphthyl optionally having one to two substituents selected from among a group consisting of methyl, ethyl, methoxy, ethoxy, fluorine, chlorine, and trifluoromethyl, (iv) a phenyl- or naphthyl-C₁-C₂ alkyl optionally having in the phenyl or naphthyl portion one to two substituents selected from among a group consisting of methyl, ethyl, methoxy, ethoxy, fluorine, chlorine, and trifluoromethyl, (v) formyl, a C₁-C₂ alkylcarbonyl, or acryloyl, (vi) benzoyl or naphthylcarbonyl optionally having one to three substituents selected from among a group consisting of C₁-C₄ alkyls, C₁-C₄ alkoxys, halogens, and C₁-C₂ halogenoalkyls, (vii) phenyl- or naphthyl-C₁-C₂ alkylcarbonyl optionally having in the phenyl or naphthyl portion one to three substituents selected from among a group consisting of C₁-C₄ alkyls, C₁-C₄ alkoxys, halogens, and C₁-C₂ halogenoalkyls, (viii) a C₃-C₄ cycloalkylcarbonyl, (ix) furylcarbonyl, thienylcarbonyl, or nicotinoyl optionally having one to two substituents selected from among a group consisting of methyl, ethyl, methoxy, fluorine, and chlorine, (x) carbamoyl optionally having one to two substituents selected from among a group consisting of C₁-C₄ alkyls, phenyl or naphthyl optionally having one to three substituents selected from among a group consisting of C₁-C₄ alkyls, C₁-C₄ alkoxys, halogens, and C₁-C₂ halogenoalkyls, and phenyl- or naphthyl-C₁-C₂ alkyls optionally having in the phenyl or naphthyl portion one to three substituents selected from among a group consisting of C₁-C₄ alkyls, C₁-C₄ alkoxys, halogens, and C₁-C₂ halogenoalkyls, (xi) a C₁-C₂ alkoxycarbonyl, (xii) phenoxycarbonyl optionally having one to two substituents selected from among a group consisting of methyl, ethyl, methoxy, ethoxy, fluorine, chlorine, and trifluoromethyl, or (xiii) benzyloxycarbonyl optionally having in the phenyl portion one to two substituents selected from among a group consisting of methyl, ethyl, methoxy, ethoxy, fluorine, chlorine, and trifluoromethyl)), or
   (d) a group represented by the expression -N(W^{c2})-CH₂COOH (where W^{c2} is (i) hydrogen, (ii) a C₁-C₄ alkyl, (iii) phenyl or naphthyl optionally having one to two substituents selected from among a group consisting of methyl, ethyl, methoxy, ethoxy, fluorine, chlorine, and trifluoromethyl, (iv) a phenyl- or naphthyl-C₁-C₂ alkyl optionally having in the phenyl or naphthyl portion one to three substituents selected from among a group consisting of C₁-C₄ alkyls, C₁-C₄ alkoxys, halogens, and C₁-C₂ halogenoalkyls, (v) formyl, a C₁-C₄ alkylcarbonyl, or a C₂-C₃ alkenylcarbonyl, (vi) benzoyl or naphthylcarbonyl optionally having one to three substituents selected from among a group consisting of C₁-C₄ alkyls, C₁-C₄ alkoxys, halogens, and C₁-C₂ halogenoalkyls, (vii) a phenyl- or naphthyl-C₁-C₂ alkylcarbonyl optionally having in the phenyl or naphthyl portion one to three substituents selected from among a group consisting of C₁-C₄ alkyls, C₁-C₄ alkoxys, halogens, and C₁-C₂ halogenoalkyls, (viii) a C₃-C₆ cycloalkylcarbonyl, (ix) furylcarbonyl, thienylcarbonyl, or nicotinoyl optionally having one to two substituents selected from among a group consisting of methyl, ethyl, methoxy, fluorine, and chlorine, (x) carbamoyl optionally having one to two substituents selected from among a group consisting of C₁-C₄ alkyls, phenyl or naphthyl optionally having one to three substituents selected from among a group consisting of C₁-C₄ alkyls, C₁-C₄ alkoxys, halogens, and C₁-C₂ halogenoalkyls, and phenyl- or naphthyl-C₁-C₂ alkyls optionally having in the phenyl or naphthyl portion one to three substituents selected from among a group consisting of C₁-C₄ alkyls, C₁-C₄ alkoxys, halogens, and C₁-C₂ halogenoalkyls, (xi) a C₁-C₄ alkoxycarbonyl, (xii) a phenoxy- or naphthyloxy-carbonyl optionally having one to three substituents selected from among a group consisting of C₁-C₄ alkyls, C₁-C₄ alkoxys, halogens, and C₁-C₂ halogenoalkyls, or (xiii) a phenyl- or naphthyl-C₁-C₂ alkoxycarbonyl optionally having in the phenyl or naphthyl portion one to three substituents selected from among a group consisting of C₁-C₄ alkyls, C₁-C₄ alkoxys, halogens, and C₁-C₂ halogenoalkyls),

(C-2) more advantageously
(a) carboxyl,
(b) thiazolidine-2,4-dion-5-yl, oxazolidine-2,4-dion-5-yl, or 1,2,4-oxadiazolidine-3,5-dion-2-yl,
(c) a group represented by the expression -C(R^{c1})(COOH)-W^{c1}-R^{c2} (where R^{c1} is (i) hydrogen, (ii) a C₁-C₂ alkyl, (iii) phenyl optionally having one to two substituents selected from among a group consisting of methyl, ethyl, methoxy, ethoxy, fluorine, chlorine, and trifluoromethyl, or (iv) benzyl optionally having in the phenyl portion one to two substituents selected from among a group consisting of methyl, ethyl, methoxy, ethoxy, fluorine, chlorine, and trifluoromethyl, R^{c2} is (i) hydrogen, (ii) a C₁-C₄ alkyl, (iii) phenyl optionally having one to two substituents selected from among a group consisting of C₁-C₄ alkyls, C₁-C₃ alkoxys, fluorine, chlorine, trifluoromethyl, formyl, C₁-C₂ alkylcarbonyls, and benzoyl optionally having one to two substituents selected from among a group consisting of methyl, methoxy, fluorine, chlorine, and trifluoromethyl, (iv) benzyl optionally having in the phenyl portion one to two substituents selected from among a group consisting of C₁-C₄ alkyls, C₁-C₃ alkoxys, fluorine, chlorine, and trifluoromethyl, (v) a C₁-C₃ alkylsulfonyl, (vi) a C₁-C₂ alkylsulfonyl substituted with fluorine, (vii) phenylsulfonyl optionally having one to two substituents selected from among a group consisting of methyl, ethyl, methoxy, ethoxy, fluorine, chlorine, and trifluoromethyl, or (viii) benzylsulfonyl optionally having in the phenyl portion one to two substituents selected from among a group consisting of methyl, ethyl, methoxy, ethoxy, fluorine, chlorine, and trifluoromethyl, W^{c1} is (i) oxygen, (ii) sulfur, or (iii) a group represented by the expression =N-R^{c3} (where R^{c3} is (i) hydrogen, (ii) a C₁-C₃ alkyl, (iii) phenyl optionally having one to two substituents selected from among a group consisting of methyl, methoxy, fluorine, chlorine, and trifluoromethyl, (iv) benzyl optionally having in the phenyl portion one to two substituents selected from among a group consisting of methyl, methoxy, fluorine, chlorine, and trifluoromethyl, (v) formyl or acetyl, (vi) benzoyl optionally having one to two substituents selected from among a group consisting of methyl, ethyl, methoxy, ethoxy, fluorine, chlorine, and trifluoromethyl, (vii) a phenyl-C₁-C₂ alkylcarbonyl optionally having in the phenyl portion one to two substituents selected from among a group consisting of methyl, ethyl, methoxy, ethoxy, fluorine, chlorine, and trifluoromethyl, (viii) cyclopropylcarbonyl, (ix) furylcarbonyl, thienylcarbonyl, or nicotinoyl optionally substituted with methyl, methoxy, fluorine, or chlorine, or (x) carbamoyl optionally having one to two substituents selected from among a group consisting of C₁-C₃ alkyls, phenyl optionally having one to two substituents selected from among a group consisting of methyl, ethyl, methoxy, ethoxy, fluorine, chlorine, and trifluoromethyl, and phenyl-C₁-C₂ alkyls optionally having in the phenyl portion one to two substituents selected from among a group consisting of methyl, ethyl, methoxy, ethoxy, fluorine, chlorine, bromine, and trifluoromethyl), or
(d) a group represented by the expression -N(W^{c2})-CH₂COOH (where W^{c2} is (i) hydrogen, (ii) a C₁-C₂ alkyl, (iii) phenyl optionally having one to two substituents selected from among a group consisting of methyl, methoxy, fluorine, chlorine, and trifluoromethyl, (iv) a phenyl-C₁-C₂ alkyl optionally having in the phenyl portion one to two substituents selected from among a group consisting of methyl, ethyl, methoxy, ethoxy, fluorine, chlorine, and trifluoromethyl, (v) formyl, acetyl, propionyl, butyryl, or valeryl, (vi) benzoyl optionally having one to two substituents selected from among a group consisting of methyl, ethyl, methoxy, ethoxy, fluorine, chlorine, and trifluoromethyl, (vii) a phenyl-C₁-C₂ alkylcarbonyl optionally having in the phenyl portion one to two substituents selected from among a group consisting of methyl, ethyl, methoxy, ethoxy, fluorine, chlorine, and trifluoromethyl, (viii) a C₅-C₆ cycloalkylcarbonyl, (ix) furylcarbonyl, thienylcarbonyl, or nicotinoyl optionally substituted with methyl, methoxy, fluorine, or chlorine, (x) carbamoyl optionally having one to two substituents selected from among a group consisting of C₁-C₃ alkyls, phenyl optionally having one to two substituents selected from among a group consisting of methyl, ethyl, methoxy, ethoxy, fluorine, chlorine, and trifluoromethyl, and phenyl-C₁-C₂ alkyls optionally having in the phenyl or naphthyl portion one to two substituents selected from among a group consisting of methyl, ethyl, methoxy, ethoxy, fluorine, chlorine, bromine, and trifluoromethyl, (xi) a C₁-C₂ alkoxycarbonyl, (xii) phenoxycarbonyl optionally having one to two substituents selected from among a group consisting of methyl, ethyl, methoxy, ethoxy, fluorine, chlorine, and trifluoromethyl, or (xiii) benzyloxycarbonyl optionally having in the phenyl portion one to two substituents selected from among a group consisting of methyl, ethyl, methoxy, ethoxy, fluorine, chlorine, and trifluoromethyl),

(C-3) even more advantageously
   (a) carboxyl,
   (b) thiazolidine-2,4-dion-5-yl, oxazolidine-2,4-dion-5-yl, or 1,2,4-oxadiazolidine-3,5-dion-2-yl, (c) a group represented by the expression -C(R^{c1})(COOH)-W^{c1}-R^{c2} (where R^{c1} is (i) hydrogen, (ii) methyl, (iii) phenyl, or (iv) benzyl, R^{c2} is (i) hydrogen, (ii) methyl, ethyl, or butyl, (iii) phenyl optionally having one to two substituents selected from among a group consisting of methyl, ethyl, propyl, t-butyl, methoxy, ethoxy, fluorine, chlorine, trifluoromethyl, and benzoyl optionally having one to two substituents selected from among a group consisting of methyl, methoxy, fluorine, chlorine, and trifluoromethyl, (iv) benzyl optionally having in the phenyl portion one to two substituents selected from among a group consisting of methyl, ethyl, methoxy, ethoxy, propoxy, fluorine, chlorine, and trifluoromethyl, (v) methane, ethane, or propanesulfonyl, (vi) trifluoromethanesulfonyl, (vii) phenylsulfonyl optionally having one to two substituents selected from among a group consisting of methyl, methoxy, fluorine, chlorine, and trifluoromethyl, or (viii) benzylsulfonyl optionally having in the phenyl portion one to two substituents selected from among a group consisting of methyl, methoxy, fluorine, chlorine, and trifluoromethyl, W^{c1} is (i) oxygen, (ii) sulfur, or (iii) a group represented by the expression =N-R^{c3} (where R^{c3} is (i) hydrogen, (ii) methyl or ethyl, (iii) phenyl, (iv) benzyl, (v) acetyl, (vi) benzoyl optionally having one to two substituents selected from among a group consisting of methyl, methoxy, fluorine, chlorine, and trifluoromethyl, (vii) benzylcarbonyl optionally having in the phenyl portion one to two substituents selected from among a group consisting of methyl, methoxy, fluorine, chlorine, and trifluoromethyl, (ix) furylcarbonyl, thienylcarbonyl, or nicotinoyl, or (x) carbamoyl optionally having one to two substituents selected from among a group consisting of methyl and ethyl, phenyl optionally having one to two substituents selected from among a group consisting of methyl, methoxy, fluorine, chlorine, and trifluoromethyl, and benzyl optionally having in the phenyl portion one to two substituents selected from among a group consisting of methyl, methoxy, fluorine, chlorine, bromine, and trifluoromethyl), or (d) a group represented by the expression -N(W^{c2})-CH₂COOH (where W^{c2} is (i) hydrogen, (ii) methyl, (iii) phenyl, (iv) benzyl optionally having in the phenyl portion one to two substituents selected from among a group consisting of methyl, methoxy, fluorine, chlorine, and trifluoromethyl, (v) acetyl, propionyl, butyryl, or valeryl, (vi) benzoyl optionally having one to two substituents selected from among a group consisting of methyl, methoxy, fluorine, chlorine, and trifluoromethyl, (vii) benzylcarbonyl optionally having in the phenyl portion one to two substituents selected from among a group consisting of methyl, methoxy, fluorine, chlorine, and trifluoromethyl, (ix) furylcarbonyl, thienylcarbonyl, or nicotinoyl, (x) carbamoyl optionally having one to two substituents selected from among a group consisting of methyl and ethyl, phenyl, and benzyl optionally having in the phenyl portion one to two substituents selected from among a group consisting of methyl, methoxy, fluorine, chlorine, bromine, and trifluoromethyl, (xi) methoxycarbonyl, (xii) phenoxycarbonyl optionally having one to two substituents selected from among a group consisting of methyl, methoxy, fluorine, chlorine, and trifluoromethyl, or (xiii) benzyloxycarbonyl optionally having in the phenyl portion one to two substituents selected from among a group consisting of methyl, methoxy, fluorine, chlorine, and trifluoromethyl),

(C-4) even more advantageously
   (a) carboxyl,
   (b) thiazolidine-2,4-dion-5-yl, oxazolidine-2,4-dion-5-yl, or 1,2,4-oxadiazolidine-3,5-dion-2-yl,
   (c) (ethoxy)(carboxy)methyl, (4-t-butylphenoxy)(carboxy)methyl, (4-chlorophenoxy)(carboxy)methyl, (benzyloxy)(carboxy)methyl, (4-propoxybenzyloxy)(carboxy)methyl, (3-chlorobenzyloxy)(carboxy)methyl, (4-chlorobenzyloxy)(carboxy)methyl, (4-methoxybenzyloxy)(carboxy)methyl, (N-benzoylamino)(carboxy)methyl, (N-methyl-N-benzoylamino)(carboxy)methyl, (propylsulfonylamino)(carboxy)methyl, (phenylsulfonylamino)(carboxy)methyl, (benzylsulfonylamino)(carboxy)methyl, (N-methyl-N-phenylsulfonylamino)(carboxy)methyl, (N-ethyl-N-benzylamino)(carboxy)methyl, (N-ethyl-N-nicotinoylamino)(carboxy)methyl, (3-phenylureido)(carboxy)methyl, [1-butyl-3-(3-bromobenzyl)ureido](carboxy)methyl, [N-ethyl-N-(4-methoxybenzylcarbonyl)amino](carboxy)methyl, or (2-benzoylphenylamino)(carboxy)methyl, or
   (d) N-(4-methoxyphenoxycarbonyl)-N-carboxymethylamino, N-(4-chlorobenzyl)-N-carboxymethylamino, N-phenoxycarbonyl-N-carboxymethylamino, N-benzoyl-N-carboxymethylamino, N-(3-trifluoromethylbenzoyl)-N-carboxymethylamino, N-benzylcarbonyl-N-carboxymethylamino, N-valeryl-N-carboxymethylamino, N-(N-benzylcarbamoyl)-N-carboxymethylamino, N-(N-benzyl-N-methylcarbamoyl)-N-carboxymethylamino, or N-(N,N-diethylcarbamoyl)-N-carboxymethylamino, and

(C-5) optimally (a) carboxyl, (b) thiazolidine-2,4-dion-5-yl, (c) (4-chlorobenzyloxy)(carboxy)methyl, or (d) N-phenoxycarbonyl-N-carboxymethylamino,
   E is

(D-1) advantageously
   (1) a group represented by general formula (d-1) 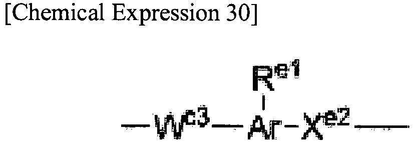 (also written as "-W^{c3}-Ar(R^{e1})-X^{e2}-")
      (where R^{e1} is the same or different zero or one to three groups, and R^{e1} is (i) a C₁-C₄ alkyl, (ii) a C₁-C₄ halogenoalkyl, (iii) a C₁-C₄ alkoxy, (iv) a halogen, (v) hydroxy, (vi) cyano, (vii) nitro, (viii) a C₃-C₆ cycloalkyl, (ix) phenyl or naphthyl optionally having one to three substituents selected from among a group consisting of C₁-C₄ alkyls, C₁-C₂ halogenoalkyls, C₁-C₄ alkoxys, and halogens, (x) a phenyl- or naphthyl-C₁-C₂ alkyl optionally having in the phenyl or naphthyl portion one to three selected from among a group consisting of C₁-C₄ alkyls, C₁-C₂ halogenoalkyls, C₁-C₄ alkoxys, and halogens, (xi) formyl or a C₁-C₄ alkylcarbonyl or C₂-C₃ alkenylcarbonyl, (xii) a C₃-C₆ cycloalkylcarbonyl, (xiii) benzoyl or naphthylcarbonyl optionally having one to three substituents selected from among a group consisting of C₁-C₄ alkyls, C₁-C₂ halogenoalkyls, C₁-C₄ alkoxys, and halogens, (xiv) a phenyl- or naphthyl-C₁-C₂ alkylcarbonyl optionally having in the aryl portion one to three substituents selected from among a group consisting of C₁-C₄ alkyls, C₁-C₂ halogenoalkyls, C₁-C₄ alkoxys, and halogens, (xv) furylcarbonyl, thienylcarbonyl, imidazolylcarbonyl, oxazolylcarbonyl, isooxazolylcarbonyl, thiazolylcarbonyl, isothiazolylcarbonyl, nicotinoyl, isonicotinoyl, pyridazinylcarbonyl, pyrimidinylcarbonyl, or pyrazinylcarbonyl optionally having one to two substituents selected from among a group consisting of C₁-C₄ alkyls, C₁-C₂ halogenoalkyls, C₁-C₄ alkoxys, and halogens, (xvi) carbamoyl, (xvii) phenylcarbamoyl or naphthylcarbonyl optionally having one to three substituents selected from among a group consisting of C₁-C₄ alkyls, C₁-C₂ halogenoalkyls, C₁-C₄ alkoxys, and halogens, (xviii) amino optionally substituted with one to two substituents selected from among a group consisting of C₁-C₄ alkyls, C₁-C₂ alkyls substituted with fluorine, and C₁-C₄ alkoxys; phenyl and naphthyl optionally having one to three substituents selected from among a group consisting of methyl, ethyl, methoxy, ethoxy, fluorine, chlorine, and trifluoromethyl; phenyl- and naphthyl-C₁-C₂ alkyls optionally having in the phenyl or naphthyl portion one to three substituents selected from among a group consisting of methyl, ethyl, methoxy, ethoxy, fluorine, chlorine, and trifluoromethyl; formyl, C₁-C₂ alkylcarbonyls, and acryloyl; benzoyl and naphthylcarbonyl optionally having one to three substituents selected from among a group consisting of methyl, ethyl, methoxy, ethoxy, fluorine, chlorine, and trifluoromethyl; phenyl- and naphthyl-C₁-C₂ alkylcarbonyls optionally having one to three substituents selected from among a group consisting of methyl, ethyl, methoxy, ethoxy, fluorine, chlorine, and trifluoromethyl; C₃-C₄ cycloalkylcarbonyls; furylcarbonyl, thienylcarbonyl, imidazolylcarbonyl, oxazolylcarbonyl, isooxazolylcarbonyl, thiazolylcarbonyl, isothiazolylcarbonyl, nicotinoyl, isonicotinoyl, pyridazinylcarbonyl, pyrimidinylcarbonyl, or pyrazinylcarbonyl optionally having one to two substituents selected from among a group consisting of methyl, ethyl, methoxy, ethoxy, fluorine, chlorine, and trifluoromethyl; carbamoyl, and phenylcarbamoyl and naphthylcarbomoyl optionally having one to three substituents selected from among a group consisting of methyl, ethyl, methoxy, ethoxy, fluorine, chlorine, and trifluoromethyl, (xix) a C₁-C₄ halogenoalkoxy, (xx) phenoxy or naphthyloxy optionally having one to three substituents selected from among a group consisting of C₁-C₄ alkyls, C₁-C₂ halogenoalkyls, C₁-C₄ alkoxys, and halogens, (xxi) a phenyl- or naphthyl-C₁-C₂ alkoxy optionally having in the phenyl or naphthyl portion one to three substituents selected from among a group consisting of C₁-C₄ alkyls, C₁-C₂ halogenoalkyls, C₁-C₄ alkoxys, and halogens, (xxii) a C₁-C₄ alkoxycarbonyl, (xxiii) phenoxycarbonyl or naphthyloxycarbonyl optionally having one to three substituents selected from among a group consisting of C₁-C₄ alkyls, C₁-C₂ halogenoalkyls, C₁-C₄ alkoxys, and halogens, or (xxiv) a phenyl- or naphthyl-C₁-C₂ alkoxycarbonyl optionally having in the phenyl or naphthyl portion one to three substituents selected from among a group consisting of C₁-C₄ alkyls, C₁-C₂ halogenoalkyls, C₁-C₄ alkoxys, and halogens,
      W^{c3} is (a) oxygen, (b) a group represented by the expression -S(O)_{q}- (where q is 0 or an integer from 1 to 2), (c) a group represented by the expression -N(R^{e2})- (where R^{e2} is (i) hydrogen, (ii) a C₁-C₄ alkyl, (iii) phenyl or naphthyl optionally having one to three substituents selected from among a group consisting of C₁-C₄ alkyls, C₁-C₂ halogenoalkyls, C₁-C₄ alkoxys, and halogens, (iv) a phenyl- or naphthyl-C₁-C₂ alkyl optionally having in the phenyl or naphthyl portion one to three substituents selected from among a group consisting of C₁-C₄ alkyls, C₁-C₂ halogenoalkyls, C₁-C₄ alkoxys, and halogens, (v) a C₁-C₄ alkylsulfonyl, (vi) a C₁-C₄ halogenoalkylsulfonyl, (vii) phenylsulfonyl or naphthylsulfonyl optionally having in the phenyl or naphthyl portion one to three substituents selected from among a group consisting of C₁-C₄ alkyls, C₁-C₂ halogenoalkyls, C₁-C₄ alkoxys, and halogens, (viii) a phenyl- or naphthyl-C₁-C₂ alkylsulfonyl optionally having in the phenyl or naphthyl portion one to three substituents selected from among a group consisting of C₁-C₄ alkyls, C₁-C₂ halogenoalkyls, C₁-C₄ alkoxys, and halogens, (ix) formyl, a C₁-C₂ alkylcarbonyl, or acryloyl, (x) benzoyl or naphthylcarbonyl optionally having one to three substituents selected from among a group consisting of C₁-C₄ alkyls, C₁-C₂ halogenoalkyls, C₁-C₄ alkoxys, and halogens, (xi) a phenyl- or naphthyl-C₁-C₂ alkylcarbonyl optionally having in the phenyl or naphthyl portion one to three substituents selected from among a group consisting of C₁-C₄ alkyls, C₁-C₂ halogenoalkyls, C₁-C₄ alkoxys, and halogens, (xii) a C₃-C₆ cycloalkylcarbonyl, (xiii) furylcarbonyl, thienylcarbonyl, imidazolylcarbonyl, oxazolylcarbonyl, isooxazolylcarbonyl, thiazolylcarbonyl, isothiazolylcarbonyl, nicotinoyl, isonicotinoyl, pyridazinylcarbonyl, pyrimidinylcarbonyl, or pyrazinylcarbonyl optionally having one to two substituents selected from among a group consisting of C₁-C4 alkyls, C₁-C₂ halogenoalkyls, C₁-C₄ alkoxys, and halogens, or (xiv) carbamoyl optionally substituted with one to two substituents selected from among a group consisting of C₁-C₄ alkyls, phenyl and naphthyl optionally having one to three substituents selected from among a group consisting of C₁-C₄ alkyls, C₁-C₂ halogenoalkyls, C₁-C₄ alkoxys, and halogens, and phenyl- and naphthyl-C₁-C₂ alkyls optionally having in the phenyl or naphthyl portion one to three substituents selected from among a group consisting of C₁-C₄ alkyls, C₁-C₂ halogenoalkyls, C₁-C₄ alkoxys, and halogens), (d) a group represented by the expression -CO-N(R^{e2})- (where R^{e2} is defined as described earlier), (e) a group represented by the expression -N(R^{e2})-CO- (where R^{e2} is defined as described earlier), (f) a group represented by the expression -CO-, (g) a C₁-C₆ alkylene optionally containing one to three atoms and/or groups selected from among a group consisting of (a) to (f), or (h) a dangling bond,
      X^{e2} is (a) oxygen, (b) sulfur, (c) a group represented by the expression -N(R^{e2})- (where R^{e2} is defined as described earlier), (d) a group represented by the expression -CO-N(R^{e2})- (where R^{e2} is defined as described earlier), (e) a C₁-C₆ alkylene optionally containing one to three atoms and/or groups selected from among a group consisting of oxygen, sulfur, groups represented by the expression -N(R^{e2})- (where R^{e2} is defined as described earlier), and groups represented by the formula -CO-N (R^{e2})- (where R^{e2} is defined as described earlier), (f) a group represented by the expression -W^{c4}-Phe-X^{e3}- (where W^{c4} is oxygen, a group represented by the expression - S(O)_{q}- (where q is defined as described earlier) or the expression -N(R^{e2})- (where R^{e2} is defined as described earlier), a C₁-C₄ alkylene optionally containing one to two atoms and/or groups selected from among a group consisting of oxygen and groups represented by the expression - S(O)_{q}- (where q is defined as described earlier) and the expression -N(R^{e2})- (where R^{e2} is defined as described earlier), or a dangling bond, Phe is phenylene optionally having one to three substituents selected from among a group consisting of C₁-C₄ alkyls, C₁-C₂ halogenoalkyls, C₁-C₄ alkoxys, and halogens, and X^{e3} is oxygen, a group represented by the expression -S(O)_{q}-(where q is defined as described earlier) or the expression -N(R^{e2})- (where R^{e2} is defined as described earlier), a C₁-C₄ alkylene optionally containing one to two atoms and/or groups selected from among a group consisting of oxygen and groups represented by the expression - S(O)_{q}- (where q is defined as described earlier) and the expression -N(R^{e2})- (where R^{e2} is defined as described earlier), or a dangling bond), or (g) a dangling bond, and Ar is a divalent phenyl, naphthyl, pyrrole, furan, thiophen, pyrazole, imidazole, 1,2,4-triazole, tetrazole, isoxazole, oxazole, 1,3,4-oxadiazole, thiazole, isothiazole, 1,3,4-thiadiazole, pyridine, pyridazine, pyrimidine, pyrazine, indole, benzofuran, benzo[B]thiophen, benzimidazole, benzotriazole, benzisoxazole, benzoxazole, benzisothiazole, benzothiazole, oxazolo[4,5-B]pyridine, benzofurazan, pyrazolo[4,5-C]pyridine, pyrazolo[3,4-B]pyridine, 1,2,3-benzothiadiazole, pyrazolo-[3,4-D]pyrimidine, quinoline, isoquinoline, quinoxaline, pyrido[2,3-B]pyrazine, pyridoimidazole, quinazoline, pyrido[2,3-B]pyrazine, pyridoimidazole, quinazoline, phthalazine, or naphthylidine cyclic group); or
   (2) the group represented by the expression -W^{c3}-W^{c3}-X^{e4}- is oxygen, a group represented by the expression -S(O)_{q}- (where q is defined as described earlier), a group represented by the expression -N(R^{e2})- (where R^{e2} is defined as described earlier), a group represented by the expression -CO-N(R^{e2})- (where R^{e2} is defined as described earlier), a group represented by the expression -N(R^{e2})-CO- (where R^{e2} is defined as described earlier), a C₁-C₁₀ alkylene optionally containing one to three atoms and/or groups selected from among a group consisting of oxygen, groups represented by the expression -S(O)_{q}- (where q is defined as described earlier), groups represented by the expression -N(R^{e2})- (where R^{e2} is defined as described earlier), groups represented by the expression -CO-N(R^{e2})- (where R^{e2} is defined as described earlier), and groups represented by the expression -N(R^{e2})-CO- (where R^{e2} is defined as described earlier) (advantageously a C₁-C₁₀ alkylene containing these atoms and/or groups), or a dangling bond),

(D-2) more advantageously
   (1) a group represented by general formula (d-1)
      (where R^{e1} is the same or different zero or one to two groups, and R^{e1} is (i) methyl or ethyl, (ii) a C₁-C₂ alkyl substituted with fluorine, (iii) methoxy or ethoxy, (iv) fluorine or chlorine, (viii) cyclopropyl, (ix) phenyl, (x) benzyl, (xi) formyl or acetyl, (xii) cyclopropylcarbonyl, (xiii) benzoyl, (xiv) benzylcarbonyl, (xv) furylcarbonyl, thienylcarbonyl, or nicotinoyl, (xvi) carbamoyl, (xvii) phenylcarbamoyl, (xviii) amino, N-methylamino, N-ethylamino, N,N-dimethylamino, or N-methyl-N-ethylamino, (xix) fluoromethoxy or trifluoromethoxy, (xx) phenoxy, (xxi) benzyloxy, (xxiii) a C₁-C₂ alkoxycarbonyl, (xxiii) phenoxycarbonyl, or (xxiv) benzyloxycarbonyl,
      W^{c3} is (a) oxygen, (b) a group represented by the expression -S(O)_{q}- (where q is 0 or an integer from 1 to 2), (c) a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, (v) methylsulfonyl, (vi) trifluoromethylsulfonyl, (vii) phenylsulfonyl, (viii) benzylsulfonyl, (ix) formyl or acetyl, (x) benzoyl, (xi) benzylcarbonyl optionally having in the phenyl portion one to two substituents selected from among a group consisting of methyl, methoxy, fluorine, chlorine, and trifluoromethyl, (xii) cyclopropylcarbonyl, (xiii) furylcarbonyl, thienylcarbonyl, or nicotinoyl, or (xiv) carbamoyl optionally substituted with one to two substituents selected from among a group consisting of methyl and ethyl, phenyl, and benzyl optionally having in the phenyl portion one to two substituents selected from among a group consisting of methyl, methoxy, fluorine, chlorine, and trifluoromethyl), (d) a group represented by the expression -CO-N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), (e) a group represented by the expression -N(R^{e2})-CO- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), (f) a group represented by the expression -CO-, (g) a C₁-C₄ alkylene optionally containing one to three atoms and/or groups selected from among a group consisting of (a) to (f), or (h) a dangling bond, X^{e2} is (a) oxygen, (b) sulfur, (c) a group represented by the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; or acetyl), (d) a group represented by the expression -CO-N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), (e) a C₁-C₄ alkylene optionally containing one to three atoms and/or groups selected from among a group consisting of oxygen, sulfur, groups represented by the expression -N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), and groups represented by the formula -CO-N (R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), (f) a group represented by the expression -W^{c4}-Phe-X^{e3}- (where W^{c4} is oxygen, a group represented by the expression -S(O)_{q}- (where q is 0 or an integer from 1 to 2) or the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; formyl, or acetyl), a C₁-C₃ alkylene optionally containing one to two atoms and/or groups selected from among a group consisting of oxygen and groups represented by the expression -S(O)_{q}- (where q is 0 or an integer from 1 to 2) and the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; formyl, or acetyl), or a dangling bond, Phe is phenylene optionally having one to two substituents selected from among a group consisting of methyl, ethyl, trifluoromethyl, methoxy, ethoxy, fluorine, and chlorine, and X^{e3} is oxygen, a group represented by the expression -S(O)_{q}- (where q is defined as described earlier) or the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; formyl, or acetyl), a C₁-C₃ alkylene optionally containing one to two atoms and/or groups selected from among a group consisting of oxygen and groups represented by the expression -S(O)_{q}- (where q is defined as described earlier) and the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; formyl, or acetyl), or a dangling bond), or (g) a dangling bond, and
      Ar is a divalent phenyl, naphthyl, furan, thiophen, imidazole, tetrazole, oxazole, thiazole, pyridine, pyridazine, pyrimidine, pyrazine, indole, benzofuran, benzo[B]thiophen, benzimidazole, benzisoxazole, benzoxazole, benzisothiazole, benzothiazole, quinoline, isoquinoline, or pyridoimidazole cyclic group); or
   (2) the group represented by the expression -W^{c3}-W^{c3}-X^{e4}- is oxygen, a group represented by the expression -S(O)_{q}- (where q is 0 or an integer from 1 to 2), a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, (v) methylsulfonyl, (vi) trifluoromethylsulfonyl, (vii) phenylsulfonyl, (viii) benzylsulfonyl, (ix) formyl or acetyl, (x) benzoyl, (xi) benzylcarbonyl optionally having in the phenyl portion one to two substituents selected from among a group consisting of methyl, methoxy, fluorine, chlorine, and trifluoromethyl, (xii) cyclopropylcarbonyl, (xiii) furylcarbonyl, thienylcarbonyl, or nicotinoyl, or (xiv) carbamoyl optionally substituted with one to two substituents selected from among a group consisting of methyl and ethyl, phenyl, and benzyl optionally having in the phenyl portion one to two substituents selected from among a group consisting of methyl, methoxy, fluorine, chlorine, and trifluoromethyl), a group represented by the expression -CO-N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), a group represented by the expression -N(R^{e2})-CO- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), a C₁-C₄ alkylene optionally containing one to three atoms and/or groups selected from among a group consisting of oxygen, groups represented by the expression - S(O)_{q}- (where q is defined as described earlier), groups represented by the expression -N(R^{e2})-(where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, (v) methylsulfonyl, (vi) trifluoromethylsulfonyl, (vii) phenylsulfonyl, (viii) benzylsulfonyl, (ix) formyl or acetyl, (x) benzoyl, (xi) benzylcarbonyl optionally having in the phenyl portion one to two substituents selected from among a group consisting of methyl, methoxy, fluorine, chlorine, and trifluoromethyl, (xii) cyclopropylcarbonyl, (xiii) furylcarbonyl, thienylcarbonyl, or nicotinoyl, or (xiv) carbamoyl optionally substituted with one to two substituents selected from among a group consisting of methyl and ethyl, phenyl, and benzyl optionally having in the phenyl portion one to two substituents selected from among a group consisting of methyl, methoxy, fluorine, chlorine, and trifluoromethyl), groups represented by the expression -CO-N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), and groups represented by the expression -N(R^{e2})-CO- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl) (advantageously a C₁-C₄ alkylene containing these atoms and/or groups), or a dangling bond,

(D-3) even more advantageously
   (1) a group represented by general formula (d-1)
      (where R^{e1} is the same or different zero or one to two groups, and R^{e1} is (i) methyl or ethyl, (ii) trifluoromethyl, (iii) methoxy or ethoxy, or (iv) fluorine or chlorine,
      W^{c3} is (a) oxygen, (b) a group represented by the expression -S(O)_{q}- (where q is 0 or an integer from 1 to 2), (c) a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, or (ix) formyl or acetyl), (d) a group represented by the expression -CON(R^{e2})- (where R^{e2} is hydrogen or methyl), (e) a group represented by the expression -N(R^{e2})-CO- (where R^{e2} is hydrogen or methyl), (g) a C₁-C₄ alkylene optionally containing one to three atoms and/or groups selected from among a group consisting of (a) to (e), or (h) a dangling bond, X^{e2} is (a) oxygen, (b) sulfur, (c) a group represented by the expression -N(R^{e2})- (where R^{e2} is hydrogen, methyl, benzyl, or acetyl), (e) a C₁-C₄ alkylene optionally containing one to two atoms and/or groups selected from among a group consisting of oxygen, sulfur, and groups represented by the expression -N(R^{e2})- (where R^{e2} is hydrogen, methyl, benzyl, or acetyl) or (g) a dangling bond, and
      Ar is a divalent phenyl, pyridine, benzimidazole, or pyridoimidazole); or
   (2) the group represented by the expression -W^{c3}-W^{c3}-X^{e4}- is oxygen, a group represented by the expression -S(O)_{q}- (where q is 0 or an integer from 1 to 2), a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, or (ix) formyl or acetyl), a group represented by the expression -CO-N(R^{e2})- (where R^{e2} is hydrogen or methyl), a group represented by the expression -N(R^{e2})-CO- (where R^{e2} is hydrogen or methyl), a C₁-C₄ alkylene optionally containing one to three atoms and/or groups selected from among a group consisting of oxygen, groups represented by the expression -S(O)_{q}- (where q is defined as described earlier), groups represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, or (ix) formyl or acetyl), groups represented by the expression -CO-N(R^{e2})-(where R^{e2} is hydrogen or methyl), and groups represented by the expression -N(R^{e2})-CO-(where R^{e2} is hydrogen or methyl) (advantageously a C₁-C₄ alkylene containing these atoms and/or groups), or a dangling bond,

(D-4) even more advantageously
   (1) a group represented by the expression -CH₂O-1,4-phenylene-S-, -CH₂S-1, 3-phenylene-O-, - CH₂S-1,4-phenylene-O-, -CH₂SO₂-1,4-phenylene-O-, -CH₂NH-1,4-phenylene-O-, -CH₂NH-1,4-(3,5-dimethylphenylene)-O-, -CH(CH₃)S-1,4-phenylene-O-, -CH₂SCH₂-1,4-phenylene-O-, - CH₂SCH₂CH₂-1,3-phenylene-O-, -SCH₂-1,4-phenylene-O-, -CONHCH₂-1,4-phenylene-O-, - CH₂S-3,5-pyridinylene-S-, -CH₂S-3,6-pyridinylene-O-, -CH(CH₃)S-3,6-pyridinylene-O-, -SCH₂-3,6-pyridinylene-O-, -CH₂SCH₂-2,5-pyridinylene-O-, -CH₂SCH₂-3,6-pyridinylene-O-, - CH₂SCH₂-2,5-pyridinylene-S-, 2,6-(1-methylbenzimidazolylene)-S-, 2,6-(1-methylbenzimidazolylene)-O-CH₂CH₂-O, or 2,6-(1-methylbenzimidazolylene)-N(CH₃)-CH₂CH₂-O-; or
   (2) a group represented by the expression -CH₂O-, -CH₂S-, -CH(-CH₃)S-, -CH₂SO-, -CH₂SO₂-, - CH₂N(-CH₃)-, -CH₂CH₂O-, -CH₂SCH₂CH₂O-, -C(-CH₃)₂SCH₂CH₂O-, -N(-CH₃)-CH₂CH₂-O-, - CH₂SCH₂CH₂NHCOCH₂S-, or -SCH₂-, or a dangling bond, and

(D-5) optimally
   (1) a group represented by the expression -CH₂O-1,4-phenylene-S-, -CH₂S-1, 3-phenylene-O-, - CH₂S-1,4-phenylene-O-, -CH₂SO₂-1,4-phenylene-O-, -CH₂NH-1,4-phenylene-O-, -CH₂NH-1,4-(3,5-dimethylphenylene)-O-, -CH(CH₃)S-1,4-phenylene-O-, -CH₂SCH₂-1,4-phenylene-O-, - CH₂SCH₂CH₂-1,3-phenylene-O-, -SCH₂-1,4-phenylene-O-, -CONHCH₂-1,4-phenylene-O-, 2,6-(1-methylbenzimidazolylene)-S-, or 2,6-(1-methylbenzimidazolylene)-N(CH₃)-CH₂CH₂-O-; or (2) a group represented by the expression -CH₂O-, -CH₂S-, -CH(-CH₃)S-, -CH₂SO-, -CH₂SO₂-, - CH₂N(-CH₃)-, -CH₂CH₂O-, -CH₂SCH₂CH₂O-, -C(-CH₃)₂SCH₂CH₂O-, -N(-CH₃)-CH₂CH₂-O-, - CH₂SCH₂CH₂NHCOCH₂S-, or -SCH₂-, or a dangling bond,
      G is

(E-1) advantageously
   a dangling bond, oxygen, sulfur, or a group represented by the expression -N(R^{e2})- (where R^{e2} is (i) hydrogen, (ii) a C₁-C₄ alkyl, (iii) phenyl or naphthyl optionally having one to three substituents selected from among a group consisting of C₁-C₄ alkyls, C₁-C₂ halogenoalkyls, C₁-C₄ alkoxys, and halogens, (iv) a phenyl- or naphthyl-C₁-C₂ alkyl optionally having in the phenyl or naphthyl portion one to three substituents selected from among a group consisting of C₁-C₄ alkyls, C₁-C₂ halogenoalkyls, C₁-C₄ alkoxys, and halogens, (v) a C₁-C₂ alkylsulfonyl, (vi) a C₁-C₂ alkylsulfonyl substituted with fluorine, (vii) phenylsulfonyl optionally having one to two substituents selected from among a group consisting of methyl, methoxy, fluorine, chlorine, and trifluoromethyl, (viii) a phenyl-C₁-C₂ alkylsulfonyl optionally having in the phenyl portion one to two substituents selected from among a group consisting of methyl, ethyl, methoxy, ethoxy, fluorine, chlorine, and trifluoromethyl, (ix) formyl or acetyl, (x) benzyl optionally having one to two substituents selected from among a group consisting of methyl, methoxy, fluorine, chlorine, and trifluoromethyl, (xi) benzylcarbonyl optionally having in the phenyl portion one to two substituents selected from among a group consisting of methyl, methoxyethoxy, fluorine, chlorine, and trifluoromethyl, (xii) cyclopropylcarbonyl, (xiii) furylcarbonyl, thienylcarbonyl, or nicotinoyl, or (xiv) carbamoyl optionally having one to two substituents selected from among a group consisting of methyl, ethyl, phenyl, and benzyl optionally having one to two substituents selected from among a group consisting of methyl, methoxy, fluorine, chlorine, and trifluoromethyl),

(E-2) more advantageously
   a dangling bond, oxygen, sulfur, or a group represented by the expression -N(R^{e2})- (where R^{e2} is (i) hydrogen, (ii) a C₁-C₃ alkyl, (iii) phenyl optionally substituted with methyl, methoxy, fluorine, chlorine, or trifluoromethyl, or (iv) benzyl optionally substituted in the phenyl portion with methyl, methoxy, fluorine, chlorine, or trifluoromethyl),
(E-3) even more advantageously
a dangling bond, oxygen, sulfur, or a group represented by the expression -N(R^{e2})- (where R^{e2} is (i) hydrogen, or (ii) methyl or ethyl), and

(E-4) optimally
   a dangling bond or a group represented by the expression -N(R^{e2})- (where R^{e2} is methyl or ethyl),
   in the group represented by the expression -V-R,

(F-1) advantageously
   V is a dangling bond, oxygen, sulfur, or a group represented by the expression -N(R ²)- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, or (ix) acetyl, and R is (i) hydrogen, (ii) a C₁-C₆ alkyl, (iii) phenyl or naphthyl optionally having one to two substituents selected from among a group consisting of methyl and ethyl; trifluoromethyl; methoxy and ethoxy; fluorine, chlorine, and bromine; phenyl; benzoyl optionally having one to two substituents selected from among a group consisting of methyl, methoxy, fluorine, chlorine, and trifluoromethyl; and trifluoromethoxy, or (iv) a phenyl- or naphthyl-C₁-C₃ alkyl optionally having in the phenyl or naphthyl portion one to two substituents selected from among a group consisting of methyl and ethyl; trifluoromethyl; methoxy and ethoxy; fluorine, chlorine, and bromine; phenyl; benzoyl optionally having one to two substituents selected from among a group consisting of methyl, methoxy, fluorine, chlorine, and trifluoromethyl; and trifluoromethoxy,

(F-2) more advantageously
   V is a dangling bond, oxygen, or sulfur, and R is (i) hydrogen, (ii) methyl, ethyl, propyl, i-propyl, butyl, i-butyl, s-butyl, pentyl, or hexyl, (iii) phenyl optionally having one to two substituents selected from among a group consisting of methyl; trifluoromethyl; methoxy; fluorine, chlorine, and bromine; benzoyl optionally having one to two substituents selected from among a group consisting of methyl, methoxy, fluorine, chlorine, and trifluoromethyl; and trifluoromethoxy, or (iv) a phenyl-C₁-C₃ alkyl optionally having in the phenyl portion one to two substituents selected from among a group consisting of methyl; trifluoromethyl; methoxy; fluorine, chlorine, and bromine; phenyl; benzoyl optionally having one to two substituents selected from among a group consisting of methyl, methoxy, fluorine, chlorine, and trifluoromethyl; and trifluoromethoxy, and

(F-3) optimally
   V is a dangling bond or oxygen, and R is (i) hydrogen, (ii) methyl, i-propyl, or hexyl, (iii) phenyl, 2-, 3-, or 4-trifluoromethylphenyl, 4-methoxyphenyl, 4-chloro-3-methoxyphenyl, 3- or 4-bromophenyl, 4-methylbenzoylphenyl, 3-chlorobenzoylphenyl, or 3-trifluoromethoxyphenyl, or (iv) benzyl, 3- or 4-trifluoromethylbenzyl, 2-, 3-, or 4-methoxybenzyl, 4-chlorobenzyl, 3-bromobenzyl, 4-(4-methylbenzoyl)benzyl, 3-chlorobenzoylbenzyl, 3-trifluoromethoxybenzyl, α-methylbenzyl, 2-(4-methoxyphenyl)ethyl, or 3-phenylpropyl, in the group represented by the expression -(CH₂)ₙ-Q-,

(G-1) advantageously
   n is an integer of 1 to 4 (provided, however, that n is an integer of 2 to 4 when G is oxygen, sulfur, or a group represented by the expression -N(R^{e2})- (where R^{e2} is defined as described earlier)), and Q is oxygen or sulfur,
(G-2) more advantageously
   n is an integer of 1 to 4 (provided, however, that n is an integer of 2 to 4 when G is oxygen, sulfur, or a group represented by the expression -N(R^{e2})- (where R^{e2} is defined as described earlier)), and Q is oxygen,
(G-3) even more advantageously
   n is an integer of 1 to 3 (provided, however, that n is an integer of 2 to 3 when G is oxygen, sulfur, or a group represented by the expression -N(R^{e2})- (where R^{e2} is defined as described earlier)), and Q is oxygen, and
(G-4) optimally
   n is an integer of 1 to 2 (provided, however, that n is an integer of 2 when G is oxygen, sulfur, or a group represented by the expression -N(R^{e2})- (where R^{e2} is defined as described earlier)), and Q is oxygen, and

p is
(H-1) advantageously an integer of 1 to 4,
(H-2) more advantageously an integer of 1 to 3, and
(H-3) optimally an integer of 1 to 2.

Advantageously, a compound may be obtained by selecting A from among (A-1) to (A-5), B from among (B-1) to (B-4), C from among (C-1) to (C-5), E from among (D-1) to (D-5), G from among (E-1) to (E-4), the group represented by the expression -V-R from among (F-1) to (F-3), the group represented by the expression -(CH₂)ₙ-Q- from among (G-1) to (G-4), and p from among (H-1) to (H-3), and combining these selections. For example, an advantageous compound is one in which
A is (A-2)

(a) a group represented by general formula (a-1)
   (where R^{a1} is (i) a C₃-C₅ alkyl, (ii) a C₃-C₄ alkyl substituted with fluorine, (iii) a C₂-C₄ alkoxy, (iv) a C₂-C₄ alkylthio, (v) a group represented by the expression -N(R^{a3})(R^{a4}) (where R^{a3} is (i) hydrogen or (ii) a C₂-C₄ alkyl, and R^{a4} is hydrogen), (vi) a C₃-C₄ cycloalkyl, (vii) cyclopropyloxy, or (viii) cyclopropylthio,
   R^{a2} is (i) hydrogen, (ii) a halogen, (iii) formyl, (iv) a C₁-C₃ alkylcarbonyl, (v) carboxyl or a C₁-C₄ alkoxycarbonyl, (vi) carbamoyl, (vii) a mono- or di-C₁-C₃ alkylcarbamoyl, (ix) formylamino or acetylamino, (x) a C₁-C₃ hydroxyalkyl, (xi) a formyl-C₁-C₂ alkyl, or (xii) a C₁-C₂ alkylcarbonyl-C₁-C₂ alkyl, and
   D is (i) carboxyl, (ii) 5-tetrazolyl, (iii) 5-oxo-1,2,4-oxadiazolin-3-yl, (iv) 2,4-dioxooxazolidin-5-yl or 2,4-dioxothizolidin-5-yl, (v) a group represented by the expression CF₃SO₂-NH-, (vi) tetrazol-5-ylaminocarbonyl, or (viii) acetylaminosulfonyl),

(b) a group represented by the expressions (b-1) to (b-7)
   (where D and R^{a1} are defined as in the expression (a-1) of (A-2), R^{a5} is (i) hydrogen, (ii) a halogen, (iii) formyl, (iv) a C₁-C₃ alkylcarbonyl, (v) carboxyl or a C₁-C₄ alkoxycarbonyl, (vi) carbamoyl, (vii) a mono- or di-C₁-C₄ alkylcarbamoyl, (ix) formylamino or acetylamino, (x) a C₁-C₂ hydroxyalkyl, (xi) a formyl-C₁-C₂ alkyl, (xii) a C₂-C₄ alkylcarbonyl-C₁-C₂ alkyl, (xiii) a C₁-C₄ alkyl or hexyl, (xiv) a C₁-C₃ alkyl substituted with fluorine or chlorine, or (xv) a C₁-C₃ alkoxy, the bond embraced by the dotted line is a single bond or a double bond, R^{a3} is (i) hydrogen or (ii) a C₁-C₄ alkyl, and R^{a3}a is (ii) a C₁-C₄ alkyl),
(e) a group represented by general formula (e-1)
   (where D and R^{a1} are defined as in the expression (a-1) of (A-2), and R^{a7} is cyclohexyl), (f) a group represented by general formula (f-1)
   (where D and R^{a1} are defined as in the expression (a-1) of (A-2), R^{a8} is hydrogen or methyl, and L^{d} is a group represented by the expression =N-), or
(g) a group represented by general formula (g-1)
   (where D is defined as in the expression (a-1) of (A-2), and R^{a9} is a C₂-C₄ alkyl, a C₃-C₄ alkyl substituted with fluorine, or a C₃-C₆ cycloalkyl),

B is (B-3)
a group represented by general formula (bb-1)
(where R^{a3} is (i) hydrogen, (ii) methyl, ethyl, propyl, i-propyl, or butyl, or (iv) benzyl optionally substituted in the phenyl portion with methyl, methoxy, fluorine, chlorine, or trifluoromethyl, T is the expression -CH= or -N=, and B is bonded to E by a portion of a benzene or pyridine ring and to G by a portion of an imidazole ring),

C is (C-3)
(a) carboxyl,
(b) thiazolidine-2,4-dion-5-yl, oxazolidine-2,4-dion-5-yl, or 1,2,4-oxadiazolidine-3,5-dion-2-yl,
(c) a group represented by the expression -C(R^{c1})(COOH)-W^{c1}-R^{c2} (where R^{c1} is (i) hydrogen, (ii) methyl, (iii) phenyl, or (iv) benzyl, R^{c2} is (i) hydrogen, (ii) methyl, ethyl, or butyl, (iii) phenyl optionally having one to two substituents selected from among a group consisting of methyl, ethyl, propyl, t-butyl, methoxy, ethoxy, fluorine, chlorine, trifluoromethyl, and benzoyl optionally having one to two substituents selected from among a group consisting of methyl, methoxy, fluorine, chlorine, and trifluoromethyl, (iv) benzyl optionally having in the phenyl portion one to two substituents selected from among a group consisting of methyl, ethyl, methoxy, ethoxy, propoxy, fluorine, chlorine, and trifluoromethyl, (v) methane, ethane, or propanesulfonyl, (vi) trifluoromethanesulfonyl, (vii) phenylsulfonyl optionally having one to two substituents selected from among a group consisting of methyl, methoxy, fluorine, chlorine, and trifluoromethyl, or (viii) benzylsulfonyl optionally having in the phenyl portion one to two substituents selected from among a group consisting of methyl, methoxy, fluorine, chlorine, and trifluoromethyl, W^{c1} is (i) oxygen, (ii) sulfur, or (iii) a group represented by the expression =N-R^{c3} (where R^{c3} is (i) hydrogen, (ii) methyl or ethyl, (iii) phenyl, (iv) benzyl, (v) acetyl, (vi) benzoyl optionally having one to two substituents selected from among a group consisting of methyl, methoxy, fluorine, chlorine, and trifluoromethyl, (vii) benzylcarbonyl optionally having in the phenyl portion one to two substituents selected from among a group consisting of methyl, methoxy, fluorine, chlorine, and trifluoromethyl, (ix) furylcarbonyl, thienylcarbonyl, or nicotinoyl, or (x) carbamoyl optionally having one to two substituents selected from among a group consisting of methyl and ethyl, phenyl optionally having one to two substituents selected from among a group consisting of methyl, methoxy, fluorine, chlorine, and trifluoromethyl, and benzyl optionally having in the phenyl portion one to two substituents selected from among a group consisting of methyl, methoxy, fluorine, chlorine, bromine, and trifluoromethyl), or
(d) a group represented by the expression -N(W^{c2})-CH₂COOH (where W^{c2} is (i) hydrogen, (ii) methyl, (iii) phenyl, (iv) benzyl optionally having in the phenyl portion one to two substituents selected from among a group consisting of methyl, methoxy, fluorine, chlorine, and trifluoromethyl, (v) acetyl, propionyl, butyryl, or valeryl, (vi) benzoyl optionally having one to two substituents selected from among a group consisting of methyl, methoxy, fluorine, chlorine, and trifluoromethyl, (vii) benzylcarbonyl optionally having in the phenyl portion one to two substituents selected from among a group consisting of methyl, methoxy, fluorine, chlorine, and trifluoromethyl, (ix) furylcarbonyl, thienylcarbonyl, or nicotinoyl, (x) carbamoyl optionally having one to two substituents selected from among a group consisting of methyl and ethyl, phenyl, and benzyl optionally having in the phenyl portion one to two substituents selected from among a group consisting of methyl, methoxy, fluorine, chlorine, bromine, and trifluoromethyl, (xi) methoxycarbonyl, (xii) phenoxycarbonyl optionally having one to two substituents selected from among a group consisting of methyl, methoxy, fluorine, chlorine, and trifluoromethyl, or (xiii) benzyloxycarbonyl optionally having in the phenyl portion one to two substituents selected from among a group consisting of methyl, methoxy, fluorine, chlorine, and trifluoromethyl),

E is (D-4)
(1) a group represented by the expression -CH₂O-1,4-phenylene-S-, -CH₂S-1, 3-phenylene-O-, - CH₂S-1,4-phenylene-O-, -CH₂SO₂-1,4-phenylene-O-, -CH₂NH-1,4-phenylene-O-, -CH₂NH-1,4-(3,5-dimethylphenylene)-O-, -CH(CH₃)S-1,4-phenylene-O-, -CH₂SCH₂-1,4-phenylene-O-, - CH₂SCH₂CH₂-1,3-phenylene-O-, -SCH₂-1,4-phenylene-O-, -CONHCH₂-1,4-phenylene-O-, - CH₂S-3,5-pyridinylene-S-, -CH₂S-3,6-pyridinylene-O-, -CH(CH₃)S-3,6-pyridinylene-O-, -SCH₂-3,6-pyridinylene-O-, -CH₂SCH₂-2,5-pyridinylene-O-, -CH₂SCH₂-3,6-pyridinylene-O-, - CH₂SCH₂-2,5-pyridinylene-S-, 2,6-(1-methylbenzimidazolylene)-S-, 2,6-(1-methylbenzimidazolylene)-O-CH₂CH₂-O, or 2,6-(1-methylbenzimidazolylene)-N(CH₃)-CH₂CH₂-O-; or
(2) a group represented by the expression -CH₂O- -CH₂S-, -CH(-CH₃)S-, -CH₂SO-, -CH₂SO₂-, - CH₂N(-CH₃)-, -CH₂CH₂O-, -CH₂SCH₂CH₂O-, -C(-CH₃)₂SCH₂CH₂O-, -N(-CH₃)-CH₂CH₂-O-, - CH₂SCH₂CH₂NHCOCH₂S-, or -SCH₂-, or a dangling bond,

G is (E-1)
a dangling bond, oxygen, sulfur, or a group represented by the expression -N(R^{e2})- (where R^{e2} is (i) hydrogen, (ii) a C₁-C₄ alkyl, (iii) phenyl or naphthyl optionally having one to three substituents selected from among a group consisting of C₁-C₄ alkyls, C₁-C₂ halogenoalkyls, C₁-C₄ alkoxys, and halogens, (iv) a phenyl- or naphthyl-C₁-C₂ alkyl optionally having in the phenyl or naphthyl portion one to three substituents selected from among a group consisting of C₁-C₄ alkyls, C₁-C₂ halogenoalkyls, C₁-C₄ alkoxys, and halogens, (v) a C₁-C₂ alkylsulfonyl, (vi) a C₁-C₂ alkylsulfonyl substituted with fluorine, (vii) phenylsulfonyl optionally having one to two substituents selected from among a group consisting of methyl, methoxy, fluorine, chlorine, and trifluoromethyl, (viii) benzylsulfonyl optionally having in the phenyl portion one to two substituents selected from among a group consisting of methyl, methoxy, fluorine, chlorine, and trifluoromethyl, (ix) formyl or acetyl, (x) benzoyl optionally having one to two substituents selected from among a group consisting of methyl, methoxy, fluorine, chlorine, and trifluoromethyl, (xi) benzylcarbonyl optionally having in the phenyl portion one to two substituents selected from among a group consisting of methyl, methoxy, fluorine, chlorine, and trifluoromethyl, (xii) cyclopropylcarbonyl, (xiii) furylcarbonyl, thienylcarbonyl, or nicotinoyl, or (xiv) carbamoyl optionally having one to two substituents selected from among a group consisting of methyl, ethyl, phenyl, and benzyl optionally having one to two substituents selected from among a group consisting of methyl, methoxy, fluorine, chlorine, and trifluoromethyl),

the group represented by the expression -V-R is (F-2)
V is a dangling bond, oxygen, or sulfur, and R is (i) hydrogen, (ii) methyl, ethyl, propyl, i-propyl, butyl, i-butyl, s-butyl, pentyl, or hexyl, (iii) phenyl optionally having one to two substituents selected from among a group consisting of methyl; trifluoromethyl; methoxy; fluorine, chlorine, and bromine; benzoyl optionally having one to two substituents selected from among a group consisting of methyl, methoxy, fluorine, chlorine, and trifluoromethyl; and trifluoromethoxy, or (iv) a phenyl-C₁-C₃ alkyl optionally having in the phenyl portion one to two substituents selected from among a group consisting of methyl; trifluoromethyl; methoxy; fluorine, chlorine, and bromine; phenyl; benzoyl optionally having one to two substituents selected from among a group consisting of methyl, methoxy, fluorine, chlorine, and trifluoromethyl; and trifluoromethoxy,

the group represented by the expression -(CH₂)ₙ-Q- is (G-1)
n is an integer of 1 to 4 (provided, however, that n is an integer of 2 to 4 when G is oxygen, sulfur, or a group represented by the expression -N(R^{e2})- (where R^{e2} is defined as described earlier)), and Q is oxygen or sulfur, and
p is (H-1)
an integer of 1 to 4.

Specific advantageous compounds are indicated in Tables 1 to 5.

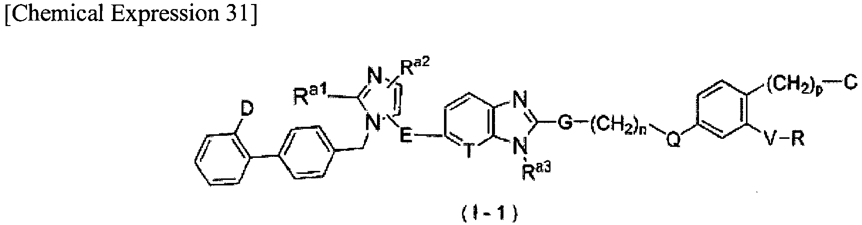

**TABLE 1**

| No. | D | R^{a1} | R^{a2} | E | T | R^{a3} | G | n | Q | -V-R | P | C |
|---|---|---|---|---|---|---|---|---|---|---|---|---|
| I-1 | Tz | Pr | 5-CO₂Et | MS4PhO | CH | Me | - | 1 | 0 | H | 1 | Tzd |
| 1-2 | Tz | Pr | 5-CO₂Et | MS4PhO | N | Me | - | 1 | 0 | H | 1 | Tzd |
| 1-3 | Cx | Pr | 5-CO₂Et | MS4PhO | CH | Me | - | 1 | 0 | H | 1 | Tzd |
| I-4 | Cx | Pr | 5-CO₂Me | MS4PhO | CH | Me | - | 1 | 0 | H | 1 | Tzd |
| I-5 | CONT z | Pr | 5-CO₂Me | MS4PhO | CH | Me | 0 | 2 | 0 | H | 1 | Tzd |
| I-6 | NSCF | Pr | 5-CO₂Me | MS4PhO | CH | Me | - | 1 | 0 | H | 1 | Tzd |
| I-7 | SNCO Pr | Pr | 5-CO₂Me | MS4PhO | CH | Me | NMe | 2 | S | H | 1 | Tzd |
| I-8 | SNCO MClN P | Pr | 5-CO₂Et | MS4PhO | N | Et | - | 1 | 0 | H | 1 | Tzd |
| I-9 | Tzd | Pr | 5-CO₂Et | MS4PhO | CH | Me | - | 1 | 0 | H | 1 | Tzd |
| I-10 | Dioz | Pr | 5-CO₂Et | MS4PhO | N | Me | - | 1 | 0 | H | 1 | Tzd |
| I-11 | Hyd | Pr | 5-CO₂Et | MS4PhO | CH | Me | - | 1 | 0 | H | 1 | Tzd |
| I-12 | Oxdz | Pr | 5-CO₂Et | MS4PhO | CH | Me | - | 1 | 0 | H | 1 | Tzd |
| I-13 | Oxtd | Pr | 5-CO₂Et | MS4PhO | CH | Me | - | 1 | 0 | H | 1 | MNBzCx |
| I-14 | Tz | Pr | 5-CO₂Et | MS4PbO | CH | Me | - | 1 | 0 | H | 1 | MNEBnC x |
| I-15 | Tz | Pr | 5-CO₂Et | MS4PhO | CH | Me | - | 1 | 0 | H | 1 | MNEPyC OCx |
| I-16 | Tz | Pr | 5-CO₂Et | MS4PhO | CH | Me | - | 1 | 0 | H | 1 | MNMBzC x |
| I-17 | Tz | Pr | 5-CO₂Et | MS4PhO | CH | Me | - | 1 | 0 | H | 1 | MNBCON BrBnCx |
| I-18 | Thox dz | Pr | 5-CO₂Et | MS4PhO | CH | Me | - | 1 | 0 | H | 1 | Tzd |
| I- 19 | Thox tdz | Pr | 5-CO₂Et | MS4PhO | CH | Me | - | 1 | 0 | H | 1 | Tzd |
| I-20 | Dikb nl | Pr | 5-CO₂Et | MS4PhO | CH | Et | - | 1 | 0 | H | 1 | NCON-C lPhMCx |
| I-21 | Tz | Bu | 5-CO₂Me | MS4PhO | CH | Me | - | 1 | 0 | H | 1 | Tzd |
| I-22 | Tz | iBu | 5-CO₂Et | MS4PhO | CH | Me | - | 3 | 0 | O3M OBn | 2 | Cx |
| I-23 | Tz | cPr | 5-CO₂Et | MS4PhO | CH | Me | - | 1 | 0 | H | 1 | Tzd |
| I-24 | Tz | EtO | 5-CO₂Et | MS4PhO | CH | Me | - | 1 | 0 | H | 1 | Tzd |
| I-25 | Tz | PrO | 5-CO₂Et | MS4PhO | CH | Me | - | 1 | 0 | H | 1 | Tzd |
| I-26 | Tz | cPr O | -CO₂Et | MS4PhO | N | Me | - | 2 | S | OEt | 1 | Tzd |
| I-27 | Tz | MeS | 5-CO₂Et | MS4PhO | CH | Me | - | 1 | 0 | H | 1 | Tzd |
| I-28 | Tz | EtS | 5-CO₂Me | MS4PhO | CH | Me | - | 1 | 0 | H | 1 | Tzd |
| I-29 | PrHy d | PrS | 5-CO₂Et | MS4PhO | CH | Me | - | 1 | 0 | H | 3 | Cx |
| I-30 | Tz | cPr S | 5-CO₂Et | MS4PhO | CH | Me | NPh | 4 | 0 | H | 1 | Tzd |
| I-31 | cPnP hHyd | EtN H | 5-CO₂Et | MS4Ph0 | CH | Me | - | 1 | 0 | 0Ph | 1 | Cx |
| I-32 | CbmP hHyd | PrN Me | 5-CO₂Et | MS4PhO | N | Pr | S | 2 | S | H | 1 | Tzd |
| I-33 | Tz | C₃F₅ | 5-CO₂Et | MS4PhO | CH | Me | - | 1 | 0 | H | 1 | Tzd |
| I-34 | Tz | C₃F₇ | 5-CO₂Et | MS4PhO | CH | Me | - | 1 | 0 | H | 1 | Tzd |
| I-35 | Tz | C₄F₉ | 5-CO₂Et | MS4PhO | CH | Me | - | 1 | 0 | H | 1 | Tzd |
| I-36 | Dioz | CF Ph N | 5-CO₂Et | MS4PhO | CH | Me | - | 1 | 0 | H | 1 | Tzd |
| I-37 | Tz | EO Ph PN M | 5-CO₂Et | MS4PhO | CH | Me | - | 1 | 0 | H | 1 | Tzd |
| I-38 | Tz | Pr | 5-CO₂Me | MS4PhO | CH | Me | - | 1 | 0 | H | 1 | Tzd |
| I-39 | Tz | Pr | 5-Cx | MS4PhO | CH | Me | - | 1 | 0 | H | 1 | Tzd |
| I-40 | CONT z | Pr | 5-CO₂Pr | MS4PhO | CH | Me | - | 1 | 0 | H | 1 | Tzd |
| I-41 | Tz | Pr | 5-CO₂Bu | MMeS4Ph O | H | Me | - | 1 | 0 | H | 1 | Tzd |
| I-42 | Tz | Pr | 5-CO₂iBu | MS4PhO | CH | Me | - | 1 | 0 | H | 1 | Tzd |
| I-43 | SNCO Et₂P h | Pr | 5-CO₂neP n | MS4PhO | CH | Me | - | 1 | 0 | H | 1 | Tzd |
| I-44 | Tz | Pr | 5-CO₂Et | MS4PhO | CH | Me | - | 1 | 0 | H | 1 | MOC1Bn Cx |
| I-45 | Tz | Pr | 5-CONH₂ | MS4PhO | CH | Me | - | 1 | 0 | H | 1 | Tzd |
| I-46 | Tz | Pr | 5-CONHM e | MS4PhO | CH | Me | - | 1 | 0 | H | 1 | Tzd |
| I-47 | Tz | Pr | 5-CONHP r | MS4PhO | CH | Me | O | 2 | 0 | H | 1 | Tzd |
| I-48 | Tz | Pr | 5-CONHi Pr | MS4PhO | CH | Me | - | 1 | 0 | H | 1 | Tzd |
| I-49 | Tz | Pr | 5-CONMe₂ | MS4PhO | N | Me | - | 1 | 0 | H | 1 | Tzd |
| I-50 | SNCO BMPh | Pr | 5-CONMe Bu | MS4PhO | CH | iPr | - | 1 | 0 | O4C FBn | 2 | Cx |
| I-51 | Tz | Pr | 5-H | MS4PhO | CH | Me | - | 1 | 0 | H | 1 | Tzd |
| I-52 | Tz | Pr | 5-Cl | MS4PhO | | Me | - | 1 | 0 | H | 1 | Tzd |
| I-53 | Tz | Pr | 5-Br | MS4PhO | CH | Me | - | 1 | 0 | H | 1 | Tzd |
| I-54 | Tz | Pr | 5-CHO | MS4PhO | CH | Me | - | 1 | 0 | H | 1 | Tzd |
| I-55 | Tz | Pr | 5-COMe | MS4PhO | CH | Me | - | 1 | 0 | H | 1 | Tzd |
| I-56 | Tz | Pr | 5-COEt | MS4PhO | CH | Me | - | 1 | 0 | H | 1 | Tzd |
| I-57 | Tz | Pr | 5-COPr | MS4PhO | CH | Me | - | 1 | 0 | H | 1 | Tzd |
| I-58 | Tz | Pr | 5-COiBu | MS4PhO | CH | Me | - | 1 | 0 | H | 1 | Tzd |
| I-59 | Tz | Pr | 5-COHx | MS4PhO | CH | Me | - | 1 | 0 | H | 1 | MOECx |
| I-60 | Tz | Pr | 5-NH₂ | MS4PhO | CH | Me | - | 1 | 0 | H | 1 | Tzd |
| I-61 | Tz | Pr | 5-NHCHO | MS4PhO | CH | Me | - | 3 | 0 | H | 1 | Tzd |
| I-62 | Tz | Pr | 5-NHCOM e | MS4PhO | CH | Me | - | 1 | 0 | OMe | 1 | MOECx |
| I-63 | Tz | Pr | 5-NHCOP r | MS4PhO | CH | Me | - | 1 | 0 | H | 1 | Tzd |
| I-64 | Tz | Pr | 5-NHC0i Bu | MS4PhO | CH | Me | - | 1 | 0 | H | 1 | Tzd |
| I-65 | Tz | Pr | 5-NHCOs Bu | US4PhO | CH | Me | - | 1 | 0 | H | 1 | Tzd |
| I-66 | Tz | Pr | 5-NHCOi Hx | MS4PhO | CH | Me | - | 1 | 0 | OMM Ph | 2 | NPhMCx |
| I-67 | Tz | Pr | 5-CH₂OH | MS4PhO | CH | Me | - | 1 | 0 | H | 1 | Tzd |
| I-68 | Tz | Pr | 5-C₂H₄OH | MS4PhO | CH | Me | - | 1 | 0 | H | 1 | Tzd |
| I-69 | Tz | Pr | 5-C₅H₁₀O H | MS4PhO | N | Me | - | 1 | 0 | H | 1 | Tzd |
| I-70 | Tz | Pr | 5-CH₂CHO | MS4PhO | CH | Me | - | 1 | 0 | H | 1 | Tzd |
| I-71 | Tz | Pr | 5-CHO | MS4PhO | CH | Me | - | 1 | 0 | H | 1 | Tzd |
| I-72 | Tz | Pr | 5-MMCHO | MS4PhO | CH | Me | - | 1 | 0 | H | 1 | Tzd |
| I-73 | Tz | Pr | 5-(C₆H₁₂C HO | MS4PhO | CH | Me | - | 1 | 0 | H | 1 | Tzd |
| I-74 | Tz | Pr | 5-COMe | MS4PhO | CH | Me | - | 1 | 0 | H | 1 | Tzd |
| I-75 | Tz | Pr | 5-CH₂COE t | MS4PhO | CH | Me | - | 1 | 0 | H | 1 | Tzd |
| I-76 | Tz | Pr | 5-COPr | MS4PhO | CH | Me | - | 1 | 0 | H | 1 | Tzd |
| I-77 | Tz | Pr | 5-CH₂COi Pr | MS4PhO | CH | Me | NM e | 2 | 0 | H | 1 | Tzd |
| I-78 | Tz | Pr | 5-CH₂COi Bu | MS4PhO | N | Me | - | 1 | 0 | H | 1 | Tzd |
| I-79 | Cx | Pr | 5-COsBu | MS4PbO | CH | Me | - | 1 | 0 | H | 1 | Tzd |
| I-80 | Tz | Pr | 5-C₂H₄CO Pn | MS4PhO | CH | Me | - | 1 | 0 | OcH x | 2 | Cx |
| I-81 | Tz | Pr | 5-COPn | MS4PhO | CH | Me | - | 1 | 0 | H | 1 | Tzd |
| I-82 | Tz | Pr | 5-COHx | MS4PhO | N | Bu | 0 | 1 | 0 | H | 1 | Tzd |
| I-83 | Tz | Pr | 5-CODc | MS4PhO | CH | Me | - | 1 | 0 | H | 1 | Tzd |
| I-84 | Tz | Pr | 5-COPn | MS4PhMO | CH | Me | - | 1 | 0 | H | 1 | Tzd |
| I-85 | Tz | Pr | 5-PCOPn | MS4PhO | CH | Me | - | 1 | 0 | H | 1 | Tzd |
| I-86 | Tz | Pr | 5-CO₂Et | MS4BPhO | CH | Me | - | 3 | 0 | OHx | 3 | NMOBzM Cx |
| I-87 | Tz | Pr | 5-CO₂Et | MS4PhO | CH | Me | - | 1 | 0 | H | 1 | NClBnM Cx |
| I-88 | Tz | Pr | 5-CO₂Et | MS4PhO | CH | Me | - | 1 | 0 | H | 1 | MNSMCx |
| I-89 | Tz | Pr | 5-CO₂Et | MS4PhO | CH | Me | - | 1 | 0 | H | 1 | MNSCFC x |
| I-90 | Tz | Pr | 5-CO₃Et | MS4PhO | CH | Me | - | 1 | 0 | H | 1 | MNSMPh Cx |
| I-91 | Tz | Pr | 5-CO₂Et | MS4PhO | CH | Me | - | 1 | 0 | H | 1 | MNSBnC x |
| I-92 | Tz | Pr | 5-CO₂Et | MS4PhO | CH | Me | - | 1 | 0 | H | 1 | MNMSMP hCx |
| I-93 | Tz | Pr | 5-CO₂Et | MS4PhO | CH | Me | - | 1 | 0 | H | 1 | MNClBn SMCx |
| I-94 | Tz | Pr | 5-CO₂Et | MS4PhO | CH | Me | - | 1 | 0 | H | 1 | MNBrPh SCFCx |
| I-95 | Tz | Pr | 5-CO₂Et | MS4PhO | CH | Me | - | 1 | 0 | H | 1 | MNCO₂P hCx |
| I-96 | Tz | Pr | 5-CO₂Et | MS4PhO | CH | Me | - | 1 | 0 | H | 1 | MNCO₂B nCx |
| I-97 | Tz | Pr | 5-CO₂Et | MS4PhO | CH | Me | - | 1 | 0 | H | 1 | MNCO₂B uCx |
| I-98 | Tz | Pr | 5-CO₂Et | MS4PhO | CH | Me | - | 2 | 0 | H | 2 | Tzd |
| I-99 | Tz | Pr | 5-CO₂Me | MS4PhO | CH | Me | - | 2 | 0 | H | 3 | Tzd |
| I-100 | Tz | Pr | 5-CO₂Et | MSM4PhO | CH | Me | - | 2 | 0 | H | 3 | Tzd |
| I-101 | Tz | Pr | 5-CO₂Et | MS3PhO | CH | Me | - | 1 | 0 | H | 1 | Tzd |
| I-102 | Tzd | cP r | 5-CO₂Et | MS3PhS | CH | Me | - | 1 | 0 | H | 1 | Tzd |
| I-103 | Cx | Pr | 5-CO₂Et | MS2PhO | CH | Me | - | 1 | 0 | H | 1 | Tzd |
| I-104 | Tz | Pr | 5-CO₂Et | MO4PhS | CH | Me | - | 1 | 0 | H | 1 | Tzd |
| I-105 | Tz | Pr | 5-CO₂,Et | MO3PhS | CH | Me | 0 | 2 | 0 | H | 1 | Tzd |
| I-106 | Tz | Pr | 5-CO₂Et | MS4PhMO | CH | Me | - | 1 | 0 | H | 1 | NCO₂Ph MCx |
| I-107 | Tz | cP r | 5-CO₃Et | MS3PhMO | N | Me | - | 1 | 0 | H | 1 | Tzd |
| I-108 | Tz | Pr | 5-CO₂Et | MS4PhEO | N | Me | - | 1 | 0 | H | 1 | Tzd |
| I-109 | Tz | C₃ F₇ | 5-CO₂Et | MS3PhEO | CH | Et | - | 1 | 0 | OHx | 2 | Cx |
| I-110 | Tz | Pr | 5-CO₂Et | MN4PhO | CH | Me | - | 1 | 0 | H | 1 | Tzd |
| I-111 | Tz | Pr | 5-CO₂Et | MM4Me₂Ph O | H | Me | - | 1 | 0 | H | 1 | Tzd |
| I-112 | Tz | Pr | 5-CO₂Et | MN4PhS | CH | Me | NM e | 2 | 0 | H | 1 | Tzd |
| I-113 | Tz | Et 0 | 5-CO₂Et | MS4PhN | CH | Me | - | 1 | 0 | H | 1 | Tzd |
| 1-114 | Tz | Pr | 5-CO₂Et | MS4PhMM | CH | Me | - | 1 | 0 | H | 1 | Tzd |
| I-115 | Tz | Pr | 5-CO₂Et | MS4PhCO N | CH | Me | - | 1 | 0 | OBu | 2 | Cx |
| I-116 | Tz | Pr | 5-CO₂Et | MS4PhMC ON | CH | Me | - | 1 | 0 | H | 1 | Tzd |
| I-117 | Tz | Pr | 5-CO₂Et | MS3PhMC ON | CH | Me | - | 1 | 0 | H | 1 | Tzd |
| I-118 | Dikb nl | Pr | 5-CO₂Et | ES4PhO | CH | Me | - | 1 | 0 | H | 1 | Tzd |
| I-119 | Tz | Pr | 5-CONEt₂ | ES4PhMO | N | Me | - | 1 | 0 | H | 1 | Tzd |
| I-120 | Tzd | Pr | 5-CO₂Et | EMeS4Ph O | CH | Me | - | 1 | 0 | H | 1 | Cx |
| I-121 | Tz | Bu | 5-CO₂Et | MMeMS4P hMO | CH | Me | - | 4 | 0 | OE4 MOP h | 1 | Tzd |
| I-122 | Tz | Pr | 5-CO₂Et | O4BnPhO | CH | Me | - | 1 | 0 | H | 1 | Tzd |
| I-123 | Tz | Pr | 5-CO₂Et | O3NpO | CH | Me | - | 1 | 0 | Ph | 1 | Tzd |
| I-124 | Tz | Pr | 5-CO₂Et | MSES4Ph O | CH | Me | - | 1 | 0 | H | 1 | Tzd |
| I-125 | Tz | Pr | 5-CO₂Et | MSEO4Ph S | CH | Me | - | 1 | 0 | H | 1 | Tzd |
| I-126 | Tz | Pr | 5-CO₂Et | MSES4Ph MO | N | Me | - | 1 | 0 | Me | 1 | Tzd |
| I-127 | Tz | Pr | 5-CO₂Et | MSEO | CH | Me | - | 1 | 0 | H | 1 | Tzd |
| I-128 | Tz | Pr | 5-CO₂Et | MOEO | CH | Me | - | 1 | 0 | H | 1 | Tzd |
| I-129 | Tz | Pr | 5-CO₂Et | MSES | CH | Me | - | 1 | 0 | H | 1 | Tzd |
| I-130 | Tz | Pr | 5-CO₂Et | MNES | CH | Me | NMe | 2 | 0 | OiB u | 3 | MOBnCx |
| I-131 | Tz | Pr | 5-CO₂Et | MSEN | CH | Me | - | 1 | 0 | H | 1 | Tzd |
| I-132 | Tz | Pr | 5-CO₂Et | O4MePhS O₂4MePhO | CH | Me | - | 1 | 0 | H | 1 | Tzd |
| I-133 | Tz | Pr | 5-CO₂Et | 06NphMO | CH | Me | - | 1 | 0 | H | 1 | Tzd |
| I-134 | Tz | Pr | 5-CO₂Et | M7BThiM O | H | Me | - | 1 | 0 | H | 1 | Tzd |
| I-135 | Tz | Pr | 5-CO₂Et | Ms5PyN | CH | Me | - | 1 | 0 | H | 1 | Tzd |
| I-136 | Tz | Pr | 5-CO₂Et | MN5PyN | CH | Me | - | 1 | 0 | H | 1 | Tzd |
| I-137 | Tz | Pr | 5-CO₂Et | MSC1MeB ImMO | N | Me | - | 1 | 0 | H | 1 | Tzd |
| I-138 | Tz | Pr | 5-CO₂Et | M50xzEO | CH | Me | 0 | 2 | 0 | H | 1 | Tzd |
| I-139 | Tz | Pr | 5-CO₂Et | MS5ThiM O | H | Me | S | 3 | 0 | H | 1 | Tzd |
| I-140 | Tz | Pr | 5-CO₂Et | M5TtzN | CH | Me | - | 1 | 0 | H | 1 | Tzd |
| I-141 | Tz | Pr | 5-CO₂Et | MS2BImM O | H | Me | - | 1 | 0 | H | 1 | Tzd |
| I-142 | Thox dz | Pr | 5-CO₂Et | MS7PyIm O | H | Me | Ni Pr | 2 | 0 | PH | 2 | Cx |
| I-143 | Tz | Pr | 5-CO₂Et | MS6BOxz O | H | Me | - | 1 | 0 | OPP h | 1 | Tzd |
| I-144 | Cx | Pr | 5-CO₂Et | MS6BOxz MO | CH | Me | - | 1 | 0 | H | 1 | Tzd |
| T-145 | Tz | Pr | 5-CO₂Et | CON3MeN PhS | CH | Me | - | 1 | 0 | H | 1 | Tzd |
| I-146 | Tz | Bu | 5-CONEt₂ | CON3MeN PhS | N | Me | NM e | 2 | 0 | H | 1 | Tzd |
| I-147 | Tz | Pr | 5-CO₂Et | NCOMS | CH | Me | - | 1 | 0 | H | 1 | Tzd |
| I-148 | Tz | Pr | 5-CO₂Et | MSENCOM S | CH | Me | - | 1 | 0 | H | 1 | Tzd |
| I-149 | Tz | Pr | 5-CO₂Et | MO | CH | Me | - | 1 | 0 | H | 1 | Tzd |
| I-150 | Tz | Pr | 5-Cx | MO | CH | Me | - | 1 | 0 | H | 1 | Tzd |
| I-151 | Tz | Pr | 5-CO₂Et. | MSES | CH | Me | - | 1 | 0 | H | 1 | Tzd |
| I-152 | Tz | Pr | 5-CO₂Et | MSEO | CH | Me | - | 1 | 0 | H | 1 | Tzd |
| I-153 | CONT z | Pr | 5-CO₂Et | MSENMe | CH | Me | - | 1 | 0 | H | 1 | Tzd |
| I-154 | Tz | Pr | 5-CO₂Et | MOEO | CH | Me | - | 1 | 0 | H | 1 | Tzd |
| I-155 | Tz | Pr | 5-CO₂Et | MMeSEO | CH | Me | - | 1 | 0 | H | 1 | Tzd |
| I-156 | Tz | Pr | S-CO₃Et | MSESPS | N | Me | - | 1 | 0 | H | 1 | NCO₃Bu MCx |
| I-157 | Tz | Pr | 5-CO₂Et | MS4PhO | CH | Me | - | 2 | 0 | H | 1 | Tzd |
| I-158 | Tz | Pr | 5-CO₃Et | MS4PhO | CH | Me | - | 3 | 0 | H | 1 | Tzd |
| I-159 | Tz | Pr | 5-CO₂cHx | MEtS4Ph O | CH | Me | - | 6 | 0 | H | 1 | Cx |
| I-160 | Tz | Pr | 5-CO₂Et | MS4PhO | CH | Me | NH | 2 | 0 | H | 1 | Tzd |
| I-161 | Tz | Pr | 5-CO₂Et | MS4PhO | CH | Me | NM e | 3 | 0 | OMe | 1 | Tzd |
| I-162 | Tz | Pr | 5-Cx | MS4PhO | CH | Me | NM e | 4 | 0 | H | 1 | MMSBnC x |
| I-163 | Tz | Pr | 5-CO₂Et | MS4PhO | CH | Me | NM e | 6 | 0 | H | 1 | Tzd |
| I-164 | Tz | Pr | 5-CO₂Et | MS4PhO | CH | Me | NE t | 2 | 0 | H | 1 | Tzd |
| I-165 | Tz | Pr | 5-CO₂Bt | MS4Ph0 | CH | Me | Ni Pr | 2 | 0 | H | 1 | Tzd |
| I-166 | Tz | Pr | 5-CO₂cBu | MS4PhO | N | Me | NB u | 4 | 0 | OPh | 2 | Cx |
| I-167 | Tz | Pr | 5-CO₂Et | MS4PhO | CH | Me | NP h | 2 | 0 | H | 1 | Tzd |
| I-168 | Tz | Pr | 5-CO₂Et | MS4PhO | CH | Me | NB n | 2 | 0 | H | 1 | Tzd |
| I-169 | Tz | Pr | 5-CO₂cPn | MS4PhO | CH | Me | O | | 0 | H | 1 | Tzd |
| I-170 | Tz | Pr | 5-CO₂Et | MS4PhO | CH | Me | S | 2 | 0 | O2M OPh | 3 | Cx |
| I-171 | Tz | Pr | 5-CO₂Et | MS4PhO | CH | Me | NA c | 2 | 0 | H | 1 | Tzd |
| I-172 | Tz | Pr | 5-CO₂Et | MS4PhO | N | Me | NB z | 3 | 0 | H | 1 | Tzd |
| I-173 | Tz | Pr | 5-CO₂Et | MS4PhO | CH | Pr | NM s | 3 | 0 | H | 1 | Tzd |
| I-174 | Tz | Pr | 5-CO₂Oc | MS4PhO | CH | Me | N Ts | 4 | 0 | H | 1 | Tzd |
| I-175 | Tz | Pr | 5-CO₂Et | MS4PhO | CH | Me | - | 1 | S | H | 1 | Tzd |
| I-176 | Tz | Bu | 5-CO₂Et | MS4PhO | CH | Me | - | 1 | S | H | 1 | Tzd |
| I-177 | Tz | Pr | 5-CO₂Et | MS4PhO | CH | Me | - | 1 | 0 | H | 2 | Tzd |
| I-178 | Tz | Pr | 5-CO₂Et | MS4MPhO | CH | Me | - | 1 | 0 | 04B rPh | 3 | Cx |
| I-179 | Tz | Pr | 5-CO₂Et | MS4PhO | CH | Me | - | 1 | 0 | H | 6 | Tzd |
| I-180 | Tz | Pr | 5-CO₂Bu | MS4PhO | CH | iPr | 0 | 3 | 0 | H | 3 | Tzd |
| I-181 | Tz | Pr | 5-CO₂Et | MSO₂4PhO | CH | Me | - | 1 | 0 | H | 1 | Tzd |
| I-182 | Tz | Pr | 5-CO₂Et | MS | CH | Me | - | 1 | 0 | H | 1 | Tzd |
| I-183 | Tz | Pr | 5-CO₂Et | MMeS | CH | Me | - | 1 | 0 | H | 1 | Tzd |
| I-184 | Cx | Bu | 5-CONMe₂ | MMeS | CH | Me | - | 1 | 0 | H | 1 | Tzd |
| I-185 | Tz | cP r | 5-CO₂Et | MMe₂S | CH | Me | - | 1 | 0 | H | 1 | Tzd |
| I-186 | Tz | Pr | 5-CO₂Et | MEtS | CH | Me | - | 1 | 0 | H | 1 | Tzd |
| I-187 | Tz | Pr | 5-CO₂Et | ES | CH | Me | - | 2 | 0 | H | 2 | Tzd |
| I-188 | Tz | Pr | 5-CO₂Hp | PS | N | Me | - | 1 | 0 | H | 3 | Tzd |
| I-189 | Tz | cP r | 5-CO₂Et | MS | CH | Me | - | 1 | 0 | H | 1 | Tzd |
| I-190 | Tz | Pr O | -CO₂Et | MS | CH | Me | - | 1 | 0 | H | 4 | Cx |
| I-191 | Tz | Et S | 5-CO₂Et | MS | CH | Me | - | 4 | 0 | H | 1 | Tzd |
| I-192 | Tz | Pr | 5-CO₂Me | MS | N | Me | - | 1 | 0 | H | 1 | Tzd |
| I-193 | Tz | Pr | 5-CO₂iPr | MS | CH | Me | - | 1 | 0 | H | 1 | Tzd |
| I-194 | Tz | Pr | 5-CONEt₂ | MNiPr | CH | Me | - | 1 | 0 | OMe | 1 | Tzd |
| I-195 | Tz | Pr | 5-CO₂Et | MS | CH | Me | NM e | 2 | 0 | H | 1 | Tzd |
| I-196 | Cx | Pr | 5-CO₂Et | MS | CH | Me | - | 6 | 0 | H | 1 | Tzd |
| I-197 | Tz | sB u | 5-CO₂Et | MS | CH | Me | NM e | 2 | 0 | OPh | 2 | Tzd |
| I-198 | Tz | Pr | 5-CO₂Et | MS | CH | Me | NM e | 2 | 0 | 4Me OBn | 0 | Cx |
| I-199 | Tz | Pr | 5-CO₂Et | MS | CH | Me | O | 2 | 0 | H | 1 | Tzd |
| 1-200 | CONT z | Pr | 5-CO₂Et | MS | CH | Me | - | 1 | 0 | H | 1 | Tzd |
| I-201 | NSCF | Pr | 5-CO₂Et | MS | CH | Me | - | 1 | 0 | H | 1 | Tzd |
| I-202 | SNCO Me | Pr | 5-CO₂Et | MS | CH | Me | - | 1 | 0 | H | 1 | Tzd |
| I-203 | SNCO iPr | iB u | 5-CO₂cPr | MS | CH | Me | - | 1 | 0 | OBn | 2 | Tzd |
| I-204 | SNCO Ph | Pr | 5-CO₂Et | MS | CH | Me | - | 1 | 0 | H | 1 | Tzd |
| I-205 | SNCO FCFP h | Pr | 5-CO₂Et | MS | CH | Me | - | 1 | 0 | H | 1 | Tzd |
| I-206 | SNCO MMOP h | cP r | 5-CO₂Et | MS | CH | Me | - | 1 | 0 | H | 1 | Tzd |
| I-207 | Tzd | Et 0 | CO₂Et | MS | CH | Me | O | 2 | 0 | H | 1 | Tzd |
| I-208 | Dioz | Pr | 5-CO₂Et | MS | CH | Me | - | 1 | 0 | H | 1 | Tzd |
| I-209 | Oxtd | Pr | 5-CO₂Et | MS | CH | Me | - | 1 | 0 | H | 1 | Tzd |
| I-210 | Hyd | Pr | 5-CO₂Hx | MS | CH | Me | - | 1 | 0 | H | 1 | Tzd |
| I-211 | Thox dz | Pn | 5-CO₂Et | MNMe | CH | Me | - | 1 | 0 | H | 1 | Tzd |
| I-212 | Tz | Pr | 5-CO₂Et | MS4PhO | CH | Me | - | 1 | 0 | H | 1 | Cx |
| I-213 | Tz | Pr | 5-CO₂Et | MS4PhO | CH | Me | - | 1 | 0 | H | 2 | Cx |
| I-214 | Tz | Pr | 5-CO₂Et | MS4PhO | CH | Me | - | 1 | 0 | OMe | 2 | Cx |
| I-215 | Tz | Pr 0 | 5-CO₂Et | MS4PhO | CH | Me | - | 1 | 0 | OBu | 2 | Cx |
| I-216 | Tz | Bu | 5-CO₂Et | MS4PhO | CH | Me | - | 1 | 0 | OPh | 2 | Cx |
| I-217 | Tz | cP r | 5-CO₂Et | MS4PhO | CH | Me | - | 1 | 0 | OBn | 2 | Cx |
| I-218 | Tz | Pr | 5-CO₂Et | MS4PhO | CH | Me | - | 1 | 0 | OC₂H₄ Ph | 2 | Cx |
| I-219 | Tz | Pr | 5-CO₂Et | MS4PhO | CH | Me | NM e | 2 | 0 | O4C lBn | 2 | Cx |
| I-220 | Tz | Bu | 5-CO₂Et | MS4PhO | CH | Me | - | 1 | 0 | O4M OBn | 3 | Cx |
| I-221 | Cx | Pr | 5-CO₂Et | MS4PhO | CH | Me | - | 1 | 0 | O4M OBn | 3 | Cx |
| I-222 | Cx | Pr | 5-cx | MS4PhO | CH | Me | - | 1 | 0 | SMN p | 2 | Cx |
| I-223 | Tz | Pr | 5-CO₂Et | MS4PhO | CH | Me | - | 1 | 0 | H | 1 | MOBPhC x |
| I-224 | Cx | Pr | 5-Cx | MS4PhO | CH | Me | - | 1 | 0 | H | 1 | MOECx |
| I-225 | Tz | Pr | 5-CO₂Et | MS | CH | Me | - | 1 | 0 | H | 1 | Cx |
| I-226 | Tz | Pr | 5-CO₂Et | MS | N | Me | - | 1 | 0 | H | 2 | Cx |
| I-227 | Tz | Pr | 5-CO₂Et | MS | CH | Me | O | 0 | 0 | M4Bz Ph | 2 | Cx |
| I-228 | Tz | cP r | 5-CO₂Et | MS | CH | Me | S | 2 | 0 | H | 2 | Cx |
| I-229 | Tz | C₂ F₅ | 5-CO₂Et | MS | CH | Me | NM e | 2 | 0 | H | 2 | Cx |
| I-230 | Tz | Pr | 5-CO₂Et | MS | N | Me | O | 0 | 3 | 0Bu | 3 | Cx |
| I-231 | Tz | Pr | 5-CO₂Et | MMeS | CH | Me | - | 1 | 0 | H | 1 | MNBzPh Cx |
| I-232 | Tz | Pr | 5-CO₂Et | MMeS | CH | Me | - | 1 | 0 | H | 2 | NCOBuM Cx |
| I-233 | Cx | Bu | 5-CONMe₂ | MMe₂S | CH | Me | - | 1 | 0 | Hx | 2 | Cx |
| I-234 | Tz | Pr | 5-CO₂Et | MMe₂S | CH | Me | - | 1 | 0 | OBn | 2 | Cx |
| I-235 | Tz | Pr | 5-CO₂Et | MEtS | CH | Me | 0 | 2 | S | OC₆H₁₂ Ph | 2 | Cx |
| I-236 | Tz | Pr | 5-CO₂Et | ES | CH | Me | - | 1 | 0 | OBn | 2 | Cx |
| I-237 | Tz | Pr | 5-CO₂Hp | PS | CH | Me | - | 1 | 0 | OC₂H₄ Ph | 2 | Cx |
| I-238 | Tz | Pr | 5-CO₂Et | NMeCOMS | CH | Me | - | 1 | 0 | H | 1 | Tzd |
| I-239 | Thox tdz | Pr | 5-CO₂Et | NMeCOMS | CH | Me | NM e | 2 | 0 | H | 1 | MNSMPh Cx* |
| I-240 | Tz | Pr | 4-CO₂Et | MS4PhO | CH | Me | - | 1 | 0 | H | 1 | Tzd |
| I-241 | Cx | Pr | 4-CO₂Me | MS4PhO | CH | Me | - | 1 | 0 | H | 1 | Tzd |
| I-242 | Tz | Pr | 4-CO₂Et | MS4PhO | CH | Me | - | 1 | 0 | H | 1 | MMSBnC x |
| I-243 | Cx | Pr | 4-CO₂Et | MS4PhO | CH | Me | - | 1 | 0 | H | 1 | MOiPrC x |
| I-244 | Tz | Pr | 4-CO₂Et | MS4PhO | CH | Me | - | 1 | 0 | O3Cl Ph | 2 | Cx |
| I-245 | Cx | Pr | 4-CO₂Et | MS4PhO | CH | Me | - | 1 | 0 | O4Cl Bn | 2 | Cx |
| I-246 | Tz | Pr | 4-CO₂Bt | MS4PhO | CH | Me | - | 1 | 0 | H | 1 | NBzMCx |
| I-247 | Cx | Pr | 4-CO₂Et | MS4PhO | CH | Me | - | 1 | 0 | H | 1 | NCO₂EP hMCx |
| I-248 | CONT z | Pr | 4-CO₂Et | MS4PhO | CH | Me | - | 1 | 0 | H | 1 | Tzd |
| I-249 | Dioz | Pr | 4-CO₂Et | MS4PhO | CH | Me | - | 1 | 0 | Me | 1 | MNSBnC x |
| I-250 | Hyd | Pr | 4-CO₂Et | MS4PhO | N | Me | - | 1 | 0 | H | 1 | Tzd |
| I-251 | Oxdz | Pr | 4-CO₂Et | MS4PhO | CH | Me | - | 1 | 0 | H | 1 | Tzd |
| I-252 | Thox dz | Pr | 4-CO₂Et | MS4PHO | CH | Me | - | 1 | 0 | H | 1 | Tzd |
| I-253 | Dikb nl | Pr | 4-CO₂Et | MS4PhO | CH | Me | - | 1 | 0 | H | 1 | Tzd |
| I-254 | Tz | Et | 4-CO₂Et | MS4PhO | CH | Me | - | 1 | 0 | H | 1 | MNCO₂B nCx |
| I-255 | Tz | Bu | 4-CO₂Et | MS4PhO | CH | Me | - | 1 | 0 | H | 1 | Tzd |
| I-256 | Tz | cP r | 4-CO₂Et | MS4PhO | CH | Me | - | 1 | 0 | H | 1 | Tzd |
| I-257 | Tz | Et O | -CO₂Et | MS4PhO | CH | Me | - | 1 | 0 | H | 1 | Tzd |
| I-258 | Tz | Pr O | -CO₂Et | MS4PhO | CH | Me | - | 1 | 0 | H | 1 | Tzd |
| I-259 | Tz | cP rO | 4-CO₂Et | MS4PhO | CH | Me | - | 1 | 0 | H | 1 | Tzd |
| I-260 | Tz | Et S | 4-CO₂Et | MS4PhO | CH | Me | - | 1 | 0 | H | 1 | MNCOBu BnCx |
| I-261 | PrH yd | Pr S | 4-CO₃Et | MS4PhO | CH | Me | - | 1 | 0 | H | 1 | Tzd |
| I-262 | Tz | cP rS | 4-CO₂Et | MS4PhO | CH | Me | - | 1 | 0 | H | 1 | Tzd |
| I-263 | Cx | Bu | 4-CONMes | MMeS | CH | Me | - | 1 | 0 | H | 1 | Tzd |
| I-264 | Tz | Pr | 4-CO₂Et | MEtS | CH | Me | - | 1 | 0 | H | 1 | Tzd |
| I-265 | Tz | Pr | 4-CO₂Hp | PS | CH | Me | - | 1 | 0 | H | 3 | Tzd |
| I-266 | Tz | Pr O | -CO₂Et | MS | CH | Me | - | 1 | 0 | H | 4 | Tzd |
| I-267 | Tz | Et S | 4-CO₂Et | MS | N | Me | - | 4 | 0 | H | 1 | Tzd |
| I-268 | Tz | Pr | 4-CO₂Me | MS | CH | Me | - | 1 | 0 | H | 1 | Tzd |
| I-269 | Tz | Pr | 4-CONEt₂ | MNiP | CH | Me | - | 1 | 0 | OMe | 1 | Tzd |
| I-270 | Tz | Pr | 4-CO₂Et | MS | CH | Me | NM e | 2 | 0 | H | 1 | Tzd |
| I-271 | Cx | Pr | 4-CO₂Et | MS | CH | Me | - | 6 | 0 | H | 1 | Tzd |
| I-272 | Tz | Pr | 4-CO₂Et | MS | CH | Me | NM e | 2 | 0 | 4MeO Bn | 3 | Tzd |
| I-273 | Tz | Pr | 4-CO₂Et | MS | CH | Me | O | | 0 | H | 1 | Tzd |
| I-274 | CON Tz | Pr | 4-CO₂Et | MS | CH | Me | - | 1 | 0 | H | 1 | Tzd |
| I-275 | NSC F | Pr | 4-CO₂Et | MS | CH | Me | NM e | 2 | 0 | H | 1 | Tzd |
| I-276 | SNC OPr | Pr | 4-CO₂Et | MS | CH | Me | - | 1 | 0 | H | 1 | Tzd |
| I-277 | SNCO iPr | iB u | 4-CO₂cPr | MS | CH | Me | - | 1 | 0 | H | 1 | Tzd |
| I-278 | SNC OPh | Pr | 4-CO₂Et | MS | CH | Et | - | 1 | 0 | H | 1 | Tzd |
| I-279 | SNCO MMOP h | Pr | 4-CO₂Et | MS | N | Me | - | 1 | 0 | H | 1 | Tzd |
| I-280 | Tz | Pr | 4-CONEt₂ | MS4PhO | CH | Me | O | | 0 | H | 1 | Tzd |
| I-281 | Tz | Pr | 4-CONEt Bu | MS4PhO | CH | Me | - | 1 | 0 | H | 1 | NCONBu MCx |
| I-282 | Tz | Pr | 4-Cl | MS4PhO | N | Me | NM e | 2 | 0 | H | 1 | MOECx |
| I-283 | Tz | Bu | 4-Cl | MS | CH | Me | - | 1 | 0 | H | 1 | Tzd |
| I-284 | Tz | Pr | 4-Br | MS4PhO | CH | Me | - | 1 | 0 | H | 1 | Tzd |
| I-285 | Tz | Bu | 4-Cl | MSO | CH | Me | - | 1 | 0 | H | 1 | Tzd |
| I-286 | Tz | Bu | 4-Cl | MSO₂ | CH | Me | - | 1 | 0 | H | 1 | Tzd |
| I-287 | Tz | Pr | 4-CO₂Et | CON3MeN PhS | CH | Me | - | 1 | 0 | H | 1 | Tzd |
| I-288 | Tz | Bu | 4-CONEt₂ | CON3MeN PhS | CH | Me | NMe | 2 | 0 | H | 1 | Tzd |
| I-289 | Tz | Bu | 4-Cl | CONMeEO | CH | Me | NM e | 2 | 0 | H | 1 | MSMClC FPhCx |
| I-290 | Tz | Bu | 4-COMe | COE4PhO | CH | Me | NM e | 2 | 0 | H | 1 | Tzd |
| I-291 | Tz | Bu | 4-CMe₂OH | COEO | CH | Me | - | 1 | 0 | H | 1 | Tzd |
| I-292 | Tz | Pr | 4-Cl | CON3PhO | N | Me | - | 1 | 0 | H | 1 | Tzd |
| I-293 | Tz | Pr | 5-CO₂Et | MSM4PhO | CH | Me | - | 1 | 0 | H | 1 | Tzd |
| I-294 | Tz | Bu | 4-Cl | CONMeM4 PhEO | CH | Me | NM e | 2 | 0 | 04C FPh | 2 | Cx |
| I-295 | Tz | Pr | 5-CO₂Et | MSM4PhO | CH | Me | - | 1 | 0 | H | 1 | MNECOM MOPhCx |
| I-296 | Tz | Pr | 5-CO₂Et | MSM4PhO | CH | Me | - | 1 | 0 | H | 1 | NCO₂MO PhMCx |
| I-297 | Tz | Pr | 4-CO₂Et | MS4PhO | CH | Me | - | 1 | 0 | O4C 1Ph | 2 | Cx |
| I-298 | Tz | Pr | 4-CO₂Et | MS4PhO | CH | Me | - | 1 | 0 | O3C FPh | 2 | Cx |
| I-299 | Tz | Pr | 4-CO₃Me | MMeS4PhO | CH | Me | - | 1 | 0 | H | 1 | Tzd |
| I-300 | Tz | Pr | 5-CO₂Et | MS4PhO | CH | Me | NM e | 2 | 0 | H | 1 | MNBzPh Cx |
| I-301 | Tz | Pr | 5-CO₂Et | MS4PhO | CH | Me | - | 1 | 0 | H | 1 | MNBzPh Cx |
| I-302 | Tz | Pr | 5-CO₂Et | MS3PyS | CH | Me | - | 1 | 0 | H | 1 | Tzd |
| I-303 | Tz | Pr | 5-CO₂Et | MSO4PhO | CH | Me | - | 1 | 0 | H | 1 | Tzd |
| I-304 | Tz | Pr | 5-CO₂Et | MS | CH | Me | - | 1 | 0 | H | 1 | MO4ClB nCx |
| I-305 | Tz | Pr | 5-CO₂Et | MS | CH | Me | - | 1 | 0 | H | 1 | NCO₂Ph MCx |
| I-306 | Tz | Pr | 5-CO₂Et | SM | CH | Me | - | 1 | 0 | H | 1 | Tzd |
| I-307 | Tz | Pr | 5-CO₃Et | SM4PhO | CH | Me | - | 1 | 0 | H | 1 | Tzd |
| I-308 | Tz | Pr | 5-CO₃Et | SM3PhO | CH | Me | - | 1 | 0 | H | 1 | Tzd |
| I-309 | Tz | Pr | 5-CO₂Et | MSM4PhO | CH | Me | - | 1 | 0 | H | 1 | Tzd |
| I-310 | Tz | Pr | 5-CO₂Et | MSM4PhO | N | Me | - | 1 | 0 | H | 1 | Tzd |
| I-311 | Tz | Pr | 5-CO₂Et | 5MSM2Py O | CH | Me | - | 1 | 0 | H | 1 | Tzd |
| I-312 | Tz | Pr | 5-CO₂Et | 6MSM3Py O | CH | Me | - | 1 | 0 | H | 1 | Tzd |
| I-313 | Tz | Pr | 5-CO₂Et | 5MSM2Py S | CH | Me | - | 1 | 0 | H | 1 | Tzd |
| I-314 | Tz | Pr | 5-CO₂Et | 6MSM3Py S | CH | Me | - | 1 | 0 | H | 1 | Tzd |
| I-315 | Tz | Pr | 5-CO₂Et | MSM4PhO | CH | Me | - | 1 | 0 | H | 1 | NCO₂MO PhMCx |
| 1-316 | Tz | Pr | 5-CO₂Et | MSM4PhO | CH | Me | - | 1 | 0 | H | 1 | MO24ClB nCx |
| I-317 | Tz | Pr | 5-CO₂Et | 5MSE2Py O | CH | Me | - | 1 | 0 | H | 1 | Tzd |
| I-318 | Tz | Pr | 5-CO₂Et | MSM4PhS | CH | Me | - | 1 | 0 | H | 1 | Tzd |
| I-319 | Tz | Pr | 5-CO₂Et | MS5Py20 | CH | Me | - | 1 | 0 | H | 1 | Tzd |
| I-320 | Tz | Bu | 6-CO₂Et | MS | CH | Me | - | 1 | 0 | H | 1 | Tzd |
| I-321 | Tz | Pr | 5-CO₂Et | MMeSM4P hO | CH | Me | - | 1 | 0 | H | 1 | Tzd |
| I-322 | Tz | Pr | 5-CO₂Et | MMeSM5P y20 | CH | Me | - | 1 | 0 | H | 1 | Tzd |
| I-323 | Tz | Pr | 5-CO₂Et | MMeS | CH | Me | - | 1 | 0 | H | 1 | Tzd |
| I-324 | Tz | C₃F 7 | 5-CO₂Et | MS4PhO | CH | Me | - | 1 | 0 | H | 1 | Tzd |
| I-325 | Tz | C₃F 7 | 5-CO₂Et | MS | CH | Me | - | 1 | 0 | H | 1 | Tzd |
| I-326 | Tz | Pr | 5-CO₂Et | SM | CH | Me | - | 1 | 0 | H | 1 | Tzd |
| I-327 | Tz | Pr | 5-CO₂Et | SMMe | CH | Me | - | 1 | 0 | H | 1 | Tzd |
| I-328 | Tz | Pr | 5-CO₂Et | SE | CH | Me | - | 1 | 0 | H | 1 | Tzd |
| I-329 | Tz | Pr | 5-CO₂Et | SM4PhO | CH | Me | - | 1 | 0 | H | 1 | Tzd |
| I-330 | Tz | Pr | 5-CO₂Et | SE4PhS | CH | Me | - | 1 | 0 | H | 1 | Tzd |
| I-331 | Tz | Pr | 6-CO₂Et | SM5Py20 | CH | Me | - | 1 | 0 | H | 1 | Tz |
| I-332 | Tz | Pr | 5-CO₂Et | MS | CH | Me | - | 1 | 0 | H | 1 | NCO₃MO PhMCx |
| I-333 | Tz | Pr | 5-CO₂Et | MS | CH | Me | - | 1 | 0 | H | 1 | NC03CF PhMCx |
| I-334 | Tz | Pr | 5-CO₂Et | MS | CH | Me | - | 1 | 0 | H | 1 | MOMMeO PhCx |
| I-335 | Tz | Pr | 5-CO₂Et | MS | CH | Me | - | 1 | 0 | H | 1 | MOMClP hCx |
| I-336 | Tz | Pr | 5-CO₂Et | MS | CH | Me | - | 1 | 0 | H | 1 | MOM3Py Cx |
| I-337 | Tz | Pr | 5-CO₂Et | MS | CH | Me | - | 1 | 0 | H | 1 | MOEClP hCx |
| I-338 | Tz | Pr | 5-CO₂Et | MS | CH | Me | - | 1 | 0 | H | 1 | MOtBuP hCx |
| I-339 | Tz | Pr | 5-CO₂Et | SM4PhO | CH | Me | - | 1 | 0 | H | 1 | MOMClP hCx |
| I-340 | Tz | Pr | 5-CO₂Et | SE4PhS | CH | Me | - | 1 | 0 | H | 1 | MOMClP hCx |
| I-341 | Tz | Pr | 5-CO₂Et | SM5Py2O | CH | Me | - | 1 | 0 | H | 1 | MOMClP hCx |
| I-342 | Tz | Pr | 5-CO₂Et | MMeSEO | CH | Me | - | 1 | 0 | H | 1 | MOMClP hCx |
| I-343 | Tz | Pr | 5-CO₂Et | MS | N | Me | - | 1 | 0 | H | 1 | Tzd |
| I-344 | Tz | Pr | 5-CO₂Et | MMeS4Ph O | CH | Me | - | 1 | 0 | H | 1 | Tzd |
| I-345 | Tz | Pr | 5-CO₂Et | MMeS | CH | Me | - | 1 | 0 | H | 1 | MOClBn Cx |
| I-346 | Tz | Pr | 5-CO₂Et | MMeS4Ph O | CH | Me | - | 1 | 0 | H | 1 | MOClBn Cx |
| I-347 | Tz | Pr | 5-CO₂Et | MMeSM4P hO | CH | Me | - | 1 | 0 | H | 1 | MOClBn Cx |
| I-348 | Tz | Pr | 5-CO₂Et | MMe₂S4P hO | CH | Me | - | 1 | 0 | H | 1 | Tzd |
| I-349 | Tz | Pr | 5-CO₂Et | MMeSM5P y2O | CH | Me | - | 1 | 0 | H | 1 | MOClBn Cx |

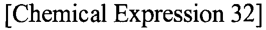

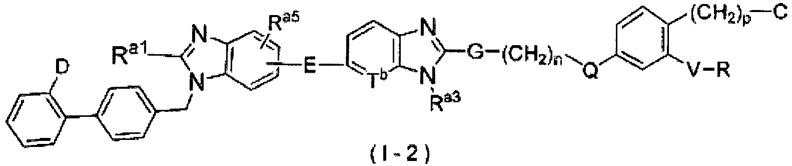

**TABLE 2**

| No. | D | R^{a1} | R^{a5} | E | T^{b} | R^{a3} | G | n | Q | -V-R | p | C |
|---|---|---|---|---|---|---|---|---|---|---|---|---|
| II-1 | Tz | Pr | 4-Me | MO | CH | Me | - | 1 | 0 | H | 1 | Tzd |
| II-2 | Tz | Bu | 4-Cl | EO | N | Me | NM e | 2 | 0 | H | 1 | Tzd |
| II-3 | Tz | EtS | 4-Me | COEO | CH | Me | - | 1 | 0 | H | 1 | MOECx |
| II-4 | Cx | Per | 4-Me | MO | CH | Me | - | 1 | 0 | H | 1 | NCOBnMCx |
| II-5 | Tz | Pr | 4-Me | MO3PhS | CH | Me | - | 1 | 0 | H | 1 | NCONClBn MCx |
| II-6 | Cx | Pr | 4-Me | MO | CH | Me | - | 1 | 0 | H | 1 | Tzd |
| II-7 | Tz | Pr | 4-Me | 6MeBImNM eEO | CH | Me | - | 1 | 0 | H | 1 | Tzd |
| II-8 | Tz | Pr | 4-Me | 6MeBImSE O | CH | Me | - | 1 | 0 | H | 1 | Tzd |
| II-9 | Tz | Pr | 4-Me | 6MeBImM eEO | CH | Me | NP h | 3 | 0 | H | 1 | Tzd |
| II-10 | Tz | Pr | 4-Me | 6MeBImNM eEO | CH | Me | - | 1 | 0 | O4C1 Bn | 2 | Cx |
| II-11 | Tz | Pr | 4-Me | 6MeBImNM eEO | CH | Me | - | 1 | 0 | O4Cl PhBn | 3 | Cx |
| II-12 | Tz | Pr | 4-Me | 6MeBImSE NMe | CH | Me | - | 1 | 0 | H | 1 | MNMCOMOP hCx |
| II-13 | Tz | EtO | 4-Cl | 6MeBImOE 4PhNMe | N | Me | - | 2 | S | OMe | 1 | MNCONFPh Cx |
| II-14 | Tz | Pr | 4-Me | 2TtzEO | CH | Me | NP h | 3 | 0 | H | 2 | MMOBnCx |
| II-15 | Tz | Pr | 4-Me | 2TtzEO | CH | Me | - | 1 | 0 | H | 1 | Tzd |
| II-16 | Tz | cPr | 7-Cx | OE3PhNMe | CH | Pr | - | 3 | 0 | O4MO Bn | 1 | NCONMBnM Cx |
| II-17 | Tz | cPr | 4-Me | OE3PhNMe | CH | Me | - | 3 | 0 | O4MO Bn | 1 | NCO₂MOPh MCx |
| II-18 | NSC F | cPr | 4-Me | NMeEOEO | CH | Me | - | 1 | 0 | O4MO Bn | 1 | Cx |
| II-19 | Cx | Pr | 4-H | NMeEO | CH | Me | - | 1 | 0 | H | 1 | Tzd |
| II-20 | Cx | cPr | 4-Cl | NBuEO | CH | Me | 0 | 2 | 0 | H | 1 | Tzd |
| II-21 | Cx | cPr | 4-Cl | NBuEO | CH | Me | S | 6 | 0 | H | 1 | Tzd |
| II-22 | Cx | Pr | 4-Me | OEO4PhMO | CH | Me | 0 | 2 | 0 | OPh | 1 | Tzd |
| II-23 | Tz | EtO | 4-Et | OEO6NpNM e | N | Me | - | 2 | S | OMe | 1 | MNCONPhC x* |
| II-24 | Oxt d | Pr | 4-Me | OEO4PhMO | CH | Me | S | 2 | S | OBn | 2 | Tzd |
| II-25 | Cx | Pr | 4-H | NMeEO | CH | Me | - | 1 | 0 | H | 1 | Tzd |
| II-26 | Dik bnl | Pr | 4-H | S3PhCONE O | H | Me | - | 1 | 0 | H | 1 | Tzd |
| II-27 | Cx | Pr | 4-Me | MS4PhO | CH | Me | - | 1 | 0 | H | 1 | Tzd |
| II-28 | Cx | Pr | 4-Me | ES4PhO | N | Me | - | 1 | 0 | H | 1 | Tzd |
| II-29 | Cx | cPr | 4-Me | MS4PhO | CH | Me | - | 1 | 0 | O4MO Bn | 2 | Cx |
| II-30 | Tz | Pr | 4-Me | MS4PhO | CH | Me | NM e | 2 | 0 | O4MO Bn | 2 | Cx |
| II-31 | CO₂M e | Pr | 4-Me | MNMe | N | Me | - | 1 | 0 | H | 1 | Tzd |
| II-32 | CON Tz | Pr | 4-Me | PNMe | CH | Me | - | 1 | 0 | H | 1 | Tzd |
| II-33 | CO₂M e | Pr | 4-Me | MNMe | CH | Me | - | 1 | 0 | O4tB Ph | 2 | Tzd |
| II-34 | Dio z | Pr | 4-Me | MNMe | N | Me | - | 1 | 0 | H | 1 | Tzd |
| II-35 | Cx | Pr | 4-Me | MNMe | N | Me | - | 1 | 0 | H | 1 | Tzd |
| II-36 | Tz | Pr | 4-Me | MNMe | N | Me | - | 1 | 0 | H | 1 | Tzd |
| II-37 | Cx | EtO | 4-Me | MNMe | N | Me | - | 1 | 0 | H | 1 | Tzd |
| II-38 | Cx | Pr | 4-Me | MNMe | N | Me | - | 1 | 0 | H | 1 | MPOBnOCx |
| II-39 | Tz | Pr | 4-Me | CONEN | N | Me | - | 1 | 0 | H | 1 | Tzd |
| II-40 | Tz | Pr | 4-Me | CONMeENM e | N | Me | - | 1 | 0 | H | 1 | Tzd |
| II-41 | Tz | cPr | 4-Me | CONMeENM e | N | Me | NM e | 2 | 0 | OE3C IPh | 2 | Cx |
| II-42 | Tz | Pr | 4-Me | MNMe | N | Me | - | 1 | 0 | OcHx | 1 | Tzd |
| II-43 | Cx | Pr | 4-Me | ENMe | N | Me | - | 1 | 0 | O2F4 ClBn | 1 | Cx |
| II-44 | Cx | Pr | 4-Me | COMCONMe | CH | Me | - | 1 | 0 | O4Br Bn | 2 | Cx |
| II-45 | Tz | Pr | 4-Me | 6MeBImS | CH | Me | - | 1 | 0 | H | 1 | Tzd |
| II-46 | Tz | Pr | 4-Me | 6MeBImSE O | H | Me | - | 1 | S | H | 1 | Tzd |
| II-47 | Cx | Pr | 4-Me | 6MeBInNM ePO | CH | Me | NM e | 2 | 0 | O4Br Bn | 2 | Cx |
| II-48 | Tz | Pr | 4-Me | CON4PhO | CH | Me | - | 1 | 0 | H | 1 | Tzd |
| II-49 | Cx | EtO | 4-H | OES | N | Me | 0 | 2 | S | 34M OPh | 1 | MNCONBnC x |
| II-50 | Cx | cPr | 4-Me | COMS | N | Me | 0 | 2 | S | OHx | 1 | MCO₂BnCx |
| II-51 | Tzd | Pr | 4-Me | CON4PhO | CH | Me | - | 1 | | H | 1 | Tzd |
| II-52 | Tz | EtO | 4-H | CON4PhO | CH | Me | - | 1 | 0 | H | 1 | Tzd |
| II-53 | Tz | EtO | 4-H | CONMe4Ph O | CH | Me | NE t | 2 | 0 | H | 1 | Tzd |
| II-54 | Cx | Pr | 4-Me | 6MeBImNM eEO | CH | Me | - | 1 | 0 | H | 1 | Tzd |
| II-55 | Oxd z | Pr | 4-MeO | 6MeBImNM eEO | CH | Me | - | 1 | 0 | H | 1 | Tzd |
| II-56 | Cx | Pr | 4-Me | 6MeBImNM eEO | CH | Me | NM e | 3 | 0 | H | 1 | Tzd |
| II-57 | Cx | Pr | 4-Me | 6MeBImNB uEO | CH | Me | - | 1 | 0 | H | 1 | Tzd |
| II-58 | Cx | Pr | 4-Me | 6MeBImNM eEO | CH | Me | - | 1 | 0 | H | 1 | NCO₂BrPh MCx |
| II-59 | Cx | Pr | 4-Me | 6MeBImNM eEO | CH | Me | - | 1 | 0 | OPr | 1 | Tzd |
| II-60 | Tz | Pr | 4-Me | CONM4PhO | CH | Me | - | 1 | 0 | H | 1 | Tzd |
| II-61 | Tz | Pr | 4-Me | CONEO4Ph O | H | Me | - | 1 | 0 | H | 1 | Tzd |
| II-62 | Tz | Pr | 4-Me | CONM4PhO | CH | Me | - | 1 | 0 | H | 1 | Dioz |
| II-63 | Tzd | Pr | 4-Me | CONE4PhO | CH | Me | - | 1 | 0 | H | 1 | Odz |
| II-64 | Tz | cPr | 4-Me | CONE4PhN Me | N | Me | - | 1 | 0 | H | 1 | Tzd |
| II-65 | Tz | Pr | 4-Me | CONM5PyN Me | N | Me | NM e | 2 | 0 | H | 1 | Tzd |
| II-66 | Tz | Pr | 4-Me | CONMeENM e5PyMN | N | Me | - | 1 | 0 | H | 1 | Tzd |
| II-67 | Tz | Pr | 4-Me | CONMeENM e5PyMN | N | Me | - | 1 | 0 | O4MO Bn | 2 | Cx |
| II-68 | Tz | Pr | 4-Me | CONMeENM e5PyMN | N | Me | - | 1 | 0 | H | 1 | NCONCFPh MCx |
| II-69 | Tz | Pr | 4-Me | CONMeENM e5PyMN | N | Me | - | 1 | 0 | H | 1 | MSBnCx |
| II-70 | Tz | Pr | 4-Me | CONMeENM e5PyMN | N | Me | - | 1 | 0 | H | 1 | MOBFBnCx |
| II-71 | Cx | Pr | 4-Me | CONMeENM e5PyMN | CH | Me | - | 1 | 0 | OiPr | 1 | MOEFBnCx |
| II-72 | Tz | cPr | 4-Me | EO | CH | Me | NM e | 2 | 0 | H | 1 | Tzd |
| II-73 | Tz | Pr | 4-Me | NMeEO | CH | Me | NM e | 2 | 0 | H | 1 | MEOCx |

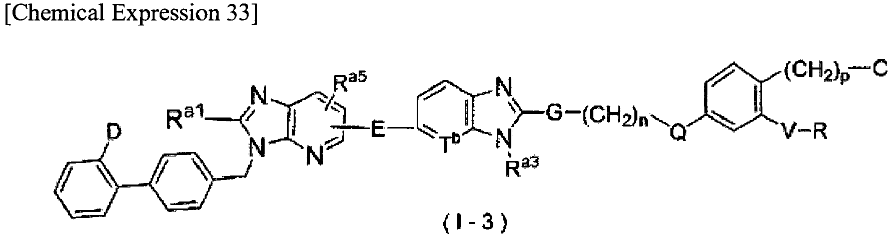

**TABLE 3**

| No. | D | R^{a1} | R^{b5} | E | T^{b} | R^{a3} | G | n | Q | -V-R | p | C |
|---|---|---|---|---|---|---|---|---|---|---|---|---|
| II-74 | NSCF | cPr | 4-Me | NMeEOEO | CH | Me | - | 1 | 0 | O4MO Bn | 1 | Cx |
| II-75 | Cx | EtO | 4-H | OES | N | Me | 0 | 2 | S | O34M OPh | 1 | MNCONBn Cx |
| II-76 | Cx | cPr | 4-Me | COMS | N | Me | 0 | 2 | S | OHx | 1 | NCO₂BnCx |

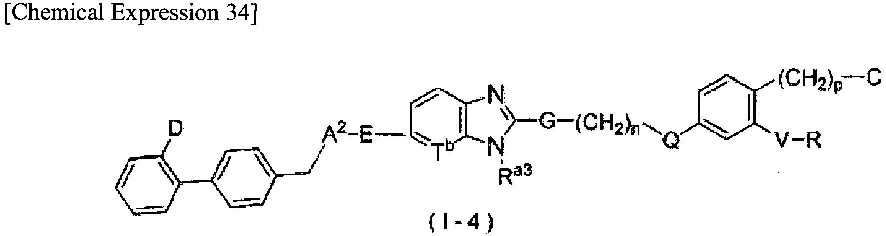

**TABLE 4**

| No. | D | A² | E | T^{b} | R^{a3} | G | n | Q | -V-R | p | C |
|---|---|---|---|---|---|---|---|---|---|---|---|
| III-1 | Tz | PM4N2O | EO | CH | Me | - | 1 | 0 | H | 1 | Tzd |
| III-2 | Cx | EOM4N2O | MCON | CH | Me | NM e | 2 | 0 | H | 1 | Tzd |
| III-3 | Tz | BE4N2O | EO4PhS | N | Bn | - | 2 | 0 | O4CF Ph | 2 | Cx |
| III-4 | Tz | cPP4N2O* | ES | CH | Me | - | 1 | 0 | H | 1 | Tzd |
| III-5 | Tz | P4N2O* | ESMCONMe | CH | Me | - | 1 | 0 | H | 1 | Tzd |
| III-6 | Tz | P4NO | ESEMBu | CH | Me | - | 1 | 0 | H | 1 | Tzd |
| III-7 | Tz | P2NOS | MNEOES | CH | Me | - | 1 | 0 | H | 1 | Tzd |
| III-8 | Tz | PNOM2NS | PNES3PhP hO | N | Me | - | 2 | S | OBn | 1 | Tzd |
| III-9 | Cx | PNO2NS | M4PhO | CH | Et | S | 4 | 0 | 04CF Bn | 2 | Cx |
| III-10 | Tzd | P2N3OS | ENEOESO₂ | CH | Me | - | 1 | 0 | H | 1 | Tzd |
| III-11 | Tz | P3NO | MS4PhO | CH | Me | - | 1 | 0 | H | 1 | Tzd |
| III-12 | Tz | PM3NO | MS | CH | Me | - | 1 | 0 | H | 1 | Tzd |
| III-13 | Tz | PM3NO* | ES4cPPhO | CH | Me | - | 1 | 0 | H | 1 | Tzd |
| III-14 | Tz | P2NO | 6MeBImNM eEO | CH | Me | - | 3 | 0 | H | 1 | Tzd |
| III-15 | Tz | P2NO | MS4cPPhO | CH | Me | - | 1 | 0 | H | 1 | Tzd |
| III-16 | Tz | PM2NO | MS4BzPhO | N | Me | - | 1 | 0 | H | 1 | Tzd |
| III-17 | Cx | PCF2NO | MO | CH | Me | NM e | 2 | 0 | O3Cl Bn | 2 | Cx |
| III-18 | Tz | PCl2NO | 6MeBImNM eEO | CH | Me | - | 1 | 0 | H | 1 | Tzd |
| III-19 | Tz | POM2NO | MS4MeOPh O | H | Me | - | 1 | 0 | H | 1 | Tzd |
| III-20 | Tz | P2NO | OES5PyO | N | Me | - | 1 | 0 | H | 3 | Tzd |
| III-21 | Tz | cPE2N** | MS | CH | Me | - | 1 | 0 | H | 1 | Tzd |
| III-22 | Tz | BE2N** | MS4BuOPh O | H | Me | - | 1 | 0 | H | 1 | Tzd |
| III-23 | Tz | PcH2NO* | ES6PyN | CH | Me | 0 | 2 | 0 | PrO | 1 | MNMCONP HCx |
| III-24 | Cx | POM2NO* | ES3PhNMe | CH | Me | - | 1 | 0 | H | 1 | Tzd |
| III-25 | Tz | PiB2NO* | MS4BuOPh O | | iB u | S | 2 | 0 | H | 1 | Tzd |
| III-26 | Tz | cPMNO | MS4BuOPh O | H | Me | - | 1 | 0 | H | 1 | Tzd |
| III-27 | Tz | PNO | ES4PhNMe | N | Me | - | 4 | 0 | H | 1 | Tzd |
| III-28 | Tz | PNO* | 5Ttz | CH | Me | - | 1 | 0 | H | 1 | Tzd |
| III-29 | Tz | P2NO** | ES3PhNMe | N | Me | - | 1 | 0 | H | 1 | Tzd |
| III-30 | Tz | NCOBu | ES | CH | Me | - | 1 | 0 | H | 1 | Tzd |
| III-31 | Tz | NCOC₄F₉ | EMeS | CH | Me | - | 2 | 0 | H | 1 | MNMCOHC x |
| III-32 | Tz | NCOcPr | EMeS | CH | Me | - | 2 | 0 | H | 2 | NCONPBM Cx |
| III-33 | Cx | NCOBu | ES3PhNMe | N | Me | - | 1 | 0 | OMNp | 2 | Cx |
| III-34 | Tz | NCOC₃F₇ | EMeS | CH | Me | - | 1 | 0 | H | 1 | Tzd |
| III-35 | Tz | P4N2O* | EO | CH | Me | - | 1 | 0 | H | 1 | Tzd |
| III-36 | Tz | P4N2O* | EO | N | Me | - | 1 | 0 | H | 1 | Tzd |
| III-37 | Tz | P4N2O* | ES | CH | Me | - | 1 | 0 | H | 1 | Tzd |
| III-38 | Tz | P4N2O* | EO | CH | Me | - | 1 | 0 | H | 1 | MOMClPh Cx |
| III-39 | Tz | P4N2O* | M4PhO | CH | Me | - | 1 | 0 | H | 1 | Tzd |
| III-40 | Tz | P4N2O* | M5Py20 | CH | Me | - | 1 | 0 | H | 1 | Tzd |
| III-41 | Tz | P4N2O** | EO | CH | Me | - | 1 | 0 | H | 1 | Tzd |
| III-42 | Tz | P4N2O** | EO | N | Me | - | 1 | 0 | H | 1 | Tzd |
| III-43 | Tz | P4N2O** | ES | CH | Me | - | 1 | 0 | H | 1 | Tzd |
| III-44 | Tz | P4N2O** | EO | CH | Me | - | 1 | 0 | H | 1 | MOMClPh Cx |
| III-45 | Tz | P4N2O** | M4PhO | CH | Me | - | 1 | 0 | H | 1 | Tzd |
| III-46 | Tz | P4N2O** | M5Py2O | CH | Me | - | 1 | 0 | H | 1 | Tzd |
| III-47 | Tz | PM4N2O** | EO | CH | Me | - | 1 | 0 | H | 1 | Tzd |
| III-48 | Tz | PM4N2O** | EO | N | Me | - | 1 | 0 | H | 1 | Tzd |
| III-49 | Tz | PM4N2O** | ES | CH | Me | - | 1 | 0 | H | 1 | Tzd |
| III-50 | Tz | PM4N2O** | EO | CH | Me | - | 1 | 0 | H | 1 | MOMClPh Cx |
| III-51 | Tz | PM4N2O** | M4PhO | CH | Me | - | 1 | 0 | H | 1 | Tzd |
| III-52 | Tz | PM4N2O** | M5Py2O | CH | Me | - | 1 | 0 | H | 1 | Tzd |
| III-53 | Tz | PM4M2O* | EO | CH | Me | - | 1 | 0 | H | 1 | Tzd |
| III-54 | Tz | PM4N2O* | EO | N | Me | - | 1 | 0 | H | 1 | Tzd |
| III-55 | Tz | PM4N2O* | ES | CH | Me | - | 1 | 0 | H | 1 | Tzd |
| III-56 | Tz | PM4N2O* | EO | CH | Me | - | 1 | 0 | H | 1 | MOMClPh Cx |
| III-57 | Tz | PM4N2O* | M4PhO | CH | Me | - | 1 | 0 | H | 1 | Tzd |
| III-58 | Tz | PM4N2O* | M5Py2O | CH | Me | - | 1 | 0 | H | 1 | Tzd |
| III-59 | Tz | PMO4N1O* | EO | CH | Me | - | 1 | 0 | H | 1 | Tzd |
| III-60 | Tz | PMO4N1O* | EO | N | Me | - | 1 | 0 | H | 1 | Tzd |
| III-61 | Tz | PMO4N1O* | ES | CH | Me | - | 1 | 0 | H | 1 | Tzd |
| III-62 | Tz | PMO4N1O* | EO | CH | Me | - | 1 | 0 | H | 1 | MOMClPh Cx |
| III-63 | Tz | PMO4N1O* | M4PhO | CH | Me | - | 1 | 0 | H | 1 | Tzd |
| III-64 | Tz | PMO4N1O* | M5Py2O | CH | Me | - | 1 | 0 | H | 1 | Tzd |
| III-65 | Tz | PMO4N1O* * | EO | CH | Me | - | 1 | 0 | H | 1 | Tzd |
| III-66 | Tz | PMO4M1O* * | EO | N | Me | - | 1 | 0 | H | 1 | Tzd |
| III-67 | Tz | PMO4N1O* * | ES | CH | Me | - | 1 | 0 | H | 1 | Tzd |
| III-68 | Tz | PMO4N1O* * | EO | CH | Me | - | 1 | 0 | H | 1 | MOMClPh Cx |
| III-69 | Tz | PMO4N1O* * | M4PhO | CH | Me | - | 1 | 0 | H | 1 | Tzd |
| III-70 | Tz | PMO4N1O* * | M5Py2O | CH | Me | - | 1 | 0 | H | 1 | Tzd |

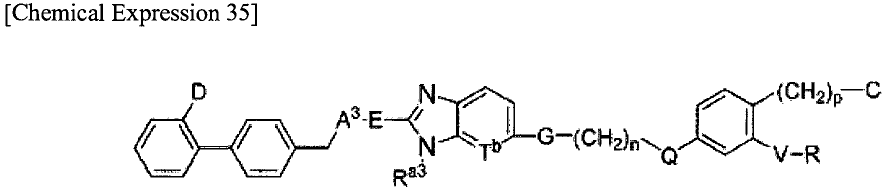

**TABLE 5**

| No. | D | A3 | E | T^{b} | R^{a3} | G | n | Q | -V-R | p | C |
|---|---|---|---|---|---|---|---|---|---|---|---|
| IV-1 | Cx | PM2N | - | CH | Me | NMe | 2 | 0 | H | 1 | Tzd |
| IV-2 | Tz | PM2N | - | CH | Me | NMe | 2 | 0 | H | 1 | Tzd |
| IV-3 | Cx | PM2N | MS4PhS | N | Me | O | 2 | 0 | O4CFP h | 2 | Cx |
| IV-4 | Cx | PM2N | ES | CH | Me | - | 1 | 0 | H | 1 | Tzd |
| IV-5 | Tz | PM2N | MS4PhO | CH | Me | NMe | 2 | 0 | H | 1 | Tzd |
| IV-6 | Tz | PM3N* | NMeES | CH | Me | NMe | 2 | 0 | H | 1 | MOECx |
| IV-7 | Tz | PM3N* | CONES | CH | Me | NMe | 2 | 0 | H | 1 | NMOPhOC OMCx |
| IV-8 | Tz | PEOC2N* | MS | CH | Me | NMe | 2 | 0 | H | 1 | Tzd |
| IV-9 | Tz | PEOC2N* | MS4PhO | CH | Me | NMe | 2 | 0 | H | 1 | Tzd |
| IV-10 | Tz | PEOC2N* | MS4PhO | N | Me | NMe | 3 | 0 | O4ClB n | 2 | Cx |
| IV-11 | Cx | cPENCO2N * | MS5PyMO | CH | Bn | O | 2 | S | OHx | 3 | Cx |
| IV-12 | Tz | PEOC2N* | MS5PyMO | CH | Me | NMe | 2 | 0 | H | 1 | Tzd |
| IV-13 | Tz | EOM2N | CONES3Ph 0 | CH | Me | NMe | 2 | 0 | H | 1 | NCONBnM Cx |
| IV-14 | Tz | P2NO | MSM | N | Me | NPh | 2 | 0 | H | 1 | Tzd |
| IV-15 | Cx | POM2NO* | MSM | CH | Me | NMe | 3 | 0 | H | 1 | MOBuCx |
| IV-16 | Tz | PM2N | OEO | CH | Me | NMe | 2 | 0 | H | 1 | Tzd |
| IV-17 | Tz | EOM3N* | NMeENMe | CH | Me | NPh | 2 | 0 | H | 1 | Tzd |

"Tz" in the tables indicates 1H-tetrazol-5-yl, "Pr" indicates propyl, "Et" indicates ethyl, "MS4PhO" indicates 1-methylenethio-phenylene-4-oxy (a group represented by the expression 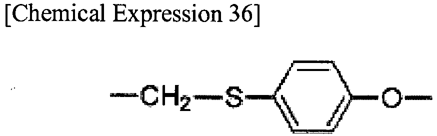 ), "Me" indicates methyl, "-" indicates a dangling bond, "Tzd" indicates 2,4-dioxothiazolidin-5-yl, "Cx" indicates carboxy, "CONTz" indicates N-(1H-tetrazol-5-yl)carbamoyl, "NSCF" indicates trifluoromethylsulfonylamino, "SNCOPr" indicates N-butylsulfamoyl, "SNCOMCINp" indicates N-[2-(5-chloronaphtyl)acetyl]sulfamoyl, "Dioz" indicates 2,4-dioxooxazolidin-5-yl, "Hyd" indicates 2,4-dioxoimidazolidin-5-yl, "Oxdz" indicates 2-oxo-1,3,5-oxadiazol-4-yl, "Oxtd" indicates 2-oxo-1,3,5-thiadiazolin-4-yl, "MNBzCx" indicates (benzoylamino)(carboxy)methyl, "MNEBnCx" indicates (N-ethyl-N-benzylamino)(carboxy)methyl, "MNEPyCOCx" indicates (N-ethyl-N-nicotinoylamino)(carboxy)methyl, "MNMBzCx" indicates (N-methyl-N-benzoylamino)(carboxy)methyl, "MNBCONBrBnCx" indicates (3-(3-bromobenzyl)-1-butylureido)(carboxy)methyl, "Thoxdz" indicates 2-thioxo-1,3,5-oxadiazolin-4-yl, "Dikbnl" indicates 3,4-dioxo-2-hydroxycyclo-1-butenyl, "NCONClPhMCx" indicates N-[N-(4-chlorophenyl)carbamoyl]-N-carboxymethylamino, "Bu" indicates butyl, "iBu" indicates isobutyl, "03MOBn" indicates 3-methoxybenzyloxy, "cPr" indicates cyclopropyl, "PrHyd" indicates 1-propyl-2,4-dioxoimidazolidin-5-yl, "Ph" indicates phenyl, "cPnPhHyd" indicates 1-(3-cyclopentylphenyl)-2,4-dioxoimidazolidin-5-yl, "CbmPhHyd" indicates 1-(3-carbamoylphenyl)-2,4-dioxoimidazolidin-5-yl, "CFPhN" indicates 4-trifluoromethylphenylamino, "EOPhPNM" indicates N-[3-(3-ethoxyphenyl)propyl]-N-methylamino, "MMeS4PhO" 1-(1-methylmethylenethio)-phenylene-4-oxy, "SNCOEt₂Ph" indicates N-(3,5-diethylbenzoyl)sulfamoyl, "nePn" indicates neopentyl, "MOClBnCx" indicates (4-chlorobenzyloxy)(carboxy)methyl, "iPr" indicates isopropyl, "SNCOBMPh" indicates N-[5-(3-methylphenyl)valeryl]sulfamoyl, "04CFBn" indicates 4-trifluoromethylbenzyloxy, "Hx" indicates hexyl, "MOECx" indicates (ethoxy)carboxy)methyl, "sBu" indicates s-butyl, "iHx" indicates isohexyl, "OMMPh" indicates α-methylbenzyloxy, "NPhMCx" indicates N-phenyl-N-carboxymethylamino, "MMCHO" indicates (methyl)(formyl)methyl, "Pn" indicates pentyl, "OcHx" indicates cyclohexyloxy, "De" indicates decyl, "MS4PhMO" indicates 1-methylenethio-phenylene-4-methyleneoxy, "PCOPn" indicates 3-hexanoylpropyl, "MS4BPhO" indicates 1-methylenethio-2-t-butyl-phenylene-4-xy, "NMOBzMCx" indicates N-(2-methoxybenzoyl)-N-carboxymethylamino, "NClBnMCx" indicates N-(4-chlorobenzyl)-N-carboxymethylamino, "MNSMCx" indicates (methylsulfonylamino)(carboxy)methyl, "MNSCFCx" indicates (trifluoromethylsulfonylamino)(carboxy)methyl, "MNSMPhCx" indicates (4-methylphenylsulfonylamino)(carboxy)methyl, "MNSBnCx" indicates (benzylsulfonylamino)(carboxy)methyl, "MNMSMPhCx" indicates [N-(4-methylphenylsulfonyl)-N-methylamino](carboxy)methyl, "MNClBnSMCx" indicates [N-methylsulfonyl-N-(4-chlorobenzyl)amino](carboxy)methyl, "MNBrPhSCFCx" indicates [N-trifluoromethylsulfonyl-N-(3-bromophenyl)amino](carboxy)methyl, "MNCO₂PhCx" indicates (phenoxycarbonylamino)(carboxy)methyl, "MNCO₂BnCx" indicates (benzyloxycarbonylamino)(carboxy)methyl, "MNCO₂BuCx" indicates (butoxycarbonylamino)(carboxy)methyl, "MSM4PhO" indicates 1-methylenethiomethylene-phenylene-4-oxy, "MS3PhO" indicates 1-methylenethio-phenylene-3-oxy, "MS3PhS" indicates 1-methylenethio-phenylene-3-thio, "MS2PhO" indicates 1-methylenethio-phenylene-2-oxy, "M04PhS" indicates 1-methyleneoxy-phenylene-4-thio, "M03PhS" indicates 1-methyleneoxy-phenylene-3-thio, "NCO₂PhMCx" N-phenoxycarbonyl-N-carboxymethylamino, "MS3PhMO" indicates 1-methylenethio-phenylene-3-methyleneoxy, "MS4PhEO" indicates 1-methylenethio-phenylene-4-ethylene-2-oxy, "MS3PhEO" indicates 1-methylenethio-phenylene-3-ethylene-2-oxy, "MN4PhO" indicates 1-methyleneimino-phenylene-4-oxy, "MN4Me₂PhO" indicates 1-methyleneimino-3,5-dimethyl-phenylene-4-oxy, "MN4PhS" indicates 1-methyleneimino-phenylene4-thio, "MS4PhN" indicates 1-methylenethio-phenylene-4-imino, MS4PhMN" indicates 1-methylenethio-phenylene-4-methyleneimino, "MS4PhCON" indicates 1-methylenethio-phenylene-4-carbonylimino, "MS4PhMCON" indicates 1-methylenethio-phenylene-4-methylenecarbonylimino, "MS3PhMCON" indicates 1-methylenethio-phenylene-3-methylenecarbonylimino, "ES4PhO" 1-(ethylene-2-thio)-phenylene-4-oxy, "ES4PhMO" indicates 1-(ethylene-2-thio)-phenylene-4-methyleneoxy, "EMeS4PhO" indicates 1-(ethylene-2-methyl-2-thio)-phenylene-4-oxy (a group represented by the expression

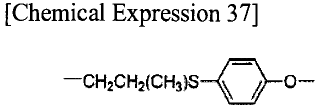

), "MMeMS4PhMO" indicates 1-(1-methylethylene-2-thio)-phenylene-4-methyleneoxy, "OE4MOPh" indicates 2-(4-methoxyphenyl)ethoxy, "O4BnPhO" indicates 1-oxy-3-benzyl-phenylene-4-oxy, "O3NpO" indicates 1-oxy-naphthylene-3-oxy "MSES4PhO" indicates 1-(1-methylenethio-ethylene-2-thio)-phenylene-4-oxy, "MSE04PhS" indicates 1-(1-methylenethio-ethylene-2-oxy)-phenylene-4-thio, "MSES4PhMO" indicates 1-(1-methylenethio-ethylene-2-thio)-phenylene-4-methyleneoxy, "MSEO" indicates 1-methylenethio-ethylene-2-oxy (a group represented by the expression -CH₂SCH₂CH₂O-, "MOEO" indicates 1-methyleneoxy-ethylene-2-oxy, "MSES" indicates 1-metylenethio-ethylene-2-thio, "MNES" indicates 1-methyleneimino-ethylene-2-thio, "MOBnCx" indicates (benzyloxy)(carboxy)methyl, "MSEN" indicates 1-methylenethio-ethylene-2-imino, "O4MePhSO₂4MePhO" indicates 1-(1-oxy-3-methylphenylene-4-sulfonyl)-3-methylphenylene-4-oxy (a group represented by the expression

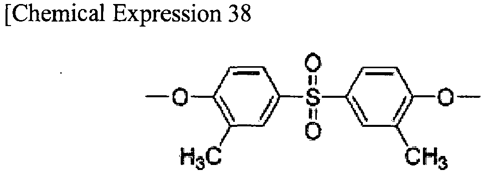

), "O6NpMO" indicates 2-oxy-naphthylene-6-methyleneoxy, "M7BThiMO" indicates 4-methylene-benzothiazolylene-7-methyleneoxy, "MS5PyN" indicates 2-methylenethio-pyridinylene-5-imino, "MN5PyN" indicates 2-methyleneimino-pyridinylene-5-imino, "M5ClMeBImMO" indicates 1-methylene-4-chloro-2-methyl-benzimidazolylene-5-methyleneoxy, "M5OxzEO" indicates 2-methylene-oxazolylene-5-ethylene-2-oxy, "MS5ThiMO" indicates 2-methylenethio-thiazolylene-5-methyleneoxy, "M5TtzN" indicates 2-methylene-tetrazolylene-5-imino, "MS2BImMO" indicates 6-methylenethio-benzimidazolylene-2-methyleneoxy, "MS7PyImO" indicates 2-methylenethio-pyridoimidazolylene-7-oxy, "PPh" indicates 3-phenylpropyl, "MS6BOxzO" indicates 2-methylenethio-benzoxazolylene-6-oxy, "OPPh" indicates 3-phenylpropyloxy, "MS6BOxzMO" indicates 2-methylenethio-benzoxazolylene-6-methyleneoxy, "CON3MeNPhS" indicates 1-carbonylimino-4-methylamino-phenylene-3-thio, "NCOMS" indicates iminocarbonylmethylenethio (a group represented by the expression -NHCOCH₂S-), "MSENCOMS" indicates 1-methylenethio-ethylene-2-iminocarbonylmethylenethio,"MO" indicates methyleneoxy, "MSENMe" indicates 1-methylenethio-ethylene-2-(N-methylimino), "MMeSEO" indicates 1-(1-methylmethylenethio)-ethylene-2-oxy, "MSESPS" indicates 1-(1-methylenethio-ethylene-2-thio)-trimethylene-3-thio (a group represented by the expression -CH₂SCH₂CH₂SCH₂CH₂CH₂-S-), "NCO₂BuMCx" indicates N-butoxycarbonyl-N-carboxymethylamino, "MEtS4PhO" indicates 1-(1-ethylenemethylenethio)-phenylene-4-oxy, "MMSBnCx" indicates (methyl)(benzylthio)(carboxy)methyl, "cBu" indicates cyclobutyl, "cPn" indicates cyclopentyl, "02MOPh" indicates 2-methoxyphenoxy, "NAc" indicates acetylimino, "NBz" indicates benzoylimino, "NMs" indicates methanesulfonylimino, "Oc" indicates octyl, "NTs" indicates p-toluenesulfonylimino, "MS4MPhO" indicates 1-methylenethio-2-methyl-phenylene-4-oxy, "04BrPh" indicates 4-bromophenoxy, "MSO₂4PhO" indicates 1-methylenesulfonyl-4-phenylene-4-oxy, "MS" indicates methylenethio, "MMeS" indicates 1-methylmethylenethio, "MMe₂S" indicates 1,1-dimethylmethylenethio, "MEtS" indicates 1-ethylmethylenethio, "ES" indicates ethylene-2-thio, "Hp" indicates heptyl, "PS" indicates trimethylene-3-thio, "MNiP" indicates methylene-N-isopropylimino, "4MeOBn" indicates 4-methoxybenzyl, "SNCOMe" indicates N-acetylsulfamoyl, "SNCOiPr" indicates N-isobutyrylsulfamoyl, "SNCOPh" indicates N-benzoylsulfamoyl, "SNCOFCFPh" indicates N-(2-fluoro-5-trifluoromethylbenzoyl)sulfamoyl, "SNCOMMOPh" indicates N-(2-methoxybenzylcarbonyl)sulfamoyl, "MNMe" indicates methylene-N-methylimino, "O4ClBn" indicates 4-chlorobenzyloxy, "04MOBn" indicates 4-methoxybenzyloxy, "SMNp" 1-naphthylmethylthio, "MOBPhCx" indicates (4-t-butylphenoxy)(carboxy)methyl, "M4BzPh" indicates 4-benzoylbenzyl, "MNBzPhCx" indicates (2-benzoylphenylamino)(carboxy)methyl, "NCOBuMCx" indicates N-pentanoyl-N-carboxymethylamino, "NMeCOMS" indicates N-methyliminocarbonylmethylenethio (a group represented by the expression -N(CH₃)COCH₂S-), "MNSMPhCx*" indicates (2-methylphenylsulfonylamino)(carboxy)methyl, "MOiPrCx" indicates "isopropoxy(carboxy)methyl, "O3ClPh" indicates 3-chlorophenoxy, "NMzMCx" indicates N-benzoyl-N-carboxymethylamino, "NCO₂EPhMCx" indicates N-(3-phenylpropionyl)-N-carboxymethylamino, "MNCOBuBnCx" indicates (3-t-butylbenzylcarbonylamino)(carboxy)methyl, "NCONBuMCx" indicates N-(N-butylcarbamoyl)-N-carboxymethylamino, "MSO" indicates methylene-sulfanyl, "MSO₂" indicates methylene-sulfonyl, "CONMeEO" indicates 1-(carbonyl-N-methylimino)-ethylene-2-oxy, "MSMCLCFPhCx" indicates (3-chloro-4-trifluoromethylbenzylthio)(carboxy)methyl, "COE4PhO" indicates 1-(carbonyl-2-ethylene)-phenylene-4-oxy, "COEO" indicates 1-carbonyl-ethylene-2-oxy, "CON3PhO" indicates 1-carbonylimino-phenylene-3-oxy, "CONMeM4PhEO" indicates 1-(carbonyl-N-methyliminomethylene)-phenylene-4-(ethylene-2-oxy), "04CFPh" indicates 4-trifluoromethylphenoxy, "MNECOMMOPhCx" indicates [N-(4-methoxybenzylcarbonyl)-N-ethylamino](carboxy)methyl, "NCO₂MOPhMCx" indicates N-(4-methoxyphenoxycarbonyl)-N-carboxymethylamino, "O4ClPh" indicates 4-chlorophenoxy, "O3CFPh" indicates 3-trifluoromethylphenoxy, "MS3PyS" indicates 3-methylenethio-pyridinylene-5-thio, "MSO4PhO" indicates 1-methylenesulfinyl-phenylene-4-oxy, "MO4ClBnCx" indicates (carboxy)(4-chlorobenzyloxy)methyl, "NCO₂PhMCx" indicates N-carboxymethyl-N-phenoxycarbonylamino, "SM" thiomethylene, "SM4PhO" indicates 1-thiomethylene-phenylene-4-oxy, "SM3PhO" indicates 1-thiomethylene-phenylene-3-oxy, "EO" indicates ethylene-2-oxy, "NCOBnMCx" indicates N-benzylcarbonyl-N-carboxymethylamino, "NCONClBnMCx" indicates N-(3-chlorobenzylaminocarbonyl)-N-carboxymethylamino, "6MeBImNMeEO" indicates 2-(1-methylbenzimidazolylene)-6-(N-methyliminoethylene-2-oxy, "6MeBImSEO" indicates 2-(1-methylbenzimidazolylene)-6-thioethylene-2-oxy, "O4ClPhBn" indicates 4-(4-chlorophenyl)benzyloxy, "6MeBImSENMe" indicates 2-(1-methylbenzimidazolylene)-6-thioethylene-2-(N-methylimino), "MNMCOMOPhCx" indicates [N-methyl-N-(3-methoxyphenylcarbonyl)amino](carboxy)methyl, "Bz" indicates benzyl, "CONE4PhO" indicates 1-(carbonylimino-2-ethylene)phenylene-4-oxy, "OHx" indicates hexyloxy, "6MeBImOE4PhNMe" indicates 1-[2-(1-methylbenzimidazolylene)-6-oxy-2-ethylene]phenylene-4-(N-methylimino) (a group represented by the expression

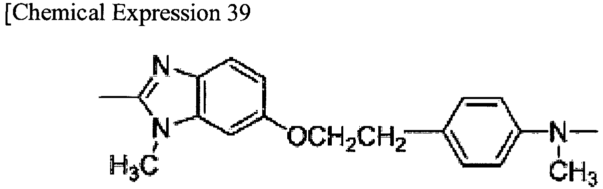

), "MNCONFPhCx" indicates [3-(4-fluorophenyl)ureido](carboxy)methyl, "2TtzEO" indicates 5-tetrazolylene-ethylene-2-oxy,"MMOBnCx" indicates 1-benzyloxy-1-carboxyethyl, "OE3PhNMe" indicates 1-(oxy-2-ethylene)-phenylene-3-(N-methylimino) (a group represented by the expression 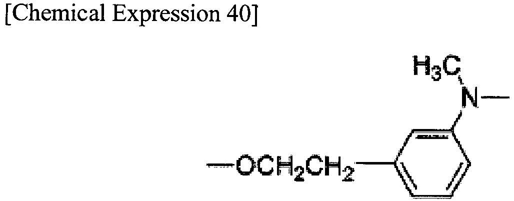 ), "NCONMBnMCx" indicates N-(N-methyl-N-benzylcarbamoyl)-N-carboxymethylamino, "NMeEOEO" indicates 1-(N-methyliminoethylene-2-oxy)ethylene-2-oxy (a group represented by the expression -N(CH₃)C₂H₄OC₂H₄O-), "NMeEO" indicates 1-(N-methylimino)ethylene-2-oxy (a group represented by the expression -N(CH₃)C₂H₄O-), "NBuEO" indicates 1-(N-butylimino)ethylene-2-oxy, "OEO4PhMO" indicates 1-(1-oxyethylene-2-oxy)phenylene-4-methyleneoxy,"OE06NpNMe" indicates 2-(1-oxyethylene-2-oxy)naphthylene-6-(N-methylimino), "MNCONPhCx*" indicates [3-(4-fluorophenyl)ureido](carboxy)methyl, "S3PhCONEO" indicates 1-thiophenylene-3-carbonylimino-ethylene-2-oxy (a group represented by the expression

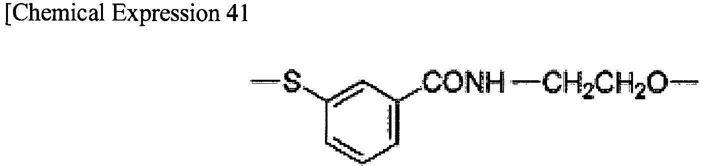

), "PNMe" indicates trimethylene-3-(N-methylimino), "04tBPh" indicates 4-(t-butyl)phenoxyl, "MPOBnOCx" indicates (4-propoxybenzyloxy)(carboxy)methyl, "CONEN" indicates 1-carbonyliminoethylene-2-imino, "CONMeENMe" indicates 1-(carbonyl-N-methylimino)ethylene-2-(N-methylimino) (a group represented by the expression - CON(CH₃)CH₂CH₂N(CH₃)-), "OE3ClPh" indicates 2-(3-chlorophenyl)ethoxy, "ENMe" indicates ethylene-2-(N-methylimino), "O2F4ClBn" indicates 2-fluoro-4-chlorobenzyloxy, "COMCONMe" indicates carbonylmethylene-carbonyl(N-methylimino) (a group represented by the expression -COCH₂CON(CH₃)-), "04BrBn" indicates 4-bromobenzyloxy, "6MeBImS" indicates 2-(1-methylbenzimidazolylene)-6-thio, "6MeBImNMePO" indicates 2-(1-methylbenzimidazolylene)-6-(N-methylimino)trimethylene-3-oxy (a group represented by the expression

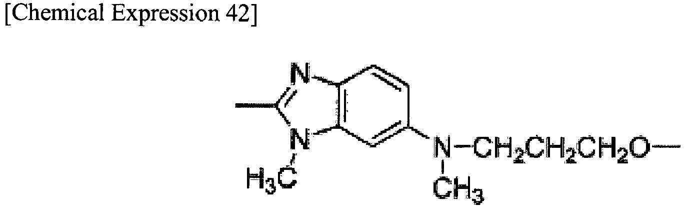

), "CON4PhO" indicates 1-(carbonylimino)phenylene-4-oxy, "OES" indicates 1-oxyethylene-2-thio, "034MOPh" indicates 3,4-dimethoxyphenoxy, "MNCONBnCx" indicates (3-benzylureido)(carboxy)methyl, "COMS" indicates carbonylmethylenethio, "NCO₂BnCx" indicates N-benzyloxycarbonyl-N-carboxymethylamino, "CONMe4PhO" indicates 1-(carbonyl-N-methylimino)phenylene-4-oxy, "6MeBImNBuEO" indicates 2-(1-methylbenzimidazolylene)-6-(N-butylimino)ethylene-2-oxy, "NCO₂BrPhMCx" indicates N-(4-bromophenoxycarbonyl-N-carboxymethylamino, "CONM4PhO" indicates 1-(carbonyliminomethylene)phenylene-4-oxy (a group represented by the expression 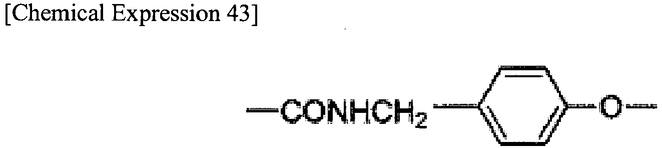 ), "CONEO4PhO" indicates 1-(1-carbonyliminoethylene-2-oxy)phenylene-4-oxy, "CONE4PhO" indicates 1-(carbonylimino-2-ethylene)phenylene-4-oxy, "Odz" indicates 2,4-dioxo-1,3,5-oxadiazolidin-5-yl, "CONE4PhNMe" indicates 1-(carbonylimino-2-ethylene)phenylene-4-(N-methylimino, "CONM5PyNMe" indicates 2-(carbonyliminomethylene)pyridinylene-5-(N-methylimino), "MMESEO" indicates 1-(1,1-dimethylmethylenethio)ethylene-2-oxy, "CONMeENMe5PyMN" indicates 2-[1-(carbonyl-N-methylimino)ethylene-2-(N-methylimino)]pyridinylene-5-methyleneimino (a group represented by the expression 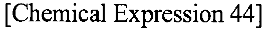 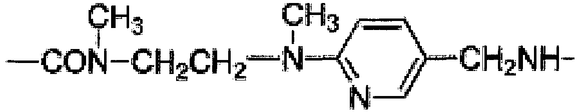 ), "NCONCPFhMCx" indicates N-[N-(3-trifluoromethylphenyl)carbamoyl]-N-carboxymethylamino, "MSBnCx" indicates (benzylthio)(carboxy)methyl, "MOEFBnCx" is (3-ethyl-2-fluorobenzyloxy)(carboxy)methyl, "MEOCx" indicates (ethoxy)(carboxy)methyl, "PM4N2O" indicates a group represented by the expression 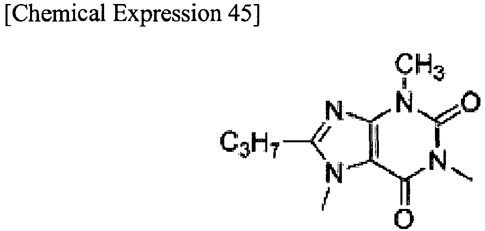 , "EOM4N20" indicates a group represented by the expression

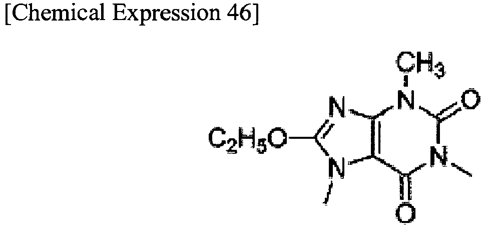

, "MCON" indicates methylene-carbonylimino, "BE4N20" indicates a group represented by the expression 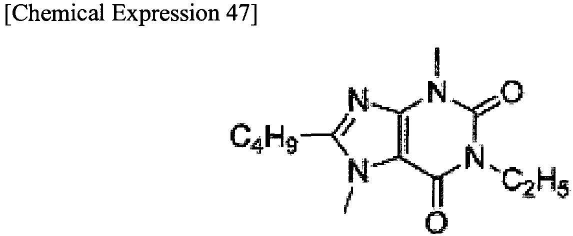 , "EO4PHs" indicates 1-(ethylene-2-oxy)phenylene-4-thio, "cPP4N2O*" indicates a group represented by the expression 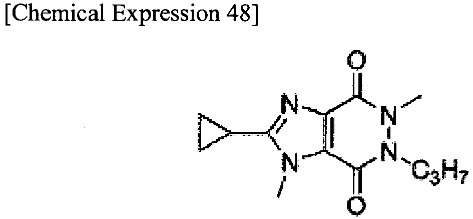 , "P4N2O*" indicates a group represented by the expression 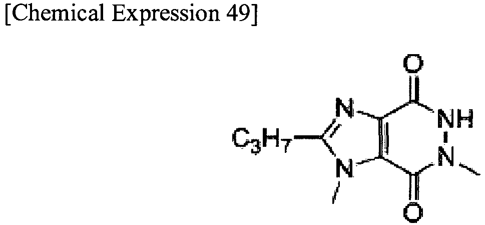 , "ESMCONMe" indicates ethylene-2-thiomethylenecarbonyl-N-methylimino (a group represented by the expression -CH₂CH₂SCH₂CON(CH₃)-, "P4NO" indicates a group represented by the expression

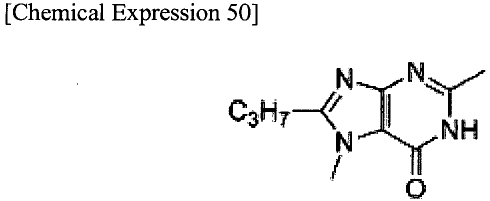

, "ESENBu" indicates 1-(ethylene-2-thio)ethylene-2-N-butylimino, "P2NOS" indicates a group represented by the expression 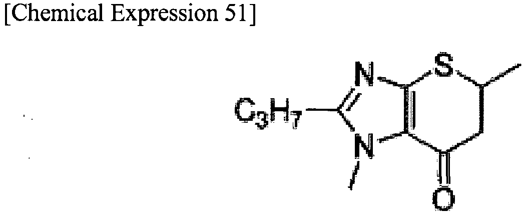 , "MNEOES" indicates 1-(methyleneiminoethylene-2-oxy)ethylene-2-thio, "PNOM2NS" indicates a group represented by the expression 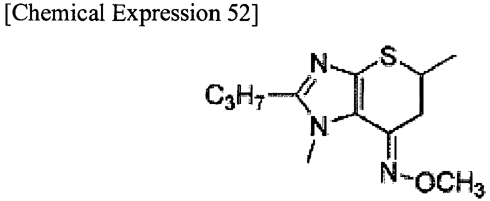 , "PNES3PhPhO" indicates 1-(trimethylene-3-iminoethylene-2-thio)-2-phenylphenylene-3-oxy (a group represented by the expression 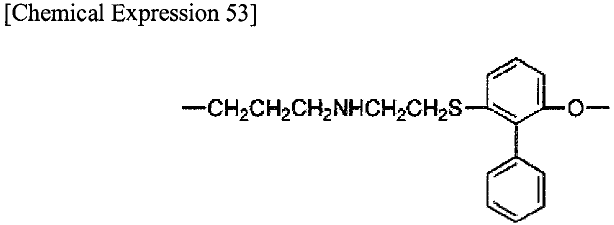 ), "PN02NS" indicates a group represented by the expression 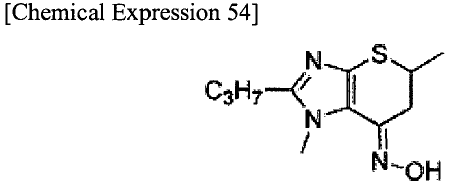 , "M4PhO" indicates 1-methylene-phenylene-3-oxy, "P2N3OS" indicates a group represented by the expression

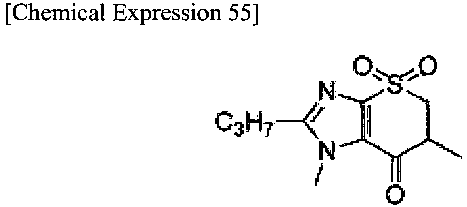

, "ENEOESO₂" indicates 1-(ethylene-2-imino)ethylene-2-(oxyethylene-2-sulfonyl) (a group represented by the expression -CH₂CH₂NHCH₂CH₂OCH₂CH₂SO₂-), "P3NO" indicates a group represented by the expression 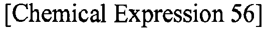 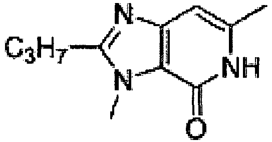 , "PM3NO" indicates a group represented by the expression 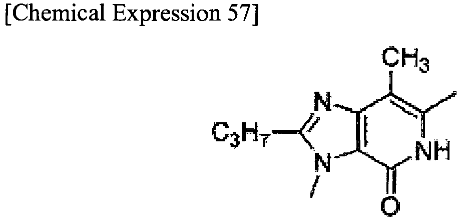 , "PM3NO*" indicates a group represented by the expression 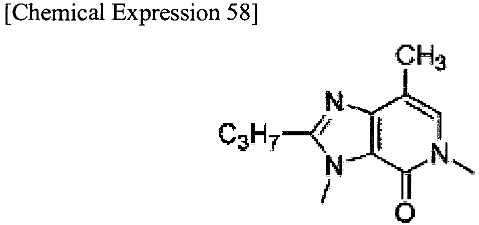 , "ES4cPPhO" indicates 1-(ethylene-2-thio)-2-cyclopropylphenylene-4-oxy, "P2NO" indicates a group represented by the expression 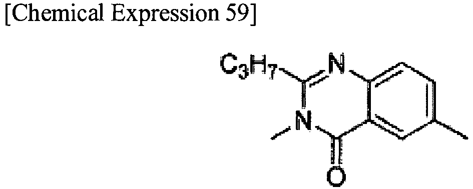 , "MS4cPPhO" indicates 1-methylenethio-2-cyclopropylphenylene-4-oxy, "PM2NO" indicates a group represented by the expression 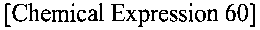 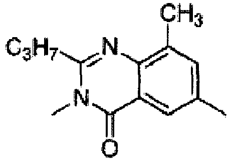 , "MS4BzPhO" indicates 1-methylenethio-3-benzoylphenylene-4-oxy, "PCF2NO" indicates a group represented by the expression

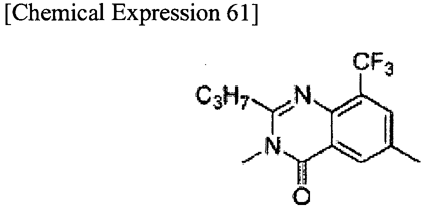

, "O3ClBn" indicates 3-chlorobenzyloxy, "PCl2NO" indicates a group represented by the expression 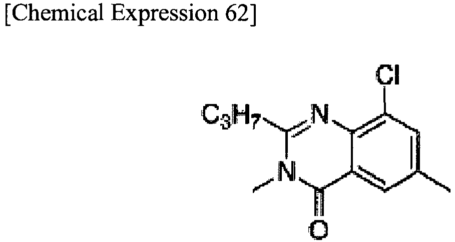 , "POM2NO" indicates a group represented by the expression 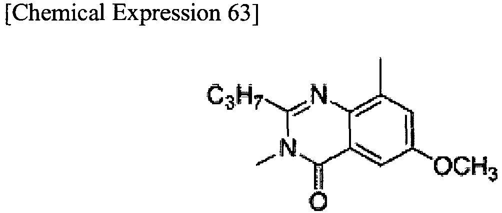 , "MS4OMePhO" indicates 1-methylenethio-3-methoxyphenylene-4-oxy, "OES5PyO" indicates 2-(oxyethylene-2-thio)-pyridinylene-5-oxy, "cPE2N**" indicates a group represented by the expression 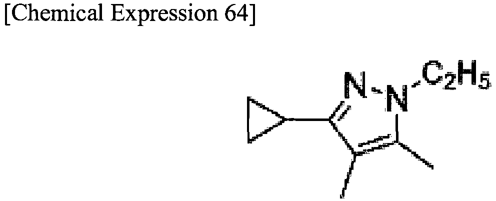 , "BE2N**" indicates a group represented by the expression

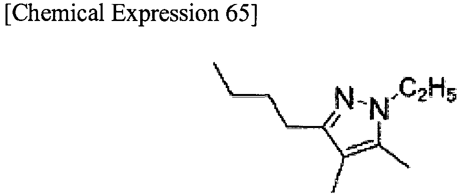

, "MS4BuOPhO" indicates 1-methylenethio-3-propoxyphenylene-4-oxy, "PcH2NO*" indicates a group represented by the expression 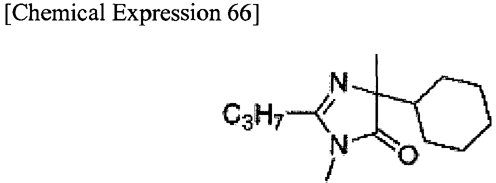 , "ES6PyN" indicates 3-(ethylene-2-thio)pyridinylene-6-imino, "MNMCONPHCx" indicates (3-isopropyl-3-cyclohexyl-1-methylureido)(carboxy)methyl, "POM2NO*" indicates a group represented by the expression 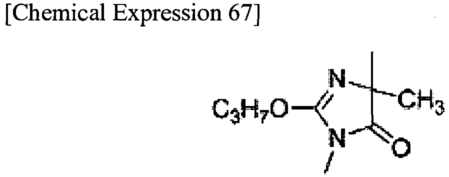 , "ES4PhNMe" indicates 1-(ethylene-2-thio)phenylene-3-(N-methylimino), "PiB2NO*" a group represented by the expression 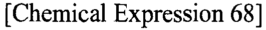 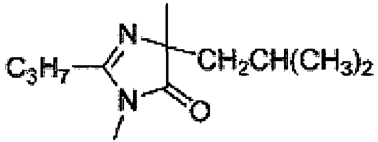 , "cPMNO" indicates a group represented by the expression 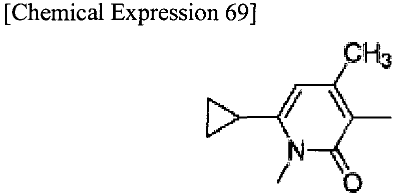 , "PNO" indicates a group represented by the expression 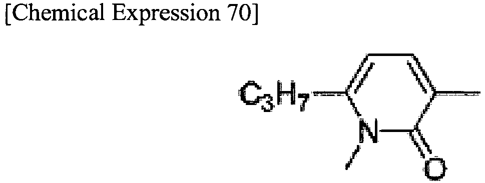 , "ES3PhNMe" indicates 1-(ethylene-2-thio)phenylene-3-(N-methylimino), "PNO*" indicates a group represented by the expression 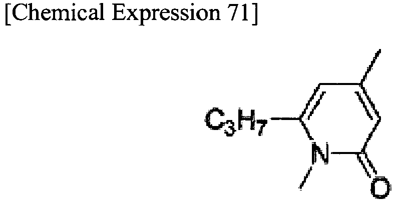 , "5Ttz" indicates 2,5-tetrazolylene, "P2NO**" indicates a group represented by the expression

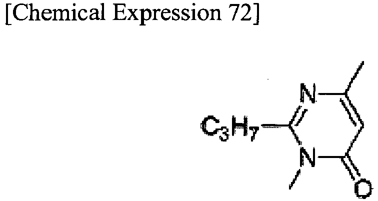

, "EMeS" indicates ethylene-2-methyl-2-thio, "MNMCOHCx" indicates (N-cyclohexylcarbonyl-N-methylamino)(carboxy)methyl, "NCONPPMCx" indicates "N-(N-propyl-N-butylcarbamoyl)-N-carboxymethylamino, "OMNp" indicates 2-naphthylmethoxy, "PM2N" indicates a group represented by the expression 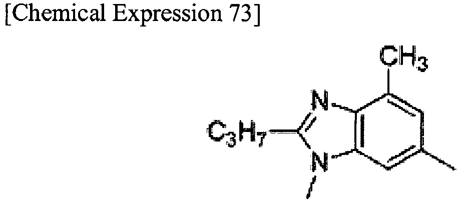 , "MS4PhS" indicates 1-methylenethiophenylene-4-thio, "PM3N*" indicates a group represented by the expression 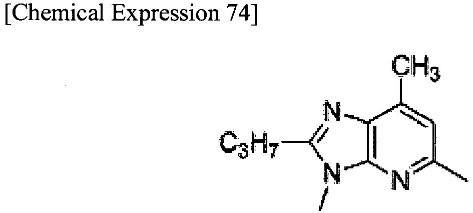 , "NMeES" indicates 1-(N-methylimino)ethylene-2-thio, "CONES" indicates 1-carbonyliminoethylene-2-thio, "NMOPhOCOMCx" indicates N-(4-methoxyphenoxycarbonyl)-N-carboxymethylamino, "PEOC2N*" indicates a group represented by the expression 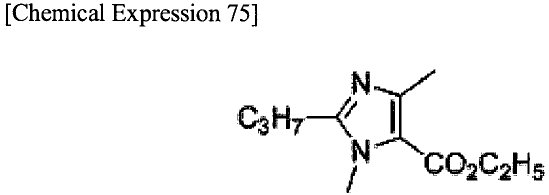 , "cPENCO2N*" indicates a group represented by the expression

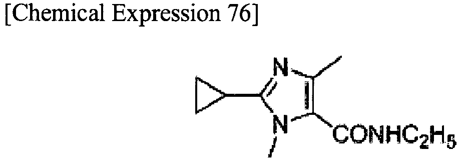

, "MS5PyMO" indicates 2-methylenethiopyridinylene-5-methyleneoxy, "EOM2N" indicates a group represented by the expression 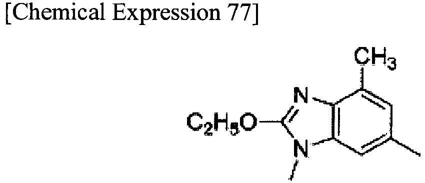 , "CONES3PhO" indicates 1-(carbonyliminoethylene-2-thio)phenylene-3-oxy, "NCONBnMCx" indicates N-(N-benzylcarbamoyl)-N-carboxymethylamino, "MSM" indicates methylenethiomethylene, "MOBuCx" indicates (butoxy)(carboxy)methyl, "OEO" indicates ethylenedioxy, "EOM3N*" indicates a group represented by the expression 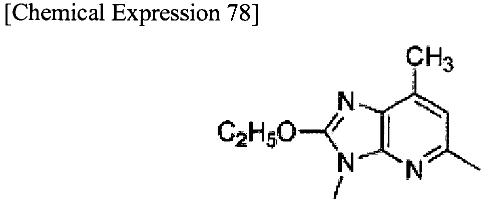 , "NMeENMe" indicates ethylenebis(N-methylimino), "5MSM2PyO" indicates 5-methylenethiomethylene-pyridinylene-2-oxy, "6MSM3PyO" indicates 6-methylenethiomethylene-pyridinylene-3-oxy, "5MSM2PyS" indicates 5-methylenethiomethylene-pyridinylene-2-thio, "6MSM3PyS" indicates 6-methylenethiomethylene-pyridinylene-3-thio, "5MSE2PyO" indicates 5-(1-methylenethio-2-ethylene)-pyridinylene-2-oxy, "MSM4PhS" indicates 1-methylenethiomethylene-phenylene-4-thio, "MS5Py2O" indicates 5-methylenethio-pyridinylene-2-oxy, "MMeSM4PhO" indicates 1-(1-methylmethylenethiomethylene)-phenylene-4-oxy, "MMeSM5Py2O" indicates 5-(1-methylmetylenethiomethylene)-pyridinylene-2-oxy, "SMMe" indicates thio-1-methylmethylene, "SE" indicates 1-thio-2-ethylene, "SM4PhS" indicates 1-thiomethylene-phenylene-4-thio, "SE4PhS" indicates 1-(1-thio-2-ethylene)-phenylene-4-thio, "SM5Py2O" indicates 5-thiomethylene-pyridiniylene-2-oxy, "NCO3CFPhMCx" indicates N-(3-trifluoromethylbenzoyl)-N-carboxymethylamino, "MOMMeOPhCx" indicates (4-methoxybenzyloxy)(carboxy)methyl, "MOMClPhCx" indicates (3-chlorobenzyloxy)(carboxy)methyl "MOM3PyCx" indicates (pyridin-3-ylmethyloxy)(carboxy)methyl, "MOEClPhCx" indicates [2-(4-chlorophenylethoxy](carboxy)methyl, "MOtBuPhCx" indicates (4-t-butylphenoxy)(carboxy)methyl, "MMeSEO" indicates 1-(1,1-dimethylmethylenethio)ethylene-2-oxy, "MmeS4PhO" indicates 1-(methylmethylenethio)phenylene-4-oxy, "MMe₂S4PhO" indicates 1-(1,1-dimethylmethylenethio)phenylene-4-oxy, "P4N2O**" indicates a group represented by the expression 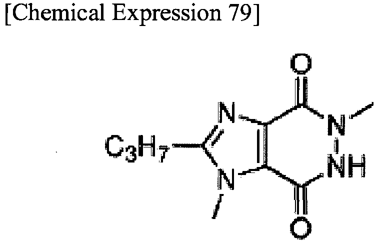 , "PM4N2O**" indicates a group represented by the expression 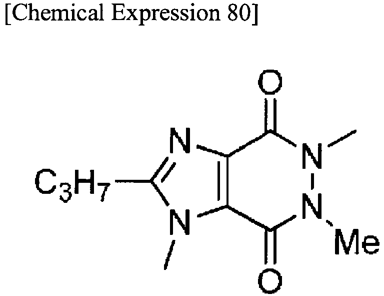 , "PM4N2O*" indicates a group represented by the expression 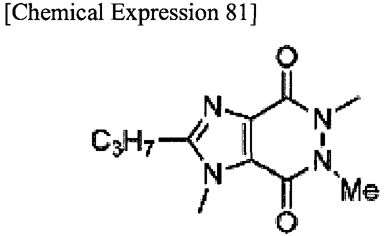 , "M4PhO" indicates 1-methylene-phenylene-4-oxy, "M5Py2O" indicates 5-methylene-pyridinylene-2-oxy, "PMO4N10*" indicates a group represented by the expression 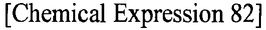 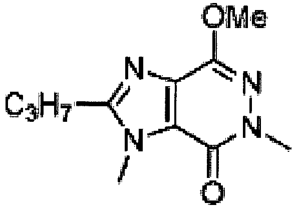 , and "PMO4N1O**" indicates a group represented by the expression

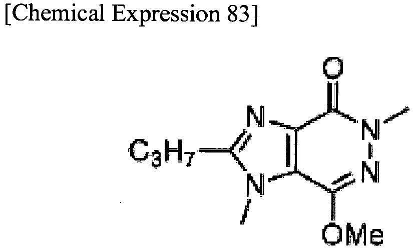

Unless specified otherwise, "E" in the compounds of example compound nos. II-1 to II-76 bonds to position 6 of a group represented by the expression 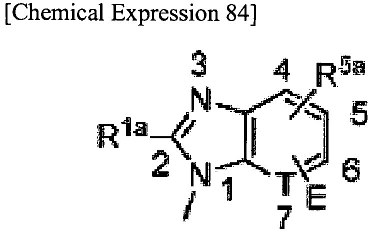 (where R^{1a}, R^{5a}, and E are defined as described earlier, and T indicates a group represented by the expression -CH= or -N=)
and bonds to position 5 of this expression in example compound nos. II-16, 17, 25, 26, and 73, and to position 7 of this expression in example compound nos. II-52 and 53.

In Tables 1 to 5, advantageous compounds are example compound nos. I-1, I-14, I -15, I-16, I-17, I-21, I-24, I-28, I-38, I-39, I-44, I-45, I-46, I-87, I-101, I-1 04, I-110, I-111, I-124, I-127, I-145, I -147, I-148, I-149, I-150, I-180, I-181 , I-182I I-223, I-241, I-283, I-285, I-2 86, I-293, I-295, I-296, I-297, I-299, I -300, I-303, I-304, I-305, I-309, I-311 , I-315, I-319, I-320, I-321, I-322, I-3 23, I-324, I-325, I-326, I-329, I-331, I -333, I-334, I-335, I-336, I-337, I-338 , I-342, I-343, I-344, I-345, I-346, II-1, II-6, II-7, II-19, II-25, II-27, II-3 1, II-35, II-36, II-37, II-38, II-45, I I -48, II-52, II-54, II-60, II-72, II-73, III-35, III-41, I I I-53, or IV-1,

more advantageous compounds are I-1, I-14, I-15, I-16, I-17, I-21, I-24, I-28, I-38, I-44, I-45 , I-46, I-87, I-101, I-104, I-127, I-148 , I-149, I-150, I-181, I-182, I-223, I-2 41, I-293, I-295, I-296, I-297, I-304, I -305, I-309, I-311, I-315, I-320, I-321 , I-322, I-323, I-333, I-334, I-335, I-3 42, I-343, I-344, I-345, I-346, II-1, I I -6, II-7, II-19, II-27, II-35, II-36, II -54, II-60, or IV-1,

even more advantageous compounds are example compound no. I-1: 4-[4-{2-[4-(2,4-dioxothiazolidin-5-ylmethyl)phenoxymethyl]-1-methyl-1H-benzimidazol-6-yloxy}phenylthiomethyl]-2-propyl-1-[2'-(1H-tetrazol-5-yl)biphenyl-4-ylmethyl]-1H-imidazole-5-carboxylic acid ethyl ester, example compound I-38: 4-[4-{2-[4-(2,4-dioxothiazolidin-5-ylmethyl)phenoxymethyl]-1-methyl-1H-benzimidazol-6-yloxy}phenylthiomethyl]-2-propyl-1-[2'-(1H-tetrazol-5-yl)biphenyl-4-ylmethyl]-1H-imidazole-5-carboxylic acid methyl ester, example compound no. I-46: 4-[4-{2-[4-(2,4-dioxothiazolidin-5-ylmethyl)phenoxymethyl]-1-methyl-1H-benzimidazol-6-yloxy}phenylthiomethyl]-2-propyl-1-[2'-(1H-tetrazol-5-yl)biphenyl-4-ylmethyl]-1H-imidazole-5-carboxylic acid N-methylamide, example compound no. I-101: 4-[3-{2-[4-(2,4-dioxothiazolidin-5-ylmethyl)phenoxymethyl]-1-methyl-1H-benzimidazol-6-yloxty}phenylthiomethyl]-2-propyl-1-[2'-(1H-tetrazol-5-yl)biphenyl-4-ylmethyl]-1H-imidazole-5-carboxylic acid ethyl ester, example compound no. I-104: 4-[4-{2-[4-(2,4-dioxothiazolidin-5-ylmethyl)phenoxymethyl]-1-methyl-1H-benzimidazol-6-ylthio}phenoxymethyl]-2-propyl-1-[2'-(1H-tetrazol-5-yl)biphenyl-4-ylmethyl]-1H-imidazole-5-carboxylic acid ethyl ester, example compound no. I-127: 4-[2-[1-methyl-2-{4-(2,4-dioxothiazolidin-5-ylmethyl)phenoxymethyl}-1H-benzimidazol-6-yloxy]ethylthiomethyl]-2-propyl-1-[2'-(1H-tetrazol-5-yl)biphenyl-4-ylmethyl]-1H-imidazole-5-carboxylic acid ethyl ester, example compound no. I-148: 4-[2-[1-methyl-2-{4-(2,4-dioxothiazolidin-5-ylmethyl)phenoxymethyl}-1H-benzimidazol-6-ylthioacetylamino]ethylthiomethyl]-2-propyl-1-[2'-(1H-tetrazol-5-yl)biphenyl-4-ylmethyl]-1H-imidazole-5-carboxylic acid ethyl ester, example compound no. I-149: 4-{2-[4-(2,4-dioxothiazolidin-5-ylmethyl)phenoxymethyl]-1-methyl-1H-benzimidazol-6-yloxymethyl}-2-propyl-1-[2'-(1H-tetrazol-5-yl)biphenyl-4-ylmethyl]-1H-imidazole-5-carboxylic acid ethyl ester (or hydrochloric acid salt thereof), example compound no. I-150: 4-{2-[4-(2,4-dioxothiazolidin-5-ylmethyl)phenoxymethyl]-1-methyl-1H-benzimidazol-6-yloxymethyl}-2-propyl-1-[2'-(1H-tetrazol-5-yl)biphenyl-4-ylmethyl]-1H-imidazole-5-carboxylic acid, example compound no. I-181: 4-[4-{2-[4-(2,4-dioxothiazolidin-5-ylmethyl)phenoxymethyl]-1-methyl-1H-benzimidazol-6-yloxy}phenylsulfonylmethyl]-2-propyl-1-[2'-(1H-tetrazol-5-yl)biphenyl-4-ylmethyl]-1H-imidazole-5-carboxylic acid ethyl ester, example compound no. I-182: 4-{2-[4-(2,4-dioxothiazolidin-5-ylmethyl)phenoxymethyl]-1-methyl-1H-benzimidazol-6-ylthiomethyl}-2-propyl-1-[2'-(1H-tetrazol-5-yl)biphenyl-4-ylmethyl]-1H-imidazole-5-carboxylic acid ethyl ester, example compound no. I-304: 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-chlorobenzyloxy)propionic acid, example compound no. I-305: N-[4-{6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenylmethyl]-N-phenoxycarbonylamino acetic acid, example compound no. I-309: 4-[4-{2-[4-(2,4-dioxothiazolidin-5-ylmethyl)phenoxymethyl]-1-methyl-1H-benzimidazol-6-yloxy}benzylthiomethyl]-2-propyl-1-[2'-(1H-tetrazol-5-yl)biphenyl-4-ylmethyl]-1H-imidazole-5-carboxylic acid ethyl ester, example compound no. I-311: 4-[2-{2-[4-(2,4-dioxothiazolidin-5-ylmethyl)phenoxymethyl]-1-methyl-1H-benzimidazol-6-yloxy}pyridin-5-ylmethylthiomethyl]-2-propyl-1-[2'-(1H-tetrazol-5-yl)biphenyl-4-ylmethyl]-1H-imidazole-5-carboxylic acid ethyl ester, example compound no. I-333: N-[4-{6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenylmethyl]-N-(3-trifluoromethylbenzoyl)amino acetic acid, example compound II-1: 5-(4-{6-[4-methyl-2-propyl-1-[2'-(2-tetrazol-5-yl)biphenyl-4-ylmethyl]-1H-benzimidazol-6-ylmethoxy]-1-methyl-1H-benzimidazol-2-ylmethoxy}benzyl)thiazolidine-2,4-dione, example compound II-7: 5-[4-[6-{2-(N-(1-methyl-2-[4-methyl-2-propyl-1-(2'-(1H-tetrazol-5-yl)biphenylmethyl)-1H-benzimidazol-6-yl]-1H-benzamidazol-6-yl)-N-methylamino)ethoxy}-1-methyl-1H-benzimidazol-2-ylmethoxy]benzyl]thiazolidine-2,4-dione, example compound II-35: 4'-[6-(N-{2-[4-(2,4-dioxothiazolidin-5-ylmethyl)phenoxymethyl]-3-methyl-3H-imidazo[4,5-b]pyridin-5-yl}-N-methylaminomethyl)-4-methyl-2-propyl-1H-benzimidazol-1-ylmethyl]biphenyl-2-carboxylic acid, example compound II-54: 4'-[6-{6-(N-2-[4-(2,4-dioxothiazolidin-5-ylmethyl)phenoxy]ethyl-N-methylamino)-1-methyl-1H-benzimidazol-2-yl}-4-methyl-2-propyl-1H-benzimidazol-1-ylmethyl]biphenyl-2-carboxylic acid, example compound II-60: 5-[4-[6-{4-(4-methyl-2-propyl-1-<2'-(1H-tetrazol-5-yl)biphenyl-4-ylmethyl>-1H-benzimidazol-6-ylcarbonylaminomethyl)phenoxy}-1-methyl-1H-benzimidazol-2-ylmethoxy]benzyl]thiazolidin-2,4-dione, or example compound IV-1: 4'-[6-{6-(N-2-[4-(2,4-dioxothiazolidin-5-ylmethyl)phenoxy]ethyl-N-methylamino)-1-methyl-1H-benzimidazol-2-yl}-4-methyl-2-propyl-1H-benzimidazol-1-ylmethyl]biphenyl-2-carboxylic acid, and

optimally example compound no. I-1, I-38, I-46, I-182, I-304, I-305, II-7, II-60, or IV-1.

The compound represented by general formula (I) of the present invention may be produced according to the following methods.

### <Method A>

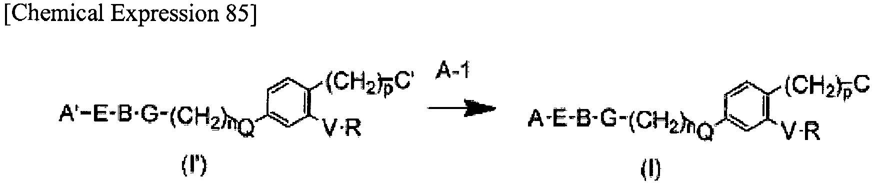

A, B, C, E, G, Q, V, R, n, and p in this expression are defined as described earlier, and A' and C' are defined the same as A and C except that tetrazole and thiazolidine-2,4-dion-5-yl included in A and thiazolidine-2,4-dion-5-yl included in C are protected. The carboxyl protecting group, the tetrazole protecting group, and the thiazolidine-2,4-dion-5-yl protecting group may be a conventional protecting group known in the literature. The carboxyl protecting group is advantageously a C₁-C₆ alkyl, benzyl, or benzhydryl, and optimally methyl, ethyl, t-butyl, or benzyl. The tetrazole protecting group is advantageously triphenylmethyl, benzhydryl, or benzyl, and optimally triphenylmethyl.

Method A is a method for producing a compound represented by general formula (I). A step A-1 is achieved by removing the protecting group from the compound represented by general formula (I').
Methods for removing this protecting group are conventional methods in organic chemistry; specifically, (a) a method of reacting compound (I') with an acid, (b) a method of reacting compound (I') with a base, or (c) a method of catalytic reduction of compound (I').

In the case that the protecting group is triphenylmethyl or a C₁-C₆ alkyl, (a) a method of reacting compound (I') with an acid may be used to remove the protecting group.
Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ether such as diethyl ether, diisopropyl ether, tetrahydrofuran, dioxane, dimethoxyethane, or diethylene glycol dimethyl ether; an amide such as dimethylformamide, dimethylacetamide, or hexamethylphosphoric acid triamide; an alcohol such as methanol, ethanol, or propanol; an organic acid such as trifluoroacetic acid, acetic acid, or propionic acid; water; or a mixture of these solvents; advantageously a halogenated hydrocarbon, an ether, an alcohol, an amide, water, or a mixture of these solvents; more advantageously water, an alcohol, or an ether; and optimally water, methanol, ethanol, dioxane, tetrahydrofuran, water, or a mixture of these organic solvents (in the case that the protecting group is a C₁-C₆ alkyl).

Although not specifically limited provided that it is used as an acid in a conventional reaction and does not negatively impact other portions of the compound, the acid used may be, for example, an inorganic acid such as hydrochloric acid, hydrobromic acid, sulfuric acid, perchloric acid, or phosphoric acid; an organic acid such as acetic acid, formic acid, oxalic acid, methanesulfonic acid, p-toluenesulfonic acid, camphor sulfonic acid, trifluoroacetic acid, or trifluoromethanesulfonic acid; a Lewis acid such as zinc chloride, tin tetrachloride, boron trichloride, boron trifluoride, or boron tribromide; or an acidic ion exchange resin; advantageously an inorganic acid or an organic acid; and optimally hydrochloric acid, acetic acid, or trifluoroacetic acid.
Although differing depending on factors such as the ingredient compounds, the acid, and the solvent, the reaction temperature is usually -20°C to 150°C, and advantageously 10°C to 100°c.
Although differing depending on factors such as the ingredient compounds, the acid, and the solvent, the reaction time is usually thirty minutes to ten days, and advantageously two hours to five days.

In the case that the protecting group is a C₁-C₆ alkyl, (b) a method of reacting compound (I') with a base may be used to remove the protecting group.
Although not specifically limited provided that it is inert in this reaction, the inert solvent used may be, for example, an inert solvent used in the "reaction (a)" described earlier; advantageously a halogenated hydrocarbon, an ether, an alcohol, an amide, water, or a mixture of these solvents; more advantageously water, an alcohol, an ether, or a mixture of a water and a solvent; and optimally, water, methanol, ethanol, dioxane, tetrahydrofuran, or a mixture of these organic solvents and water.
Although not specifically limited provided that it is used as a base in a conventional reaction and does not negatively impact other portions of the compound, the base used may be, for example, an alkali metal carbonate such as lithium carbonate, sodium carbonate, or potassium carbonate; an alkali metal bicarbonate such as lithium bicarbonate, sodium bicarbonate, or potassium bicarbonate; an alkali metal hydroxide such as lithium hydroxide, sodium hydroxide, or potassium hydroxide; a metal alkoxide such as lithium methoxide, sodium methoxide, sodium ethoxide, or potassium t-butoxide; an ammonia such as ammonia water or concentrated ammonia-methanol; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously an alkali metal hydroxide or an alkali metal carbonate; and optimally potassium hydroxide, sodium hydroxide, potassium hydroxide, or potassium carbonate.

The inert solvent, reaction temperature, and reaction time are the same as those used for the "reaction (a)" described earlier.
In the case that the protecting group is triphenylmethyl, benzhydryl, or benzyl, (c) a method of catalytic reduction of compound (I') may be used to remove the protecting group. With this method, compound (I') is reacted in an inert solvent with hydrogen in the presence of a contact reduction catalyst.
The contact reduction catalyst used may be a contact reduction catalyst conventionally used in organic chemistry; advantageously platinum oxide, palladium, palladium on carbon, or Raney nickel; and optimally palladium on carbon.
The hydrogen pressure may be, for example, atmospheric pressure to 10 atm, advantageously atmospheric pressure to 3 atm, and optimally atmospheric pressure to 2 atm.
The inert solvent used is a solvent conventionally used in a contact reduction reaction; for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ether such as diethyl ether, diisopropyl ether, tetrahydrofuran, dioxane, dimethoxyethane, or diethylene glycol dimethyl ether; an amide such as dimethylformamide, dimethylacetamide, or hexamethylphosphoric acid triamide; an alcohol such as methanol, ethanol, or propanol; water; or a mixture of these solvents; advantageously, an ether, an amide, an alcohol, or a mixture of these solvents; more advantageously an ether or an alcohol; and optimally tetrahydrofuran, methanol, or ethanol.
Although differing depending on factors such as the ingredient compounds, the contact reduction catalyst, and the solvent, the reaction temperature is usually -20°C to 150°C, and advantageously 10°C to 100°c.
Although differing depending on factors such as the ingredient compounds, the contact reduction catalyst, and the solvent, the reaction time is usually thirty minutes to ten days, and advantageously five hours to five days.

### <Method B>

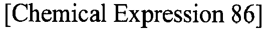

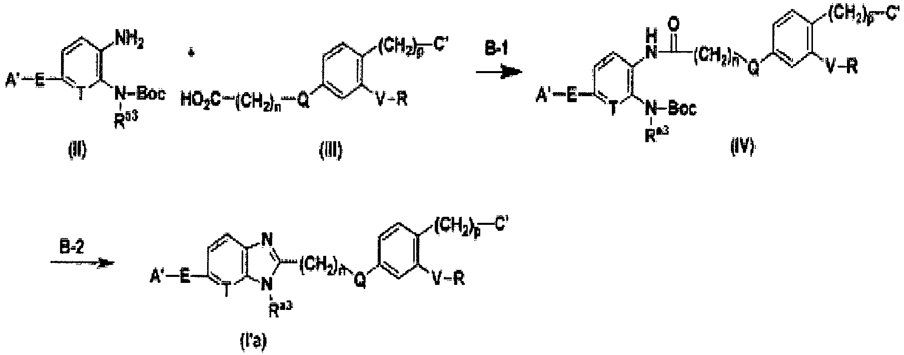

A', E, T, R^{a3}, n, Q, V, R, p, and C' in this expression are defined as described earlier, and Boc indicates t-butoxycarbonyl. The tetrazole or thiazolidin-2,4-dione in A' and C', however, need not be protected, and in the case that T is nitrogen, the group represented by the expression -N(R^{a3})-Boc may be a group represented by the expression -N(R^{a3})-H.

Method B is a step for producing a compound represented by general formula (I'a) in which B in the compound represented by general formula (I') is a group represented by the expression 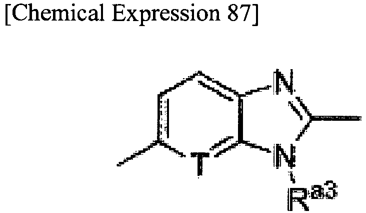 (a group bonding E to a portion of a benzene ring or a portion of a pyridine ring) and G is a dangling bond. Method B is carried out by condensing a compound represented by general formula (II) and a compound represented by general formula (III) (step B-1), then reacting the resulting compound represented by general formula (IV) with an acid to cyclize (step B-2).
In this method, the intermediary (IV) may be reacted with an acid to cyclize without isolating and refining. During cyclization, a triphenylmethyl protecting group contained in A' or C' may be removed simultaneously, and a t-butyl protecting group (a protecting group of carboxyl) may also be removed simultaneously.

The step B-1 is carried out by the following methods.
(a) Acid halide method
The acid halide method is carried out by reacting compound (III) in an inert solvent with a halogenating agent (for example, thionyl chloride, thionyl bromide, oxalyl chloride, oxalyl dichloride, phosphorus oxychloride, phosphorus trichloride, or phosphorus pentachloride, and advantageously oxalyl dichloride), then reacting the resulting acid halide with compound (II) or an acid addition salt thereof in an inert solvent in the presence or not of a base (advantageously in the presence).

The base used is, for example, an alkali metal carbonate such as lithium carbonate, sodium carbonate, or potassium carbonate; an alkali metal bicarbonate such as lithium bicarbonate, sodium bicarbonate, or potassium bicarbonate; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously an organic amine; and optimally triethylamine or N,N-diisopropyl ethylamine.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as dichloromethane, chloroform, 1,2-dichloroethane, or carbon tetrachloride; an ether such as diethyl ether, diisopropyl ether, tetrahydrofuran, dioxane, dimethoxyethane, or diethylene glycol dimethyl ether; a ketone such as acetone; an amide such as formamide, dimethylformamide, dimethylacetamide, or hexamethylphosphoric acid triamide; a sulfoxide such as dimethylsulfoxide; or sulfolane; advantageously a halogenated hydrocarbon, an ether, or an amide; and optimally dichloromethane, chloroform, tetrahydrofuran, dioxane, or dimethylformamide.

Although differing depending on factors such as the ingredient compounds and the halogenating agent, the reaction temperature is usually -20°C to 150°C when reacting a halogenating agent with compound (III) and when heating and reacting an acid halide with compound (II) or an acid addition salt thereof, and advantageously -10°C to 100°C when reacting a halogenating agent with compound (III), and -20°C to 100°C when reacting an acid halide with compound (II) or an acid addition salt thereof.
Although differing depending on factors such as the ingredient compounds, the halogenating agent, and the reaction temperature, the reaction time is usually thirty minutes to eighty hours and advantageously one to forty-eight hours when reacting a halogenating agent with compound (III) and when heating and reacting an acid halide with compound (II) or an acid addition salt thereof.

(b) Active ester method
The active ester method is carried out by reacting compound (III) with an active esterifying agent in an inert solvent, then reacting the resulting active ester with compound (II) or an acid addition salt thereof in an inert solvent and in the presence or not of a base (advantageously in the presence).

The active ester used is, for example, an N-hydroxy compound such as N-hydroxysuccinimide, 1-hydroxybenzotriazole, or N-hydroxy-5-norbornene-2,3-dicarboximide; a disulfide compound such as dipyridyl disulfide; a carbodiimide such as dicyclohexylcarbodiimide or 1-ethyl-3-(3-dimethylaminopropyl)carbodiimide hydrochloride; 1,1'-carbonylbis-1H-imidazole; 4-(4,6-dimethoxy-1,3,5-triadin-2-yl)-4-methylmorpholinium chloride (DMTMM), diphenyl phosphoric azide, hexafluorophosphoric benzotrazol-1-yloxytris(dimethylamino)phosphonium, or triphenylphosphine; advantageously dicyclohexylcarbodiimide, 1-ethyl-3-(3-dimethylaminopropyl)carbodiimide hydrochloride, 4-(4,6-dimethoxy-1,3,5-triadin-2-yl)-4-methylmorpholinium chloride (DMTMM), diphenyl phosphoric azide, or 1,1'-carbonylbis-1H-imidazole; and optimally 1-ethyl-3-(3-dimethylaminopropyl)carbodiimide hydrochloride, 4-(4,6-dimethoxy-1,3,5-triadin-2-yl)-4-methylmorpholinium chloride (DMTMM), or 1,1'-carbonylbis-1H-imidazole.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as dichloromethane, chloroform, 1,2-dichloroethane, or carbon tetrachloride; an ether such as diethyl ether, diisopropyl ether, tetrahydrofuran, dioxane, dimethoxyethane, or diethylene glycol dimethyl ether; a ketone such as acetone; an amide such as formamide, dimethylformamide, dimethylacetamide, or hexamethylphosphoric acid triamide; a sulfoxide such as dimethylsulfoxide; or sulfolane; advantageously a halogenated hydrocarbon, an ether, or an amide; and optimally dichloromethane, 1,2-dichloroethane, dioxane, tetrahydrofuran, or dimethylformamide.

The base used may be the same base as that used in the "acid halide method (a)," advantageously an organic amine, and optimally triethylamine or N,N-diisopropyl ethylamine.
Although differing depending on factors such as the ingredient compounds and the active esterifying agent, the reaction temperature is usually -70°C to 150°C and advantageously -10°C to 100°C during the active esterification reaction, and -20°C to 100°C and advantageously 0°C to 50°C when reacting an active ester with compound (II) or an acid addition salt thereof.
Although differing depending on factors such as the ingredient compounds, the active esterifying agent, and the reaction temperature, the reaction time is usually thirty minutes to ten days and advantageously one to forty-eight hours both during the active esterification reaction and when reacting an active ester with compound (II) or an acid addition salt thereof.

(c) Mixed acid anhydride method
The mixed acid anhydride method is carried out by reacting compound (III) with a mixed acid anhydridizing agent in an inert solvent and in the presence or not of a base (advantageously in the presence), then reacting the resulting mixed acid anhydride with compound (II) or an acid addition salt thereof in an inert solvent.

The base used is, for example, an alkali metal carbonate such as lithium carbonate, sodium carbonate, or potassium carbonate; an alkali metal bicarbonate such as lithium bicarbonate, sodium bicarbonate, or potassium bicarbonate; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously an organic amine; and optimally triethylamine or N,N-diisopropyl ethylamine.

The mixed acid anhydridizing agent is, for example, a carbonic acid C₁-C₆ alkyl halide such as ethyl chlorocarbonate or isobutyl chlorocarbonate; a C₁-C₆ alkanoyl halide such as pivaloyl chloride; or a di-C₁-C₆ alkyl or di-C₆-C₁₄ aryl cyanophosphonate such as diethyl cyanophosphonate or diphenyl cyanophosphonate; advantageously isobutyl chlorocarbonate or a di-C₁-C₄ alkyl or di-C₆-C₁₄ aryl cyanophosphoric acid; and optimally isobutyl chlorocarbonate or diethyl cyanophosphonate.

Although not specifically limited provided that it does not damage the reaction and the starting material dissolves to some extent, the inert solvent used when producing a mixed acid anhydride may be, for example, an aliphatic hydrocarbon such as hexane, heptane, ligroin, or petroleum ether; an aromatic hydrocarbon such as benzene, toluene, or xylene; a halogenated hydrocarbon such as dichloromethane, chloroform, 1,2-dichloroethane, or carbon tetrachloride; an ether such as diethyl ether, diisopropyl ether, tetrahydrofuran, dioxane, dimethoxyethane, or diethylene glycol dimethyl ether; a ketone such as acetone; an amide such as formamide, dimethylformamide, dimethylacetamide, or hexamethylphosphoric acid triamide; a sulfoxide such as dimethylsulfoxide; or sulfolane; advantageously an ether or an amide; and optimally tetrahydrofuran or dimethylformamide.

Although differing depending on factors such as the ingredient compounds and the mixed acid anhydridizing agent, the reaction temperature during the reaction for producing a mixed acid anhydride is usually -50°C to 100°C, and advantageously -20°C to 60°C.
Although differing depending on factors such as the ingredient compounds, the mixed acid anhydridizing agent, and the reaction temperature, the reaction time is usually thirty minutes to one week, and advantageously one hour to three days.
The mixed acid anhydride is reacted with compound (II) or an acid addition salt thereof in an inert solvent in the presence or not of a base (advantageously in the presence). The base and the inert solvent used are the same as those used in the reaction to produce the mixed acid anhydride.
Although differing depending on factors such as the ingredient compounds, and the base, the reaction temperature when reacting the mixed acid anhydride with compound (II) or an acid addition salt thereof is usually -30°C to 100°C, and advantageously 0°C to 80°C.
Although differing depending on factors such as the ingredient compounds, the base, and the reaction temperature, the reaction time when reacting the mixed acid anhydride with compound (II) or an acid addition salt thereof is usually five minutes to twenty-four hours, and advantageously thirty minutes to sixteen hours.

In the case that a di-C₁-C₆ alkyl or dᵢ-C₆-C₁₄ aryl cyanophosphonate is used in this reaction, compound (II) may be reacted directly with compound (III) in the presence of a base.

After the reactions have ended, the intended compounds of the reactions are collected from the reaction mixtures following a conventional method. For example, after suitably neutralizing a reaction mixture and suitably filtering and removing the inert solvent, if any, water and an organic solvent which is immiscible with water, such as ethyl acetate, are added to separate an organic layer containing the intended compound, which is washed with water or the like and dried using anhydrous magnesium sulfate, anhydrous sodium sulfate, anhydrous sodium bicarbonate, or the like to give the intended compound of the reaction after removing the solvent. As required, the intended compound may be refined again (or the reaction mixture may be refined directly) following a conventional method (for example, distillation, recrystallization, or silica gel column chromatography).

In step B-2, compound (IV) is reacted with an acid, and cyclization reaction is carried out by reacting compound (IV) with an acid in an inert solvent.

Although not specifically limited provided that it is used as an acid in a conventional reaction, the acid used may be, for example, an inorganic acid such as hydrochloric acid, hydrobromic acid, sulfuric acid, perchloric acid, or phosphoric acid; an organic acid such as acetic acid, formic acid, oxalic acid, methanesulfonic acid, p-toluenesulfonic acid, camphor sulfonic acid, trifluoroacetic acid, or trifluoromethanesulfonic acid; a Lewis acid such as zinc chloride, tin tetrachloride, boron trichloride, boron trifluoride, or boron tribromide; or an acidic ion exchange resin; advantageously an inorganic acid or an organic acid; and optimally hydrochloric acid, acetic acid, or trifluoroacetic acid.

Although not specifically limited provided that it does not damage the reaction and the starting material dissolves to some extent, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as dichloromethane, chloroform, 1,2-dichloroethane, or carbon tetrachloride; an ester such as methyl acetate, ethyl acetate, propyl acetate, butyl acetate, or diethyl carbonate; an ether such as diethyl ether, diisopropyl ether, tetrahydrofuran, dioxane, dimethoxyethane, or diethylene glycol dimethyl ether; an alcohol such as methanol, ethanol, n-propanol, isopropanol, n-butanol, isobutanol, t-butanol, isoamyl alcohol, diethylene glycol, glycerin, octanol, cyclohexanol, or methyl Cellosolve; an amide such as formamide, dimethylformamide, dimethylacetamide, or hexamethylphosphoric acid triamide; an organic acid such as trifluoroacetic acid, acetic acid, or propionic acid; water, or a mixture of these solvents; advantageously an organic acid, an ether or an amide; and optimally acetic acid, dioxane, tetrahydrofuran, or dimethylformamide.

Although differing depending on factors such as the ingredient compounds, the acid and the solvent used, the reaction temperature is usually -20°C to the boiling point of the solvent, and advantageously 0°C to 100°C.
Although differing depending on factors such as the ingredient compounds, the acid, the solvent used, and the reaction temperature, the reaction time is usually fifteen minutes to forty-eight hours, and advantageously thirty minutes to twenty-four hours.

The ingredient compound (III) of this method may be readily produced using the following method.

Of compound (III), C' is a group represented by the expression -C(R^{c1})(COOP¹)-W^{c1}-R^{c2} (where W^{c1}, R^{c1}, and R^{c2} are defined as described earlier, and P¹ indicates a C₁-C₆ alkyl) (the expression -V-R is especially hydrogen). This compound (IIIa) may be a conventional compound, or may be produced following a conventional method (for example, the method described in Japanese Unexamined Patent Publication No. 2002-193948).

Of compound (III), C' is a group represented by the expression COOP¹ (where P¹ is defined as described earlier) (V is especially oxygen). This compound (IIIb) may be a conventional compound, or may be produced following a conventional method (for example, the method described in Japanese Unexamined Patent Publication No. 2004-123711.

Of compound (III), p is 1 and C' is 2,4-dioxo-1,3-thiazolidin-5-yl (the expression -V-R is especially hydrogen). This compound (IIIc) may be a conventional compound, or may be produced following a conventional method (for example, the method described in Japanese Unexamined Patent Publication No. 11-193276).

### <Method C>

Of compound (III), a compound (IIId) in which C' is a group represented by the expression -N(W^{c2})-COOP¹ (where W^{c2} and P¹ are defined as described earlier) may be produced by the following method Ca.

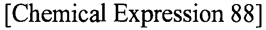

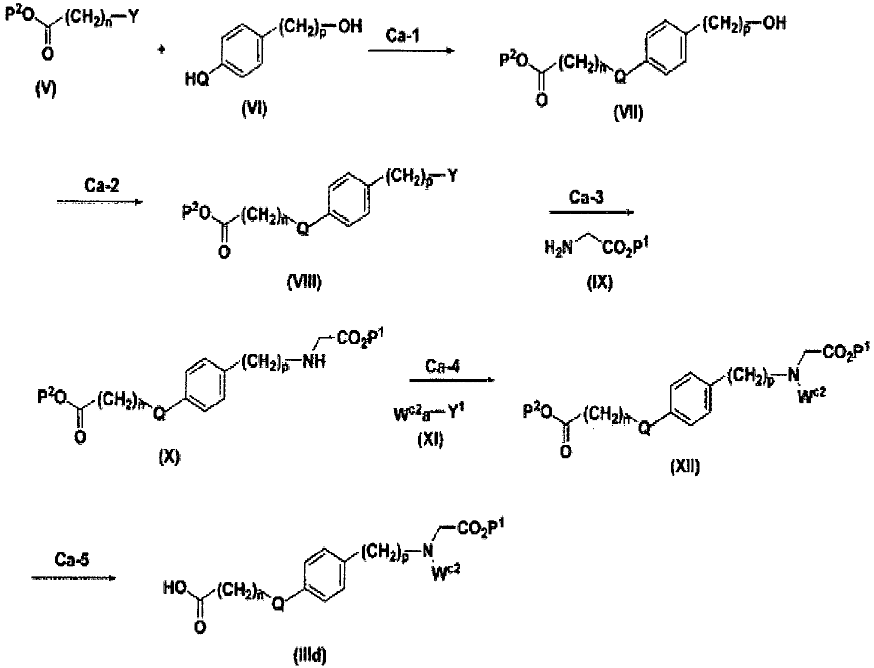

Q, n, p, P¹, and W^{c2} in this expression are defined as described earlier, W^{c2}a indications the same as W^{c2} except for excluding hydrogen, P² indicates a C₁-C₆ alkyl (advantageously methyl, ethyl, or t-butyl, and optimally t-butyl), benzyl, or benzhydryl, Y indicates a C₁-C₆ alkylsulfonyloxy, a C₆-C₁₀ arylsulfonyloxy, or a halogen (advantageously methanesulfonyloxy, ethanesulfonyloxy, benzenesulfonyloxy, toluenesulfonyloxy, chlorine, bromine, or iodine, and optimally methanesulfonyloxy or chlorine), and Y¹ indicates a halogen (advantageously chlorine or bromine).

Step Ca-1 is a step for reacting a compound represented by general formula (V) with a compound represented by general formula (VI) in an inert solvent in the presence of a base to produce a compound represented by general formula (VII).

The base used may be, for example, an alkali metal carbonate such as lithium carbonate, sodium carbonate, or potassium carbonate; an alkali metal bicarbonate such as lithium bicarbonate, sodium bicarbonate, or potassium bicarbonate; an alkali metal hydride such as lithium hydride, sodium hydride, or potassium hydride; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously an alkali metal hydroxide or an alkali metal carbonate; and optimally sodium hydride, potassium carbonate, or cesium carbonate.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously a ketone, an ether, or an amide; and optimally acetone, dioxane, tetrahydrofuran, or dimethylformamide.

Although differing depending on factors such as the ingredient compounds and the base and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually fifteen minutes to ten days, and advantageously thirty minutes to three days.

Step Ca-2 is a step for reacting an alcohol derivative represented by general formula (VII) with a sulfonyl halide (for example, a C₁-C₆ alkyl or C₆-C₁₀ arylsulfonyl halide such as methanesulfonyl chloride, methanesulfonyl bromide, ethanesulfonyl chloride, benzenesulfonyl chloride, or p-toluenesulfonyl chloride, advantageously methanesulfonyl chloride or p-toluenesulfonyl chloride) or a halogenating agent (for example, a thionyl halide such as thionyl chloride or thionyl bromide, a phosphorus halide such as phosphorus tribromide, phosphorus pentabromide, phosphorus pentachloride, or phosphorus oxychloride, or an acid halide such as acetic acid chloride, oxalic acid chloride, or oxalic acid bromide; and advantageously thionyl chloride, thionyl bromide, or oxalic acid chloride) in an inert solvent in the presence or not of a base (advantageously in the presence) to produce a compound represented by general formula (VIII).

The base used may be, for example, an alkali metal carbonate such as lithium carbonate, sodium carbonate, or potassium carbonate; an alkali metal bicarbonate such as lithium bicarbonate, sodium bicarbonate, or potassium bicarbonate; an alkali metal hydride such as lithium hydride, sodium hydride, or potassium hydride; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously an amine or an alkali metal carbonate; and optimally N,N-diisopropyl ethylamine or potassium carbonate.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously a ketone, an ether, a halogenated hydrocarbon, or an amide; and optimally acetone, dioxane, tetrahydrofuran, chloroform, or dichloromethane.

Although differing depending on factors such as the ingredient compounds and the acid and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually one hour to five days, and advantageously thirty minutes to twenty-four hours.

Step Ca-3 is a reaction for reacting compound (VIII) with compound (IX) in an inert solvent in the presence of a base to produce a compound represented by general formula (X).

The base used may be, for example, an alkali metal carbonate such as lithium carbonate, sodium carbonate, potassium carbonate, or cesium carbonate; an alkali metal bicarbonate such as lithium bicarbonate, sodium bicarbonate, or potassium bicarbonate; an alkali metal hydride such as lithium hydride, sodium hydride, or potassium hydride; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously an amine or an alkali metal carbonate; and optimally N,N-diisopropyl ethylamine or potassium carbonate.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously a halogenated hydrocarbon, a ketone, an ether, or an amide; and optimally dichloromethane, acetone, dioxane, tetrahydrofuran, or dimethylformamide.

Although differing depending on factors such as the ingredient compounds and the base and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually thirty minutes to five days, and advantageously thirty minutes to three days.

Step Ca-4 is a step for reacting compound (X) with the compound represented by general formula (XI) in an inert solvent in the presence of a base. The ingredient compound (X) is a compound in which W^{c2} in compound (XII) is hydrogen.
In the case that W^{c2} in compound (XII) is acyl, (W^{c2}a)₂O (a corresponding acid anhydride) or W^{c2}a-Cl (a corresponding acid chloride) may be reacted with compound (X). In the case that W^{c2} in compound (XII) is carbamoyl, a corresponding isocyanate optionally substituted with the substituent α may be reacted with compound (X). The corresponding isocyanate optionally substituted with the substituent α to be reacted with compound (X), however, may not be an isocyanate in which the substituent α is (iii) a C₁-C₆ alkoxy, (iv) a halogen, (v) hydroxy, (vi) cyano, (vii) nitro, (xi) formyl, a C₁-C₆ alkylcarbonyl, or a C₂-C₆ alkenylcarbonyl, (xii) a C₃-C₁₀ cycloalkylcarbonyl, (xiii) a C₆-C₁₀ arylcarbonyl optionally having one to five substituents β, (xiv) a C₆-C₁₀ aryl-C₁-C₆ alkylcarbonyl optionally having in the aryl portion one to five substituents β, (xv) a five- to seven-membered cyclic heteroaryl carbonyl optionally having one to two substituents β and containing one to three heteroatoms selected from among a group consisting of oxygen, nitrogen, and sulfur, (xvi) carbamoyl, (xvii) a C₆-C₁₀ aryl carbamoyl optionally having one to five substituents β, (xviii) amino optionally substituted with one to two substituents β (excluding, however, (ii) a halogen), (xix) a C₁-C₆ halogenoalkoxy, (xx) a C₆-C₁₀ aryloxy optionally having one to five substituents β, (xxi) a C₆-C₁₀ aryl-C₁-C₆ alkoxy optionally having in the aryl portion one to five substituents β, (xxii) a C₁-C₆ alkoxycarbonyl, (xxiii) a C₆-C₁₀ aryloxycarbonyl optionally having in the aryl portion one to five substituents β, or (xxiv) a C₆-C₁₀ aryl-C₁-C₆ alkoxycarbonyl optionally having in the aryl portion one to five substituents β.
These reactions may be carried out using a method described in the literature to be described later.

The base used may be, for example, an alkali metal carbonate such as lithium carbonate, sodium carbonate, potassium carbonate, or cesium; an alkali metal bicarbonate such as lithium bicarbonate, sodium bicarbonate, or potassium bicarbonate; an alkali metal hydride such as lithium hydride, sodium hydride, or potassium hydride; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously an amine or an alkali metal carbonate; and optimally N,N-diisopropyl ethylamine or potassium carbonate.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously a halogenated hydrocarbon, a ketone, an ether, or an amide; and optimally dichloromethane, acetone, dioxane, tetrahydrofuran, or dimethylformamide.

Although differing depending on factors such as the ingredient compounds and the base and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually thirty minutes to five days, and advantageously one hour to three days.

Step Ca-5 is a step for removing the protecting group (P²) from compound (XII) to produce a compound (IIId).
In the case that the protecting group (P²) is a C₁-C₆ alkyl, benzyl, or benzhydryl, the protecting group is removed by reacting compound (XII) with an acid or a base in an inert solvent (advantageously a mixture of water and an organic solvent).
This reaction is carried out in the same manner as (a) the method of reacting with an acid and (b) the method of reacting with a base in method A.

In the case that the protecting group (P²) is t-butyl, the protecting group is removed by reacting compound (XII) with an acid in an inert solvent.
This reaction is carried out in the same manner as (a) the method of reacting with an acid in method A.

In the case that the protecting group (P²) is benzyl or benzhydryl, the protecting group is removed by reacting compound (XII) with hydrogen in an inert solvent in the presence of a contact reducing agent.
This reaction is carried out in the same manner as (c) the contact reduction method in method A.

The intermediary (XII) in method Ca may be produced separately by the following method Cb.

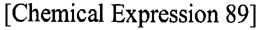

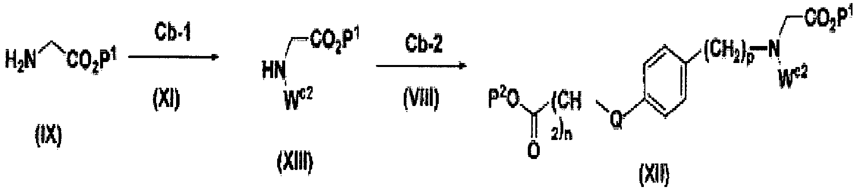

W^{c2}, Q, P¹, P², p, and n in this expression are defined as described earlier.

Step Cb-1 is a step for producing a compound represented by general formula (XIII) by alkylating, for example, compound (IX). This step may be carried out in the same manner as step Ca-4. The ingredient compound (IX) is a compound in which W^{c2} in compound (XIII) is hydrogen.

Step Cb-2 is a step for reacting compound (XIII) with compound (VIII) to produce a compound (XII). This step may be carried out in the same manner as step Ca-3.
Method Cc is a method for separately producing the intermediary (X) in method Ca. 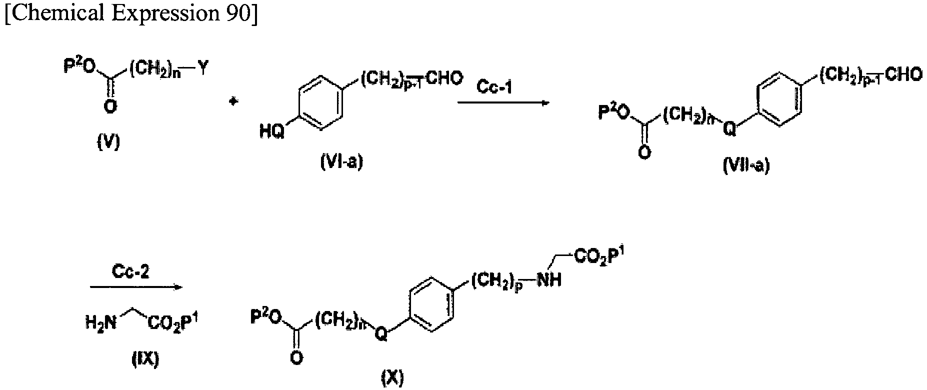 Y, Q, P¹, P², p, and n in this expression are defined as described earlier.
Step Cc-1 is a step for producing a compound represented by general formula (VIIa), and is achieved by reacting compound (V) with the compound represented by general formula (VIa). This step is carried out in the same manner as step Ca-1.
Step Cc2 is a step (reductive alkylation of an amine by an aldehyde) for producing compound (X), and is achieved by reacting compound (VII-a) with compound (IX) in an inert solvent in the presence of a reducing agent.
The reducing agent used may be, for example, sodium borohydride, sodium cyanoborohydride, sodium triacetoxy borohydride, or borane/pyridine; advantageously sodium cyanoborohydride, sodium triacetoxy borohydride, or borane/pyridine; and optimally sodium cyanoborohydride or sodium triacetoxy borohydride.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; an ether such as diethyl ether, diisopropyl ether, tetrahydrofuran, dioxane, dimethoxyethane, or diethylene glycol dimethyl ether; an alcohol such as methanol, ethanol, propanol, or isopropanol; an amide such as formamide, dimethylformamide, dimethylacetamide, or hexamethylphosphoric acid triamide; or a mixture of these solvents; advantageously an alcohol, an ether, or an amide; and optimally methanol, ethanol, or a mixture of methanol and dioxane, tetrahydrofuran, or dimethylformamide.

Although differing depending on factors such as the ingredient compounds and the base and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually fifteen minutes to ten days, and advantageously thirty minutes to three days.

### <Method D>

Method D is a step for producing compound (II).

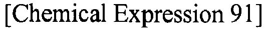

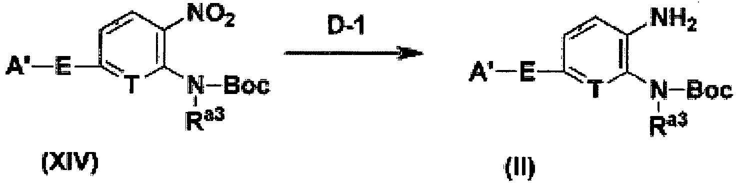

A', E, T, R^{a3}, and Boc in this expression are defined as described earlier. The tetrazole or thiazolidine-2,4-dione in A', however, need not be protected, and in the case that T is nitrogen, the group represented by the expression -N(R^{a3})Boc may be -N(R^{a3})H.

Step D-1 is a step for producing compound (II), and is carried out by reducing the nitro of the compound represented by general formula (XIV) to convert to an amino. Triphenylmethyl, which is a protecting group in A', or benzyloxycarbonyl, which is a protecting group of the amino in compound (XIV), may also be removed simultaneously during this reaction.

This step is a reaction to reduce an aromatic nitro, and is carried out in an inert solvent by a contact reduction reaction, a reduction reaction combining a metal and an acid (for example, iron-hydrochloric acid or sulfuric acid, zinc-acetic acid or hydrochloric acid, tin-alcohol, or tin-hydrochloric acid; advantageously iron-hydrochloric acid, zinc-acetic acid, or tin-hydrochloric acid; and more advantageously iron-hydrochloric acid or zinc-acetic acid), or a reaction with sodium hydrosulfite used in conventional organic reactions.

Although not specifically limited provided that it does not negatively impact this reaction, the inert solvent used for contact reduction is advantageously an alcohol or a mixture of an alcohol and an ether or an amide, and more advantageously methanol or a mixture of methanol and tetrahydrofuran, dioxane, or dimethylformamide; the inert solvent used for reduction by a combining a metal and an acid is advantageously an alcohol (especially methanol or ethanol); and the inert solvent used for a reaction with sodium hydrosulfite is advantageously a mixture of water and an alcohol (especially methanol or ethanol).
The contact reduction catalyst used is the same as that used in method A.
Although differing depending on factors such as the ingredient compounds, the contact reduction catalyst, the reducing agent, and the solvent, the reaction temperature is usually -20°C to 150°C, and advantageously 10°C to 100°C.
Although differing depending on factors such as the ingredient compounds, the contact reduction catalyst, the reducing agent, the solvent, and the reaction temperature, the reaction time is usually thirty minutes to ten days, and advantageously five hours to five days.

### <Method E>

Method Ea is a method for producing compound (XIVa) in which E in compound (XIV) is E' (a group represented by the following expression).

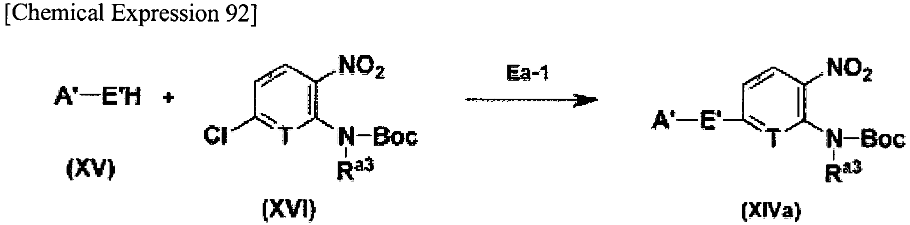

A', T, R^{a3}, and Boc in this expression are defined as described earlier, and E' is defined the same as E except that the terminal which will react with an aromatic ring is a group represented by the expression -O-, -S-, or -N(R^{e2})- (where R^{e2} indicates hydrogen, a C₁-C₆ alkyl, an optionally substituted C₆-C₁₀ aryl, or an optionally substituted C₆-C₁₀ aryl-C₁-C₆ alkyl). The tetrazole or thiazolidine-2,4-yl in A', however, need not be protected, and in the case that T is nitrogen, the group represented by the expression -N(R^{a3})Boc may be -N(R^{a3})H.

Step Ea-1 is a step for producing compound (XIVa), and is carried out by reacting a compound represented by general formula (XV) with a compound represented by general formula (XVI) in an inert solvent in the presence of a base.

Although not specifically limited provided that it is a base used in a conventional organic reaction, the base used may be, for example, an alkali metal carbonate such as lithium carbonate, sodium carbonate, potassium carbonate, or cesium carbonate; an alkali metal bicarbonate such as lithium bicarbonate, sodium bicarbonate, or potassium bicarbonate; an alkali metal hydride such as lithium hydride, sodium hydride, or potassium hydride; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously an organic amine, an alkali metal hydride, or an alkali metal carbonate; and optimally triethylamine, N,N-diisopropyl ethylamine, sodium hydride, potassium carbonate, or cesium carbonate.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously an ether or an amide; and optimally dioxane, tetrahydrofuran, dimethylformamide, or dimethylacetamide.

Although differing depending on factors such as the ingredient compounds and the base and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually thirty minutes to five days, and advantageously one hour to three days.
In the case that R^{e2}a in the resulting compound (XIVa) is hydrogen, as desired, a corresponding acyl halide (acetyl chloride, benzoyl chloride, benzoylcarbonyl chloride, cyclopropylcarbonyl chloride, nicotinoyl chloride, or the like), a corresponding sulfonyl halide (methanesulfonyl chloride, trifluoromethanesulfonyl chloride, benzenesulfonyl chloride, benzoylsulfonyl chloride, or the like), a substituted carbamoyl halide having one to two substituents comprising alkyl, halogen alkyl, cycloalkyl, aryl, and aryl alkyl (N-methylcarbamoyl chloride, N,N-dimethylcarbamoyl chloride, N-phenylcarbamoyl chloride, or the like), or an anhydride of formic acid and acetic acid may be reacted in the same manner as described earlier to a compound in which R^{e2}a is (v) an alkylsulfonyl, (vi) a halogenoalkylsulfonyl, (vii) an optionally substituted arylsulfonyl, (viii) an optionally substituted aryl-alkylsulfonyl, (ix) formyl, an alkylcarbonyl, or an alkenylcarbonyl, (x) an optionally substituted arylcarbonyl, (xi) an optionally substituted aryl-alkylcarbonyl, (xii) a cycloalkylcarbonyl, (xiii) an optionally substituted heteroarylcarbamoyl, or (xiv) a carbamoyl having a substituent ((i) an alkyl, (ii) a halogenoalkyl, (viii) a cycloalkyl, (ix) an optionally substituted aryl, or (x) an optionally substituted aryl-alkyl) in R^{e2}.

Method Eb is a method for producing a compound (XIVb) in which E in compound (XIV) is a group represented by the expression -(CH₂)ₙ₁-W^{c3}a-Ar(R^{e1})-X^{e2}a- (where Ar and R^{e1} are defined as described earlier, W^{c3}a indicates a group represented by the expression -O-, -S-, or -N(R^{e2}a) (where R^{e2}a is defined as described earlier), X^{e2}a is defined the same as X^{e2} except that the terminal which will react with an aromatic ring is a group represented by the expression -O-, -S-, or -N(R^{e2})- (where R^{e2} is defined as described earlier), and n1 indicates an integer from 1 to 10).

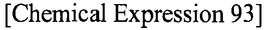

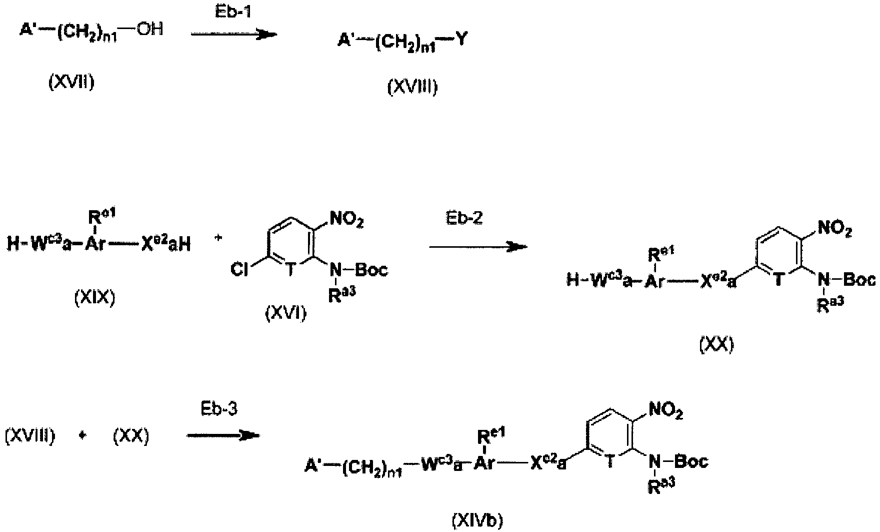

A', n1, W^{c3}a, Ar, R^{e1}, X^{e2}a, T, Y, R^{a3}, and Boc are defined as described earlier.

Step Eb-1 is a step for producing a compound represented by general formula (XVIII), and is achieved by reacting an alcohol represented by general formula (XVII) with a sulfonyl halide or a halogenating agent.
This step is carried out in the same manner as step Ca-2 of method Ca.

Step Eb-2 is a step for producing a compound represented by general formula (XX), and is achieved by reacting a compound represented by general formula (XIX) with compound (XVI). This step is carried out in the same manner as step Ea-1.

Step Eb-3 is a step for producing compound (XIVb), and is achieved by reacting compound (XX) with compound (XVIII) in an inert solvent in the presence of a base.

Although not specifically limited provided that it is a base used in conventional organic reactions, the base used may be, for example, an alkali metal carbonate such as lithium carbonate, sodium carbonate, potassium carbonate, or cesium carbonate; an alkali metal bicarbonate such as lithium bicarbonate, sodium bicarbonate, or potassium bicarbonate; an alkali metal hydride such as lithium hydride, sodium hydride, or potassium hydride; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously N,N-diisopropyl ethylamine, an alkali metal hydride, or an alkali metal carbonate; and optimally sodium hydride, potassium carbonate, or cesium carbonate.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously a halogenated hydrocarbon, an ether, or an amide; and optimally dichloromethane, dioxane, tetrahydrofuran, dimethylformamide, or dimethylacetamide.

Although differing depending on factors such as the ingredient compounds and the base and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually thirty minutes to five days, and advantageously one hour to three days.

Method Ec is a method for producing a compound (XIVc) in which E in compound (XIV) is a group represented by the expression 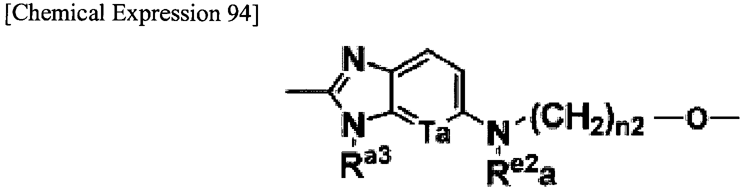 (where R^{a3} and R^{e2}a are defined as described earlier, n2 indicates an integer from 2 to 10, and Ta indicates a group represented by the expression -CH= or -N=).

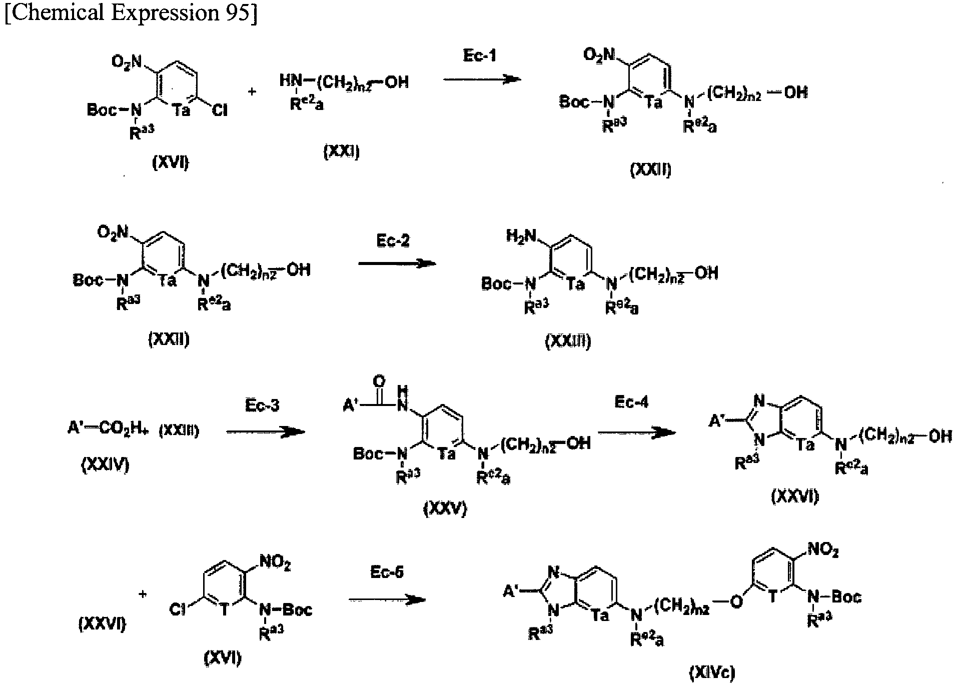

A', R^{a3}, Ta, n2, R^{e2}a, T, and Boc in this expression are defined as described earlier. R^{a3} in compound (XIVc), however, may be the same or different, and in the case that T is nitrogen, the group represented by the expression -N(R^{a3})Boc may be -N(R^{a3})H.

Step Ec-1 is a step for producing a compound represented by general formula (XXII), and is achieved by reacting compound (XVI) with a compound represented by general formula (XXI). This step is carried out in the same manner as step Ea-1.

Step Ec-2 is a step for producing a compound represented by general formula (XXIII), and is achieved by reducing the nitro in compound (XXII). This step is carried out in the same manner as step D-1 of method D.

Step Ec-3 is a step for producing a compound represented by general formula (XXV), and is achieved by reacting a compound represented by general formula (XXIV) with compound (XXIII). This step is carried out in the same manner as step B-1 of method B.

Step Ec-4 is a step for producing a compound represented by general formula (XXVI), and is achieved by removing the Boc in compound (XXV), then cyclizing. This step is carried out in the same manner as step B-2 of method B.

Step Ec-5 is a step for producing compound (XIVc), and is achieved by reacting compound (XXVI) with compound (XVI). This step is carried out in the same manner as step Ea-1.

Compound (XXII) obtained in step Ec-1 in this method may be converted to an ester (for example, an acetic acid ester, a propionic acid ester, or a t-butoxycarboxylic acid ester), and the resulting ester may be provided to the reactions from step Ec-2 to step Ec-4. The ester portion of the resulting compound may then be hydrolyzed in the same manner as step A-1 of method A to produce compound (XXVIc).

Method Ed is a method for producing a compound (XIVd) in which E in compound (XIV) is a group represented by the expression -CONH-W^{c3}b-Ar(R^{e1})-X^{e2}a- (where R^{e1}, Ar, and X^{e2} are defined as described earlier, the group represented by the expression -CONH-W^{c3}bis defined the same as W^{c3} except that the terminal which will bond with A is a group represented by the expression -CONH-).

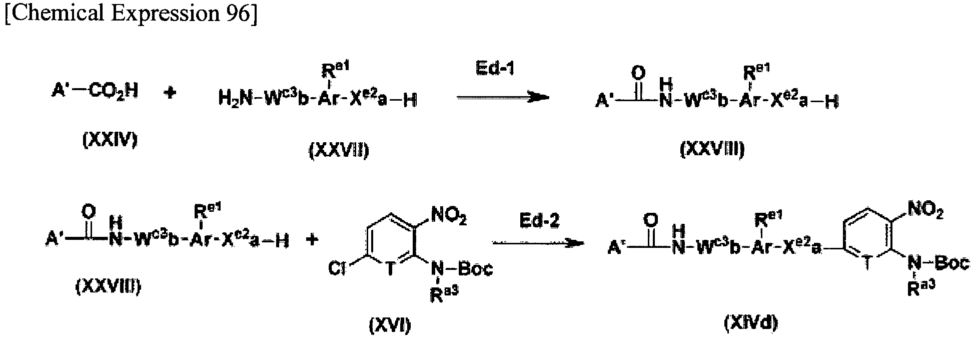

A_{'}, W^{c3}b, Ar, R^{e1}, X^{e2}a, T, R^{a3}, and Boc in this expression are defined as described earlier. The tetrazole or thiazolidin-2,4-dione in A', however, need not be protected, and in the case that T is nitrogen, the group represented by the expression -N(R^{a3})-Boc may be a group represented by the expression -N(R^{a3})-H.

Step Ed-1 is a step for producing a compound represented by general formula (XXVIII), and is achieved by reacting a compound represented by general formula (XXIV) with a compound represented by general formula (XXVII). This step is carried out in the same manner as step B-1 of method B.

Step Ed-2 is a step for producing compound (XIVd), and is achieved by reacting compound (XXVIII) with compound (XVI). This step is carried out in the same manner as step Ea-1.
A compound represented by the expression -CONH- in the group represented by the expression-W^{c3}- is produced in the same manner as this method.
Compound (XIVd) may also be produced by reacting compound (XXVII) with compound (XVI) in the same manner as step Ed-2 to form a compound represented by general formula (XXVIIIa), and reacting this compound with compound (XXIV) in the same manner as step Ed-1.

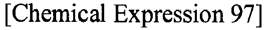

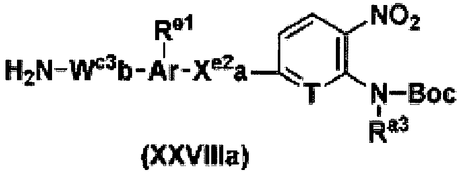

Method Ee is a method for producing a compound (XIVe) in which E in compound (XIV) is a group represented by the expression -N(R^{e2}a)-(CH₂)ₙ₂-O- (where R^{e2} and n2 are defined as described earlier).

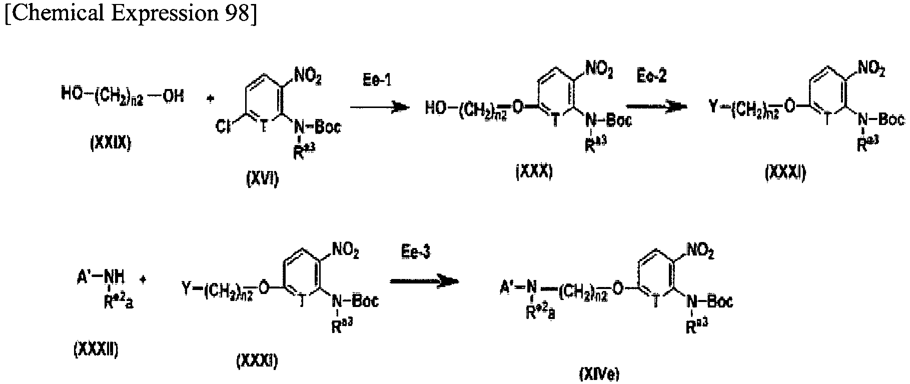

A', R^{e2}a, n2, T, R^{a3}, Boc, and Y in this expression are defined as described earlier. In the case that T is nitrogen, however, the group represented by the expression -N(R^{a3})-Boc may be a group represented by the expression -N(R^{a3})-H.

Step Ee-1 is a step for producing a compound represented by general formula (XXX), and is achieved by reacting a compound represented by general formula (XXIX) with compound (XVI). This step is carried out in the same manner as step Ea-1.

Step Ee-2 is a step for producing a compound represented by general formula (XXXI), and is achieved by reacting an alcohol compound represented by general formula (XXX) with a sulfonyl halide or a halogenating agent. This step is carried out in the same manner as step Ca-2 of method Ca.

Step Ee-3 is a step for producing compound (XIVe), and is achieved by reacting a compound represented by general formula (XXXII) with compound (XXXI). This step is carried out in the same manner as step Ca-3 of method C.

Method Ef is a method for producing a compound (XIVf) in which E in compound (XIV) is a group represented by the expression -(CH₂)ₙ₁-S(O)_{q}-Ar(R^{e1})-O- (where n1, q, Ar, and R^{e1} are defined as described earlier).

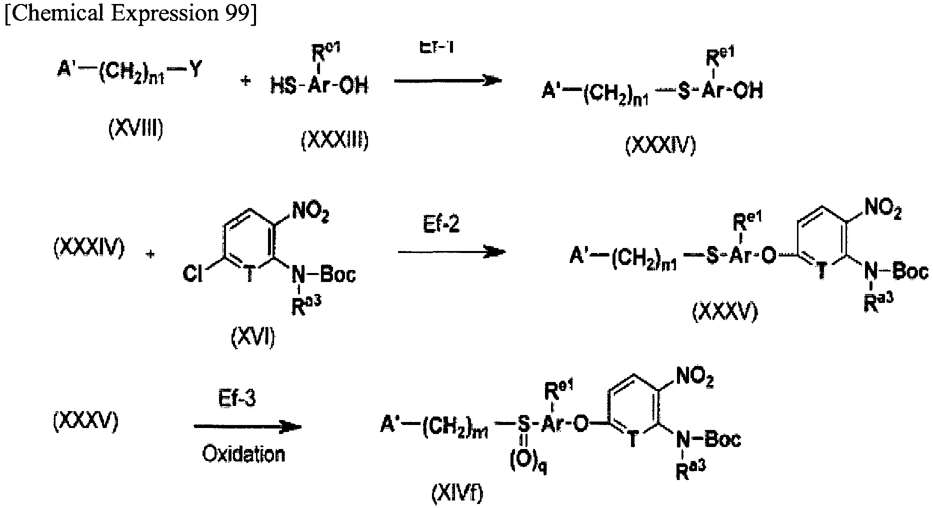

A', n1, q, Ar, R^{e1}, T, R^{a3}, Boc, and Y in this expression are defined as described earlier. In the case that T is nitrogen, however, the group represented by the expression -N(R^{a3})-Boc may be a group represented by the expression -N(R^{a3})-H.

Step Ef-1 is a step for producing a compound represented by general formula (XXXIV), and is achieved by reacting compound (XVIII) with a compound represented by general formula (XXXIII). This step is carried out in the same manner as step Eb-3.

Step Ef-2 is a step for producing a compound represented by general formula (XXXV), and is achieved by reacting a phenol compound (XXXIV) with compound (XVI). This step is carried out in the same manner as step Ea-1.

Step Ef-3 is a step for producing compound (XIVf), and is achieved by reacting a sulfide compound (XXXV) with an oxidizing agent in an inert solvent. The ingredient compound (XXXV) is a compound in which q in compound (XIVf) is 0
The oxidizing agent used may be, for example, a peroxy acid such as m-chloroperbenzoic acid or peracetic acid, a hydroperoxide such as t-butyl hydroperoxide or cumyl hydroperoxide, a dialkyl peroxide such as di-t-butyl peroxide, or hydrogen peroxide; advantageously a peroxy acid, a hydroperoxide, or hydrogen peroxide; and optimally m-chloroperbenzoic acid.

A sulfoxide in which q in compound (XIVf) is 1 may be produced by using one to 1.5 equivalents of the oxidizing agent to the sulfide compound (XXXV), and a sulfone in which q in compound (XIVf) is 2 may be produced by using two to three (two or more) equivalents of the oxidizing agent.

Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; an alcohol such as methanol, ethanol, isopropanol, or butanol; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously a halogenated hydrocarbon; and optimally chloroform, dichloromethane, or 1,2-dichloroethane.

Although differing depending on factors such as the ingredient compounds, the oxidizing agent used, and the solvent used, the reaction temperature is usually -70°C to the boiling point of the solvent, and advantageously -20°C to 50°C.
Although differing depending on factors such as the ingredient compounds, the solvent used, the oxidizing agent used, and the reaction temperature, the reaction time is usually thirty minutes to five days, and advantageously one hour to two days.

Method Eg is a method for producing a compound (XIVg) in which E in compound (XIV) is a group represented by the expression -(CH₂)ₙ₁-S(O)_{q}- (where n1 and q are defined as described earlier).

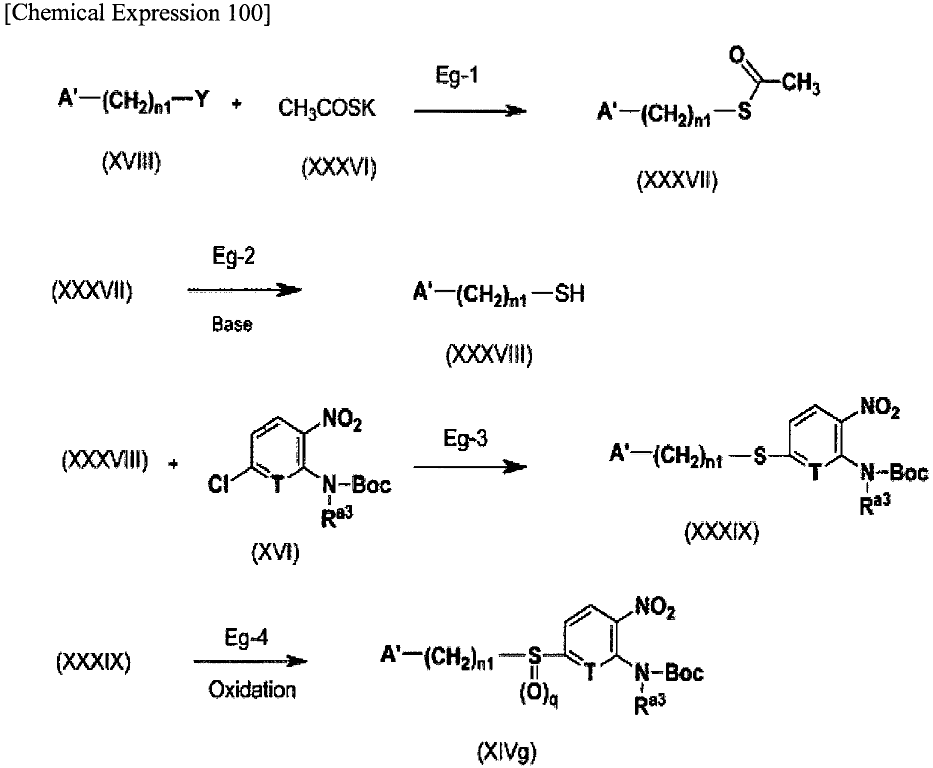

A', n1, q, T, R^{a3}, Boc, and Y in this expression are defined as described earlier. In the case that T is nitrogen, however, the group represented by the expression -N(R^{a3})-Boc may be a group represented by the expression -N(R^{a3})-H.

Step Eg-1 is a step for producing a compound represented by general formula (XXXVII), and is achieved by reacting compound (XVIII) with compound (XXXVI). This step is carried out in the same manner as step Eb-3, advantageously not in the presence of a base.

Step Eg-2 is a step for producing a compound represented by general formula (XXXVIII), and is achieved by degrading compound (XXXVII) in an inert solvent in the presence of a base (catalyst). This step is advantageously carried out also in the presence of dithioerythritol.
Although not specifically limited provided that it is used as a base in a conventional organic reaction, the base (catalyst) used may be, for example, an alkali metal carbonate such as lithium carbonate, sodium carbonate, or potassium carbonate; an alkali metal bicarbonate such as lithium bicarbonate, sodium bicarbonate, or potassium bicarbonate; an alkali metal hydroxide such as lithium hydroxide, sodium hydroxide, or potassium hydroxide; a metal alkoxide such as lithium methoxide, sodium methoxide, sodium ethoxide, or potassium t-butoxide; an organic primary amine such as methylamine, ethylamine, propylamine, isopropylamine, or butylamine; or an organic secondary amine such as diethylamine, dibutylamine, N,N-diisopropylamine, morpholine, piperidine, pyrrolidine, or diethanolamine; advantageously an alkali metal hydroxide, an alkali metal carbonate, or an organic secondary amine; and optimally morpholine or dibutylamine.

Although not specifically limited provided that it is inert in this reaction, the inert solvent used may be, for example, an alcohol such as methanol, ethanol, or propanol; a nitrile such as acetonitrile or propionitrile; an ester such as methyl acetate, ethyl acetate, propyl acetate, butyl acetate, or diethyl carbonate; a halogenated hydrocarbon such as chloroform, dichloromethane, 1,2-dichloroethane, or carbon tetrachloride; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; water; or a mixture of these solvents; advantageously an alcohol, a ketone, an ether, an amide, or a halogenated hydrocarbon; and optimally methanol, ethanol, acetone, dioxane, tetrahydrofuran, dimethylformamide, or dichloromethane.

Although differing depending on factors such as the ingredient compounds and the base and solvent used, the reaction temperature is usually -20°C to the boiling point of the solvent, and advantageously 0°C to 70°C.
Although differing depending on factors such as the ingredient compounds, the base and solvent used, and the reaction temperature, the reaction time is usually one hour to five days, and advantageously thirty minutes or three days.

Step Eg-3 is a step for producing a compound represented by general formula (XXXIX), and is achieved by reacting a mercaptan compound (XXXVIII) with compound (XVI). This step is carried out in the same manner as step Ea-1.

Step Eg-4 is a step for producing compound (XIVg), and is achieved by reacting a sulfide compound (XXXIX) with an oxidizing agnet. This step is carried out in the same manner as step Ef-3.

### <Method F>

Method F is a method for producing the starting materials of method E.
Method Fa is a method for producing the ingredient compound (XVII) for method Eb.

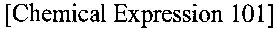

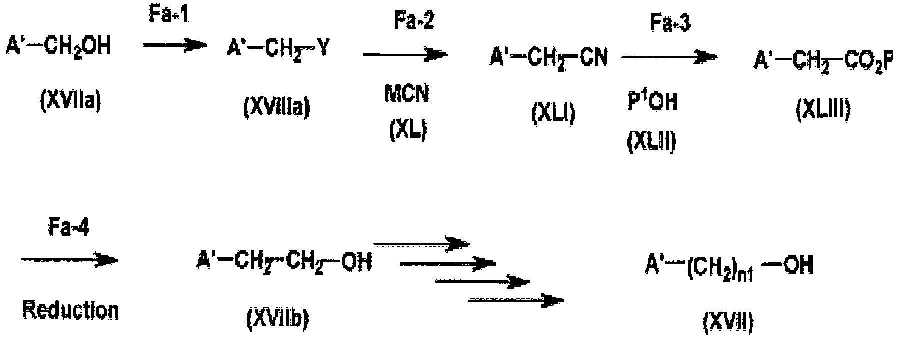

A', n1, and Y in this expression are defined as described earlier, and M indicates an alkali metal. The compound represented by general formula (XVIIa) and the compound represented by general formula (XVIIb) are compound (XVII) in which n1 is 1 (XVIIa) or 2 (XVIIb).

Step Fa-1 is a step for producing a compound represented by general formula (XVIIIa), and is achieved by sulfonylating or halogenating an alcohol compound represented by general formula (XVIIa). This step is carried out in the same manner as step Ca-2 of method Ca.
The ingredient compound (XVIIa) in this step may be produced by reacting compound (XXIV: a carboxylic acid represented by the expression A'-COOH (where A' is defined as described earlier) or a C₁-C₆ alkyl ester thereof with a reducing agent (for example, a metal hydride such as lithium aluminum hydride or diisobutylaluminum hydride) in an inert solvent (for example, an ether such as diethyl ether, diisopropyl ether, tetrahydrofuran, dioxane, dimethoxyethane, or diethylene glycol dimethyl ether) at -20°C to the boiling point of the solvent for thirty minutes to seven hours.

Step Fa-2 is a step for producing a compound represented by general formula (XLI), and is achieved by reacting compound (XVIIIa) with a compound represented by general formula (XL) in an inert solvent in the presence or not of a base.
Although not specifically limited provided that it is a base used in a conventional organic reaction, the base used may be, for example, an alkali metal carbonate such as lithium carbonate, sodium carbonate, potassium carbonate, or cesium carbonate; an alkali metal bicarbonate such as lithium bicarbonate, sodium bicarbonate, or potassium bicarbonate; an alkali metal hydride such as lithium hydride, sodium hydride, or potassium hydride; an alkali metal hydroxide such as lithium hydroxide, sodium hydroxide, or potassium hydroxide; a metal alkoxide such as lithium methoxide, sodium methoxide, sodium ethoxide, or potassium t-butoxide; or an organic amine such as triethylamine, tributylamine, N,N-diisopropyl ethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), or 1,8-diazabicyclo[5.4.0]-7-undecene (DBU); advantageously N,N-diisopropyl ethylamine or an alkali metal carbonate; and optimally potassium carbonate or cesium carbonate.
Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously a halogenated hydrocarbon, an ether, or an amide; and optimally dioxane, tetrahydrofuran, dimethylformamide, or dimethylacetamide.
Although differing depending on factors such as the ingredient compounds and the base and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually thirty minutes to five days, and advantageously one hour to three days.

Step Fa-3 is a step for producing a compound represented by general formula (XLIII), and is achieved by reacting an alcohol compound represented by general formula (XLII) in an inert solvent in the presence of an acid to cause compound (XLI) to degrade by alcoholysis.
The alcohol used is advantageously methanol or ethanol.
Although not specifically limited provided that it is used as an acid in a conventional reaction, the acid used may be, for example, an inorganic acid such as hydrochloric acid, hydrobromic acid, sulfuric acid, perchloric acid, or phosphoric acid; an organic acid such as acetic acid, formic acid, oxalic acid, methanesulfonic acid, p-toluenesulfonic acid, camphor sulfonic acid, trifluoroacetic acid, or trifluoromethanesulfonic acid; a Lewis acid such as zinc chloride, tin tetrachloride, boron trichloride, boron trifluoride, or boron tribromide; or an acidic ion exchange resin; advantageously an inorganic acid or an organic acid; and optimally hydrochloric acid or sulfuric acid
Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; an alcohol such as methanol, ethanol, or propanol; an organic acid such as trifluoroacetic acid, acetic acid, or propionic acid; or a mixture of these solvents; advantageously a halogenated hydrocarbon, an ether, an alcohol, an amide, or a mixture of these solvents; more advantageously an alcohol or an ether; and optimally methanol, ethanol, dioxane, or tetrahydrofuran.
Although differing depending on factors such as the ingredient compounds and the acid and solvent used, the reaction temperature is usually 20°C to 150°C, and advantageously 50°C to 100°C.
Although differing depending on factors such as the ingredient compounds, the acid and solvent used, and the reaction temperature, the reaction time is usually one hour to ten days, and advantageously two hours to five days.

Step Fa-4 is a step for producing a compound represented by general formula (XVIIb), and is achieved by reacting an ester compound (XLIII) with a reducing agent in an inert solvent.
The reducing agent used may be, for example, sodium borohydride, lithium tri(s-butyl)borohydride, lithium triethyl borohydride, sodium bis(2-methoxyethoxy)aluminum hydride (Red-Al), lithium tri-t-butoxyaluminum hydride, lithium aluminum hydride, or diisobutylaluminum hydride; and advantageously Red-Al, lithium aluminum hydride, or diisobutylaluminum hydride.
Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ether such as diethyl ether, diisopropyl ether, tetrahydrofuran, dioxane, dimethoxyethane, or diethylene glycol dimethyl ether; or a mixture of these solvents; advantageously an aromatic hydrocarbon, an ether, or a mixture of these solvents; more advantageously an ether; and optimally diethyl ether, dioxane, or tetrahydrofuran.
Although differing depending on factors such as the ingredient compounds and the reducing agent and solvent used, the reaction temperature is usually -20°C to the boiling point of the solvent, and advantageously 0°C to 100°C.
Although differing depending on factors such as the ingredient compounds, the reducing agent and solvent used, and the reaction temperature, the reaction time is usually thirty minutes to ten days, and advantageously one hour to five days.
Applying steps Fa-1 to Fa-4 using the resulting compound (XVIIb) as a starting material produces a compound in which "-CH₂-" is appended to compound (XLIIb) (A'-CH₂CH₂CH₂-OH), and repeating these steps produces a compound having a desired number of "-CH₂-" appended (that is, a compound (XVII) in which n1 is 4-10).

Compound (XVIIIa) and a compound having a desired number of "-CH₂-" appended to this compound are the ingredient compound (XVIII) of step Ef-1 of method Ef. Reacting compound (XVIII) with a compound represented by the expression N(R^{e2}a)H₂ (where R^{e2}a is defined as described earlier) in the same manner as step Ca-3 of method C produces a compound represented by the expression A'-(CH₂)ₙ₁-(R^{e2}a)H (where A', n1, and R^{e2} are defined as described earlier), and reducing compound (XLI) and a compound having a desired number of "-CH₂-" appended to this compound (for example, contact-reducing in the same manner as step A-1 of method A, or reacting with a reducing agent (for example, lithium aluminum hydride, diisobutylaluminum hydride, or Red-Al) in an inert solvent (for example, diethyl ether, or tetrahydrofuran) at -20°C to the boiling point of the solvent for thirty minutes to ten hours) produces a compound represented by the expression A'-(CH₂)ₙ₁-NH₂ (where A' and n1 are defined as described earlier).
The method of elongating the carbon chain (-CH₂-) one at a time in this method is carried using a conventional method in the literature or a method following such a conventional literature (for example, "Formation of C-C Bonds," Jean Mathieu & Jean Weill-Raynal, Preface by D. H. R. Barton, Georg Thieme Publishers).

Method Fb is another method for producing a compound (XVIIc) in which n1 in compound (XVII) is an odd number.

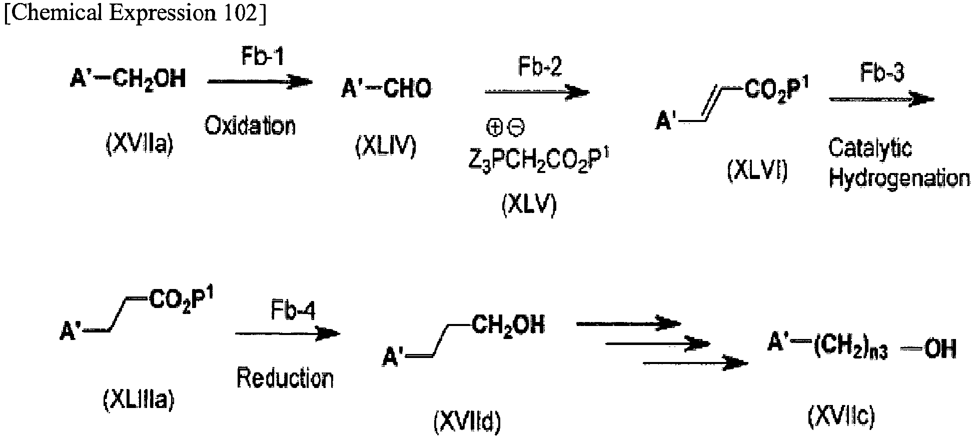

A' and P¹ in this expression are defined as described earlier, n3 indicates an odd-number integer n1 and Z indicates an C₁-C₆ alkyl, a C₆-C₁₀ aryl, or a C₁-C₆ alkoxy (advantageously phenyl, methoxy, or ethoxy).

Step Fb-1 is a step for producing a compound represented by general formula (XLIV), and is achieved by reacting an alcohol compound represented by general formula (XVIIa) with an oxidizing agent in an inert solvent.
Although not specifically limited provided that it oxidizes a primary alcohol compound to an aldehyde compound, the oxidizing agent used may be, for example, anhydrous chromic acid (CrO₃), chromic acid (H₂CrO₄), dichromic acid (H₂Cr₂O₇), pyridine-anhydrous chromic acid complex ((C₅H₅N)₂·CrO₃), pyridinium chlorochromate ((C₅H₅N⁺H)·ClCrO₃⁻), pyridinium dichromate ((C₅H₅N⁺H)²·Cr₂O₇²⁻), pyridine-SO₃-dimethylsulfoxide (DMSO) (Parrikh-Doering oxidation), oxalyl chloride-DMSO, N,N'-dicyclohexylcarbodiimide (DCC)-DMSO (Pfitzner-Moffatt oxidation), trifluoroacetic acid-DMSO (modified Swern oxidation), or acetic anhydride-DMSO (Albright-Goldmann oxidation); and advantageously pyridinium chlorochromate ((C₅H₅N⁺H)·ClCrO₃⁻), pyridinium dichromate ((C₅H₅N⁺H)²·Cr₂O₇²⁻), pyridine-SO₃-DMSO, or oxalyl chloride-DMSO.
The inert solvent used is usually dichloromethane, but is dimethylsulfoxide in the case that pyridine-SO₃ or oxalyl chloride is used.
Although differing depending on factors such as the ingredient compounds, the oxidizing agent used, and the solvent, the reaction temperature is usually -20°C to the boiling point of the solvent, and advantageously 0°C to 50°C.
Although differing depending on factors such as the ingredient compounds, the solvent, and the reaction temperature, the reaction time is usually ten minutes to twenty-four hours, and advantageously thirty minutes to ten hours.

Step Fb-2 is a step for producing a compound represented by general formula (XLVI), and is achieved by reacting compound (XLIV) with a compound represented by general formula (XLV) in an inert solvent.
The inert solvent used is an alcohol, an ether, a nitrile, an amide, or a sulfoxide; advantageously tetrahydrofuran, 1,2-dimethoxyethane, acetonitrile, or dimethylsulfoxide.
Although differing depending on factors such as the ingredient compounds, the base used, and the solvent, the reaction temperature is usually -78°C to the boiling point of the solvent, and advantageously 0°C to 70°C.
Although differing depending on factors such as the ingredient compounds, the solvent, and the reaction temperature, the reaction time is usually thirty minutes to five days, and advantageously one hour to three days.
This step may also be carried out by a conventional method in the literature or a method following such a conventional method (for example, W. S. Wadsworth, Jr., W. D. Emmons, J. Am. Chem. Soc., vol. 83, 1733 (1961)).

Step Fb-3 is a step for producing a compound represented by general formula (XLIIIa), and is achieved by contact-reducing compound (XLVI). This step is carried out in the same manner as step A-1 of method A.

Step Fb-4 is a step for producing a compound represented by general formula (XVIId), and is achieved by reacting compound (XLIIIa) with a reducing agent.
This step is carried out in the same manner as step Fa-4.
Applying steps Fb-1 to Fb-4 using the resulting compound (XVIId) as a starting material produces a compound in which "-CH₂-CH₂-" is appended to compound (XVIId) (A'-CH₂CH₂CH₂CH₂CH₂-OH: compound (XVIIc) in which n3 is 5), and repeating these steps produces a compound in which a desired number of "-CH₂CH₂-" is appended (that is, compound (XVIIc) in which n3 is 7 or 9). Compounds (XVIIa) and (XVIId) are compound (XVIIc) in which n3 is 1 and 3, respectively.

Reacting compound (XLIV) and a compound having a desired number of "-CH₂-CH₂-" appended to this compound with a compound represented by the expression N(R^{e2}a)H₂ (where R^{e2}a is defined as described earlier) under the same conditions as the contact reduction in step A-1 of method A produces a compound represented by the expression A'-(CH₂)ₙ₃-N(R^{e2}a)H (where A', R^{e2}, and n3 are defined as described earlier).

### <Method G>

Method G is a method for producing a compound containing the material compound (XIV) of method D.

Method Ga is a method for producing compound (XIVh) in which E in compound (XIV) is a group represented by the expression -(CH₂)ₙ₁-S-(CH₂)ₙ₄-W^{c3}a-(CH₂)ₙ₅-Ar(R^{e1})-X^{e2}a-(where n1, W^{c3}a, Ar, R^{e1}, and X^{e2} are defined as described earlier, n4 indicates an integer of 2 to 8, and n5 indicates an integer of 1 to 7; provided, however, that the sum of n1, n4, and n5 is not greater than 10).

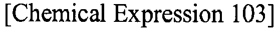

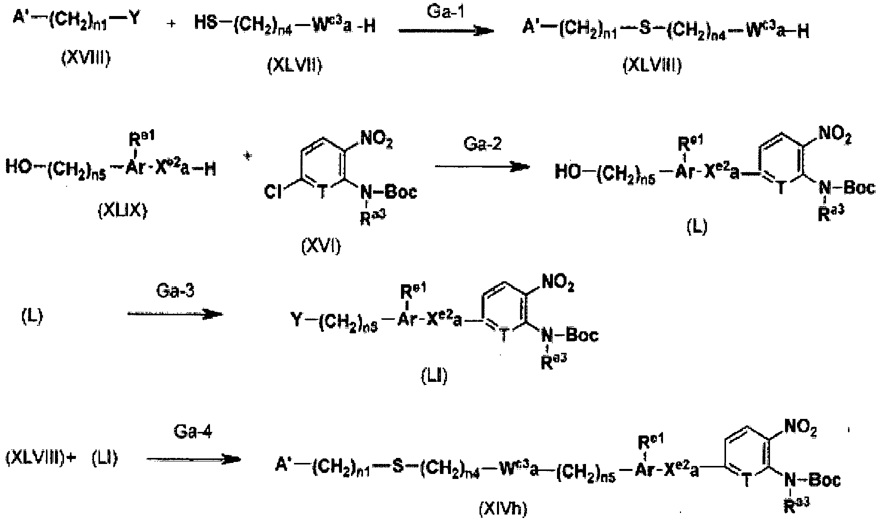

A', n1, n4, W^{c3}a, n5, Ar, R^{e1}, X^{e2}a, T, R^{a3}, Boc, and Y in this expression are defined as described earlier. In the case that T is nitrogen, the group represented by the expression -N(R^{a3})-Boc may be a group represented by the expression -N(R^{a3})-H.

Step Ga-1 is a step for producing a compound represented by general formula (XLVIII), and is achieved by reacting compound (XVIII) with a compound represented by general formula (XLVII). This step is carried out in the same manner as step Eb-3 of method Eb.

Step Ga-2 is a step for producing a compound represented by general formula (L), and is achieved by reacting a compound represented by general formula (XLIX) with compound (XVI). This step is carried out in the same manner as step Ea-1 of method Ea.

Step Ga-3 is a step for producing a compound represented by general formula (LI), and is achieved by sulfonylating or halogenating the alcohol compound (L). This step is carried out in the same manner as step Eb-1 of method Eb.

Step Ga-4 is a step for producing compound (XIVh), and is achieved by reacting compound (XLVIII) with compound (LI). This step is carried out in the same manner as step Eb-3 of method Eb.

Method Gb is a method for producing compound (XIVi) in which E in compound (XIV) is a group represented by the expression -(CH2)ₙ₁-O-(CH₂)ₙ₄-W^{c3}a-(CH₂)ₙ₅-Ar(R^{e1})-X^{e2}a-(where n1, n4, W^{c3}a, n5, Ar, R^{e1}, and X^{e2}a are defined as described earlier; provided, however, that the sum of n1, n4, and n5 is not greater than 10).

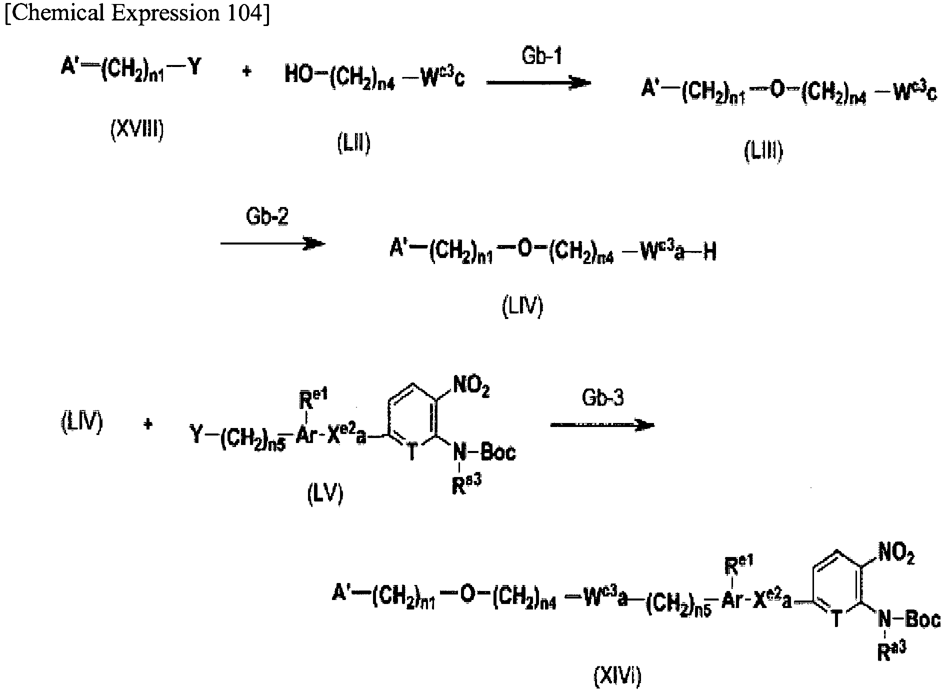

A', n1, n4, W^{c3}ₐ, n5, Ar, R^{e1}, X^{e2}a, R^{e3}, T, Boc, and Y in this expression are defined as described earlier, W^{c3}c indicates a protected oxygen (the protecting group may be, for example, a C₁-C₆ alkylcarbonyl, a C₁-C₆ alkoxycarbonyl, pyranyl, or a C₁-C₆ alkyl-C₆-C₁₀ aryl; and advantageously acetyl, pyranyl, or benzyl), a protected sulfur (protecting group may be, for example, a C₁-C₆ alkylcarbonyl or triphenylmethyl; and advantageously acetyl or triphenylmethyl), or a group represented by the expression -N(R^{e2}a)-Boc (where R^{e2}a and Boc are defined as described earlier). In method, however, the group represented by the expression-N(R^{a3})-Boc may be a group represented by the expression -N(R^{a3})-H.

Step Gb-1 is a step for producing a compound represented by general formula (LIII), and is achieved by reacting compound (XVIII) with a compound represented by general formula (LII). This step is carried out in the same manner as step Eb-3 of method Eb.

Step Gb-2 is a step for producing a compound represented by general formula (LIV), and is achieved by removing the protecting group of oxygen, sulfur, and the group represented by the expression -N(R^{e3}a)-Boc contained in compound (LIII). This step is carried out in the same manner as step A-1 of method A.

Step Gb-3 is a step for producing compound (XIVi), and is achieved by reacting compound (LIV) with compound (LI). This step is carried out in the same manner as step Eb-3 of method Eb.

Method Gc is a method for producing compound (XIVj) in which E in compound (XIV) is a group represented by the expression -(CH₂)ₙ₁-N(R^{e2}a)-(CH₂)ₙ₄-W^{c3}a-(CH₂)ₙ₅-Ar(R^{e1})-X^{e2}a- (where n1, R^{e2}a, n4, W^{c3}a, n5, Ar, R^{e1}, and X^{e2}a are defined as described earlier; provided, however, that the sum of n1, n4, and n5 is not greater than 10).

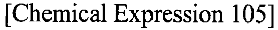

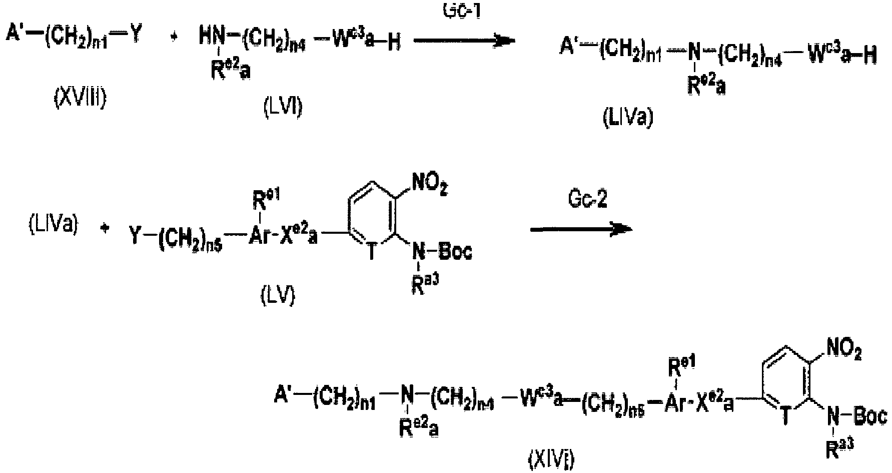

A', n1, R^{e2}a, n4, W^{c3}a, n5, Ar, R^{e1}, X^{e2}a, R^{a3}, T, Boc, and Y are defined as described earlier. In the case that T is nitrogen, however, the group represented by the expression -N(R^{a3})-Boc may be a group represented by the expression -N(R^{a3})-H.

Step Gc-1 is a step for producing a compound represented by general formula (LIVa), and is achieved by reacting compound (XVIII) with a compound represented by general formula (LVI). This step is carried out in the same manner as step Eb-3 of method Eb.
In the case that W^{c3}a is sulfur in this step, the group is advantageously protected by a group represented by the expression -SH (this protecting group is the same as that in method Gb), the protecting group is removed after this reaction in the same manner as step A-1 of method A.

Step Gc-2 is a step for producing compound (XIVj), and is achieved by reacting compound (LIVa) with compound (LI). This step is carried out in the same manner as step Eb-3 of method Eb.

Method Gd is a method for producing compound (XIVk) in which E in compound (XIV) is a group represented by the expression -(CH₂)ₙ₁-W^{c3}a- (CH₂)ₙ₄-CON(R^{e2}a)-(CH₂)ₙ₅-Ar(R^{e1})-X^{e2}a- (where n1, W^{c3}a, n4, R^{e2}a, n5, Ar, R^{e1}, and X^{e2}a are defined as described earlier; provided, however, that the sum of n1, n4, and n5 is not greater than 10).

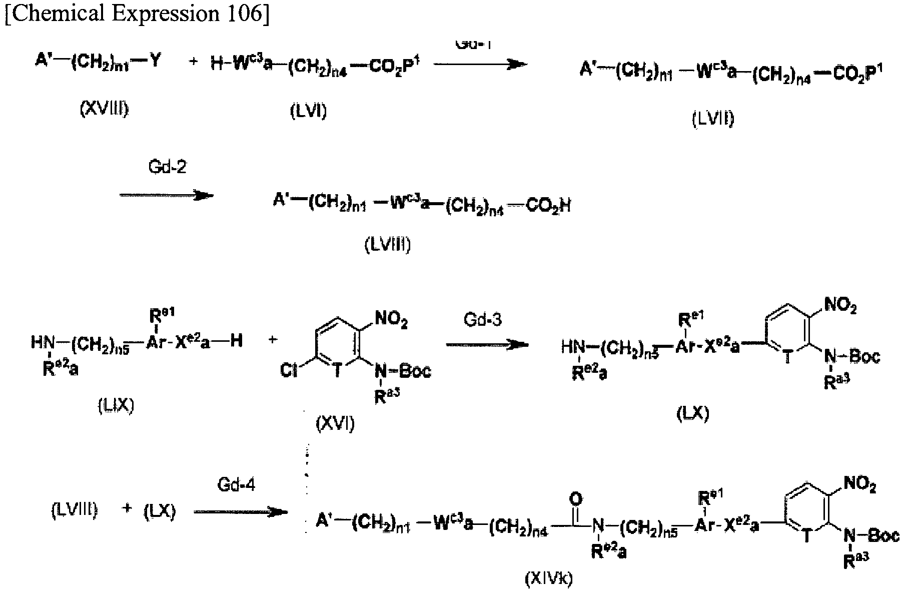

A', n1, W^{c3}a, n4, R^{e2}a, n5, Ar, R^{e1}, X^{e2}a, T, R^{a3}, Boc, Y, and P¹ in this expression are defined as described earlier. The tetrazole or thiazolidin-2,4-dione in A', however, need not be protected, and in the case that T is nitrogen, the group represented by the expression -N(R^{a3})-Boc may be a group represented by the expression -N(R^{a3})-H.

Step Gd-1 is a step for producing a compound represented by general formula (LVII), and is achieved by reacting compound (XVIII) with a compound represented by general formula (LVI). This step is carried out in the same manner as step Eb-3 of method Eb.

Step Gd-2 is a step for producing a compound represented by general formula (LVIII), and is achieved by hydrolyzing the ester compound (LVII). This step is carried out in the same manner as step A-1 of method A.

Step Gd-3 is a step for producing a compound represented by general formula (LX), and is achieved by reacting a compound represented by general formula (LIX) with compound (XVI). This step is carried out in the same manner as step Ea-1 of method Ea.
To avoid reacting the group represented by the expression -NH- contained in compound (LIX) in this step, this group may be protected as desired by a protecting group such as Boc before carrying out the reaction, after which the protecting group may be removed in the same manner as step A-1 of method A to produce compound (LX).

Step Gd-4 is a step for producing compound (XIVk), and is achieved by reacting compound (LX) with compound (LVIII). This step is carried out in the same manner as step B-1 of method B.
Method Ge is a method for producing compound (XIVm) in which E in compound (XIV) is a group represented by the expression -(CH₂)ₙ₁-W^{c3}a- (CH₂)ₙ₄-N(R^{e2}a)-CO-(CH₂)ₙ₅-Ar(R^{e1})-X^{e2}a- (where n1, W^{c3}a, n4, R^{e2}a, n5, Ar, R^{e1}, and X^{e2}a are defined as described earlier; provided, however, that the sum of n1, n4, and n5 is not greater than 10).

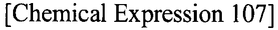

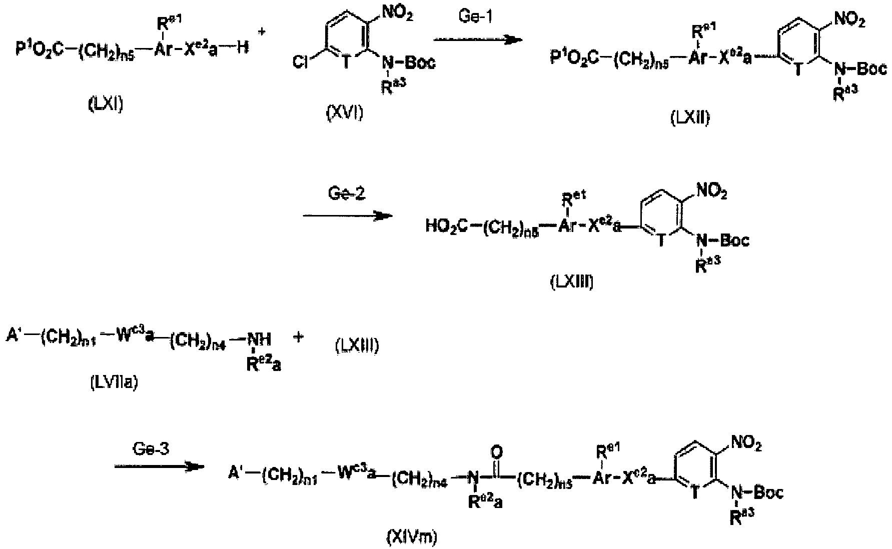

A', n1, W^{c3}a, n4, R^{e2}a, n5, R^{e1}, Ar, X^{e2}a, T, R^{a3}, Boc, and P¹ in this expression are defined as described earlier. The tetrazole or thiazolidin-2,4-dione in A', however, need not be protected, and in the case that T is nitrogen, the group represented by the expression -N(R^{a3})-Boc may be a group represented by the expression -N(R^{a3})-H.

Step Ge-1 is a step for producing a compound represented by general formula (LXII), and is achieved by reacting a compound represented by general formula (LXI) with compound (XVI). This step is carried out in the same manner as step Ea-1 of method Ea.

Step Ge-2 is a step for producing a compound represented by general formula (LXIII), and is achieved by hydrolyzing the ester compound (LXII). This step is carried out in the same manner as step A-1 of method A.

Step Ge-3 is a step for producing compound (XIVm), and is achieved by reacting a compound represented by general formula (LVIIa) with compound (LXIII). This step is carried out in the same manner as step B-1 of method B.
Method Gf is a method for producing compound (XIVn) in which E in compound (XIV) is a group represented by the expression -(CH₂)ₙ₁- N(R^{e2}a)CO-(CH₂)ₙ₄-W^{c3}a-(CH₂)ₙ₅-Ar(R^{e1})-X^{e2}a- (where n1, R^{e2}a, n4, W^{c3}a, n5, Ar, R^{e1}, and X^{e2}a are defined as described earlier; provided, however, that the sum of n1, n4, and n5 is not greater than 10).

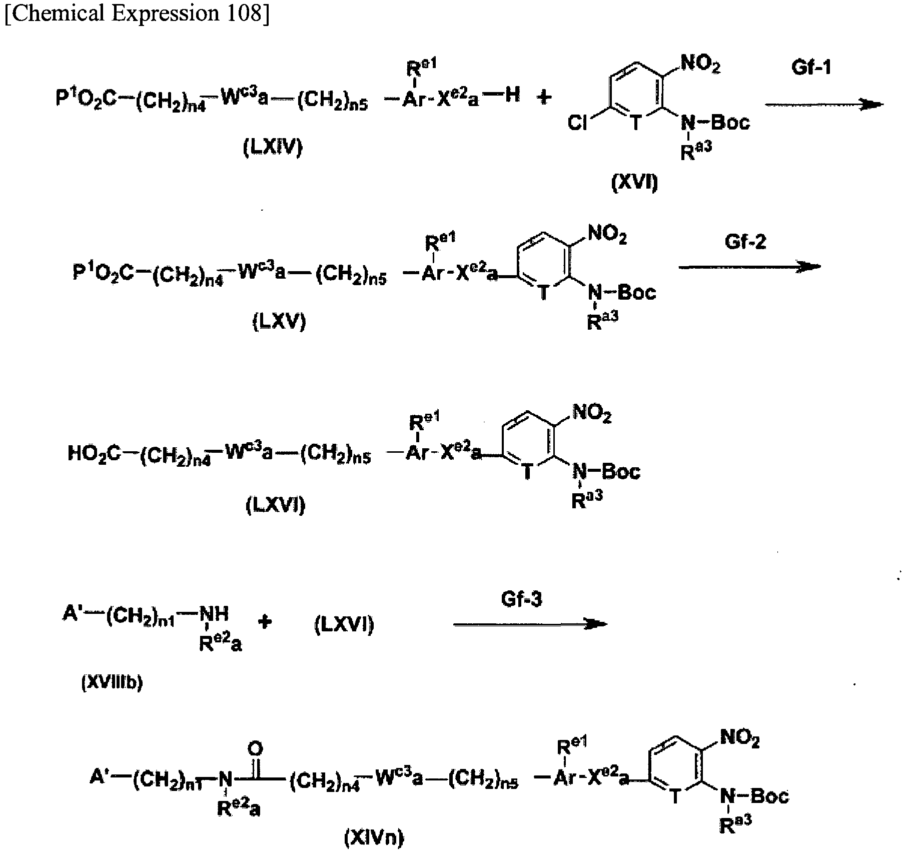

A', n1, R^{e2}a, n4, W^{c3}a, n5, Ar, R^{e1}, X^{e2}a, T, R^{a3}, Boc, and P¹ in this expression are defined as described earlier. The tetrazole or thiazolidin-2,4-dione in A', however, need not be protected, and in the case that T is nitrogen, the group represented by the expression -N(R^{a3})-Boc may be a group represented by the expression -N(R^{a3})-H.

Step Gf-1 is a step for producing a compound represented by general formula (LXV), and is achieved by reacting a compound represented by general formula (LXIV) with compound (XVI). This step is carried out in the same manner as step Ea-1 of method Ea.

Step Gf-2 is a step for producing a compound represented by general formula (LXVI), and is achieved by hydrolyzing the ester compound (LXV). This step is carried out in the same manner as step A-1 of method A.

Step Gf-3 is a step for producing compound (XIVn), and is achieved by reacting compound represented by general formula (XVIIIb) with compound (LXVI). This step is carried out in the same manner as step B-1 of method B.
Method Gg is a method for producing compound (XIVo) in which E in compound (XIV) is a group represented by the expression -(CH₂)ₙ₁- CON(R^{e2}a)-(CH₂)ₙ₄-W^{c3}a-(CH₂)ₙ₅-Ar(R^{e1})-X^{e2}a- (where n1, R^{e2}a, n4, W^{c3}a, n5, Ar, R^{e1}, and X^{e2}a are defined as described earlier; provided, however, that the sum of n1, n4, and n5 is not greater than 10).

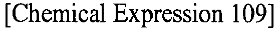

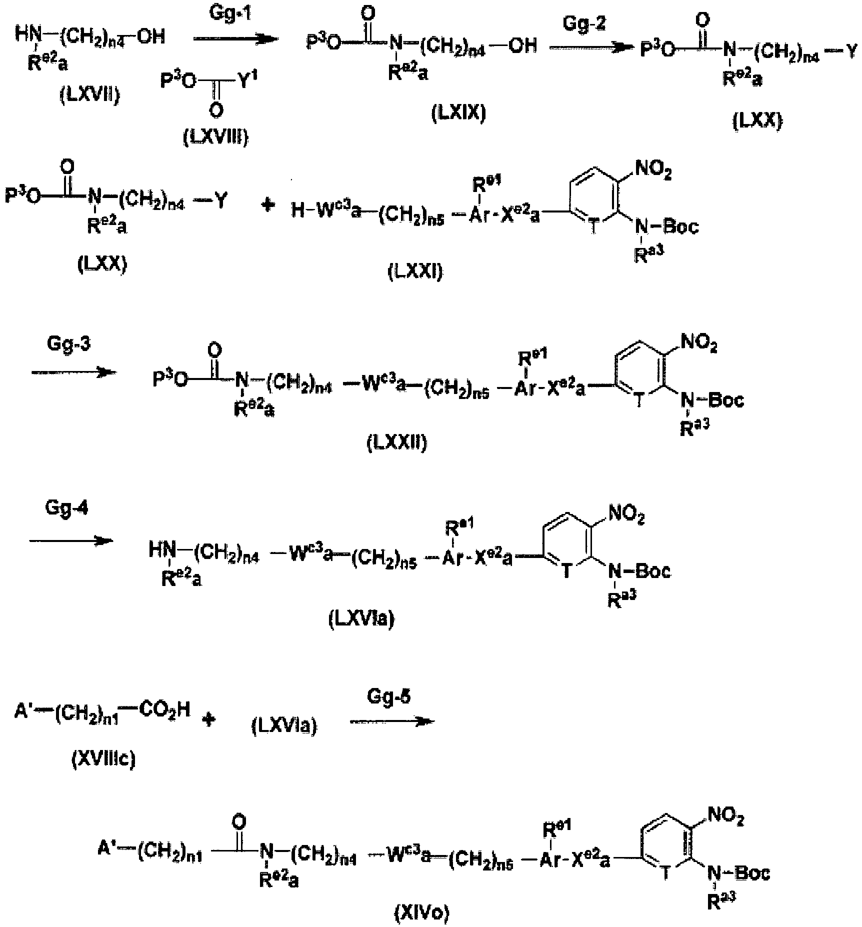

A', n1, R^{e2}a, n4, W^{c3}a, Ar, n5, R^{e1}, X^{e2}a, T, R^{a3}, Boc, Y and Y¹ in this expression are defined as described earlier, and P³ indicates a C₆-C₁₀ aryl (advantageously phenyl). The tetrazole or thiazolidin-2,4-dione in A', however, need not be protected, the sum of n1, n4, and n5 may not exceed an integer of 10, and in the case that T is nitrogen, the group represented by the expression -N(R^{a3})-Boc may be a group represented by the expression -N(R^{a3})-H.

Step Gg-1 is a step for producing a compound represented by general formula (LXIX), and is achieved by reacting a compound represented by general formula (LXVII) with a compound represented by general formula (LXVIII). This step is carried out in the same manner as step B-1 of method B. Y¹ is advantageously chlorine.

Step Gg-2 is a step for producing a compound represented by general formula (LXX), and is achieved by sulfonylating or halogenating the alcohol compound (LXIX). This step is carried out in the same manner as step Ca-2 of method Ca.

Step Gg-3 is a step for producing a compound represented by general formula (LXXII), and is achieved by reacting compound (LXX) with a compound represented by general formula (LXXI). This step is carried out in the same manner as step Eb-3 of method Eb.
Compound (LXXI) may be produced by reacting a compound represented by general formula H-W^{c3}a-(CH₂)ₙ₅-Ar(R^{e1})-X^{e2}a-H (where W^{c3}a, n5, Ar, R^{e1}, and X^{e2}a are defined as described earlier) with compound (XVI) in the same manner as step Ea-1 of method Ea.

Step Gg-4 is a step for producing a compound represented by general formula (LXVIa), and is achieved by hydrolyzing the aryl carbamite compound (LXXII) in the presence of a base. This step is carried out in the same manner as step A-1 of method A.

Step Gg-5 is a step for producing compound (XIVo), and is achieved by reacting a compound represented by general formula (XVIIIc) with compound (LXVIa). This step is carried out in the same manner as step B-1 of method B.

Method Gh is a method for producing compound (XIVp) in which E in compound (XIV) is a group represented by the expression 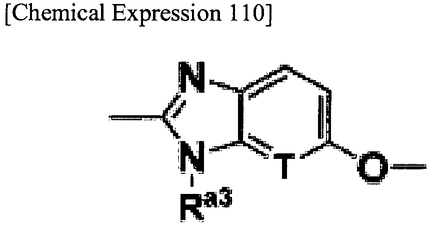 (where R^{a3} and T are defined as described earlier) (a group bonding A to a portion of an imidazole ring). 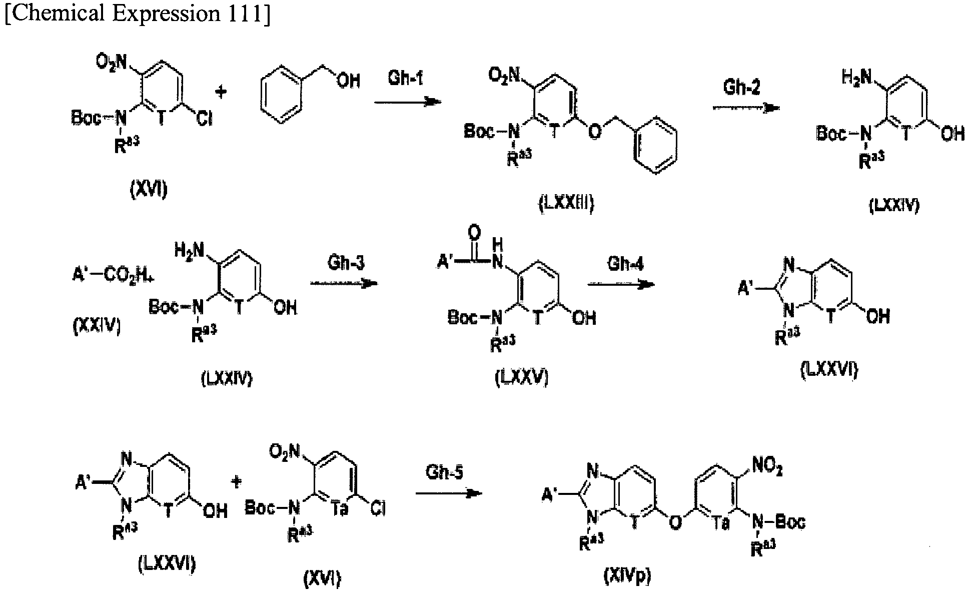 A', R^{a3}, T, Boc, and Ta in this expression are defined as described earlier. Two R^{a3} in the same molecule, however, may be the same or different, and in the case that T and Ta are nitrogen, the group represented by the expression -N(R^{a3})-Boc may be a group represented by the expression -N(R^{a3})-H.

Step Gh-1 is a step for producing a compound represented by general formula (LXXIII), and is achieved by reacting compound (XVI) with benzyl alcohol. This step is carried out in the same manner as step Ea-1 of method Ea.

Step Gh-2 is a step for producing a compound represented by general formula (LXXIV), and is achieved by reducing the compound (LXXIII). This step is carried out in the same manner as step D-1 of method D.

Step Gh-3 is a step for producing a compound represented by general formula (LXXV), and is achieved by reacting compound (XXIV) with compound (LXXIV). This step is carried out in the same manner as step B-1 of method B.

Step Gh-4 is a step for producing a compound represented by general formula (LXXVI), and is achieved by reacting compound (LXXV) with an acid. This step is carried out in the same manner as step B-2 of method B.

Step Gh-5 is a step for producing compound (XIVp), and is achieved by reacting compound (LXXVI) with compound (XVI). This step is carried out in the same manner as step Ea-1 of method Ea.

Instead of benzyl alcohol in step Gh-1, compound (LXVII) may be used to produce compound (XIVq) represented by the expression

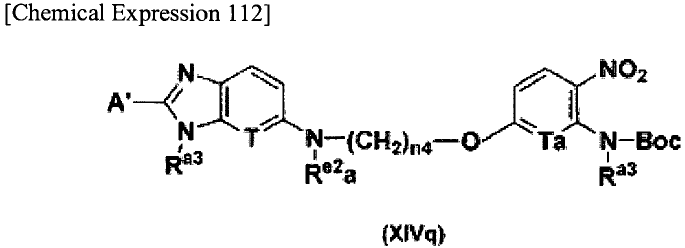

(where A', R^{a3}, T, R^{e2}a, n4, Ta, and Boc are defined as described earlier, and the two R^{a3} may be the same or different) by applying steps Gh-1 to Gh-5.

Instead of compound (XXIV), a compound produced in the same manner as described earlier using compound (XXIVa) represented by the expression A'aCO₂H (where A'a indicates the expression 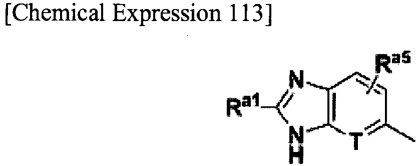 (where R^{a1}, R^{a5}, and T are defined as described earlier) - that is, compounds (XIVpa) and (XIVqa) in which A' in compounds (XIVp) and (XIVq) is A'a - may be reacted with compound (LXXVII) represented by the expression 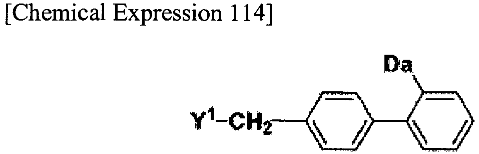 (where Y¹ is defined as described earlier, and Da indicates a C₁-C₆ alkoxycarbonyl or 1-triphenylmethyl-1H-tetrazol-5-yl)
(advantageously 4'-bromomethylbiphenyl-2-carboxylic acid C₁-C₆ alkyl ester or 4'-chloromethylbephenyl-2-carboxylic acid C₁-C₆ alkyl ester (the C₁-C₆ alkyl ester is advantageously methyl ester or t-butyl ester), 4-chloromethyl-2'-(1-triphenylmethyl-1H-tetrazol-5-yl)biphenyl, or 4-bromomethyl-2'-(1-triphenylmethyl-1H-tetrazol-5-yl)biphenyl) in the same manner as step Ca-4 of method Ca to produce a compound in which A' in compounds (XIVp) and (XIVq) is the expression 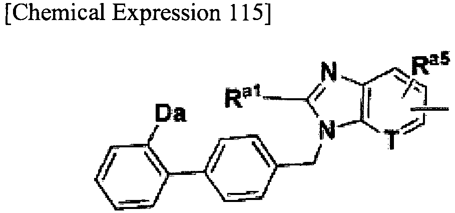 (where R^{a1,} R^{a5}, Da, and T are defined as described earlier).

### <Method H>

Method H is a method for producing compounds contained in compound (I').
Method Ha a method for producing compound (I'a) in which a group represented by the expression -E-B-G- in compound (I') is a group represented by the expression 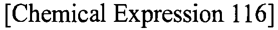 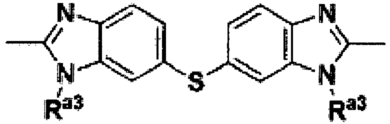 (where R^{a3} is defined as described earlier, and the two R^{a3} may be the same or different) (a group in which E is bonded to B and a portion of a benzene ring, and G is a dangling bond).

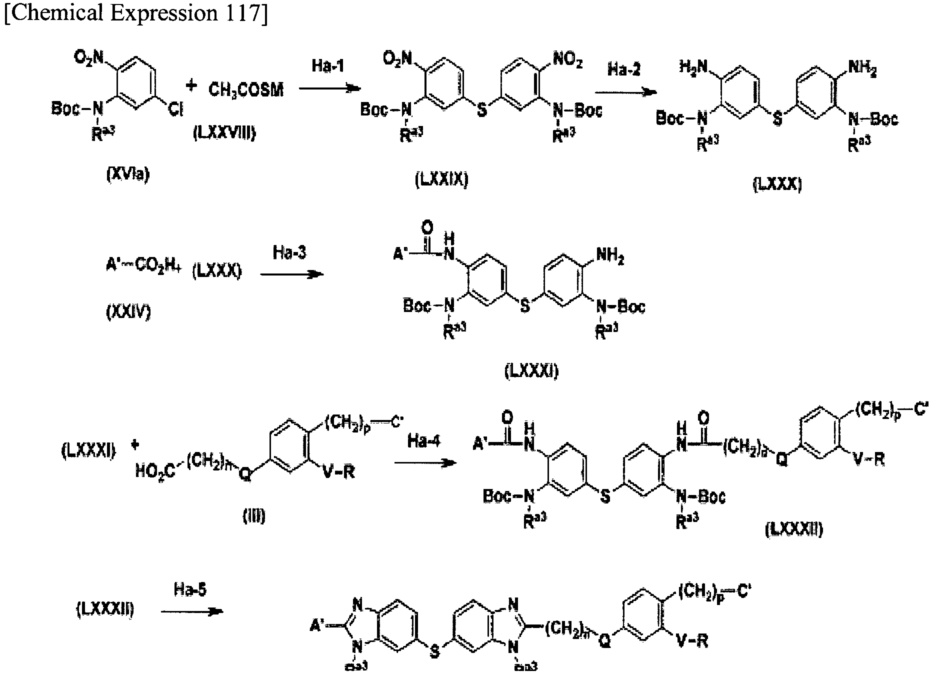

A', R^{a3}, n, Q, V, R, p, C', Boc, and M in this expression are defined as described earlier. The tetrazole or thiazolidine-2,4-dione in A' and C', however, need not be protected.

Step Ha-1 is a step for producing a compound represented by general formula (LXXIX), and is achieved by reacting compound (XVIa) with a compound represented by general formula (LXXVIII) (M is advantageously potassium).
Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously an ether or an amide; and optimally dimethylformamide or dimethylacetamide.
Although differing depending on factors such as the ingredient compounds and the solvent used, the reaction temperature is usually 0°C to the boiling point of the solvent, and advantageously 20°C to 100°C.

Although differing depending on factors such as the ingredient compounds, the solvent used, and the reaction temperature, the reaction time is usually thirty minutes to five days, and advantageously one hour to two days.

Step Ha-2 is a step for producing a compound represented by general formula (LXXX), and is achieved by reducing the nitro in compound (LXXIX).
This step is carried out in the same manner as step D-1 of method D.

Step Ha-3 is a step for producing a compound represented by general formula (LXXXI), and is achieved by reacting compound (XXIV) with compound (LXXX). This step is carried out in the same manner as step B-1 of method B.
Step Ha-4 is a step for producing a compound represented by general formula (LXXXII), and is achieved by reacting compound (III) with compound (LXXXI). This step is carried out in the same manner as step B-1 of method B.
Step Ha-5 is a step for producing compound (I'a), and is achieved by reacting compound (LXXXII) with an acid. This step is carried out in the same manner as step B-2 of method B.
Intermediary (LXXXIII) is obtained by selecting the type of acid, the reaction temperature, and the reaction time (for example, reacting at 30°C to 100°C for one to ten hours using acetic acid as the acid).

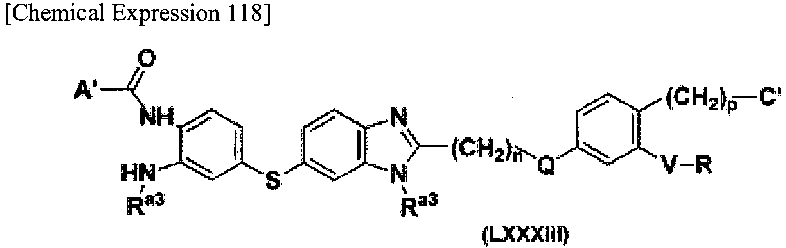

Method Hb is a method for producing compound (I'b) in which the group represented by the expression -E-B- in compound (I') is a group represented by the expression 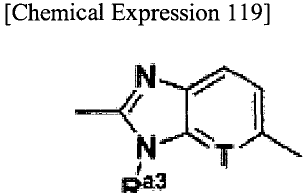 (where R^{a3} and T are defined as described earlier) (a group in which E is a dangling bond, and B bonds to a portion of an imidazole ring). 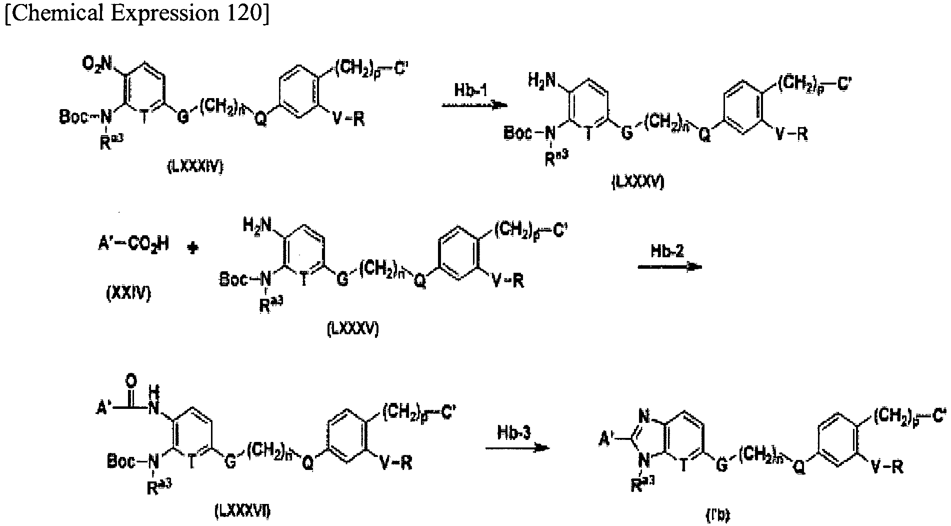 A', R^{a3}, T, G, n, Q, V, R, p, C', and Boc in this expression are defined as described earlier. The tetrazole or thiazolidine-2,4-dione in A' and C', however, need not be protected, and in the case that T is nitrogen, the group represented by the expression -N(R^{a3})-Boc may be a group represented by the expression -N(R^{a3})-H.

Step Hb-1 is a step for producing a compound represented by general formula (LXXXV), and is achieved by reducing the nitro of a compound represented by general formula (LXXXIV). This step is carried out in the same manner as step D-1 of method D.

Step Hb-2 is a step for producing compound represented by general formula (LXXXVI), and is achieved by reacting compound (XXIV) with compound (LXXXV). This step is carried out in the same manner as step B-1 of method B.

Step Hb-3 is a step for producing compound (I'b), and is achieved by reacting compound (LXXXVI) with an acid. This step is carried out in the same manner as step B-2 of method B.

### <Method I>

Method I is a method for producing compound (LXXXIV), which is an ingredient in method Hb.
Method Ia is a method for producing compound (LXXXIV)

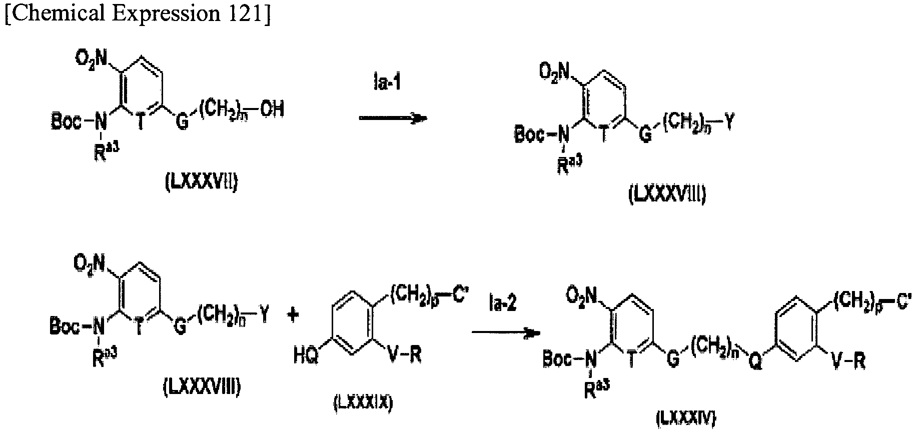

R^{a3}, Boc, T, G, n, Q, V, R, p, C', and Y in this expression are defined as described earlier. In the case that T is nitrogen, however, the group represented by the expression -N(R^{a3})-Boc may be a group represented by the expression -N(R^{a3})-H.

Step Ia-1 is a step for producing a compound represented by general formula (LXXXVIII), and is achieved by sulfonylating or halogenating an alcohol compound represented by general formula (LXXXVII). This step is carried out in the same manner as step Ca-2 of method C.

Step Ia-2 is a step for producing compound (LXXXIV), and is achieved by reacting compound (LXXXVIII) with a compound represented by general formula (LXXXIX). This step is carried out in the same manner as step Ca-1 of method C.
Method Ib is a method for producing compound (LXXXIVa) in which G in compound (LXXXIV) is Ga (Ga indicates oxygen, sulfur, or a group represented by the expression - N(R^{e2}a)- (where R^{e2}a is defined as described earlier). 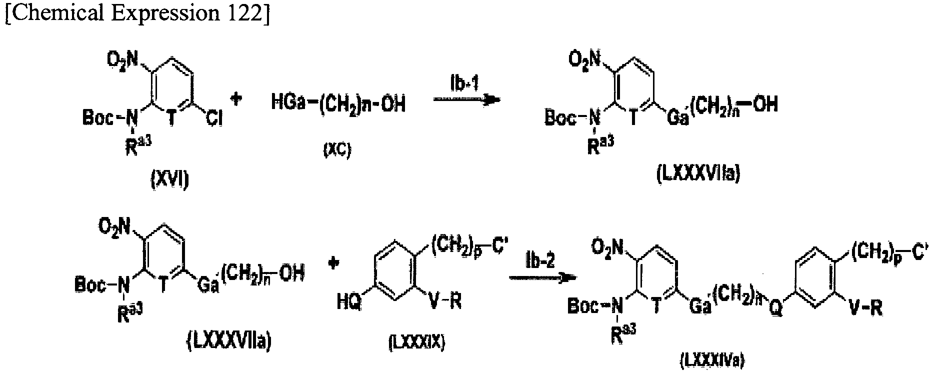 R^{a3}, Boc, T, Ga, n, Q, V, R, p, and C' in this expression are defined as described earlier. In the case that T is nitrogen, however, the group represented by the expression -N(R^{a3})-Boc may be a group represented by the expression -N(R^{a3})-H.
Step Ib-1 is a step for producing a compound represented by general formula (LXXXVIIa), and is achieved by reacting compound (XVI) with a compound represented by general formula (XC).
This step is carried out in the same manner as step Ea-1 of method Ea.

Step Ib-2 is a step for producing compound (LXXXIVa), and is achieved by reacting compound (LXXXVIIa) with compound (LXXXIX).
This step is carried out in the same manner as step If-1 of method If.
Method Ic is another method for producing compound (LXXXIVa). 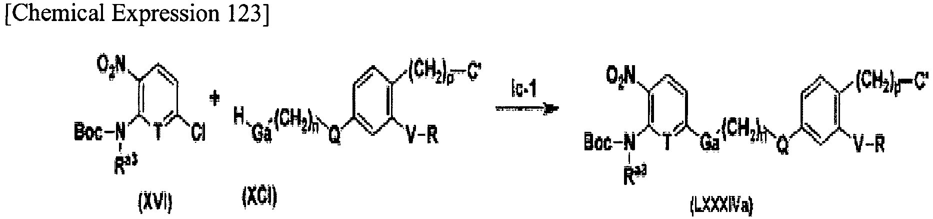 R^{a3}, Boc, T, Ga, n, Q, V, R, p, and C' in this expression are defined as described earlier. The thiazolidine-2,4-dione in C', however, need not be protected, and in the case that T is nitrogen, the group represented by the expression -N(R^{a3})-Boc may be a group represented by the expression -N(R^{a3})-H.
Step Ic-1 is a step for producing compound (LXXXIVa), and is achieved by reacting compound (XVI) with a compound represented by general formula (XCI).
This step is carried out in the same manner as step Ea-1 of method Ea.
Method Id is a method for producing compound (LXXXIVb) in which G in compound (LXXXIV) is a dangling bond. 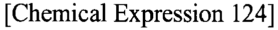 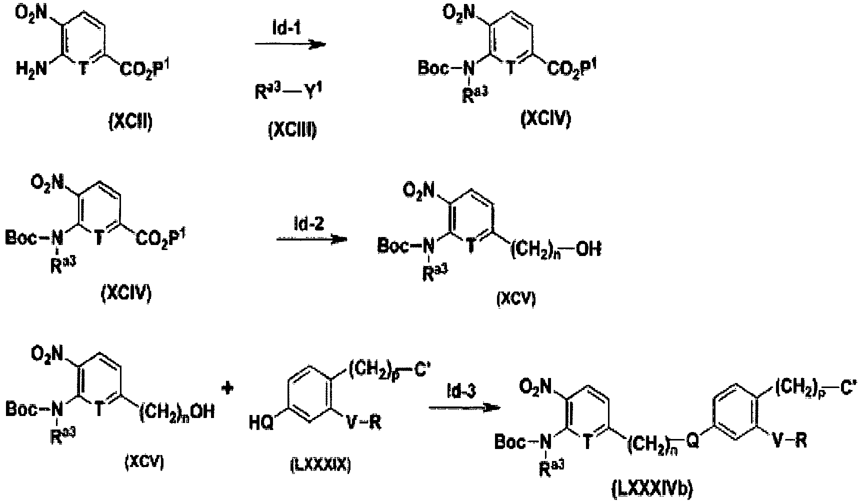 R^{a3}, Boc, T, n, Q, V, R, p, C', P¹, and Y¹ in this expression are defined as described earlier. In the case that T is nitrogen, however, the group represented by the expression -N(R^{a3})-Boc may be a group represented by the expression -N(R^{a3})-H.
Step Id-1 is a step for producing a compound represented by general formula (XCIV), and is achieved by sequentially reacting a compound represented by general formula (XCII) with a t-butoxycarbonyl halide (advantageously a chloride or bromide) or di-t-butyl dicarbonate and a compound represented by general formula (XCIII).
This step is carried out in the same manner as step Ca-3 of method Ca.
In the case that T is nitrogen, reacting with t-butoxycarbonyl halide or di-t-butyl dicarbonate may be omitted.

Step Id-2 is a step for producing a compound represented by general formula (XCV), and is achieved by reducing compound (XCIV) and carrying out a series of carburation reactions.
This step is carried out in the same manner as the steps of method Fa.

Step Id-3 is a step for producing a compound represented by general formula (LXXXIVb), and is achieved by reacting compound (XCV) with a compound represented by general formula (LXXXIX).
This step is carried out in the same manner as step If-1 of method If.

Method Ie is another method for producing compound (LXXXIVb). 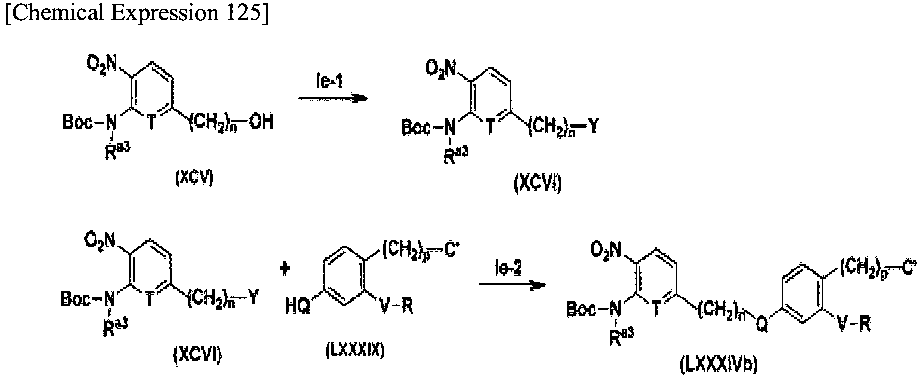 R^{a3}, Boc, T, n, Q, V, R, p, C', and Y in this expression are defined as described earlier. In the case that T is nitrogen, however, the group represented by the expression -N(R^{a3})-Boc may be a group represented by the expression -N(R^{a3})-H.
Step Ie-1 is a step for producing a compound represented by general formula (XCVI), and is achieved by sulfonylating or halogenating compound (XCV).
This step is carried out in the same manner as step Ca-2 of method Ca.
Step Ie-2 is a step for producing compound (LXXXIVb), and is achieved by reacting compound (XCVI) with compound (LXXXIX).
This step is carried out in the same manner as step Ca-1 of method Ca.

Method If is a method for producing compound (XCIc). 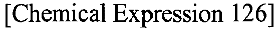 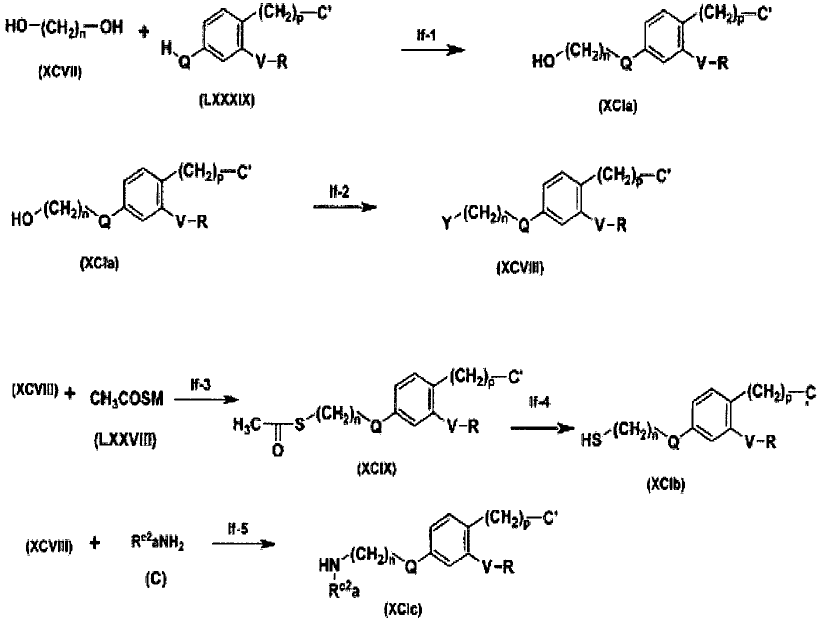 R^{e2}a, n, Q, V, R, p, C', Y, and M in this expression are defined as described earlier.

Step If-1 is a step for producing a compound represented by general formula (XCIa) (a compound in which Ga in compound (XCI) is oxygen), and is achieved by reacting a compound represented by general formula (XCVII) with a compound represented by general formula (LXXXIX) in an inert solvent in the presence of a condensing agent.
Although not specifically limited provided it is used in a so-called Mitsunobu reaction (Bull. Chem. Soc. Jpn., vol. 40, pages 935-939 (1967), Bull. Chem. Soc. Jpn., vol. 40, pages 2380-2382 (1967)), the condensing agent used may be, for example, an azodicarboxylic acid diester such as dimethyl azodicarboxylate, diethyl azodicarboxylate, diisopropyl azodicarboxylate, or bis(2-methoxyethyl)azodicarboxylate; a combination of an azodicarboxylic acid diamide such as 1,1'-azobis(N,N-dimethylformamide) or 1,1'-(azodicarbonyl)dipiperidine and phosphine such as triphenylphosphine, dicyclohexylphenylphosphine, diethylphenylphosphine, 4-(dimethylamino)phenyldiphenylphosphine, diphenyl-2-pyridylphosphine, tributylphosphine, trihexylphosphine, or tricyclohexylphosphine; or (cyanomethylene)tri-n-butylphosphorane; and advantageously dimethyl azodicarboxylate, diethyl azodicarboxylate, or a combination of bis(2-methoxyethyl)azodicarboxylate and triphenylphosphine; a combination of 1,1'-azobis(N,N-dimethylformamide) or 1,1'-(azodicarbonyl)dipiperidine and tributylphosphine or trihexylphosphine; or (cyanomethylene)tri-n-butylphosphorane.
Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously an aromatic hydrocarbon or an ether; and optimally benzene, toluene, diethyl ether, dioxane, or tetrahydrofuran.
Although differing depending on factors such as the ingredient compounds, the condensing agent, and the solvent, the reaction temperature is usually 0°C to the boiling point of the solvent, and advantageously 20°C to 150°c.

Although differing depending on factors such as the ingredient compounds, the condensing agent, the solvent, and the reaction temperature, the reaction time is usually thirty minutes to five days, and advantageously one hour to three days.
After one hydroxy of compound (XCVII) has been protected, the other hydroxy may be sulfonylated or halogenated to convert to a group represented by the expression Y (where Y is defined as described earlier), then reacted with the compound (LXXXIX). Subsequently removing the protecting group produces the alcohol compound (XCIa).

Step If-2 is a step for producing a compound represented by general formula (XCVIII), and is achieved by sulfonylating or halogenating the alcohol compound (XCIa). This step is carried out in the same manner as step Ca-2 of method Ca.
Step If-3 is a step for producing a compound represented by general formula (XCIX), and is achieved by reacting compound (XCVIII) with compound (LXXVIII).
This step is carried out in the same manner as step Eb-3 of method Eb (advantageously not in the presence of a base).

Step If-4 is a step for producing a compound represented by general formula (XCIb) (a compound in which Ga in compound (XCI) is sulfur), and is achieved by reacting compound (XCIX) with a base and hydrolyzing. This step is carried out in the same manner as step Eg-2 of method Eg.

Step If is a step for producing a compound represented by general formula (XCIc) (a compound in which Ga in compound (XCI) is the expression -N(R^{e2}a)- (where R^{e2}a is defined as described earlier), and is achieved by reacting compound (XCVIII) with an amino derivative represented by the general formula (C).
This step is carried out in the same manner as step Eb-3 of method Eb.

The compound contained in compound (XCIa) or compound (XCIc), such as a compound represented by general formula 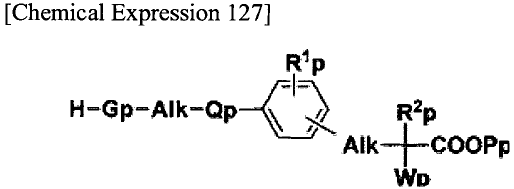 may be a conventional compound or produced using a conventional method (for example, Japanese Unexamined Patent Publication 2000-344666).
Gp in this expression indicates amino or hydroxyl, Alk indicates a C₁-C₆ alkylene, R¹p indicates (i) hydrogen, (ii) a C₁-C₆ alkyl, (iii) a C₁-C₄ alkoxy, (iv) a C₁-C₄ alkylthio, (v) a halogen, or (vi) nitro, Qp indicates a single bond, oxygen, sulfur, or a group represented by the expression -N(R³p)- (where R³p indicates hydrogen, a C₁-C₆ alkyl, a C₁-C₈ aliphatic acyl, or a C₇-C₁₁ aromatic acyl), R²p indicates hydrogen or a C₁-C₆ alkyl, Wp indicates (i) a C₁-C₆ alkyl, (ii) hydroxy, (iii) a C₁-C₄ alkoxy, (iv) a C₁-C₄ alkylthio, (v) a C₆-C₁₀ aryl optionally having one to five substituents, (vi) a C₆-C₁₀ aryloxy optionally having one to five substituents in the aryl portion, (vii) a C₆-C₁₀ arylthio optionally having one to five substituents in the aryl portion, (viii) a C₇-C₁₂ aralkyl optionally having one to five substituents in the aryl portion, (ix) a C₇-C₁₂ aralkyloxy optionally having one to five substituents in the aryl portion, (x) a C₇-C₁₂ aralkylthio optionally having one to five substituents in the aryl portion, (xi) an aryloxyalkyl having a C₆-C₁₀ aryl optionally having one to five substituents in the aryl portion and a C₁-C₄ alkyl in the alkyl portion, (xii) a monocyclic or dicyclic five- to ten-member aromatic heterocycle containing one to four heteroatoms selected from among a group consisting of oxygen, nitrogen, and sulfur, (xiii) a monocyclic or dicyclic five- to ten-member heteroaromatic oxy containing one to four heteroatoms selected from among a group consisting of oxygen, nitrogen, and sulfur, (xiv) monocyclic or dicyclic five- to ten-member heteroaromatic thio containing one to four heteroatoms selected from among a group consisting of oxygen, nitrogen, and sulfur, (xv) monocyclic or dicyclic five- to ten-member saturated heterocycle containing one to four heteroatoms selected from among a group consisting of oxygen, nitrogen, and sulfur, (xvi) amino, (xvii) a monoalkylamino having C₁-C₄ in the alkyl portion, (xviii) a dialkylamino in which the same or different alkyls have a C₁-C₄ alkyl, (xix) an N-alkyl-N-arylamino having a C₁-C₄ alkyl and a C₆-C₁₀ aryl optionally having one to five substituents, (xx) a C₆-C₁₀ arylamino optionally having one to five substituents in the aryl portion, (xxi) a C₇-C₁₂ ararylamino optionally having one to five substituents in the aryl portion, or (xxii) an aralkyloxycarbonylamino having a C₇-C₁₂ aralkyl optionally having one to five substituents in the aryl portion, and Pp indicates an ester residue.

Method Ig is a method for producing compound (XCVIII) 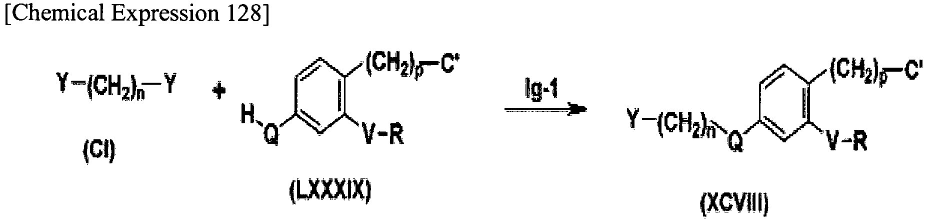 Y, n, Q, V, R, p, and C' in this expression are defined as described earlier.
Step Ig-1 is a step for producing compound (XCVIII), and is achieved by reacting a compound represented by general formula (CI) with compound (LXXXIX).
This step is carried out in the same manner as step Ca-1 of method Ca.

### <Method J>

Method J is a method for producing compound (I'c) in which E in compound (I') is a group represented by the expression -Eb-W^{c3}d-Ea- (where the group represented by the expression -Eb-W^{c3}d- (where W^{c3}d indicates oxygen, sulfur, a group represented by the expression -N(R^{e2}a)- (where R^{e2}a is defined as described earlier), or a group represented by the expression -CO-N(R^{e2}a)- (where R^{e2}a is defined as described earlier)) is defined the same as W^{c3} except that the terminal which will bond to Ea is W^{c3}d, and Ea indicates a group represented by the expression -Ar(R^{e1})-X^{e2}- or -W^{c3}-X^{e4}- (where Ar, R^{e1}, X^{e2}, W^{c3}, and X^{e4} are defined as described earlier) in which the terminal (in X^{e2} or X^{e4}) which will react with the aromatic ring is oxygen, sulfur, or a group represented by the expression -N(R^{e2}a)- (where R^{e2}a is defined as described earlier) (E bonds B to a portion of a benzene ring or a portion of a pyridine ring, and G is a dangling bond)).

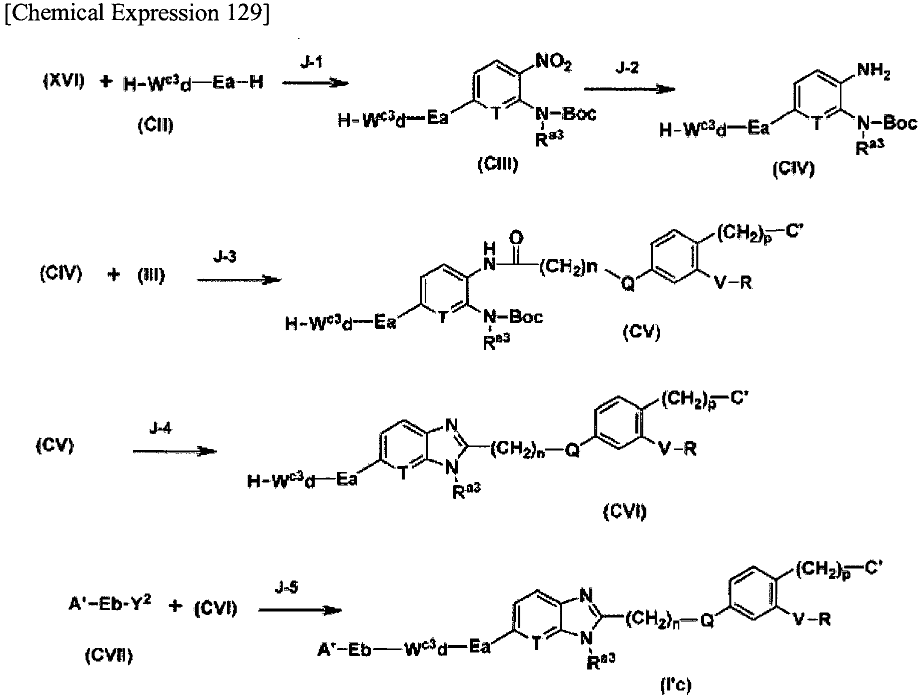

A', Eb, W^{c3}d, Ea, T, R^{a3}, n, Q, V, R, p, C', and Boc in this expression are defined as described earlier, and Y² indicates Y or carboxy. The group represented by the expression - N(R^{e2}a)- (where R^{e2}a is defined as described earlier) in Eb may be protected by a suitable protecting group (for example, Boc, triphenylmethyl, benzyloxycarbonyl, or phthalimido), the protecting group may be suitably removed as required, and in the case that T is nitrogen, the group represented by the expression -N(R^{a3})-Boc may be a group represented by the expression -N(R^{a3})-H_{.}

Step J-1 is a step for producing a compound represented by general formula (CIII), and is achieved by reacting compound (XVI) with a compound represented by general formula (CII). This step is carried out in the same manner as step Ea-1 of method Ea.
Step J-2 is a step for producing a compound represented by general formula (CIV), and is achieved by reducing the nitro of compound (CIII).
This step is carried out in the same manner as step D-1 of method D.
Step J-3 is a step for producing a compound represented by general formula (CV), and is achieved by reacting compound (CIV) with compound (III).
This step is carried out in the same manner as step B-1 of method B.

Step J-4 is a step for producing a compound represented by general formula (CVI), and is achieved by reacting compound (CV) with an acid.
This step is carried out in the same manner as step B-2 of method B.

Step J-5 is a step for producing compound (I'c), and is achieved by reacting compound (CVI) with a compound represented by general formula (CVII).
This step is carried out in the same manner as step Eb-3 of method Eb in the case that Y² is Y and W^{c3}a is oxygen, sulfur, or a group represented by the expression -N(R^{e2}a)- (where R^{e2}a is defined as described earlier), and in the same manner as step B-1 of method B in the case that Y² is carboxy and W^{c3}a is a group represented by the expression -N(R^{e2}a)- (where R^{e2}a is defined as described earlier.

### <Method K>

Method K is a method for oxidizing a sulfur present in compound (I) to convert the compound to a sulfoxide or a sulfone, and is carried out in the same manner as step Ef-3 of method Ef.

### <Method L>

Method L is a method for producing compound (XVII), and is achieved by reducing a carboxylic acid ester represented by the expression (CVIII).
This method (step L-1) is carried out in the same manner as step Fa-4 of method Fa.

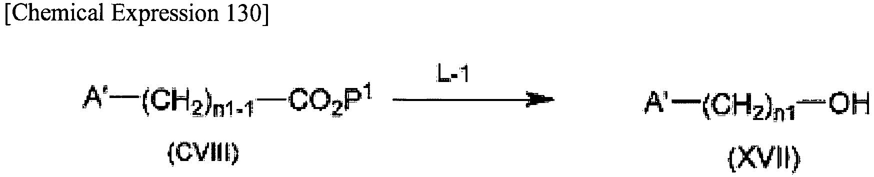

A', n1, and P¹ in this expression are defined as described earlier.

### <Method M>

Method M is a method for producing a compound represented by general formula 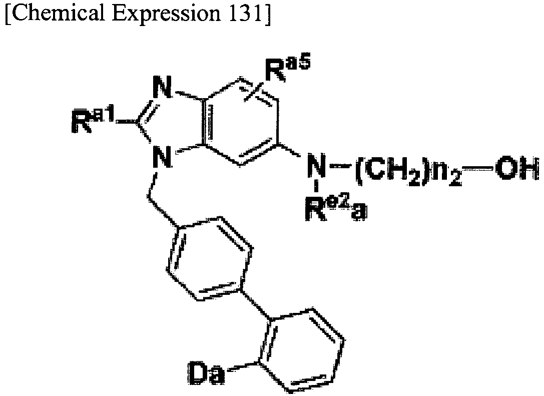 (where R^{a1}, R^{a5}, Da, R^{e2}a, and n2 are defined as described earlier).

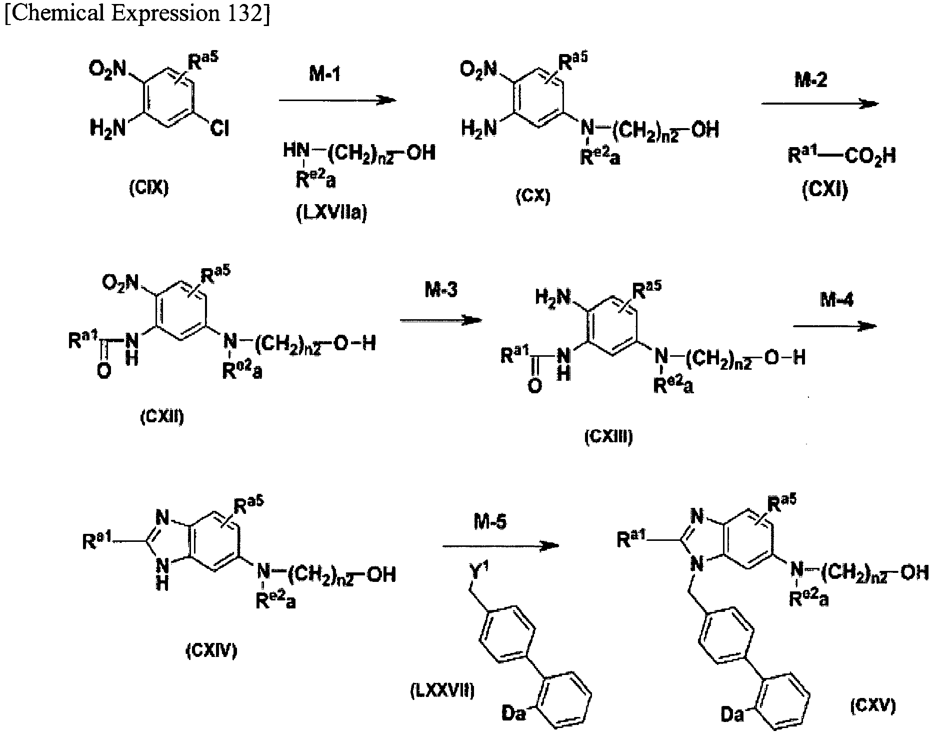

R^{a1}, R^{a5}, R^{e2}a, n2, Da, and Y¹ in this expression are defined as described earlier.

Step M-1 is a step for producing a compound represented by general formula (CX), and is achieved by reacting a compound represented by general formula (CIX) with a compound represented by general formula (LXVIIa).
This step is carried out in the same manner as step Ea-1 of method Ea. The reaction is carried out not in the presence of a base when an excess of compound (LXVIIa) is used.

Step M-2 is a step for producing a compound represented by general formula (CXII), and is achieved by reacting a compound (CX) represented by general formula (CXI) with a carboxylic acid.
This step is carried out in the same manner as step B-1 of method B. The hydroxyl of compound (CX) may be acylated (become R^{a1}CO) simultaneously, in which case, the compound in which hydroxyl was simultaneously acylated is used in the next step, and may be hydrolyzed to remove the acyl (R^{a1}CO) in the same manner as step A-1 of method A during step M-4 to be described later or after the reaction of step M-5 to be described later.

Step M-3 is a step for producing a compound represented by general formula (CXIII), and is achieved by reducing the nitro of compound (CXII).
This step is carried out in the same manner as step D-1 of method D.

Step M-4 is a step for producing a compound represented by general formula (CXIV), and is achieved by reacting compound (CXIII) with an acid.
This step is carried out in the same manner as step B-2 of method B. The acyl bonded to hydroxyl in this reaction may be removed simultaneously.

Step M-5 is a step for producing a compound represented by general formula (CXV), and is achieved by reacting compound (CXIV) with a compound represented by general formula (LXXVII).
This step is carried out in the same manner as step Eb-3 of method Eb.

### <Method N>

Method N is another method for producing compound (I').

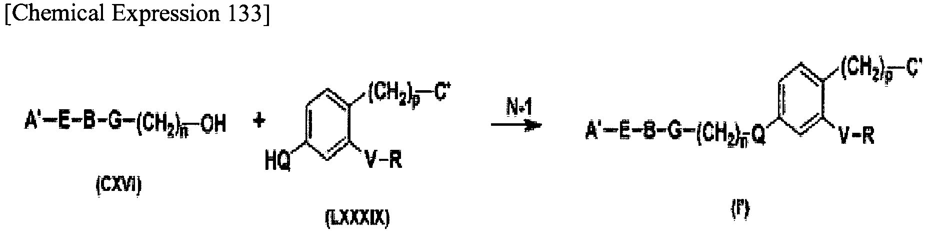

A', E, B, G, n, Q, V, R, p, and C' are defined as described earlier.
Step N-1 is a step for producing compound (I'), and is achieved by reacting a compound represented by general formula (CXVI) with compound (LXXXIX).
This step is carried out in the same manner as step If-1 of method If.

### <Method O>

Method O is a method for producing a compound represented by general formula Ab-(CH₂)ₙ₁-OH (where n1 is defined as described earlier, and Ab indicates a group represented by the expression 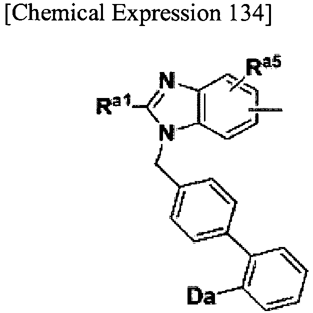 or 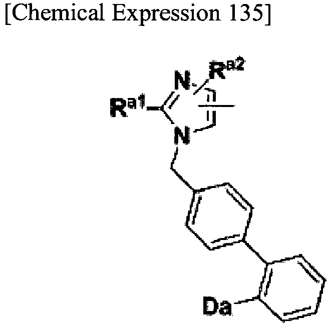 (where R^{a1}, R^{a2}, R^{a5}, and Da are defined as described earlier).

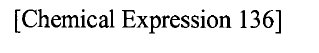

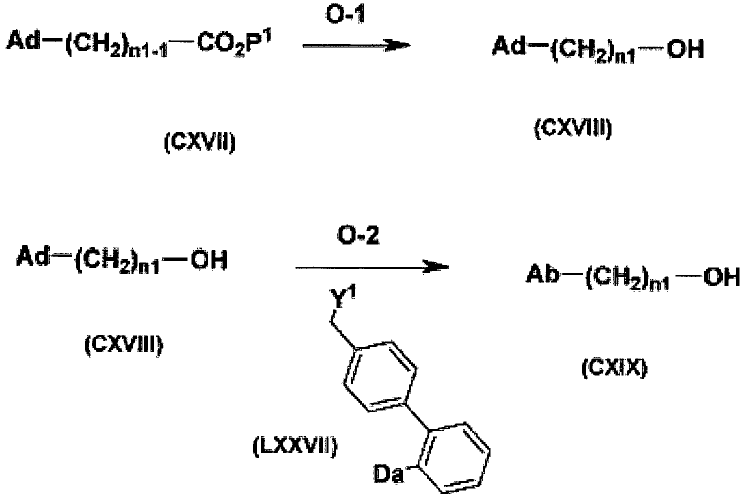

Ab, n1, P¹, Da, and Y¹ are defined as described earlier, and Ad is a case in which the nitrogen on the imidazole ring in Ab is substituted with hydrogen.

Step O-1 is a step for producing a compound represented by general formula (CXVIII), and is achieved by reducing a carboxylic acid ester compound represented by general formula (CXVII).
This step is carried out in the same manner as step Fa-4 of method Fa.

Step O-2 is a step for producing a compound represented by general formula (CXIX), and is achieved by reacting compound (CXVIII) with compound (LXXVII).
This step is carried out in the same manner as step Eb-3 of method Eb.

In the case that Da is a group that excludes C₁-C₆ alkoxycarbonyl in this method, after compound (CXVII) has been reacted with compound (LXXVII), the resulting carboxylic acid ester compound may be reduced to produce compound (CXIX).

### <Method P>

Method P is a method for producing a compound represented by general formula 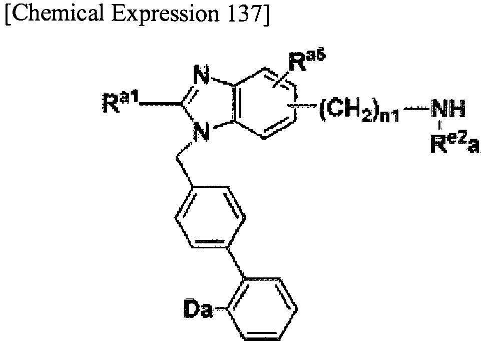 (where R^{a1}, Da, R^{a5}, n1, and R^{e2}a are defined as described earlier).

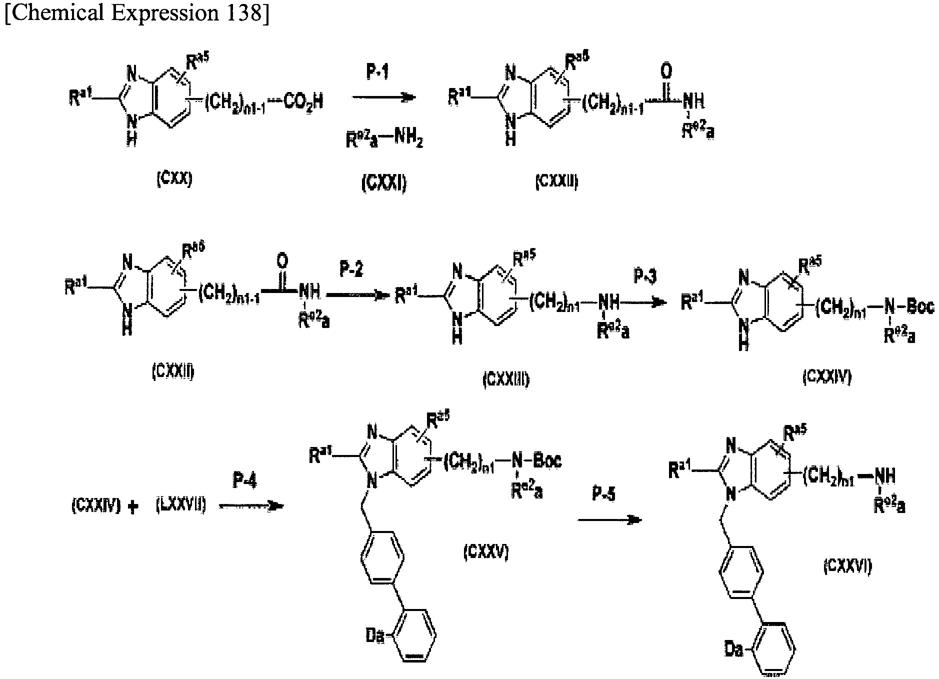

R^{a1}, R^{a5}, Da, n1, R^{e2}a, Boc, and Y¹ in this expression are defined as described earlier.

Step P-1 is a step for producing a compound represented by general formula (CXXII), and is achieved by reacting the carboxylic acid compound (CXX) with a compound represented by general formula (CXXI).
This step is carried out in the same manner as step B-1 of method B.

Step P-2 is a step for producing a compound represented by general formula (CXXIII), and is achieved by reducing an amide compound represented by general formula (CXII).
This step is carried out in the same manner as step Fa-4 of method Fa.

Step P-3 is a step for producing a compound represented by general formula (CXXIV), and is achieved by reacting compound (CXXIII) with a t-butoxycarbonyl halide (advantageously a chloride or a bromide) or di-t-butyl dicarbonate.
This step is carried out in the same manner as the reaction between an amine and an acid halide or acid anhydride in step B-1 of method B.

Step P-4 is a step for producing a compound represented by general formula (CXXV), and is achieved by reacting compound (CXXIV) with compound (LXXVII).
This step is carried out in the same manner as step Eb-3 of method Eb.

Step P-5 is a step for producing a compound represented by general formula (CXXVI), and is achieved by removing the amino protecting group (Boc) of compound (CXXV).
This step is carried out in the same manner as step B-2 of method B.

Compound (CXXIII) may also be reacted with compound (LXXVII) in the same manner as step P-4 in this method to produce compound (CXXVI).

### <Method Q>

Method Q is a method for producing compound (XIVr) in which E in compound (XIV) is a group represented by the expression -(CH₂)ₙ₆-W^{c3}d-(CH₂)ₙ₇-W^{c3}d-(CH₂)ₙ₈-Ar(R^{e1})-X^{e2}a-(where W^{c3}d, Ar, R^{e1}, and X^{e2}a are defined as described earlier, the two W^{c3}d may be the same or different, n6 indicates an integer of 1 to 6, n7 indicates an integer of 2 to 6, and n8 indicates an integer of 1 to 6; provided, however, that the sum of n6, n7, and n8 is not greater than 10).

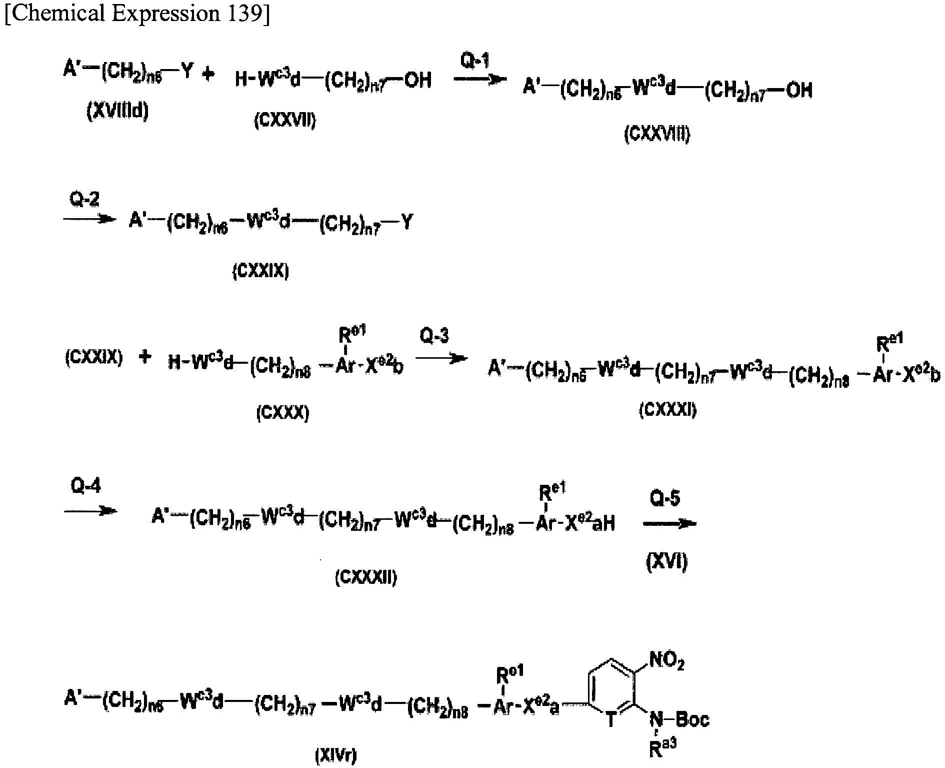

A', n6, W^{c3}d, n7, n8, Ar, R^{e1}, X^{e2}a, T, R^{a3}, Boc, and Y in this expression are defined as described earlier, the two W^{c3}d may be the same or different, X^{e2}b is defined the same as X^{e2} except that the terminal which will react with an aromatic ring is a group represented by the expression -OH, -SH, or -N(R^{e2}a)H (where R^{e2}a is defined as described earlier), which has been protected. The protecting groups for -OH, -SH, and -N(R^{e2}a)H are the same as in method Gb, and in the case that T is nitrogen, the group represented by the expression -N(R^{a3})-Boc may be - N(R^{a3})-H_{.}

Step Q-1 is a step for producing a compound represented by general formula (CXXVIII), and is achieved by reacting a compound represented by general formula (XVIIId) with a compound represented by general formula (CXXVII).
This step is carried out in the same manner as step Eb-3 of method Eb.

Step Q-2 is a step for producing a compound represented by general formula (CXXIX), and is achieved by sulfonylating or halogenating the alcohol compound (CXXVIII).
This step is carried out in the same manner as step Ca-2 of method Ca.

Step Q-3 is a step for producing a compound represented by general formula (CXXXI), and is achieved by reacting compound (CXXIX) with a compound represented by general formula (CXXX).
This step is carried out in the same manner as step Eb-3 of method Eb.
Protecting groups such as described earlier can be obtained by applying a method described in the literature, to be described later, to a compound corresponding to compound (CXXX) in which the hydroxy, the mercapto, and the group represented by the expression - N(R^{e2}a)H are not protected.

Step Q-4 is a step for producing a compound represented by general formula (CXXXII), and is achieved by removing the protecting groups of oxygen, sulfur, and the group represented by the expression -N(R^{e2}a)- contained in compound (CXXXI).
This step is carried out in the same manner as step A-1 of method A.

Step Q-5 is a step for producing a compound represented by general formula (XIVr), and is achieved by reacting compound (CXXXII) with compound (XVI).
This step is carried out in the same manner as step Ea-1 of method Ea.

The intended compound (CXXXI) may be produced even in the case that there are no protecting groups in the compound (CXXX) used in step Q-3 of this method.

### <Method R>

Method R is a method for producing compound (I'd) in which E in compound (I') is a group represented by the expression -(CH₂)ₙ₆-W^{c3}a-(CH₂)ₙ₇-Wc-(CH₂)ₙ₈-X^{e4}a- (where n6, W^{c3}a, n7, and n8 are defined as described earlier, Wc indicates a group represented by the expression -CON(R^{e2}a)- or -N(R^{e2}a)CO- (where R^{e2}a is defined as described earlier), and X^{e4}a is defined the same as X^{e4} except that the terminal that will bond to the aromatic ring is oxygen, sulfur, or a group represented by the expression -N(R^{e2}a)- (where R^{e2}a is defined as described earlier)) (E bonds B to a portion of a benzene ring or a portion of a pyridine ring, and G is a dangling bond)).

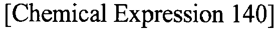

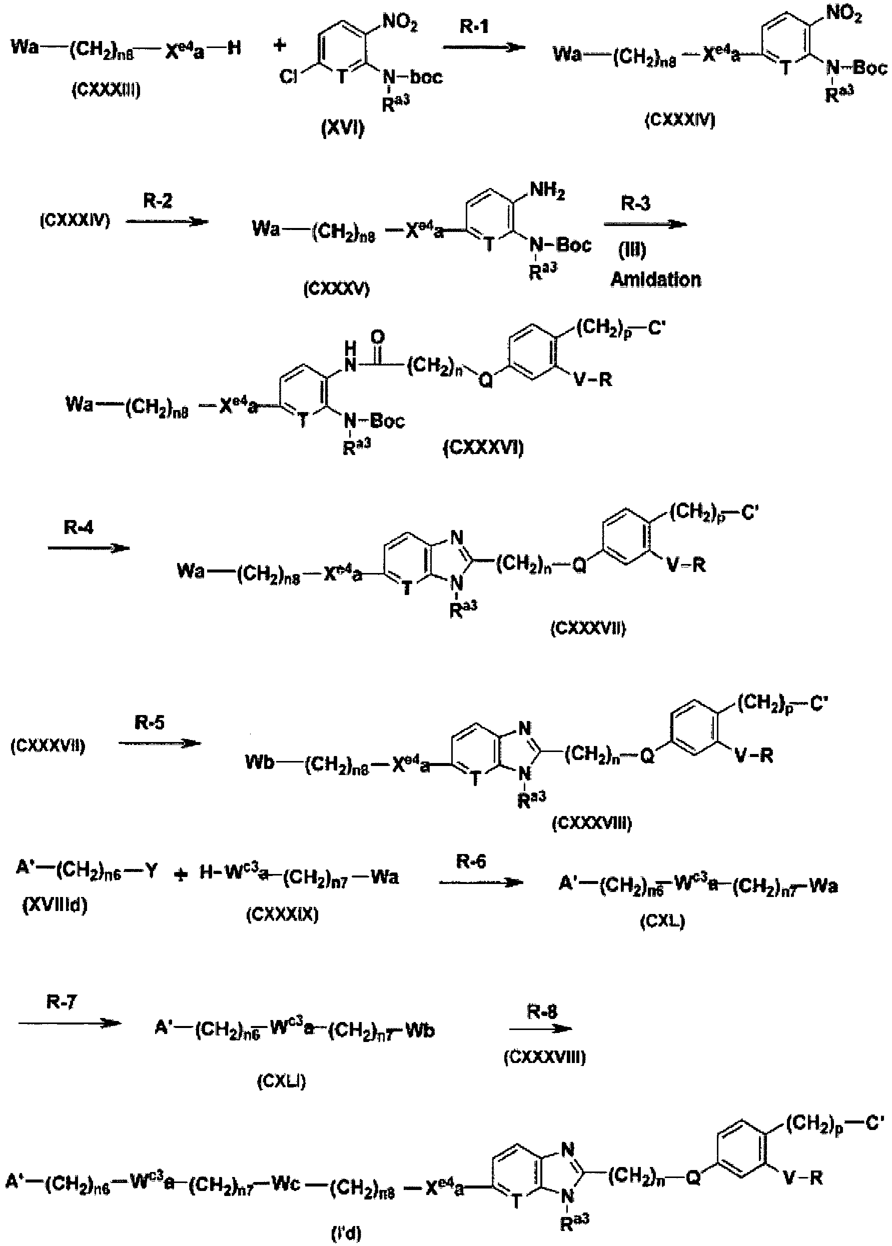

A', n6, W^{c3}a, n7, Wc, n8, X^{e4}a, T, R^{a3}, Boc, n, Q, V, R, p, C', and Y in this expression are defined as described earlier, Wa indicates a protected group represented by the expression - N(R^{e2}a)H (where R^{e2}a is defined as described earlier) or a protected carboxy, and Wb indicates a group represented by the expression -N(R^{e2}a)H (where R^{e2}a is defined as described earlier) or carboxy. The protecting group for -N(R^{e2}a)H or carboxy is the same as in methods A and Gb, and in the case that T is nitrogen, the group represented by the expression -N(R^{a3})-Boc may be - N(R^{a3})-H_{.}

Step R-1 is a step for producing a compound represented by general formula (CXXXIV), and is achieved by reacting a compound represented by general formula (CXXXIII) with compound (XVI).
This step is carried out in the same manner as step Ea-1 of method Ea.

Step R-2 is a step for producing a compound represented by general formula (CXXXV), and is achieved by reducing the nitro of compound (CXXXIV).
This step is carried out in the same manner as step D-1 of method D.

Step R-3 is a step for producing a compound represented by general formula (CXXXVI), and is achieved by reacting compound (CXXXV) with compound (III).
This step is carried out in the same manner as step B-1 of method B.

Step R-4 is a step for producing a compound represented by general formula (CXXXVII), and is achieved by reacting compound (CXXXVI) with an acid.
This step is carried out in the same manner as step B-2 of method B.

Step R-5 is a step for producing a compound represented by general formula (CXXXVIII), and is achieved, by removing the protecting group of the protected group represented by the expression -N(R^{e2}a)H (where R^{e2}a is defined as described earlier) or the protected carboxy of Wa in compound (CXXXVII).
This step is carried out in the same manner as step A-1 of method A.

Step R-6 is a step for producing a compound represented by general formula (CXL), and is achieved by reacting a compound represented by general formula (XVIIId) with a compound represented by general formula (CXXXIX).
This step is carried out in the same manner as step Eb-3 of method Eb.

Step R-7 is a step for producing a compound represented by general formula (CXLI), and is achieved by removing the protecting group of the protected group represented by the expression -N(R^{e2}a) (where R^{e2}a is defined as described earlier) or the protected carboxy of Wa in compound (CLX).
This step is carried out in the same manner as step A-1 of method A

Step R-8 is a step for producing compound (I'd), and is achieved by reacting compound (CXXXVIII) in which the terminal is a group represented by the expression -N(R^{e2}a)H (where R^{e2}a is defined as described earlier) with compound (CXLI) in which the terminal is carboxy, or by reacting compound (CXXXVIII) in which the terminal is carboxy with compound (CXLI) in which the terminal is a group represented by the expression -N(R^{e2}a)H (where R^{e2}a is defined as described earlier).
This step is carried out in the same manner as step B-1 of method B.

The intended compound may be produced even if there are no protecting groups for the group represented by the expression -N(R^{e2}a)H (where R^{e2}a is defined as described earlier) and/or the carboxy in this method, in which case, the step for removing protecting groups is not required.

### <Method S>

Method S is a method for producing compound (I'e) in which -E-B- in compound (I') is a group represented by the expression 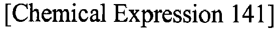 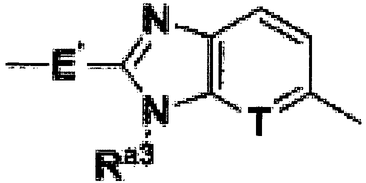 (where E', R^{a3}, and T are defined as described earlier) (E bonds B to a portion of an imidazole ring).

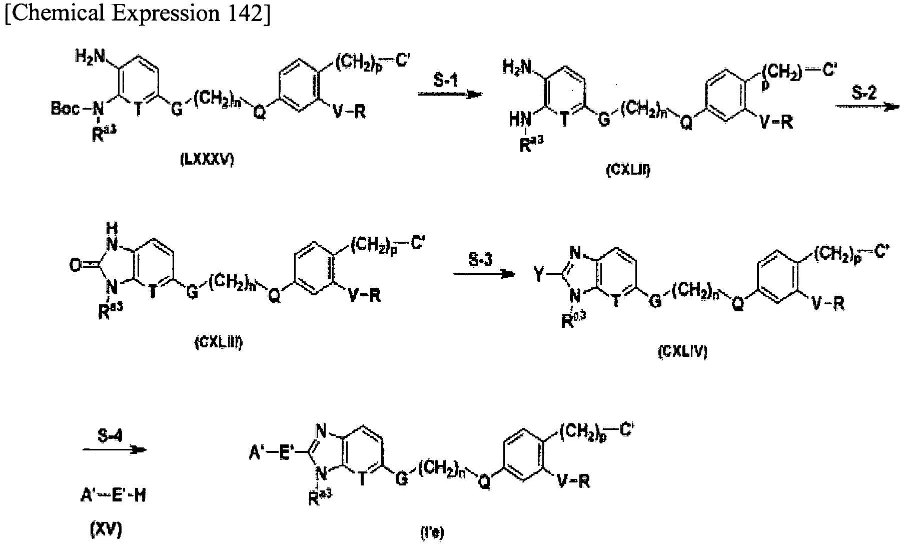

A', E', R^{a3}, T, G, n, Q, V, R, p, C', Boc, and Y in this expression are defined as described earlier.

Step S-1 is a step for producing a compound represented by general formula (CXLII), and is achieved by removing the Boc of compound (LXXXV).
This step is carried out in the same manner as step A-1 of method A (or step B-2 of method B).

Step S-2 is a step for producing a compound represented by general formula (CXLIII), and is achieved by reacting compound (CXLII) with a carbonylation agent in an inert solvent in the presence or not of a base.
The carbonylation agent may be, for example, 1,1'-carbonylbis-1H-imidazole, phenyl chloroformate, diphenyl carbonate, phosgene, or triphosgene; and advantageously 1,1'-carbonylbis- 1H-imidazole.
In the case that phenyl chloroformate, diphenyl carbonate, phosgene, or triphosgene is used, the reaction may be carried out advantageously in the presence of a base. The base used is the same as that described in step B-1 of method B.
Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as chloroform, dichloromethane, 1,2-dichloroethane, or carbon tetrachloride; an ester such as methyl acetate, ethyl acetate, propyl acetate, butyl acetate, or diethyl carbonate; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; advantageously an aromatic hydrocarbon, a halogenated hydrocarbon, an ether, or an amide; and optimally dichloromethane, dioxane, tetrahydrofuran, or dimethylformamide.

Although differing depending on factors such as the ingredient compounds and the base and solvent used, the reaction temperature is usually -20°C to the boiling point of the solvent, and advantageously 0°C to 100°C.
Although differing depending on factors such as the ingredient compounds, the base and solvent used, and the reaction temperature, the reaction time is usually fifteen minutes to 48 hours, and advantageously thirty minutes to twenty-four hours.

Step S-3 is a step for producing a compound represented by general formula (CXLIV), and is achieved by sulfonylating or halogenating compound (CXLIII).
This step is carried out in the same manner as step Ca-2 of method Ca.

Step S-4 is a step for producing compound (I'e), and is achieved by reacting compound (CXLIV) with compound (XV).
This step is carried out in the same manner as step Ea-1 of method Ea.
This method may also be applied to produce compound (CXVIa) in which the group represented by the expression -E-B- in compound (CXVI) is a group represented by the expression 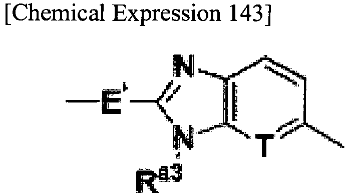 (where E', R^{a3}, and T are defined as described earlier) (E is a group for bonding B to a portion of an imidazole ring) (method Sb). 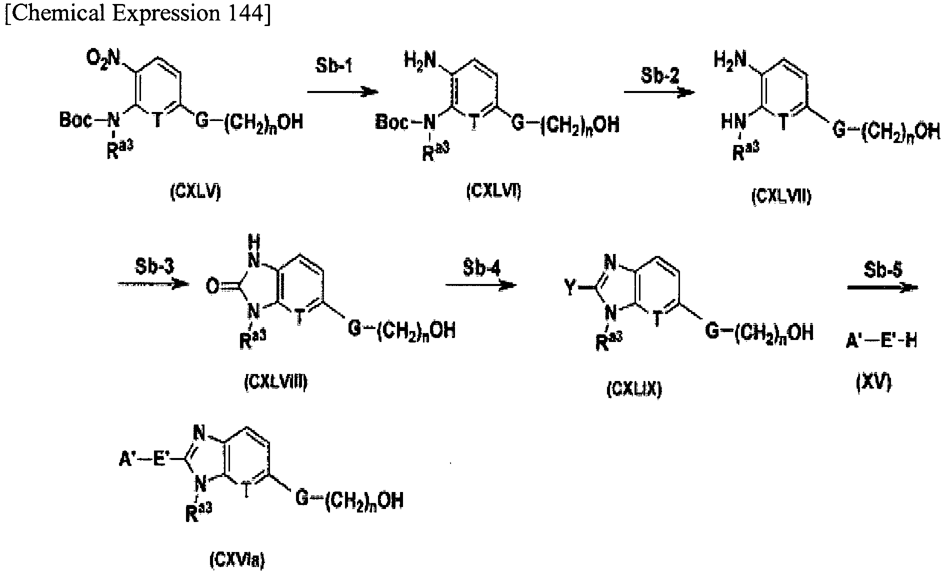 A', E', R^{a3}, T, G, n, Boc, and Y in this expression are defined as described earlier.

Step Sb-1 is a step for producing a compound represented by general formula (CXLVI), and is achieved by reducing the nitro of a compound represented by general formula (CXLV).
This step is carried out in the same manner as step D-1 of method D.

Step Sb-2 is a step for producing a compound represented by general formula (CXLVII), and is achieved by removing the Boc of compound (CXLVI).
This step is carried out in the same manner as step A-1 of method A.

Step Sb-3 is a step for producing a compound represented by general formula (CXLVIII), and is achieved by carbonylating compound (CXLVII).
This step is carried out in the same manner as step S-2 of this method.

Step Sb-4 is a step for producing a compound represented by general formula (CXLIX), and is achieved by sulfonylating or halogenating compound (CXLVIII).
This step is carried out in the same manner as step Ca-2 of method Ca.

Step Sb-5 is a step for producing compound (CXVIa), and is achieved by reacting compound (CXLIX) with compound (XV).
This step is carried out in the same manner as step Ea-1 of method Ea.
Compound (CXVIa) may also be produced in step Sb by placing a protecting group on the hydroxyl in compound (CXLV), then applying steps Sb-1 to Sb-5 before removing the hydroxyl protecting group. The hydroxy protecting group may be, for example, a group described in method Gb. The protecting group is removed in the same manner as step A-1 of method A.

The intermediary (CXLVIII) in step Sb may be produced by applying method S (method Sc). 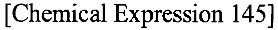 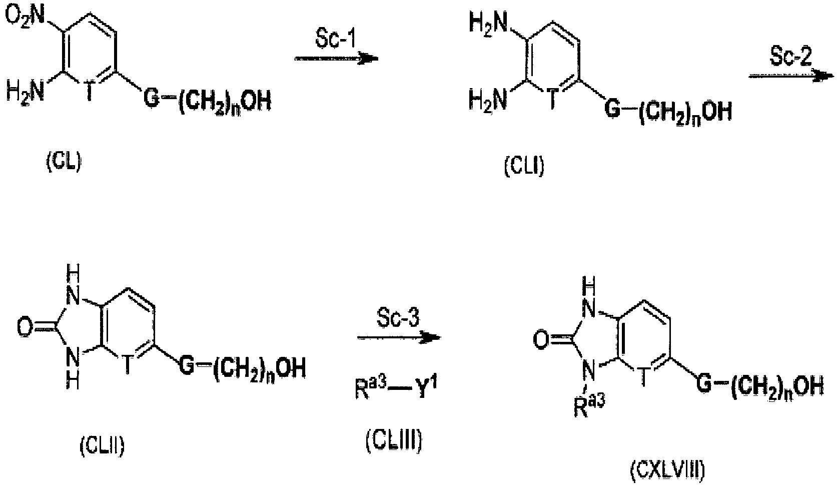 R^{a3}, T, G, n, and Y¹ in this expression are defined as described earlier.

Step Sc-1 is a step for producing a compound represented by general formula (CLI) and is achieved by reducing the nitro of a compound represented by general formula (CL).
This step is carried out in the same manner as step D-1 of method D.

Step Sc-2 is a step for producing a compound represented by general formula (CLII), and is achieved by carbonylating compound (CLI).
This step is carried out in the same manner as step S-2.

Step Sc-3 is a step for producing compound (CXLVIII), and is achieved by reacting compound (CLII) with a compound represented by general formula (CLIII).
This step is carried out in the same manner as step Ca-4 of method Ca.
The hydroxy in compound (CLII) may be protected in this step, in which case, the protecting group is suitably removed after this reaction. The hydroxy protecting group is a group such as descbed in method Gb, and is removed in the same manner as step A-1 of method A.

### <Method T>

Method T is a method for producing compound (I'f) in which E in compound (I') bonds B to a portion of a benzene ring or a portion of a pyridine ring, and G is oxygen, sulfur, or a group (Ga) represented by the expression -N(R^{e2}a)- (where R^{e2}a is defined as described earlier). 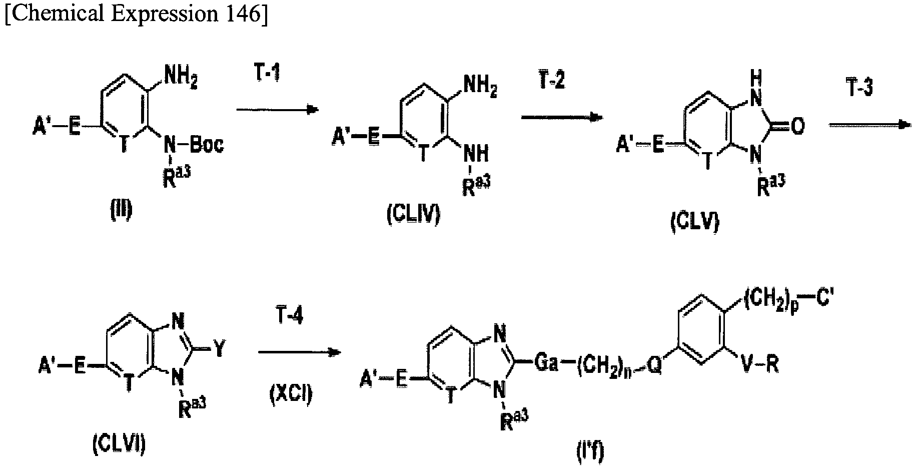 A', E, R^{a3}, T, Ga, n, Q, V, R, p, C', Boc, and Y in this expression are defined as described earlier.
Step T-1 is a step for producing a compound represented by general formula (CLIV), and is achieved by removing the Boc group in compound (II). This step is carried out in the same manner as step A-1 of method A (or step B-2 of method B).
Step T-2 is a step for producing a compound represented by general formula (CLV), and is achieved by reacting compound (CLIV) with a carbonylating agent. This step is carried out in the same manner as step S-2 of method S.
Step T-3 is a step for producing a compound represented by general formula (CLVI), and is achieved by sulfonylating or halogenating compound (CLV). This step is carried out in the same manner as step Ca-2 of method Ca.
Step T-4 is a step for producing compound (I'f), and is achieved by reacting compound (CLVI) with compound (XCI). This step is carried out in the same manner as step Ea-1 of method Ea.

### <Method U>

Method U is a method for producing compounds (XIV) having different E.
Method Ua is a method for producing compound (XIVs) in which E is a group represented by the expression -(CH₂)n1-N(R^{e2}a)- (where n1 and R^{e2}a are defined as described earlier). 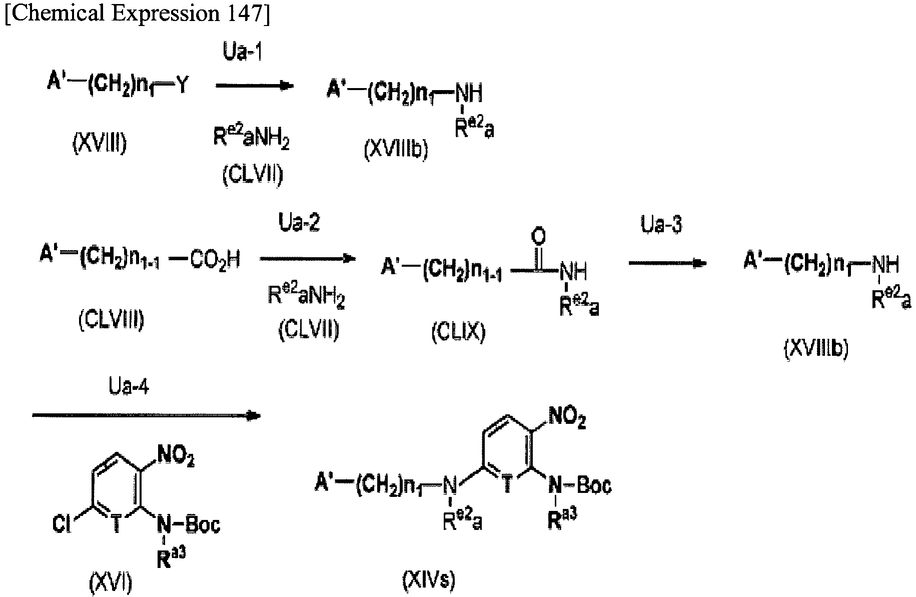 A', n1, Y, R^{e2}a, T, Boc, and R^{a3} are defined as described earlier.
Step Ua-1 is a step for producing compound (XVIIIb), and is achieved by reacting compound (XVIII) with a compound represented by general formula (CLVII). This step is carried out in the same manner as step Ea-1 of method Ea.
Step Ua-2 is a step for producing a compound represented by general formula (CLIX), and is achieved by reacting a compound represented by general formula (CLVIII) with compound (CLVII). This step is carried out in the same manner as step B-1 of method B.
Step Ua-3 is a step for producing compound (XVIIIb), and is achieved by reducing the amide compound (CLIX). This step is carried out in the same manner as step Fa-4 of method Fa.
Step Ua-4 is a step for producing compound (XIVs), and is achieved by reacting compound (XVIIIb) with compound (XVI). This step is carried out in the same manner as step Ea-1 of method Ea.

Method Ub is a method for producing compound (XIVt) in which E is a group represented by the expression -(CH₂)n1-W^{c3}a-(CH₂)n4-W^{c3}a-(CH₂)n5-Ar(R^{e1})-X^{e2}a- (where n1, n4, W^{c3}a, n5, Ar, R^{e1}, and X^{e2}a are defined as described earlier, and the two W^{c3}a may be the same or different). 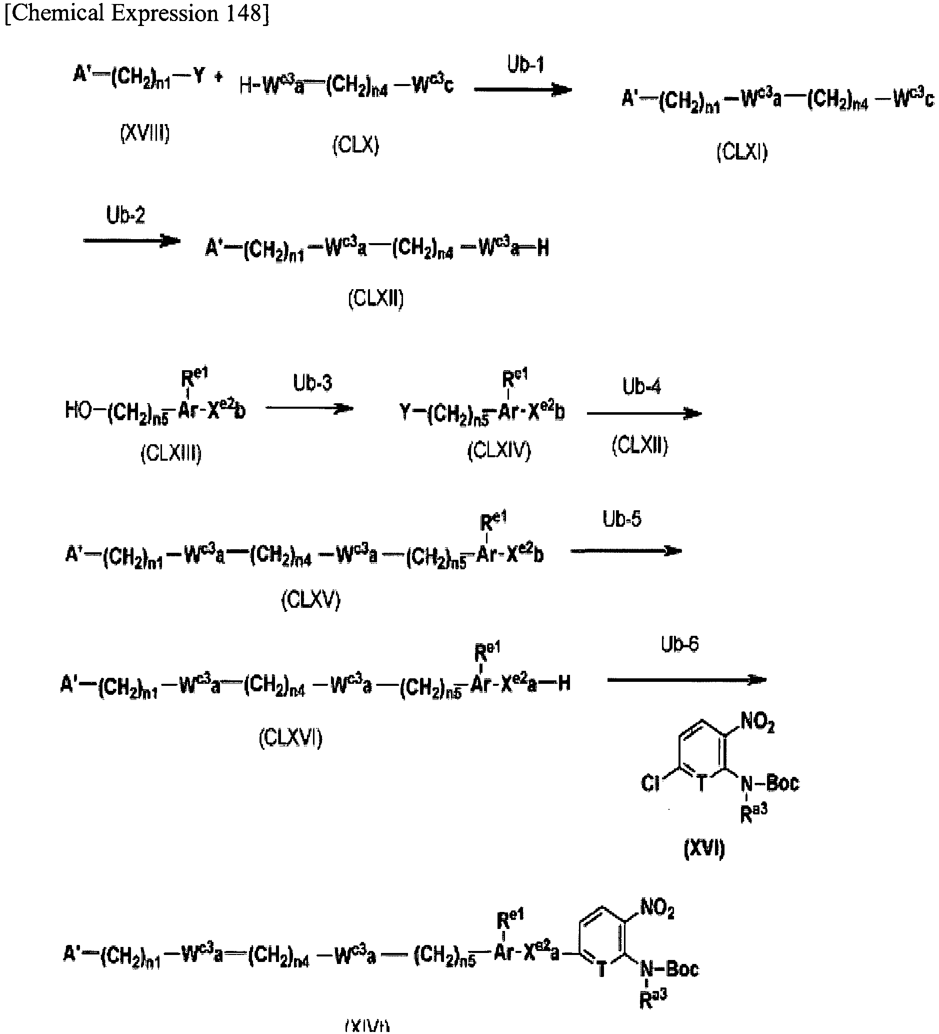 A', n1, n4, n5, Y, W^{c3}a, W^{c3}c, Ar, R^{e1}, X^{e2}a, X^{e2}b, T, Boc, and R^{a3} are defined as described earlier.
Step Ub-1 is a step for producing a compound represented by general formula (CLXI), and is achieved by reacting compound (XVIII) with a compound represented by general formula (CLXI). This step is carried out in the same manner as step Ea-1 of method Ea.
Step Ub-2 is a step for producing a compound represented by general formula (CLXII), and is achieved by removing the protecting group of the group represented by the expression - W^{c3}c. This step is carried out in the same manner as step A-1 of method A.
Step Ub-3 is a step for producing a compound represented by general formula (CLXIV), and is achieved by sulfonylating or hydrogenating compound (CLXII). This step is carried out in the same manner as step Ca-2 of method Ca.
Step Ub-4 is a step for producing a compound represented by general formula (CLXV), and is achieved by reacting compound (CLXIV) with compound (CLXII). This step is carried out in the same manner as step Ca-3 of method Ca.
Step Ub-5 is a step for producing a compound represented by general formula (CLXVI), and is achieved by removing the protecting group of the group represented by the expression - X^{e2}b. This step is carried out in the same manner as step A-1 of method A.
Step Ub-6 is a step for producing compound (XIVt), and is achieved by reacting compound (CLXVI) with compound (XVI). This step is carried out in the same manner as step Ea-1 of method Ea.
Method Uc is a method for producing compound (XIVu) in which E is a group represented by the expression -(CH₂)n1-W^{c3}a-Wd-W^{c3}a-X^{e4}a- (where n1, W^{c3}a, and X^{e4}a are defined as described earlier, the two W^{c3}a may be the same or different, and the group represented by the expression -(CH₂)n1-W^{c3}a-Wd-W^{c3}a- is defined the same as a group represented by the expression -W^{c3}-W^{c3}- except that the terminal that will bond to A' is a group represented by the expression -(CH₂)n1-W^{c3}a-, and the terminal that will bond to X^{e4}a is W^{c3}a). 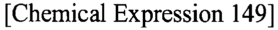 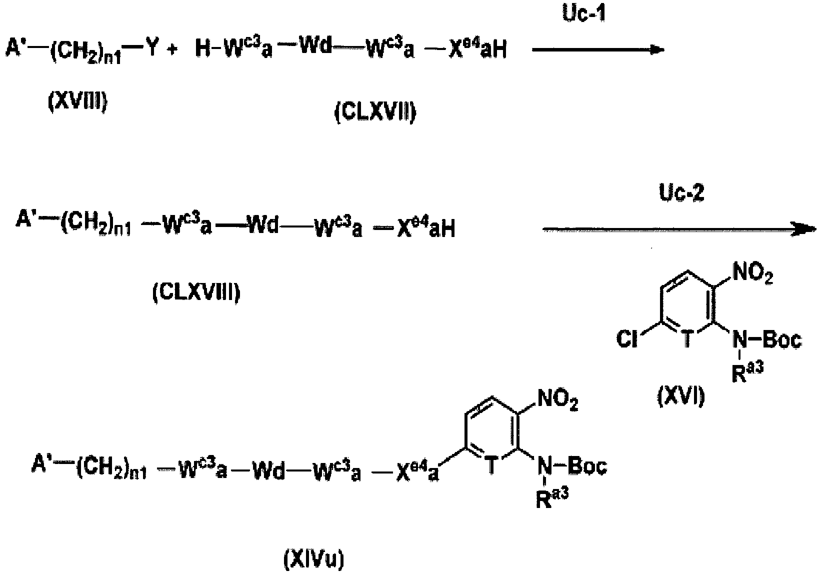 A', n1, Y, W^{c3}a, Wd, X^{e4}a, T, Boc, and R^{a3} in this expression are defined as described earlier.
Step Uc-1 is a step for producing a compound represented by general formula (CLXVIII), and is achieved by reacting compound (XVIII) with a compound represented by general formula (CLXVII). This step is carried out in the same manner as step Eb-3 of method Eb.
Step Uc-2 is a step for producing compound (XIVu), and is achieved by reacting compound (CLXVIII) with compound (XVI). This step is carried out in the same manner as step Ea-1 of method Ea.
Method Ud is a method for producing compound (XIVu) in which E is a group represented by the expression -(CH₂)n1-W^{c3}a-We-X^{e4}a- (where n1, W^{c3}a, and X^{e4}a are defined as described earlier, and the group represented by the expression -(CH₂)n1-W^{c3}a-We-X^{e4}a- is defined the same as a group represented by the expression -W^{c3}-W^{c3}-X^{e4}- except that the terminal that will bond to A' is a group represented by the expression -(CH₂)n1-W^{c3}a-, and the terminal that will bond to a portion of a benzene or pyridine ring is X^{e4}a). 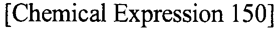 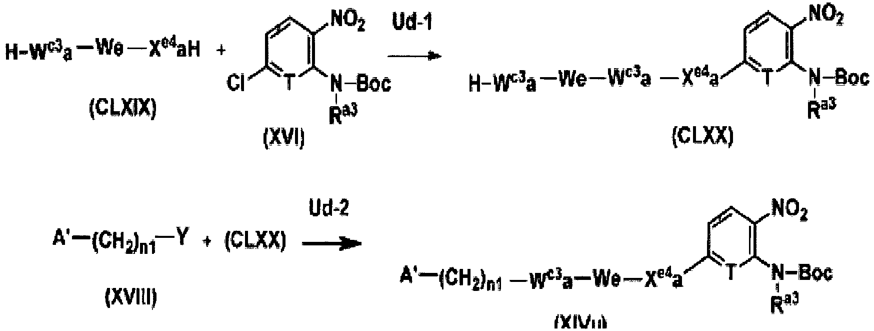 A', n1, Y, W^{c3}a, We, X^{e4}a, T, Boc, and R^{a3} in this expression are defined as described earlier.
Step Ud-1 is a step for producing a compound represented by general formula (CLXX), and is achieved by reacting a compound represented by general formula (CLXIX) with compound (XVI). This step is carried out in the same manner as step Eb-3 of method Eb.
Step Ud-2 is a step for producing compound (XIVu), and is achieved by reacting compound (CLXX) with compound (XVIII). This step is carried out in the same manner as step Ea-1 of method Ea.
Method Ue is a method for producing compound (XIVw) in which E is a group represented by the expression -(CH₂)n1-W^{c3}a-Wf-W^{c3}a-X^{e4}a- (where n1, W^{c3}a, and X^{e4}a are defined as described earlier, the two W^{c3}a may be the same or different, and the group represented by the expression -(CH₂)n1-W^{c3}a-Wf-W^{c3}a-X^{e4}a- is defined the same as a group represented by the expression -W^{c3}-W^{c3}-X^{e4}- except that the terminal that will bond to A' is a group represented by the expression -(CH₂)n1-W^{c3}a-, and the terminal that will bond to a portion of a benzene or pyridine ring is X^{e4}a). 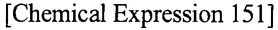 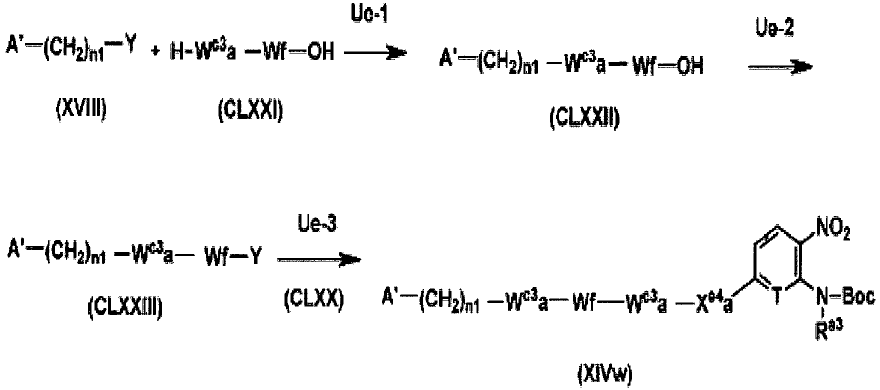 A', n1, Y, W^{c3}a, Wf, X^{e4}a, T, Boc, and R^{a3} in this expression are defined as described earlier.
Step Ue-1 is a step for producing a compound represented by general formula (CLXXII), and is achieved by reacting compound (XVIII) with a compound represented by general formula (CLXXI). This step is carried out in the same manner as step Eb-3 of method Eb.
Step Ue-2 is a step for producing a compound represented by general formula (CLXXIII), and is achieved by reacting compound (CLXXII) with a sulfonyl halide or a halogenating agent. This step is carried out in the same manner as step Ca-2 of method Ca.
Step Ue-3 is a step for producing compound (XIVw), and is achieved by reacting compound (CLXXIII) with compound (CLXX). This step is carried out in the same manner as step Eb-3 of method Eb.
Method Uf is a method for producing compound (XIVx) in which E is a group represented by the expression -W^{c3}a-(CH₂)n9-Wg-X^{e4}a- (where W^{c3}a and X^{e4}a are defined as described earlier, n9 indicates an integer of 1 to 10, and the group represented by the expression -W^{e3}a-(CH₂)n9-Wg-X^{e4}a- is defined the same as a group represented by the expression -W^{c3}-W^{c3}-X^{e4}- except that the terminal that will bond to A' is a group represented by the expression - W^{c3}a-(CH₂)n9-, and the terminal that will bond to a portion of a benzene or pyridine ring is X^{e4}a)_{.} 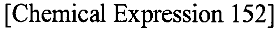 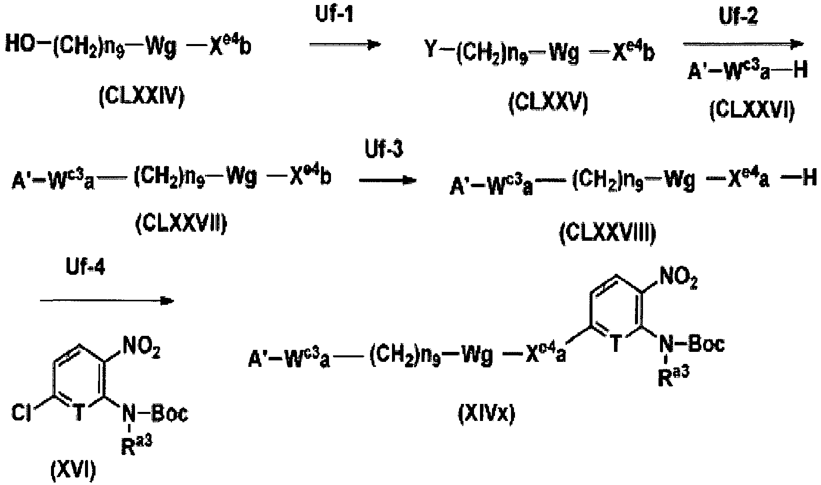 A', W^{c3}a, n9, Wg, X^{e4}a, Y, T, Boc, and R^{a3} in this expression are defined as described earlier, and X^{e4}b is defined the same as X^{e4}a except that oxygen, sulfur, or a group represented by the expression -N(R^{e2}a)- (where R^{e2}a is defined as described earlier) is protected.
Step Uf-1 is a step for producing a compound represented by general formula (CLXXV), and is achieved by reacting a compound represented by general formula (CLXXIV) with asulfonyl halide or a halogenating agent. This step is carried out in the same manner as step Ca-2 of method Ca.
Step Uf-2 is a step for producing a compound represented by general formula (CLXXVII), and is achieved by reacting compound (CLXXV) with a compound represented by general formula (CLXXVI). This step is carried out in the same manner as step Eb-3 of method Eb.
Step Uf-3 is a step for producing a compound represented by general formula (CLXXVIII), and is achieved by removing the protecting group of the group represented by the expression X^{e4}b. This step is carried out in the same manner as step A-1 of method A.
Step Uf-4 is a step for producing compound (XIVx), and is achieved by reacting compound (CLXXVIII) with compound (XVI). This step is carried out in the same manner as step Ea-1 of method Ea.
Method Ug is another method for producing compound (XIVx). 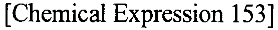 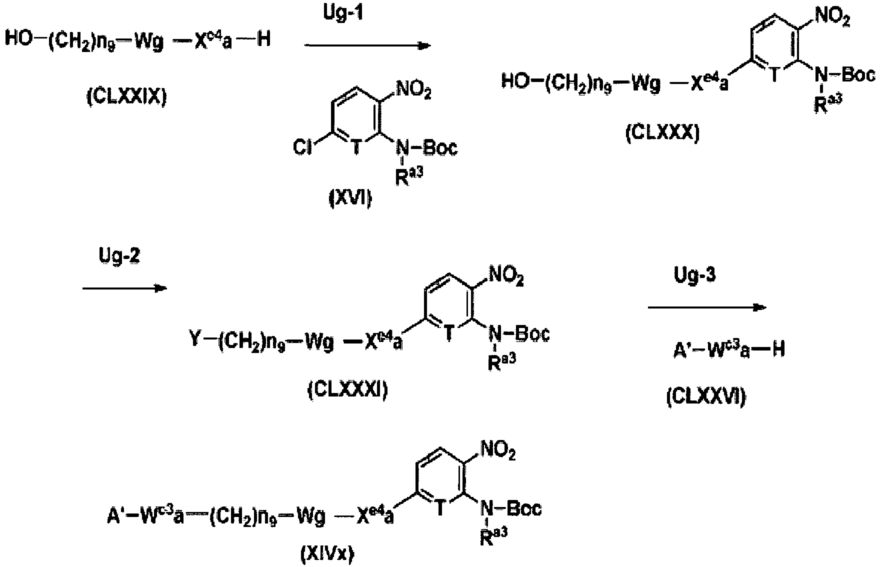 A', W^{c3}a, n9, Wg, X^{e4}a, Y, T, Boc, and R^{a3} in this expression are defined as described earlier.
Step Ug-1 is a step for producing a compound represented by general formula (CLXXX), and is achieved by reacting a compound represented by general formula (CLXXIX) with compound (XVI). This step is carried out in the same manner as step Ea-1 of method Ea.
Step Ug-2 is a step for producing a compound represented by general formula (CLXXXI), and is achieved by reacting compound (CLXXX) with a sulfonyl halide or a halogenating agent. This step is carried out in the same manner as step Ca-2 of method Ca.
Step Ug-3 is a step for producing compound (XIVx), and is achieved by reacting compound (CLXXXI) with compound (CLXXVI). This step is carried out in the same manner as step Eb-3 of method Eb.
Method Uh is a method for producing compound (XIVy) in which E is a group represented by the expression -W^{c3}a-(CH₂)n9-Ar(R^{e1})-X^{e2}a- (where W^{c3}a, n9, Ar, R^{e1}, and X^{e2}a are defined as described earlier). 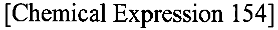 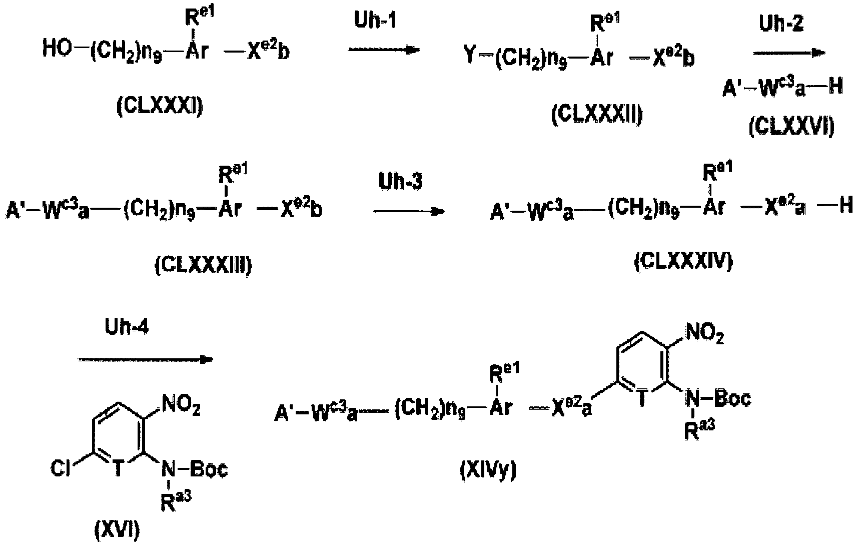 A', W^{c3}a, n9, Ar, R^{e1}, X^{e2}a, X^{e2}b, Y, T, Boc, and R^{a3} in this expression are defined as described earlier.
Step Uh-1 is a step for producing a compound represented by general formula (CLXXXII), and is achieved by reacting compound (CLXXXI) with a sulfonyl halide or a halogenating agent. This step is carried out in the same manner as step Ca-2 of method Ca.
Step Uh-2 is a step for producing a compound represented by general formula (CLXXXIII), and is achieved by reacting compound (CLXXXII) with compound (CLXXVI). This step is carried out in the same manner as step Eb-3 of method Eb.
Step Uh-3 is a step for producing a compound represented by general formula (CLXXXIV), and is achieved by removing the protecting group of the group represented by the expression X^{e2}b. This step is carried out in the same manner as step A-1 of method A.
Step Uh-4 is a step for producing compound (XIVy), and is achieved by reacting compound (CLXXXIV) with compound (XVI). This step is carried out in the same manner as step Ea-1 of method Ea.
Method Ui is another method for producing compound (XIVy). 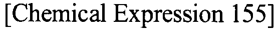 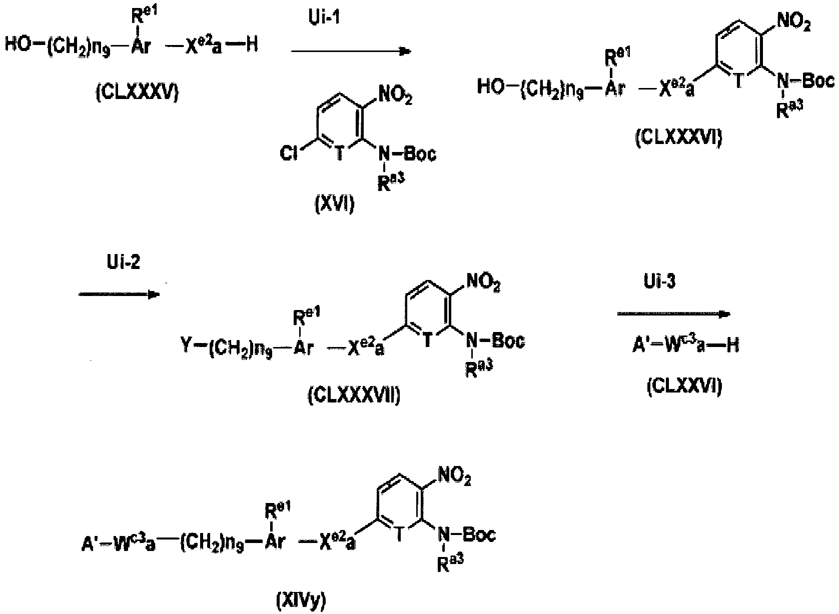 A', n9, Y, Ar, R^{e1}, X^{e2}a, W^{c3}a, T, Boc, and R^{a3} in this expression are defined as described earlier.
Step Ui-1 is a step for producing a compound represented by general formula (CLXXXVI), and is achieved by reacting a compound represented by general formula (CLXXXV) with compound (XVI). This step is carried out in the same manner as step Ea-1 of method Ea.
Step Ui-2 is a step for producing a compound represented by general formula (CLXXXVII), and is achieved by reacting compound (CLXXXVI) with a sulfonyl halide or a halogenating agent. This step is carried out in the same manner as step Ca-2 of method Ca.
Step Ui-3 is a step for poroducing compound (XIVy), and is achieved by reacting compound (CLXXXVII) with compound (CLXXVI). This step is carried out in the same manner as step Eb-3 of method Eb.
Method Uj is a method for producing compound (XIVz) in which E is a group represented by the expression -(CH₂)n1-N(R^{e2}a)-CO-X^{e4}a- (where n1, R^{e2}a, and X^{e4}a are defined as described earlier). 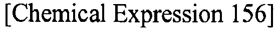 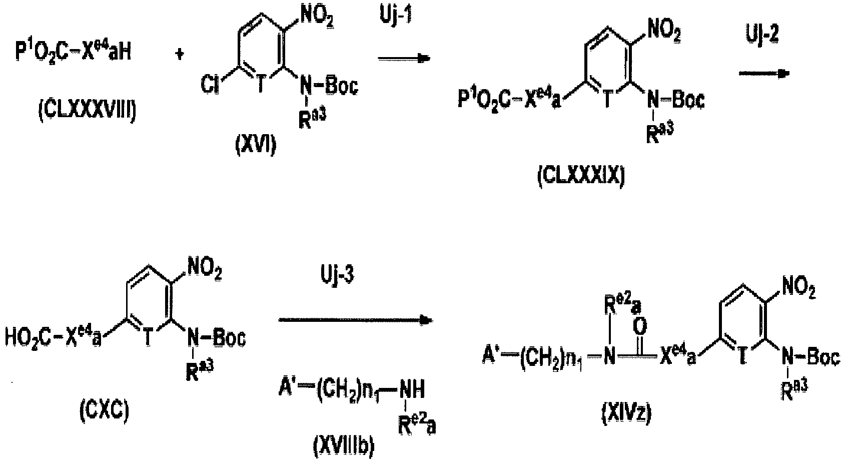 A', n1, R^{e2}a, X^{e4}a, T, R^{a3}, Boc, and P¹ in this expression are defined as described earlier. The tetrazole or thiazolidin-2,4-dione in A', however, need not be protected, and in the case that T is nitrogen, the group represented by the expression -N(R^{a3})-Boc may be a group represented by the expression -N(R^{a3})-H.
Step Uj-1 is a step for producing a compound represented by general formula (CLXXXIX), and is achieved by reacting a compound represented by general formula (CLXXXVIII) with compound (XVI). This step is carried out in the same manner as step Ea-1 of method Ea.
Step Uj-2 is a step for producing a compound represented by general formula (CXC), and is achieved by hydrolyzing the ester compound (CLXXXIX). This step is carried out in the same manner as step A-1 of method A.
Step Uj-3 is a step for producing compound (XIVz), and is achieved by reacting compound (CXC) with compound (XVIIIb). This step is carried out in the same manner as step B-1 of method B.
Compound (XIVaa) in which E in compound (XIV) is a group represented by the expression -N(R^{e2}a)-CO-X^{e4}a- (where R^{e2}a and X^{e4}a are defined as described earlier) may be produced by reacting compound (CXC) with a compound represented by general formula A'-N(R^{e2}a)H (where A' and R^{e2}a are defined as described earlier) in the same manner as step Uj-3. 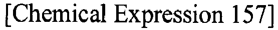 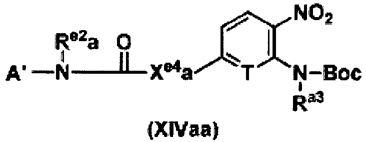 Method Uk is a method for producing compound (XIVbb) in which E is a group represented by the expression -(CH₂)n1-W^{c3}a-(CH₂)n4-N(R^{e2}a)-CO-X^{e4}a- (where n1, W^{c3a}, n4, R^{e2}a, and X^{e4}a are defined as described earlier). 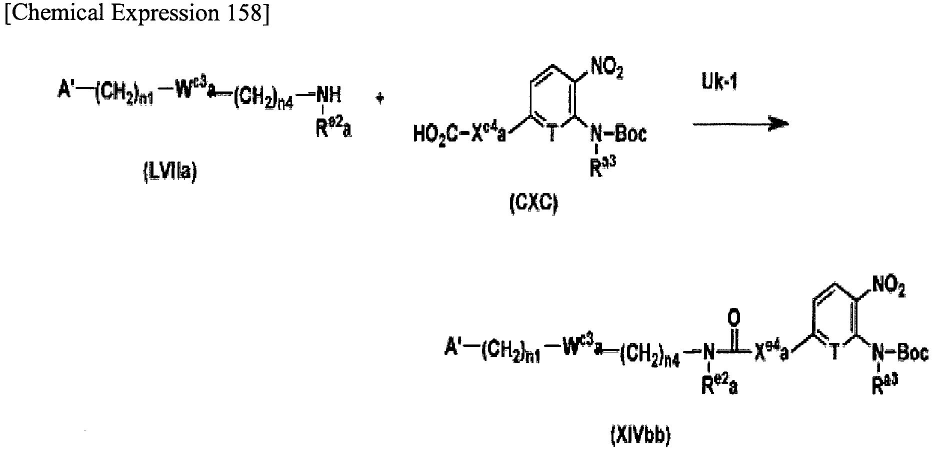 A', n1, n4, R^{e2}a, X^{e4}a, T, R^{a3}, Boc, and W^{c3}a in this expression are defined as described earlier. The tetrazole or thiazolidin-2,4-dione in A', however, need not be protected, and in the case that T is nitrogen, the group represented by the expression -N(R^{a3})-Boc may be a group represented by the expression -N(R^{a3})-H.
Step Uk-1 is a step for producing compound (XIVbb), and is achieved by reacting compound (CXC) with compound (LVIIa). This step is carried out in the same manner as step B-1 of method B.

### <Method Va>

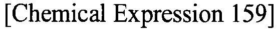

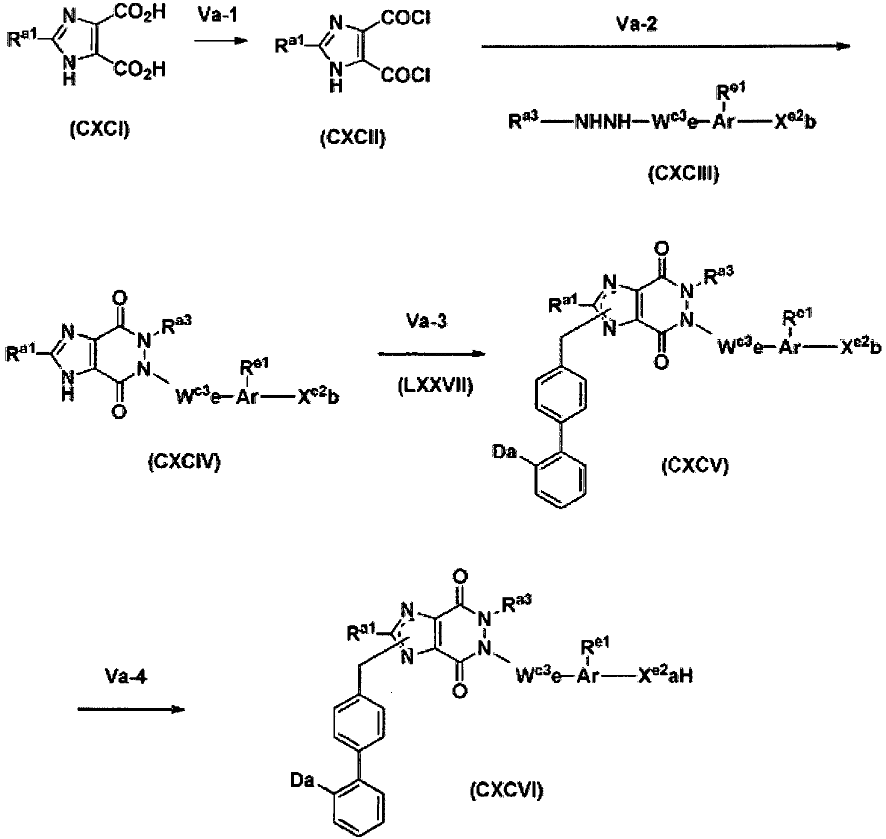

R^{a1}, R^{a3}, Ar, R^{e1}, X^{e2}a, X^{e2}b, Da, and the bond embraced by the dotted line in this expression are defined as described earlier, and W^{c3}e is defined the same as W^{c3} except for bonding to the portion represented by the expression -CO-N(N=)- by a carbon.
Step Va-1 is a step for producing a compound represented by general formula (CXCII), and is achieved by making a compound represented by general formula (CXCI) an acid chloride. This step is carried out in the same manner as the acid halide method (a) in step B-1 of method B.
Step Va-2 is a step for producing a compound represented by general formula (CXCIV), and is achieved by reacting compound (CXCII) with a compound represented by general formula (CXCIII). This step is carried out in the same manner as the acid halide method (a) in step B-1 of method B.
Step Va-3 is a step for producing a compound represented by general formula (CXCV), and is achieved by reacting compound (CXCIV) with compound (LXXVII). This step is carried out in the same manner as step Eb-3 of method Eb.
Step Va-4 is a step for producing a compound represented by general formula (CXCVI), and is achieved by removing the protecting group of the group represented by the expression X^{e2}b. This step is carried out in the same manner as step A-1 of method A.

### <Method Vb>

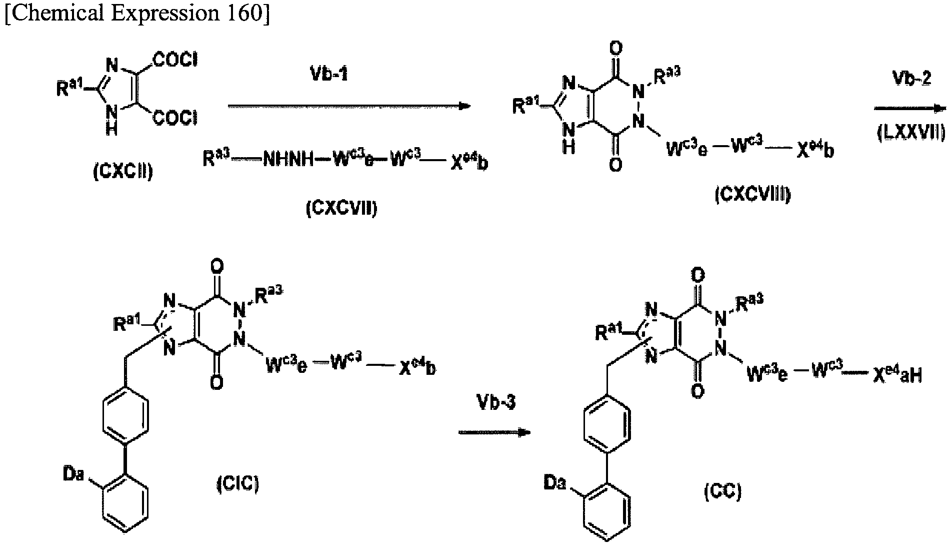

R^{a1}, R^{a3}, W^{c3}, W^{c3}e, X^{e4}a, X^{e4}b, Da, and the bond embraced by the dotted line in this expression are defined as described earlier.
Step Vb-1 is a step for producing a compound represented by general formula (CXCVIII), and is achieved by reacting compound (CXCII) with a compound represented by general formula (CXCVII). This step is carried out in the same manner as the acid halide method (a) in step B-1 of method B. Compound (CXCVIII) may also be produced by reacting compound (CXCI) with compound (CXCVII) in the same manner as step B-1 of method B.
Step Vb-2 is a step for producing a compound represented by general formula (CIC), and is achieved by reacting compound (CXCVIII) with a compound represented by general formula (LXXVII). This step is carried out in the same manner as step Eb-3 of method Eb.
Step Vb-3 is a step for producing a compound represented by general formula (CC), and is achieved by removing the protecting group of the group represented by the expression X^{e4}b. This step is carried out in the same manner as step A-1 of method A.

### <Method Vc>

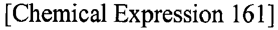

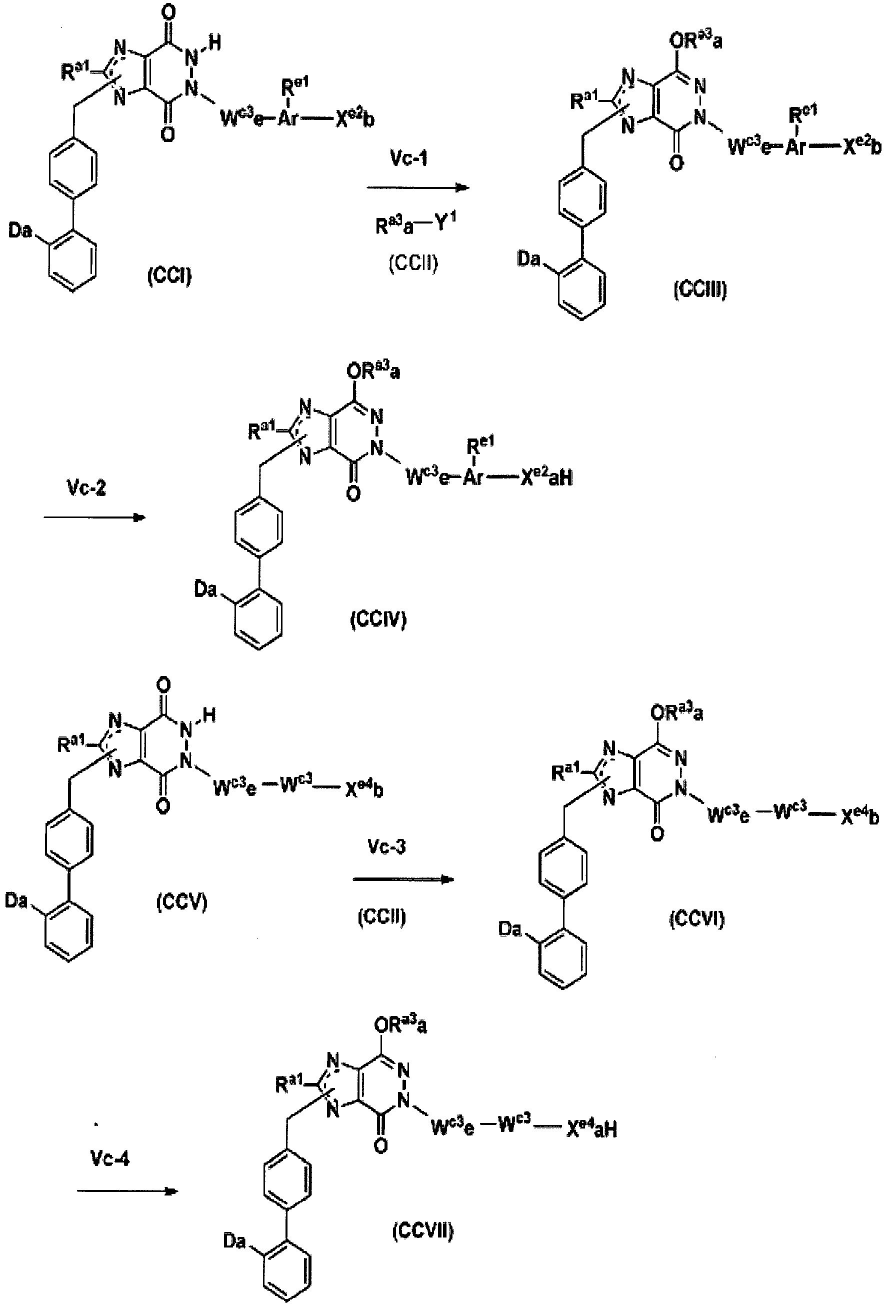

R^{a1}, W^{c3}e, W^{c3}, Ar, R^{e1}, X^{e2}a, X^{e2}b, X^{e4}a, X^{e4}b, Da, Y¹, and the bond embraced by the dotted line in this expression are defined as described earlier, and R^{a3}a is defined the same as R^{a3} except for excluding hydrogen.
Step Vc-1 is a step for producing a compound represented by general formula (CCIII), and is achieved by reacting a compound represented by general formula (CCI) with a compound represented by general formula (CCII). This step is carried out in the same manner as step Ea-1 of method Ea.
Step Vc-2 is a step for producing a compound represented by general formula (CCIV), and is achieved by removing the protecting group of the group represented by the expression X^{e2}b. This step is carried out in the same manner as step A-1 of method A.
Step Vc-3 is a step for producing a compound represented by general formula (CCVI), and is achieved by reacting compound represented by general formula (CCV) with the compound (CCII). This step is carried out in the same manner as step Ea-1 of method Ea.
Step Vc-4 is a step for producing a compound represented by general formula (CCVII), and is achieved by removing the protecting group of the group represented by the expression X^{e4}b. This step is carried out in the same manner as step A-1 of method A.

After the each reaction of the steps of every method has ended, the intended compound of the reaction is collected from the reaction mixture following a conventional method. For example, a reaction mixture is suitably neutralized, or in the case that insoluble matter is present, the insoluble matter is suitably removed by filtration, then the reaction mixture is combined with water and an organic solvent that is immiscible with water, such as ethyl acetate. After an organic layer containing the intended compound has been separated and washed with water or the like and dried using anhydrous magnesium sulfate, anhydrous sodium sulfate, anhydrous sodium bicarbonate, or the like, the solvent is distilled off to give the intended compound of the reaction. As required, the intended compound may also be refined (or the reaction mixture may be refined directly) following a conventional method (for example, distillation, recrystallization, or silica gel column chromatography).

The carboxylic acid form, carboxylic acid amide form, or carboxylic acid ester form contained in compound (XXIV) is known conventionally or produced following a conventional method (for example, Exp. Opin. Ther. Patents (1994) 4(5): 505-524, Exp. Opin. Ther. Patents (1995) 5(5): 431-458, Journal of Medicinal Chemistry, 1996, Vol. 39, No. 3, 625-656, Journal of Medicinal Chemistry, 1996, Vol. 39, No. 1, 323-338, Bioorganic & Medicinal Chemistry Letters, Vol. 4, No. 1, 81-86, 1994, and Japanese Unexamined Patent Publication No. 7-316005). A corresponding carboxylic acid form is produced by hydrolyzing the amide or ester form in the same manner as step A-1 of method A.

The compounds indicated below (where R^{a1}, R^{a2}, R^{a3}, R^{a5}, the group represented by the expression 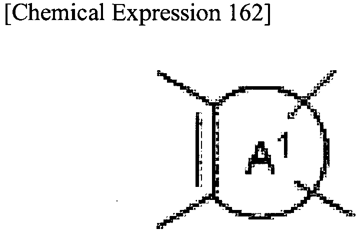 , R^{a6}, R^{a7}, L^{d}, R^{a8}, and the bond embraced by the dotted line are defined as described earlier, Db is defined as D described earlier, other than that a carboxy or imidazole may be protected, and Zp indicates amino, carboxy, methoxycarbonyl, ethoxycarbonyl, hydroxymethyl, or carboxymethyl) are known conventionally or produced following a conventional method (the literature is indicated below).

(a) Compound represented by the expression 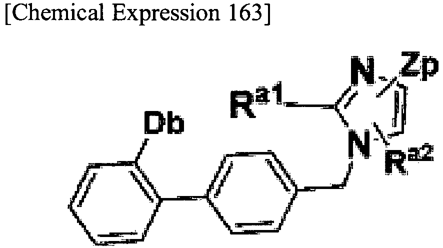 (literature: Japanese Unexamined Patent Publication No. 7-215944, Japanese Patent Application Publication No. 2-417045, Bioorganic & Medicinal Chemistry Letters, Vol. 4, No.1, pp. 63-68, 1994, Bioorganic & Medicinal Chemistry Letters, Vol. 4, No. 1, pp. 69-74, 1994, Journal of Medicinal Chemistry, 1996, Vol. 39, No. 1, pp. 323-338, International Unexamined Patent Publication No. 09/04059, Journal of Medicinal Chemistry, 1990, Vol. 33, No. 1, pp. 1312-1329, Journal of Medicinal Chemistry, 1993, Vol. 36, pp. 1772-1784, Journal of Medicinal Chemistry, 1995, Vol. 38, pp. 2357-2377, or Bioorganic & Medicinal Chemistry Letters, Vol. 4, No. 1, pp. 177-182, 1994),

(b) Compound represented by the expression 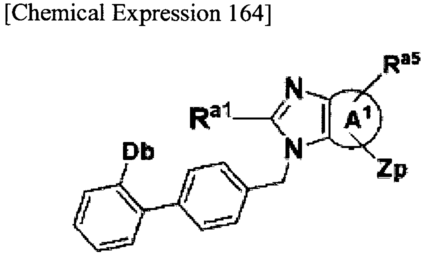
(literature: Japanese Unexamined Patent Publication No. 7-145150, Japanese Patent Application Publication No. 6-201576, Japanese Unexamined Patent Publication No. 2002-30085, Bioorganic & Medicinal Chemistry Letters, Vol. 4, No. 1, pp. 17-22, 1994, Journal of Medicinal Chemistry, 1993, Vol. 36, No. 12, pp. 1772-1784, Journal of Medicinal Chemistry, 1994, Vol. 37, No. 11, pp. 1632-1645, Journal of Medicinal Chemistry, 1996, Vol. 39, No. 26, pp. 5228-5235, or Journal of Medicinal Chemistry, 1993, Vol. 36, No. 1, pp. 2182-2195),

(c) Compound represented by the expression 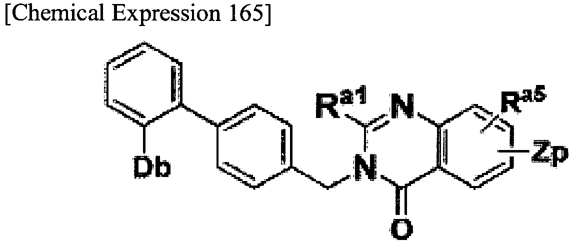 (literature: Bioorganic & Medicinal Chemistry Letters, Vol. 6, No. 8, pp. 923-928, 1996, or Bioorganic & Medicinal Chemistry Letters, Vol. 4, No. 1, pp. 81-86, 1994),

(d) Compound represented by the expression 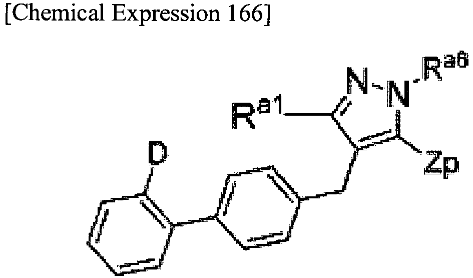 (literature: Bioorganic & Medicinal Chemistry Letters, Vol. 4, No. 1, pp. 151-156, 1994),

(e) Compound represented by the expression 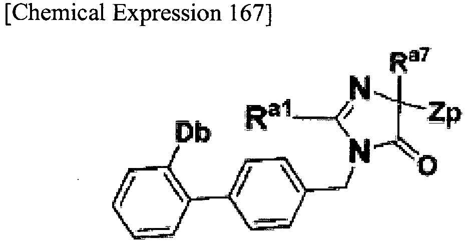 (literature: European Patent Publication No. EP 0 454 511 A1, Journal of the American Chemical Society, Vol. 74, 2892-2894, 1952, or Bioorganic & Medicinal Chemistry Letters, Vol. 4, No. 1, pp. 45-50, 1994),

(f) Compound represented by the expression 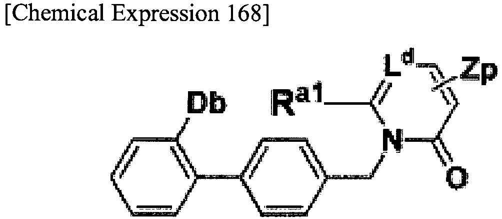
(literature: Japanese Unexamined Patent Publication No. 7-316005, or Japanese Patent Application Publication No. 6-108567),

(g) Compound represented by the expression 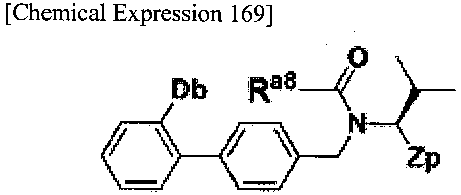 (literature: Bioorganic & Medicinal Chemistry Letters, Vol. 4, No. 1, pp. 29-34, 1994), or
(h) Compound represented by the expression 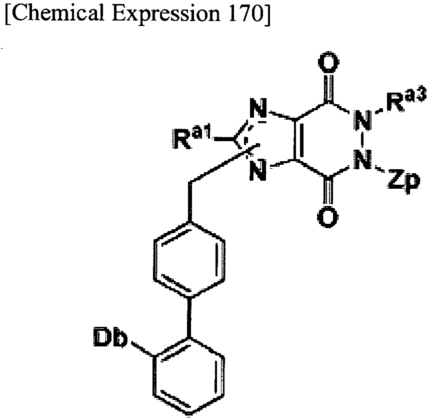 (literature: Japanese Unexamined Patent Publication No. 4-235988).

Of these compounds, for compounds in which Zp (except for the compound represented by general formula (h)) is methoxycarbonyl or ethoxycarbonyl, a corresponding carboxylic acid is produced by hydrolyzing in the same manner as step A-1 of method A, and for compounds in which Zp is hydroxymethyl, a corresponding carboxylic acid is produced by a conventional method (for example, Jones oxidation (reacting in acetone solvent at 0°C to the boiling point of the solvent for thirty minutes to three days using a sulfuric acid acidic solution of anhydrous chromic acid (a Jones reagent) as an oxidizing agent) or Sarett oxidation (reacting in chloroform at 0°C to the boiling point of the solvent for thirty minutes to three days using a pyridine solution of anhydrous chromic acid as an oxidizing agent).

In the case that the compound represented by general formula (I) or a production intermediary thereof or the like in these production methods has A or C and the structure of this A or C has tetrazol-5-yl, 2,4-dioxo-thiazolidin-5-yl, 2,4-dioxo-oxazolidin-5-yl, and/or an optionally substituted amino, (i) triphenylmethyl or (ii) Boc may be suitably bonded as a protecting group, and the protecting group may be suitably removed from these protected groups. These procedures may be carried out following a conventional method in the art of organic synthetic chemistry; for example, T. W. Green, (Protective Groups in Organic Synthesis), John Wiley & Sons; J. F. W. Mcomie (Protective Groups in Organic Synthesis), Plenum Press.

For example, when a compound is to be protected by (i) triphenylmethyl, triphenylmethyl may be bonded by reacting with triphenylmethyl chloride or triphenylmethyl bromide in dimethylformamide, tetrahydrofuran, dichloromethane, or a mixture of these solvents and in the presence of triethylamine or N,N-diisopropyl ethylamine, usually at 0°C to the boiling point of the solvent (advantageously 0°C to 70°C) for one hour to five days (advantageously two hours to two days).

When the protecting is to be removed, triphenylmethyl is removed by reacting with an acid or by contact reduction in the same manner as step A-1 of method A.

When a compound is to be protected by (ii) Boc, Boc is bonded by reacting the compound, which is to be protected, with di-t-butyl-di-carbonate in an inert solvent.
Although not specifically limited provided that it is inert in this reaction, the inert solvent used may be, for example, an aliphatic hydrocarbon such as hexane, heptane, ligroin, or petroleum ether; an aromatic hydrocarbon such as benzene, toluene, or xylene; a halogenated hydrocarbon such as dichloromethane, chloroform, 1,2-dichloroethane, or carbon tetrachloride; an ester such as methyl acetate, ethyl acetate, propyl acetate, butyl acetate, or diethyl carbonate; a ketone such as acetone or methyl ethyl ketone; an ether such as diethyl ether, diisopropyl ether, tetrahydrofuran, dioxane, dimethoxyethane, or diethylene glycol dimethyl ether; an amide such as formamide, dimethylformamide, dimethylacetamide, or hexamethylphosphoric acid triamide; or a mixture of these solvents; advantageously a halogenated hydrocarbon or an ether; and optimally chloroform, dichloromethane, 1,2-dichloroethane, diethyl ether, tetrahydrofuran, or dioxane.
Although differing depending on factors such as the ingredient compounds and the solvent, the reaction temperature is usually 0°C to the boiling point of the solvent, and advantageously 0°C to 70°c.
Although differing depending on factors such as the ingredient compounds, the solvent, and the reaction temperature, the reaction time is usually one hour to five days, and advantageously two hours to two days.

When removing, Boc is removed by reacting with acid in the same manner as step A-1 of method A.

In the case that the compound represented by general formula (I) or a production intermediary thereof or the like in these production methods has A or C and the structure of this A or C has carboxyl, the carboxy may be suitably converted to a C₁-C₆ alkoxycarbonyl, and the C₁-C₆ alkoxycarbonyl may be suitably reconverted to carboxyl. Conversion from carboxyl to a C₁-C₆ alkoxycarbonyl is carried out in the same manner as step B-1 of method B, and conversion from a C₁-C₆ alkoxycarbonyl to carboxyl is carried out in the same manner as step A-1 of method A.

In the case that the compound represented by general formula (I) or a production intermediary thereof or the like in these production methods has a hydroxy, the hydroxy may be made a protecting group as desired, and the hydroxy protecting group may be suitably removed when necessary. A hydroxy protecting group was described, for example, in method Gb. The protecting group is removed in the same manner as step A-1 of method A.

The production methods described herein may be carried out, for example, following the methods in the following literature.
Literature: Formation of C-C Bonds, Jean Matheiu and Jean Weill-Raynal, Preface by D.H.R. Barton, Georg Thieme Publishers;
Organic Syntheses, Henry Gilman, Editor-in-Chief, John Wiley & Sons;
Organic Reactions, Roger Adams, Editor-in-Chief, John Wiley & Sons;
Reagents for Organic Synthesis, Louis F. Fieser and Mary Fieser, John Wiley & Sons;
The Chemistry of Functional Groups, Saul Patai, Interscience Publishers, a division of John Wiley & Sons;
Comprehensive Organic Transformations, Richard C. Larock, VCH Publishers, Inc.;
Comprehensive Organic Chemistry, Sir Derek Barton, F.R.S. and W. David Ollis, F.R.S, Pergamon Press;
Comprehensive Organic Synthesis, Barry M. Trost, Pergamon Press; or
Comprehensive Organic Functional Group Transformations, Alan R. Katritzky, FRS, Otto Meth-Cohn, Charles W. Rees, FRS, Pergamon Press.

The following is literature regarding protecting groups.
Literature: Protective Groups in Organic Synthesis, Theodora W. Green & Peter G.M. Wuts, John Wiley & Sons;
Protecting Groups in Organic Synthesis, James R. Hanson, Sheffield Academic Press, Blackwell Science; or
Protective Groups in Organic Chemistry, J. F. W. McOmie, Plenum Press.

Methods of producing benzimidazole derivatives are also described in the following literature.
Literature: The Chemistry of Heterocyclic Compounds, A Series of Monographs, Arnold Weissberger and Edward C. Taylor, (Benzimidazoles and Congeneric Tricyclic Compounds, Edited by P.N. Preston), an Interscience Publication, John Wiley & Sons.

### EXAMPLES

The present invention will be described in greater detail hereinafter by indicating examples, reference examples, test examples, and formulation examples, but the scope of the present invention is not to be taken as limited to these examples.

### Example 1

5-(4-{6-[4-methyl-2-propyl-1-[2'-(1H-tetrazol-5-yl)biphenyl-4-ylmethyl]-1H-benzimidazol-6-ylmethoxy]-1-methyl-1H-benzimidazol-2-ylmethoxy}benzyl)thiazolidine-2,4-dione (example compound II-1)
A weight of 3.5 g of 5-(4-{4-[4-methyl-2-propyl-1-[2'-(1H-tetrazol-5-yl)biphenyl-4-ylmethyl]-1H-benzimidazol-6-ylmethoxy]-2-methylaminophenylaminocarbonylmethoxy}benzyl)thiazolidine-2,4-dione coarse product material (the compound of Reference Example 4) and 60 mL of a 4 N hydrogen chloride-dioxane solution were heated to a water-bath temperature of 60°C for 4.5 hours. The reaction mixture was combined with water and buffered to pH 4-5 using a sodium hydroxide aqueous solution. After unwanted precipitated matter had been filtered out, the residue was dried to give 2.32 g of a brown coarse product material. Refining by silica gel column chromatography (elution solvent: ethyl acetate/ethanol = 1/0 to 10/1) gave 0.13 g of the title compound as a light yellow solid.
Rf value (relative difference) (silica gel thin layer chromatography, elution solvent: ethyl acetate/ethanol = 10/1): 0.49.

¹H-NMR: spectra 00MHz, DMSO-d₆), δppm : 0.9 6 (t, 3H), 1.77 (m, 2H), 2.67 (s, 3H), 3. 11 (d, 1H), 3.23 (t, 2H), 3.33 (q. 1H), 3.90 ( s, 3H), 4.90 (q, 1H), 5. 31 (s, 2H) , 5. 52 (s ,2H), 5.83 (s, 2H), 7. 09-7. 81 (m, 17H), 1 2. 03 (s, 1H).

### Example 2

### 4'- {4-methyl-6-(1-methyl-2-[4-(2,4-dioxothiazolidin-5-ylmethyl)phenoxymethyl]-1H-benzimidazol-6-yloxymethyl)-2-propyl-1H-benzimidazol-1-ylmethyl}biphenyl-2-carboxylic acid (example compound II-6)

A weight of 0.80 g of 4'-{6-(3-(N-t-butoxycarbonyl-N-methylamino)-4-[4-(2,4-dioxothiazolidin-5-ylmethyl)phenoxymethylcarbonylamino]phenoxymethyl)-4-methyl-2-propyl-1H-benzimidazol-1-ylmethyl}biphenyl-2-carboxylic acid t-butyl ester (the compound of Reference Example 9) and 25 mL of a 4 N hydrogen chloride-dioxane solution were heated to an oil-bath temperature of 60 to 70°C for 7 hours. The reaction mixture was combined with dioxane and insoluble matter was collected by filtration, then refined by silica gel column chromatography (elution solvent: ethyl acetate/methanol = 5/1) to give 0.44 g of the title compound as a light yellow solid.
Rf value (relative difference) (silica gel thin layer chromatography, elution solvent: ethyl acetate/methanol = 5/1): 0.64.

### Example 3

### 5-[4-[6-{2-(N-(1-methyl-2-[4-methyl-2-propyl-1-(2'-(1H-tetrazoI-5-yl)biphenyl-4-ylmethyl)-1H-benzimidazol-6-yl]-1H-benzamidazol-6-yl)-N-methylamino)ethoxy} -1-methyl-1H-benzimidazol-2-ylmethoxy]benzyl]thiazolidine-2,4-dione (example compound II-7)

A weight of 0.62 g of 5-[4-[4-{2-(N-(1-methyl-2-[4-methyl-2-propyl-1-(2'-(1H-tetrazol-5-yl)biphenyl-4-ylmethyl)-1H-benzimidazol-6-yl]-1H-benzamidazol-6-yl)-N-methylamino)ethoxy} -2-(N-t-butoxycarbonyl-N-methylamino)phenylaminocarbonylmethoxy]benzyl]thiazolidine-2,4-dione (the compound of Reference Example 17) and 50 mL of a 4 N hydrogen chloride-dioxane solution were heated to an oil-bath temperature of 60°C for 6.5 hours. The reaction mixture yielded insoluble matter using the gradient method. After the insoluble matter had been combined with water and buffered to pH 9 using a 0.1 N sodium hydroxide aqueous solution, then to pH 4 using acetic acid, 0.5 g of the insoluble matter was collected by filtration. Refining by silica gel column chromatography (elution solvent: ethyl acetate/ethanol = 10/1) gave 0.04 g of the title compound as a yellow solid.
Rf value (relative difference) (silica gel thin layer chromatography, elution solvent: ethyl acetate/ethanol = 5/1): 0.51.

### Example 4

### 4'-[2-propyl-6-{N-2-[2-[4-(2,4-dioxothiazolidin-5-ylmethyl)phenoxymethyl]-1-methyl-1H-benzimidazol-6-yloxy]ethyl-N-methylamino}-1H-benzimidazol-1-ylmethoxy]biphenyl-2-carboxylic acid (example compound II-19)

A weight of 1.88 g of 4'-[2-propyl-6-{N-2-(3-(N-t-butoxycarbonyl-N-methylamino)-4-[4-(2,4-dioxothiazolidin-5-ylmethyl)phenoxymethylcarbonylamino]phenoxy)ethyl-N-methylamino}-1H-benzimidazol-1-ylmethyl]biphenyl-2-carboxylic acid t-butyl ester (the compound of Reference Example 27) and 30 mL of a 4 N hydrogen chloride-dioxane solution were heated to an oil-bath temperature of 70°C for 7 hours. The reaction mixture yielded insoluble matter using the gradient method. After the insoluble matter had been washed with diisopropyl ether and combined with ethanol and water and the solvent had been distilled off under reduced pressure, the residue was refined by silica gel column chromatography (elution solvent: methylene chloride/methanol = 5/1) to give 0.98 g of the title compound as a white solid.
Rf value (relative difference) (silica gel thin layer chromatography, methylene chloride/methanol = 5/1): 0.77.

### Example 5

### 4'-[2-propyl-5- {N-2-[2-[4-(2,4-dioxothiazolidin-5-ylmethyl)phenoxymethyl]-1-methyl-1H-benzimidazol-6-yloxy]ethyl-N-methylamino}-1H-benzimidazol-1-ylmethyl]biphenyl-2-carboxylic acid (example compound II-25)

A weight of 0.67 g of 4'-[2-propyl-5-{N-2-(3-(N-t-butoxycarbonyl-N-methylamino)-4-[4-(2,4-dioxothiazolidin-5-ylmethyl)phenoxymethylcarbonylamino]phenoxy)ethyl-N-methylamino}-1H-benzimidazol-1-ylmethyl]biphenyl-2-carboxylic acid t-butyl ester (the compound of Reference Example 28) and 20 mL of a 4 N hydrogen chloride-dioxane solution were heated to an oil-bath temperature of 70°C for 8 hours. The reaction mixture yielded insoluble matter using the gradient method. This insoluble matter was washed with diisopropyl ether, then refined by silica gel column chromatography (elution solvent: methylene/methanol = 5/1) to give 0.29 g of the title compound as a white solid.
Rf value (relative difference) (silica gel thin layer chromatography, methylene chloride/methanol = 5/1): 0.74.

### Example 6

### 4'-[4-methyl-2-propyl-6- {4-(1-methyl-2-[4-(2,4-dioxothiazolidin-5-ylmethyl)phenoxymethyl]-1H-benzimidazol-6-yloxy)phenylthiomethyl}-1H-benzimidazol-1-ylmethyl]biphenyl-2-carboxylic acid (example compound II-27)

A weight of 0.94 g of 4'-[6-{4-(3-(N-t-butoxycarbonyl-N-methylamino)-4-[4-(2,4-dioxothazolidin-5-ylmethyl)phenoxymethylcarbonylamino]phenoxy)phenylthiomethyl} -4-methyl-2-propyl-1H-benzimidazol-1-ylmethyl]biphenyl-2-carboxylic acid t-butyl ester (the compound of Reference Example 33) and 30 mL of a 4 N hydrogen chloride-dioxane solution were heated to an oil-bath temperature of 60-70°C for 7 hours. The reaction mixture yielded insoluble matter using the gradient method. This insoluble matter was washed with dioxane, then refined by silica gel column chromatography (elution solvent: ethyl acetate/methanol = 10/1) to give 0.10 g of the title compound as a light red powder.
Rf value (relative difference) (silica gel thin layer chromatography, elution solvent: ethyl acetate/methanol = 5/1): 0.70.

### Example 7

### 4- {2-[4-(2,4-dioxothiazolidin-5-ylmethyl)phenoxymethyl]-1-methyl-1H-benzimidazol-6-yloxymethyl}-2-propyl-1-[2'-(1H-tetrazol-5-yl)biphenyl-4-ylmethyl]-1H-imidazole-5-carboxylic acid ethyl ester (example compound I-149)

A weight of 2.57 g of 4-[3-(N-t-butoxycarbonyl-N-methylamino)-4-{4-(2,4-dioxothiazolidin-5-ylmethyl)phenoxyacetylamino}phenoxymethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 36) and 50 mL of a 4 N hydrogen chloride-dioxane solution were heated to an oil-bath temperature of 60 to 120°C for 6.5 hours. The reaction mixture was combined with 50 mL of toluene, and the insoluble matter was collected by filtration and dried to give 2.14 g of a light red-brown solid. Of this quantity, 0.60 g was stirred with 30 mL of a 4 N hydrogen chloride-dioxane solution and 10 mL of anhydrous ethanol at room temperature for three days. Precipitated insoluble matter was collected by filtration to give 0.44 g of a hydrochloride of the title compound as a light red powder.
Rf value (relative difference) (silica gel thin layer chromatography, elution solvent: ethyl acetate/methanol = 9/1): 0.78.

### Example 8

### 4-[4- {2-[4-(2,4-dioxothiazolidin-5-ylmethyl)phenoxymethyl]-1-methyl-1H-benzimidazol-6-yloxy}phenylthiomethyl]-2-propyl-1-[2'-(1H-tetrazol-5-yl)biphenyl-4-ylmethyl]-1H-imidazole-5-carboxylic acid ethyl ester (example compound I-1)

(a) A weight of 1.22 g of 4-{4-[4-[4-(2,4-dioxothiazolidin-5-ylmethyl)phenoxyacetylamino]-3-(N-t-butoxycarbonyl-N-methylamino)phenoxy]phenylthiomethyl} -2-propyl-1-[2'-(1-triphenylmethyl-1H-tetrazol-5-yl)biphenyl-4-ylmethyl]-1H-imidazol-5-carboxylic acid ethyl ester (the compound of Reference Example 40-1) and 30 mL of a 4 N hydrogen chloride-dioxane solution were heated to an oil-bath temperature of 70 to 80°C for 5 hours. The solvent was removed from the reaction mixture using the gradient method, and the residue was dried, then refined by silica gel column chromatography (elution solvent: ethyl acetate/methanol = 5/2) to give 0.53 g of the title compound as a white solid.
   Rf value (relative difference) (silica gel thin layer chromatography, elution solvent: ethyl acetate/methanol = 5/1): 0.50.

¹H-NMR spectra (500MHz, DMSO-d₆). *δ*ₚₚₘ :0.8 3 (t. 3H), 1.15 (t, 3H), 1.56 (m, 2H), 2.72 (t, 2H), 3.11 (q, 1H), 3.32 (d, 1H), 3.86 ( s, 3H), 4.08 (q. 2H), 5. 54 (s. 2H), 5. 55 (s , 2H), 6.92-7.72 (m, 19H), 12.04(s,1H).

(b) A weight of 1.55 g of 4-{4-[4-[4-(2,4-dioxothiazolidin-5-ylmethyl)phenoxyacetylamino]-3-(N-t-butoxycarbonyl-N-methylamino)phenoxy]phenylthiomethyl}-2-propyl-1-[2'-(1H-tetrazol-5-yl)biphenyl-4-ylmethyl]-1H-imidazol-5-carboxylic acid ethyl ester (the compound of Reference Example 40-2) and 25 mL of a 4 N hydrogen chloride-dioxane solution were heated to an oil-bath temperature of 80°C for 10 hours. The solvent was removed from the reaction mixture using the gradient method, and the residue was combined with water and made weakly acidic using ammonia water and dilute hydrochloric acid, following which, the precipitate was collected by filtration. The precipitate was refined by silica gel column chromatography (elution solvent: ethyl acetate) to give 1.13 g of the title compound as a white solid.
   Of the resulting title compound, 0.6 g was dissolved in ethanol and combined with an excess of a 4 N hydrogen chloride-dioxane solution, then condensed and combined with a large volume of ethyl acetate. A precipitated white solid was collected by filtration to give 0.6 g of a hydrochloride of the title compound.

### Example 9

### 4'-[6-(N-{2-[4-(2,4-dioxothiazolidin-5-ylmethyl)phenoxymethyl]-3-methyl-3H-imidazo[4,5-b]pyridin-5-yl}-N-methylaminomethyl)-4-methyl-2-propyl-1H-benzimidazol-1-ylmethyl]biphenyl-2-carboxylic acid methyl ester (example compound II-31)

A weight of 0.60 g of 4'-[6-(N-{3-[4-(2,4-dioxothiazolidin-5-ylmethyl)phenoxyacetylamino]-2-(N-methylamino)pyridin-6-yl}-N-methylaminomethyl)-4-methyl-2-propyl-1H-benzimidazol-1-ylmethyl]biphenyl-2-carboxylic acid methyl ester (the compound of Reference Example 48) and 20 mL of a 4 N hydrogen chloride-dioxane solution were heated to an oil-bath temperature of 80°C for 7 hours. The reaction mixture was condensed, and combined with methanol and ammonia water. The residue resulting from distilling off the solvent was refined by silica gel column chromatography (elution solvent: ethyl acetate) to give 0.25 g of the title compound as a light yellow powder.
Rf value (relative difference) (silica gel thin layer chromatography, ethyl acetate): 0.44.

Mass spectrum: 808 (M+1)⁺.
¹H-NMR spectra (500MHz, DMSO-d₆), δₚₚₘ: 0.9 4 (t, 3H), 1. 7 5 (m, 2H) , 2. 4 7 (s, 3H), 2. 81 (t, 2H) , 3. 05 (q. 1H) 3. 08 (s, 3H), 3. 30 ( q, 1H), 3. 53 (s, 2H) , 3. 68 (s, 3H) , 4.87 (m 1H) , 5. 24 (s, 2H), 5. 43 (s, 2H) , 6. 58-7. 76 (m, 16H)

### Example 10

### 4'-[6-(N-{2-[4-(2,4-dioxothiazolidin-5-ylmethyl)phenoxymethyl]-3-methyl-3H-imidazo[4,5-b]pyridin-5-yl} -N-methylaminomethyl)-4-methyl-2-propyl-1H-benzimidazol-1-ylmethyl]biphenyl-2-carboxylic acid (example compound II-35)

A weight of 0.23 g of 4'-[6-(N-{2-[4-(2,4-dioxothiazolidin-5-ylmethyl)phenoxymethyl]-3-methyl-3H-imidazo[4,5-b]pyridin-5-yl} -N-methylaminomethyl)-4-methyl-2-propyl-1H-benzimidazol-1-ylmethyl]biphenyl-2-carboxylic acid methyl ester (the compound of Example 9) and a mixture of 5 mL of concentrated hydrochloric acid and 15 mL of acetic acid were heated and stirred at 80-90°C overnight, then combined with 10 mL of concentrated hydrochloric acid and heated and stirred at 80-90°C all day long. The solvent was distilled off under reduced pressure, and the residue was dissolved in 75% ethanol, then the solvent was distilled off again under reduced pressure to give a coarse product material. Next, this material was refined by silica gel column chromatography (elution solvent: ethyl acetate/methanol = 4/1 to 2/1) to give 0.10 g of the title compound as a white powder.
Rf value (relative difference) (silica gel thin layer chromatography, elution solvent: ethyl acetate/methanol = 5/1): 0.60.

Mass spectrum: 794 (M+1)⁺.
¹H-NMR spectra (500MHz, DMSO-d₆). δₚₚₘ : 0 . 9 5 (t, 3 H) , 1. 76 (m, 2H) , 2. 57 (s, 3H) , 3. 06 (t, 2H) , 3. 09 (s, 3H) , 3. 17 (q, 1H) , 3. 69 ( s, 2H) , 4. 88 (m, 1H) , 4. 93 (s, 2H) , 5. 26 (s , 2H) , 5. 68 (s, 2H), 6. 61 - 7. 8 0 (m, 16H), 1 2. 02 (s, 1H) .

### Example 11

### 5-(4-{6-[2-butyl-4-chloro-1-[2'-(1H-tetrazol-5-yl)biphenyl-4-ylmethyl]-1H-imidazol-5-ylmethylthio]-1-methyl-1H-benzimidazol-2-ylmethoxy}benzyl)thiazolidine-2,4-dione (example compound I-283)

A weight of 0.23 g of 5-(4-{4-(4-chloro-2-butyl-1-[2'-(1H-tetrazol-5-yl)biphenyl-4-ylmethyl]-1H-imidazol-5-ylmethylthio)-2-(N-t-butoxycarbonyl-N-methylamino)phenylaminocarbonylmethoxy}benzyl)thiazolidine-2,4-dione (the compound of Reference Example 53) and 10 mL of a 4 N hydrogen chloride-dioxane solution were heated to an oil-bath temperature of 80°C for 7 hours. The solvent was removed from the reaction mixture using the gradient method, and the residue was dried, then dissolved in methanol and combined with a large volume of ethyl acetate. A precipitated white solid was collected by filtration to give 0.22 g of a hydrochloride of the title compound.

Mass spectrum: 804 (M+1)⁺.
¹H-NMR spectra 500MHz, DMSO-d₆) , δₚₚₘ : 0 7 (t, 3H) , 1. 44 (m, 2H) , 2. 4 6 (t, 2H) , 3. 11 (q, 1H) , 3. 34 (q, 2H), 3. 95 (s, 3H) , 4. 23 ( s, 2H) , 4. 91 (m, 1H) , 5. 3 2 (s, 2H) , 5. 63 (s 2H) ,6. 94-7. 71 (m, 15H), 7. 91 (s, 1H), 1 2. 03 (s, 1H) .

### Example 12

### 5-(4-{6-[2-(4-methyl-2-propyl-1-[2'-(1H-tetrazol-5-yl)biphenyl-4-ylmethyl]-1H-benzimidazol-6-yl)-1-methyl-1H-benzimidazol-6-ylthio]-1-methyl-1H-benzimidazol-2-ylmethoxy}benzyl)thiazolidine-2,4-dione (example compound II-45)

A weight of 0.90 g of 5-(4-{4-[3-(N-t-butoxycarbonyl-N-methylamino)-4-(4-methyl-2-propyl-1-[2'-(1H-tetrazol-5-yl)biphenyl-4-ylmethyl]-1H-benzimidazol-6-ylcarbonylamino)phenylthio]-2-(N-t-butoxycarbonyl-N-methylamino)phenylaminocarbonylmethoxy}benzyl)thiazolidine-2,4-dione (the compound of Reference Example 57) and 23 mL of a 4 N hydrogen chloride-dioxane solution were heated to an oil-bath temperature of 75°C for one day. The solvent was removed from the reaction mixture using the gradient method, and the residue was dissolved in methanol and poured into a large volume of ethyl acetate. A precipitated light yellow powder was collected by filtration to give 0.52 g of a hydrochloride of the title compound.

Mass spectrum: 936 (M+1)⁺.
¹H-NMR spectra 500MHz, DMSO-d₆) δₚₚₘ : 0. 9 7 (t. 3H) , 1. 76 (m, 2H) , 2. 74 (s, 3H) ,3. 10 (q, 1H) , 3. 19 (t, 2H) , 3. 33 (q, 1H) , 3. 91 ( s, 3H) ,3. 9 4 (s, 3H) , 4. 90 (q, 1H), 5. 59 (s , 2 H) , 5. 81 (s, 2H) , 7.10-8.16 (m, 20H), 1 2. 03 (s, 1H) .

### Example 13

4-[4- {2-[4-(2,4-dioxothiazolidin-5-ylmethyl)phenoxymethyl]-1-methyl-1H-benzimidazol-6-ylthio} -2-methylaminophenylaminocarbonyl]-2-propyl-1-[2'-(1H-tetrazol-5-yl)biphenyl-4-ylmethyl]-1H-imidazole-5-carboxylic acid ethyl ester (example compound I-145)
A weight of 0.45 mg of 4-[4-{4-[4-(2,4-dioxothiazolidin-5-ylmethyl)phenoxyacetylamino]-3-(N-t-butoxycarbonyl-N-methylamino)phenylthio}-2-(N-t-butoxycarbonyl-N-methylamino)phenylaminocarbonyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 60) and 20 mL of a 4 N hydrogen chloride-dioxane solution were heated to an oil-bath temperature of 60°C for fifteen hours. The solvent was removed from the reaction mixture using the gradient method, and the residue was dissolved in methanol and poured into a large volume of ethyl acetate. A precipitated light yellow solid was collected by filtration to give 0.34 g of a hydrochloride of the title compound.

Mass spectrum: 962 (M+1)⁺.

### Example 14

### 4-[2-[4- {1-methyl-2-(4-(2,4-dioxothiazolidin-5-ylmethyl)phenoxymethyl)-1H-benzimidazol-6-yloxy}phenylthio]ethylthiomethyl]-2-propyl-1-[2'-(1H-tetrazol-5-yl)biphenyl-4-ylmethyl]-1H-imidazole-5-carboxylic acid ethyl ester (example compound I-124)

A weight of 0.64 mg of 4-[2-[4-{3-(N-t-butoxycarbonyl-N-methylamino)-4-[4-(2,4-dioxothiazolidin-5-ylmethyl)phenoxyacetylamino]phenoxy}phenylthio]ethylthiomethyl]-2-propyl-1-[2'-(1H-tetrazol-5-yl)biphenyl-4-ylmethyl]-1H-imidazole-5-carboxylic acid ethyl ester (the compound of Reference Example 66) and 20 mL of a 4 N hydrogen chloride-dioxane solution were heated to an oil-bath temperature of 65°C for seventeen hours. The solvent was removed from the reaction mixture using the gradient method, and the residue was dissolved in methanol and poured into a large volume of ethyl acetate. A precipitated light red solid was collected by filtration to give 0.48 g of a hydrochloride of the title compound.

Mass spectrum: 980 (M+1)⁺.
¹H-NMR spectra (500MHz, DMSO-d₆) δₚₚₘ:0. 8 4 (t, 3H), 1. 17 (t, 3H), 1.57 (m, 2H) , 2. 7 8 (m, 2H+2H) , 3. 1 2 q, 1H) , 3. 14 (t, 2H, 3. 33 (d, 1H), 3. 92 (s, 3H) , 4. 03 (s, 2H). 4. 2 0 (q, 2H), 4. 91 (q, 1H), 5. 61 (s, 2H+2H), 6 . 97 - 7. 81 (m, 19H)., 12. 04 (s, 1H).

### Example 15

### 4-[2-[1-methyl-2- {4-(2,4-dioxothiazolidin-5-ylmethyl)phenoxymethyl} -1H-benzimidazol-6-yloxy]ethylthiomethyl]-2-propyl-1-[2'-(1H-tetrazol-5-yl)biphenyl-4-ylmethyl]-1H-imidazole-5-carboxylic acid ethyl ester (example compound I-127)

A weight of 0.125 g of 4-[2-[3-(N-t-butoxycarbonyl-N-methylamino)-4-{4-(2,4-dioxothiazolidin-5-ylmethyl)phenoxyacetylamino}phenoxy]ethylthiomethyl]-2-propyl-1-[2'-(1H-tetrazol-5-yl)biphenyl-4-ylmethyl]imidazole-5-carboxylic acid ethyl ester (the compound of Reference Example 69) and 10 mL of a 4 N hydrogen chloride-dioxane solution were heated to an oil-bath temperature of 65°C for seventeen hours. The solvent was removed from the reaction mixture using the gradient method, and the residue was dissolved in methanol and poured into a large volume of ethyl acetate. A precipitated light yellow solid was collected by filtration to give 65 mg of a hydrochloride of the title compound.

Mass spectrum: 872 (M+1)⁺.
¹H-NMR. spectra (500MHz, DMSO-d₆) , δₚₚₘ 0.8 3 (t, 3H) , 1. 1 7 (t, 3H), 1. 5 7 (m, 2H), 2. 7 6 (t, 2H), 3. 04 (t, 2H), 3. 12 (q, 1H), 3. 33 ( d, 1H), 3. 99 (s, 3H), 4. 11 (s, 2H) , 4. 19 (q , 2H) , 4. 30 (t, 2H) , 4. 91 (q, 1H), 5. 62 (s, 2 H), 5. 6 5 (s, 2H) , 6. 98-7. 7 2 (m, 15H), 1 2 . 04 (s. 1H) .

### Example 16

### 5-(4- {6-[4-(4-methyl-2-propyl-1-[2'-(1H-tetrazol-5-yl)biphenyl-4-ylmethyl]-1H-benzimidazol-6-ylcarbonylamino)phenoxy]-1-methyl-1H-benzimidazol-2-ylmethoxy}benzyl)thiazolidine-2,4-dione (example compound II-48)

A mixture of 0.43 g of 5-(4-{6-[4-(4-methyl-2-propyl-1-[2'-(1-triphenylmethyl-1H-tetrazol-5-yl)biphenyl-4-ylmethyl]-1H-benzimidazol-6-ylcarbonylamino)phenoxy]-1-methyl-1H-benzimidazol-2-ylmethoxy}benzyl)thiazolidine-2,4-dione (the compound of Reference Example 71), 20 mL of methanol, and 5 mL of acetic acid was heated and stirred at 60-65°C for four hours. The solvent was distilled off, and the residue was combined with ethyl acetate and water. The aqueous layer was made pH 5-6 using a sodium hydroxide aqueous solution, and the ethyl acetate layer was separated. After drying the ethyl acetate layer over anhydrous sodium sulfate, the solvent was distilled off. The resulting residue was refined by silica gel column chromatography (elution solvent: ethyl acetate/ethanol = 1/0 to 20/1 then 5/1), the solution was condensed and combined with n-hexane, and a precipitated solid was collected by filtration to give 0.27 g of the title compound as a white solid.
Rf value (relative difference) (silica gel thin layer chromatography, elution solvent: ethyl acetate/ethanol = 10/1): 0.45.

### Example 17

### 5-(4-{6-[4-(2-ethoxy-1-[2'-(1H-tetrazol-5-yl)biphenyl-4-ylmethyl]-1H-benzimidazol-7-ylcarbonylamino)phenoxy]-1-methyl-1H-benzimidazol-2-ylmethoxy}benzyl)thiazolidine-2,4-dione (example compound II-52)

Weights of 147 mg of 2-ethoxy-1-[2'-(1H-tetrazol-5-yl)biphenyl-4-ylmethyl]-1H-benzimidazole-7-carboxylic acid and 170.3 mg of 5-[4-{6-(4-aminophenoxy)-1-methyl-1H-benzimidazol-2-ylmethoxy}benzyl]thiazolidine-2,4-dione dihydrochloride were combined with 1 mL of dry dimethylformamide followed by 45 mg of triethylamine. The mixture was sonicated, then combined with 64 mg of WSC (1-ethyl-3-(3-dimethylaminopropyl)carbodiimide hydrochloride) and stirred at room temperature for fifteen hours. The reaction solution was poured into water, and a precipitated sediment was collected by filtration and refined by silica gel column chromatography (elution solvent: ethyl acetate/methanol = 10/1) to give 0.18 g of the title compound as a light yellow powder.
Rf value (relative difference) (silica gel thin layer chromatography, elution solvent: ethyl acetate/methanol = 5/1): 0.68.

Mass spectrum: 897 (M+1)⁺.

### Example 18

### 4-1-methyl-2-{4-(2,4-dioxothiazolidin-5-ylmethyl)phenoxymethyl}-1H-benzimidazol-6-ylthioacetylamino]-2-propyl-1-[2'-(1H-tetrazol-5-yl)biphenyl-4-ylmethyl]-1H-imidazole-5-carboxylic acid ethyl ester (example compound I-147)

A weight of 0.563 mg of 4-[1-methyl-2-{4-(2,4-dioxothiazolidin-5-ylmethyl)phenoxymethyl} -1H-benzimidazol-6-ylthioacetylamino]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 77) and 20 mL of a 4 N hydrogen chloride-dioxane solution were heated to an oil-bath temperature of 60°C for six hours. The solvent was removed from the reaction mixture using the gradient method, and the residue was washed with dioxane, then dissolved in a small volume of methanol and poured into a large volume of ethyl acetate. A precipitated white solid was collected by filtration to give 0.327 g of a hydrochloride of the title compound.

Mass spectrum: 871 (M+1)⁺.
¹H-NMR spectra (500MHz, DMSO-d₆) , δₚₚₘ: 0. 8 6 (t, 3H), 1. 09 (t, 3H), 1. 56 (m, 2H), 2. 65 (t, 2H), 3. 1 2 (q, 1H) , 3. 34 (d, 1H), 3. 98 ( s, 3H), 4. 09 (s, 2H), 4. 1 0 (q, 2H), 4. 91 (q 1H), 5. 52 (s, 2H), 5. 64 (s, 2 H) , 6. 96-7. 76 (m, 15 H), 8 . 02 (s, 1H), 12. 04 (s, 1H).

### Example 19

### 4-[2-[1-methyl-2-{4-(2,4-dioxothiazolidin-5-ylmethyl)phenoxymethyl} -1H-benzimidazol-6-ylthioacetylamino]ethylthiomethyl]-2-propyl-1-[2'-(1H-tetrazol-5-yl)biphenyl-4-ylmethyl]-1H-imidazole-5-carboxylic acid ethyl ester (example compound I-148)

A weight of 0.18 g of 4-[2-[-methyl-2-{4-(2,4-dioxothiazolidin-5-ylmethyl)phenoxymethyl}-1H-benzimidazol-6-ylthioacetylamino]ethylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 79) and 10 mL of a 4 N hydrogen chloride-dioxane solution were heated to an oil-bath temperature of 60°C for sixteen hours. The solvent was removed from the reaction mixture using the gradient method, and the residue was dissolved in a small volume of methanol and poured into a large volume of ethyl acetate. A precipitated light yellow powder was collected by filtration and dried to give 95 mg of a hydrochloride of the title compound.

Mass spectrum: 945 (M+1)⁺.
¹H-NMR spectra 500MHz, DMSO-d₆) , δₚₚₘ : 0. 8 4 (t, 3H), 1. 17 (t, 3H), 1. 58 (m, 2H), 2. 62 (t, 2H) , 2. 80 (t, 2H), 3.10 (q, 1H), 3.26 ( t, 2H), 3. 33 (d, 1H), 3.96 (s, 3H) , 3.99 (s 2H), 4. 21 (q, 2H), 4. 91 (q, 1H), 5. 61 (s, 2H), 5. 63 (s,2H), 7. 00-7, 93 (m, 15H), 8. 46 (s, 1H), 12. 04 (s, 1H).

### Example 20

### 4- {2-[4-(2,4-dioxothiazolidin-5-ylmethyl)phenoxymethyl]-1-methyl-1H-benzimidazol-6-yloxymethyl}-2-propyl-1-[2'-(1H-tetrazol-5-yl)biphenyl-4-ylmethyl]-1H-imidazole-5-carboxylic acid (example compound I-150)

A mixture of 0.75 g of a hydrochloride of 4-{2-[4-(2,4-dioxothiazolidin-5-ylmethyl)phenoxymethyl]-1-methyl-1H-benzimidazol-6-yloxymethyl} -2-propyl-1-[2'-(1H-tetrazol-5-yl)biphenyl-4-ylmethyl]-1H-imidazole-5-carboxylic acid ethyl ester (the compound of Example 7), 10 mL of concentrated hydrochloric acid, and 20 mL of acetic acid was heated and stirred at 90°C for seventeen hours. The mixture was combined with 15 mL more of concentrated hydrochloric acid and heat-refluxed for ten hours. The reaction solution was condensed under reduced pressure, and the residue was refined by silica gel column chromatography (elution solvent: ethyl acetate/methanol = 3/2) and recrystallized from methanol to give 0.18 g of the title compound as a white crystal.
Rf value (relative difference) (silica gel thin layer chromatography, elution solvent: ethyl acetate/methanol = 3/2): 0.52.

### Example 21

### 4-[4- {2-[4-(2,4-dioxothiazolidin-5-ylmethyl)phenoxymethyl]-1-methyl-1H-benzimidazol-6-yloxy}phenylthiomethyl]-2-propyl-1-[2'-(1H-tetazol-5-yl)biphenyl-4-ylmethyl]-1H-imidazole-5-carboxylic acid (example compound I-39)

The reaction of Example 8 was carried out using 1.35 g of 4-{4-[4-[4-(2,4-dioxothiazolidin-5-ylmethyl)phenoxyacetylamino]-3-(N-t-butoxycarbonyl-N-methylamino)phenoxy]phenylthiomethyl} -2-propyl-1-[2'-(1-triphenylmethyl-1H-tetrazol-5-yl)biphenyl-4-ylmethyl]-1H-imidazole-5-carboxylic acid ethyl ester (the compound of Reference Example 40-1) and 9.5 mL of a 4 N hydrogen chloride-dioxane solution. A mixture of the resulting coarse product material of 4-[4-{2-[4-(2,4-dioxothiazolidin-5-ylmethyl)phenoxymethyl]-1-methyl-1H-benzimidazol-6-yloxy} phenylthiomethyl]-2-propyl-1-[2'-(1H-tetrazol-5-yl)biphenyl-4-ylmethyl]-1H-imidazole-5-carboxylic acid ethyl ester, 20 mL of concentrated hydrochloric acid, and 20 mL of acetic acid was heated and stirred at 80-90°C for four days. The reaction mixture was emptied into water, and a precipitated gray powder was collected by filtration, dissolved in a small volume of methanol, and poured into a large volume of ethyl acetate. A precipitated gray solid was filtered out, and the filtrate was condensed. The precipitated solid was collected by filtration and dried to give 0.336 g of a hydrochloride of the title compound.

Mass spectrum: 892 (M+1)⁺.
¹H-NMR: spectra (500MHz, DMSO-d₆), δₚₚₘ : 0. 8 1 (t, 3H), 1. 52 (m, 2H), 2. 7 4 (t, 2H), 3. 10 (q, 1H), 3. 3 2 (q, 1H) , 3. 83 (t, 3H), 4. 38 ( s, 2H), 4. 90 (q, 1H), 5.51 (s, 2H), 5. 61 (s 2H), 6. 92-7. 70 (m, 19H), 12. 03 (s, 1H).

### Example 22

### 4'-[6-{6-(N-2-[4-(2,4-dioxothiazolidin-5-ylmethyl)phenoxy]ethyl-N-methylamino)-1-methyl-1H-benzimidazol-2-yl} -4-methyl-2-propyl- 1H-benzimidazol-1-ylmethyl]biphenyl-2-carboxylic acid (example compound IV-1)

A solution of 0.20 g of 4'-[6-{6-(N-2-[4-(2,4-dioxo-3-triphenylmethylthiazolidin-5-ylmethyl)phenoxy]ethyl-N-methylamino)-1-methyl-1H-benzimidazol-2-yl} -4-methyl-2-propyl-1H-benzimidazol-1-ylmethyl]biphenyl-2-carboxylic acid t-butyl ester (the compound of Reference Example 84) in 10 mL of acetic acid was combined with 10 mL of a 4 N hydrogen chloride-dioxane solution, and stirred at room temperature for four days. The mixture was then heated and stirred at an oil-bath temperature of 70°C for two days. This solvent was removed under reduced pressure, and the residue was combined with toluene and sonicated. A precipitated solid was collected by filtration, and dried to give 0.105 g of a hydrochloride of the title compound as a light yellow powder.

Mass spectrum: 793 (M+1)⁺.
¹H-NMR spectra 500MHz, DMSO-d₆), δₚₚₘ : 1. 0 0 (t, 3H) , 1. 83 (m, 2H), 2. 74 (s, 3H), 3. 06 (q, 1H), 3. 12 (s, 3H), 3. 28 (q, 1H), 3. 90 ( t, 2H), 3. 91 (s, 3H), 4. 1 8 (t, 2H), 4. 85 (q 1H), 5. 81 (s, 2H), 6. 84-7. 72 (m, 17H), 8 . 26 (s, 1H), 11. 99 (s, 1H).

### Example 23

### 5-(4- {6-[2-butyl-4-chloro-1-[2'-(1H-tetrazol-5-yl)biphenyl-4-ylmethyl]-1H-imidazol-5-ylmethylsulfinyl]-1-methyl-1H-benzimidazol-2-ylmethoxy}benzyl)thiazolidine-2,4-dione (example compound I-285)

A mixture of 115 mg of a hydrochloride of 5-(4-{6-[2-butyl-4-chloro-l-[2'-(1H-tetrazol-5-yl)biphenyl-4-ylmethyl]-1H-imidazol-5-ylmethylthio]-1-methyl-1H-benzimidazol-2-ylmethoxy}benzyl)thiazolidine-2,4-dione (the compound of Example 11), 3 mL of methylene chloride, and 1 mL of methanol was combined with 35 mg of m-chloroperbenzoic acid (purity: 65% or greater) and stirred at room temperature for two hours. The solvent was distilled off, and the resulting residue was combined with ethyl acetate and water. The ethyl acetate layer was separated, washed with saturated physiological saline, and dried over anhydrous sodium sulfate. The solvent was condensed to about 100 mL and combined with 0.5 mL of a 4 N hydrogen chloride-dioxane solution. A precipitated solid was collected by filtration and dried to give 83 mg of a hydrochloride of the title compound as a white solid.

Mass spectrum: 820 (M+1)⁺.
¹H-NMR spectra 500MHz, DMSO-d₆), δₚₚₘ: 0. 8 1 (t, 3H), 1. 24 (m, 2H), 1. 4 6 (m, 2H), 2. 49 (t, 2H), 3.10 (q, 1H), 3. 32 (d, 1H), 3. 95 ( s, 3H), 4. 1 1 (d, 2H), 4. 31 (d, 1H), 4. 90 (q , 1H), 5. 30 (q, 2H), 5. 58 (s, 2H), 6. 94-7. 93 (m, 15H), 12. 03 (s, 1H).

### Example 24

### 5-(4- {6-[2-butyl-4-chloro-1-[2'-(1H-tetrazol-5-yl)biphenyl-4-ylmethyl]-1H-imidazol-5-ylmethylsulfonyl]-1-methyl-1H-benzimidazol-2-ylmethoxy} benzyl)thiazolidine-2,4-dione (example compound I-286)

A mixture of 0.30 g of a hydrochloride of 5-(4-{6-[2-butyl-4-chloro-1-[2'-(1H-tetrazol-5-yl)biphenyl-4-ylmethyl]-1H-imidazol-5-ylmethylthio]-1-methyl-1H-benzimidazol-2-ylmethoxy}benzyl)thiazolidine-2,4-dione (the compound of Example 11), 3 mL of methylene chloride, and 2 mL of methanol was combined with 0.19 g of m-chloroperbenzoic acid (purity: 65% or greater) and stirred at room temperature for 5.5 hours. The reaction mixture was poured into a large volume of ethyl acetate, and a precipitated solid was collected by filtration and dried to give 0.18 g of a hydrochloride of the title compound as a light red solid.

Mass spectrum: 836 (M+1)⁺.
¹H-NMR spectra (500MHz, DMSO-d₆), δₚₚₘ: 0. 8 0 (t, 3H), 1. 21 (m, 2H),1. 43 (m, 2H), 2. 46 (t, 2H), 3. 1 0 q, 1H), 3. 32 (q, 1H), 3. 96 ( s, 3 H), 4. 63 (s, 2H), 4. 90 (q, 1H), 5. 30 (s , 2H), 5. 53 (s, 2H), 6.93-8.17 (m, 15H), 1 2. 0 3 (s, 1H) .

### Example 25

### 4-[4-{2-[4-(2,4-dioxothiazolidin-5-ylmethyl)phenoxymethyl]-1-methyl-1H-benzimidazol-6-yloxy} phenylsulfinylmethyl]-2-propyl-1-[2'-(1H-tetrazol-5-yl)biphenyl-4-ylmethyl]-1H-imidazole-5-carboxylic acid ethyl ester (example compound I-303)

A weight of 2.25 g of 4-{4-[4-[4-(2,4-dioxothiazolidin-5-ylmethyl)phenoxyacetylamino]-3-(N-t-butoxycarbonyl-N-methylamino)phenoxy]phenylsufinylmethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 85) and 50 mL of a 4 N hydrogen chloride-dioxane solution were heated to an oil-bath temperature of 70°C for sixteen hours. A precipitated yellow solid was collected by filtration, dissolved in a small volume of methanol, and poured into a large volume of ethyl acetate. A precipitated light yellow solid was collected by filtration and dried to give 1.85 g of a hydrochloride of the title compound.

¹H-NMR spectra 500MHz, DMSO-d₆), δₚₚₘ: 0. 8 3 (t, 3H), 1. 15 (t, 3H), 1. 56 (m, 2H), 3. 08 (t, 2H), 3. 11 (q, 1H), 3. 33 (d, 1H), 3. 88 ( s, 3H), 4. 09 (q, 2H), 4. 36 (s, 2H) 4. 91 (q _{,}1H), 5. 57 (s, 2H), 5. 60 (s, 2H), 6. 94-7. 9 2 (m, 19H), 12. 04 (s, 1H).

### Example 26

### 4-[4- {2-[4-(2,4-dioxothiazolidin-5-ylmethyl)phenoxymethyl]-1-methyl-1H-benzimidazol-6-yloxyl}phenylsulfonylmethyl]-2-propyl-1-[2'-(1H-tetrazol-5-yl)biphenyl-4-ylmethyl]-1H-imidazole-5-carboxylic acid ethyl ester (example compound I-181)

A mixture of 0.53 g of 4-[4-{2-[4-(2,4-dioxothiazolidin-5-ylmethyl)phenoxymethyl]-1-methyl- 1H-benzimidazol-6-yloxy} phenylthiomethyl]-2-propyl-1-[2'-(1H-tetrazol-5-yl)biphenyl-4-ylmethyl]-1H-imidazole-5-carboxylic acid ethyl ester (the compound of Example 8), 5 mL of methylene chloride, and 1 mL of ethanol was combined with 0.153 g of m-chloroperbenzoic acid (purity: 65% or greater) and stirred at room temperature for one hour and forty minutes. The mixture was combined with 0.077 g more of m-chloroperbenzoic acid (purity: 65% or greater) and stirred at room temperature overnight. The solvent was distilled off, and the resulting residue was refined by silica gel column chromatography (elution solvent: ethyl acetate/methanol = 3/1) to give 0.22 g of the title compound as a white solid.

Mass spectrum: 952 (M+1)⁺.
¹H-NMR spectra (500MHz, DMSO-d₆), δₚₚₘ:0. 8 0 (t, 3H), 1. 1 8 (t, 3H), 1. 50 (m, 2H),2. 5 2 (t, 2H), 3. 07 (q, 1H), 3. 33 (d, 1H), 3. 80 ( s, 3H), 4. 08 (q, 2H), 4. 77 (s, 2H), 4. 89 (q , 1H), 5. 3 8 (s, 2H), 5. 48 (s, 2H), 6. 85-7. 6 9 (m, 19H).

### Example 27

### N-methyl-(4-[4- {2-[4-(2,4-dioxothiazolidyn-5-ylmethyl)phenoxymethyl]-1-methyl-1H-benzimidazol-6-yloxy}phenylthiomethyl]-2-propyl-1-[2'-(1H-tetrazol-5-yl)biphenyl-4-ylmethyl]-1H-imidazole)-5-carboxamide (example compound I-46)

A mixture of 297 mg of 4-[4-{2-[4-(2,4-dioxothiazolidyn-5-ylmethyl)phenoxymethyl]-1-methyl-1H-benzimidazol-6-yloxy} phenylthiomethyl]-2-propyl-1-[2' -(1H-tetrazol-5-yl)biphenyl-4-ylmethyl]-1H-imidazole-5-carboxylic acid (the compound of Example 21) and 35 mg of methylamine hydrochloride was combined with 1 mL of dimethylformamide, then 57 mg of triethylamine, then 67 mg of WSC (1-ethyl-3-(3-dimethylaminopropyl)carbodiimide hydrochloride) and stirred at room temperature for two days. The mixture was combined with an excess of ethyl acetate and water, the ethyl acetate layer was separated, and the ethyl acetate layer was washed with saturated physiological saline and dried over anhydrous sodium sulfate. The solvent was condensed, and a precipitated insoluble matter was collected by filtration and dried to give 185 mg of the title compound as a light yellow solid.

Mass spectrum: 905 (M+1)⁺.
¹H-NMR. spectra 500MHz, DMSO-d₆), δₚₚₘ: 0.8 2 (t, 3H), 1.52(m, 2H), 2.4 6 (t, 2 H), 2.6 6 (d, 3H), 3.07(q, 1H), 3.3 3 (q, 1H), 3. 7 7 ( s, 3H), 4. 1 7 (s, 2H), 4.89(q, 1H), 5.32(s , 2H), 5.36(s, 2H), 6.90-7.64(m, 19H), 7 .95 (d, 1H).

### Example 28

### 5-[4-[6-{4-(4-methyl-2-propyl-1-[2'-(1H-tetrazol-5-yl)biphenyl-4-ylmethyl]-1H-benzimidazol-6-ylcarbonylaminomethyl)phenoxy}-1-methyl-1H-benzimidazol-2-ylmethoxy]benzyl]thiazolidin-2,4-dione (example compound II-60)

A weight of 160 mg of 5-[4-{2-(N-t-butoxycarbonyl-N-methylamino)-4-[4-(4-methyl-2-propyl-1-[2'-(1H-tetrazol-5-yl)biphenyl-4-ylmethyl]-1H-benzimidazol-6-ylcarbonylaminomethyl)phenoxy]phenylaminocarbonylmethoxy}benzyl]thiazolidin-2,4-dione (the compound of Reference Example 91) and 10 mL of a 4 N hydrogen chloride-dioxane solution were heated to an oil-bath temperature of 70°C for five hours. The solvent was removed from the reaction mixture using the gradient method, and the residue was dissolved in methanol and poured into a large volume of ethyl acetate. A precipitated light yellow powder was collected by filtration to give 119 mg of a hydrochloride of the title compound.

Mass spectrum: 923 (M+1)⁺.
¹H-NMR. spectra 500MHz, DMSO-d₆), δₚₚₘ:0.9 2 (t, 3H), 1.69(q, 2H), 2. 6 7 (s, 3H), 3. 1 4 (q, 1H), 3.16(t, 2H), 3.33 (d, 1H), 3.89( s, 3H), 4.50(5, 2H), 4.90(q, 1H), 5.56(s , 2H), 5.80(s, 2H), 6.99-8. 29(m, 21H), 9 , 25 (s, 1H), 12.03(s, 1H).

### Example 29

### 4-[4-{2-[4-(2,4-dixothiazolidin-5-ylmethyl)phenoxymethyl]-1-methyl-1H-benzimidazol-6-ylthio}phenoxymethyl]-2-propyl-1-[2'-(1H-tetrazol-5-yl)biphenyl-4-ylmethyl]-1H-imidazole-5-carboxylic acid ethyl ester (example compound I-104)

A weight of 510 mg of 4-{4-[4-[4-(2,4-dixothiazolidin-5-ylmethyl)phenoxyacetylamino]-3-(N-t-butoxycarbonyl-N-methylamino)phenylthio]phenoxymethyl}-2-propyl-1-[2'-(1H-tetrazol-5-yl)biphenyl-4-ylmethyl]-1H-imidazole-5-carboxylic acid ethyl ester (the compound of Reference Example 95) and 20 mL of a 4 N hydrogen chloride-dioxane solution were heated to an oil-bath temperature of 75°C for seven hours. The solvent was removed from the reaction mixture using the gradient method, and the residue was dissolved in methanol and poured into a large volume of ethyl acetate. A precipitated light yellow powder was collected by filtration to give 317 mg of a hydrochloride of the title compound.

Mass spectrum: 920(M+1)⁺.
¹H-NMR. spectra 500MHz, DMSO-d₆), δₚₚₘ:0.8 6 (t, 3H), 1.07(t, 3H), 1.59(m, 2H), 2.76 (t, 2H), 3. 1 2 (q. 1H), 3. 3 2 (d, 1H), 3. 92( s, 3H), 4. 1 8 (q, 2H), 4. 90 (q, 1H, 5.20(s , 2H), 5.60(s, 2H), 5.64(s, 2H), 7.00-7. 8 5 (m, 1 9 H), 12. 04(s, 1H).

### Example 30

### 4-[4-{2-[4-(2,4-dioxothiazolidin-5-ylmethyl)phenoxymethyl]-1-methyl-1H-benzimidazol-6-yloxy}phenylthiomethyl]-2-propyl-1-[2'-(1H-tetrazol-5-yl)biphenyl-4-ylmethyl]-1H-imidazole-5-carboxylic acid methyl ester (example compound I-38)

A weight of 85 mg of 4-[4-{4-[4-(2,4-dixothiazolidin-5-ylmethyl)phenoxyacetylamino]-3-(N-t-butoxycarbonyl-N-methylamino)phenoxy]phenylthiomethyl}-2-propyl-1-[2'-(1H-tetrazol-5-yl)biphenyl-4-ylmethyl]-1H-imidazole-5-carboxylic acid methyl ester (the compound of Reference Example 101) and 10 mL of a 4 N hydrogen chloride-dioxane solution were heated to an oil-bath temperature of 70°C for seven hours. The solvent was removed from the reaction mixture using the gradient method, and the residue was washed with dioxane, then dissolved in a small volume of methanol and poured into a large volume of ethyl acetate. A precipitated light red powder was collected by filtration to give 60 mg of a hydrochloride of the title compound.

Mass spectrum: 906 (M+1)⁺.
¹H-NMR spectra 500MHz, DMSO-d₆), δₚₚₘ:0.8 3 (t, 3H), 1.54(m, 2H), 2.76(t, 2H), 3.12 (q, 1H), 3.33(d, 1H), 3.63(s, 3H), 3.87( s, 3H), 4.35 (s, 2H), 4.91(q, 1H), 6.56 (s , 2H), 5.58(s, 2H), 6.91-7. 7 2 (m, 19H, 1 2. 0 4 (s, 1H).

### Example 31

### 4-{N-[4-[2-(4-[2,4-dioxothiazolidin-5-ylmethyl]phenoxymethyl)-1-methyl-1H-benzimidazol-6-yloxy]phenyl]aminomethyl}-2-propyl-1-[2'-(1H-tetrazol-5-yl)biphenyl-4-ylmethyl]-1H-imidazole-5-carboxylic acid ethyl ester (example compound I-110)

A weight of 0.90 g of 4-{N-t-butoxycarbonyl-N-[4-[4-(4-[2,4-dioxothiazolidin-5-ylmethyl]phenoxyacetylamino)-3-(N-t-butoxycarbonyl-N-methylamino)phenoxy]phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 105) and 20 mL of a 4 N hydrogen chloride-dioxane solution were heated to an oil-bath temperature of 70-75°C for seven hours. The solvent was removed from the reaction mixture by the gradient method, and the residue was washed with dioxane, then dissolved in a small volume of methanol and combined with a large volume of ethyl acetate. A precipitated light red powder was collected by filtration to give 0.58 g of a hydrochloride of the title compound.

Mass spectrum: 920 (M+1)⁺.
¹H-NMR spectra (500MHz, DMSO-d₆), δₚₚₘ:0.8 3 (t, 3H), 1.19(t, 3H), 1.55(m, 2H), 2.83 (t, 2H), 3. 1 2 (q, 1H), 3. 33 (d, 1H), 3. 89 ( s, 3H), 4.26 (q, 2H), 4.60 (s, 2H), 4.91(q , 1H), 5.62(s, 2H), 5.6 7 (s, 2H), 6.89-7. 7 5 (m, 1 9H), 1 2. 04(s, 1H).

### Example 32

### 4-{N-[4-[2-(4-[2,4-dioxothiazolidin-5-ylmethyl]phenoxymethyl)-1-methyl-1H-benzimidazol-6-yloxy]-2,6-dimethylphenyl]aminomethyl}-2-propyl-1-[2'-(1H-tetrazol-5-yl)biphenyl-4-ylmethyl]-1H-imidazole-5-carboxylic acid ethyl ester (example compound I-111)

A weight of 51 g of 4-{N-[4-[4-(4-[2,4-dioxothiazolidin-5-ylmethyl]phenoxyacetylamino)-3-(N-t-butoxycarbonyl-N-methylamino)phenoxy]-2,6-dimethylphenyl]aminomethyl}-2-propyl-1-[2'-(1H-tetrazol-5-yl)biphenyl-4-ylmethyl]-1H-imidazole-5-carboxylic acid ethyl ester (the compound of Reference Example 108) and 2 mL of a 4 N hydrogen chloride-dioxane solution were heated to an oil-bath temperature of 70°C for six hours. The solvent was removed from the reaction mixture by the gradient method, and the residue was washed with dioxane, then dissolved in a small volume of methanol and combined with a large volume of ethyl acetate. A precipitated white powder was collected by filtration to give 35 mg of a hydrochloride of the title compound.

Mass spectrum: 931 (M+1)⁺.
¹H-NMR spectra 500MHz, DMSO-d₆), δₚₚₘ:0.8 7 (t, 3H), 1.15(t, 3H), 1. 5 7 (m, 2H), 2. 24 (s, 6H), 2. 72 (t, 2H), 3. 11(q, 1H), 3.33( d, 1H), 4. 1 5 (q, 2H), 3. 8 5 (s, 3H), 4.52 (s , 2H), 4.9 1 (q, 1H), 5.54(s, 2H), 5. 5 7 (s, 2H), 6.74-7.7 2 (m, 17H), 12.03(s, 1H).

### Example 33

### 4-{2-[4-(2,4-dioxothiazolidin-5-ylmethyl)phenoxymethyl]-1-methyl-1H-benzimidazol-6-ylthiomethyl}-2-propyl-1-[2'-(1H-tetrazol-5-yl)biphenyl-4-ylmethyl]-1H-imidazole-5-carboxylic acid ethyl ester (example compound I-182)

A weight of 0.276 g of 4-{4-[4-(2,4-dioxothiazolidin-5-ylmethyl)phenoxyacetylamino]-3-(N-t-butoxycarbonyl-N-methylamino)phenylthiomethyl}-2-propyl-1-[2'-(1H-tetrazol-5-yl)biphenyl-4-ylmethyl]-1H-imidazole-5-carboxylic acid ethyl ester (the compound of Reference Example 113) and 4 mL of a 4 N hydrogen chloride-dioxane solution were heated to an oil-bath temperature of 60°C to 75°C for 9.5 hours. The insoluble matter obtained by the gradient method was washed with dioxane, pulverized in dioxane and ethyl acetate, and collected by filtration. This powder was dissolved in a small volume of methanol, and the solution was poured into a large volume of ethyl acetate. A precipitated powder was collected by filtration and dried to give 0.20 g of a hydrochloride of the title compound as a light yellow solid.

Mass spectrum: 828 (M+1)⁺.

¹H-NMR spectra 500MHz, DMSO-d₆), δₚₚₘ:0.7 7 (t, 3H), 1.08 (t, 3H)1.51(m, 2H), 2.74 (t, 2H), 3.11(q, 1H, 3.33(d, 1H), 3.94( s, 3H), 4.04(q, 2H), 4. 5 0 (s, 2H), 4. 9 0 (q, , 1H), 5.5 7 (s, 2H), 5.58(s, 2H), 6.89-8. 01(m, 15H), 12.04 (s, 1H).

### Example 34

### 4-[3-{2-[4-(2,4-dioxothiazolidin-5-ylmethyl)phenoxymethyl]-1-methyl-1H-benzimidazol-6-yloxy}phenylthiomethyl]-2-propyl-1-[2'-(1H-tetrazol-5-yl)biphenyl-4-ylmethyl]-1H-imidazole-5-carboxylic acid ethyl ester (example compound I-101)

A weight of 193 mg of 4-[3-[4-[4-(2,4-dioxothiazolidin-5-ylmethyl)phenoxyacetylmethyl]-3-(N-t-butoxycarbonyl-N-methylamino)phenoxy]phenylthiomethyl}-2-propyl-1-[2'-(1-trimethylphenyl-1H-tetrazol-5-yl)biphenyl-4-ylmethyl]-1H-imidazole-5-carboxylic acid ethyl ester (the compound of Reference Example 117) and 3 mL of a 4 N hydrogen chloride-dioxane solution were heated to an oil-bath temperature of 70°C for 5.5 hours. The solvent was removed from the reaction mixture by the gradient method, and the residue was dissolved in methanol and combined with a large volume of ethyl acetate. A precipitated white solid was collected by filtration to give 120 mg of a hydrochloride of the title compound.

Mass spectrum: 920 (M+1)⁺.
¹H-NMR spectra 400MHz, DMSO-d₆) , δₚₚₘ**:**0.7 9 (t, 3H), 1. 1 3 (t, 3H), 1.51(m, 2H), 2.71 (t, 2H), 3.12(q, 1H), 3.34 (d, 1H), 3.92( s, 3H), 4.12(q, 2H), 4.43(s, 2H), 4.91(q , 1H), 5.57(s, 2H), 5.63(s, 2H), 6.87-7. 8 0 (m, 19H), 12.05(s, 1H).

### Example 35

### 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-chlorobenzyloxy)propionic acid (example compound I-304)

### (a) 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-chlorobenzyloxy)propionic acid methyl

A weight of 840 mg of 3-[4-[4-(5-ethoxycarbonyl-1-{2'-(1H-tetrazol-5-yl)biphenyl-4-ylmethyl}-2-propyl-1H-imidazol-4-ylmethylthio)-2-(N-t-butoxycarbonyl-N-methylamino)phenylaminocarbonylmethoxy]phenyl]-2-(4-chlorobenzyloxy)propionic acid methyl (the compound of Reference Example 122) was combined with 30 mL of a 4 N hydrogen chloride-dioxane solution and stirred at 75°C for seven hours. After the reaction had ended, the solvent was distilled off from the reaction mixture, and the residue was dissolved in methanol, combined with ethyl acetate and water, made weakly basic using 1 N sodium hydroxide, and extracted with ethyl acetate. The extract was washed with water and dried over anhydrous magnesium sulfate. The solvent was distilled off, and the residue was refined by silica gel column chromatography (elution solvent: methylene chloride/methanol = 10/1) to give 280 mg of the title compound as a solid.
Rf value (relative difference) (silica gel thin layer chromatography, methylene chloride/methanol = 10/1): 0.50.

¹H-NMR spectra (400MHz, chloroform ,-d), δₚₚₘ:0. 89(t, 3H), 1.09(t, 3H), 1.58(m, 2H), 2.6 1 (t, 2H), 3.00(m, 2H), 3. 73(s, 3H), 3.85 (s, 3H), 8.94(q, 2H), 4.09(q, 1H), 4. 32 ( d, 1H), 4.63 (d, 1H), 4.98 (s, 2H), 5.25 (s , 2H), 5.31(s, 2H), 6.69-7.88(m, 20H).

### (b) 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-chlorobenzyloxy)propionic acid

A weight of 280 mg of 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylemthoxy]phenyl]-2-(4-chlorobenzyloxy)propionic acid methyl (the compound of (a)) was combined with 20 mL of ethanol and 5 mL of tetrahydrofuran to dissolve, then combined with 1 mL of 2 N sodium hydroxide and stirred at room temperature for one hour. After the reaction had ended, the reaction mixture was combined with ethyl acetate and water, neutralized using 1N hydrochloric acid, and extracted with ethyl acetate. The extract was washed with water and dried over anhydrous magnesium sulfate. The solvent was distilled off, and the residue was dissolved in a small volume of methanol and combined with 3 mL of a 2 N hydrochloric acid/ether solution. The solvent was distilled off, and the residue was combined with ether and ethyl acetate to give 150 mg of the title compound as a solid.

¹H-NMR spectra 400MHz, DMSO-d₆), δₚₚₘ:0. 8 1 (t, 3H), 1.09(t, 3H), 1.55(m, 2H), 2. 5 5 (t, 2H), 2. 86(m, 2H), 2. 9 8 (s, 3H), 3. 3 7 ( m, 1H), 3.81(s, 3H), 4.02(q, 2H), 4.38(s , 2H), 4.44 (dd, 2H), 5.36 (s, 2H), 5.49(s . 2H), 6. 84-7. 70(m, 21H).

### Example 36

### N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-phenyloxycarbonylaminoacetic acid (example compound I-305)

### (a) N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy} phenylmethyl]-N-phenyloxycarbonylaminoacetic acid ethyl

A weight of 480 mg of N-[4-{4-[5-ethoxycarbonyl-1-{2'-(1-triphenylmethyl-1H-tetrazol-5-yl)biphenyl-4-ylmethyl}-2-propyl-1H-imidazol-4-ylmethylthio]-2-(N-t-butoxycarbonyl-N-methylamino)phenylaminocarbonylmethoxy}phenylmethyl]-N-phenyloxycarbonylaminoacetic acid ethyl ester (the compound of Reference Example 127) was combined with 30 mL of a 4 N hydrogen chloride-dioxane solution and stirred at 60°C for four hours. After the reaction had ended, the solvent was distilled off from the reaction mixture and the residue was dissolved in a small amount of methanol, combined with ethyl acetate and water, neutralized using 1 N hydrochloric acid, and extracted with ethyl acetate. The extract was washed with water and dried over anhydrous magnesium sulfate. The solvent was distilled off, and the residue was refined by silica gel column chromatography (elution solvent: methylene chloride/methanol = 10/1) to give 260 mg of the title compound as a solid.
Rf value (relative difference) (silica gel thin layer chromatography, methylene chloride/methanol = 10/1): 0.50.

Mass spectrum: 934 (M+1)⁺.
¹H-NMR. spectra (400MHz, chloroform, d), δₚₚₘ:0. 86 (t, 3H), 1.10 (t, 3H), 1. 26 (t, 3H), 1.5 6 (m, 2H), 2. 5 6 (q, 2H), 3.84(s, 3H), 3.95 (q, 2H), 4.00(d, 2H), 4. 1 8 (q, 2H), 4. 3 2 ( s, 2H), 4. 63 (d, 2H), 5.07(d, 2H), 5.29(d , 2H), 6.67-7.87(m, 20H).

### (b) N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-phenyloxycarbonylaminoacetic acid

A weight of 260 mg of N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-phenyloxycarbonylaminoacetic acid ethyl (the compound of (a)) was dissolved in 20 mL of ethanol. The solution was combined with 1 mL of 1 N sodium hydroxide and stirred at room temperature for one hour. After the reaction had ended, the reaction mixture was combined with ethyl acetate and water, then neutralized using 1 N hydrochloric acid and extracted with ethyl acetate. The ethyl acetate extract was washed with water and dried over anhydrous magnesium sulfate. The solvent was distilled off, and the residue was dissolved in a small volume of methanol and combined with 3 mL of 2 N hydrogen chloride-ether. The solvent was distilled off, and the residue was combined with ether to give 160 mg of the title compound as a solid.

Mass spectrum: 906 (M+1)⁺.
¹H-NMR; spectra (400MHz, DMSO-d₆), δₚₚₘ:0.7 8 (t, 3H), 1.08 (t, 3H), 1.52(m, 2H), 2.7 2 (t, 2H), 3.93(s, 3H), 4. 06 (m, 4H), 4. 47( s, 2H), 4. 56(d, 2H), 5.56(s, 2H), 5.58(s , 2H), 6.88-7. 9 9 (m, 20H).

### Example 37

### N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-(3-trifluoromethylbenzoyl)aminoacetic acid (example compound I-333)

### (a) N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-(3-trifluoromethylbenzoyl)aminoacetic acid ethyl

A weight of 390 mg N-[4-{4-[5-ethoxycarbonyl-1-{2'-(1-triphenylmethyl-1H-tetrazol-5-yl)biphenyl-4-ylmethyl}-2-propyl-1H-imidazol-4-ylmethylthio] -2-(N-t-butoxycarbonyl-N-methylamino)phenylaminocarbonylmethoxy}phenylmethyl]-N-(3-trifluoromethylbenzoyl)aminoacetic acid ethyl (the compound of Reference Example 130) was combined with 30 mL of a 4 N hydrogen chloride-dioxane solution and heated at 70°C for seven hours. After the reaction had ended, the solvent was distilled off from the reaction mixture, and the residue was dissolved in a small volume of methanol, combined with ethyl acetate and water, made weakly basic using 1 N sodium hydroxide, and extracted with ethyl acetate. The ethyl acetate extract was washed with water and dried over anhydrous magnesium sulfate. The solvent was distilled off, and the residue was refined by silica gel column chromatography (elution solvent: methylene chloride/methanol = 10/1) to give 120 mg of the title compound as a solid.
Rf value (relative difference) (silica gel thin layer chromatography, methylene chloride/methanol = 10/1): 0.50.
Mass spectrum: 986 (M+1)+.

### (b) N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-(3-trifluoromethylbenzoyl)aminoacetic acid (example compound I-333)

A weight of 120 mg of N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-(3-trifluoromethylbenzoyl)aminoacetic acid ethyl (the compound of (a)) was dissolved in 20 mL of ethanol and 5 mL of tetrahydrofuran. The solution was combined with 0.5 mL of 2 N sodium hydroxide and stirred at room temperature for one hour. After the reaction had ended, the reaction mixture was combined with ethyl acetate and water, then made pH 6 using 1 N hydrochloric acid and extracted with ethyl acetate. The ethyl acetate extract was washed with water and dried over anhydrous magnesium sulfate. The solvent was distilled off, and the residue was combined with ether to give 72 mg of the title compound as a solid.
Mass spectrum: 958 (M+1)⁺.
¹H-NMR spectra (400MHz, DMSO-d₆), δₚₚₘ:0.8 1 (t, 3H), 1.09 (t, 3H), 1.55 (m, 2H), 2. 54 (t, 2H), 3.80(d, 3H), 4.01(q, 4H), 4.37( s, 2H), 4.41(s, 1H), 4.61 (s, 1H), 5.37(d , 2H), 5.48(s, 2H), 6, 83-7.84 (m, 19H).

### Example 38

### 4-[4- {2-[4-(2,4-dioxothiazolidin-5-ylmethyl)phenoxymethyl]-1-methyl-1H-benzimidazol-6-yloxy}benzylthiomethyl]-2-propyl-1-[2'-(H-tetrazol-5-yl)biphenyl-4-ylmethyl]-1H-imidazole-5-carboxylic acid (example compound I-309)

A mixture of 1.80 g of 4-{4-[4-[4-(2,4-dioxothiazolidin-5-ylmethyl)phenoxyacetylamino]-3-(N-t-butoxycarbonyl-N-methylamino)phenoxy]benzylthiomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 137) and 50 mL of a 4 N hydrogen chloride-dioxane solution was heated and stirred at an oil-bath temperature of 60°C for fifteen hours. The mixture was then heated and stirred at 80°C for three hours. The solvent was removed from the reaction mixture using the gradient method, and the residue was dissolved in methanol and poured into a large volume of ethyl acetate. A precipitated white powder was collected by filtration to give 1.09 g of a hydrochloride of the title compound.
Mass spectrum: 934 (M+1)⁺.
¹H-NMR spectra .00MHz, DMSO-d₆), δₚₚₘ:0. 8 7 (t, 3H), 1.15(t, 3H), 1.60(m, 2H), 2.80 (t, 2H), 3.1 (q. 1H), 3. 33 (d, 1H), 3.85( s, 2H), 3.9 2 (s, 3H), 3.98(s, 2H), 4. 1 7 (q , 2H), 4.91(q, 1H), 5.62(s, 2H), 5.63 (s, 2H), 6.96-7.81(m, 19H), 12. 05(s, 1H).

### Example 39

### 4-[2-{2-[4-(2,4-dioxothiazolidin-5-ylmethyl)phenoxymethyl]-1-methyl-1H-benzimidazol-6-yloxy}pyridin-5-ylmethylthiomethyl]-2-propyl-1-[2'-(1H-tetrazol-5-yl)biphenyl-4-ylmethyl]-1H-imidazole-5-carboxylic acid ethyl ester (example compound I-311)

Using 4-{2-[4-[4-(2,4-dioxothiazolidin-5-ylmethyl)phenoxyacetylamino]-3-(N-t-butoxycarbonyl-N-methylamino)phenoxy]pyridin-5-ylmethylthiomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 144) and a 4 N hydrogen chloride-dioxane solution, these were reacted and refined in the same manner as Example 38 to give the title compound.

### Example 40

### 4-[1-{2-[4-(2,4-dioxothiazolidin-5-ylmethyl)phenoxymethyl]-1-methyl-1H-benzimidazol-6-ylthio}ethyl]-2-propyl-1-[2'-(1H-tetrazol-5-yl)biphenyl-4-ylmethyl]-1H-imidazole-5-carboxylic acid ethyl ester (example compound I-323)

Using 4-[1-{4-[4-(2,4-dioxothiazolidin-5-ylmethyl)phenoxyacetylamino]-3-(N-t-butoxycarbonyl-N-methylamino)phenylthio}ethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 150) and a 4 N hydrogen chloride-dioxane solution, these were reacted and refined in the same manner as Example 33 to give the title compound.

### Example 41

### 4-[1-[2-{2-[4-(2,4-dioxothiazolidin-5-ylmethyl)phenoxymethyl]-1-methyl-1H-benzimidazol-6-yloxy} pyridin-5-ylmethylthio]ethan-1-yl]-2-propyl-1-[2'-(1H-tetrazol-5-yl)biphenyl-4-ylmethyl]-1H-imidazole-5-carboxylic acid ethyl ester (example compound I-322)

Using 4-{1-[2-[4-[4-(2,4-dioxothiazolidin-5-ylmethyl)phenoxyacetylamino]-3-(N-t-butoxycarbonyl-N-methylamino)phenoxy]pyridin-5-ylmethylthio]ethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 153) and 4 N hydrogen chloride-dioxane solution, these were reacted and refined in the same manner as Example 33 to give the title compound.

### Example 42

### 2-butyl-4- {2-[4-(2,4-dioxothiazolidin-5-ylmethyl)phenoxymethyl]-1-methyl-1H-benzimidazol-6-ylthiomethyl}-1-[2'-(1H-tetrazol-5-yl)biphenyl-4-ylmethyl]-1H-imidazole-5-carboxylic acid ethyl ester (example compound I-320)

Using 0.20 g of 2-butyl-4-{4-[4-(2,4-dioxothiazolidin-5-ylmethyl)phenoxyacetylamino]-3-(N-t-butoxycarbonyl-N-methylamino)phenylthiomethyl}-1-[2'-(1H-tetrazol-5-yl)biphenyl-4-ylmethyl]-1H-imidazole-5-carboxylic acid ethyl ester (the compound of Reference Example 159) and 5 mL of a 4 N hydrogen chloride-dioxane solution, these were reacted and refined in the same manner as Example 33 to give 0.14 g of a hydrochloride of the title compound as a white powder.
Mass spectrum: 842 (M+1)⁺.
¹H-NMR spectra 400MHz, DMSO-d₆), δ ₚₚₘ : 0.7 7 (t, 3H), 1. 08 (t, 3H), 1. 1 9 (m, 2H), 1. 4 7 (m, 2H), 2. 7 4 (t, 2H), 3.11 (q, 1H), 3. 3 3 ( d, 1H), 3.93 (s, 3H), 4. 04 (q, 2H), 4.49 (s, 2H), 4. 90 (q, 1H), 5. 56 (s, 2H+2H), 6.88 -7.8 (m, 15H), 1 2.05 (s, 1H) .

### Example 43

### 4-[2-[2-[1-methyl-2-{4-(2,4-dioxothiazolidin-5-ylmethyl)phenoxymethyl}-1H-benzimidazol-6-yloxy]ethylthio]propan-2-yl]-2-propyl-1-[2'-(1H-tetrazol-5-yl)biphenyl-4-ylmethyl]-1H-imidazole-5-carboxylic acid ethyl ester (example compound I-342)

Using 4-[2-[2-[3-(N-t-butoxycarbonyl-N-methylamino)-4-{4-(2,4-dioxothiazolidin-5-ylmethyl)phenoxyacetylamino}phenoxy]ethylthio]propan-2-yl]-2-propyl-1-[2'-(1H-tetrazol-5-yl)biphenyl-4-ylmethyl]-1H-imidazole-5-carboxylic acid ethyl ester (the compound of Reference Example 164) and a 4 N hydrogen chloride-dioxane solution, these were reacted and refined in the same manner as Example 33 to give the title compound.

### Example 44

### 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(3-chlorobenzyloxy)propionic acid (example compound I-335)

### (a) 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(3-chlorobenzyloxy)propionic acid methyl

Using 490 mg of 3-[4-[4-(5-ethoxycarbonyl-1-{2'-(1-triphenylmethyl-1H-tetrazol-5-yl)biphenyl-4-ylmethyl}-2-propyl-1H-imidazol-4-ylmethylthio)-2-(N-t-butoxycarbonyl-N-methylamino)phenylaminocarbonylmethoxy]phenyl]-2-(3-chlorobenzyloxy)propionic acid methyl (the compound of Reference Example 167) and 30 mL of a 4 N hydrogen chloride-dioxane solution, these were reacted and refined in the same manner as Example 35(a) to give 200 mg of the title compound.
Rf value (relative difference) (silica gel thin layer chromatography, methylene chloride/methanol = 10/1): 0.50.
Mass spectrum: 911 (M+1)⁺.
¹H-NMR spectra 400MHz, DMSO-d₆), δₚₚₘ : 0.8 1 (t, 3H), 1.09 (t, 3H), 1. 55 (m, 2H), 2. 5 4 (t, 2H), 2.9 3 (m, 2H), 3.64 (s, 3H), 3. 8 0 ( s, 3H), 4.02 (q, 2H), 4.21(q, 1H), 4.37(s 2H), 4.39 (d, 1H), 4.58 (d, 1H), 5.34 (s, 2H), 5. 49 (s, 2H), 6.83-7. 68 (m, 19H).

### (b) 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(3-chlorobenzyloxy)propionic acid (example compound I-335)

Using 200 mg of 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(3-chlorobenzyloxy)propionic acid methyl (the compound of (a)), 20 mL of ethanol, 5 mL of tetrahydrofuran, and 2 mL of 2 N sodium hydroxide, these were reacted and refined in the same manner as Example 35(b) to give 100 mg of the title compound.
Mass spectrum: 911 (M+1)⁺.
¹H-NMR spectra (400MHz, DMSO-d₆) , δₚₚₘ : 0.7 8 (t, 3H), 1.08 (t, 3H), 1.52 (m, 2H), 2.73 (t, 2H), 2.96 (m, 2H), 3. 94 (s, 3H), 4.04 ( q, 2H), 4.12 (q, 1H), 4.37 (d, 1H), 4.50 (s , 2H), 4.62 (d, 1H), 5.56 s, 4H), 6.88-8. 01 (m, 19H).

### Example 45

### 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-methoxybenzyloxy)propionic acid (example compound I-334)

### (a) 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-methoxybenzyloxy)propionic acid methyl

Using 3-[4-[4-(5-ethoxycarbonyl-1-{2'-(1-triphenylmethyl-1H-tetrazol-5-yl)biphenyl-4-ylmethyl}-2-propyl-1H-imidazol-4-ylmethylthio)-2-(N-t-butoxycarbonyl-N-methylamino)phenylaminocarbonylmethoxy]phenyl-2-(4-methoxybenzyloxy)propionic acid methyl (the compound of Reference Example 170) and a 4 N hydrogen chloride-dioxane solution, these were reacted and refined in the same manner as Example 35(a) to give the title compound.

### (b) 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-methoxybenzyloxy)propionic acid (example compound I-334)

Using 3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-methoxybenzyloxy)propionic acid methyl (the compound of (a)), ethanol, tetrahydrofuran, and 2 N sodium hydroxide, these were reacted and refined in the same manner as Example 35(b) to give the title compound.

### Example 46

### 4-[1-[4-{2-[4-(2,4-dioxothiazolidin-5-ylmethyl)phenoxymethyl]-1-methyl-1H-benzimidazol-6-yloxy}phenylthio]ethan-1-yl]-2-propyl-1-(2'-(1H-tetrazol-5-yl)biphenyl-4-ylmethyl]-1H-imidazole-5-carboxylic acid (example compound I-344)

Using 4- [1-[4-{4-[4-(2,4-dioxothiazolidin-5-ylmethyl)phenoxyacetylamino]-3-(N-t-butoxycarbonyl-N-methylamino)phenoxy}phenylthio]ethyl]-1-[2'-(1-triphenylmethyl-1H-tetrazol-5-yl)biphenyl-4-ylmethyl]-2-propyl-1H-imidazole-5-carboxylic acid ethyl ester (the compound of Reference Example 174) and a 4 N hydrogen chloride-dioxane solution, these were reacted and refined in the same manner as Example 33 to give the title compound.

### Example 47

### 4- {2-[4-(2,4-dixothiazolidin-5-ylmethyl)phenoxymethyl]-3-methyl-3H-imidazo[4,5-b]pyridin-5-ylthiomethyl}-2-propyl-1-[2'-(1H-tetrazol-5-yl)biphenyl-4-ylmethyl]-1H-imidazole-5-carboxylic acid ethyl ester (example compound I-343)

Using 1.67 g of 4-[3-[4-(2,4-dixothiazolidin-5-ylmethyl)phenoxyacetylamino]-2-(N-methylamino)pyridin-6-ylthiomethyl]-2-propyl-1-[2'-(1H-tetrazol-5-yl)biphenyl-4-ylmethyl]-1H-imidazole-5-carboxylic acid ethyl ester (the compound of Reference Example 177) and 30 mL of a 4 N hydrogen chloride-dioxane solution, these were reacted and refined in the same manner as Example 33 to give 0.87 g of the title compound as a white powder.
Mass spectrum: 829 (M+1)⁺.
¹H-NMR spectra 400MHz, DMSO-d₆), δₚₚₘ : 0. 8 0 (t, 3H), 1.1 1 (t, 3H), 1.5 2 (m, 2H), 2.7 9 (t, 2H), 3.09 (q, 1H), 3.32 (d, 1H), 3.84 ( s, 3H), 4.1 8 (q, 2H), 4.80(s, 2H), 4.89 (q , 1H), 5.45 (s, 2H), 5.63 (s, 2H), 6.96-8. 02 (m, 14H), 1 2.05(s, 1H).

### Example 48

### N-[4- {6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-4-methoxyphenoxycarbonylaminoacetic acid (example compound I-332)

### (a) N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-4-methoxyphenoxycarbonylaminoacetic acid ethyl

Using 250 mg of N-[4-{4-[5-ethoxycarbonyl-1-{2'-(1-triphenylmethyl-1H-tetrazol-5-yl)biphenyl-4-ylmethyl}-2-propyl-1H-imidazol-4-ylmethylthio]-2-(N-t-butoxycarbonyl-N-methylamino)phenylaminocarbonylmethoxy}phenylmethyl]-N-4-methoxyphenoxycarbonylaminoacetic acid ethyl (the compound of Reference Example 180) and 30 mL of a 4 N hydrogen chloride-dioxane solution, these were reacted, then refined in the same manner as Example 36(a) to give 160 mg of the title compound.
Rf value (relative difference) (silica gel thin layer chromatography, methylene chloride/methanol = 10/1): 0.50.
Mass spectrum: 964 (M+1)⁺.
¹H-NMR spectra (400MHz, DMSO-d₆), δₚₚₘ : 0.8 1 (t, 3H), 1.09 (t, 3H), 1.18 (t, 3H), 1.55 (m, 2H), 2.54 (t, 2H), 3. 74 (s, 3H), 3.80 ( s, 3H), 3.99-4.12 (m, 6H), 4.37(s, 2H), 4 .44 (s, 1H), 4. 5 8 (s, 1H), 5.38 (s, 2H), 5. 49 (s, 2H), 6.84-7.69 (m, 19H).

### (b) N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-4-methoxyphenoxycarbonylaminoacetic acid (example compound I-332)

Using 160 mg of N-[4-{6-[5-ethoxycarbonyl-1-{2'-(1H-tetrazol-5-yl)biphenyl-4-ylmethyl}-2-propyl-1H-imidazol-4-ylmethylthio]-1-methyl-1H-benzimidazol-2-ylmethoxy}phenylmethyl]-N-4-methoxyphenoxycarbonylaminoacetic acid ethyl (the compound of (a)), 20 mL of ethanol, 5 mL of tetrahydrofuran, and 2 mL of a 1 N hydrogen chloride-dioxane solution, these were reacted and refined in the same manner as Example 36(b) to give 80 mg of the title compound.
¹H-NMR: spectra 100MHz, DMSO-d₆), *δ*ₚₚₘ : 0.7 8 (t, 3H), 1.08 (t, 3H), 1.52 (m, 2H), 2.72 (t, 2H), 3.74 (s, 3H), 3.93 (s, 3H), 4.02-4.06 (m, 4H), 4. 4 6 (s, 1H), 4. 4 9 (s, 2H), 4 .59 (s, 1H), 5.56 (s, 2H), 5.57 (s, 2H), 6. 88-7. 9 1 (m, 1 9H).

### Example 49

### 5-[4-[6-{2-(4,7-dioxo-2-propyl-1-[2'-(1H-tetrazol-5-yl)biphenyl-4-ylmethyl]-5,6-dihydro-1H-imidazo[4,5-d]pyridazin-5-yl)ethoxy}-1-methyl-1H-benzimidazol-2-ylmethoxy]benzyl]thiazolidine-2,4-dione (example compound III-35: 5-substituent) and 5-[4-[6-{2-(4,7-dioxo-2-propyl-1-[2'-(1H-tetrazol-5-yl)biphenyl-4-ylmethyl]-5,6-dihydro-1H-imidazo[4,5-d]pyridazin-6-yl)ethoxy}-1-methyl-1H-benzimidazol-2-ylmethoxy]benzyl]thiazolidine-2,4-dione (example compound III-35: 6-substituent)

Using 5-[4-[4-{2-(1-{2'-(1-triphenylmethyl-1H-tetrazol-5-yl)biphenyl-4-ylmethyl}-4,7-dioxo-2-propyl-5,6-dihydro-1H-imidazo[4,5-d]pyridazin-5-yl)ethoxy} -3-(N-t-butoxycarbonyl-N-methylamino)phenylaminocarbonylmethoxy]benzyl]thiazolidine-2,4-dione (the 5-substituent of Reference Example 185) and a 4 N hydrogen chloride-dioxane solution, these were reacted and refined in the same manner as Example 33 to give the title compound.
The 6-substituent of the title compound was obtained in the same manner using the 6-substiuent of Reference Example 185.

### Reference Example 1

### 6-hydroxymethyl-4-methyl-2-propyl-1-[2'-(1-triphenylmethyl-1H-tetrazol-5-yl)biphenyl-4-ylmethyl]-1H-benzimidazole

A solution of 10.62 g of 4-methyl-2-propyl-1-[2'-(1-triphenylmethyl-1H-tetrazol-5-yl)biphenyl-4-ylmethyl]-1H-benzimidazole-6-carboxylic acid methyl ester in 100 mL of tetrahydrofuran was dropped into a suspension of 1.6 g of lithium aluminum hydride in 150 mL of tetrahydrofuran, and stirred at room temperature overnight. The reaction mixture was combined with 1.5 mL of water and 4.5 mL of a 1 N sodium hydroxide aqueous solution while cooling with ice, then combined with 1.5 mL of water and filtered using Celite. The filtrate was condensed to give 10.58 g of a coarse product material, which was recrystallized from ethyl acetate-n-hexane. Next, the product material was refined by silica gel column chromatography (elution solvent: n-hexane/ethyl acetate = 1/5) to give 3.28 g of the title compound as a white crystal.
Rf value (relative difference) (silica gel thin layer chromatography, n-hexane/ethyl acetate = 1/1): 0.33.

### Reference Example 2

### 6-(3-(N-t-butoxycarbonyl-N-methylamino)-4-nitrophenoxymethyl)-4-methyl-2-propyl-1-[2'-(1-triphenylmethyl-1H-tetrazol-5-yl)biphenyl-4-ylmethyl]-1H-benzimidazole

A suspension of 0.176 g of sodium hydride (60%) in 5 mL dry tetrahydrofuran was combined with 30 mL of dry dimethylformamide while cooling with ice, then combined with 3.0 g of 6-hydroxymethyl-4-methyl-2-propyl-1-[2'-(1-triphenylmethyl-1H-tetrazol-5-yl)biphenyl-4-ylmethyl]-1H-benzimidazole (the compound of Reference Example 1). After stirring while cooling with ice for thirty minutes, then stirring at room temperature for 1.5 hours, the mixture was combined with 1.26 g of 4-chloro-2-(N-t-butoxycarbonyl-N-methylamino)nitrobenzene and stirred at room temperature overnight. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 5.15 g of the resulting residue was refined by silica gel column chromatography (elution solvent: n-hexane/ethyl acetate = 2/1) to give 2.1 g of the intended compound as a light yellow solid.
Rf value (relative difference) (silica gel thin layer chromatography, n-hexane/ethyl acetate = 1/1): 0.73.

### Reference Example 3

### 6-(4-amino-3-methylaminophenoxymethyl)-4-methyl-2-propyl-1-[2'-(1H-tetrazol-5-yl)biphenyl-4-ylmethyl]-1H-benzimidazole

A mixture of 2.1 g of 6-(3-(N-t-butoxycarbonyl-N-methylamino)-4-nitrophenoxymethyl)-4-methyl-2-propyl-1-[2'-(1-triphenylmethyl-1H-tetrazol-5-yl)biphenyl-4-ylmethyl]-1H-benzimidazole (the compound of Reference Example 2), 3 g of iron powder, 3 mL of tetrahydrofuran, 4 mL of ethanol, and 6 mL of methanol was combined with 0.3 mL of concentrated hydrochloric acid and stirred at room temperature for three hours. The mixture was combined with 17 mL more of concentrated hydrochloric acid and stirred at room temperature for one hour. The residue resulting from filtering off insoluble matter and condensing the filtrate was washed three times with diisopropyl ether to give 3.89 g of a coarse product material (containing inorganic matter) of a hydrochloride of the intended compound.

### Reference Example 4

### 5-(4- {4-[4-methyl-2-propyl-1-[2'-(1H-tetrazol-5-yl)biphenyl-4-ylmethyl]-1H-benzimidazol-6-ylmethoxy]-2-methylaminophenylaminocarbonylmethoxy}benzyl)thiazolidine-2,4-dione

A weight of 2.8 g of 4-(2,4-dioxothiazolidin-5-ylmethyl)phenoxyacetic acid and 1.6 g of 1,1'-carbonylbis-1H-imidazole was combined with 30 mL of dry tetrahydrofuran, made liquid by sonication, and stirred at room temperature for 1.5 hours. This solution was combined with 3.88 g of a coarse product material of 6-(4-amino-3-methylaminophenoxymethyl)-4-methyl-2-propyl-1-[2'-(1H-tetrazol-5-yl)biphenyl-4-ylmethyl]-1H-benzimidazole hydrochloride (the compound of Reference Example 3) and stirred at room temperature for thirty minutes, then combined with 1.5 g of triethylamine and stirred at room temperature overnight. The reaction mixture was poured into water and a precipitated solid was collected by filtration and dried to give 3.5 g of a coarse product material of the intended compound as a light brown powder.

### Reference Example 5

### 6-hydroxymethyl-4-methyl-2-propyl-1H-benzimidazole

A suspension of 14 g of lithium aluminum hydride in 600 mL of tetrahydrofuran was dropped into a solution of 29.9 g of 4-methyl-2-proyl-1H-benzimidazole-6-carboxylic acid methyl ester in 150 mL of tetrahydrofuran while cooling with ice. After stirring the reaction mixture at room temperature for twenty minutes, 42 mL of 1 N sodium hydroxide aqueous solution and a solution of 14 mL of water and 100 mL of tetrahydrofuran were dropped into the reaction mixture while cooling with ice, then 14 mL of water were dropped in and insoluble matter was filtered out. The filtrate was condensed to give 24.9 g of a coarse product material of the intended compound.

### Reference Example 6

### 4'-(6-hydroxymethyl-4-methyl-2-propyl-1H-benzimidazol-1-ylmethyl)biphenyl-2-carboxylic acid t-butyl ester

A solution of 10.22 g of 6-hydroxymethyl-4-methyl-2-propyl-1H-benzimidazole (the compound of Reference Example 5) and 17.5 g of 4'-(bromomethyl)biphenyl-2-carboxylic acid t-butyl ester in 100 mL of dry dimethylformamide was combined with 10.3 g of anhydrous potassium carbonate and stirred at room temperature overnight. The reaction solution was poured into water, a precipitated insoluble matter was collected using the gradient method, and this insoluble matter was dissolved in ethyl acetate, then dried over anhydrous sodium sulfate. The residue obtained by distilling off the solvent was refined by silica gel column chromatography (elution solvent: n-hexane/ethyl acetate = 1/3) to give 10.61 g of the intended compound as a white solid.
Rf value (relative difference) (silica gel thin layer chromatography, n-hexane/ethyl acetate = 1/3): 0.56.

### Reference Example 7

### 4'-[6-(3-(N-t-butoxycarbonyl-N-methylamino)-4-nitrophenoxymethyl)-4-methyl-2-propyl-1H-benzimidazol-1-ylmethyl]biphenyl-2-carboxylic acid t-butyl ester

A solution of 3.06 g of 4'-(6-hydroxymethyl-4-methyl-2-propyl-1H-benzimidazol-1-ylmethyl)biphenyl-2-carboxylic acid t-butyl ester (the compound of Reference Example 6) in 30 mL of dry dimethylformamide was combined with 0.26 g of 60% sodium hydride while cooling with ice, and stirred for thirty minutes. The mixture was then combined with 1.9 g of 4-chloro-2-(N-t-butoxycarbonyl-N-methylamino)nitrobenzene and stirred at room temperature for three days. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 5.03 g of the resulting residue was refined by silica gel column chromatography (elution solvent: n-hexane/ethyl acetate = 1/1) to give 3.02 g of the intended compound as a light yellow powder.
Rf value (relative difference) (silica gel thin layer chromatography, n-hexane/ethyl acetate = 1/1): 0.45.

### Reference Example 8

### 4'-[6-(4-amino-3-(N-t-butoxycarbonyl-N-methylamino)phenoxymethyl)-4-methyl-2-propyl-1H-benzimidazol-1-ylmethyl]biphenyl-2-carboxylic acid t-butyl ester

A mixture of 1.0 g of 4'-[6-(3-(N-t-butoxycarbonyl-N-methylamino)-4-nitrophenoxymethyl)-4-methyl-2-propyl-1H-benzimidazol-1-ylmethyl]biphenyl-2-carboxylic acid t-butyl ester (the compound of Reference Example 7) and 1 g of 10% palladium on carbon was combined with 17 mL of methanol, then purged with hydrogen gas at room temperature for about 2.5 hours. After insoluble matter from the reaction mixture had been filtered out, the filtrate was condensed and 0.87 g of the resulting residue was refined by silica gel column chromatography (elution solvent: n-hexane/ethyl acetate = 2/3) to give 0.75 g of the intended compound as a light yellow solid.
Rf value (relative difference) (silica gel thin layer chromatography, n-hexane/ethyl acetate = 1/1): 0.37.

### Reference Example 9

### 4'-{6-(3-(N-t-butoxycarbonyl-N-methylamino)-4-[4-(2,4-dioxothiazolidin-5-ylmethyl)phenoxymethylcarbonylamino]phenoxymethyl)-4-methyl-2-propyl-1H-benzimidazol-1-ylmethyl}biphenyl-2-carboxylic acid t-butyl ester

A solution of 0.75 g of 4'-[6-(4-amino-3-(N-t-butoxycarbonyl-N-methylamino)phenoxymethyl)-4-methyl-2-propyl-1H-benzimidazol-1-ylmethyl]biphenyl-2-carboxylic acid t-butyl ester (the compound of Reference Example 8) and 0.305 g of 4-(2,4-dioxothiazolidin-5-ylmethyl)phenoxyacetic acid diethyl in 5 mL of dry tetrahydrofuran was combined with 0.177 g of diethyl cyanophosphonate, then combined with 0.13 g of triethylamine and stirred at room temperature for two days. The reaction mixture was refined by silica gel column chromatography (elution solvent: n-hexane/ethyl acetate = 1/1 to 1/2) to give 0.81 g of the intended compound as a light yellow solid.
Rf value (relative difference) (silica gel thin layer chromatography, n-hexane/ethyl acetate = 1/1): 0.20.

### Reference Example 10

### 2-(N-t-butoxycarbonyl-N-methylamino)-4-(N-2-hydroxyethyl-N-methylamino)nitrobenzene

A mixture of 50 g of 2-(methylamino)ethanol and 35 g of 4-chloro-2-(N-t-butoxycarbonyl-N-methylamino)nitrobenzene was heated and stirred at an oil-bath temperature of 50°C for two hours, then heated and stirred at 80°C for 0.5 hour, 100°C for 2.5 hours, and 110°C for one hour. The residue resulting from distilling off excess 2-(methylamino)ethanol from the reaction mixture under reduced pressure was refined by silica gel column chromatography (elution solvent: n-hexane/ethyl acetate = 1/2) to give 35.37 g of the intended compound as a orange-yellow crystal.
Rf value (relative difference) (silica gel thin layer chromatography, n-hexane/ethyl acetate = 1/3): 0.56.

### Reference Example 11

### 4-(N-2-acetoxyethyl-N-methylamino)-2-(N-t-butoxycarbonyl-N-methylamino)nitrobenzene

A solution of 35.3 g of 2-(N-t-butoxycarbonyl-N-methylamino)-4-(N-2-hydroxyethyl-N-methylamino)nitrobenzene (the compound of Reference Example 10) in 200 mL of dry tetrahydrofuran was combined with 500 mL of pyridine, then combined with 15 g of acetic anhydride. After stirring at room temperature overnight, the reaction mixture was condensed. The resulting residue was combined with ethyl acetate and physiological saline, and the ethyl acetate layer was separated. The ethyl acetate layer was washed with water followed by saturated physiological saline, then dried over anhydrous sodium sulfate. After the solvent had been distilled off, the resulting residue was recrystallized from ethyl acetate/n-hexane to give 38.3 g of the intended compound as a yellow crystal.
Rf value (relative difference) (silica gel thin layer chromatography, n-hexane/ethyl acetate = 1/1): 0.56.

### Reference Example 12

### 4-(N-2-acetoxyethyl-N-methylamino)-2-(N-t-butoxycarbonyl-N-methylamino)aniline

A mixture of 34 g of 4-(N-2-acetoxyethyl-N-methylamino)-2-(N-t-butoxycarbonyl-N-methylamino)nitrobenzene (the compound of Reference Example 11) and 250 mL of methanol was purged with argon, then combined with 3.13 g of 10% palladium on carbon and purged with hydrogen gas at room temperature for three hours. After insoluble matter had been filtered out from the reaction mixture, the filtrate was condensed to give 29.3 g of a coarse product material of the intended compound as a colorless liquid.
Rf value (relative difference) (silica gel thin layer chromatography, n-hexane/ethyl acetate = 1/1): 0.42.

### Reference Example 13

### N-[4-(N-2-acetoxyethyl-N-methylamino)-2-(N-t-butoxycarbonyl-N-methylamino)phenyl]-4-methyl-2-propyl-1-[2'-(1-trifphenylmethyl-1H-tetrazol-5-yl)biphenyl-4-ylmethyl]-1H-benzimidazole-6-carboxamide

A solution of 1.693 g of 4-(N-2-acetoxyethyl-N-methylamino)-2-(N-t-butoxycarbonyl-N-methylamino)aniline (the compound of Reference Example 12) and 3.474 g of 4-methyl-2-propyl-1--[2'-(1-trifphenylmethyl-1H-tetrazol-5-yl)biphenyl-4-ylmethyl]-1H-benzimidazole-6-carboxylic acid in 30 mL of dry tetrahydrofuran was combined with 1.08 g of diethyl cyanophosphonate, then combined with 0.56 g of triethylamine and stirred at room temperature for four days. The reaction solution was combined with ethyl acetate and water, and the ethyl acetate layer was separated. The ethyl acetate layer was washed twice with water, then washed with 0.5 N hydrochloric acid and 1 N hydrochloric acid followed by physiological saline, and dried over anhydrous sodium sulfate. The solvent was distilled off to give 3.69 g of a coarse product material of the intended compound.
Rf value (relative difference) (silica gel thin layer chromatography, n-hexane/ethyl acetate = 1/1): 0.26.

### Reference Example 14

### 6- {6-(N-2-hydroxyethyl-N-methylamino)-1-methyl-1H-benzimidazol-2-yl}-4-methyl-2-propyl-1-[2'-(1H-tetrazol-5-yl)biphenyl-4-ylmethyl]-1H-benzimidazole

A solution of 3.69 g of N-[4-(N-2-acetoxyethyl-N-methylamino)-2-(N-t-butoxycarbonyl-N-methylamino)phenyl]-4-methyl-2-propyl-1-[2'-(1-trifphenylmethyl-1H-tetrazol-5-yl)biphenyl-4-ylmethyl]-1H-benzimidazole-6-carboxamide (the compound of Reference Example 13) in 50 mL of a 4 N hydrogen chloride-dioxane solution was heated at an oil-bath temperature of 50°C to 60°C for ten hours. Insoluble matter was obtained from the reaction mixture using the gradient method, and this insoluble matter was washed twice with ethyl acetate and twice with n-hexane. After combining insoluble matter with diisopropyl ether and sonicating to pulverize, the powder was collected by filtration. This powder was then combined with 50 mL of methanol and a solution of 2 g of potassium hydroxide in 10 mL of water and left to stand overnight. After buffering to pH 4 using 0.1 N hydrochloric acid, this reaction mixture was combined with ammonia water, the solvent was distilled off under reduced pressure, and the residue was refined by silica gel column chromatography (elution solvent: ethyl acetate/ethanol = 5/1 to 3/1, then 2/1) to give 1.15 g of the intended compound as a light yellow powder.
Rf value (relative difference) (silica gel thin layer chromatography, elution solvent: ethyl acetate/ethanol = 10/1): 0.20.

### Reference Example 15

### 6- {6-[N-[2-(3-(N-t-butoxycarbonyl-N-methylamino)-4-nitrophenoxy)ethyl]-N-methylamino]-1-methyl-1H-benzimidazol-2-yl}-4-methyl-2-propyl-1-[2'-(1H-tetrazol-5-yl)biphenyl-4-ylmethyl]-1H-benzimidazole

A mixture of 1.15 g of 6-{6-(N-2-hydroxyethyl-N-methylamino)-1-methyl-1H-benzimidazol-2-yl}-4-methyl-2-propyl-1-[2'-(1H-tetrazol-5-yl)biphenyl-4-ylmethyl]-1H-benzimidazole (the compound of Reference Example 14) and 0.15 g of 60% sodium hydride was combined with 10 mL of dry tetrahydrofuran and 5 mL of dry dimethylformamide and stirred at room temperature for thirty minutes, then sonicated for thirty minutes. The reaction mixture was combined with 0.54 g of 4-chloro-2-(N-t-butoxycarbonyl-N-methylamino)nitrobenzene and stirred at room temperature overnight. The reaction mixture was combined with 0.5 g more of 4-chloro-2-(N-t-butoxycarbonyl-N-methylamino)nitrobenzene and 0.08 g more of 60% sodium hydride and stirred at room temperature for six hours. The reaction mixture was combined with ethyl acetate and water, and buffered to pH 5 using 0.1 N hydrochloric acid, then the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/ethanol = 10/1) to give 1.1 g of the intended compound as a light yellow solid.
Rf value (relative difference) (silica gel thin layer chromatography, ethyl acetate): 0.33.

### Reference Example 16

### 6- {6-[N-[2-(4-amino-3-(N-t-butoxycarbonyl-N-methylamino)phenoxy)ethyl]-N-methylamino]-1-methyl-1H-benzimidazol-2-yl}-4-methyl-2-propyl-1-[2'-(1H-tetrazol-5-yl)biphenyl-4-ylmethyl]-1H-benzimidazole

A mixture of 1.1 g of 6-{6-[N-[2-(3-(N-t-butoxycarbonyl-N-methylamino)-4-nitrophenoxy)ethyl]-N-methylamino]-1-methyl-1H-benzimidazol-2-yl -4-methyl-2-propyl-1-[2'-(1H-tetrazol-5-yl)biphenyl-4-ylmethyl]-1H-benzimidazole (the compound of Reference Example 15) and 30 mL of methanol was purged with argon, then combined with 0.5 g of 10% palladium on carbon and purged with hydrogen gas through at room temperature for three hours. The reaction system was purged with argon, then allowed to stand at room temperature overnight. The reaction mixture was combined with 0.5 g of 10% palladium on carbon, and purged with hydrogen gas at room temperature for three hours. After purging the reaction system with argon, insoluble matter was filtered out from the reaction mixture and the filtrate was condensed to give 0.94 g of a coarse product material of the intended compound as a light brown solid.

### Reference Example 17

### 5-[4-[4- {2-(N-(1-methyl-2-[4-methyl-2-propyl-1-(2'-(1H-tetrazol-5-yl)biphenyl-4-ylmethyl)-1H-benzimidazol-6-yl]-1H-benzamidazol-6-yl)-N-methylamino)ethoxy} -2-(N-t-butoxycarbonyl-N-methylamino)phenylaminocarbonylmethoxy]benzyl]thiazolidine-2,4-dione

A solution of 0.94 g of 6-{6-[N-[2-(4-amino-3-(N-t-butoxycarbonyl-N-methylamino)phenoxy)ethyl]-N-methylamino]-1-methyl-1H-benzimidazol-2-yl}-4-methyl-2-propyl-1-[2'-(1H-tetrazol-5-yl)biphenyl-4-ylmethyl]-1H-benzimidazole (the compound of Reference Example 16) and 0.32 g of 4-(2,4-dioxothiazolidin-5-ylmethyl)phenoxyacetic acid in 7 mL of dry tetrahydrofuran was combined with 0.273 g of diethyl cyanophosphonate, then combined with 0.185 g of triethylamine, sonicated for ten minutes, and stirred at room temperature for two days. The reaction mixture was refined by silica gel column chromatography (elution solvent: n-hexane/ethyl acetate = 1/1 to 10/1) to give 0.62 g of the intended compound as a yellow solid.
Rf value (relative difference) (silica gel thin layer chromatography, ethyl acetate): 0.37.

### Reference Example 18

### 2-amino-4-(N-2-hydroxyethyl-N-methylamino)nitrobenzene

A mixture of 69 g of 2-(methylamino)ethanol and 34.5 g of 2-amino-4-chloronitrobenzene was heated and stirred at an oil-bath temperature of 120°C for three hours. The residue resulting from distilling off excess 2-(methylamino)ethanol from the reaction mixture under reduced pressure was refined by silica gel column chromatography (elution solvent: n-hexane/ethyl acetate = 1/3) to give 39.1 g of the intended compound as an orange crystal.
Rf value (relative difference) (silica gel thin layer chromatography, n-hexane/ethyl acetate = 1/3): 0.41.

### Reference Example 19

### N-(2-nitro-5-(N-2-butyryloxyethyl-N-methylamino)phenylbutyric acid amide

Butyryl chloride was dropped into a mixture of 23.7 g of 2-amino-4-(N-2-hydroxyethyl-N-methylamino)nitrobenzene (the compound of Reference Example 18), 30 g of anhydrous potassium carbonate, and 200 mL of dry tetrahydrofuran while cooling with ice. The reaction mixture was stirred at room temperature for five days, then combined with 10 mL of triethylamine and allowed to stand at room temperature for nine days. The reaction mixture was condensed and combined with ethyl acetate, and the ethyl acetate layer was separated. The ethyl acetate layer was washed with physiological saline, then dried over anhydrous sodium sulfate. Distilling off the solvent gave 39.3 g of a coarse product material of the intended compound as a light yellow-brown solid.

### Reference Example 20

### N-(2-amino-5-(N-2-butyryloxyethyl-N-methylamino)phenylbutyric acid amide

A mixture of 17 g of N-(2-nitro-4-(N-2-butyryloxyethyl-N-methylamino)phenylbutyric acid amide (the compound of Reference Example 19) and 30 mL of methanol was purged with argon, then combined with 5 g of 10% of palladium on carbon and purged with hydrogen gas at room temperature for 3.5 hours. After insoluble matter had been filtered out, the reaction mixture was combined with toluene and the solvent was distilled off to give 14.5 g of a coarse product material of the intended compound as a colorless liquid.
Rf value (relative difference) (silica gel thin layer chromatography, n-hexane/ethyl acetate = 1/3): 0.43.

### Reference Example 21

### 6-(N-2-hydroxyethyl-N-methylamino)-2-propyl-1H-benzimidazole

A solution of 14.5 g of N-(2-amino-5-(N-2-butyryloxyethyl-N-methylamino)phenylbutyric acid amide (the compound of Reference Example 20) in 100 mL of a 4 N hydrogen chloride-dioxane solution was heated at an oil-bath temperature of 60°C for eight hours. The reaction mixture was combined with 100 mL of methanol and allowed to stand at room temperature for two days. The reaction mixture was condensed and combined with methanol and ammonia water, then combined with silica gel and dried under reduced pressure. The result was refined by silica gel column chromatography (elution solvent: n-hexane/ethanol = 10/1 to 5/1) to give 6.9 g of the intended compound as a white solid.

### Reference Example 22

### 4'-[6-(N-2-hydroxyethyl-N-methylamino)-2-propyl-1H-benzimidazol-1-ylmethyl]biphenyl-2-carboxylic acid t-butyl ester and 4'-[5-(N-2-hydroxyethyl-N-methylamino)-2-propyl-1H-benzimidazol-1-ylmethyl]biphenyl-2-carboxylic acid t-butyl ester

A solution of 6.9 g of 6-(N-2-hydroxyethyl-N-methylamino)-2-propyl-1H-benzimidazole (the compound of Reference Example 21) and 10.6 g of 4'-(bromoethyl)biphenyl-2-carboxylic acid t-butyl ester in 90 mL of dry dimethylformamide was combined with 10 g of anhydrous potassium carbonate and stirred at room temperature for three days. The reaction mixture was condensed, the residue was combined with ethyl acetate and physiological saline, and the ethyl acetate layer was separated. The ethyl acetate layer was washed with saturated physiological saline, then driec over anhydrous sodium sulfate. After the solvent had been distilled off, 15.4 g of the resulting residue was was refined by silica gel column chromatography (elution solvent: n-hexane/ethyl acetate/ethanol = 10/30/1) to give 3.3 g of the intended compound (6-(N-2-hydroxyethyl-N-methylamino) form) as a light yellow solid, and 2.56 g of the intended compound (5-(N-2-hydroxyethyl-N-methylamino) form) as a light yellow solid. 6-(N-2-hydroxyethyl-N-methylamino) form
Rf value (relative difference) (silica gel thin layer chromatography, n-hexane/ethyl acetate = 1/3): 0.47.
5-(N-2-hydroxyethyl-N-methylamino) form
Rf value (relative difference) (silica gel thin layer chromatography, n-hexane/ethyl acetate = 1/3): 0.29.

### Reference Example 23

### 4'-[6-{N-2-(3-(N-t-butoxycarbonyl-N-methylamino)-4-nitrophenoxy)ethyl-N-methylamino}-2-propyl-1H-benzimidazol-1-ylmethyl]biphenyl-2-carboxylic acid t-butyl ester

A solution of 3.2 g of 4'-[6-(N-2-hydroxyethyl-N-methylamino)-2-propyl-1H-benzimidazol-1-ylmethyl]biphenyl-2-carboxylic acid t-butyl ester (the 6-(N-2-hydroxyethyl-N-methylamino) form of the compound of Reference Example 22) in 32 mL of dry dimethylformamide was combined with 0.256 g of 60% sodium hydride while cooling with ice, stirred for thirty minutes, then combined with 2.0 g of 4-chloro-2-(N-t-butoxycarbonyl-N-methylamino)nitrobenzene and stirred at room temperature for two days. The reaction mixture was combined with ethyl acetate and physiological saline, and the ethyl acetate layer was separated. The ethyl acetate layer was washed with saturated physiological saline, then dried over anhydrous sodium sulfate. After distilling of the solvent, 7.69 g of the resulting residue was refined by silica gel column chromatography (elution solvent: n-hexane/ethyl acetate = 1/3) to give 3.38 g of the intended compound as a light yellow solid.
Rf value (relative difference) (silica gel thin layer chromatography, n-hexane/ethyl acetate = 1/3): 0.69.

### Reference Example 24

### 4'-[5-{N-2-(3-(N-t-butoxycarbonyl-N-methylamino)-4-nitrophenoxy)ethyl-N-methylamino}-2-propyl-1H-benzimidazol-1-ylmethyl]biphenyl-2-carboxylic acid t-butyl ester

Using 2.56 g of 4'-[5-(N-2-hydroxyethyl-N-methylamino)-2-propyl-1H-benzimidazol-1-ylmethyl]biphenyl-2-carboxylic acid t-butyl ester (the 5-(N-2-hydroxyethyl-N-methylamino) form of the compound of Reference Example 22), 0.21 g of 60% sodium hydride, and 2.0 g of 4-chloro-2-(N-t-butoxycarbonyl-N-methylamino)nitrobenzene, the same reaction as Reference Example 23 was carried out (reaction time: 5 hours) to give 2.8 g of the intended compound as a light yellow solid.
Rf value (relative difference) (silica gel thin layer chromatography, n-hexane/ethyl acetate = 1/3): 0.62.

### Reference Example 25

### 4'-[6-[N-2-{N-2-(4-amino-3-(N-t-butoxycarbonyl-N-methylamino)phenoxy)ethyl-N-methylamino}-2-propyl-1H-benzimidazol-1-ylmethyl]biphenyl-2-carboxylic acid t-butyl ester

A mixture of 1.88 g of 4'-[6-{N-2-(3-(N-t-butoxycarbonyl-N-methylamino)-4-mtrophenoxy)ethyl-N-methylamino}-2-propyl-1H-benzimidazol-1-ylmethyl]biphenyl-2-carboxylic acid t-butyl ester (the compound of Reference Example 23) in 50 mL of ethanol was purged with argon, then combined with 1 g of 10% palladium on carbon and purged with hydrogen gas at room temperature for five hours. After the reaction system was purged with argon, insoluble matter was filtered off, the filtrate was condensed, and 1.84 g of the resulting residue was refined by silica gel column chromatography (elution solvent: n-hexane/ethyl acetate = 1/3) to give 1.66 g of the intended compound as a colorless solid.
Rf value (relative difference) (silica gel thin layer chromatography, n-hexane/ethyl acetate = 1/3): 0.58.

### Reference Example 26

### 4'-[5- {N-2-(4-amino-3-(N-t-butoxycarbonyl-N-methylamino)phenoxy)ethyl-N-methylamino}-2-propyl-1H-benzimidazol-1-ylmethyl]biphenyl-2-carboxylic acid t-butyl ester

The same reaction as Reference Example 25 was carried out (reaction time: 6 hours) using 2 g of 10% palladium on carbon and a solution of 1.78 g of 4'-[5-{N-2-(3-(N-t-butoxycarbonyl-N-methylamino)-4-nitrophenoxy)ethyl-N-methylamino} -2-propyl-1H-benzimidazol-1-ylmethyl]biphenyl-2-carboxylic acid t-butyl ester (the compound of Reference Example 24) in 40 mL of methanol. Insoluble matter was filtered off, the filtrate was condensed, and 1.8 g of the resulting residue were combined with a small volume of dilute sulfuric acid and ethyl acetate and buffered to pH 8-9 using a sodium hydroxide aqueous solution, then the ethyl acetate layer was separated and the ethyl acetate layer was dried over anhydrous sodium sulfate. After the solvent had been distilled off, 1.4 g of the resulting residue was refined by silica gel column chromatography (elution solvent: n-hexane/ethyl acetate = 1/3) to give 0.72 g of the intended compound as a light red solid.
Rf value (relative difference) (silica gel thin layer chromatography, n-hexane/ethyl acetate = 1/3): 0.52.

### Reference Example 27

### 4'-[2-propyl-6- {N-2-(3-(N-t-butoxycarbonyl-N-methylamino)-4-[4-(2,4-dioxothiazolidin-5-ylmethyl)phenoxymethylcarbonylamino]phenoxy)ethyl-N-methylamino}-1H-benzimidazol-1-ylmethoxy]biphenyl-2-carboxylic acid t-butyl ester

A solution of 1.64 g of 4'-[6-[N-2-{N-2-(4-amino-3-(N-t-butoxycarbonyl-N-methylamino)phenoxy)ethyl-N-methylamino }-2-propyl-1H-benzimidazol-1-ylmethyl]biphenyl-2-carboxylic acid t-butyl ester (the compound of Reference Example 25) and 0.65 g of 4-(2,4-dioxothiazolidin-5-ylmethyl)phenoxyacetic acid in 10 mL of dry tetrahydrofuran was combined with 0.38 g of diethyl cyanophosphonate, then combined with 0.25 g of triethylamine and stirred at room temperature for five days. The reaction mixture was combined with ethyl acetate, then refined by silica gel column chromatography (elution solvent: n-hexane/ethyl acetate = 1/3) to give 1.88 g of the intended compound as a white solid.
Rf value (relative difference) (silica gel thin layer chromatography, n-hexane/ethyl acetate = 1/3): 0.54.

### Reference Example 28

### 4'-[2-propyl-5- {N-2-(3-(N-t-butoxycarbonyl-N-methylamino)-4-[4-(2,4-dioxothiazolidin-5-ylmethyl)phenoxymethylcarbonylamino]phenoxy)ethyl-N-methylamino}-1H-benzimidazol-1-ylmethyl]biphenyl-2-carboxylic acid t-butyl ester

A solution of 0.72 g of 4'-[5-{N-2-(4-amino-3-(N-t-butoxycarbonyl-N-methylamino)phenoxy)ethyl-N-methylamino}-2-propyl-1H-benzimidazol-1-ylmethyl]biphenyl-2-carboxylic acid t-butyl ester (the compound of Reference Example 26) and 0.282 g of 4-(2,4-dioxothiazolidin-5-ylmethyl)phenoxyacetic acid in 4 mL of dry tetrahydrofuran was combined with 0.164 g of diethyl cyanophosphonate, then combined with 0.12 g of triethylamine and stirred at room temperature for two days. The reaction mixture was treated and refined in the same manner as Reference Example 27 to give 0.68 g of the intended compound as a white solid.
Rf value (relative difference) (silica gel thin layer chromatography, n-hexane/ethyl acetate = 1/3): 0.50.

### Reference Example 29

### 4' -(6-chloromethyl-4-methyl-2-propyl-1H-benzimidazol-1-ylmethyl]biphenyl-2-carboxylic acid t-butyl ester

A solution of 5.22 g of 4'-(6-hydroxymethyl-4-methyl-2-propyl-1H-benzimidazol-1-ylmethyl]biphenyl-2-carboxylic acid t-butyl ester in 30 mL of dry dimethylformamide was combined with 1.4 g of methanesulfonyl chloride, then combined with 1.7 g of N,N-diisopropyl ethylamine while cooling with ice, and stirred at room temperature for four days. The reaction mixture was combined with ethyl acetate/n-hexane and water, and the organic layer was separated. The organic layer was washed with saturated physiological saline, then dried over anhydrous sodium sulfate. After the solvent had been distilled off, 6.3 g of the resulting residue was refined by silica gel column chromatography (elution solvent: n-hexane/ethyl acetate = 3/1) to give 4.75 g of the intended compound as a colorless liquid. This compound was allowed to stand at room temperature to crystallize.
Rf value (relative difference) (silica gel thin layer chromatography, n-hexane/ethyl acetate = 3/1): 0.31.

### Reference Example 30

### 4'-[6-(4-hydroxyphenylthiomethyl)-4-methyl-2-propyl-1H-benzimidazol-1-ylmethyl]biphenyl-2-carboxylic acid t-butyl ester

A suspension of 0.4 g of 60% sodium hydride in 5 mL of dry tetrahydrofuran and 20 mL of dry dimethylformamide was purged with argon, then combined with 1.6 g of 4-mercaptophenol while cooling with ice, then combined with a solution of 4.73 g of 4'-(6-chloromethyl-4-methyl-2-propyl-1H-benzimidazol-1-ylmethyl]biphenyl-2-carboxylic acid t-butyl ester (the compound of Reference Example 29) in 10 mL of dry dimethylformamide. The reaction mixture was stirred at room temperature overnight, then combined with ethyl acetate and physiological saline, and the ethyl acetate layer was separated. The ethyl acetate layer was washed with saturated physiological saline, then dried over anhydrous sodium sulfate. After the solvent had been distilled off, 6.66 g of the resulting residue was refined by silica gel column chromatography (elution solvent: n-hexane/ethyl acetate = 1/1) to give a white crystal, which was recrystallized from ethyl acetate to give 2.81 g of the intended compound.
Rf value (relative difference) (silica gel thin layer chromatography, n-hexane/ethyl acetate = 2/1): 0.19.

### Reference Example 31

### 4'-[6- {4-(3-(N-t-butoxycarbonyl-N-methylamino)-4-nitrophenoxy)phenylthiomethyl}-4-methyl-2-propyl-1H-benzimidazol-1-ylmethyl]biphenyl-2-carboxylic acid t-butyl ester

A solution of 2.8 g of 4'-[6-(4-hydroxyphenylthiomethyl)-4-methyl-2-propyl-1H-benzimidazol-1-ylmethyl]biphenyl-2-carboxylic acid t-butyl ester (the compound of Reference Example 30) in 15 mL of dry dimethylformamide was combined with 0.2 g of 60% sodium hydride while cooling with ice, and stirred at room temperature for thirty minutes, then combined with 2.0 g of 4-chloro-2-(N-t-butoxycarbonyl-N-methylamino)nitrobenzene, stirred at room temperature overnight, and heated and stirred at an oil-bath temperature of 60°C for nine hours. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 5.28 g of the resulting residue was refined by silica gel column chromatography (elution solvent: n-hexane/ethyl acetate = 2/1) to give 3.72 g of the intended compound as a light yellow glassy solid.
Rf value (relative difference) (silica gel thin layer chromatography, n-hexane/ethyl acetate = 2/1): 0.26.

### Reference Example 32

### 4'-[6-{4-(4-amino-3-(N-t-butoxycarbonyl-N-methylamino)phenoxy)phenylthiomethyl}-4-methyl-2-propyl-1H-benzimidazol-1-ylmethyl]biphenyl-2-carboxylic acid t-butyl ester

A mixture of 1.71 g of 4'-[6-{4-(3-(N-t-butoxycarbonyl-N-methylamino)-4-nitrophenoxy)phenylthiomethyl}-4-methyl-2-propyl-1H-benzimidazol-1-ylmethyl]biphenyl-2-carboxylic acid t-butyl ester(the compound of Reference Example 31) and 20 mL of methanol was purged with argon, then with 1.71 g of 10% palladium on carbon and purged with hydrogen gas at room temperature for one hour. After insoluble matter had been filtered off, the reaction mixture was refined by silica gel column chromatography (elution solvent: n-hexane/ethyl acetate = 1/1) to give 1.39 g of the intended compound as a light yellow crystal.
Rf value (relative difference) (silica gel thin layer chromatography, n-hexane/ethyl acetate = 1/1): 0.36.

### Reference Example 33

### 4'-[6-{4-(3-(N-t-butoxycarbonyl-N-methylamino)-4-[4-(2,4-dioxothazolidin-5-ylmethyl)phenoxymethylcarbonylamino]phenoxy)phenylthiomethyl }-4-methyl-2-propyl-1H-benzimidazol-1-ylmethyl]biphenyl-2-carboxylic acid t-butyl ester

A solution of 1.39 g of 4'-[6-{4-(4-amino-3-(N-t-butoxycarbonyl-N-methylamino)phenoxy)phenylthiomethyl}-4-methyl-2-propyl-1H-benzimidazol-1-ylmethyl]biphenyl-2-carboxylic acid t-butyl ester (the compound of Reference Example 32) and 0.52 g of 4-(2,4-dioxothiazolidin-5-ylmethyl)phenoxyacetic acid in 6 mL of dry tetrahydrofuran was combined with 0.31 g of diethyl cyanophosphonate, then combined with 0.2 g of triethylamine and stirred at room temperature for five days. The residue resulting from distilling off the solvent was refined by silica gel column chromatography (elution solvent: n-hexane/ethyl acetate = 5/7) to give 0.96 g of the intended compound as a light yellow powder.
Rf value (relative difference) (silica gel thin layer chromatography, n-hexane/ethyl acetate = 1/1): 0.24.

### Reference Example 34

### 4-(3-(N-t-butoxycarbonyl-N-methylamino)-4-nitrophenoxymethyl)-2-propyl-1-[2'-(1-triphenylmethyl-1H-tetrazol-5-yl)biphenyl-4-ylmethyl]-1H-imidazole-5-carboxylic acid ethyl ester

A solution of 7.0 g of 4-hydroxymethyl-2-propyl-1-[2'-(1-triphenylmethyl-1H-tetrazol-5-yl)biphenyl-4-ylmethyl]-1H-imidazole-5-carboxylic acid ethyl ester in 40 mL of dry dimethylformamide was combined with 0.41 g of 60% sodium hydride while cooling with ice and stirred for thirty minutes, then stirred at room temperature for thirty minutes before combining with 3.4 g of 4-chloro-2-(N-t-butoxycarbonyl-N-methylamino)nitrobenzene and stirring at room temperature for one day. The reaction mixture was combined with ethyl acetate and physiological saline, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 3/2) to give 6.4 g of the intended compound as a light yellow solid.
Rf value (relative difference) (silica gel thin layer chromatography, n-hexane/ethyl acetate = 1/1): 0.69.

### Reference Example 35

### 4-(4-amino-3-(N-t-butoxycarbonyl-N-methylamino)phenoxymethyl)-2-propyl-1-[2'-(1-triphenylmethyl-1H-tetrazol-5-yl)biphenyl-4-ylmethyl]-1H-imidazole-5-carboxylic acid ethyl ester

A mixture of 6.3 g of 4-(3-(N-t-butoxycarbonyl-N-methylamino)-4-nitrophenoxymethyl)-2-propyl-1-[2'-(1-triphenylmethyl-1H-tetrazol-5-yl)biphenyl-4-ylmethyl]-1H-imidazole-5-carboxylic acid ethyl ester (the compound of Reference Example 34) and 15 mL of ethanol was purged with argon, then combined with 3 g of 10% palladium on carbon and purged with hydrogen gas at room temperature for one hour. The reaction mixture was combined with 10 mL of tetrahydrofuran and purged with hydrogen gas at room temperature for five hours. After insoluble matter had been filtered off from the reaction mixture, the filtrate was condensed and the resulting residue was refined by silica gel column chromatography (elution solvent: n-hexane/ethyl acetate = 1/1) to give 2.44 g of the intended compound as a colorless glassy solid.
Rf value (relative difference) (silica gel thin layer chromatography, n-hexane/ethyl acetate = 1/1): 0.26.

### Reference Example 36

### 4-[3-(N-t-butoxycarbonyl-N-methylamino)-4-{4-(2,4-dioxothiazolidin-5-ylmethyl)phenoxyacetylamino}phenoxymethyl]-2-propyl-1-[2'-(1-triphenylmethyl-1H-tetrazol-5-yl)biphenyl-4-ylmethyl]-1H-imidazole-5-carboxylic acid ethyl ester

A solution of 2.4 g of 4-(4-amino-3-(N-t-butoxycarbonyl-N-methylamino)phenoxymethyl)-2-propyl-1-[2'-(1-triphenylmethyl-1H-tetrazol-5-yl)biphenyl-4-ylmethyl]-1H-imidazole-5-carboxylic acid ethyl ester (the compound of Reference Example 35) and 0.75 g of 4-(2,4-dioxothiazolidin-5-ylmethyl)phenoxyacetic acid in 5 mL of dry tetrahydrofuran was combined with 0.44 g of diethyl cyanophosphonate, then combined with 0.3 g of triethylamine and stirred at room temperature for five days. The reaction mixture was combined with ethyl acetate and silica gel, the solvent was distilled off, and the residue was refined by silica gel column chromatography (elution solvent: n-hexane/ethyl acetate = 3/2) to give 2.69 g of the intended compound as a light yellow solid.
Rf value (relative difference) (silica gel thin layer chromatography, n-hexane/ethyl acetate = 1/1): 0.17.

### Reference Example 37

### 4-(4-hydroxyphenylthiomethyl)-2-propyl-1-[2'-(1-triphenylmethyl-1H-tetrazol-5-yl)biphenyl-4-ylmethyl]-1H-imidazole-5-carboxylic acid ethyl ester

A solution of 3.45 g of 4-hydroxymethyl-2-propyl-1-[2'-(1-triphenylmethyl-1H-tetrazol-5-yl)biphenyl-4-ylmethyl]-1H-imidazole-5-carboxylic acid ethyl ester in 20 mL of dry methylene chloride was combined with 0.58 g of methanesulfonyl chloride, then combined with first 0.66 g of N,N-diisopropyl ethylamine and stirred at room temperature for fifteen hours, then 0.65 g of 4-mercaptophenol, then 0.67 g of N,N-diisopropyl ethylamine and stirred at room temperature for one day. After the solvent had been distilled off under reduced pressure, the residue was combined with ethyl acetate and water, and the ethyl acetate layer was separated. The ethyl acetate layer was washed with saturated physiological saline, then dried over anhydrous sodium sulfate. After the solvent had been distilled off, 4.28 g of the resulting residue was refined by silica gel column chromatography (elution solvent: n-hexane/ethyl acetate = 2/1) to give 2.65 g of the intended compound as a white powder.
Rf value (relative difference) (silica gel thin layer chromatography, n-hexane/ethyl acetate = 1/1): 0.47.

### Reference Example 38

### 4-[4-{3-(N-t-butoxycarbonyl-N-methylamino)-4-nitrophenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester

A suspension of 0.137 g of sodium hydride (purity: 60%) in 15 mL of dry dimethylformamide was combined with 2.65 g of 4-(4-hydroxyphenylthiomethyl)-2-propyl-1-[2'-(1-triphenylmethyl-1H-tetrazol-5-yl)biphenyl-4-ylmethyl]-1H-imidazole-5-carboxylic acid ethyl ester (the compound of Reference Example 37) and stirred at room temperature for one hour. This solution was combined with 1.5 g of 4-chloro-2-(N-t-butoxycarbonyl-N-methylamino)nitrobenzene and heated and stirred at an oil-bath temperature of 70°C for seven hours. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 4.93 g of the resulting residue was refined by silica gel column chromatography (elution solvent: n-hexane/ethyl acetate = 2/1) to give 2.75 g of the intended compound as a light yellow powder.
Rf value (relative difference) (silica gel thin layer chromatography, n-hexane/ethyl acetate = 1/1): 0.70.

### Reference Example 39

### 4-[4- {4-amino-3-(N-t-butoxycarbonyl-N-methylamino)phenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester and 4-[4-{4-amino-3-(N-t-butoxycarbonyl-N-methylamino)phenoxy}phenylthiomethyl]-2-propyl-1-[2'-(1H-tetrazol-5-yl)biphenyl-4-ylmethyl]-1H-imidazole-5-carboxylic acid ethyl ester

### (a) 4-[4-{4-amino-3-(N-t-butoxycarbonyl-N-methylamino)phenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester

A mixture of 14.2 g of 4-[4-{3-(N-t-butoxycarbonyl-N-methylamino)-4-nitrophenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 38), 100 mL of methanol, and 50 mL of tetrahydrofuran was purged with argon, then combined with 10.2 g of 10% palladium on carbon and purged with hydrogen gas at room temperature for five hours. The mixture was combined with 5.1 g more of 10% palladium on carbon and purged with hydrogen gas at room temperature for twenty hours. After insoluble matter had been filtered out from the reaction mixture, the filtrate was condensed and the resulting residue was refined by silica gel column chromatography (elution solvent: n-hexane/ethyl acetate = 1/1 to 1/5) to give 6.02 g of the intended compound as a white solid.
Rf value (relative difference) (silica gel thin layer chromatography, n-hexane/ethyl acetate = 1/1): 0.45.

Mass spectrum: 1017 (M+1)⁺.

### (b) 4-[4-{4-amino-3-(N-t-butoxycarbonyl-N-methylamino)phenoxy}phenylthiomethyl]-2-propyl-1-[2'-(1H-tetrazol-5-yl)biphenyl-4-ylmethyl]-1H-imidazole-5-carboxylic acid ethyl ester

Using 2.69 g of the fraction following silica gel column chromatography in (a), 30 mL of methanol, and 3 g of 10% palladium on carbon, this system was purged with hydrogen gas at 50°C for eight hours, then purged with hydrogen gas at room temperature for three days and subjected to the same after-treatment as (a) and refined by silica gel column chromatography (elution solvent: ethyl acetate) to give 1.40 g of the intended compound as a white solid.
Rf value (relative difference) (silica gel thin layer chromatography, ethyl acetate): 0.42.

Mass spectrum: 775 (M+1)⁺.

### Reference Example 40-1

### 4- {4-[4-[4-(2,4-dioxothiazolidin-5-ylmethyl)phenoxyacetylamino]-3-(N-t-butoxycarbonyl-N-methylamino)phenoxy]phenylthiomethyl}-2-propyl-1-[2'-(1-triphenylmethyl-1H-tetrazol-5-carboxylic acid ethyl ester

(a) A solution of 2.12 g of 4-[4-{4-amino-3-(N-t-butoxycarbonyl-N-methylamino)phenoxy} phenylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 39(a)) and 0.616 g of 4-(2,4-dixothiazolidin-5-ylmethyl)phenoxyacetic acid in 5 mL of dry tetrahydrofuran was combined with 0.374 g of diethyl cyanophosphonate, then combined with 0.253 g of triethylamine and stirred at room temperature for one day. The system was then heated and stirred at an oil-bath temperature of 50-60°C for three hours. After the solvent had been distilled off from the reaction mixture, the residue was refined by silica gel column chromatography (elution solvent: n-hexane/ethyl acetate = 1/1) to give 1.23 g of the intended compound as a white solid.
Rf value (relative difference) (silica gel thin layer chromatography, n-hexane/ethyl acetate: 1/1): 0.26.

(b) A mixture of 6.0 g of 4-[4-{4-amino-3-(N-t-butoxycarbonyl-N-methylamino)phenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 39(a)), 1.66 g of 4-(2,4-dixothiazolidin-5-ylmethyl)phenoxyacetic acid, and 7 mL of drydimethylformamide was combined with 1.13 g of WSC (1-ethyl-3-(3-dimethylaminopropyl)carbodiimide hydrochloride) and stirred at room temperature for one day. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 7.8 g of the resulting residue was refined by silica gel column chromatography (elution solvent: n-hexane/ethyl acetate = 2/1 to 1/1) to give 4.49 g of the intended compound as a white powder.

¹H-NMR spectra 100MHz, DMSO-d₆), *δ*ₚₚₘ : 0.7 4 (t, 3H), 1.12 (t, 3H), 1.1-1.5 (broad, 9H) 1. 53 (m, 2H), 1.99 (s, 3H), 2. 41 (t, 2H) , 2.98 (s, 3H), 3.07 (q, 1H), 3.31 (1H. overlapping H₂O), 4.04 (t, 1H), 4.31 (s, 2H), 4.70 (s, 2H), 4.87 (q, 1H), 5.45 (s, 2H), 6.78-7.7 7 (m, 19H), 12.00 (s, 1H).

### Reference Example 40-2

### 4-{4-[4-[4-(2,4-dioxothiazolidin-5-ylmethyl)phenoxyacetylamino]-3-(N-t-butoxycarbonyl-N-methylamino)phenoxy]phenylthiomethyl}-2-propyl-1-[2'-(1H-tetrazol-5-yl)biphenyl-4-ylmethyl]-1H-imidazol-5-carboxylic acid ethyl ester

A mixture of 1.38 g of 4-[4-{4-amino-3-(N-t-butoxycarbonyl-N-methylamino)phenoxy}phenylthiomethyl]-2-propyl-1-[2'-(1H-tetrazol-5-yl)biphenyl-4-ylmethyl]-1H-imidazole-5-carboxylic acid ethyl ester (the compound of Reference Example 39(b)), 0.501 g of 4-(2,4-dixothiazolidin-5-ylmethyl)phenoxyacetic acid, and 3 mL of dry dimethylformamide was combined with 0.341 g of WSC (1-methyl-3-(3-dimethylaminopropyl)carbodiimide hydrochloride) and stirred at room temperature overnight. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 1.96 g of the resulting residue was refined by silica gel column chromatography (elution solvent: ethyl acetate/methanol = 40/1) to give 1.56 g of the intended compound as a light yellow powder.

### Reference Example 41

### N-methyl-4-methyl-2-propyl-1H-benzimidazole-6-carboxamide

A mixture of 15 g of 4-methyl-2-propyl-1H-benzimidazole-6-carboxylic acid and 100 mL of sulfonyl chloride was allowed to stand at room temperature overnight, heated and stirred at an oil-bath temperature of 80°C for five hours, then combined with 0.1 mL of dimethylformamide and again heated and stirred at 80°C for five hours. After the sulfonyl chloride had been distilled off under reduced pressure, the residue was combined with 300 mL of dry dimethylformamide and 15 g of methylamine hydrochloride, then 40 g of triethylamine were dropped in while cooling with ice. The residue resulting from stirring at room temperature for two days, then distilling off the solvent was combined with ethyl acetate and physiological saline, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 10/1) to give 4.4 g of the intended compound as a white solid.

Mass spectrum: 232 (M+1)⁺.

### Reference Example 42

### 4-methyl-6-N-methylaminomethyl-2-propyl-1H-benzimidazole

A suspension of 2.5 g of lithium aluminum hydride in 40 mL of dry tetrahydrofuran was combined with 4.35 g of N-methyl-4-methyl-2-propyl-1H-benzimidazole-6-carboxamide (the compound of Reference Example 41) and heat-refluxed for eight hours. The reaction mixture was combined with 4 mL of water and 140 mL of tetrahydrofuran while cooling with ice, and stirred at room temperature for one hour. The reaction mixture was combined with active diatomite, insoluble matter was filtered out, and this insoluble matter was suspended in methanol and filtered. The filtrate was condensed to give 3.98 g of a coarse product material of the intended compound.

### Reference Example 43

### 6-(N-t-butoxycarbonyl-N-methylaminomethyl)-4-methyl-2-propyl-1H-benzimidazole

A solution of 3.96 g of 4-methyl-6-N-methylaminomethyl-2-propyl-1H-benzimidazole (the compound of Reference Example 42) in 40 mL of dry tetrahydrofuran was combined with 4.8 g of di-t-butyl bicarbonate and stirred at room temperature for twelve hours. The residue resulting by distilling off the solvent was refined by silica gel column chromatography (elution solvent: n-hexane/ethyl acetate = 1/1 to 1/3, then 1/5) to give 2.65 g of the intended compound as a white solid.
Rf value (relative difference) (silica gel thin layer chromatography, ethyl acetate): 0.44.

### Reference Example 44

### 4'-[6-(N-t-butoxycarbonyl-N-methylaminomethyl)-4-methyl-2-propyl-1H-benzimidazol-1-ylmethyl]biphenyl-2-carboxylic acid methyl ester

A solution of 2.59 g of 6-(N-t-butoxycarbonyl-N-methylaminomethyl)-4-methyl-2-propyl-1H-benzimidazole (the compound of Reference Example 43) in 25 mL of dry dimethylformamide was combined with 0.33 g of 60% sodium hydride while cooling with ice, and stirred at room temperature for thirty minutes. The solution was then combined with 3.0 g of 4'-bromomethylbiphenyl-2-carboxylic acid methyl ester while cooling with ice, and stirred at room temperature overnight. After the solvent had been distilled off, the resulting residue was combined with ethyl acetate and physiological saline, and the ethyl acetate layer was separated. The ethyl acetate layer was washed with saturated physiological saline, then dried over anhydrous sodium sulfate. After the solvent had been distilled off, 5.5 g of the resulting residue was refined by silica gel column chromatography (elution solvent: n-hexane/ethyl acetate = 3/1 to 1/1) to give 4.52 g of the intended compound as a colorless and highly viscous liquid.
Rf value (relative difference) (silica gel thin layer chromatography, n-hexane/ethyl acetate = 1/2): 0.78.

### Reference Example 45

### 4'-[6-(N-methylaminomethyl)-4-methyl-2-propyl-1H-benzimidazol-1-ylmethyl]biphenyl-2-carboxylic acid methyl ester

A solution of 4.45 g of 4'-[6-(N-t-butoxycarbonyl-N-methylaminomethyl)-4-methyl-2-propyl-1H-benzimidazol-1-ylmethyl]biphenyl-2-carboxylic acid methyl ester (the compound of Reference Example 44) in 20 mL of a 4 N hydrogen chloride-dioxane solution was stirred at room temperature for sixteen hours. The reaction mixture was condensed, and the residue was combined with ethyl acetate and physiological saline, and the ethyl acetate layer was separated. The ethyl acetate layer was washed with saturated physiological saline, then dried over anhydrous sodium sulfate. Distilling off the solvent gave 3.44 g of the intended compound as a colorless oily matter.

### Reference Example 46

### 4'-[6-{N-methyl-N-(2-(N-methylamino)-3-nitropyridin-6-yl)aminomethyl}-4-methyl-2-propyl-1H-benzimidazol-1-ylmethyl]biphenyl-2-carboxylic acid methyl ester

A mixture of 1.44 g of 4'-[6-(N-methylaminomethyl)-4-methyl-2-propyl-1H-benzimidazol-1-ylmethyl]biphenyl-2-carboxylic acid methyl ester (the compound of Reference Example 45) and 1.44 g of 2-(N-methylamino)-3-nitro-6-chloropyridine was combined with 30 mL of dry dimethylformamide and stirred for one hour, then combined with 1 g of triethylamine and stirred at room temperature for 2.5 hours. The reaction mixture was condensed, and the residue was combined with ethyl acetate and physiological saline, and the ethyl acetate layer was separated. The ethyl acetate layer was washed with saturated physiological saline, then dried over anhydrous sodium sulfate. After the solvent had been distilled off, 5.09 g of the resulting residue was refined by silica gel column chromatography (elution solvent: n-hexane/ethyl acetate = 2/3) to give 4.28 g of the intended compound as a light yellow powder.
Rf value (relative difference) (silica gel thin layer chromatography, n-hexane/ethyl acetate = 1/2): 0.60.

### Reference Example 47

### 4'-[6- {N-(3-amino-2-(N-methylamino)-3-nitropyridin-6-yl)aminomethyl}-4-methyl-2-propyl-1H-benzimidazol-1-ylmethyl]biphenyl-2-carboxylic acid methyl ester

A mixture of 0.87 g of 4'-[6-{N-methyl-N-(2-(N-methylamino)-3-nitropyridin-6-yl)aminomethyl}-4-methyl-2-propyl-1H-benzimidazol-1-ylmethyl]biphenyl-2-carboxylic acid methyl ester (the compound of Reference Example 46), 10 mL, and 10 mL of tetrahydrofuran was purged with argon, then combined with 0.5 g of 10% palladium on carbon, then ten drops of concentrated hydrochloric acid, and purged with hydrogen gas at room temperature for three hours. After insoluble matter had been filtered out from the reaction mixture, the filtrate was condensed and combined with toluene. Condensing and combining with solvent was repeated to give 0.75 g of a coarse product material of the intended compound.

### Reference Example 48

### 4'-[6-(N-{3-[4-(2,4-dioxothiazolidin-5-ylmethyl)phenoxyacetylamino]-2-(N-methylamino)pyridin-6-yl}-N-methylaminomethyl)-4-methyl-2-propyl-1H-benzimidazol-1-ylmethyl]biphenyl-2-carboxylic acid methyl ester

A solution of 0.75 g of 4'-[6-{N-(3-amino-2-(N-methylamino)-3-nitropyridin-6-yl)aminomethyl]-4-methyl-2-propyl-1H-benzimidazol-1-ylmethyl]biphenyl-2-carboxylic acid methyl ester (the compound of Reference Example 47) and 0.454 g of 4-(2,4-dixothiazolidin-5-ylmethyl)phenoxyacetic acid in 7 mL of dry tetrahydrofuran was combined with 0.263 g of diethyl cyanophosphonate, then combined with 0.50 g of triethylamine and stirred at room temperature for twenty hours. The reaction mixture was combined with ethyl acetate and silica gel, the solvent was distilled off, and the residue was refined by silica gel column chromatography (elution solvent: ethyl acetate/ethanol = 3/1 then 2/1) to give 0.63 g of the intended compound as a yellow powder.
Rf value (relative difference) (silica gel thin layer chromatography, n-hexane/ethyl acetate = 1/2): 0.59.

Mass spectrum: 826 (M+1)⁺.

### Reference Example 49

### 5-acetylthiomethyl-2-butyl-4-chloro-1-[2'-(1-triphenylmethyl-1H-tetrazol-5-yl)biphenyl-4-ylmethyl]-1H-imidazole

A solution of 21.8 g of 2-butyl-4-chloro-5-hydroxymethyl-1-[2'-(1-triphenylmethyl-1H-tetrazol-5-yl)biphenyl-4-ylmethyl]-1H-imidazole in 100 mL of dry dimethylformamide was combined with 4.5 g of N,N-diisopropyl ethylamine, then 3.77 g of methanesulfonyl chloride were dropped in while cooling with ice, and the reaction mixture was stirred at room temperature for one hour. The reaction mixture was combined with 1.5 mL more of N,N-diisopropyl ethylamine and stirred at room temperature for one hour, and combined with 0.38 g more of methanesulfonyl chloride and stirred at room temperature overnight. The reaction mixture was combined with 5.0 g of potassium thioacetate and stirred at room temperature for four hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 3/1) to give 15.53 g of the intended compound as a light yellow powder.
Rf value (relative difference) (silica gel thin layer chromatography, n-hexane/ethyl acetate = 3/1): 0.48.

### Reference Example 50

### 2-butyl-4-chloro-5-mercaptomethyl-1-[2-(1-triphenylmethyl-1H-tetrazol-5-yl)biphenyl-4-ylmethyl]-1H-imidazole

A solution of 13.84 g of 5-acetylthiomethyl-2-butyl-4-chloro-1-[2'-(1-triphenylmethyl-1H-tetrazol-5-yl)biphenyl-4-ylmethyl]-1H-imidazole(the compound of Reference Example 49) and 3.38 g of dithioerythritol in 60 mL of methylene chloride was combined with 2.57 g of morpholine and stirred at room temperature for three days. The residue resulting from distilling off the solvent was refined by silica gel column chromatography (elution solvent: n-hexane/ethyl acetate = 3/1 to 2/1) to give 10.91 g of the intended compound as a white crystal.
Rf value (relative difference) (silica gel thin layer chromatography, n-hexane/ethyl acetate = 3/1): 0.61.

Mass spectrum: 681 (M+1)⁺.

### Reference Example 51

### 5-(3-(N-t-butoxycarbonyl-N-methylamino)-4-nitrophenylthiomethyl)-2-butyl-4-chloro-1-[2'-(1-triphenylmethyl-1H-tetrazol-5-yl)biphenyl-4-ylmethyl]-1H-imidazole

A solution of 10.88 g of 2-butyl-4-chloro-5-mercaptomethyl-1-[2-(1-triphenylmethyl-1H-tetrazol-5-yl)biphenyl-4-ylmethyl]-1H-imidazole (the compound of Reference Example 50) in 50 mL of dimethylformamide was pruged with carbon, combined with 2.21 g of potassium carbonate and 5.7 g of 4-chloro-2-(N-t-butoxycarbonyl-N-methylamino)nitrobenzene, then combined with 0.15 g of sodium hydride (purity: 60%) and stirred at room temperature for fifteen minutes. The reaction mixture was then heated and stirred at an oil-bath temperature of 60-70°C for 7.5 hours. The reaction mixture was combined with ethyl acetate, n-hexane, and physiological saline, and the organic layer was separated. The organic layer was washed with saturated physiological saline, then dried over anhydrous sodium sulfate. After the solvent had been distilled off, 17.8 g of the resulting residue was refined by silica gel column chromatography (elution solvent: n-hexane/ethyl acetate = 3/1) to give 14.52 g of the intended compound as a light yellow powder.
Rf value (relative difference) (silica gel thin layer chromatography, n-hexane/ethyl acetate = 3/1): 0.40.

### Reference Example 52

### 5-[4-amino-3-(N-t-butoxycarbonyl-N-methylamino)phenylthiomethyl]-2-butyl-4-chloro-1-[2'-(1-triphenylmethyl-1H-tetrazol-5-yl)biphenyl-4-ylmethyl]-1H-imidazole

A solution of 5.02 g of 5-(3-(N-t-butoxycarbonyl-N-methylamino)-4-nitrophenylthiomethyl)-2-butyl-4-chloro-1-[2'-(1-triphenylmethyl-1H-tetrazol-5-yl)biphenyl-4-ylmethyl]-1H-imidazole (the compound of Reference Example 51) in 50 mL of methanol and 15 mL of tetrahydrofuran was combined with 2.5 g of 10% palladium on carbon and purged with hydrogen at room temperature for four hours. The residue resulting from filtering off insoluble matter and condensing the filtrate was refined by silica gel column chromatography (elution solvent: n-hexane/ethyl acetate = 2/1) to give 3.35 g of a mixture of a triphenylmethyl form (Rf value (relative difference) (silica gel thin layer chromatography, n-hexane/ethyl acetate = 2/1): about 0.29, mass spectrum: 900 (M+H)⁺) and 5-[4-hydroxyamino-3-(N-t-butoxycarbonyl-N-methylamino)phenylthiomethyl]-2-butyl-4-chloro-1-[2'-(1-triphenylmethyl-1H-tetrazol-5-yl)biphenyl-4-ylmethyl]-1H-imidazole (4-hydroxyamino-triphenylmethyl form, mass spectrum: 916 (M+H)⁺) of the intended compound as a light yellow powder.
A solution of 3.2 g of this mixture in 45 mL of methanol was combined with 4.6 g of 10% palladium on carbon and purged with hydrogen at 50°C for one full day. Insoluble matter was filtered off, the filtrate was condensed, and 2.69 g of the resulting residue was refined by silica gel column chromatography (elution solvent: ethyl acetate) to give 1.05 g of the intended compound as a light red powder.
Rf value (relative difference) (silica gel thin layer chromatography, nethyl acetate/methanol = 20/1): about 0.43.

Mass spectrum: 659 (M+1)⁺.

### Reference Example 53

### 5-(4-{4-(4-chloro-2-butyl-1-[2'-(1H-tetrazol-5-yl)biphenyl-4-ylmethyl]-1H-imidazol-5-ylmethylthio)-2-(N-t-butoxycarbonyl-N-methylamino)phenylaminocarbonylmethoxy}benzyl)thiazolidine-2,4-dione

A mixture of 0.46 g of 5-[4-amino-3-(N-t-butoxycarbonyl-N-methylamino)phenylthiomethyl]-2-butyl-4-chloro-1-[2'-(1-triphenylmethyl-1H-tetrazol-5-yl)biphenyl-4-ylmethyl]-1H-imidazole (the compound of Reference Example 52), 0.21 g of 4-(2,4-dixothiazolidin-5-ylmethyl)phenoxyacetic acid, and 2 mL of dry dimethylformamide was combined with 0.143 g of WCS (1-ethyl-3-(3-dimethylaminopropyl)carbodiimide hydrochloride) and stirred at room temperature for three days. The reaction mixture was combined with ethyl acetate, and the ethyl acetate layer was separated. The ethyl acetate layer was washed with saturated physiological saline, then dried over anhydrous sodium sulfate. After the solvent had been distilled off, 0.70 g of the resulting residue was refined by silica gel column chromatography (elution solvent: n-hexane/ethyl acetate/methanol = 2/20/1) to give 0.27 g of the intended compound as a light yellow solid.
Rf value (relative difference) (silica gel thin layer chromatography, elution solvent: ethyl acetate/methanol = 20/1): 0.50.

Mass spectrum: 922 (M+1)⁺.
¹H-NMR: spectra (500MHz, DMSO-d₆), *δ*ₚₚₘ:0.8 0(t, 3H), 1.2 5 (m, 2H), 1. 15-1.5 ( broad s, 9H), 1.45 (m, 2H), 2.45 (t, 2H), 3.00 (s, 3 H), 3.08 (q, 1H), 3.33 (d, 1H), 4.04 (s, 2H ), 4.71 (s, H), 4. 8 8 (q, 1H), 5.28 (s, 2H), 6. 92-7. 67 (m, 15H), 7. 92 (s, 1H), 9.09 (s , 1H).

### Reference Example 54

### di-(3-(N-t-butoxycarbonyl-N-methylamino)-4-nitrophenyl sulfide

A solution of 31.45 g of 4-chloro-2-(N-t-butoxycarbonyl-N-methylamino)nitrobenzene and 18.19 g of potassium thioacetate in 170 mL of dry dimethylformamide was heated and stirred at 180°C for 8.5 hours. The residue resulting from distilling off the solvent was combined with ethyl acetate and physiological saline, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 3/1) to give 23.69 g of the intended compound as a red solid.
Rf value (relative difference) (silica gel thin layer chromatography, n-hexane/ethyl acetate = 3/1): 0.46.

Mass spectrum: 535 (M+1)⁺.

### Reference Example 55

### di-(4-amino-3-(N-t-butoxycarbonyl-N-methylamino)phenyl) sulfide

### (a) di-(4-hydroxyamino-3-(N-t-butoxycarbonyl-N-methylamino)phenyl) sulfide

A solution of 20.3 g of di-(3-(N-t-butoxycarbonyl-N-methylamino)-4-nitrophenyl sulfide (the compound of Reference Example 54) in 50 mL of tetrahydrofuran and 50 mL of methanol was combined with 17 g of 10% palladium on carbon and purged with hydrogen at room temperature for three days. The residue resulting from filtering off insoluble matter and condensing the filtrate was refined by silica gel column chromatography (elution solvent: n-hexane/ethyl acetate = 1/1) to give 13.0 g of the intended compound as a light orange powder.
Rf value (relative difference) (silica gel thin layer chromatography, n-hexane/ethyl acetate = 1/1): 0.32.

### (b) di-(4-amino-3-(N-t-butoxycarbonyl-N-methylamino)phenyl) sulfide

A solution of 8.2 g of di-(4-hydroxyamino-3-(N-t-butoxycarbonyl-N-methylamino)phenyl) sulfide (the compound of Reference Example 55(a)) in 60 mL of methanol was combined with 11.2 g of 10% palladium on carbon and purged with hydrogen at 55°C for 26 hours. The residue resulting from filtering off insoluble matter and condensing the filtrate was refined by silica gel column chromatography (elution solvent: n-hexane/ethyl acetate = 1/1) to give 5.23 g of the intended compound as a white solid.

### Reference Example 56

### N-[4-{4-amino-3-(N-t-butoxycarbonyl-N-methylamino)phenylthio}-2-(N-t-butoxycarbonyl-N-methylamino)phenyl]-4-methyl-2-propyl-1-[2'-(1H-tetrazol-5-yl)biphenyl-4-ylmethyl]-1H-benzoimidazol-6-yl carboxamide

A solution of 2.6 g of di-(4-amino-3-(N-t-butoxycarbonyl-N-methylamino)phenyl) sulfide (the compound of Reference Example 55) and 3.81 g of 4-methyl-2-propyl-1-[2'-(1-triphenylmethyl-1H-tetrazol-5-yl)biphenyl-4-ylmethyl]-1H-imidazole-5-carboxylic acid in 10 mL of dry dimethylformamide was combined with 1.05 g of WSC (1-ethyl-3-(3-dimethylaminopropyl)carbodiimide hydrochloride) and stirred at room temperature for twenty hours. The reaction mixture was combined with ethyl acetate and physiological saline, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate) to give 2.33 g of the intended compound as a light yellow powder.
Rf value (relative difference) (silica gel thin layer chromatography, n-hexane/ethyl acetate = 1/3): 0.18

Mass spectrum: 909 (M+1)⁺.

### Reference Example 57

### 5-(4-{4-[3-(N-t-butoxycarbonyl-N-methylamino)-4-(4-methyl-2-propyl-1-[2'-(1H-tetrazol-5-yl)biphenyl-4-ylmethyl]-1H-benzimidazol-6-ylcarbonylamino)phenylthio]-2-(N-t-butoxycarbonyl-N-methylamino)phenylaminocarbonylmethoxy}benzyl)thiazolidine-2,4-dione

A mixture of 2.32 g of N-[4-{4-amino-3-(N-t-butoxycarbonyl-N-methylamino)phenylthio}-2-(N-t-butoxycarbonyl-N-methylamino)phenyl]-4-methyl-2-propyl-1-[2'-(1H-tetrazol-5-yl)biphenyl-4-ylmethyl]-1H-benzoimidazol-6-yl carboxamide (the compound of Reference Example 56), 0.57 g of 4-(2,4-dixothiazolidin-5-ylmethyl)phenoxyacetic acid, and 10 mL of dry dimethylformamide was combined with 0.42 g of WSC (1-ethyl-3-(3-dimethylaminopropyl)carbodiimide hydrochloride) and stirred at room temperature for ten days. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 3.08 g of the resulting residue was refined by silica gel column chromatography (elution solvent: ethyl acetate) to give 0.99 g of the intended compound as a light yellow powder.
Rf value (relative difference) (silica gel thin layer chromatography, ethyl acetate): 0.43.

Mass spectrum: 1172 (M+1)⁺.

### Reference Example 58

### 5-ethoxycarbonyl-2-propyl-1-[2'-(1-triphenylmethyl-1H-tetrazol-5-yl)biphenyl-4-ylmethyl]-1H-imidazole-4-carboxylic acid

A solution of 0.1 g of potassium hydroxide in 0.5 mL of water was combined with a solution of 2.4 g of 2-propyl-1-[2'-(1-triphenylmethyl-1H-tetrazol-5-yl)biphenyl-4-ylmethyl]-1H-imidazole-4,5-carboxylic acid ethyl ester in 10 mL of ethanol and 10 mL of tetrahydrofuran and stirred at room temperature for sixteen hours. The solvent was distilled off, and the residue was combined with water and buffered to pH 5 using 0.1 N hydrochloric acid. The reaction solution was combined with ethyl acetate and physiological saline, and the ethyl acetate layer was separated. The ethyl acetate layer was washed with saturated physiological saline, then dried over anhydrous sodium sulfate. After the solvent had been distilled off, 2.4 g of the resulting residue was refined by silica gel column chromatography (elution solvent: ethyl acetate/methanol = 20/1), then refined again by silica gel column chromatography (elution solvent: n-hexane/ethyl acetate = 3/2 to 1/1 then 0.1) to give 0.20 g of the intended compound as a white solid.

### Reference Example 59

### 4-[4-{4-amino-3-(N-t-butoxycarbonyl-N-methylamino)phenylthio}-2-(N-t-butoxycarbonyl-N-methylamino)phenyl]-2-propyl-1-[2'-(1H-tetrazol-5-yl)biphenyl-4-ylmethyl]-1H-benzoimidazole-5-carboxylic acid ethyl ester

A mixture of 509 mg of di-(4-amino-3-(N-t-butoxycarbonyl-N-methylamino)phenyl) sulfide (the compound of Reference Example 55), 710 mg of 5-ethoxycarbonyl-2-propyl-1-[2'-(1-triphenylmethyl-1H-tetrazol-5-yl)biphenyl-4-ylmethyl]-1H-imidazole-4-carboxylic acid (the compound of Reference Example 58), and 3 mL of dry dimethylformamide was combined with 195 mg of WSC (1-ethyl-3-(3-dimethylaminopropyl)carbodiimide hydrochloride) and heated at room temperature for three days. The reaction mixture was combined with ethyl acetate and physiological saline, and the ethyl acetate layer was separated. The ethyl acetate layer was washed with saturated physiological saline, then dried over anhydrous sodium sulfate. After distilling off the solvent, 1.47 g of the resulting residue was refined by silica gel column chromatography (elution solvent: n-hexane/ethyl acetate = 1/1) to give 456 mg of the intended compound as a light yellow powder.
Rf value (relative difference) (silica gel thin layer chromatography, n-hexane/ethyl acetate = 1/1): 0.36.

### Reference Example 60

### 4-[4-{4-[4-(2,4-dioxothiazolidin-5-ylmethyl)phenoxyacetylamino]-3-(N-t-butoxycarbonyl-N-methylamino)phenylthio}-2-(N-t-butoxycarbonyl-N-methylamino)phenylaminocarbonyl]-2-propyl-1-[2'-(1-triphenylmethyl-1H-tetrazol-5-yl)biphenyl-4-ylmethyl]-1H-imidazole-5-carboxylic acid ethyl ester

A mixture of 430 mg of 4-[4-{4-amino-3-(N-t-butoxycarbonyl-N-methylamino)phenylthio}-2-(N-t-butoxycarbonyl-N-methylamino)phenyl]-2-propyl-1-[2'-(1H-tetrazol-5-yl)biphenyl-4-ylmethyl]-1H-benzoimidazole-5-carboxylic acid ethyl ester (the compound of Reference Example 59), 109 mg of 4-(2,4-dixothiazolidin-5-ylmethyl)phenoxyacetic acid, and 1.5 mL of dry dimethylformamide was combined with 74.3 mg of WSC (1-ethyl-3-(3-dimethylaminopropyl)carbodiimide hydrochloride) and stirred at room temperature for seventeen hours. The reaction mxture was combined with 70 mg more of 4-(2,4-dixothiazolidin-5-ylmethyl)phenoxyacetic acid and 45 mg more of WSC (1-ethyl-3-(3-dimethylaminopropyl)carbodiimide hydrochloride) and stirred at room temperature for five hours. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 0.57 g of the resulting residue was refined by silica gel column chromatography (elution solvent: n-hexane/ethyl acetate = 1/1 to 1/2) to give 0.48 g of the intended compound as a light yellow powder.
Rf value (relative difference) (silica gel thin layer chromatography, n-hexane/ethyl acetate = 2/3): 0.50.

Mass spectrum: 1422 (M+1)⁺.
¹H-NMR; spectra (500MHz, DMSO-d₆), *δ*ₚₚₘ:0. 7 6(t, 3H), 1.1-1. 5 (^{broad,} 18H), 1.1 6 (t, 3 H), 1.54(m, 2H), 2.47 (t, 2H), 2.85 (s, 2H ), 2. 97 (s, 3H), 3. 07 (q, 1H), 3. 33 (d, 1H) , 4.32(s, 2H), 4.71 (s, 2H), 4.87 (q, 1H), 5.52(s, 2H), 6.83-8.00 (m, 33H), 12.02 ( s, 1H).

### Reference Example 61

### 4-methanesulfonyloxymethyl-2-propyl-1-[2'-(1-triphenylmethyl-1H-tetrazol-5-yl)biphenyl-4-ylmethyl]-1H-imidazole-5-carboxylic acid ethyl ester and 4-chloromethyl-2-propyl-1-[2'-(1-triphenylmethyl-1H-tetrazol-5-yl)biphenyl-4-ylmethyl]-1H-imidazole-5-carboxylic acid ethyl ester

A solution of 47.5 g of 4-hydroxymethyl-2-propyl-1-[2'-(1-triphenylmethyl-1H-tetrazol-5-yl)biphenyl-4-ylmethyl]-1H-imidazole-5-carboxylic acid in 200 mL of dry methylene chloride was combined with 9.5 g of methanesulfonyl chloride, 9.8 g of N,N-diisopropyl ethylamine were dropped in while cooling with ice, and the reaction mixture was stirred at room temperature for twenty hours. The reaction mixture was condensed, and the residue was combined with ethyl acetate and water, and the organic layer was separated. The organic layer was washed with saturated physiological saline, then dried over anhydrous sodium sulfate. Distilling off the solvent gave a coarse product material of a 4-methanesulfonyloxymethyl form and a 4-chloromethyl form. (This coarse product material may be used in the next reaction without further refining.) This coarse product material was refined by silica gel column chromatography (elution solvent: n-hexane/ethyl acetate = 3/1 to 2/1, then 1/1, then 1/2) to give 2.6 g of the 4-methanesulfonyloxymethyl form and 35.45 g of the 4-chloromethyl form.
4-methanesulfonyloxymethyl form
Rf value (relative difference) (silica gel thin layer chromatography, n-hexane/ethyl acetate = 1/1): 0.33.

Mass spectrum: 767 (M+1)⁺.
¹H-NMR. spectra (400MHz. DMSO-d₆),δₚₚₘ: 0.7 7 (t, 3H), 1.19 (t, 3H), 1.56 (m, 2H), 2.47 (t, 2H), 4. 1 7 (q, 2H), 5.40 (q, 1H), 5.53 ( s, 2H), 6.83-7.7 9 (m, 23H).

4-chloromethyl form
Rf value (relative difference) (silica gel thin layer chromatography, n-hexane/ethyl acetate = 1/1): 0.79
Mass spectrum: 707 (M+1)⁺.
¹H-NMR; spectra (400MHz, DMSO-d₆) *δ*ₚₚₘ:0.7 5(t, 3H), 1.19 (t, 3H), 1.55 (m, 2H), 2.45 (t, 2H), 4.17 (q, 2H), 4.88 (q, 1H), 5. 51 ( s, 2H), 6. 84-7.79 (m, 23H).

### Reference Example 62

### 4-(2-hydroxyethylthiomethyl-2-propyl-1-[2'-(1-triphenylmethyl-1H-tetrazol-5-yl)biphenyl-4-ylmethyl]-1H-imidazole-5-carboxylic acid ethyl ester

A solution of 2.5 g of 4-chloromethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 61) in 10 mL of dry dimethylformamide was combined with 1 g of 2-mercaptoethanol, and the reaction system was purged with argon. The reaction system was then combined with 2 g of potassium carbonate and stirred at room temperature for three hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 2.99 g of the resulting residue was refined by silica gel column chromatography (elution solvent: n-hexane/ethyl acetate = 1/2) to give 2.03 g g of the intended compound as a colorless glassy solid.
Rf value (relative difference) (silica gel thin layer chromatography, n-hexane/ethyl acetate = 1/2): 0.52.

### Reference Example 63

### 4-[2-(4-hydroxyphenylthio)ethylthiomethyl]-2-propyl-1-[2'-(1-triphenylmethyl-1H-tetrazol-5-yl)biphenyl-4-ylmethyl]-1H-imidazole-5-carboxylic acid ethyl ester

### (a) 4-(2-methanesulfonyloxyethylthiomethyl)-2-propyl-1-[2'-(1-triphenylmethyl-1H-tetrazol-5-yl)biphenyl-4-ylmethyl]-1H-imidazole-5-carboxylic acid ethyl ester

A solution of 1.625 g of 4-(2-hydroxyethylthiomethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 62) in 10 mL of dry methylene chloride was combined with 0.273 g of methanesulfonyl chloride, a solution of 0.336 g of N,N-diisopropyl ethylamine in 2 mL of dry methylene chloride was dropped in while cooling with ice, and the reaction mixture was stirred at room temperature for six hours. The solvent was distilled off from the reaction mixture, and the residue was combined with ethyl acetate and physiological saline, and the ethyl acetate layer was separated. The ethyl acetate layer was washed with saturated physiological saline, then dried over anhydrous sodium sulfate. Distilling off the solvent gave 1.80 g of the intended compound.

### (b) 4-[2-(4-hydroxyphenylthio)ethylthiomethyl]-2-propyl-1-[2'-(1-triphenylmethyl-1H-tetrazol-5-yl)biphenyl-4-ylmethyl]-1H-imidazole-5-carboxylic acid ethyl ester

A weight of 1.78 g of the methanesulfonyloxy form (the compound of Reference Example 63(a)) was combined with 0.5 g of 4-mercaptophenol, 15 mL of dry dimethylformamide, and 0.6 g of anhydroux potassium carbonate and stirred at room temperature for four hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 2.48 g of the resulting residue was refined by silica gel column chromatography (elution solvent: n-hexane/ethyl acetate = 1/1) to give 1.71 g of the intended compound as a white powder.
Rf value (relative difference) (silica gel thin layer chromatography, n-hexane/ethyl acetate = 1/1): 0.48.

### Reference Example 64

### 4-[2-{4-(3-(N-t-butoxycarbonyl-N-methylamino)-4-nitrophenoxy)phenylthio}-2-propyl-1-[2'-(1-triphenylmethyl-1H-tetrazol-5-yl)biphenyl-4-ylmethyl]-1H-imidazole-5-carboxylic acid ethyl ester

A mixture of 1.7 g of 4-[2-(4-hydroxyphenylthio)ethylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 63), 1.2 g of 4-chloro-2-(N-t-butoxycarbonyl-N-methylamino)nitrobenzene, 1 g of potassium carbonate, and 8 mL of dry dimethylformamide was heated and stirred at an oil-bath temperature of 60°C for seventeen hours. The mixture was then combined with 1.2 g of 4-chloro-2-(N-t-butoxycarbonyl-N-methylamino)nitrobenzene and heated and stirred at an oil-bath temperature of 90°C for four hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 2/1) to give 1.33 g of the title compound as a light yellow powder.
Rf value (relative difference) (silica gel thin layer chromatography, n-hexane/ethyl acetate = 1/1): 0.75.

### Reference Example 65

### 4-[2-{4-(4-amino-3-(N-t-butoxycarbonyl-N-methylamino)phenoxy)phenylthio}ethylthiomethyl]-2-propyl-1-[2'-(1-triphenylmethyl-H-tetrazol-5-yl)biphenyl-4-ylmethyl]-1H-imidazole-5-carboxylic acid ethyl ester

A solution of 1.31 g of 4-[2-{4-(3-(N-t-butoxycarbonyl-N-methylamino)-4-nitrophenoxy)phenylthio}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 64) in 30 mL of methanol was purged with argon, then combined with 3.13 g of 10% palladium on carbon and purged with hydrogen gas at an oil-bath temperature of 50°C for five hours. Insoluble matter was filtered off from the reaction mixture, the filtrate was condensed, and the residue was washed with a washing solution mixture of a small volume of toluene and a large volume of n-hexane. Afterward, the residue was washed with n-hexane, then dissolved in a solvent mixture of toluene and a small volume of methanol, and filtered. The filtrate was condensed and dried to give 0.69 g of a coarse product material of the intended compound.

### Reference Example 66

### 4-[2-[4-{3-(N-t-butoxycarbonyl-N-methylamino)-4-[4-(2,4-dioxothiazolidin-5-ylmethyl)phenoxyacetylamino]phenoxy}phenylthio]ethylthiomethyl]-2-propyl-1-[2'-(1H-tetrazol-5-yl)biphenyl-4-ylmethyl]-1H-imidazole-5-carboxylic acid ethyl ester

A solution of 651 mg of 4-[2-{4-(4-amino-3-(N-t-butoxycarbonyl-N-methylamino)phenoxy)phenylthio}ethylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 65) in 3 mL of dry dimethylformamide was combined with 240 mg of 4-(2,4-dixothiazolidin-5-ylmethyl)phenoxyacetic acid and 165 mg of WSC (1-methyl-3-(3-dimethylaminopropyl)carbodiimide hydrochloride), and stirred at room temperature for five days. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 0.89 g of the resulting residue was refined by silica gel column chromatography (elution solvent: ethyl acetate/methanol =10/1) to give 0.67 g of the intended compound.
Rf value (relative difference) (silica gel thin layer chromatography, n-hexane/ethyl acetate = 1/5): 0.62.

¹H-NMR. spectra (500MHz, DMSO-d₆), *δ*ₚₚₘ:0.8 3 (t, 3H), 1.17 (t. 3H), 1.1-1.5 ( 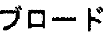 s, 9 H), 1.56 (m, 2H), 2. 54 (t, 2H), 2, 56 (t, 2H ), 3.11 (s, 3H), 3.13 (q, 1H), 3.14 (t, 2 H) , 3.33 (q, 1H), 3.92(s, 2H), 4.15 (q, 2H), 4.70(s, 2H), 4.89 (q, 1H), 5.52 (s, 2H), 6 . 74-7.89 (m, 19H).

### Reference Example 67

### 4-[2-(3-(N-to-butoxycarbonyl-N-methylamino)-4-nitrophenoxy)ethylthiomethyl]-2-propyl-1-[2'-(1-triphenylmethyl-1H-tetrazol-5-yl)biphenyl-4-ylmethyl]-1H-imidazole-5-carboxylic acid ethyl ester

A solution of 1.77 g of 4-(2-hydroxyethylthiomethyl-2-propyl-1-[2'-(1-triphenylmethyl-1H-tetrazol-5-yl)biphenyl-4-ylmethyl]-1H-imidazole-5-carboxylic acid ethyl ester in 5 mL of dry dimethylformamide was combined with 0.2 g of sodium hydride (purity: 60%) and stirred at room temperature for thirty minutes. The reaction solution was combined with 1.51 g of 4-chloro-2-(N-t-butoxycarbonyl-N-methylamino)nitrobenzene and stirred at room temperature for five hours, then heated and stirred at an oil-bath temperature of 50°C for twenty hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 1/1) to give 0.32 g of the intended compound as a yellow-orage powder.
Rf value (relative difference) (silica gel thin layer chromatography, n-hexane/ethyl acetate = 1/1):0.50.

### Reference Example 68

### 4-[2-(4-amino-3-(N-t-butoxycarbonyl-N-methylamino)phenoxy)ethylthiomethyl]-2-propyl-1-[2'-(1H-tetrazol-5-yl)biphenyl-4-ylmethyl]-1H-imidazole-5-carboxylic acid ethyl ester

A solution of 0.31 g of 4-[2-(3-(N-t-butoxycarbonyl-N-methylamino)-4-nitrophenoxy)ethylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 67) in 10 mL of methanol was purged with argon, then combined with 0.5 g of 10% palladium on carbon and purged with hydrogen gas at an oil-bath temperature of 60°C for six hours. Insoluble matter was filtered off from the reaction mixture, and the filtrate was condensed and dried to give 0.19 g of a light yellow glassy substance containing a coarse product material of the intended compound.

### Reference Example 69

### 4-[2-[3-(N-t-butoxycarbonyl-N-methylamino)-4-{4-(2,4-dioxothiazolidin-5-ylmethyl)phenoxyacetylamino}phenoxy]ethylthiomethyl]-2-propyl-1-[2'-(1H-tetrazol-5-yl)biphenyl-4-ylmethyl]imidazole-5-carboxylic acid ethyl ester

A solution of 0.181 g of 4-[2-(4-amino-3-(N-t-butoxycarbonyl-N-methylamino)phenoxy)ethylthiomethyl]-2-propyl-1-[2'-(1H-tetrazol-5-yl)biphenyl-4-ylmethyl]-1H-imidazole-5-carboxylic acid ethyl ester (the compound of Reference Example 68) in 1.5 mL of dry dimethylformamide was combined with 87 mg of 4-(2,4-dixothiazolidin-5-ylmethyl)phenoxyacetic acid and 59 mg of WSC (1-ethyl-3-(3-dimethylaminopropyl)carbodiimide hydrochloride), stirred at room temperature for eleven hours, then heated and stirred at an oil-bath temperature of 45°C for five hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate/methanol = 3/6/1) to give 0.138 g of the intended compound as a light yellow solid.
Rf value (relative difference) (silica gel thin layer chromatography, elution solvent: ethyl acetate/methanol = 3/1): 0.60.

### Reference Example 70

### 6-(imidazol-1-ylcarbonyl)-4-methyl-2-propyl-1-[2'-(1-triphenylmethyl-1H-tetrazol-5-yl)biphenyl-4-ylmethyl]-1H-benzimidazole

A weight of 0.69 g of 4-methyl-2-propyl-1-[2'-(1-triphenylmethyl-1H-tetrazol-5-yl)biphenyl-4-ylmethyl]-1H-benzimidazole-6-carboxylic acid and 0.17 g of 1,1'-carbonylbis-1H-imidazole was combined with 5 mL of dry dimethylformamide, and stirred at room temperature for 2.5 hours. The reaction solution was combined with 0.029 g more of 1,1-carbonylbis-1H-imidazole and stirred at room temperature for two hours. The reaction solution was combined with ethyl acetate and physiological saline, and the ethyl acetate layer was separated. The ethyl acetate layer was washed with saturated physiological saline, then dried over anhydrous sodium sulfate. After the solvent had been distilled off, 0.69 g of the resulting residue was refined by silica gel column chromatography (elution solvent: n-hexane/ethyl acetate = 1/2) to give 0.446 g of the intended compound as a white crystal.
Rf value (relative difference) (silica gel thin layer chromatography, n-hexane/ethyl acetate = 1/3): 0.61.

### Reference Example 71

### 5-(4-{6-[4-(4-methyl-2-propyl-1-[2'-(1-triphenylmethyl-1H-tetrazol-5-yl)biphenyl-4-ylmethyl]-1H-benzimidazol-6-ylcarbonylamino)phenoxy]-1-methyl-1H-benzimidazol-2-ylmethoxy}benzyl)thiazolidine-2,4-dione

A weight of 0.44 g of 6-(imidazol-1-ylcarbonyl)-4-methyl-2-propyl-1-[2'-(1-triphenylmethyl-1H-tetrazol-5-yl)biphenyl-4-ylmethyl]-1H-benzimidazole (the compound of Reference Example 70), 0.32 g of 5-[4-{6-(4-aminophenoxy)-1-methyl-1H-benzimidazol-2-ylmethosy}benzyl]thiazolidine-2,4-dione dihydrochloride, and 0.16 g of anhydrous sodium carbonate was combined with 3 mL of dry dimethylformamide, and stirred at room temperature for three hours.The reaction solution was combined with ethyl acetate and physiological saline, and the ethyl acetate layer was separated. The ethyl acetate layer was washed with 1) physiological saline, 2) water, and 3) saturated physiological saline in this order, then dried over anhydrous sodium sulfate. After the solvent had been distilled off, 0.69 g of the resulting residue was refined by silica gel column chromatography (elution solvent: n-hexane/ethyl acetate = 1/2 to 1/3, then 1/5, then 0.1) to give 0.44 g of the intended compound as a white solid.
Rf value (relative difference) (silica gel thin layer chromatography, n-hexane/ethyl acetate = 1/3): 0.37.

### Reference Example 72

### 3-(N-t-butoxycarbonyl-N-methylamino)-4-nitrophenylthioacetic acid methyl

A suspension of 2.0 g of sodium hydride (purity: 60%) in 70 mL of dry dimethylformamide was combined with 5.31 g of methyl mercaptoacetate while cooling with ice, and stirred for twenty minutes. The suspension was combined with 14.34 g of 4-chloro-2-(N-t-butoxycarbonyl-N-methylamino)nitrobenzene and stirred at room temperature for twenty minutes, then heated and stirred at an oil-bath temperature of 50-60°C for two hours. Afterward, the reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 3/1) to give 17.31 g of the intended compound as a light yellow crystal.

Rf value (relative difference) (silica gel thin layer chromatography, n-hexane/ethyl acetate = 3/1): 0.44.

### Reference Example 73

### 4-amino-3-(N-t-butoxycarbonyl-N-methylamino)phenylthioacetic acid methyl

A mixture of 6.66 g of 3-(N-t-butoxycarbonyl-N-methylamino)-4-nitrophenylthioacetic acid methyl (the compound of Reference Example 72) and 35 mL of methanol was purged with argon, then combined with 5 g of 10% palladium on carbon and purged with hydrogen gas at an oil-bath temperature of 50°C for twelve hours. After insoluble matter had been filtered out from the reaction mixture, the filtrate was condensed, then refined by silica gel column chromatography (elution solvent: n-hexane/ethyl acetate = 2/1) to give 5.5 g of the intended compound as a light yellow liquid.
Rf value (relative difference) (silica gel thin layer chromatography, n-hexane/ethyl acetate = 1/1):0.60.

### Reference Example 74

### 3-(N-t-butoxycarbonyl-N-methylamino)-4-[4-(2,4-dioxothiazolidin-5-ylmethyl)phenoxyacetaylamino]phenylthioacetic acid methyl

A mixture of 5.44 g of 4-amino-3-(N-t-butoxycarbonyl-N-methylamino)phenylthioacetic acid methyl (the compound of Reference Example 73), 4.685 g of 4-(2,4-dixothiazolidin-5-ylmethyl)phenoxyacetic acid, and 15 mL of dry dimethylformamide was combined with 3.193 g of WSC (1-ethyl-3-(3-dimethylaminopropyl)carbodiimide hydrochloride) and stirred at room temperature for 27 hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 10.7 g of the resulting residue was refined by silica gel column chromatography (elution solvent: n-hexane/ethyl acetate = 1/1) to give 7.53 g of the intended compound as a light yellow solid.
Rf value (relative difference) (silica gel thin layer chromatography, n-hexane/ethyl acetate = 1/1): 0.37.

### Reference Example 75

### 1-methyl-2-[4-(2,4-dioxothiazolidin-5-ylmethyl)phenoxymethyl]-1H-benzimidazol-6-ylthioacetatic acid methyl

A solution of 5.25 g of 3-(N-t-butoxycarbonyl-N-methylamino)-4-[4-(2,4-dioxothiazolidin-5-ylmethyl)phenoxyacetaylamino]phenylthioacetic acid methyl (the compound of Reference Example 74) in 40 mL of a 4 N hydrogen chloride-dioxane solution was heated and stirred at an oil-bath temperature of 70°C for 23 hours. A precipitated white powder was collected by filtration. Of this precipitate, 4.1 g was combined with 30 mL of methanol and 50 mL of a 4 N hydrogen chloride-dioxane solution, and heated and stirred at an oil-bath temperature of 55°C for four hours. After the solvent had been distilled off, the residue was dissolved in a small volume of methanol and poured into a large volume of ethyl acetate. A precipitated white solid was collected by filtration to give 3.97 g of a hydrochloride of the intended compound.

### Reference Example 76

### 1-methyl-2-[4-(2,4-dioxothiazolidin-5-ylmethyl)phenoxymethyl]-1H-benzimidazol-6-ylthioacetatic acid

A mixture of 2.13 g of 1-methyl-2-[4-(2,4-dioxothiazolidin-5-ylmethyl)phenoxymethyl]-1H-benzimidazol-6-ylthioacetatic acid methyl (the compound of Reference Example 75), 30 mL of acetic acid, and 15 mL of concentrated hydrochloric acid was heated and stirred at an oil-bath temperature of 90°C for 21 hours. A precipitated white powder was collected by filtration and dried to give 1.80 g of the intended compound as a white powder.

### Reference Example 77

### 4-[1-methyl-2-{4-(2,4-dioxothiazolidin-5-ylmethyl)phenoxymethyl}-1H-benzimidazol-6-ylthioacetylamino]-2-propyl-1-[2'-(1-triphenylmethyl-1H-tetrazol-5-yl)biphenyl-4-ylmethyl]-1H-imidazole-5-carboxylic acid ethyl ester

A mixture of 674 mg of 1-methyl-2-[4-(2,4-dioxothiazolidin-5-ylmethyl)phenoxymethyl]-1H-benzimidazol-6-ylthioacetatic acid (the compound of Reference Example 76), 458 g of 4-amino-2-propyl-1-[2'-(1-triphenylmethyl-1H-tetrazol-5-yl)biphenyl-4-ylmethyl]-1H-imidazole-5-carboxylic acid ethyl ester, and 3 mL of dry dimethylformamide was combined with 192 mg of WSC (1-ethyl-3-(3-dimethylaminopropyl)carbodiimide hydrochloride), and stirred at 45°C for five hours, then at 55°C for two hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 10/1) to give 0.593 g of the intended compound as a colorless glassy solid.

¹H-NMR spectra (500MHz, DMSO-d₆), *δ*_{ppm:}0.7 5 (t, 3H), 1.05 (t, 3H), 1.52 (m, 2H), 2.41 (t, 2H), 3.06 (q, 1H), 3.31 (d, 1H), 3.81 ( s, 3H), 3.83 (s, 2H), 4.00 (t, 2H), 4.87 (q , 1H), 5.36 (s,2H), 5.38 (s, 1H), 5. 44 (s, 2H), 6.86-7.79 (m, 19H).

### Reference Example 78

### 4-(2-aminoethylthiomethyl)-2-propyl-1-[2'-(1-triphenylmethyl-1H-tetrazol-5-yl)biphenyl-4-ylmethyl]-1H-imidazole-5-carboxylic acid ethyl ester

A solution of 2.5 g of 4-chloromethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 61) in 10 mL of dry dimethylformamide was combined with 1 g of 2-aminoethanethiol and stirred at the same temperature for three hours. The reaction mixture was then combined with 0.56 g of potassium carbonate and stirred at room temperature for fifteen hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 3.25 g of the resulting residue was refined by silica gel column chromatography (NH silica/elution solvent: ethyl acetate) to give 2.2 g of the intended compound as a colorless glassy solid.
Rf value (relative difference) (silica gel thin layer chromatography, NH silica, ethyl acetate/methanol = 10/1): 0.58.

### Reference Example 79

### 4-[2-[1-methyl-2-{4-(2,4-dioxothiazolidin-5-ylmethyl)phenoxymethyl}-1H-benzimidazol-6-ylthioacetylamino]ethylthiomethyl]-2-propyl-1-[2'-(1-triphenylmethyl-1H-tetrazol-5-yl)biphenyl-4-ylmethyl]-1H-imidazole-5-carboxylic acid ethyl ester

A mixture of 883 mg of 4-(2-aminoethyl)thiomethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 78), 540 mg of 1-methyl-2-[4-(2,4-dioxothiazolidin-5-ylmethyl)phenoxymethyl]-1H-benzimidazol-6-ylthioacetatic acid (the compound of Reference Example 76), and 3 mL of dry dimethylformamide was combined with 226 mg of WSC (1-ethyl-3-(3-dimethylaminopropyl)carbodiimide hydrochloride), and stirred at room temperature for fourteen hours. The reaction mixture was combined with ethyl acetate and a sodium hydroxide aqueous solution, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 15/1) to give 929 mg of the intended compound as a white solid.
Rf value (relative difference) (silica gel thin layer chromatography, elution solvent: ethyl acetate/methanol = 9/1): 0.71.

¹H-NMR spectra (500MHz. DMSO-d₆), *δ*ₚₚₘ:0.7 8 (t, 3H), 1.18 (t, 3H), 1.52 (m, 2H), 2.41 (t, 2H), 2.59 (t, 2H), 3.04 (q, 1H), 3.29( d, 1H), 3.30 (t, 2H), 3, 65 (s, 2H), 3.81 (s , 3H), 3.90 (s, 2H), 4.09 (q, 2H), 4.87 (q, 1H), 5.36 (s, 2H), 5.48 (s, 2H), 6.82-7.7 6 (m, 30H), 8.25 (s, 1H), 12.02 (s, 1H).

### Reference Example 80

### 2-(N-t-butoxycarbonyl-N-methylamino)-4-(N-2-hydroxyethyl-N-methylamino)aniline

A mixture of 42.8 g of 2-(N-t-butoxycarbonyl-N-methylamino)-4-(N-2-hydroxyethyl-N-methylamino)nitrobenzene (the compound of Reference Example 10) and 300 mL of methanol was purged with argon, then combined with 15 g of 10% palladium on carbon and purged with hydrogen gas at room temperature for three hours. After insoluble matter had been filtered off from the reaction mixture, the filtrate was condensed and the residue was refined by silica gel column chromatography (elution solvent: n-hexane/ethyl acetate = 1/2) to give 40.1 g of the intended compound as a colorless liquid.

### Reference Example 81

### N-[2-(N-t-butoxycarbonyl-N-methylamino)-4-(N-2-hydroxyethyl-N-methylamino)phenyl]-4-methyl-2-propyl-1H-benzimidazole-6-carboxamide

A mixture of 4.94 g of 2-(N-t-butoxycarbonyl-N-methylamino)-4-(N-2-hydroxyethyl-N-methylamino)aniline (the compound of Reference Example 80), 3.65 g of 4-methyl-2-propyl-1H-benzimidazole-6-carboxylic acid, and 20 mL of dry dimethylformamide was combined with 3.21 g of 1-ethyl-3-(3-dimethylaminopropyl)carbodiimide hydrochloride (WSC) and stirred at room temperature overnight, then heated and stirred at an oil-bath temperature of 50-60°C for three hours. The reaction mixture was combined with ethyl acetate and physiological saline, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 20/1) to give 5.3 g of the intended compound as a light yellow solid.
Rf value (relative difference) (silica gel thin layer chromatography, elution solvent: ethyl acetate/methanol = 19/1): 0.49.

### Reference Example 82

### 6-[6-(N-2-hydroxyethyl-N-methylamino)-1-methyl-1H-benzimidazol-2-yl]-4-methyl-2-propyl-1H-benzimidazole

A solution of 5.3 g of N-[2-(N-t-butoxycarbonyl-N-methylamino)-4-(N-2-hydroxyethyl-N-methylamino)phenyl]-4-methyl-2-propyl-1H-benzimidazole-6-carboxamide (the compound of Reference Example 81) in 60 mL of a 4 N hydrogen chloride-dioxane solution was heated and stirred at an oil-bath temperature of 80°C to 90°C for thirteen hours. After the solvent had been distilled off under reduced pressure, the residue was combined with methanol and ammonia water. The residue resulting from distilling off the solvent was refined by silica gel column chromatography (elution solvent: ethyl acetate/methanol = 5/1) to give 4.41 g of the intended compound as a white solid.
Rf value (relative difference) (silica gel thin layer chromatography, elution solvent: ethyl acetate/methanol = 9/1): 0.28 (tailing from the base point).

### Reference Example 83

### 4'-[6-{6-(N-2-hydroxyethyl-N-methylamino)-1-methyl-1H-benzimidazol-2-yl}-4-methyl-2-propyl-1H-benzimidazol-1-ylmethyl]biphenyl-2-carboxylic acid t-butyl ester

A mixture of 4.41 g of 6-[6-(N-2-hydroxyethyl-N-methylamino)-1-methyl-1H-benzimidazol-2-yl]-4-methyl-2-propyl-1H-benzimidazole (the compound of Reference Example 82) and 0.47 g of 60% sodium hydride was combined with 20 mL of dry tetrahydrofuran and 30 mL of dry dimethylformamide and stirred at room temperature for one hour. The reaction mixture was combined with 4.90 g of 4'-bromomethylbiphenyl-2-carboxylic acid t-butyl ester while cooling with ice, and stirred at room temperature for five hours. The reaction mixture was combined with 4 g of anhydrous potassium carbonate and 2 g of 4'-bromomethylbiphenyl-2-carboxylic acid t-butyl ester, stirred at room temperature for twenty hours, then combined with 0.14 g of 60% sodium hydride and stirred at room temperature for ten hours. The residue resulting from distilling off the solvent was combined with ethyl acetate and water, and the ethyl acetate layer was separated. The ethyl acetate layer was washed with saturated physiological saline, then dried over anhydrous sodium sulfate. After the solvent had been distilled off, 8.46 g of the resulting residue was refined by silica gel column chromatography (elution solvent: n-hexane/ethyl acetate = 1/3) to give 0.54 g of the intended compound as a white solid.
Rf value (relative difference) (silica gel thin layer chromatography, elution solvent: ethyl acetate/methanol = 20/1): 0.36.

### Reference Example 84

### 4'-[6-{6-(N-2-[4-(2,4-dioxo-3-triphenylmethylthiazolidin-5-ylmethyl)phenoxy]ethyl-N-methylamino)-1-methyl-1H-benzimidazol-2-yl}-4-methyl-2-propyl-1H-benzimidazol-1-ylmethyl]biphenyl-2-carboxylic acid t-butyl ester

A weight of 0.54 g of 4'-[6-{6-(N-2-hydroxyethyl-N-methylamino)-1-methyl-1H-benzimidazol-2-yl}-4-methyl-2-propyl-1H-benzimidazol-1-ylmethyl]biphenyl-2-carboxylic acid t-butyl ester (the compound of Reference Example 83) and 0.39 g of 5-(4-hydroxybenzyl)-3-triphenylmethylthiazolidine-2,4-dione was dried and combined with 0.23 g of triphenylphosphine and 7 mL of toluene, then with 0.4 g of diethyl azodicarboxylate (40% toluene solution) and stirred at room temperature for two days. The reaction mixture was refined by silica gel column chromatography (elution solvent: n-hexane/ethyl acetate = 1/3 to 1/4, then 1/5) to give 0.45 g of a coarse product material, which was refined by silica gel column chromatography (NH silica, elution solvent: methylene chloride) to give 0.22 g of the intended compound as a light yellow solid.
Rf value (relative difference) (silica gel thin layer chromatography, n-hexane/ethyl acetate = 1/5): 0.49.

### Reference Example 85

### 4- {4-[4-[4-(2,4-dioxothiazolidin-5-ylmethyl)phenoxyacetylamino]-3-(N-t-butoxycarbonyl-N-methylamino)phenoxy]phenylsufinylmethyl}-2-propyl-1-[2'-(1-triphenylmethyl-1H-tetrazol-5-yl)biphenyl-4-ylmethyl]-1H-imidazole-5-carboxylic acid ethyl ester

A mixture of 2.6 g of 4-{4-[4-[4-(2,4-dioxothiazolidin-5-ylmethyl)phenoxyacetylamino]-3-(N-t-butoxycarbonyl-N-methylamino)phenoxy]phenylthiomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 40-1) and 20 mL of methylene chloride was combined with 0.41 g of m-chlorobenzoic acid (purity: 65% or greater) and stirred at room temperature for two hours. The residue resulting from distilling off the solvent was refined by silica gel column chromatography (elution solvent: n-hexane/ethyl acetate = 1/2 to 1/4, then 0/1) to give 2.26 g of the intended compound as a white solid.
Rf value (relative difference) (silica gel thin layer chromatography, n-hexane/ethyl acetate = 1/2): 0.27.

¹H-NMR spectra (500MHz, DMSO-d₆), *δ*ₚₚₘ:0.7 3 (t, 3H), 1.18 (t, 3H), 1.15-1.45 ( 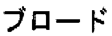 s , 9H), 1.49 (m, 2H), 2. 38 (t, 2H), 2.99 (s, 3H), 3.09 (d, 1H), 3.33 (q, 1H), 4.04 (t, 2 H), 4, 33(d, 1H), 4.53(d, 1H), 4. 71 (s, 2H ), 4.86 (q, 1H), 5.43(q, 2H), 6.77-7.76 ( m, 16H), 12. 0 1 (s, 1H).

### Reference Example 86

### 4-(4-hydroxymethylphenoxy)-2-(N-t-butoxycarbonyl-N-methylamino)nitrobenzene

A solution of 7.0 g of 4-hydroxymethylphenol in 50 mL of dry dimethylformamide was combined with 2.0 g of sodium hydride (purity: 60%) while cooling with ice, stirred at room temperature for thirty minutes, then combined with 14.34 g of 4-chloro-2-(N-t-butoxycarbonyl-N-methylamino)nitrobenzene and stirred and heated to 60°C for twenty hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 23 g of the resulting residue was refined by silica gel column chromatography (elution solvent: n-hexane/ethyl acetate = 2/1) to give 12.5 g of the intended compound as a light yellow solid.
Rf value (relative difference) (silica gel thin layer chromatography, n-hexane/ethyl acetate=3/1): 0.23.

### Reference Example 87

### 4-(4-N-phthaloylaminomethylphenoxy)-2-(N-t-butoxycarbonyl-N-methylamino)nitrobenzene

A solution of 8.51 g of 4-(4-hydroxymethylphenoxy)-2-(N-t-butoxycarbonyl-N-methylamino)nitrobenzene (the compound of Reference Example 86) in 45 mL of dry tetrahydrofuran was combined with 2.66 g of methanesulfonyl chloride, and 3.01 g of N,N-diisobutyl ethylamine were dropped in while cooling with ice. This mixture was stirred at room temperature for four hours, then combined with 4.5 g of potassium phthalimide and stirred at room temperature for two days. The residue resulting from distilling off the solvent was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 3/1) to give 3.8 g of the intended compound as a yellow powder.
Rf value (relative difference) (silica gel thin layer chromatography, n-hexane/ethyl acetate = 2/1): 0.64.

### Reference Example 88

### 4-(4-aminomethylphenoxy)-2-(N-t-butoxycarbonyl-N-methylamino)nitrobenzene

A mixture of 5.3 g of 4-(4-N-phthaloylaminomethylphenoxy)-2-(N-t-butoxycarbonyl-N-methylamino)nitrobenzene (the compound of Reference Example 87), 50 mL of 97% ethanol, and 10 mL of tetrahydrofuran was combined with 1.58 g of hydrazine hydrate and heated and stirred at 60°C for three hours. The reaction mixture was combined with 95% ethanol, then insoluble matter was filtered off, and the filtrate was condensed and combined with toluene and anhydrous sodium sulfate. Insoluble matter was filtered off, and the filtrate was condensed to give 4.32 g of the intended compound as a yellow oily matter.

### Reference Example 89

### N-[4-(3-(N-t-butoxycarbonyl-N-methylamino)-4-nitrophenoxy)benzyl]-4-methyl-2-propyl-1-[2'-(1H-tetrazol-5-yl)biphenyl-4-ylmethyl]-1H-benzimidazole-6-carboxamide

A mixture of 1.33 g of 4-methyl-2-propyl-1-[2'-(1-triphenylmethyl-1H-tetrazol-5-yl)biphenyl-4-ylmethyl]-1H-benzimidazole-6-carboxylic acid, 715 mg of 4-(4-aminomethylphenoxy)-2-(N-t-butoxycarbonyl-N-methylamino)nitrobenzene (the compound of Reference Example 88), and 15 mL of dry tetrahydrofuran was combined with 320 mg of diethyl cyanophosphonate, then combined with 500 mg of triethylamine and stirred at room temperature for five days. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 5/1 to 3/1) to give 0.599 g of the intended compound as a light yellow powder.

Mass spectrum: 808 (M+1)⁺.

### Reference Example 90

### N-[4-(4-amino-3-(N-t-butoxycarbonyl-N-methylamino)phenoxy)benzyl]-4-methyl-2-propyl-1-[2'-(1H-tetrazol-5-yl)biphenyl-4-ylmethyl]-1H-benzimidazole-6-carboxamide

Using 0.58 g of N-[4-(3-(N-t-butoxycarbonyl-N-methylamino)-4-nitrophenoxy)benzyl]-4-methyl-2-propyl-1-[2'-(H-tetrazol-5-yl)biphenyl-4-ylmethyl]-1H-benzimidazole-6-carboxamide (the compound of Reference Example 89), 0.4 g of 10% palladium on carbon, and 3 mL of methanol, these were reacted following Reference Example 80, and refined by silica gel column chromatography (elution solvent: ethyl acetate/methanol = 10/1) to give 0.353 g of the intended compound as a light brown solid.

### Reference Example 91

### 5-[4-{2-(N-t-butoxycarbonyl-N-methylamino)-4-[4-(4-methyl-2-propyl-1-[2'-(1H-tetrazol-5-yl)biphenyl-4-ylmethyl]-1H-benzimidazol-6-ylcarbonylaminomethyl)phenoxy]phenylaminocarbonylmethoxy}benzyl]thiazolidin-2,4-dione

A weight of 0.35 g of N-[4-(4-amino-3-(N-t-butoxycarbonyl-N-methylamino)phenoxy)benzyl]-4-methyl-2-propyl-1-[2'-(1H-tetrazol-5-yl)biphenyl-4-ylmethyl]-1H-benzimidazole-6-carboxamide (the compound of Reference Example 90) was combined with 1 mL of dry dimethylformamide, 127 mg of 4-(2,4-dixothiazolidin-5-ylmethyl)phenoxyacetic acid, and 87 mg of WSC (1-ethyl-3-(3-dimethylaminopropyl)carbodiimide hydrochloride) and stirred at room temperature for four days. The reaction solution was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 10/1) to give 0.17 g of the intended compound as a light brown powder.

### Reference Example 92

### 4-(4-hydroxyphenylthio)-2-(N-t-butoxycarbonyl-N-methylamino)nitrobenzene

A solution of 15 g of 4-mercaptophenol, 14 g of 4-chloro-2-(N-t-butoxycarbonyl-N-methylamino)nitrobenzene, and 50 mL of dry dimethylformamide was combined with 20 g of anhydrous potassium carbonate and stirred at room temperature for four hours. The reaction mixture was then combined with ethyl acetate and water, followed by 1 N hydrochloric acid to bring to pH 7. The ethyl acetate layer was separated, and the ethyl acetate layer was washed with saturated physiological saline, then dried over anhydrous sodium sulfate. The residue resulting from distilling off the solvent was refined by silica gel column chromatography (elution solvent: n-hexane/ethyl acetate = 3/1) to give 16 g of the intended compound as a light yellow solid.
Rf value (relative difference) (silica gel thin layer chromatography, n-hexane/ethyl acetate = 3/1): 0.27.

Mass spectrum: 376 (M)⁺.
¹H-NMR spectra (500MHz, DMSO-d₆), *δ*ₚₚₘ:1.2 1&1.40(d, 9H), 3.13(s, 3H), 6.86(d, 1H) , 6.9 3 (d, 2H), 7.23(s, 1H), 7.44 (d, 2H, 7.88(d, 1H), 10.12 (s, 1H).

### Reference Example 93

### 4-[4- {3-(N-t-butoxycarbonyl-N-methylamino)-4-nitrophenylthio}phenoxymethyl]-2-propyl-1-[2'-(1-triphenylmethyl-1H-tetrazol-5-yl)biphenyl-4-ylmethyl]-1H-imidazole-5-carboxylic acid ethyl ester

A suspension of 40 mg of 60% sodium hydride and 2 mL of dry dimethylformamide was combined with 377 mg of 4-(4-hydroxyphenylthio)-2-(N-t-butoxycarbonyl-N-methylamino)nitrobenzene (the compound of Reference Example 92) and stirred at room temperature for twenty minutes. This mixture was combined with 767 mg of 4-chloromethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 61) and heated and stirred at 50°C for two days. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 2/1) to give 0.77 g of the intended compound as a light yellow crystal.

Mass spectrum: 1047 (M+1)⁺.
¹H-NMR spectra (500MHz, DMSO-d₆), δₚₚₘ:0.7 7 (t, 3H), 1.01 (t, 3H), 1.20 & 1.39 (d, 9H) , 1.57 (q, 2H), 2.47 (t, 2H), 3. 13 (s, 3H), 4.09 (q, 2H), 5.26 (s, 2H), 5.54 (s, 2H) 6 . 87-7. 87 (m, 30H).

### Reference Example 94

### 4-[4-{4-amino-3-(N-t-butoxycarbonyl-N-methylamino)phenylthio}phenoxymethyl]-2-propyl-1-[2'-(1H-tetrazol-5-yl)biphenyl-4-ylmethyl]-1H-imidazole-5-carboxylic acid ethyl ester

Using 0.73 g of 4-[4-{3-(N-t-butoxycarbonyl-N-methylamino)-4-nitrophenylthio}phenoxymethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 93), 1.0 g of 10% palladium on carbon, and 20 mL of methanol, these were reacted following Reference Example 80, and refined by silica gel column chromatography (elution solvent: ethyl acetate/methanol = 10/1) to give 0.38 g of the intended compound as a light yellow powder.

Mass spectrum: 775 (M+1)⁺.
¹H-NMR spectra (500MHz, DMSO-d₆), δₚₚₘ:0.8 5(t, 3H), 1.04(t, 3H), 1.25- broad d 9)H), 1.59 (q, 2H), 2.60 (t, 2H), 2.96 (s, 3H), 4.12(q, 2H), 5.10(s. 2H), 5.55(s, 2 H), 6.70-7.67(m, 30H).

### Reference Example 95

### 4-{4-[4-[4-(2,4-dixothiazolidin-5-ylmethyl)phenoxyacetylamino]-3-(N-t-butoxycarbonyl-N-methylamino)phenylthio]phenoxymethyl}-2-propyl-1-[2'-(1H-tetrazol-5-yl)biphenyl-4-ylmethyl]-1H-imidazole-5-carboxylic acid ethyl ester

A solution of 0.367 g of 4-[4-{4-amino-3-(N-t-butoxycarbonyl-N-methylamino)phenylthio}phenoxymethyl]-2-propyl-1-[2'-(1H-tetrazol-5-yl)biphenyl-4-ylmethyl]-1H-imidazole-5-carboxylic acid ethyl ester (the compound of Reference Example 94) and 0.28 g of 4-(2,4-dixothiazolidin-5-ylmethyl)phenoxyacetic acid in 2 mL of dry dimethylformamide was combined with 0.19 g of WSC (1-ethyl-3-(3-dimethylaminopropyl)carbodiimide hydrochloride) and stirred at room temperature for two days. The reaction mixture was combined with ethyl acetate and physiological saline, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 10/1 to 5/1) to give 0.545 g of the intended compound.

Mass spectrum: 1038 (M+1)⁺.
¹H-NMR, spectra (500MHz, DMSO-d₆), δppm:0.8 6(t, 3H), 1.03(t, 3H), 1.1-1.5(broad d, 9 H), 1.60(m, 2H), 2.61 (t, 2H), 2.96(s, 3H ), 3.0 7 (q, 1H), 3. 3 0 (1H, overlapping H₂O), 4.13 ( q, 1H), 4.64(s, 1H), 4.70(s, 2H), 4.89(q 1H), 5.17(s, 2H), 5.57(s. 2H), 6.84-7. 6 7 (m, 1 9 H).

### Reference Example 96

### 2-propyl-1-[2'-(1-triphenylmethyl-1H-tetrazol-5-yl)biphenyl-4-ylmethyl]-1H-imidazole-4,5-dicarboxylic acid dimethyl ester

A volume of 20 mL (2.0 M) of trimethylsilyldiazomethane was dropped into a solution of 13.5 g of 2-propyl-1-[2'-(1-triphenylmethyl-1H-tetrazol-5-yl)biphenyl-4-ylmethyl]-1H-imidazole-4,5-dicarboxylic acid in 50 mL of tetrahydrofuran and 30 mL of methanol while cooling with ice, stirred at the same temperature for one hour, then stirred at room temperature for three hours. The residue resulting from distilling off the solvent was refined by silica gel column chromatography (elution solvent: n-hexane/ethyl acetate = 1/1) and recrystallized from methanol to give 2.24 g of the intended compound as a white solid.
Rf value (relative difference) (silica gel thin layer chromatography, n-hexane/ethyl acetate = 1/1): 0.29.

### Reference Example 97

### 4-hydroxymethyl-2-propyl-1-[2'-(1-triphenylmethyl-1H-tetrazol-5-yl)biphenyl-4-ylmethyl]-1H-imidazole-5-carboxylic acid methyl ester

A volume of 9.6 mL diisobutylaluminum hydride (1 M toluene solution) was dropped into a solution of 3.19 g of 2-propyl-1-[2'-(1-triphenylmethyl-1H-tetrazol-5-yl)biphenyl-4-ylmethyl]-1H-imidazole-4,5-dicarboxylic acid dimethyl ester (the compound of Reference Example 96) in 30 mL of dry tetrahydrofuran while cooling using a dry ice-acetone bath, stirred at the same temperature for thirty minutes, then stirred at room temperature for twenty hours. The reaction mixture was combined with ethyl acetate and water, insoluble matter was filtered off using Celite, and the ethyl acetate layer was separated from the filtrate. After the solvent had been distilled off from the ethyl acetate layer, 3.26 g of the resulting residue was refined by silica gel column chromatography (elution solvent: ethyl acetate/methanol = 1/0 to 20/1) to give 3.04 g of the intended compound as a white powder.

Mass spectrum: 675 (M+1)⁺.
¹H-NMR spectra (500MHz, DMSO-d₆), δₚₚₘ:0.7 7 (t, 3H), 1.56(m, 2H), 2.44(t, 2H), 3.66 (s, 3H), 4.59(d, 2H), 4.80(t, 1H), 5.49( s, 2H), 6.84-7.78(m, 15H).

### Reference Example 98

### 4-(4-hydroxyphenylthiomethyl)-2-propyl-1-[2'-(1-triphenylmethyl-1H-tetrazol-5-yl)biphenyl-4-ylmethyl]-1H-imidazole-5-carboxylic acid methyl ester

A solution of 3.0 g of 4-hydroxymethyl-2-propyl-1-[2'-(1-triphenylmethyl-1H-tetrazol-5-yl)biphenyl-4-ylmethyl]-1H-imidazole-5-carboxylic acid methyl ester (the compound of Reference Example 97) in 15 mL of dry methylene chloride was combined with 0.54 g of methanesulfonyl chloride at room temperature, then combined with 0.9 g of N,N-diisopropyl ethylamine while cooling with ice, and stirred at room temperature for three days. Next, the reaction mixture was combined with 0.9 g of 4-mercaptophenol, then combined with 1.1 g of anhydrous potassium carbonate and stirred at room temperature for five hours. The reaction mixture was combined with 20 mL of dry dimethylformamide, the methylene chloride was distilled off under reduced pressure, and the residue was stirred at room temperature for fifteen hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 4.09 g of the resulting residue was recrystallized from a solvent mixture of ethyl acetate and methanol to give 2.58 g of the intended compound as a white powder.
Rf value (relative difference) (silica gel thin layer chromatography, n-hexane/ethyl acetate = 1/1):0.39.

### Reference Example 99

### 4-[4-{3-(N-t-butoxycarbonyl-N-methylamino)-4-nitrophenoxy}pehnylthiomethyl]-2-propyl-1-[2'-(1-triphenylmethyl-1H-tetrazol-5-yl)biphenyl-4-ylmethyl]-1H-imidazole-5-carboxylic acid methyl ester

A solution of 0.52 g of 4-(4-hydroxyphenylthiomethyl)-2-propyl-1-[2'-(1-triphenylmethyl-1H-tetrazol-5-yl)biphenyl-4-ylmethyl]-1H-imidazole-5-carboxylic acid methyl ester (the compound of Reference Example 98) in 3 mL of dry dimethylformamide was combined with 42 mg of sodium hydride (purity: 60%) and stirred at room temperature for twenty minutes. This solution was combined with 0.5 g of 4-chloro-2-(N-t-butoxycarbonyl-N-methylamino)nitrobenzene and heated and stirred at an oil-bath temperature of 70°C for seventeen hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 1.04 g of the resulting residue was refined by silica gel column chromatography (elution solvent: n-hexane/ethyl acetate = 2/1) to give 0.219 g of the intended compound as a light yellow solid.
Rf value (relative difference) (silica gel thin layer chromatography, n-hexane/ethyl acetate = 1/1): 0.60.

Mass spectrum: 1033 (M+1)⁺.
¹H-NMR spectra (500MHz. DMSO-d₆), δₚₚₘ:0.7 4(t, 3H), 1.20 & 1.44( 2 broad peaks, 9H), 1.5 4 (m, 2H), 2.41 (t, 2H), 3.15(s, 3H), 3. 59 (s, 3H), 4.35(s, 2H), 5. 44 (s, 2H), 6.77-7. 93 (m, 30H).

### Reference Example 100

### 4-[4-{4-amino-3-(N-t-butoxycarbonyl-N-methylamino)phenoxy}phenylthiomethyl]-2-propyl-1-[2'-(1H-tetrazol-5-yl)biphenyl-4-ylmethyl]-1H-imidazole-5-carboxylic acid methyl ester

A mixture of 0.20 g of 4-[4-{3-(N-t-butoxycarbonyl-N-methylamino)-4-nitrophenoxy}pehnylthiomethyl]-2-propyl-1-[2'-(1-triphenylmethyl-1H-tetrazol-5-yl)biphenyl-4-ylmethyl]-1H-imidazole-5-carboxylic acid methyl ester (the compound of Reference Example 99) and 4 mL of methanol was purged with argon, then combined with 0.3 g of 10% palladium on carbon and purged with hydrogen gas at 50°C for fifteen hours. Insoluble matter was filtered off from the reaction mixture, the filtreate was condensed, and 180 mg of the resulting residue was refined by silica gel column chromatography (elution solvent: n-hexane/ethyl acetate = 1/3 to ethyl acetate/methanol = 5/1) to give 92 mg of the intended compound as a white solid.

### Reference Example 101

### 4-[4-{4-[4-(2,4-dixothiazolidin-5-ylmethyl)phenoxyacetylamino]-3-(N-t-butoxycarbonyl-N-methylamino)phenoxy]phenylthiomethyl}-2-propyl-1-[2'-(1H-tetrazol-5-yl)biphenyl-4-ylmethyl]-1H-imidazole-5-carboxylic acid methyl ester

A mixture of 85 mg of 4-[4-{4-amino-3-(N-t-butoxycarbonyl-N-methylamino)phenoxy}phenylthiomethyl]-2-propyl-1- [2'-(1H-tetrazol-5-yl)biphenyl-4-ylmethyl]-1H-imidazole-5-carboxylic acid methyl ester (the compound of Reference Example 100), 34 mg of 4-(2,4-dixothiazolidin-5-ylmethyl)phenoxyacetic acid, and 0.8 mL of dry dimethylformamide was combined with 24 mg of WSC (1-methyl-3-(3-dimethylaminopropyl)carbodiimide hydrochloride) and stirred at room temperature for one week. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 10/1) to give 95 mg of the intended compound as a light red glassy solid.

Mass spectrum: 1024 (M+1)⁺.
¹H-NMR spectra (500MHz, DMSO-d₆), δₚₚₘ:0.8 3(t, 3H), 1.1-1.5( broad peak, 9H), 1.5 6 (m 2H), 2. 56 (t, 2H), 2.99 (s, 3H), 3.09(q, 1H), 3.63(s, 3H), 4.27(s, 2H), 4.70(s, H), 4.89(q, 1H), 5. 4 8 (s, 2H), 6.86-7.64 (m, 19H).

### Reference Example 102

### 4-(4-t-butoxycarbonylaminophenoxy)-2-(N-t-butoxycarbonyl-N-methylamino)nitrobenzene

A suspension of 4.6 g of 60% sodium hydride, 3 mL of dry tetrahydrofuran, and 30 mL of dry dimethylformamide was combined with 4.6 g of 4-t-butoxycarbonylaminophenol while coolind with ice, and stirred at room temperature for twenty minutes. This mixture was combined with 6.0 g of 4-chloro-2-(N-t-butoxycarbonyl-N-methylamino)nitrobenzene and heated and stirred at 90°C for four hours. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 11.8 g of the resulting residue was refined by silica gel column chromatography (elution solvent: n-hexane/ethyl acetate = 3/1) and recrystallized from n-hexane to give 7.58 g of the intended compound as a light yellow crystal.
Rf value (relative difference) (silica gel thin layer chromatography, n-hexane/ethyl acetate = 3/1): 0.57.

### Reference Example 103

### 4-{N-t-butoxycarbonyl-N-[4-(3-(N-t-butoxycarbonyl-N-methylamino)-4-nitrophenoxy)phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester

A suspension of 7 mg of 60% sodium hydride, one drop of dry tetrahydrofuran, and 0.5 mL of dry dimethylformamide was combined with 134 g of 4-(4-t-butoxycarbonylaminophenoxy)-2-(N-t-butoxycarbonyl-N-methylamino)nitrobenzene (the compound of Reference Example 102) and stirred at room temperature for fifteen minutes. The reaction mixture was combined with 125 mg of 4-chloromethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 61) while cooling with ice, stirred at the same temperature for thirty minutes, then stirred at room temperature for five hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 2/1 to 1/1 then 0/1) to give 152 mg of the intended compound as a solid.
Rf value (relative difference) (silica gel thin layer chromatography, n-hexane/ethyl acetate = 3/1): 0.57.

### Reference Example 104

### 4-{N-t-butoxycarbonyl-N-[4-[4-amino-3-(N-t-butoxycarbonyl-N-methylamino)phenoxy]phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester

A mixture of 2.0 g of 4-{N-t-butoxycarbonyl-N-[4-(3-(N-t-butoxycarbonyl-N-methylamino)-4-nitrophenoxy)phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 103), 3 mL of tetrahydrofuran, and 17 mL of methanol was purged with argon, then combined with 1 g of 10% palladium on carbon and purged with hydrogen gas at room temperature for one day. Insoluble matter was filtered off, the filtrate was condensed, and 1.75 g of the resulting residue was refined by silica gel column chromatography (elution solvent: n-hexane/ethyl acetate = 1/1) to give 0.833 g of the intended compound as a white solid.
Rf value (relative difference) (silica gel thin layer chromatography, n-hexane/ethyl acetate = 1/1):0.36.

### Reference Example 105

### 4-{N-t-butoxycarbonyl-N-[4-[4-(4-[2,4-dioxothiazolidin-5-ylmethyl]phenoxyacetylamino)-3-(N-t-butoxycarbonyl-N-methylamino)phenoxy]phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester

A solution of 0.806 g of 4-{N-t-butoxycarbonyl-N-[4-[4-amino-3-(N-t-butoxycarbonyl-N-methylamino)phenoxy]phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 104) and 0.230 g of 4-(2,4-dixothiazolidin-5-ylmethyl)phenoxyacetic acid in 3 mL of dry dimethylformamide was combined with 0.157 g of WSC (1-ethyl-3-(3-dimethylaminopropyl)carbodiimide hydrochloride) and stirred at room temperature for one day. The reaction solution was combined with ethyl acetate and water, and the ethyl acetate layer was separated. The ethyl acetate layer was washed with saturated physiological saline, then dried over anhydrous sodium sulfate. After the solvent had been distilled off, 1.19 g of the resulting residue was refined by silica gel column chromatography (elution solvent: n-hexane/ethyl acetate = 1/2) to give 0.92 g of the intended compound as a light red glassy solid.

Mass spectrum: 1363 (M+1)⁺.
¹H-NMR spectra (500MHz, DMSO-d₆), δₚₚₘ:0.7 3(t, 3H), 1.15(t, 3H), 1.2-1.5 ( broad peak 9H), 1.33(s, 9H), 1.54(m, 2H), 2.39(t, 2H), 2.95(s, 3H), 3.08(q, 1H), 3.33(d, 1 H), 4.08(q, 2H), 4.69(s, 2H), 4.88(q, 1H ), 4.94 (s, 2H), 5.45 (s, 2H), 6.73-7.78( m, 34H), 12.01(s, 1H).

### Reference Example 106

### 4-{N-[2,6-dimethyl-4-(3-(N-t-butoxycarbonyl-N-methylamino)-4-nitrophenoxy)phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester

A mixture of 7 mL of dry dimethylformamide, 1.6 g of 4-(4-amino-3,5,dimethylphenoxy)-2-(N-t-butoxycarbonyl-N-methylamino)nitrobenzene, and 1.5 g of 4-chloromethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 61) was combined with 0.26 g of N,N-diisopropyl ethylamine, stirred at room temperature for two hours, then stirred at 70°C for two days. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 3.1 g of the resulting residue was refined by silica gel column chromatography (elution solvent: n-hexane/ethyl acetate = 1/1) to give 0.95 g of the intended compound as a light yellow solid.
Rf value (relative difference) (silica gel thin layer chromatography, n-hexane/ethyl acetate = 1/1):0.31.

Mass spectrum: 1057 (M⁺).
¹H-NMR, spectra (500MHz, DMSO-d₆), δₚₚₘ**:**0.7 5 (t, 3 H), 1.14(t, 3 H), 1.4-1.21(. broad , 9H), 1.56(m, 2H), 2. 24 (s, 6H), 2.43(t, 2 H), 3. 12 (s, 3H), 4.08(q, 2H), 4.35(d, 2H ), 4.41( 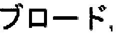 1H), 5.47(s, 2H), 6.71-7. 90(m, 28H).

### Reference Example 107

### 4-{N-[2,6-dimethyl-4-(4-amino-3-(N-t-butoxycarbonyl-N-methylamino)phenoxy)phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester

A mixture of 910 mg of 4-{N-[2,6-dimethyl-4-(3-(N-t-butoxycarbonyl-N-methylamino)-4-nitrophenoxy)phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 106) and 20 mL of methanol was purged with methanol, then combined with 10% palladium on carbon and purged with hydrogen gas at room temperature for five hours. The reaction mixture was combined with 10 mL of tetrahydrofuran, then purged with hydrogen gas at room temperature for twenty hours. Insoluble matter was filtered off from the reaction mixture, the filtrate was condensed, and 0.81 g of the resulting residue was refined by silica gel column chromatography (elution solvent: ethyl acetate/methanol = 1/0 to 20/1, then 5/1) to give 90 g of the intended compound as a white solid.
Rf value (relative difference) (silica gel thin layer chromatography, elution solvent: ethyl acetate/methanol = 9/1): 0.15 (tailing).

Mass spectrum: 786 (M+1)⁺.

### Reference Example 108

### 4-{N-[4-[4-(4-[2,4-dioxothiazolidin-5-ylmethyl]phenoxyacetylamino)-3-(N-t-butoxycarbonyl-N-methylamino)phenoxy]-2,6-dimethylphenyl]aminomethyl}-2-propyl-1-[2'-(1H-tetrazol-5-yl)biphenyl-4-ylmethyl]-1H-imidazole-5-carboxylic acid ethyl ester

A mixture of 79 mg of 4-{N-[2,6-dimethyl-4-(4-amino-3-(N-t-butoxycarbonyl-N-methylamino)phenoxy)phenyl]aminomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 107), 28.5 mg of 4-(2,4-dixothiazolidin-5-ylmethyl)phenoxyacetic acid, and 0.5 mL of dry dimethylformamide was combined with 20 mg of WSC (1-ethyl-3-(3-dimethylaminopropyl)carbodiimide hydrochloride) and stirred at room temperature for one day. The reaction mixture was combined with ethyl acetate and physiological saline, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/methanol = 10/1) to give 61 mg of the intended compound as a glassy solid.
Rf value (relative difference) (silica gel thin layer chromatography, elution solvent: ethyl acetate/methanol = 5/1):0.45.

Mass spectrum: 1049 (M+1)⁺.

### Reference Example 109

### 4-acetylthiomethyl-2-propyl-1-[2'-(1-triphenylmethyl-1H-tetrazol-5-yl)biphenyl-4-ylmethyl]-1H-imidazole-5-carboxylic acid ethyl ester

A solution of 25.54 g of 4-chloromethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 61) in 100 mL of dry tetrahydrofuran was combined with 5.0 g of potassium thioacetate, then combined with 30 mL of dry N,N-dimethylformamide and stirred at room temperature for three hours. The solvent was distilled off under reduced pressure, the residue was combined with ethyl acetate and water, and the ethyl acetate layer was separated. The ethyl acetate layer was washed with saturated physiological saline, then dried over anhydrous sodium sulfate. Distilling off the solvent gave 27.05 g of a coarse product material of the intended compound.
Rf value (relative difference) (silica gel thin layer chromatography, n-hexane/ethyl acetate = 3/1): 0.21.

Mass spectrum: 746 (M+1)⁺.
¹H-NMR⁻ spectra (500MHz, DMSO-d₆), δₚₚₘ:0.7 5(t, 3H), 1.17(t, 3H), 1.51(m, 2H), 2.3 5 (s, 3H), 2.48(t, 2H), 4.14(q, 2H), 5.51( s, 2H), 6.86-7.79(m, 23H)

### Reference Example 110

### 4-mercaptomethyl-2-propyl-1-[2'-(1-triphenylmethyl-1H-tetrazol-5-yl)biphenyl-4-ylmethyl]-1H-imidazole-5-carboxylic acid ethyl ester

A solution of 27 g of 4-acetylthiomethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 109) in 80 mL of methylene chloride was combined with 5 g of morpholine and 3 g of dithioerythritol and stirred at room temperature for twenty hours. The solvent was distilled off, and the residue was combined with ethyl acetate and water, and the ethyl acetate layer was separated. The ethyl acetate layer was washed with saturated physiological saline, then dried over anhydrous sodium sulfate. Distilling off the solvent gave 29 g of a coarse product material (including solvent) of the intended compound.
Rf value (relative difference) (silica gel thin layer chromatography, n-hexane/ethyl acetate = 3/1): 0.21.

Mass spectrum: 705 (M+1)⁺.
¹H-NMR, spectra (500MHz, DMSO-d₆), δₚₚₘ:0. 6 (t, 3H), 1.19(t, 3H), 1.54(m, 2H), 2.43 (t, 2H), 2.69(t, 1H), 3.89(d, 2H), 4.14( q, 2H), 5.49(s, 2H), 6.84-7.78(m, 23H).

### Reference Example 111

### 4-{3-(N-t-butoxycarbonyl-N-methylamino)-4-nitrophenylthiomethyl}-2-propyl-1-[2'-(1-triphenylmethyl-1H-tetrazol-5-yl)biphenyl-4-ylmethyl]-1H-imidazole-5-carboxylic acid ethyl ester

A solution of 26.69 g of 4-mercaptomethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 110) in 100 mL of dry dimethylformamide was dropped into a mixture of 1.5 g of sodium hydride (purity: 60%) and 30 mL of dry tetrahydrofuran while cooling with ice, and stirred at room temperature for thirty minutes. This solution was combined with 13.0 g of 4-chloro-2-(N-t-butoxycarbonyl-N-methylamino)nitrobenzene and heated and stirred at an oil-bath temperature of 60°C for seventeen hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 3/1 to 1/1) to give 26.6 g of the intended compound as a light yellow solid.
Rf value (relative difference) (silica gel thin layer chromatography, n-hexane/ethyl acetate = 3/1): 0.21.

### Reference Example 112

### 4-{4-amino-3-(N-t-butoxycarbonyl-N-methylamino)phenylthiomethyl}-2-propyl-1-[2'-(1H-tetrazol-5-yl)biphenyl-4-ylmethyl]-1H-imidazole-5-carboxylic acid ethyl ester (1H-tetrazole form (a)) and 4-{4-amino-3-(N-t-butoxycarbonyl-N-methylamino)phenylthiomethyl}-2-propyl-1-[2'-(1-triphenylmethyl-1H-tetrazol-5-yl)biphenyl-4-ylmethyl]-1H-imidazole-5-carboxylic acid ethyl ester (1-triphenylmethyl-1H-tetrazole form (b))

(a) A mixture of 3.0 g of 4-{3-(N-t-butoxycarbonyl-N-methylamino)-4-nitrophenylthiomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 111) and 15 mL of methanol was purged with argon, combined with 5.0 g of 10% palladium on carbon, and purged with hydrogen at 50°C for seven hours. The residue resulting from filtering off insoluble matter and distilling off methanol from the filtrate was washed with n-hexane and dissolved in toluene. Distilling off the solvent gave 1.78 g of a coarse product material of the 1H-tetrazole form (a) of the intended compound as a light gray powder.
Rf value (relative difference) of the 1H-tetrazole form (silica gel thin layer chromatography, elution solvent: ethyl acetate/methanol = 9/1): 0.49 and (silica gel thin layer chromatography, elution solvent: ethyl acetate/hexane = 1/1):0.28.

(b) A mixture of 26.5 g of 4-{3-(N-t-butoxycarbonyl-N-methylamino)-4-nitrophenylthiomethyl}-2-propyl-1-[2'-(1-triphenylmethyl-1H-tetrazol-5-yl)biphenyl-4-ylmethyl]-1H-imidazole-5-carboxylic acid ethyl ester (the compound of Reference Example 111), 100 mL of methanol, and 70 mL of tetrahydrofuran was purged with argon, combined with 20 g of 10% palladium on carbon, and purged with hydrogen at room temperature for one week. Filtering off insoluble matter and distilling off the solvent from the filtrate gave 21 g of an about 1:1 coarse product material of the 1H-tetrazole form (a) and the 1-triphenylmethyl-1H-tetrazole form (b) of the intended compound. Refining 3 g of this coarse product material by silica gel column chromatography (elution solvent: ethyl acetate/hexane = 1/1) gave 1.2 g of the 1-triphenylmethyl-1H-tetrazole form (b) of the intended compound as a light yellow powder, which when eluted with an elution solvent (dichloromethane/methanol = 10/1) gave 1.1 g of the 1H-tetrazole form (a) of the intended compound as a light yellow powder.
Rf value (relative difference) of the 1-triphenylmethyl-1H-tetrazole form (b) (silica gel thin layer chromatography, elution solvent: ethyl acetate/hexane = 1/1):0.35.

Mass spectrum: 925 (M+1)⁺.

### Reference Example 113

### 4-{4-[4-(2,4-dioxothiazolidin-5-ylmethyl)phenoxyacetylamino]-3-(N-t-butoxycarbonyl-N-methylamino)phenylthiomethyl}-2-propyl-1-[2'-(1H-tetrazol-5-yl)biphenyl-4-ylmethyl]-1H-imidazole-5-carboxylic acid ethyl ester

A mixture of 1.273 g of 4-{4-amino-3-(N-t-butoxycarbonyl-N-methylamino)phenylthiomethyl}-2-propyl-1-[2'-(1H-tetrazol-5-yl)biphenyl-4-ylmethyl]-1H-imidazole-5-carboxylic acid ethyl ester (the 1H-tetrazole form (a) of the compound of Reference Example 112), 0.46 g of 4-(2,4-dixothiazolidin-5-ylmethyl)phenoxyacetic acid, and 5 mL of dry dimethylformamide was combined with 0.32 g of WSC (1-ethyl-3-(3-dimethylaminopropyl)carbodiimide hydrochloride) and stirred at room temperature for one day. The reaction mixture was combined with ethyl acetate and physiological saline, and the ethyl acetate layer was separated. The ethyl acetate layer was washed with saturated physiological saline, then dried over anhydrous sodium sulfate. After the solvent had been distilled off, 1.75 g of the resulting residue was refined by silica gel column chromatography (elution solvent: ethyl acetate/methanol = 20/1) to give 0.57 g of the intended compound as a yellow powder.
Rf value (relative difference) (silica gel thin layer chromatography, elution solvent: ethyl acetate/methanol = 6/1): 0.68.

Mass spectrum: 946 (M+1)⁺.

### Reference Example 114

### 4-(3-hydroxyphenylthiomethyl)-2-propyl-1-[2'-(1-triphenylmethyl-1H-tetrazol-5-yl)biphenyl-4-ylmethyl]-1H-imidazole-5-carboxylic acid ethyl ester

A solution of 0.36 g of 3-mercaptophenol and 6 mL of dry dimethylformamide was combined with 0.77 g of 4-chloromethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 61) and stirred at room temperature for five hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 1.26 g of the resulting residue was refined by silica gel column chromatography (elution solvent: n-hexane/ethyl acetate = 3/1) to give 0.848 g of the intended compound as a colorless glassy solid.
Rf value (relative difference) (silica gel thin layer chromatography, n-hexane/ethyl acetate = 1/1): 0.67.

### Reference Example 115

### 4-[3-{3-(N-t-butoxycarbonyl-N-methylamino)-4-nitrophenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester

A mixture of 0.817 g of 4-(3-hydroxyphenylthiomethyl)-2-propyl-1-[2'-(1-triphenylmethyl-1H-tetrazol-5-yl)biphenyl-4-ylmethyl]-1H-imidazole-5-carboxylic acid ethyl ester (the compound of Reference Example 114), 41 mg of sodium hydride (purity: 60%), and 3.5 mL of dry dimethylformamide was stirred at room temperature for 2.5 hours. This solution was combined with 0.68 g of 4-chloro-2-(N-t-butoxycarbonyl-N-methylamino)nitrobenzene and heated and stirred at an oil-bath temperature of 90°C for three hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 1.52 g of the resulting residue was refined by silica gel column chromatography (elution solvent: n-hexane/ethyl acetate = 2/1) to give 0.82 g of the intended compound as a light yellow solid.
Rf value (relative difference) (silica gel thin layer chromatography, n-hexane/ethyl acetate = 1/1): 0.73.

### Reference Example 116

### 4-[3-{4-amino-3-(N-t-butoxycarbonyl-N-methylamino)phenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester

A mixture of 0.80 g of 4-[3-{3-(N-t-butoxycarbonyl-N-methylamino)-4-nitrophenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 115), 3mL of tetrahydrofuran, and 5 mL of methanol was purged with argon, combined with 1.23 g of 10% palladium on carbon, and purged with hydrogen at room temperature for one day. The residue resulting from filtering off insoluble matter and condensing the filtrate was refined by silica gel column chromatography (elution solvent: n-hexane/ethyl acetate = 1/1) to give 0.352 g of the intended compound as a colorless solid.
Rf value (relative difference) (silica gel thin layer chromatography, n-hexane/ethyl acetate = 1/1):0.37.

¹H-NMR, spectra (500MHz, DMSO-d₆), δₚₚₘ:0. 3(t, 3H), 1.11(t, 3H), 1.25-1.45( broad, 9H), 1. 52(q, 2H), 2. 40 (t, 2H), 2.96(s, 3 H), 4.07(q, 2H), 4.34(s, 2H), 4.86( broad : 2H), 5.47(s, 2H), 6.63-7.77(m, 30H).

### Reference Example 117

### 4-[3-{2-[4-[4-(2,4-dioxothiazolidin-5-ylmethyl)phenoxyacetylmethyl]-3-(N-t-butoxycarbonyl-N-methylamino)phenoxy]phenylthiomethy}-2-propyl-1-[2'-(1-trimethylphenyl-1H-tetrazol-5-yl)biphenyl-4-ylmethyl]-1H-imidazole-5-carboxylic acid ethyl ester

A solution of 331.3 mg of 4-[3-{4-amino-3-(N-t-butoxycarbonyl-N-methylamino)phenoxy}phenylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 116) in 2 mL of dry dimethylformamide was combined with 66 mg of WSC (1-ethyl-3-(3-dimethylaminopropyl)carbodiimide hydrochloride) and stirred at room temperature for three days. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 0.48 g of the resulting residue was refined by silica gel column chromatography (elution solvent: n-hexane/ethyl acetate = 1/1) to give 210 g of the intended compound as a colorless solid.
Rf value (relative difference) (silica gel thin layer chromatography, n-hexane/ethyl acetate = 1/1):0.27.

Mass spectrum: 1280 (M+1)⁺.
¹H-NMR,⁻ spectra (400MHz, DMSO-d₆), δₚₚₘ:0.7 2 (t, 3H), 1.11(t, 3H), 1.2-1.4 ( broad, 9 H ), 1.51(m, 2H), 2. 3 9 (t, 2H), 2. 99 (s, 3H) 3.08(q, 1H), 3.31(1H, overlapping H₂O)), 4.13 (q 1H), 4.06(t, 11H), 4.37(s, 2H), 4.47(s, 2H), 4.89(q, 1H), 5.47(s, 2H), 6.79-7.7 8 (m, 3H).

### Reference Example 118

### 4-hydroxyphenyllactic acid methyl

A weight of 8.3 g of 4-hydroxyphenyllactic acid was dissolved in 200 mL of methanol, combined with ten drops of concentrated sulfuric acid, and refluxed for two hours. After the reaction had ended, the solvent was distilled off from the reaction mixture, which was combined with ethyl acetate, washed with saturated sodium bicarbonate water and water, and dried over anhydrous magnesium sulfate. Distilling off the solvent gave 8.4 g of the intended compound.
Rf value (relative difference) (silica gel thin layer chromatography, n-hexane/ethyl acetate = 2/3): 0.70.

¹H-NMR spectra (400MHz, chloroform-d ), δₚₚₘ:2. 79(d, 1H), 2.93(d, 1H), 3.06(q, 1H), 3. 7 9 (s, 3H), 4.44(q, 1H), 5.30(s, 1H), 6.73 (d, 2H), 7.06(d, 2H).

### Reference Example 119

### 4-t-butoxycarbonylmethoxyphenyllactic acid methyl

A weight of 5.0 g of 4-hydroxyphenyllactic acid methyl (the compound of Reference Example 118) was dissolved in 60 mL of dimethylformamide, combined with 6 g of bromoacetic acid t-butyl ester and 5.3 g of potassium carbonate, and stirred at 30°C for two hours. After the reaction had ended, the reaction mixture was combined with ethyl acetate, washed with water, and dried over anhydrous magnesium sulfate. Distilling off the solvent gave 8.0 g of the intended compound as a solid.
Rf value (relative difference) (silica gel thin layer chromatography, n-hexane/ethyl acetate = 1/1):0.45.

¹H-NMR spectra (400MHz, chloroform-d ), δₚₚₘ:1. 4 9 (s, 9H), 2.70(d, 1H), 2.93(m, 1H), 3. 0 7 (q, 1H),3.77(s, 3H), 4.42(d, 1H), 4.49 (s, 2H), 6.83(d, 2H), 7.13(d, 2H).

### Reference Example 120

### 3-(4-t-butoxycarbonylmethoxyphenyl)-2-(4-chlorobenzyloxy)propionic acid methyl

A solution of 3.8 g of 4-t-butoxycarbonylmethoxyphenyllactic acid methyl (the compound of Reference Example 119) and 5.0 g of 4-chlorobenzyl bromide in 100 mL of toluene was combined with 11.4 g of silver oxide and stirred at 80°C for three hours. After the reaction had ended, the reaction mixture was filtered using Celite and washed with ethyl acetate. The solvent was distilled off, and the residue was refined by silica gel column chromatography (elution solvent: n-hexane/ethyl acetate = 5/1) to give 3.8 g of the intended compound as a solid.
Rf value (relative difference) (silica gel thin layer chromatography, n-hexane/ethyl acetate = 5/1): 0.40.

¹H-NMR spectra (400MHz, chloroform-d ), δₚₚₘ:1. 4 9 (s, 9H), 2.99(q, 2H), 3. 72 (s, 3H), 4.0 9 (q, 1H), 4.31(d, 1H), 4.51(s, 2H), 4.62 (d, 1H), 6.82(d, 2H), 7.08(d, 2H), 7.12( d, 2H), 7.24(d, 2H).

### Reference Example 121

### 3-(4-carboxymethoxyphenyl)-2-(4-chlorobenzyloxy)propionic acid methyl

A weight of 3.8 g of 3-(4-t-butoxycarbonylmethoxyphenyl)-2-(4-chlorobenzyloxy)propionic acid methyl (the compound of Reference Example 120) was combined with 60 mL of a 4 N hydrogen chloride-dioxane solution and stirred at room temperature for three hours. After the reaction had ended, the solvent was distilled off from the reaction mixture, which was combined with 60 mL of toluene. Distilling off the solvent and drying the residue under reduced pressure gave 3.3 g of the intended compound as a solid.

Mass spectrum: 379 (M+1)⁺.
¹H-NMR, spectra (400MHz, chloroform-d ), δppm:2. 29 (m, 2H), 3.73(s, 3H), 4.06(q, 1H), 4.3 1 (d, 1H), 4.63(d, 1H), 4.68(s, 2H), 6.85 (d, 2H), 7.08(d, 2H), 7. 1 5(d, 2H), 7.23( d , 2H).

### Reference Example 122

### 3-[4-[4-(5-ethoxycarbonyl-1-{2'-(1H-tetrazol-5-yl)biphenyl-4-ylmethyl}-2-propyl-1H-imidazol-4-ylmethylthio)-2-(N-t-butoxycarbonyl-N-methylamino)phenylaminocarbonylmethoxy]phenyl]-2-(4-chlorobenzyloxy)propionic acid methyl

A weight of 500 mg of 4-{4-amino-3-(N-t-butoxycarbonyl-N-methylamino)phenylthiomethyl}-2-propyl-1-[2'-(1-triphenylmethyl-1H-tetrazol-5-yl)biphenyl-4-ylmethyl]-1H-imidazole-5-carboxylic acid ethyl ester (the 1-triphenylmethyl-1H-tetrazole form (b) of Reference Example 112) and 400 mg of 3-(4-carboxymethoxyphenyl)-2-(4-chlorobenzyloxy)propionic acid methyl (the compound of Reference Example 121) was dissolved in 30 mL of ethanol, combined with 300 mg of 4-(4,6-dimethoxy-1,3,5-triazin-2-yl)-4-methylmorpholinium chloride (DMTMM), and stirred at room temperature for twelve hours. After the reaction had ended, the reaction mixture was combined with ethyl acetate, washed with saturated sodium bicarbonate water then with water, and dried over anhydrous magnesium sulfate. Distilling off the solvent gave 840 g of the intended compound as a solid.
Rf value (relative difference) (silica gel thin layer chromatography, n-hexane/ethyl acetate = 1/1): 0.50.

### Reference Example 123

### 4-formylphenoxyacetic acid t-butyl

A weight of 5.0 g of hydroxybenzaldehyde and 8 g of bromoacetic acid t-butyl ester was dissolved in 100 mL of acetaone, combined with 8.5 g of potassium carbonate, and stirred at 60°C for one hour. After the reaction had ended, the solvent was distilled off from the reaction mixture, which was extracted with ethyl acetate. The extract was washed with water and dried over anhydrous magnesium sulfate. Distilling off the solvent gave 9.6 g of the intended compound as a solid.

¹H-NMR spectra (400MHz. chloroform-d ), δₚₚₘ : 1, 49(s, 9H), 4.61(S, 2H), 7.00(d, 2H), 7.8 5(d, 2H), 9.90(s, 1H).

### Reference Example 124

### 4-ethoxycarbonylmethylaminomethylphenoxyacetic acid t-butyl

A weight of 3.0 g of 4-formylphenoxyacetic acid t-butyl (the compound of Reference Example 123) was dissolved in 50 mL of dichloroethane, combined with 1.8 g of glycine hydrochloride, 2.6 g of triethylamine, and 5.4 g of sodium triacetoxy borohydride at room temperature, and stirred at room temperature for three hours. After the reaction had ended, the reaction mixture was combined with saturated sodium bicarbonate water, and extracted with ethyl acetate. The extract was washed with water and dried over anhydrous magnesium sulfate. The solvent was distilled off, and the residue was refined by silica gel column chromatography (elution solvent: n-hexane/ethyl acetate = 1/1) to give 3.0 g of the intended compound as a solid.
Rf value (relative difference) (silica gel thin layer chromatography, hexane:ethyl acetate = 1/1): 0.30

¹H-NMR spectra (400MHz, chloroform-d ), δₚₚₘ : 1. 28(t, 3H), 1.49(s, 9H), 3.99(s, 2H), 3.7 4(s, 2H), 4.20(q, 2H), 4.50(s, 2H), 6.86 (d, 2H), 7.25(d, 2H).

### Reference Example 125

### 4-(N-ethoxycarbonylmethyl-N-phenoxycarbonylaminomethyl)phenoxyacetic acid t-butyl

A weight of 3 g of 4-ethoxycarbonylmethylaminomethylphenoxyacetic acid t-butyl (the compound of Reference Example 124) was dissolved in 50 mL of methylene chloride, then combined with 1.6 g of triethylamine. A weight of 1.93 g of phenyl chloroformate was dropped in while cooling with ice, and the reaction system was stirred at room temperature for fifteen minutes. After the reaction had ended, the reaction mixture was poured into ice water and extracted with methylene chloride. The extract was washed with water and dried over anhydrous magnesium sulfate. After the solvent had been distilled off, the residue was refined by silica gel column chromatography (elution solvent: hexane/ethyl acetate = 5/1) to give 4.0 g of the intended compound as a solid.
Rf value (relative difference) (silica gel thin layer chromatography, hexane:ethyl acetate = 5/1): 0.50.

¹H-NMR⁻ spectra (400MHz, chloroform-d ), δₚₚₘ : 1 26 (t, 3H), 1. 50 (s, 9H), 4.01 (d, 2H), 4.1 2 (q, 2H), 4.19 (q, 2H), 4.52 (d, 2H), 6.86 -7.39 (m, 9H).

### Reference Example 126

### 4-(N-ethoxycarbonylmethyl-N-phenoxycarbonylaminomethyl)phenoxyacetic acid

A weight of 4.0 g of 4-(N-ethoxycarbonylmethyl-N-phenoxycarbonylaminomethyl)phenoxyacetic acid t-butyl (the compound of Reference Example 125) was combined with 60 mL of a 4 N hydrogen chloride-dioxane solution and stirred at room temperature for three hours. After the reaction had ended, the solvent was distilled off from the reaction mixture, and the residue was combined with 60 mL of toluene. Distilling off the solvent and drying the residue under reduced pressure gave 3.8 g of the intended compound as a solid.

Mass spectrum: 388 (M+1)⁺.
¹H-NMR spectra (400MHz. chloroform-d ), δₚₚₘ : 1. 26(t, 3H), 4.02(d, 2H), 4.19(q, 2H), 4. 6 4(d, 2H), 4.70(d, 2H), 6.90-7.39 (m, 9H)

### Reference Example 127

### N-[4-{4-[5-ethoxycarbonyl-1-{2'-(1-triphenylmethyl-1H-tetrazol-5-yl)biphenyl-4-ylmethyl}-2-propyl-1H-imidazol-4-ylmethylthio]-2-(N-t-butoxycarbonyl-N-methylamino)phenylaminocarbonylmethoxy}phenylmethyl]-N-phenyloxycarbonylaminoacetic acid ethyl ester

A weight of 600 mg of 4-{4-amino-3-(N-t-butoxycarbonyl-N-methylamino)phenylthiomethyl}-2-propyl-1-[2'-(1-triphenylmethyl-1H-tetrazol-5-yl)biphenyl-4-ylmethyl]-1H-imidazole-5-carboxylic acid ethyl ester (the 1-triphenylmethyl-1H-tetrazole form (b) of Reference Example 112) and 500 mg of 4-(N-ethoxycarbonylmethyl-N-phenoxycarbonylaminomethyl)phenoxyacetic acid (the compound of Reference Example 126) was dissolved in 50 mL of ethanol, then combined with 360 mg of 4-(4,6-dimethoxy-1,3,5-triazin-2-yl)-4-methylmorpholinium chloride (DMTMM) and stirred at room temperature for twelve hours. After the reaction had ended, the reaction mixture was combined with ethyl acetate, washed with saturated sodium bicarbonate water then with water, and dried over anhydrous magnesium sulfate. After the solvent had been distilled off, the residue was refined by silica gel column chromatography (elution solvent: ethyl acetate/methylene chloride = 1/2) to give 480 mg of the intended compound as a solid.
Rf value (relative difference) (silica gel thin layer chromatography, elution solvent: ethyl acetate/methylene chloride = 1/2): 0.60.
Mass spectrum: 1294 (M+1)⁺.

### Reference Example 128

### 4-[N-ethoxycarbonylmethyl-N-(3-trifluoromethylbenzoyl)aminomethyl]phenoxyacetic acid t-butyl

A weight of 1.15 g of 3-(trifluoromethyl)benzoyl chloride was dropped into a solution of 1.5 g of 4-ethoxycarbonylmethylaminomethylphenoxyacetic acid t-butyl and 0.7 g of triethylamine in 50 mL of methylene chloride while cooling with ice, and stirred at room temperature for fifteen minutes. Next, the solvent was distilled off, and the residue was combined with ethyl acetate and washed with saturated sodium bicarbonate water, then water. The ethyl acetate solution was dried over anhydrous magnesium sulfate. Distilling off the solvent gave 2.27 g of the intended compound as a solid.
Rf value (relative difference) (silica gel thin layer chromatography, elution solvent: ethyl acetate/methylene chloride = 1/2): 0.40.
Mass spectrum: 495 (M+1)⁺.

### Reference Example 129

### 4-[N-ethoxycarbonylmethyl-N-(3-trifluoromethylbenzoyl)aminomethyl]phenoxyacetic acid

A solution of 2.27 g of 4-[N-ethoxycarbonylmethyl-N-(3-trifluoromethylbenzoyl)aminomethyl]phenoxyacetic acid t-butyl in 20 mL of dixoane was combined with 30 mL of a 4 N hydrogen chloride-dioxane solution and stirred at room temperature for twelve hours. After the reaction had ended, the solvent was distilled off from the reaction mixture, and the residue was combined with ethyl acetate, washed with an ethyl acetate solution, and dried over anhydrous magnesium sulfate. After the solvent had been distilled off, the residue was refined by silica gel column chromatography (elution solvent: ethyl acetate/methanol = 10/1) to give 1.1 g of the intended compound as a solid.
Rf value (relative difference) (silica gel thin layer chromatography, methylene chloride/methanol = 10/1): 0.15.
Mass spectrum: 440 (M+1)⁺.

### Reference Example 130

### N-[4-{4-[5-ethoxycarbonyl-1-{2'-(1-triphenylmethyl-1H-tetrazol-5-yl)biphenyl-4-ylmethyl}-2-propyl-1H-imidazol-4-ylmethylthio]-2-(N-t-butoxycarbonyl-N-methylamino)phenylaminocarbonylmethoxy}phenylmethyl]-N-(3-trifluoromethylbenzoyl)aminoacetic acid ethyl

A weight of 412 mg of 4-{4-amino-3-(N-t-butoxycarbonyl-N-methylamino)phenylthiomethyl}-2-propyl-1-[2'-(1-triphenylmethyl-1H-tetrazol-5-yl)biphenyl-4-ylmethyl]-1H-imidazole-5-carboxylic acid ethyl ester (the 1-triphenylmethyl-1H-tetrazole form (b) of Reference Example 112) and and 390 mg of 4-[N-ethoxycarbonylmethyl-N-(3-trifluoromethylbenzoyl)aminomethyl]phenoxyacetic acid (the compound of Reference Example 129) was dissolved in 30 mL of ethanol. This solution was combined with 245 mg of 4-(4,6-dimethoxy-1,3,5-triazin-2-yl)-4-methylmorpholinium chloride (DMTMM) and stirred at room temperature for twelve hours. After the reaction had ended, the reaction mixture was combined with ethyl acetate, washed with saturated sodium bicarbonate water then with water, and dried over anhydrous magnesium sulfate. The solvent was distilled off, and the residue was refined by silica gel column chromatography (elution solvent: ethyl acetate/hexane = 1/1) to give 390 mg of the intended compound as a solid.
Rf value (relative difference) (silica gel thin layer chromatography, elution solvent: ethyl acetate/hexane = 1/1): 0.50.
Mass spectrum: 1346 (M+1)⁺.

### Reference Example 131

### 4-[3-(N-t-butoxycarbonyl-N-methylamino)-4-nitrophenoxy]benzoic acid ethyl

A solution of 2.89 g of sodium hydride (purity: 60%) in 5 mL of tetrahydrofuran and 80 mL of dry dimethylformamide was combined with 12.02 g of 4-hydroxybenzoic acid ethyl while cooling with ice, and stirred at room temperature for one hour. This solution was combined with 20.74 g of 4-chloro-2-(N-t-butoxycarbonyl-N-methylamino)nitrobenzene and heated and stirred at an oil-bath temperature of 90-100°C for twenty hours. The solvent was distilled off from the reaction mixture under reduced pressure, and the residue was combined with ethyl acetate and water saline, and the ethyl acetate layer was separated. The ethyl acetate layer was washed with saturated physiological saline, then dried over anhydrous sodium sulfate. The residue resulting from distilling off the solvent was recrystallized from a solution mixture of ethyl acetate and hexane to give 21.16 g of the intended compound as a light yellow solid.
Rf value (relative difference) (silica gel thin layer chromatography, n-hexane/ethyl acetate = 3/1): 0.55

### Reference Example 132

### 4-[3-(N-t-butoxycarbonyl-N-methylamino)-4-nitrophenoxy]benzyl alcohol

A solution of 15.0 g of 4-[3-(N-t-butoxycarbonyl-N-methylamino)-4-nitrophenoxy]benzoic acid ethyl (the compound of Reference Example 131) in 70 mL of dry tetrahydrofuran was cooled in an ice-acetone bath, and 85 mL of a 1 mol solution of diisobutylaluminum hydride were dropped in. The ice-acetone bath was removed, and the reaction solution was stirred at room temperature overnight. Fifty milliliters of water were dropped into the reaction mixture, and the solvent was distilled off under reduced pressure. The residue was combined with ethyl acetate and water, and the ethyl acetate layer was separated. The ethyl acetate layer was washed with saturated physiological saline, then dried over anhydrous sodium sulfate. The residue resulting from distilling off the solvent was recrystallized from a solution mixture of ethyl acetate and hexane to give 9.0 g of the intended compound as a yellow solid.
Rf value (relative difference) (silica gel thin layer chromatography, n-hexane/ethyl acetate = 2/1): 0.25.

### Reference Example 133

### thioacetic acid S-[4-[3-(N-to-butoxycarbonyl-N-methylamino)-4-nitrophenoxy]benzyl]

A solution of 8.93 g of 4-[3-(N-t-butoxycarbonyl-N-methylamino)-4-nitrophenoxy]benzyl alcohol in 50 mL of methylene chloride was combined with 4.1 g of methanesulfonyl chloride, then combined with 3.62 g of triethylamine and stirred at room temperature overnight. The reaction solution was combined with methylene chloride and water, and the methylene chloride layer was separated. The methylene chloride layer was washed with water and saturated physiological saline, then dried over anhydrous sodium sulfate. The residue resulting from distilling off the solvent was combined with 50 mL of methylene chloride and 4.0 g of potassium thioacetate, and stirred at room temperature for three days. The reaction mixture was combined with methylene chloride and water, and the methylene chloride layer was separated. The methylene chloride layer was washed with water and saturated physiological saline, then dried over anhydrous sodium sulfate. Distilling off the solvent gave 10.53 g of the intended compound as a light yellow solid.
Rf value (relative difference) (silica gel thin layer chromatography, n-hexane/ethyl acetate = 3/1): 0.53.

### Reference Example 134

### 4-[3-(N-t-butoxycarbonyl-N-methylamino)-4-nitrophenoxy]benzylmercaptan

A solution of 10.47 g of thioacetic acid S-[4-[3-(N-to-butoxycarbonyl-N-methylamino)-4-nitrophenoxy]benzyl] (the compound of Reference Example 133) in 50 mL of methylene chloride was combined with 3.16 g of morpholine, then combined with 1.0 g of dithioerythritol and stirred at room temperature overnight. The solvent was distilled off, the residue was combined with ethyl acetate, toluene, and water, and the organic layer was separated. The organic layer was washed with water and saturated physiological saline, then dried over anhydrous sodium sulfate. The residue resulting from distilling off the solvent was refined by silica gel column chromatography (elution solvent: ethyl acetate/hexane = 1/5) to give 7.24 g of the intended compound as a solid.
Rf value (relative difference) (silica gel thin layer chromatography, elution solvent: ethyl acetate/hexane = 1/5): 0.38.

### Reference Example 135

### 4-[4-[3-(N-t-butoxycarbonyl-N-methylamino)-4-nitrophenoxy]benzylthiomethyl]-2-propyl-1-[2'-(1-triphenylmethyl-1H-tetrazol-5-yl)biphenyl-4-ylmethyl]-1H-imidazole-5-carboxylic acid ethyl ester

A solution of 4-chloromethyl-2-propyl-1-[2'-(1-triphenylmethyl-1H-tetrazol-5-yl)biphenyl-4-ylmethyl]-1H-imidazole-5-carboxylic acid ethyl ester (the 4-chloromethyl form of the compound of Reference Example 61) in 60 mL of dry dimethylformamide was combined with 4.95 g of 4-[3-(N-t-butoxycarbonyl-N-methylamino)-4-nitrophenoxy]benzylmercaptan (the compound of Reference Example 134) and 3.0 g of anhydrous potassium carbonate, and stirred at room temperature overnight. The reaction solution was combined with 2.0 g more of 4-chloromethyl-2-propyl-1-[2'-(1-triphenylmethyl-1H-tetrazol-5-yl)biphenyl-4-ylmethyl]-1H-imidazole-5-carboxylic acid ethyl ester and 2.0 g more of anhydrous potassium carbonate, and heated and stirred at 60°C for four hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: ethyl acetate/hexane = 2/1) to give 8.95 g of the intended compound as a solid.
Rf value (relative difference) (silica gel thin layer chromatography, n-hexane/ethyl acetate = 2/1): 0.42.

### Reference Example 136

### 4-[4-[4-amino-3-(N-t-butoxycarbonyl-N-methylamino)phenoxy]benzylthiomethyl]-2-propyl-1-[2'-(1-triphenylmethyl-1H-tetrazol-5-yl)biphenyl-4-ylmethyl]-1H-imidazole-5-carboxylic acid ethyl ester

A mixture of 8.85 g of 4-[4-[3-(N-t-butoxycarbonyl-N-methylamino)-4-nitrophenoxy]benzylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 135) and 6.5 go of 10% palladium on carbon in 50 mL of methanol and 20 mL of tetrahydrofuran was purged with hydrogen at 1 atm and 45°C for fifteen hours. Insoluble matter was filtered off from the reaction mixture, and the filtrate was condensed. The resulting residue was refined by silica gel column chromatography (elution solvent: ethyl acetate/hexane = 1/1) to give 2.01 g of the intended compound as a solid.
Rf value (relative difference) (silica gel thin layer chromatography, n-hexane/ethyl acetate = 1/1): 0.56.
The compound was refined again by silica gel column chromatography (elution solvent: ethyl acetate/methanol = 10/1) to give 2.53 g of a de-triphenylmethyl form of the intended compound (4-[4-[4-amino-3-(N-t-butoxycarbonyl-N-methylamino)phenoxy]benzylthiomethyl]-2-propyl-1-[2'-(1H-tetrazol-5-yl)biphenyl-4-ylmethyl]-1H-imidazole-5-carboxylic acid ethyl ester)
Rf value (relative difference) (silica gel thin layer chromatography, n-hexane/ethyl acetate = 10/1): 0.31 (tailing).

### Reference Example 137

### 4-{4-[4-[4-(2,4-dioxothiazolidin-5-ylmethyl)phenoxyacetylamino]-3-(N-t-butoxycarbonyl-N-methylamino)phenoxy]benzylthiomethyl}-2-propyl-1-[2'-(1-triphenylmethyl-1H-tetrazol-5-yl)biphenyl-4-ylmethyl]-1H-imidazole-5-carboxylic acid ethyl ester

A solution of 1.97 g of 4-[4-[4-amino-3-(N-t-butoxycarbonyl-N-methylamino)phenoxy]benzylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 136) in 5.5 mL of dry dimethylformamide was combined with 0.553 g of 4-(2,4-dixothiazolidin-5-ylmethyl)phenoxyacetic acid and 0.384 g of WSC (1-ethyl-3-(3-dimethylaminopropyl)carbodiimide hydrochloride), and stirred at 40°C for one day. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 2.71 g of the resulting residue was refined by silica gel column chromatography (elution solvent: n-hexane/ethyl acetate = 1/1) to give 1.80 g of the intended compound as a colorless solid.
Rf value (relative difference) (silica gel thin layer chromatography, n-hexane/ethyl acetate = 1/1): 0.26.

### Reference Example 138

### 2-[3-(N-t-butoxycarbonyl-N-methylamino)-4-nitrophenoxy]nicotinic acid ethyl

Using sodium hydride (purity: 60%), dry tetrahydrofuran, dry dimethylformamide, and 4-chloro-2-(N-t-butoxycarbonyl-N-methylamino)nitrobenzene, these were reacted and refined in the same manner as Reference Example 131 to give the intended compound.

### Reference Example 139

### 2-[3-(N-t-butoxycarbonyl-N-methylamino)-4-nitrophenoxy]pyridin-5-ylmethyl alcohol

Using 2-[3-(N-t-butoxycarbonyl-N-methylamino)-4-nitrophenoxy]nicotinic acid ethyl (the compound of Reference Example 138), dry tetrahydrofuran, and a 1 mol solution of diisobutylaluminum hydride, these were reacted and refined in the same manner as Reference Example 132 to give the intended compound.

### Reference Example 140

### thioacetic acid S-[2-{3-(N-t-butoxycarbonyl-N-methylamino)-4-nitrophenoxy}pyridin-5-ylmethyl]

Using 4-[3-(N-t-butoxycarbonyl-N-methylamino)-4-nitrophenoxy]pyridin-5-ylmethyl alcohol (the compound of Reference Example 139), methylene chloride, methanesulfonyl chloride, triethylamine, and potassium thioacetate, these were reacted and refined in the same manner as Reference Example 133 to give the intended compound.

### Reference Example 141

### 2-[3-(N-t-butoxycarbonyl-N-methylamino)-4-nitrophenoxy]pyridin-5-ylmethylmercaptan

Using thioacetic acid S-[2-{3-(N-t-butoxycarbonyl-N-methylamino)-4-nitrophenoxy}pyridin-5-ylmethyl] (the compound of Reference Example 140), methylene chloride, 3.16 g of morpholine, and dithioerythritol, these were reacted and refined in the same manner as Reference Example 134 to give the intended compound.

### Reference Example 142

### 4-[2-[3-(N-t-butoxycarbonyl-N-methylamino)-4-nitrophenoxy]pyridin-5-ylmethylthiomethyl]-2-propyl-1-[2'-(1-triphenylmethyl-1H-tetrazol-5-yl)biphenyl-4-ylmethyl]-1H-imidazole-5-carboxylic acid ethyl ester

Using 4-chloromethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 61), dry dimethylformamide, 2-[3-(N-t-butoxycarbonyl-N-methylamino)-4-nitrophenoxy]pyridin-5-ylmethylmercaptan (the compound of Reference Example 141), and anhydrous potassium carbonate, these were reacted and refined in the same manner as Reference Example 135 to give the intended compound.

### Reference Example 143

### 4-[2-[4-amino-3-(N-t-butoxycarbonyl-N-methylamino)phenoxy]pyridin-5-ylmethylthiomethyl]-2-propyl-1-[2'-(1-triphenylmethyl-1H-tetrazol-5-yl)biphenyl-4-ylmethyl]-1H-imidazole-5-carboxylic acid ethyl ester

Using 4-[2-[3-(N-t-butoxycarbonyl-N-methylamino)-4-nitrophenoxy]pyridin-5-ylmethylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 142), 10% palladium on carbon, methanol, tetrahydrofuran, and hydrogen, these were reacted and refined in the same manner as Reference Example 136 to give the intended compound.

### Reference Example 144

### 4-{2-[4-[4-(2,4-dioxothiazolidin-5-ylmethyl)phenoxyacetylamino]-3-(N-t-butoxycarbonyl-N-methylamino)phenoxy]pyridin-5-ylmethylthiomethyl}-2-propyl-1-[2'-(1-triphenylmethyl-1H-tetrazol-5-yl)biphenyl-4-ylmethyl]-1H-imidazole-5-carboxylic acid ethyl ester

Using 4-[2-[4-amino-3-(N-t-butoxycarbonyl-N-methylamino)phenoxy]pyridin-5-ylmethylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 143), dry dimethylformamide solution, 4-(2,4-dixothiazolidin-5-ylmethyl)phenoxyacetic acid, and WSC (1-ethyl-3-(3-dimethylaminopropyl)carbodiimide hydrochloride), these were reacted and refined in the same manner as Reference Example 137 to give the intended compound.

### Reference Example 145

### 4-(1-chloroethyl)-2-propyl-1-[2'-(1-triphenylmethyl-1H-tetrazol-5-yl)biphenyl-4-ylmethyl]-1H-imidazole-5-carboxylic acid ethyl ester (chloroethyl form) and 4-(1-acetylthioethyl)-2-propyl-1-[2'-(1-triphenylmethyl-1H-tetrazol-5-yl)biphenyl-4-ylmethyl]-1H-imidazole-5-carboxylic acid ethyl ester (acetylthioethyl form)

A solution of 4.5 g of 4-(1-hydroxyethyl)-1-[2'-(1-triphenylmethyl-1H-tetrazol-5-yl)biphenyl-4-ylmethyl]-2-propyl-1H-imidazole-5-carboxylic acid ethyl ester in 20 mL of dry methylene chloride was cooled in a methanol-ice bath and combined with 0.49 mL of thionyl chloride, then combined with 1.0 mL of 1,8-diazabicyclo[5.4.0]-7-undecene (DBU) and stirred at room temperature for 5.5 hours. The reaction solution was combined with 1.53 g of potassium thioacetate and stirred at room temperature overnight. The reaction solution was combined with 2.01 g more of potassium thioacetate and 10 mL of N,N-dimethylformamide, and stirred at room temperature for 5.5 hours. The reaction mixture was combined with ethyl acetate and water, and the ethyl acetate layer was separated. The ethyl acetate layer was washed with saturated physiological saline, then dried over anhydrous sodium sulfate. The residue resulting from distilling off the solvent was refined by silica gel column chromatography (elution solvent: n-hexane/ethyl acetate = 4/1) to give 2.3 g of the intended compound (chloroethyl form) as a colorless solid and 0.90 g of the intended compound (acetylthioethyl form) as a light red solid.

chloroethyl form
Rf value (relative difference) (silica gel thin layer chromatography, n-hexane/ethyl acetate = 3/1): 0.43.
¹H-NMR spectra (400MHz, DMSO-d₆), δₚₚₘ : 0.7 7 (t, 3H), 1.17 (t, 3H), 1.55 (m, 2H), 1.84 (d, 3H), 2.45 (t, 2H), 4.17 (q, 2H), 5.49 ( q, 2H), 5.85 (q, 1H), 6.84-7.79 (m, 23H).
acetylthioethyl form
Rf value (relative difference) (silica gel thin layer chromatography, n-hexane/ethyl acetate = 3/1): 0.30.

### Reference Example 147

### 4-(1-mercaptoethyl)-2-propyl-1-[2'-(1-triphenylmethyl-1H-tetrazol-5-yl)biphenyl-4-ylmethyl]-1H-imidazole-5-carboxylic acid ethyl ester

Using 4-(1-acetylthioethyl)-2-propyl-1-[2'-(1-triphenylmethyl-1H-tetrazol-5-yl)biphenyl-4-ylmethyl]-1H-imidazole-5-carboxylic acid ethyl ester (the acetylthioethyl form of the compound of Reference Example 145), methylene chloride, morpholine, and dithioerythritol, these were reacted and refined in the same manner as Reference Example 110 to give the intended compound.

### Reference Example 148

### 4-[1- {3-(N-t-butoxycarbonyl-N-methylamino)-4-nitrophenylthio}ethyl]-2-propyl-1-[2'-(1-triphenylmethyl-1H-tetrazol-5-yl)biphenyl-4-ylmethyl]-1H-imidazole-5-carboxylic acid ethyl ester

Using sodium hydride (purity: 60%), dry tetrahydrofuran, 4-(1-mercaptoethyl)-2-propyl-1-[2'-(1-triphenylmethyl-1H-tetrazol-5-yl)biphenyl-4-ylmethyl]-1H-imidazole-5-carboxylic acid ethyl ester (the compound of Reference Example 147), dry dimethylformamide, and 4-chloro-2-(N-t-butoxycarbonyl-N-methylamino)nitrobenzene, these were reacted and refined in the same manner as Reference Example 111 to give the intended compound.

### Reference Example 149

### 4-[1-{4-amino-3-(N-t-butoxycarbonyl-N-methylamino)phenylthio}ethyl]-2-propyl-1-[2'-(1-triphenylmethyl-1H-tetrazol-5-yl)biphenyl-4-ylmethyl]-1H-imidazole-5-carboxylic acid ethyl ester

Using 4-[1-{3-(N-t-butoxycarbonyl-N-methylamino)-4-nitrophenylthio}ethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 148), methanol, 10% palladium on carbon, and hydrogen, these were reacted and refined in the same manner as Reference Example 112 to give the intended compound.

### Reference Example 150

### 4-[1-{4-[4-(2,4-dioxothiazolidin-5-ylmethyl)phenoxyacetylamino]-3-(N-t-butoxycarbonyl-N-methylamino)phenylthio}ethyl]-2-propyl-1-[2'-(1-triphenylmethyl-1H-tetrazol-5-yl)biphenyl-4-ylmethyl]-1H-imidazole-5-carboxylic acid ethyl ester

Using 4-[1-{4-amino-3-(N-t-butoxycarbonyl-N-methylamino)phenylthio}ethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 149), 4-(2,4-dixothiazolidin-5-ylmethyl)phenoxyacetic acid, dry dimethylformamide, and WSC (1-methyl-3-(3-dimethylaminopropyl)carbodiimide hydrochloride), these were reacted and refined in the same manner as Reference Example 113 to give the intended compound.

### Reference Example 151

### 4-[1-[2-[3-(N-t-butoxycarbonyl-N-methylamino)-4-nitrophenoxy]pyridin-5-ylmethylthio]ethyl]-2-propyl-1-[2'-(1-triphenylmethyl-1H-tetrazol-5-yl)biphenyl-4-ylmethyl]-1H-imidazole-5-carboxylic acid ethyl ester

Using 4-(1-chloroethyl)-2-propyl-1-[2'-(1-triphenylmethyl-1H-tetrazol-5-yl)biphenyl-4-ylmethyl]-2-propyl-1H-imidazole-5-carboxylic acid ethyl ester (the compound of Reference Example 145), 2-[3-(N-t-butoxycarbonyl-N-methylamino)-4-nitrophenoxy]pyridin-5-ylmethylmercaptan (the compound of Reference Example 141), dry dimethylformamide, and anhydrous potassium carbonate, these were reacted and refined in the same manner as Reference Example 135 to give the intended compound.

### Reference Example 152

### 4-[1-[2-[4-amino-3-(N-t-butoxycarbonyl-N-methylamino)phenoxy]pyridin-5-ylmethylthio]ethyl]-2-propyl-1-[2'-(1-triphenylmethyl-1H-tetrazol-5-yl)biphenyl-4-ylmethyl]-1H-imidazole-5-carboxylic acid ethyl ester

Using 4-[1-[2-[3-(N-t-butoxycarbonyl-N-methylamino)-4-nitrophenoxy]pyridin-5-ylmethylthio]ethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 151), methanol, 10% palladium on carbon, and hydrogen, these were reacted and refined in the same manner as Reference Example 112 to give the intended compound.

### Reference Example 153

### 4-{1-[2-[4-[4-(2,4-dioxothiazolidin-5-ylmethyl)phenoxyacetylamino]-3-(N-t-butoxycarbonyl-N-methylamino)phenoxy]pyridin-5-ylmethylthio]ethyl}-2-propyl-1-[2'-(1H-tetrazol-5-yl)biphenyl-4-ylmethyl]-1H-imidazole-5-carboxylic acid ethyl ester

Using 4-[1-[2-[4-amino-3-(N-t-butoxycarbonyl-N-methylamino)phenoxy]pyridin-5-ylmethylthio]ethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 152), dry dimethylformamide solution, 4-(2,4-dixothiazolidin-5-ylmethyl)phenoxyacetic acid, and WSC (1-ethyl-3-(3-dimethylaminopropyl)carbodiimide hydrochloride), these were reacted and refined in the same manner as Reference Example 137 to give the intended compound.

### Reference Example 154

### 2-butyl-4-chloromethyl-1-[2'-(1-triphenylmethyl-1H-tetrazol-5-yl)biphenyl-4-ylmethyl] - 1H-imidazole-5-carboxylic acid ethyl ester

Using 3.96 g of 2-butyl-4-hydroxymethyl-1-[2'-(1-triphenylmethyl-1H-tetrazol-5-yl)biphenyl-4-ylmethyl]-1H-imidazole-5-carboxylic acid ethyl ester, 20 mL of dry methylene chloride, 5 mL of N,N-dimethylformamide, 0.71 g of methanesulfonyl chloride, and 0.90 g N,N-diisopropyl ethylamine, these were reacted and refined in the same manner as Reference Example 61 to give 4.07 g of the intended compound as a colorless solid.
Rf value (relative difference) (silica gel thin layer chromatography, n-hexane/ethyl acetate = 2/1): 0.63.

### Reference Example 155

### 4-acetylthiomethyl-2-butyl-1-[2'-(1-triphenylmethyl-1H-tetrazol-5-yl)biphenyl-4-ylmethyl]-1H-imidazole-5-carboxylic acid ethyl ester

Using 2.1 g of 2-butyl-4-chloromethyl-1-[2'-(1-triphenylmethyl-1H-tetrazol-5-yl)biphenyl-4-ylmethyl]-1H-imidazole-5-carboxylic acid ethyl ester (the compound of Reference Example 154), 20 mL of dry methylene chloride, 0.85 g of potassium thioacetate, and 20 mL of N,N-dimethylformamide, these were reacted in the same manner and subjected to the same after-treatment as Reference Example 109 to give 2.7 g of a coarse product material of the intended product as a colorless solid.
Rf value (relative difference) (silica gel thin layer chromatography, n-hexane/ethyl acetate = 3/1): 0.19.

### Reference Example 156

### 2-butyl-4-mercaptomethyl-1-[2'-(1-triphenylmethyl-1H-tetrazol-5-yl)biphenyl-4-ylmethyl]-1H-imidazole-5-carboxylic acid ethyl ester

Using 2.7 g of 4-acetylthiomethyl-2-butyl-1-[2'-(1-triphenylmethyl-1H-tetrazol-5-yl)biphenyl-4-ylmethyl]-1H-imidazole-5-carboxylic acid ethyl ester (the compound of Reference Example 155), 10 mL of methylene chloride, 1.2 g of morpholine, and 0.3 g of dithioerythritol, these were reacted in the same manner and subjected to the same after-treatmetn as Reference Example 110 to give 2.5 g of a coarse product material of the intended compound as a colorless glassy solid.
Rf value (relative difference) (silica gel thin layer chromatography, n-hexane/ethyl acetate = 1/1): 0.25 (tailing).

### Reference Example 157

### 2-butyl-4- {3-(N-t-butoxycarbonyl-N-methylamino)-4-nitrophenylthiomethyl}-1-[2'-(1-triphenylmethyl-1H-tetrazol-5-yl)biphenyl-4-ylmethyl]-1H-imidazole-5-carboxylic acid ethyl ester

Using 2.0 g of potassium carbonate, 2.4 g of 2-butyl-4-mercaptomethyl-1-[2'-(1-triphenylmethyl-1H-tetrazol-5-yl)biphenyl-4-ylmethyl]-1H-imidazole-5-carboxylic acid ethyl ester (the compound of Reference Example 156), 15 mL of dry dimethylformamide, and 1.5 g of 4-chloro-2-(N-t-butoxycarbonyl-N-methylamino)nitrobenzene, these were reacted and refined in the same manner as Reference Example 111 to give 1.71 g of a coarse product material of the intended compound as a light yellow glassy solid.
Rf value (relative difference) (silica gel thin layer chromatography, n-hexane/ethyl acetate = 2/1): 0.50.
Mass spectrum: 969 (M+1)⁺.
¹H-NMR spectra (400MHz, DMSO-d₆), δₚₚₘ : 0.7 4 (t, 3H), 1.15 (t, 3H), 1.22 (m, 2H), 1.41 -1.20 (d, 9 H) 1. 48 (m, 2H), 2.45 (t, 2H), 3 .21 (s, 3H), 4. 1 5 (q, 2H), 4.54 (s, 2H), 5. 50 (s, 2H), 6.82-7.89 (m, 26H).

### Reference Example 158

### 4-[4-amino-3-(N-t-butoxycarbonyl-N-methylamino)phenylthiomethyl]-2-butyl-1-[2'-(1-triphenylmethyl-1H-tetrazol-5-yl)biphenyl-4-ylmethyl]-1H-imidazole-5-carboxylic acid ethyl ester

Using 1.6 g of 2-butyl-4-{3-(N-t-butoxycarbonyl-N-methylamino)-4-nitrophenylthiomethyl}-1-[2'-(1-triphenylmethyl-1H-tetrazol-5-yl)biphenyl-4-ylmethyl]-1H-imidazole-5-carboxylic acid ethyl ester (the compound of Reference Example 157), 15 mL of methanol, 5 mL of tetrahydrofuran, 2.0 g of 10% palladium on carbon, and hydrogen, these were reacted and refined in the same manner as Reference Example 112 to give 0.33 g of the intended compound.
Rf value (relative difference) (silica gel thin layer chromatography, n-hexane/ethyl acetate = 1/1): 0.47.

### Reference Example 159

### 2-butyl-4-{4--[4-(2,4-dioxothiazolidin-5-ylmethyl)phenoxyacetylamino]-3-(N-t-butoxycarbonyl-N-methylamino)phenylthiomethyl} -1-[2'-(1H-tetrazol-5-yl)biphenyl-4-ylmethyl]-1H-imidazole-5-carboxylic acid ethyl ester

Using 0.31 g of 4-[4-amino-3-(N-t-butoxycarbonyl-N-methylamino)phenylthiomethyl]-2-butyl-1-[2'-(1-triphenylmethyl-1H-tetrazol-5-yl)biphenyl-4-ylmethyl]-1H-imidazole-5-carboxylic acid ethyl ester (the compound of Reference Example 158), 0.12 g of 4-(2,4-dixothiazolidin-5-ylmethyl)phenoxyacetic acid, 1.5 mL of dry dimethylformamide, and 0.075 g of WSC (1-ethyl-3-(3-dimethylaminopropyl)carbodiimide hydrochloride), these were reacted and refined in the same manner as Reference Example 113 to give 0.22 g of the intended compound.
Rf value (relative difference) (silica gel thin layer chromatography, n-hexane/ethyl acetate = 1/1): 0.24.
Mass spectrum: 1202 (M+1)⁺.

### Reference Example 160

### 4-(2-chloropropan-2-yl)-2-propyl-1-[2'-(1-triphenylmethyl-1H-tetrazol-5-yl)biphenyl-4-ylmethyl]-1H-imidazole-5-carboxylic acid ethyl ester

Using 4-(2-hydroxypropan-2-yl)-2-propyl-1-[2'-(1-triphenylmethyl-1H-tetrazol-5-yl)biphenyl-4-ylmethyl]-1H-imidazole-5-carboxylic acid ethyl ester, dry methylene chloride, methanesulfonyl chloride, N,N-dimethylformamide, and anhydrous potassium carbonate, these were reacted and refined in the same manner as Reference Example 61 to give the intended compound.

### Reference Example 161

### 4-[2-(2-hydroxyethylthio)propan-2-yl]-2-propyl-1-[2'-(1-triphenylmethyl-1H-tetrazol-5-yl)biphenyl-4-ylmethyl]-1H-imidazole-5-carboxylic acid ethyl ester

Using 4-(2-chloropropan-2-yl)-2-propyl-1-[2'-(1-triphenylmethyl-1H-tetrazol-5-yl)biphenyl-4-ylmethyl]-1H-imidazole-5-carboxylic acid ethyl ester (the compound of Reference Example 160), dry dimethylformamide, and 2-mercaptoethanol, these were reacted and refined in the same manner as Reference Example 62 to give the intended compound.

### Reference Example 162

### 4-[2-[2-(3-(N-t-butoxycarbonyl-N-methylamino)-4-nitrophenoxy)ethylthio]propan-w-yl]-2-propyl-1-[2'-(1-triphenylmethyl-1H-tetrazol-5-yl)biphenyl-4-ylmethyl]-1H-imidazole-5-carboxylic acid ethyl ester

Using 4-[2-(2-hydroxyethylthio)propan-2-yl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 161), dry dimethylformamide, sodium hydride (purity: 60%), and 4-chloro-2-(N-t-butoxycarbonyl-N-methylamino)nitrobenzene, these were reacted and refined in the same manner as Reference Example 67 to give the intended compound.

### Reference Example 163

### 4-[2-[2-(4-amino-3-(N-t-butoxycarbonyl-N-methylamino)phenoxy)ethylthio]propan-2-yl]-2-propyl-1-[2'-(1-triphenylmethyl-1H-tetrazol-5-yl)biphenyl-4-ylmethyl]-1H-imidazole-5-carboxylic acid ethyl ester

Using 4-[2-[2-(3-(N-t-butoxycarbonyl-N-methylamino)-4-nitrophenoxy)ethylthio]propan-w-yl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 162), 10% palladium on carbon, and hydrogen gas, these were reacted and refined in the same manner as Reference Example 68 to give the intended compound.

### Reference Example 164

### 4-[2-[2-[3-(N-t-butoxycarbonyl-N-methylamino)-4-{4-(2,4-dioxothiazolidin-5-ylmethyl)phenoxyacetylamino}phenoxy]ethylthio]propan-2-yl]-2-propyl-1-[2'-(1H-tetrazol-5-yl)biphenyl-4-ylmethyl]-1H-imidazole-5-carboxylic acid ethyl ester

Using 4-[2-[2-(4-amino-3-(N-t-butoxycarbonyl-N-methylamino)phenoxy)ethylthio]propan-2-yl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 163), dry dimethylformamide, 4-(2,4-dixothiazolidin-5-ylmethyl)phenoxyacetic acid, and WSC (1-ethyl-3-(3-dimethylaminopropyl)carbodiimide hydrochloride), these were reacted and refined in the same manner as Reference Example 69 to give the intended compound.

### Reference Example 165

### 3-(4-t-butoxycarbonylmethoxyphenyl)-2-(3-chlorobenzyloxy)propionic acid methyl

A solution of 1.0 g of 4-t-butoxycarbonylmethoxyphenyllactic acid methyl (the compound of Reference Example 119) and 0.72 g of 3-chlorobenzyl bromide in 20 mL of N,N-dimethylformamide was combined with 0.23 g of tetrabutylammonium iodide (20% mol) and 0.154 g of sodium hydride (60%), and stirred at room temperature twelve hours. The reaction mixture was combined with water, and extracted with ethyl acetate. the extract was washed with water and dried over anhydrous sodium sulfate. The solvent was distilled off, and the residue was refined by silica gel column chromatography (elution solvent: n-hexane/ethyl acetate = 5/1) to give 0.80 g of the intended compound as a solid.
Rf value (relative difference) (silica gel thin layer chromatography, hexane/ethyl acetate = 5/1): 0.30.

### Reference Example 166

### 3-(4-carboxymethoxyphenyl)-2-(3-chlorobenzyloxy)propionic acid methyl

Using 0.80 g of 3-(4-t-butoxycarbonylmethoxyphenyl)-2-(3-chlorobenzyloxy)propionic acid methyl (the compound of Reference Example 165) and 20 mL of a 4 N hydrogen chloride-dioxane solution, these were reacted and refined in the same manner as Reference Example 121 to give 0.70 g of the intended compound.

### Reference Example 167

### 3-[4-[4-(5-ethoxycarbonyl-1-{2'-(1-triphenylmethyl-1H-tetrazol-5-yl)biphenyl-4-ylmethyl}-2-propyl-1H-imidazol-4-ylmethylthio)-2-(N-t-butoxycarbonyl-N-methylamino)phenylaminocarbonylmethoxy]phenyl]-2-(3-chlorobenzyloxy)propionic acid methy

Using 412 mg of 4-{4-amino-3-(N-t-butoxycarbonyl-N-methylamino)phenylthiomethyl}-2-propyl-1-[2'-(1-triphenylmethyl-1H-tetrazol-5-yl)biphenyl-4-ylmethyl]-1H-imidazole-5-carboxylic acid ethyl ester (the 1-triphenylmethyl-1H-tetrazole form (b) of the compound of Reference Example 112), 340 mg of 3-(4-carboxymethoxyphenyl)-2-(3-chlorobenzyloxy)propionic acid methyl (the compound of Reference Example 166), 30 mL of ethanol, and 250 mg of 1,1'-carbonylbis-1H-imidazole; 4-(4,6-dimethoxy-1,3,5-triadin-2-yl)-4-methylmorpholinium chloride (DMTMM), these were reacted and refined in the same manner as Reference Example 122 to give 0.22 g of the intended compound.
Rf value (relative difference) (silica gel thin layer chromatography, n-hexane/ethyl acetate = 3/2): 0.40.

### Reference Example 168

### 3-(4-t-butoxycarbonylmethoxyphenyl)-2-(4-methoxybenzyloxy)propionic acid methyl

Using 4-t-butoxycarbonylmethoxyphenyllactic acid methyl (the compound of Reference Example 119), 4-methoxybenzyl bromide, toluene, and silver oxide, these were reacted and refined in the same manner as Reference Example 120 to give the intended compound.

### Reference Example 169

### 3-(4-carboxymethoxyphenyl)-2-(4-methoxybenzyloxy)propionic acid methyl

Using 3-(4-t-butoxycarbonylmethoxyphenyl)-2-(4-methoxybenzyloxy)propionic acid methyl (the compound of Reference Example 168) and a 4 N hydrogen chloride-dioxane solution, these were reacted and refined in the same manner as Reference Example 121 to give the intended compound.

### Reference Example 170

### 3-[4-[4-(5-ethoxycarbonyl-1-{2'-(1-triphenylmethyl-1H-tetrazol-5-yl)biphenyl-4-ylmethyl}-2-propyl-1H-imidazol-4-ylmethylthio)-2-(N-t-butoxycarbonyl-N-methylamino)phenylaminocarbonylmethoxy]phenyl-2-(4-methoxybenzyloxy)propionic acid methyl

Using 4- {4-amino-3-(N-t-butoxycarbonyl-N-methylamino)phenylthiomethyl}-2-propyl-1-[2'-(1-triphenylmethyl-1H-tetrazol-5-yl)biphenyl-4-ylmethyl]-1H-imidazole-5-carboxylic acid ethyl ester (the 1-triphenylmethyl-1H-tetrazole form (b) of the compound of Reference Example 112), 3-(4-carboxymethoxyphenyl)-2-(4-methoxybenzyloxy)propionic acid methyl (the compound of Reference Example 169), ethanol, and 1,1'-carbonylbis-1H-imidazole; 4-(4,6-dimethoxy-1,3,5-triadin-2-yl)-4-methylmorpholinium chloride (DMTMM), these were reacted and refined in the same manner as Reference Example 122 to give the intended compound.

### Reference Example 171

### 4-[1-(4-hydroxyphenylthio)ethyl]-1-[2'-(1-triphenylmethyl-1H-tetrazol-5-yl)biphenyl-4-ylmethyl]-1H-imidazole-5-carboxylic acid ethyl ester

A solution of 1.17 g of 4-(1-hydroxyethyl)-1-[2'-(1-triphenylmethyl-1H-tetrazol-5-yl)biphenyl-4-ylmethyl]-1H-imidazole-5-carboxylic acid ethyl ester in 3.5 mL of dry methylene chloride was combined with 0.12 mL of thionyl chloride and one drop of N,N-dimethylformamide, and stirred at room temperature overnight. The reaction mixture was combined with 0.30 g of 4-mercaptophenol and stirred at room temperature for two hours. The reaction mixture was combined with ethyl acetate and water, and the ethyl acetate layer was separated. The ethyl acetate layer was washed with saturated physiological saline, then dried over anhydrous sodium sulfate. Distilling off the solvent gave 1.56 g of a coarse product material of the intended compound as a glassy solid.
Rf value (relative difference) (silica gel thin layer chromatography, n-hexane/ethyl acetate = 3/1): 0.52.
Mass spectrum: 811 (M+1)⁺.

### Reference Example 172

### 4-[1-[4-{3-(N-t-butoxycarbonyl-N-methylamino)-4-nitrophenoxy}phenylthio]ethyl]-1-[2'-(1-triphenylmethyl-1H-tetrazol-5-yl)biphenyl-4-ylmethyl]-1H-imidazole-5-carboxylic acid ethyl ester

Using 4-[1-(4-hydroxyphenylthio)ethyl]-1-[2'-(1-triphenylmethyl-1H-tetrazol-5-yl)biphenyl-4-ylmethyl]-1H-imidazole-5-carboxylic acid ethyl ester (the compound of Reference Example 171), potassium carbonate, N,N-dimethylformamide, and 4-chloro-2-(N-t-butoxycarbonyl-N-methylamino)nitrobenzene, these were reacted and refined in the same manner as Reference Example 64 to give the intended compound.

### Reference Example 173

### 4-[1-[4-{4-amino-3-(N-t-butoxycarbonyl-N-methylamino)phenoxy}phenylthio]ethyl]-1-[2'-(1-triphenylmethyl-1H-tetrazol-5-yl)biphenyl-4-ylmethyl]-1H-imidazole-5-carboxylic acid ethyl ester

Using 4-[1-[4-{3-(N-t-butoxycarbonyl-N-methylamino)-4-nitrophenoxy}phenylthio]ethyl]-1-[2'-(1-triphenylmethyl-1H-tetrazol-5-yl)biphenyl-4-ylmethyl]-1H-imidazole-5-carboxylic acid ethyl ester (the compound of Reference Example 172), methanol, tetrahydrofuran, 10% palladium on carbon, and hydrogen gas, these were reacted and refined in the same manner as Reference Example 68 to give the intended compound.

### Reference Example 174

### 4-[1-[4-{4-[4-(2,4-dioxothiazolidin-5-ylmethyl)phenoxyacetylamino]-3-(N-t-butoxycarbonyl-N-methylamino)phenoxy}phenylthio]ethyl]-1-[2'-(1-triphenylmethyl-1H-tetrazol-5-yl)biphenyl-4-ylmethyl]-2-propyl-1H-imidazole-5-carboxylic acid ethyl ester

Using 4-[1-[4-{4-amino-3-(N-t-butoxycarbonyl-N-methylamino)phenoxy}phenylthio]ethyl]-1-[2'-(1-triphenylmethyl-1H-tetrazol-5-yl)biphenyl-4-ylmethyl]-1H-imidazole-5-carboxylic acid ethyl ester (the compound of Reference Example 173), 4-(2,4-dixothiazolidin-5-ylmethyl)phenoxyacetic acid, dry N,N-dimethylformamide, and WSC (1-ethyl-3-(3-dimethylaminopropyl)carbodiimide hydrochloride), these were reacted and refined in the same manner as Reference Example 69 to give the intended compound.

### Reference Example 175

### 4-[2-(N-methylamino)-3-nitropyridin-6-ylthiomethyl]-2-propyl-1-[2'-(1-triphenylmethyl-1H-tetrazol-5-yl)biphenyl-4-ylmethyl]-1H-imidazole-5-carboxylic acid ethyl ester

A mixture of 4.0 g of 4-mercaptomethyl-2-propyl-1-[2'-(1-triphenylmethyl-1H-tetrazol-5-yl)biphenyl-4-ylmethyl]-1H-imidazole-5-carboxylic acid ethyl ester (the compound of Reference Example 110), 1.54 g of 2-(N-methylamino-3-nitro-6-chloropyridine, 2.0 g of anhydrous potassium carbonate, and 30 mL of dry dimethylformamide was stirred at room temperature for four hours. The reaction mixture was combined with ethyl acetate and water, and the ethyl acetate layer was separated. The ethyl acetate layer was washed with saturated physiological saline, then dried over anhydrous sodium sulfate. After the solvent had been distilled off, 5.3 g of the resulting residue was refined by silica gel column chromatography (elution solvent: n-hexane/ethyl acetate = 3/1) to give 5.0 g of the intended compound as a yellow powder.
Rf value (relative difference) (silica gel thin layer chromatography, n-hexane/ethyl acetate = 2/1): 0.39.

### Reference Example 176

### 4-[3-amino-2-(N-methylamino)pyridin-6-ylthiomethyl]-2-propyl-1-[2'-(1-triphenylmethyl-1H-tetrazol-5-yl)biphenyl-4-ylmethyl]-1H-imidazole-5-carboxylic acid ethyl ester

A mixture of 5.0 g of 4-[2-(N-methylamino)-3-nitropyridin-6-ylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 175), 30 mL of methanol, and 30 mL of tetrahydrofuran was purged with argon, then combined with 5.1 g of 10% palladium on carbon and purged with hydrogen gas at 50°C for one day. Insoluble matter was filtered off from the reaction mixture, and the filtrate was condensed to give 4.2 g of a coarse product material of the intended compound as a light blue glassy powder.

### Reference Example 177

### 4-[3-[4-(2,4-dixothiazolidin-5-ylmethyl)phenoxyacetylamino]-2-(N-methylamino)pyridin-6-ylthiomethyl]-2-propyl-1-[2'-(1H-tetrazol-5-yl)biphenyl-4-ylmethyl]-1H-imidazole-5-carboxylic acid ethyl ester

A solution of 4.2 g of 4-[3-amino-2-(N-methylamino)pyridin-6-ylthiomethyl]-2-propyl-1-[2'-(1-triphenylmethyl-1H-tetrazol-5-yl)biphenyl-4-ylmethyl]-1H-imidazole-5-carboxylic acid ethyl ester (the compound of Reference Example 176) and 1.64 g of 4-(2,4-dixothiazolidin-5-ylmethyl)phenoxyacetic acid in 7 mL of dry N,N-dimethylformamide was combined with 1.12 g of WSC (1-ethyl-3-(3-dimethylaminopropyl)carbodiimide hydrochloride) and stirred at room temperature for three days. The reaction mixture was combined with ethyl acetate and water, and the ethyl acetate layer was separated. The ethyl acetate layer was washed with saturated physiological saline, then dried over anhydrous sodium sulfate. Activated carbon was filtered off, the solvent was distilled off from the filtrate, and 5.2 g of the resulting residue was refined by silica gel column chromatography (elution solvent: methylene chloride/methanol = 30/1) to give 1.7 g of the intended compound as a light red glassy solid.
Rf value (relative difference) (silica gel thin layer chromatography, methylene chloride/methanol = 30/1): 0.17.
¹H-NMR spectra (400MHz, DMSO-d₆), δₚₚₘ : 0.8 5(t, 3H), 1.18(t , 3H), 1.55 (m, 2H), 2.64 (t, 2H), 3.08(q, 1H), 3.33 (q, 1H), 4. 18 ( q, 2H), 4.61 (s, 2H), 4. 65 (s, 2H), 4. 90 (q , 1H), 5.57 (s, 2H), 6. 50-7. 6 6 (m, 14H), 9 31 (s, 1H), 1 2. 03 (s, 1H).

### Reference Example 178

### 4-(N-ethoxycarbonylmethyl-N-4-methoxyphenoxycarbonylaminomethyl)phenoxyacetic acid t-butyl

Using 1.0 g of 4-ethoxycarbonylmethylaminomethylphenoxyacetic acid t-butyl (the compound of Reference Example 124), 30 mL of methylene chloride, 0.47 g triethylamine, and 0.69 g of 4-methoxyphenyl chloroformate, these were reacted and refined in the same manner as Reference Example 125 to give 0.98 g of the intended compound.
Rf value (relative difference) (silica gel thin layer chromatography, hexane/ethyl acetate = 3/1): 0.30.

### Reference Example 179

### 4-(N-ethoxycarbonylmethyl-N-4-methoxyphenoxycarbonylaminomethyl)phenoxyacetic acid

Using 0.98 g of 4-(N-ethoxycarbonylmethyl-N-4-methoxyphenoxycarbonylaminomethyl)phenoxyacetic acid t-butyl (the compound of Reference Example 178) and 10 mL of a 4 N hydrogen chloride-dioxane solution, these were reacted and refined in the same manner as Reference Example 126 to give 0.90 g of the intended compound.

### Reference Example 180

### N-[4-{4-[5-ethoxycarbonyl-1-{2'-(1-triphenylmethyl-1H-tetrazol-5-yl)biphenyl-4-ylmethyl}-2-propyl-1H-imidazol-4-ylmethylthio]-2-(N-t-butoxycarbonyl-N-methylamino)phenylaminocarbonylmethoxy}phenylmethyl]-N-4-methoxyphenoxycarbonylaminoacetic acid ethyl

Using 412 mg of 4-{4-amino-3-(N-t-butoxycarbonyl-N-methylamino)phenylthiomethyl}-2-propyl-1-[2'-(1-triphenylmethyl-1H-tetrazol-5-yl)biphenyl-4-ylmethyl]-1H-imidazole-5-carboxylic acid ethyl ester (the 1-triphenylmethyl-1H-tetrazole form (b) of the compound of Reference Example 112), 390 mg of 4-(N-ethoxycarbonylmethyl-N-4-methoxyphenoxycarbonylaminomethyl)phenoxyacetic acid (the compound of Reference Example 179), 30 mL of ethanol, and 250 mg of 1,1'-carbonylbis-1H-imidazole; 4-(4,6-dimethoxy-1,3,5-triadin-2-yl)-4-methylmorpholinium chloride (DMTMM), these were reacted and refined in the same manner as Reference Example 127 to give 250 mg of the intended compound.
Rf value (relative difference) (silica gel thin layer chromatography, hexane/ethyl acetate = 3/2); 0.40

### Reference Example 181

### 6-(2-hydroxyethyl)-2-propyl-4,7-dioxo-5,6-dihydro-1H-imidazo[4,5-d]pyridazine

A mixture of 1.06 g of 2-propyl-1H-imidazole-4,5-dicarboxylic acid and 5 mL of methylene chloride was combined with 1.78 g of thionyl chloride and one drop of dry N,N-dimethylforammide, and heat-refluxed for one day. The solvent was distilled off from the reaction mixture, and the residue was dried under reduced pressure. The residue was combined with 15 mL of dry tetrahydrofuran and 3 mL of dry N,N-dimethylformamide, then combined with 0.38 g of hydrazinoethanol followed by 1.2 g of triethylamine, and stirred at room temperature for two days. The solvent was distilled off, and the residue was refined by silica gel column chromatography (elution solvent: methanol/ethyl acetate = 1/4) to give 0.60 g of the intended compound as a colorless solid.
Mass spectrum: 239 (M+1)⁺.
¹H-NMR spectra (400MHz, DMSO-d₆), δₚₚₘ : 0.8 9 (t, 3H), 1. 7 5 (m, 2H), 2.7 5 (t, 2H), 3. 6 7 (t, 2H), 4.03 (t, 2H).

### Reference Example 182

### 5-(2-hydroxyethyl)-2-propyl-1-{2'-(1-triphenylmethyl-1H-tetrazol-5-yl)biphenyl-4-ylmethyl}-4,7-dixo-5,6-dihydro-1H-imidazo[4,5-d]pyridine (5-substituted form) and 6--(2-hydroxyethyl)-2-propyl-1-{2'-(1-triphenylmethyl-1H-tetrazol-5-yl)biphenyl-4-ylmethyl}-4,7-dixo-5,6-dihydro-1H-imidazo[4,5-d]pyridine (6-substituted form)

Using 6-(2-hydroxyethyl)-2-propyl-4,7-dioxo-5,6-dihydro-1H-imidazo[4,5-d]pyridazine (the compound of Reference Example 181), 60% sodium hydride, and 4'-bromomethylbiphenyl-2-(1-triphenylmethyl-1H-tetradazole, these were reacted and refined in the same manner as Reference Example 44 to give the 5-substituted form and the 6-substituted form of the intended compound. This mixture was separated by liquid column chromatography (elution solvent: acetonitrile/water).

### Reference Example 183

### 5-[2-{3-(N-t-butoxycarbonyl-N-methylamino)-4-nitrophenoxy}ethyl]-2-propyl-1-{2'-(1-triphenylmethyl-1H-tetrazol-5-yl)biphenyl-4-ylmethyl}-4,7-dioxo-5,6-dihydro-1H-imidazo[4,5-3]pyridine (5-substituted form) and 6-[2-{3-(N-t-butoxycarbonyl-N-methylamino)-4-nitrophenoxy}ethyl]-2-propyl-1-{2'-(1-triphenylmethyl-1H-tetrazol-5-yl)biphenyl-4-ylmethyl}-4,7-dioxo-5,6-dihydro-1H-imidazo[4,5-d]pyridine (6-substituted form)

Using 5-(2-hydroxyethyl)-2-propyl-1-{2'-(1-triphenylmethyl-1H-tetrazol-5-yl)biphenyl-4-ylmethyl}-4,7-dixo-5,6-dihydro-1H-imidazo[4,5-d]pyridine (the 5-substituted form of the compound of Reference Example 182), dry dimethylformamide, 60% sodium hydride, dry tetrahydrofuran, and 4-chloro-2-(N-t-butoxycarbonyl-N-methylamino)nitrobenzene, these were reacted and refined in the same manner as Reference Example 2 to give the 5-substituted form of the intended compound.
Using the 6-substituted form of Reference Example 182, the same procedure was followed to give the 6-substituted form of the intended compound.

### Reference Example 184

### 5-[2-{4-amino-3-(N-t-butoxycarbonyl-N-methylamino)phenoxy}ethyl]-2-propyl-1-{2'-(1-triphenylmethyl-1H-tetrazol-5-yl)biphenyl-4-ylmethyl}-4,7-dioxo-5,6-dihydro-1H-imidazo[4,5-d]pyridine (5-substituted form) and 6-[2-{4-amino-3-(N-t-butoxycarbonyl-N-methylamino)phenoxy}ethyl]-2-propyl-1-{2'-(1-triphenylmethyl-1H-tetrazol-5-yl)biphenyl-4-ylmethyl}-4,7-dioxo-5,6-dihydro-1H-imidazo[4,5-d]pyridine (6-substituted form)

Using 5-[2-{3-(N-t-butoxycarbonyl-N-methylamino)-4-nitrophenoxy}ethyl]-2-propyl-1-{2'-(1-triphenylmethyl-lH-tetrazol-5-yl)biphenyl-4-ylmethyl}-4,7-dioxo-5,6-dihydro-1H-imidazo[4,5-3]pyridine (the 5-substituted form of the compound of Reference Example 183), methanol, tetrahydrofuran, 10% palladium on carbon, and hydrogen gas, these were reacted and refined in the same manner as Reference Example 68 to give the 5-substituted form of the intended compound.
Using the 6-substituted form of Reference Example 183, the same procedure was followed to give the 6-substituted form of the intended compound.

### Reference Example 185

### 5-[4-[4- {2-(1-{2'-(1-triphenylmethyl-1H-tetrazol-5-yl)biphenyl-4-ylmethyl}-4,7-dioxo-2-propyl-5,6-dihydro-1H-imidazo[4,5-d]pyridazin-5-yl)ethoxy}-3-(N-t-butoxycarbonyl-N-methylamino)phenylaminocarbonylmethoxy]benzyl]thiazolidine-2,4-dione (5-substituted form) and 5-[4-[4-{2-(1-{2'-(1-triphenylmethyl-1H-tetrazol-5-yl)biphenyl-4-ylmethyl}-4,7-dioxo-2-propyl-5,6-dihydro-1H-imidazo[4,5-d]pyridazin-6-yl)ethoxy}-3-(N-t-butoxycarbonyl-N-methylamino)phenylaminocarbonylmethoxy]benzyl]thiazolidine-2,4-dione (6-substituted form)

Using 5-[2-{4-amino-3-(N-t-butoxycarbonyl-N-methylamino)phenoxy}ethyl]-2-propyl-1-{2'-(1-triphenylmethyl-1H-tetrazol-5-yl)biphenyl-4-ylmethyl}-4,7-dioxo-5,6-dihydro-1H-imidazo[4,5-d]pyridine (the 5-substituted form of the compound of Reference Example 184), 4-(2,4-dixothiazolidin-5-ylmethyl)phenoxyacetic acid, dry N,N-dimethylformaide, and WSC (1-ethyl-3-(3-dimethylaminopropyl)carbodiimide hydrochloride), these were reacted and refined in the same manner as Reference Example 69 to give the 5-substituted form of the intended compound.
Using the 6-substituted form of Reference Example 184, the same procedure was followed to give the 6-substituted form of the intended compound.

### Test Example

### Angiotensin II inhibition test

A test of inhibition of angiotensin II to human recombinant (CHO cell) AT1 receptor following a method described in the literature (for example, Regul. Pept., vol. 44 (2), page 131 (1993)) was used to measure inhibitory activity to a test compound.
The ligand used was 10⁻⁹ M of a test compound dissolved in 1% DMSO (dimethylsulfoxide). This was reacted with radioactive iodine-labeled angiotensin II at 37°C for sixty minutes. Taking the inhibition rate of angiotensin II at 10 µM as nonspecific binding, the specific binding rate was calculated by subtracting this nonspecific portion. Table 6 indicates the results (the inhibition rate at test compound concentration: 10⁻⁹ M)

**TABLE 6**

| Test compound | Inhibition rate (%) |
|---|---|
| Compound of Example 1 | 51 |
| Compound of Example 3 | 60 |
| Compound of Example 7 | 52 |
| Compound of Example 8 | 73 |
| Compound of Example 10 | 41 |
| Compound of Example 14 | 38 |
| Compound of Example 15 | 47 |
| Compound of Example 19 | 65 |
| Compound of Example 22 | 60 |
| Compound of Example 23 | 22 |
| Compound of Example 25 | 32 |
| Compound of Example 26 | 44 |
| Compound of Example 27 | 66 |
| Compound of Example 28 | 66 |
| Compound of Example 29 | 40 |
| Compound of Example 30 | 66 |
| Compound of Example 31 | 22 |
| Compound of Example 33 | 87 |
| Compound of Example 35 | 90 |
| Compound of Example 36 | 83 |

The compounds of the present application which were tested exhibited an excellent angiotensin II inhibition rate of 22% to 90% at the test compound concentration (10⁻⁹ M).

### Test Example 2

### Glucose tolerance test (in vivo) and weight gain test (in vivo)

A test compound (the compound of Example 8, n = 8) was given orally to 5- to 6-week-old ob/ob mice (C57b1/6ob/ob female mice) at a dose of 10 mg/kg for 1-25 days, then a test compound (the compound of Example 8) was given at 20 mg/kg for 26-33 days. An oral load test of 2 g/kg of glucose was carried out while the mice were fasted, and the blood glucose concentration was measured at -30, 0, 30, 60, 90, 120, and 180 minutes. For comparison, 3 mg/kg of rosiglitazone (n = 8) were given for 1-25 days, then 3 mg/kg of rosiglitazone were given for 26-33 days, and the blood glucose concentration was measured in the same manner. Table 7 indicates the results.

**TABLE 7**

| Test compound | Glucose concentration (mmol/L) | | | | | |
|---|---|---|---|---|---|---|
| | -30 | 0 | 30 | 60 | 120 | 180 (min) |
| Compound of Example 8 | | | | | | |
| | 7.6±0.9 | 6.4±0.6 | 12.5±0.9 | 7.3±0.9 | 5.5±0.6 | 6.1±0.6 |
| Rosiglitazone | | | | | | |
| | 4.6±0.3 | 4.6±0.3 | 12.5±0.9 | 4.9±0.3 | 4.6±0.1 | 4.6±0.1 |
| Control | 9.5±1.2 | 9.8±1.5 | 18.6±1.8 | 14.0±2.4 | 9.5±2.0 | 8.2±1.5 |

The compound of the present application (the compound of Example 8) significantly inhibited increase in blood glucose concentration compared to the control.
The body weight of mice was measured when 10 mg/kg of the same test compound (the compound of Example 8) were given for 1-25 days, and 3 mg/kg of rosiglitazone were given for 1-25 days. Table 8 indicates the results.

**TABLE 8**

| Test compound | Weight gain (g) | | | | | |
|---|---|---|---|---|---|---|
| | 0 | 4 | 11 | 14 | 18 | 24 (days) |
| Compound of Example 8 | 0 | 0.8±0.0 | 3.3±0.2 | 4.2±0.3 | 5.9±0.3 | 6.8±0.2 |
| | | | | | | |
| Rosiglitazone | 0 | 1.2±0.1 | 4.5±0.3 | 5.8±0.1 | 7.5±0.2 | 8.9±0.2 |
| | | | | | | |
| Control | 0 | 1.0±0.0 | 3.8±0.1 | 4.5±0.3 | 6.4±0.2 | 6.8±0.2 |

The compound of the present application (the compound of Example 8) exhibited nearly comparable weight gain to the control, and significantly inhibited weight gain compared to rosiglitazone.

### Test Example 3

### PPAR agonist activity measurement test

Agonist activity to the human nuclear receptors PPARα, β, and γ was measured using a biochemical fluorescent resonance energy transfer (FRET) assay system, which is a cell-free assay system, following a method described in the literature (for example, J. Biol. Chem., vol. 281 (8), pages 4920-4930 (2006)).
Specifically, after an N-terminal biotin-labeled peptide having cofactor peptide SRC1 (amino acid sequence 676-700), which includes the nuclear receptor bonding LXXLL motif region, and the fluorescent fusion proteins of the PPAR ligand binding domain were allowed to stand at room temperature for one hour, the energy transfer between the fluorescence donor and the fluorescence acceptor was measured to detect the test compound dose reaction to the cofactor.
A Perkin-Elmer Envision Multilabel Plate Reader was used for measurement. During measurement, 320 nm was used as the excitation wavelength, and 615 nm and 665 nm were used as the emission wavelengths. The test compound was dissolved in DMSO (dimethylsulfoxide) to a final solution concentration of 1%. The measurement concentrations were the twelve points of 50 µM, 16.7 µM, 5.6 µM, 1.9 µM, 617 nM, 206 nM, 69 nM, 23 nM, 7.6 nM, 2.5 nM, 0.85 nM, and 0 nM, and ED₅₀ = 114 nM was calculated.
As a result, the test compound (the compound of Example 8) exhibited activity only against PPARγ, and did not exhibit activity against PPARα and PPARβ.

### Formulation Example 1

### Tablet

Tablets of the compound of Example 8 were obtained by the following method using the types and quantities of ingredients indicated in Table 9.
Lactose (an excipient) and croscarmellose sodium (Ac-Di-Sol: a disintegrant) were added to the compound of Example 8 and mixed using a high-speed stirring granulator. This mixture was combined with an aqueous solution of hydroxypropylcellulose (a binder) and kneaded to give a granulated substance. This granulated substance was dried using a fluid bed dryer, and the dried granulated substance was forcibly passed through a screen using a crushing granulation sizing machine. The granulated substance was then combined with magnesium stearate (a lubricant) and mixed using a V-form mixer. This mixture was molded using a 7-mm diameter frame, and the molded product was sprayed with an Opadry White aqueous solution (coating agent) containing dispersed yellow iron sesquioxide using a coating machine to give the desired tablets.

**TABLE 9**

| Ingredient | Quantity per tablet (mg) |
|---|---|
| Compound of Example 8 | 10.9 |
| Lactose | 94.4 |
| Ac-Di-Sol | 19.5 |
| Hydroxypropylcellulose | 3.9 |
| Magnesium stearate | 1.3 |
| Opadry White | 5.972 |
| Iron sesquioxide | 0.028 |
| | 136.0 |

### INDUSTRIAL APPLICATION

The benzo- or pyrido-imidazole derivative (I), pharmaceutically acceptable salt thereof, or pharmaceutically acceptable ester or amide thereof of the present invention has a specific chemical structure, and as a result, has excellent PPAR activation activity and excellent angiotensin II receptor antagonistic activity. Some compounds (especially benzylthiazolidone derivatives) have a selective PPARγ activation activity and effects such as a weight gain inhibiting effect and a body fat increase inhibiting effect, and are useful as pharmaceuticals having fewer adverse reactions and prophylactic and/or therapeutic agents for diseases such as diabetes, hyperlipidemia, obesity, glucose intolerance, hypertension, fatty liver, diabetes complications (for example, retinopathy, nephropathy, neurosis, cataracts, coronary disease, and stroke), arteriosclerosis, gestational diabetes, polycystic ovary syndrome, cardiovascular diseases (for example, ischemic heart disease), atheromatous arteriosclerosis or cellular damage caused by ischemic heart disease (for example, brain damage and the like occurring during stroke), gout, infectious diseases (for example, osteoarthritis, pain, fever, rheumatoid arthritis, infectious enteritis, acne, sunburn, psoriasis, eczema, allergic diseases, asthma, GI ulcer, cachexia, autoimmune diseases, and pancreatitis), cancer, osteoporosis, cataract, glaucoma, dry eye, Alzheimer's disease, uric acid inhibiting activity, hair growth promotion activity, and nonalcoholic steatohepatitis (NASH) activity (especially prophylactic and/or therapeutic agents for diabetes, diabetes complications, cancer, glaucoma, dry eye, Alzheimer's disease, hypertension, and hyperlipidemia).

The benzo- or pyrido-imidazole derivative (I), pharmaceutically acceptable salt thereof, or pharmaceutically acceptable ester or amide thereof of the present invention may form a pharmaceutical composition by combining with at least one of an RXR activator, a sulfonylurea agent, an α-glucosidase inhibitor, an insulin formulation, an aldose reductase inhibitor, biguanide, a DPP-IV inhibitor, a GLP-1-related compound, a statin compound, a squalene synthesis inhibitor, a fibrate compound, an LDL catabolism promoter, an angiotensin converting enzyme inhibitor, an angiotensis II receptor agonist, a renin inhibitor, a calcium antagonist, an aldosterone receptor agonist, a diuretic, insulin, a secretase inhibitor, an antitumor agent, a prophylactic and/or therapeutic agent of diabetes, and a prophylactic and/or therapeutic agent of diabetes complications (especially a statin compound, an angiotensin converting enzyme inhibitor, a renin inhibitor, a calcium agonist, an aldosterone receptor agonist, a diuretic, an antitumor agent, a prophylactic and/or therapeutic agent of diabetes, and a prophylactic and/or therapeutic agent of diabetes complications).

When used as a therapeutic or prophylactic agent as described earlier, the benzo- or pyrido-imidazole derivative (I), pharmaceutically acceptable salt thereof, or pharmaceutically acceptable ester or amide thereof of the present invention may be administered directly or suitably mixed with a pharmaceutically acceptable excipient, diluent, or the like to administer orally, for example, as a tablet, a capsule, a granule, a powder, or a syrup, or nonorally, for example, as an injection, a suppository, a transdermal absorbent, or the like.

These formulations are produced by a conventional method using additives such as an excipient (which may be, for example, an organic excipient, including a sugar derivative such as lactose, sucrose, glucose, mannitol, or sorbitol, a starch derivative such as cornstarch, potato starch, α starch, or dextrin, a cellulose derivative such as crystal cellulose, and gum arabic, dextran, and pullulan; or an inorganic excipient, including a silicate derivative such as light anhydrous silicic acid, synthetic aluminum silicate, calcium silicate, or magnesium aluminum metasilicate, a phosphate such as monobasic calcium phosphate, a carbonate such as calcium carbonate, or a sulfate such as calcium sulfate), a lubricant (which may be, for example, stearic acid or a metal stearate such as calcium stearate or magnesium stearate; talc; colloidal silica; a wax such as beeswax or spermaceti; boric acid; adipic acid; or sodium sulfate; glycol; fumaric acid; sodium benzoate; an amino acid such as DL leucine; peptide; a fatty acid sodium salt; a laurylsulfate such as sodium laurylsulfate or magnesium laurylsulfate; a silicic acid such as anhydrous silicic acid or silicic acid hydrate; or a starch derivative described earlier), a binder (which may be, for example, hydroxypropylcellulose, hydroxypropyl methylcellulose, polyvinylpyrrolidone, macrogol, or a compound similar to the excipients described earlier), a disintegrant (which may be, for example, a cellulose derivative such as low-substitution hydroxypropylcellulose, carboxymethylcellulose, calcium carboxymethylcellulose, or internally crosslinked sodium carboxymethylcellulose; or a chemically modified starch cellulose such as crosslinked polyvinylpyrrolidone), a stabilizer (which may be a paraoxybenzoic acid ester such as methylparaben or propylparaben; an alcohol such as chlorobutanol, benzyl alcohol, or phenyl ethyl alcohol; benzalkonium chloride; a phenol such as phenol or cresol; thimerosal; dehydroacetic acid; or sorbic acid), a flavoring agent (which may be, for example, any commonly used flavoring, acidifier, or spice), and/or a diluent or the like.
Although differing depending on factors such as the symptoms, the patient's age, and the route of administration, the dose of the benzo- or pyrido-imidazole derivative (I), pharmaceutically acceptable salt thereof, or pharmaceutically acceptable ester or amide thereof of the present invention may be, for example, a lower limit of 0.001 mg/kg body weight (preferably 0.01 mg/kg body weight) and an upper limit of 500 mg/kg body weight (preferably 50 mg/kg body weight) per dose when administered orally, and a lower limit of 0.005 mg/kg body weight (preferably 0.05 mg/kg body weight) and an upper limit of 50 mg/kg body weight (preferably 5 mg/kg body weight) per dose when administered intravenously. This dose is preferably administered once to several times daily depending on the symptoms.

## Claims

1. A benzo- or pyrido-imidazole derivative represented by general formula (I), a pharmaceutically acceptable salt thereof, or a pharmaceutically acceptable ester or amide thereof. 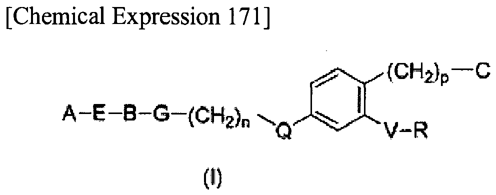 [where
A indicates the following (a)-(g):
(a) a group represented by the expression 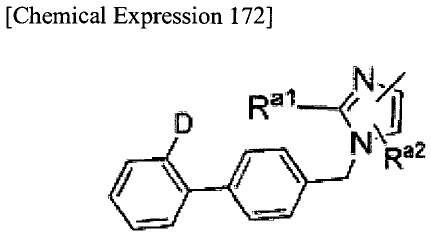 (where R^{a1} indicates the following (i)-(viii):
(i) a C₁-C₆ alkyl, (ii) a C₁-C₆ halogenoalkyl, (iii) a C₁-C₆ alkoxy, (iv) a C₁-C₆ alkylthio, (v) a group represented by the expression -N(R^{a3})(R^{a4}) (where R^{a3} indicates (i) hydrogen, (ii) a C₁-C₆ alkyl, (iii) a C₆-C₁₀ aryl optionally having one to five substituents α to be described later, or (iv) a C₆-C₁₀ aryl-C₁-C₆ alkyl optionally having in the aryl portion one to five substituents α to be described later, and R^{a4} indicates hydrogen or a C₁-C₆ alkyl), (vi) a C₃-C₁₀ cycloalkyl, (vii) a C₃-C₁₀ cycloalkyloxy, or (viii) a C₃-C₆ cycloalkylthio,
R^{a2} indicates the following (i)-(xii):
(i) hydrogen, (ii) a halogen, (iii) formyl, (iv) a C₁-C₁₀ alkylcarbonyl or a C₂-C₆ alkenylcarbonyl, (v) carboxyl, (vi) carbamoyl, (vii) a mono- or di-C₁-C₆ alkylcarbamoyl , (viii) amino, (ix) formylamino, a C₁-C₆ alkylcarbonylamino, or a C₂-C₆ alkenylcarbonylamino, (x) a C₁-C₆ hydroxyalkyl, (xi) a formyl-C₁-C₆ alkyl, or (xii) a C₁-C₆ alkylcarbonyl-C₁-C₆ alkyl,
D indicates the following (i)-(xi):
(i) carboxyl, (ii) 5-tetrazolyl, (iii) a group represented by the expression 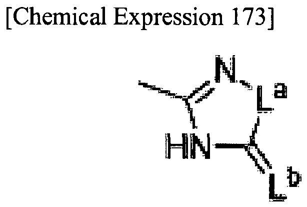 (where L^{a} and L^{b} indicate the same or different oxygen or sulfur), (iv) a group represented by the expression 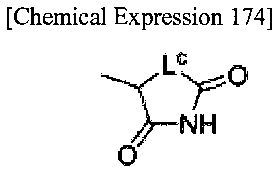 (where L^{c} indicates oxygen, sulfur, or a group represented by the expression -N(R^{a3})- (where R^{a3} is defined as described earlier), (v) a group represented by the expression CF₃SO₂-NH-, (vi) tetrazol-5-ylaminocarbonyl, (vii) a group represented by the expression 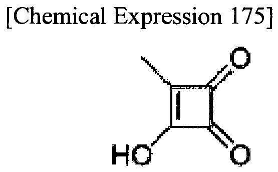
(viii) formylaminosulfonyl, a C₁-C₆ alkylcarbonylaminosulfonyl, or a C₂-C₆ alkenylcarbonylaminosulfonyl, (ix) a C₆-C₁₀ aryl carbonylaminosulfonyl optionally having in the aryl portion one to five substituents α to be described later, (x) a C₆-C₁₀ aryl-C₁-C₆ alkylcarbonylaminosulfonyl optionally having in the aryl portion one to five substituents α to be described later, or (xi) sulfo);
(b) a group represented by the expression 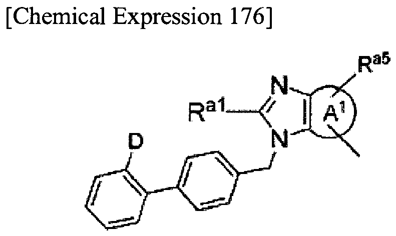 (where D and R^{a1} are defined as described earlier, the group represented by the expression 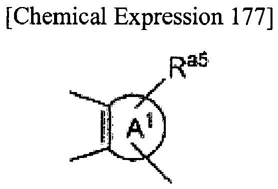 indicates a trivalent six-membered unsaturated hydrocarbon ring optionally having a substituent R^{a5}, optionally containing one to two atoms and/or groups selected from among oxygen, sulfur, the expression =N-R^{a3} (where R^{a3} is defined as described earlier), and sulfone (-S(O)₂-), and optionally having hydrogen substituted with oxo, a C₁-C₆ alkylidene, a C₁-C₆ alkylimino, hydroxyimino, or a C₁-C₆ alkoxyimino, and R^{a5} indicates (i) a group indicated by R^{a2}, (ii) a C₁-C₆ alkyl, (iii) a C₁-C₆ halogenoalkyl, (iv) a C₁-C₆ alkoxy, (v) a C₆-C₁₀ aryloxy, or (vi) a C₆-C₁₀ aryl-C₁-C₆ alkyloxy);
(c) a group represented by the expression 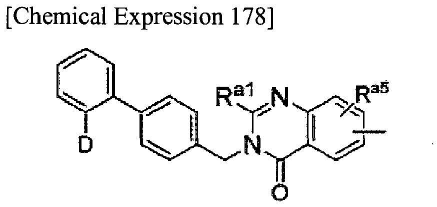 (where D, R^{a1}, and R^{a5} are defined as described earlier);
(d) a group represented by the expression 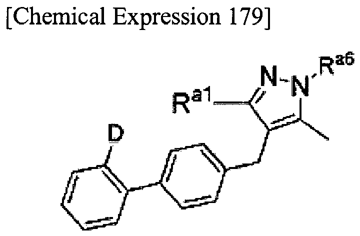 (where D and R^{a1} are defined as described earlier, and R^{a6} indicates hydrogen or a C₁-C₆ alkyl);
(e) a group represented by the expression 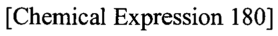 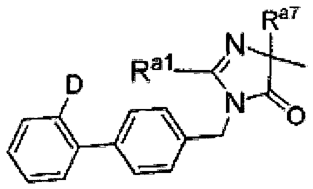 (where D and R^{a1} are defined as described earlier, and R^{a7} indicates hydrogen, a C₁-C₆ alkyl, or a C₃-C₁₀ cycloalkyl);
(f) a group represented by the expression 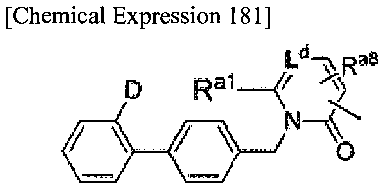 (where D and R^{a1} are defined as described earlier, R^{a8} indicates hydrogen or a C₁-C₆ alkyl, and L^{d} indicates the expression =CH- or =N-); or
(g) a group represented by the expression 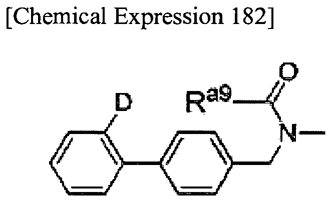 (where D is defined as described earlier, and R^{a9} indicates a C₁-C₆ alkyl, a C₁-C₆ halogenoalkyl, or a C₃-C₁₀ cycloalkyl),
B indicates a group represented by the expression 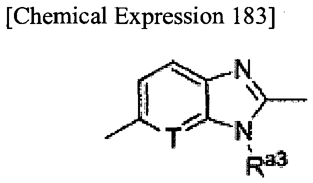
(where R^{a3} is defined as described earlier, and T indicates the expression =CH- or =N-), and B is bonded to E (or G) by a portion of an imidazole ring or a portion of a benzene or pyridine ring, C indicates the following (a)-(d):
(a) carboxyl;
(b) a group represented by the expression 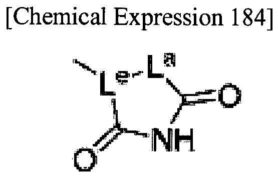 (where L^{a} is defined as described earlier, L^{e} indicates the expression =CH- or =N-, and L^{a} indicates oxygen when L^{e} indicates the expression =N-);
(c) a group represented by the expression -C(R^{c1})(COOH)-W^{c1}-R^{c2} (where R^{c1} and R^{c2} may be the same or different, and indicate the following (i)-(viii):
(i) hydrogen, (ii) a C₁-C₆ alkyl, (iii) a C₆-C₁₀ aryl optionally having one to five substituents α to be described later, (iv) a C₆-C₁₀ aryl-C₁-C₆ alkyl optionally having in the aryl portion one to five substituents α to be described later, (v) a C₁-C₆ alkylsulfonyl, (vi) a C₁-C₆ halogenoalkylsulfonyl, (vii) a C₆-C₁₀ aryl sulfonyl optionally having one to five substituents α to be described later, or (viii) a C₆-C₁₀ aryl-C₁-C₆ alkylsulfonyl optionally having in the aryl portion one to five substituents α to be described later; provided, however, that when W^{c1} to be described later indicates oxygen or sulfur, R^{c1} and R^{c2} indicate (i) hydrogen, (ii) a C₁-C₆ alkyl, (iii) a C₆-C₁₀ aryl optionally having one to five substituents α to be described later, or (iv) a C₆-C₁₀ aryl-C₁-C₆ alkyl optionally having in the aryl portion one to five substituents α to be described later,
W^{c1} indicates the following (i)-(iii):
(i) oxygen, (ii) sulfur, or (iii) a group represented by the expression -N(R^{C3})- (where R^{c3} indicates the following (i)-(xiii):
(i) hydrogen, (ii) a C₁-C₆ alkyl, (iii) a C₆-C₁₀ aryl optionally having one to five substituents α to be described later, (iv) a C₆-C₁₀ aryl-C₁-C₆ alkyl optionally having in the aryl portion one to five substituents α to be described later, (v) formyl, a C₁-C₆ alkylcarbonyl, or a C₂-C₆ alkenylcarbonyl, (vi) a C₆-C₁₀ aryl carbonyl optionally having one to five substituents α to be described later, (vii) a C₆-C₁₀ aryl-C₁-C₆ alkylcarbonyl optionally having in the aryl portion one to five substituents α to be described later, (viii) a C₃-C₁₀ cycloalkylcarbonyl, (ix) a five- to seven-membered cyclic heteroaryl carbonyl optionally having one to two substituents α to be described later and containing one to three heteroatoms selected from among a group consisting of oxygen, nitrogen, and sulfur, (x) carbamoyl optionally substituted with one to two substituents α to be described later (provided, however, that the substituents α may not be (iv) a halogen, (v) hydroxy, (vi) cyano, or (vii) nitro), (xi) a C₁-C₆ alkoxycarbonyl, (xii) a C₆-C₁₀ aryloxycarbonyl optionally having one to five substituents α to be described later, or (xiii) a C₆-C₁₀ aryl-C₁-C₆ alkoxycarbonyl optionally having one to five substituents α to be described later); or (d) a group represented by the expression -N(W^{c2})-CH₂COOH (where W^{c2} indicates the following (i)-(xiii):
(i) hydrogen, (ii) a C₁-C₆ alkyl, (iii) a C₆-C₁₀ aryl optionally having one to five substituents α to be described later, (iv) a C₆-C₁₀ aryl-C₁-C₆ alkyl optionally having in the aryl portion one to five substituents α to be described later, (v) formyl, a C₁-C₆ alkylcarbonyl, or a C₂-C₆ alkenylcarbonyl, (vi) a C₆-C₁₀ aryl carbonyl optionally having one to five substituents α to be described later, (vii) a C₆-C₁₀ aryl-C₁-C₆ alkylcarbonyl optionally having in the aryl portion one to five substituents α to be described later, (viii) a C₃-C₁₀ cycloalkylcarbonyl, (ix) a five- to seven-membered cyclic heteroaryl carbonyl optionally having one to two substituents α to be described later and containing one to three heteroatoms selected from among a group consisting of oxygen, nitrogen, and sulfur, (x) carbamoyl optionally substituted with one to two substituents α to be described later (provided, however, that the substituents α may not be (iv) a halogen, (v) hydroxy, (vi) cyano, or (vii) nitro), (xi) a C₁-C₆ alkoxycarbonyl, (xii) a C₆-C₁₀ aryloxycarbonyl optionally having one to five substituents α to be described later, or (xiii) a C₆-C₁₀ aryl-C₁-C₆ alkoxycarbonyl optionally having one to five substituents α to be described later),
E indicates the following (1) or (2):
(1) a group represented by the expression 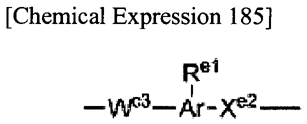 (where R^{e1} is an Ar (aryl or heteroaryl ring) substituent, of which there may be zero, one, or a same or different plurality (this plurality being the maximum number of substituents that an aryl or heteroaryl ring may have), R^{e1} indicates a substituent α to be described later, and W^{c3} indicates the following (a)-(h):
(a) oxygen, (b) a group represented by the expression -S(O)_{q}- (where q indicates 0 or an integer from 1 to 2), (c) a group represented by the expression -N(R^{e2})- (where R^{e2} indicates the following (i)-(xiv):
(i) hydrogen, (ii) a C₁-C₆ alkyl, (iii) a C₆-C₁₀ aryl optionally having one to five substituents α to be described later, (iv) a C₆-C₁₀ aryl-C₁-C₆ alkyl optionally having in the aryl portion one to five substituents α to be described later, (v) a C₁-C₆ alkylsulfonyl, (vi) a C₁-C₆ halogenoalkylsulfonyl, (vii) a C₆-C₁₀ aryl sulfonyl optionally having one to five substituents α to be described later, (viii) a C₆-C₁₀ aryl-C₁-C₆ alkylsulfonyl optionally having in the aryl portion one to five substituents α to be described later, (ix) formyl, a C₁-C₆ alkylcarbonyl, or a C₂-C₆ alkenylcarbonyl, (x) a C₆-C₁₀ aryl carbonyl optionally having one to five substituents α to be described later, (xi) a C₆-C₁₀ aryl-C₁-C₆ alkylcarbonyl optionally having in the aryl portion one to five substituents α to be described later, (xii) a C₃-C₁₀ cycloalkylcarbonyl, (xiii) a five- to seven-membered cyclic heteroaryl carbonyl optionally having one to two substituents α to be described later and containing one to three heteroatoms selected from among a group consisting of oxygen, nitrogen, and sulfur, or (xiv) carbamoyl optionally substituted with one to two substituents α to be described later (provided, however, that the substituents α may not be (iv) a halogen, (v) hydroxy, (vi) cyano, or (vii) nitro)), (d) a group represented by the expression -CON(R^{e2})- (where R^{e2} is defined as described earlier), (e) a group represented by the expression - N(R^{e2})-CO- (where R^{e2} is defined as described earlier), (f) a group represented by the expression -CO-, (g) a C₁-C₁₀ alkylene optionally containing one to three atoms and/or groups selected from among a group consisting of (a) to (f), or (h) a dangling bond,
X^{e2} indicates the following (a)-(g):
(a) oxygen, (b) sulfur, (c) a group represented by the expression -N(R^{e2})- (where R^{e2} is defined as described earlier), (d) a group represented by the expression -CO-N(R^{e2})- (where R^{e2} is defined as described earlier), (e) a C₁-C₁₀ alkylene optionally containing one to three atoms and/or groups selected from among a group consisting of oxygen, sulfur, groups represented by the expression N(R^{e2})- (where R^{e2} is defined as described earlier), and groups represented by the formula -CO-N (R^{e2})- (where R^{e2} is defined as described earlier), (f) a group represented by the expression -W^{c4}-Phe-X^{e3}- (where W^{c4} indicates oxygen, a group represented by the expression -S(O)q- (where q is defined as described earlier) or the expression -N(R^{e2})- (where R^{e2} is defined as described earlier), a C₁-C₄ alkylene optionally containing one to two atoms and/or groups selected from among a group consisting of oxygen and groups represented by the expression -S(O)_{q}- (where q is defined as described earlier) and the expression -N(R^{e2})- (where R^{e2} is defined as described earlier), or a dangling bond, Phe indicates phenylene optionally having one to four of the substituent R^{e1}, and X^{e3} indicates oxygen, a group represented by the expression -S(O)_{q}- (where q is defined as described earlier) or the expression -N(R^{e2})- (where R^{e2} is defined as described earlier), a C₁-C₄ alkylene optionally containing one to two atoms and/or groups selected from among a group consisting of oxygen and groups represented by the expression -S(O)_{q}- (where q is defined as described earlier) and the expression -N (R^{e2})- (where R^{e2} is defined as described earlier), or a dangling bond), or (g) a dangling bond, and Ar indicates phenylene, naphthylene, or a five- to ten-membered (monocyclic or annelated) heteroarylene ring containing one to five heteroatoms selected from among a group consisting of oxygen, nitrogen, and sulfur); or
(2) a group represented by the expression -W^{c3}-W^{c3}-X^{e4}- (where the two W^{c3} may be the same or different, W^{c3} is defined as described earlier, and X^{e4} indicates the following (a)-(f):
(a) oxygen, (b) sulfur, (c) a group represented by the expression -N(R^{e2})- (where R^{e2} is defined as described earlier), (d) a group represented by the expression -CO-N(R^{e2})- (where R^{e2} is defined as described earlier), (e) a C₁-C₁₀ alkylene optionally containing one to three atoms and/or groups selected from among a group consisting of oxygen, sulfur, groups represented by the expression -N(R^{e2}) (where R^{e2} is defined as described earlier), and groups represented by the expression -CO-N(R^{e2})- (where R^{e2} is defined as described earlier), or (f) a dangling bond); provided, however, that in W^{c3}, X^{e2}, W^{c4}, X^{e3}, X^{e4}, (a) oxygen, (b) a group represented by the expression -S(O)_{q}- (where q is defined as described earlier), and (c) a group represented by the expression -N(R^{e2})- (where R^{e2} is defined as described earlier) or a portion represented by the expression -N(R^{e2})- (where R^{e2} is defined as described earlier) are not bonded directly to each other or adjacent to -CH₂-, and groups formed by these,
G indicates a dangling bond, oxygen, sulfur, or a group represented by the expression -N(R^{e2})-(where R^{e2} is defined as described earlier),
Q indicates oxygen or sulfur,
n indicates an integer from 1 to 6,
p indicates an integer from 1 to 6,
V indicates a dangling bond, oxygen, sulfur, or a group represented by the expression -N(R^{e2})-(where R^{e2} is defined as described earlier),
R indicates (i) hydrogen, (ii) a C₁-C₆ alkyl, (iii) a C₆-C₁₀ aryl optionally having one to five substituents α to be described later, or (iv) a C₆-C₁₀ aryl-C₁-C₆ alkyl optionally having in the aryl portion one to five substituents α to be described later,
the substituent α indicates the following (i)-(xxiv):
(i) a C₁-C₆ alkyl, (ii) a C₁-C₆ halogenoalkyl, (iii) a C₁-C₆ alkoxy, (iv) a halogen, (v) hydroxy, (vi) cyano, (vii) nitro, (viii) a C₃-C₁₀ cycloalkyl, (ix) a C₆-C₁₀ aryl optionally having one to five substituents β to be described later, (x) a C₆-C₁₀ aryl-C₁-C₆ alkyl optionally having in the aryl portion one to five substituents β to be described later, (xi) formyl, a C₁-C₆ alkylcarbonyl, or a C₂-C₆ alkenylcarbonyl, (xii) a C₃-C₁₀ cycloalkylcarbonyl, (xiii) a C₆-C₁₀ arylcarbonyl optionally having one to five substituents β to be described later, (xiv) a C₆-C₁₀ aryl-C₁-C₆ alkylcarbonyl optionally having in the aryl portion one to five substituents β to be described later, (xv) a five-to seven-membered cyclic heteroaryl carbonyl optionally having one to two substituents β to be described later and containing one to three heteroatoms selected from among a group consisting of oxygen, nitrogen, and sulfur, (xvi) carbamoyl, (xvii) a C₆-C₁₀ aryl carbamoyl optionally having one to five substituents β to be described later, (xviii) amino optionally substituted with one to two substituents β to be described later (excluding, however, (ii) a halogen), (xix) a C₁-C₆ halogenoalkoxy, (xx) a C₆-C₁₀ aryloxy optionally having one to five substituents β to be described later, (xxi) a C₆-C₁₀ aryl-C₁-C₆ alkoxy optionally having in the aryl portion one to five substituents β to be described later, (xxii) a C₁-C₆ alkoxycarbonyl, (xxiii) a C₆-C₁₀ aryloxycarbonyl optionally having in the aryl portion one to five substituents β to be described later, or (xxiv) a C₆-C₁₀ aryl-C₁-C₆ alkoxycarbonyl optionally having in the aryl portion one to five substituents β to be described later,
the substituent β indicates the following (i)-(xi):
(i) a C₁-C₁₀ alkyl, a C₁-C₆ halogenoalkyl, or a C₁-C₆ alkoxy, (ii) a halogen, (iii) a C₆-C₁₀ aryl optionally having one to five substituents γ to be described later, (iv) a C₆-C₁₀ aryl-C₁-C₆ alkyl optionally having in the aryl portion one to five substituents γ to be described later, (v) formyl, a C₁-C₆ alkylcarbonyl, or a C₂-C₆ alkenylcarbonyl, (vi) a C₆-C₁₀ aryl carbonyl optionally having one to five substituents γ to be described later, (vii) a C₆-C₁₀ aryl-C₁-C₆ alkylcarbonyl optionally having in the aryl portion one to five substituents γ to be described later, (viii) a C₃-C₁₀ cycloalkylcarbonyl, (ix) a five- to seven-membered cyclic heteroaryl carbonyl optionally having one to two substituents γ to be described later and containing one to three heteroatoms selected from among a group consisting of oxygen, nitrogen, and sulfur, (x) carbamoyl, or (xi) a C₆-C₁₀ aryl carbamoyl optionally having one to five substituents γ to be described later, and
the substituent γ indicates (i) a C₁-C₆ alkyl or a C₁-C₆ alkoxy, (ii) a C₁-C₆ halogenoalkyl, (iii) a halogen, or (iv) hydroxy.

2. The benzo- or pyrido-imidazole derivative, the pharmaceutically acceptable salt thereof, or the pharmaceutically acceptable ester or amide thereof according to claim 1, wherein
A is
(a) a group represented by general formula (a-1) 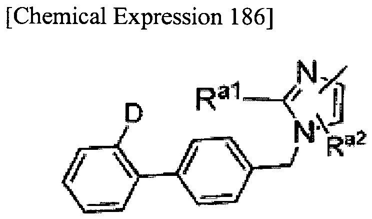 (where R^{a1} is (i) a C₂-C₅ alkyl, (ii) a C₂-C₅ alkyl substituted with fluorine or chlorine, (iii) a C₁-C₅ alkoxy, (iv) a C₂-C₅ alkylthio, (v) a group represented by the expression -N(R^{a3})(R^{a4}) (where R^{a3} is (i) hydrogen, (ii) a C₁-C₅ alkyl, (iii) phenyl optionally having one to two substituents selected from among a group consisting of methyl, ethyl, methoxy, ethoxy, fluorine, chlorine, and trifluoromethyl, or (iv) benzyl optionally having in the phenyl portion one to two substituents selected from among a group consisting of methyl, ethyl, methoxy, ethoxy, fluorine, chlorine, and trifluoromethyl, and R^{a4} is hydrogen or a C₁-C₄ alkyl), (vi) a C₃-C₆ cycloalkyl, (vii) a C₃-C₆ cycloalkyloxy, or (viii) a cycloalkylthio,
R^{a2} is (i) hydrogen, (ii) a halogen, (iii) formyl, (iv) a C₁-C₆ alkylcarbonyl or a C₂-C₃ alkenylcarbonyl, (v) carboxyl or a C₁-C₆ alkoxycarbonyl, (vi) carbamoyl, (vii) a mono- or di-C₁-C₆ alkylcarbamoyl , (viii) amino, (ix) formylamino, a C₁-C₄ alkylcarbonylamino, or a C₂-C₃ alkenylcarbonylamino, (x) a C₁-C₄ hydroxyalkyl, (xi) a formyl-C₁-C₄ alkyl, or (xii) a C₁-C₄ alkylcarbonyl-C₁-C₄ alkyl, and
D is (i) carboxyl, (ii) 5-tetrazolyl, (iii) 5-oxo-1,2,4-oxadiazolin-3-yl or 5-thioxo-1,2,4-oxadiazolin-3-yl, (iv) 2,4-dioxooxazolidin-5-yl or 2,4-dioxothizolidin-5-yl, (v) a group represented by the expression CF₃SO₂-NH-, (vi) tetrazol-5-ylaminocarbonyl, (vii) 2-hydroxy-3,4-dioxo-1-cyclobutenyl, (viii) acetylaminosulfonyl or propionylaminosulfonyl, (ix) benzoylaminosulfonyl optionally having in the phenyl portion one to two substituents selected from among a group consisting of methyl, ethyl, methoxy, ethoxy, fluorine, chlorine, and trifluoromethyl, or (x) phenylacetylaminosulfonyl optionally having in the phenyl portion one to two substituents selected from among a group consisting of methyl, ethyl, methoxy, ethoxy, fluorine, chlorine, and trifluoromethyl),
(b) a group represented by general formula (b-1) 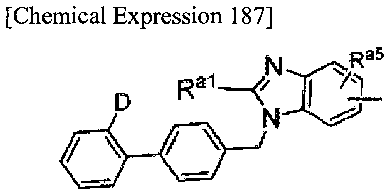 the expression (b-2) 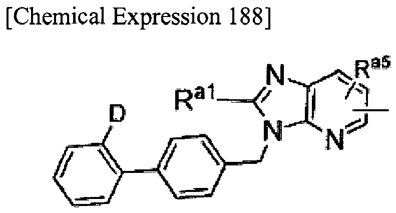 the expression (b-3) 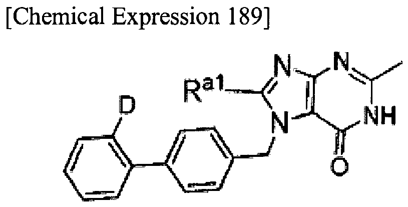 the expression (b-4) 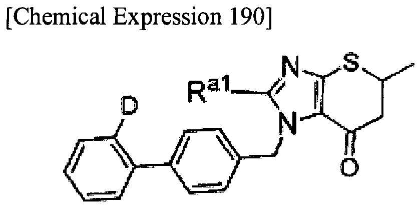 the expression (b-5) 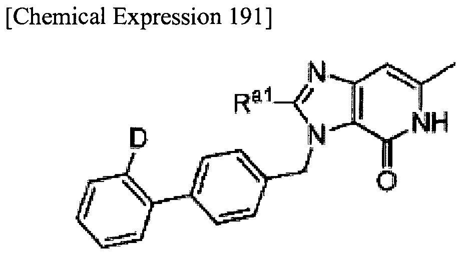 the expression (b-6) 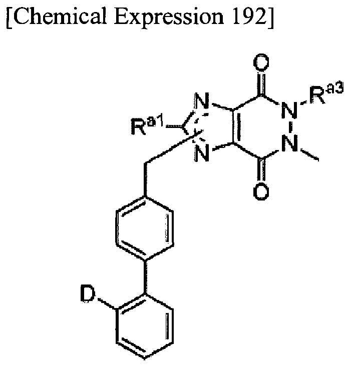 or the expression (b-7) 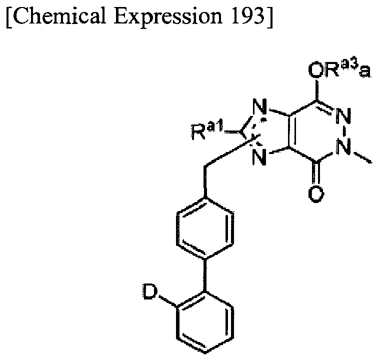 (where D and R^{a1} are defined as in the expression (a-1), and R^{a5} is (i) hydrogen, (ii) a halogen, (iii) formyl, (iv) a C₁-C₆ alkylcarbonyl or C₂-C₃ alkenylcarbonyl, (v) carboxyl or a C₁-C₆ alkoxycarbonyl, (vi) carbamoyl, (vii) a mono- or di-C₁-C₆ alkylcarbamoyl, (viii) amino, (ix) formylamino, a C₁-C₄ alkylcarbonylamino, or a C₂-C₃ alkenylcarbonylamino, (x) a C₁-C₄ hydroxyalkyl, (xi) a formyl-C₁-C₄ alkyl, (xii) a C₁-C₄ alkylcarbonyl-C₁-C₄ alkyl, (xiii) a C₁-C₄ alkyl or hexyl, (xiv) a C₁-C₄ halogenoalkyl, or (xv) a C₁-C₄ alkoxy, the bond embraced by the dotted line is a single bond or a double bond, R^{a3} is (i) hydrogen, (ii) a C₁-C₄ alkyl, or (iv) benzyl, and R^{a3}a is (ii) a C₁-C₄ alkyl or (iv) benzyl),
(c) a group represented by general formula (c-1) 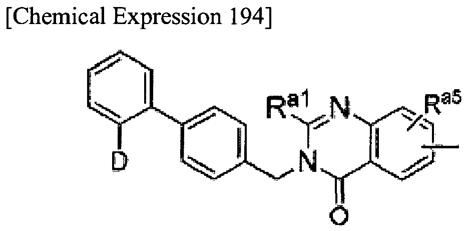 (where D is carboxyl or 5-tetrazolyl, R^{a1} is propyl, butyl, cyclopropyl, or ethoxy, and R^{a5} is hydrogen, chlorine, carboxyl, carbamoyl, methyl, ethyl, propyl, i-propyl, trifluoromethyl, or methoxy),
(d) a group represented by general formula (d-1) 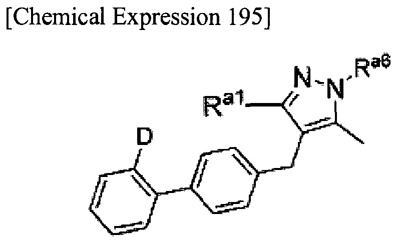 (where D is carboxyl or 5-tetrazolyl, R^{a1} is propyl, butyl, cyclopropyl, or ethoxy, and R^{a6} is methyl or ethyl),
(e) a group represented by general formula (e-1) 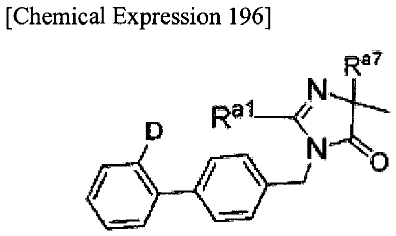 (where D and R^{a1} are defined as in the expression (a-1), and R^{a7} is hydrogen, methyl, ethyl, cyclopropyl, or cyclohexyl),
(f) a group represented by general formula (f-1) 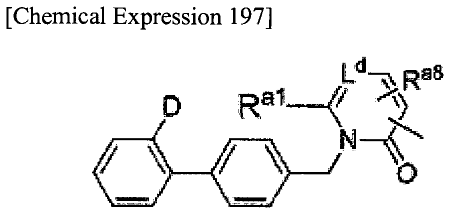 (where D and R^{a1} are defined as in the expression (a-1), R^{a8} is hydrogen, methyl, or ethyl, and L^{d} is a group represented by the expression =CH- or =N-), or
(g) a group represented by general formula (g-1) 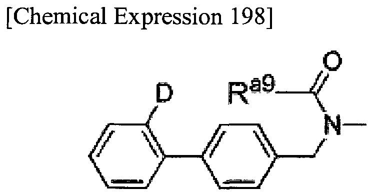 (where D is defined as in the expression (a-1), and R^{a9} is a C₁-C₄ alkyl, a C₁-C₄ halogenoalkyl, or a C₃-C₆ cycloalkyl).

3. The benzo- or pyrido-imidazole derivative, the pharmaceutically acceptable salt thereof, or the pharmaceutically acceptable ester or amide thereof according to claim 1, wherein
A is
(a) a group represented by general formula (a-1) 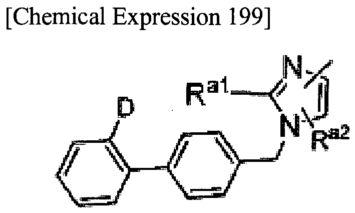 (where R^{a1} is (i) a C₃-C₅ alkyl, (ii) a C₃-C₄ alkyl substituted with fluorine, (iii) a C₂-C₄ alkoxy, (iv) a C₂-C₄ alkylthio, (v) a group represented by the expression -N(R^{a3})(R^{a4}) (where R^{a3} is (i) hydrogen or (ii) a C₂-C₄ alkyl, and R^{a4} is hydrogen), (vi) a C₃-C₄ cycloalkyl, (vii) cyclopropyloxy, or (viii) cyclopropylthio,
R^{a2} is (i) hydrogen, (ii) a halogen, (iii) formyl, (iv) a C₁-C₃ alkylcarbonyl, (v) carboxyl or a C₁-C₄ alkoxycarbonyl, (vi) carbamoyl, (vii) a mono- or di-C₁-C₃ alkylcarbamoyl , (ix) formylamino or acetylamino, (x) a C₁-C₃ hydroxyalkyl, (xi) a formyl-C₁-C₂ alkyl, or (xii) a C₁-C₂ alkylcarbonyl-C₁-C2 alkyl, and D is (i) carboxyl, (ii) 5-tetrazolyl, (iii) 5-oxo-1,2,4-oxadiazolin-3-yl, (iv) 2,4-dioxooxazolidin-5-yl, (v) a group represented by the expression CF₃SO₂-NH-, (vi) tetrazol-5-ylaminocarbonyl, or (viii) acetylaminosulfonyl),
(b) a group represented by the expressions (b-1) 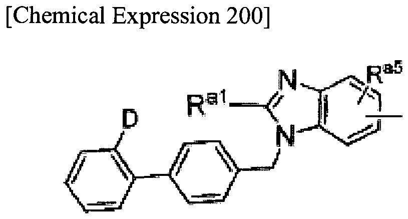 the expression (b-2) 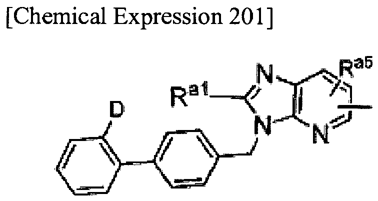 the expression (b-3) 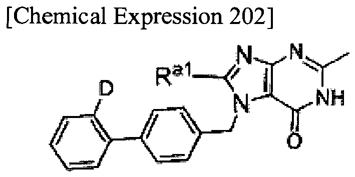 the expression (b-4) 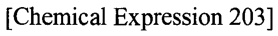 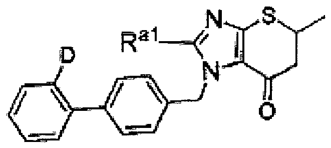 the expression (b-5) 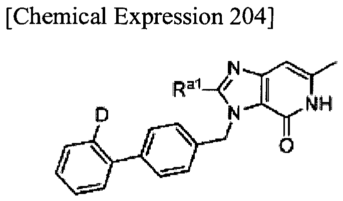 the expression (b-6) 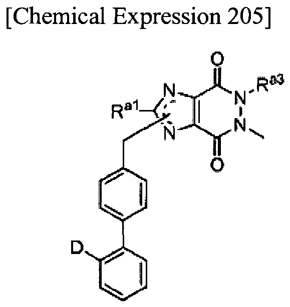 or the expression (b-7) 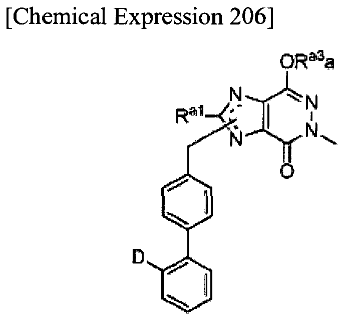 (where D and R^{a1} are defined as in the expression (a-1), R^{a5} is (i) hydrogen, (ii) a halogen, (iii) formyl, (iv) a C₁-C₄ alkylcarbonyl, (v) carboxyl or a C₁-C₄ alkoxycarbonyl, (vi) carbamoyl, (vii) a mono- or di-C₁-C₄ alkylcarbamoyl, (ix) formylamino or acetylamino, (x) a C₁-C₂ hydroxyalkyl, (xi) a formyl-C₁-C₂ alkyl, (xii) a C₂-C₄ alkylcarbonyl-C₁-C₂ alkyl, (xiii) a C₁-C₄ alkyl or hexyl, (xiv) a C₁-C₃ alkyl substituted with fluorine or chlorine, or (xv) a C₁-C₃ alkoxy, the bond embraced by the dotted line is a single bond or a double bond, R^{a3} is (i) hydrogen or (ii) a C₁-C₄ alkyl, and R^{a3}a is (ii) a C₁-C₄ alkyl),
(e) a group represented by general formula (e-1) 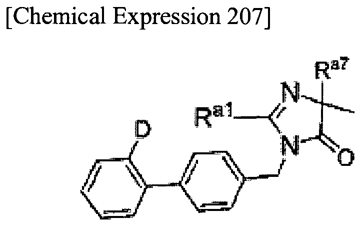 (where D and R^{a1} are defined as in the expression (a-1), and R^{a7} is cyclohexyl),
(f) a group represented by general formula (f-1) 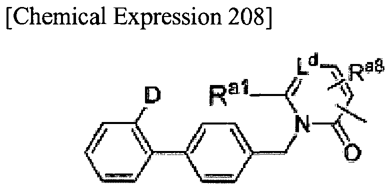 (where D and R^{a1} are defined as in the expression (a-1), R^{a8} is methyl, and L^{d} is a group represented by the expression =N-), or
(g) a group represented by general formula (g-1) 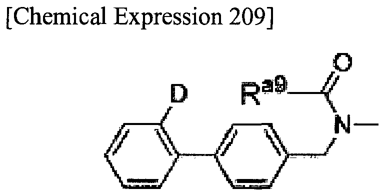 (where D is defined as in the expression (a-1), and R^{a9} is a C₂-C₄ alkyl, a C₃-C₄ alkyl substituted with fluorine, or a C₃-C₆ cycloalkyl).

4. The benzo- or pyrido-imidazole derivative, the pharmaceutically acceptable salt thereof, or the pharmaceutically acceptable ester or amide thereof according to claim 1, wherein
A is
(a) a group represented by general formula (a-1) 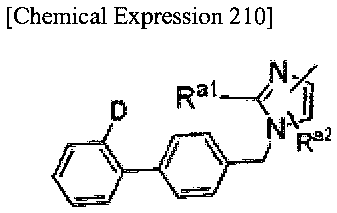 (where R^{a1} is (i) propyl, butyl, i-butyl, or pentyl, (iii) ethoxy or propoxy, (iv) ethylthio or propylthio, (v) propylamino or butylamino, (vi) cyclopropyl, or (vii) cyclopropyloxy, R^{a2} is (ii) chlorine or bromine, (iii) formyl, (iv) acetyl or propionyl, (v) carboxyl or methoxy, ethoxy, propoxy, i-propoxy, or butoxycarbonyl, (vi) carbamoyl, (vii) N-methylcarbamoyl, N-ethylcarbamoyl, or N,N-dimethylcarbamoyl, or (x) 1-hydroxyethyl or 1-hydroxy-1-methylethyl, and D is (i) carboxyl, (ii) 5-tetrazolyl, (iii) 5-oxo-1,2,4-oxadiazolin-3-yl, (v) a group represented by the expression CF₃SO₂-NH-, or (vi) tetrazol-5-ylaminocarbonyl), or
(b) a group represented by general formula (b-1) 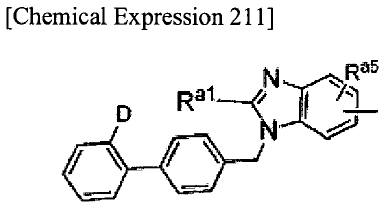 the expression (b-2) 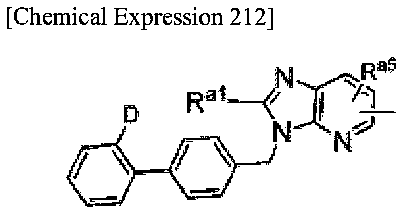 the expression (b-6) 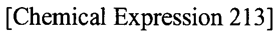 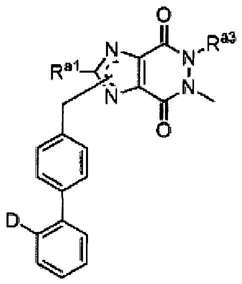 or the expression (b-7) 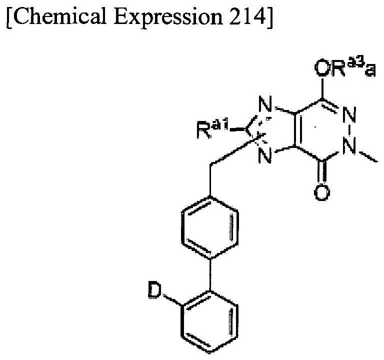 (where D and R^{a1} are defined as in the expression (a-1), R^{a5} is (i) hydrogen, (ii) fluorine or chlorine, (iii) formyl, (iv) acetyl, (v) carboxyl or a C₁-C₃ alkoxycarbonyl, (vi) carbamoyl, (vii) N-methyl, N-ethyl, N-i-propyl, N,N-dimethyl, or N-methyl-N-ethylcarbamoyl, (xiii) methyl, ethyl, propyl, i-propyl, butyl, t-butyl, or hexyl, (xiv) fluoromethyl or trifluoromethyl, or (xv) methoxy, ethoxy, propoxy, or i-propoxy, the bond embraced by the dotted line is a single bond or a double bond, R^{a3} is (i) hydrogen or (ii) methyl, ethyl, or i-propyl, and R^{a3}a is (ii) methyl or ethyl).

5. The benzo- or pyrido-imidazole derivative, the pharmaceutically acceptable salt thereof, or the pharmaceutically acceptable ester or amide thereof according to claim 1, wherein
A is
(a) a group represented by general formula (a-1) 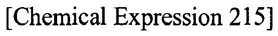 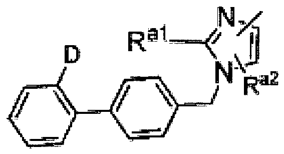 (where R^{a1} is (i) propyl or butyl, (iii) ethoxy, (iv) ethylthio or propylthio, (v) propylamino, or (vi) cyclopropyl, R^{a2} is (ii) chlorine, (iii) formyl, (iv) acetyl or propionyl, (v) carboxyl or methoxy, ethoxy, propoxy, i-propoxy, or butoxycarbonyl, (vi) carbamoyl, (vii) N-methylcarbamoyl, N-ethylcarbamoyl, or N,N-dimethylcarbamoyl, or (x) 1-hydroxyethyl, and D is (i) carboxyl, (ii) 5-tetrazolyl, or (iii) 5-oxo-1,2,4-oxadiazolin-3-yl), or
(b) a group represented by general formula (b-1) 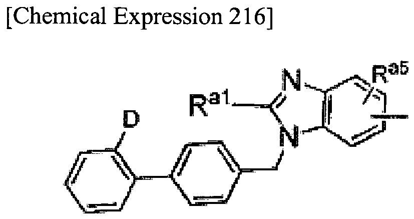 (where D and R^{a1} are defined as in the expression (a-1), and R^{a5} is (i) hydrogen, (ii) chlorine, (iii) formyl, (iv) acetyl, (v) carboxyl, methoxycarbonyl, or ethoxycarbonyl, (vi) carbamoyl, (vii) N-methyl or N,N-dimethylcarbamoyl, (xiii) methyl, ethyl, propyl, i-propyl, butyl, or t-butyl, (xiv) trifluoromethyl, or (xv) methoxy).

6. The benzo- or pyrido-imidazole derivative, the pharmaceutically acceptable salt thereof, or the pharmaceutically acceptable ester or amide thereof according to claim 1, wherein
A is
(a) a group represented by general formula (a-1) 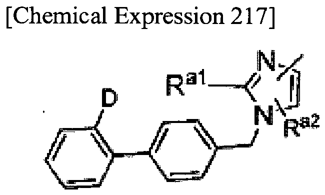 (where R^{a1} is (i) propyl or butyl, (iii) ethoxy, or (vi) cyclopropyl, R^{a2} is (ii) chlorine, (iii) formyl, (iv) acetyl or propionyl, (v) carboxyl or methoxy, ethoxy, propoxy, or i-propoxycarbonyl, (vi) carbamoyl, or (vii) N-methyl, N-ethyl, or N,N-dimethylcarbamoyl, and D is (i) carboxyl or (ii) 5-tetrazolyl), or
(b) a group represented by general formula (b-1) 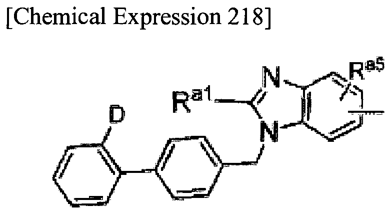 (where D and R^{a1} are defined as in the expression (a-1), and R^{a5} is (i) hydrogen, (ii) chlorine, (v) carboxyl, (vi) carbamoyl, (xiii) methyl, ethyl, propyl, or i-propyl, (xiv) trifluoromethyl, or (xv) methoxy).

7. The benzo- or pyrido-imidazole derivative, the pharmaceutically acceptable salt thereof, or the pharmaceutically acceptable ester or amide thereof according to claims 1 to 6, wherein B is
a group represented by general formula (bb-1) 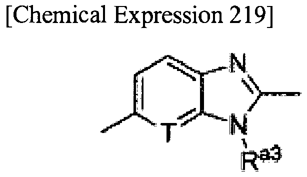 (where R^{a3} is (i) hydrogen, (ii) a C₁-C₅ alkyl, (iii) phenyl optionally having one to two substituents selected from among a group consisting of methyl, ethyl, methoxy, ethoxy, fluorine, chlorine, and trifluoromethyl, or (iv) benzyl or phenethyl optionally having in the phenyl portion one to five substituents selected from among a group consisting of C₁-C₃ alkyls, C₁-C₃ alkoxys, fluorine, chlorine, and C₁-C₃ alkyls substituted with fluorine or chlorine, T is the expression - CH= or -N=, and B is bonded to E (or G) by a portion of an imidazole ring or a portion of a benzene or pyridine ring).

8. The benzo- or pyrido-imidazole derivative, the pharmaceutically acceptable salt thereof, or the pharmaceutically acceptable ester or amide thereof according to claims 1 to 6, wherein B is
a group represented by general formula (bb-1) 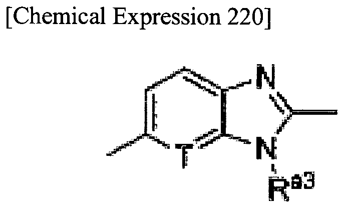 (where R^{a3} is (i) hydrogen, (ii) a C₁-C₄ alkyl, or (iv) benzyl optionally having in the phenyl portion one to two substituents selected from among a group consisting of methyl, ethyl, ethoxy, methoxy, fluorine, chlorine, and trifluoromethyl, T is the expression -CH= or -N=, and B is bonded to E (or G) by a portion of an imidazole ring or a portion of a benzene or pyridine ring).

9. The benzo- or pyrido-imidazole derivative, the pharmaceutically acceptable salt thereof, or the pharmaceutically acceptable ester or amide thereof according to claims 1 to 6, wherein B is
a group represented by general formula (bb-1) 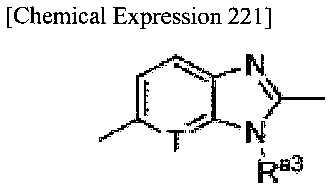 (where R^{a3} is (i) hydrogen, (ii) methyl, ethyl, propyl, i-propyl, or butyl, or (iv) benzyl optionally substituted in the phenyl portion with methyl, methoxy, fluorine, chlorine, or trifluoromethyl, T is the expression -CH= or -N=, and B is bonded to E by a portion of a benzene or pyridine ring and to G by a portion of an imidazole ring).

10. The benzo- or pyrido-imidazole derivative, the pharmaceutically acceptable salt thereof, or the pharmaceutically acceptable ester or amide thereof according to claims 1 to 6, wherein B is
a group represented by general formula (bb-1) 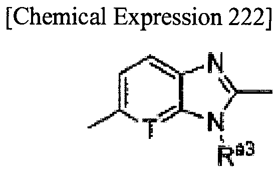 (where R^{a3} is (ii) methyl or ethyl, T is the expression -CH=, and B is bonded to E by a portion of a benzene or pyridine ring and to G by a portion of an imidazole ring).

11. The benzo- or pyrido-imidazole derivative, the pharmaceutically acceptable salt thereof, or the pharmaceutically acceptable ester or amide thereof according to claims 1 to 10, wherein C is
(a) carboxyl,
(b) thiazolidine-2,4-dion-5-yl, oxazolidine-2,4-dion-5-yl, or 1,2,4-oxadiazolidine-3,5-dion-2-yl,
(c) a group represented by the expression -C(R^{c1})(COOH)-W^{c1}-R^{c2} (where R^{c1} is (i) hydrogen, (ii) a C₁-C₄ alkyl, (iii) phenyl or naphthyl optionally having one to two substituents selected from among a group consisting of methyl, ethyl, methoxy, ethoxy, fluorine, chlorine, and trifluoromethyl, (iv) a phenyl-C₁-C₂ alkyl optionally having in the phenyl portion one to two substituents selected from among a group consisting of methyl, ethyl, methoxy, ethoxy, fluorine, chlorine, and trifluoromethyl, (v) a C₁-C₂ alkylsulfonyl, (vi) a C₁-C₂ alkylsulfonyl substituted with fluorine or chlorine, (vii) phenyl- or naphthyl-sulfonyl optionally having one to two substituents selected from among a group consisting of methyl, ethyl, methoxy, ethoxy, fluorine, chlorine, and trifluoromethyl, or (viii) a phenyl- or naphthyl-C₁-C₂ alkylsulfonyl optionally having in the phenyl or naphthyl portion one to two substituents selected from among a group consisting of methyl, ethyl, methoxy, ethoxy, fluorine, chlorine, and trifluoromethyl, R^{c2} is (i) hydrogen, (ii) a C₁-C₄ alkyl, (iii) phenyl or naphthyl optionally having one to three substituents selected from among a group consisting of C₁-C₄ alkyls, C₁-C₄ alkoxys, halogens, C₁-C₂ halogenoalkyls, formyl, C₁-C₂ alkylcarbonyls, and benzoyl optionally having one to two substituents selected from among a group consisting of methyl, ethyl, methoxy, ethoxy, fluorine, chlorine, and trifluoromethyl, (iv) a phenyl- or naphthyl-C₁-C₄ alkyl optionally having in the phenyl or naphthyl portion one to three substituents selected from among a group consisting of C₁-C₄ alkyls, C₁-C₄ alkoxys, halogens, and C₁-C₂ halogenoalkyls, (v) a C₁-C₄ alkylsulfonyl, (vi) a C₁-C₂ alkylsulfonyl substituted with fluorine or chlorine, (vii) phenyl- or naphthyl-sulfonyl optionally having one to three substituents selected from among a group consisting of C₁-C₄ alkyls, C₁-C₄ alkoxys, halogens, and C₁-C₂ halogenoalkyls, or (viii) a phenyl- or naphthyl-C₁-C₂ alkylsulfonyl optionally having in the phenyl or naphthyl portion one to three substituents selected from among a group consisting of C₁-C₄ alkyls, C₁-C₄ alkoxys, halogens, and C₁-C₂ halogenoalkyls, W^{c1} is (i) oxygen, (ii) sulfur, or (iii) a group represented by the expression =N-R^{c3} (where R^{c3} is (i) hydrogen, (ii) a C₁-C₄ alkyl, (iii) phenyl or naphthyl optionally having one to two substituents selected from among a group consisting of methyl, ethyl, methoxy, ethoxy, fluorine, chlorine, and trifluoromethyl, (iv) a phenyl- or naphthyl-C₁-C₂ alkyl optionally having in the phenyl or naphthyl portion one to two substituents selected from among a group consisting of methyl, ethyl, methoxy, ethoxy, fluorine, chlorine, and trifluoromethyl, (v) formyl, a C₁-C₂ alkylcarbonyl, or acryloyl, (vi) benzoyl or naphthylcarbonyl optionally having one to three substituents selected from among a group consisting of C₁-C₄ alkyls, C₁-C₄ alkoxys, halogens, and C₁-C₂ halogenoalkyls, (vii) phenyl- or naphthyl-C₁-C₂ alkylcarbonyl optionally having in the phenyl or naphthyl portion one to three substituents selected from among a group consisting of C₁-C₄ alkyls, C₁-C₄ alkoxys, halogens, and C₁-C₂ halogenoalkyls, (viii) a C₃-C₄ cycloalkylcarbonyl, (ix) furylcarbonyl, thienylcarbonyl, or nicotinoyl optionally having one to two substituents selected from among a group consisting of methyl, ethyl, methoxy, fluorine, and chlorine, (x) carbamoyl optionally having one to two substituents selected from among a group consisting of C₁-C₄ alkyls, phenyl or naphthyl optionally having one to three substituents selected from among a group consisting of C₁-C₄ alkyls, C₁-C₄ alkoxys, halogens, and C₁-C₂ halogenoalkyls, and phenyl- or naphthyl-C₁-C₂ alkyls optionally having in the phenyl or naphthyl portion one to three substituents selected from among a group consisting of C₁-C₄ alkyls, C₁-C₄ alkoxys, halogens, and C₁-C₂ halogenoalkyls, (xi) a C₁-C₂ alkoxycarbonyl, (xii) phenoxycarbonyl optionally having one to two substituents selected from among a group consisting of methyl, ethyl, methoxy, ethoxy, fluorine, chlorine, and trifluoromethyl, or (xiii) benzyloxycarbonyl optionally having in the phenyl portion one to two substituents selected from among a group consisting of methyl, ethyl, methoxy, ethoxy, fluorine, chlorine, and trifluoromethyl)), or
(d) a group represented by the expression -N(W^{c2})-CH₂COOH (where W^{c2} is (i) hydrogen, (ii) a C₁-C₄ alkyl, (iii) phenyl or naphthyl optionally having one to two substituents selected from among a group consisting of methyl, ethyl, methoxy, ethoxy, fluorine, chlorine, and trifluoromethyl, (iv) a phenyl- or naphthyl-C₁-C₂ alkyl optionally having in the phenyl or naphthyl portion one to three substituents selected from among a group consisting of C₁-C₄ alkyls, C₁-C₄ alkoxys, halogens, and C₁-C₂ halogenoalkyls, (v) formyl, a C₁-C₄ alkylcarbonyl, or a C₂-C₃ alkenylcarbonyl, (vi) benzoyl or naphthylcarbonyl optionally having one to three substituents selected from among a group consisting of C₁-C₄ alkyls, C₁-C₄ alkoxys, halogens, and C₁-C₂ halogenoalkyls, (vii) a phenyl- or naphthyl-C₁-C₂ alkylcarbonyl optionally having in the phenyl or naphthyl portion one to three substituents selected from among a group consisting of C₁-C₄ alkyls, C₁-C₄ alkoxys, halogens, and C₁-C₂ halogenoalkyls, (viii) a C₃-C₆ cycloalkylcarbonyl, (ix) furylcarbonyl, thienylcarbonyl, or nicotinoyl optionally having one to two substituents selected from among a group consisting of methyl, ethyl, methoxy, fluorine, and chlorine, (x) carbamoyl optionally having one to two substituents selected from among a group consisting of C₁-C₄ alkyls, phenyl or naphthyl optionally having one to three substituents selected from among a group consisting of C₁-C₄ alkyls, C₁-C₄ alkoxys, halogens, and C₁-C₂ halogenoalkyls, and phenyl- or naphthyl-C₁-C₂ alkyls optionally having in the phenyl or naphthyl portion one to three substituents selected from among a group consisting of C₁-C₄ alkyls, C₁-C₄ alkoxys, halogens, and C₁-C₂ halogenoalkyls, (xi) a C₁-C₄ alkoxycarbonyl, (xii) a phenoxy- or naphthyloxy-carbonyl optionally having one to three substituents selected from among a group consisting of C₁-C₄ alkyls, C₁-C₄ alkoxys, halogens, and C₁-C₂ halogenoalkyls, or (xiii) a phenyl- or naphthyl-C₁-C₂ alkoxycarbonyl optionally having in the phenyl or naphthyl portion one to three substituents selected from among a group consisting of C₁-C₄ alkyls, C₁-C₄ alkoxys, halogens, and C₁-C₂ halogenoalkyls).

12. The benzo- or pyrido-imidazole derivative, the pharmaceutically acceptable salt thereof, or the pharmaceutically acceptable ester or amide thereof according to claims 1 to 10, wherein C is
(a) carboxyl,
(b) thiazolidine-2,4-dion-5-yl, oxazolidine-2,4-dion-5-yl, or 1,2,4-oxadiazolidine-3,5-dion-2-yl,
(c) a group represented by the expression -C(R^{c1})(COOH)-W^{c1}-R^{c2} (where R^{c1} is (i) hydrogen, (ii) a C₁-C₂ alkyl, (iii) phenyl optionally having one to two substituents selected from among a group consisting of methyl, ethyl, methoxy, ethoxy, fluorine, chlorine, and trifluoromethyl, or (iv) benzyl optionally having in the phenyl portion one to two substituents selected from among a group consisting of methyl, ethyl, methoxy, ethoxy, fluorine, chlorine, and trifluoromethyl, R^{c2} is (i) hydrogen, (ii) a C₁-C₄ alkyl, (iii) phenyl optionally having one to two substituents selected from among a group consisting of C₁-C₄ alkyls, C₁-C₃ alkoxys, fluorine, chlorine, trifluoromethyl, formyl, C₁-C₂ alkylcarbonyls, and benzoyl optionally having one to two substituents selected from among a group consisting of methyl, methoxy, fluorine, chlorine, and trifluoromethyl, (iv) benzyl optionally having in the phenyl portion one to two substituents selected from among a group consisting of C₁-C₄ alkyls, C₁-C₃ alkoxys, fluorine, chlorine, and trifluoromethyl, (v) a C₁-C₃ alkylsulfonyl, (vi) a C₁-C₂ alkylsulfonyl substituted with fluorine, (vii) phenylsulfonyl optionally having one to two substituents selected from among a group consisting of methyl, ethyl, methoxy, ethoxy, fluorine, chlorine, and trifluoromethyl, or (viii) benzylsulfonyl optionally having in the phenyl portion one to two substituents selected from among a group consisting of methyl, ethyl, methoxy, ethoxy, fluorine, chlorine, and trifluoromethyl, W^{c1} is (i) oxygen, (ii) sulfur, or (iii) a group represented by the expression =N(R^{c3}) (where R^{c3} is (i) hydrogen, (ii) a C₁-C₃ alkyl, (iii) phenyl optionally having one to two substituents selected from among a group consisting of methyl, methoxy, fluorine, chlorine, and trifluoromethyl, (iv) benzyl optionally having in the phenyl portion one to two substituents selected from among a group consisting of methyl, methoxy, fluorine, chlorine, and trifluoromethyl, (v) formyl or acetyl, (vi) benzoyl optionally having one to two substituents selected from among a group consisting of methyl, ethyl, methoxy, ethoxy, fluorine, chlorine, and trifluoromethyl, (vii) a phenyl-C₁-C₂ alkylcarbonyl optionally having in the phenyl portion one to two substituents selected from among a group consisting of methyl, ethyl, methoxy, ethoxy, fluorine, chlorine, and trifluoromethyl, (viii) cyclopropylcarbonyl, (ix) furylcarbonyl, thienylcarbonyl, or nicotinoyl optionally substituted with methyl, methoxy, fluorine, or chlorine, or (x) carbamoyl optionally having one to two substituents selected from among a group consisting of C₁-C₃ alkyls, phenyl optionally having one to two substituents selected from among a group consisting of methyl, ethyl, methoxy, ethoxy, fluorine, chlorine, and trifluoromethyl, and phenyl-C₁-C₂ alkyls optionally having in the phenyl portion one to two substituents selected from among a group consisting of methyl, ethyl, methoxy, ethoxy, fluorine, chlorine, bromine, and trifluoromethyl), or
(d) a group represented by the expression -N(W^{c2})-CH₂COOH (where W^{c2} is (i) hydrogen, (ii) a C₁-C₂ alkyl, (iii) phenyl optionally having one to two substituents selected from among a group consisting of methyl, methoxy, fluorine, chlorine, and trifluoromethyl, (iv) a phenyl-C₁-C₂ alkyl optionally having in the phenyl portion one to two substituents selected from among a group consisting of methyl, ethyl, methoxy, ethoxy, fluorine, chlorine, and trifluoromethyl, (v) formyl, acetyl, propionyl, butyryl, or valeryl, (vi) benzoyl optionally having one to two substituents selected from among a group consisting of methyl, ethyl, methoxy, ethoxy, fluorine, chlorine, and trifluoromethyl, (vii) a phenyl-C₁-C₂ alkylcarbonyl optionally having in the phenyl portion one to two substituents selected from among a group consisting of methyl, ethyl, methoxy, ethoxy, fluorine, chlorine, and trifluoromethyl, (viii) a C₅-C₆ cycloalkylcarbonyl, (ix) furylcarbonyl, thienylcarbonyl, or nicotinoyl optionally substituted with methyl, methoxy, fluorine, or chlorine, (x) carbamoyl optionally having one to two substituents selected from among a group consisting of C₁-C₃ alkyls, phenyl optionally having one to two substituents selected from among a group consisting of methyl, ethyl, methoxy, ethoxy, fluorine, chlorine, and trifluoromethyl, and phenyl-C₁-C₂ alkyls optionally having in the phenyl or naphthyl portion one to two substituents selected from among a group consisting of methyl, ethyl, methoxy, ethoxy, fluorine, chlorine, bromine, and trifluoromethyl, (xi) a C₁-C₂ alkoxycarbonyl, (xii) phenoxycarbonyl optionally having one to two substituents selected from among a group consisting of methyl, ethyl, methoxy, ethoxy, fluorine, chlorine, and trifluoromethyl, or (xiii) benzyloxycarbonyl optionally having in the phenyl portion one to two substituents selected from among a group consisting of methyl, ethyl, methoxy, ethoxy, fluorine, chlorine, and trifluoromethyl).

13. The benzo- or pyrido-imidazole derivative, the pharmaceutically acceptable salt thereof, or the pharmaceutically acceptable ester or amide thereof according to claims 1 to 10, wherein C is
(a) carboxyl,
(b) thiazolidine-2,4-dion-5-yl, oxazolidine-2,4-dion-5-yl, or 1,2,4-oxadiazolidine-3,5-dion-2-yl,
(c) a group represented by the expression -C(R^{c1})(COOH)-W^{c1}-R^{c2} (where R^{c1} is (i) hydrogen, (ii) methyl, (iii) phenyl, or (iv) benzyl, R^{c2} is (i) hydrogen, (ii) methyl, ethyl, or butyl, (iii) phenyl optionally having one to two substituents selected from among a group consisting of methyl, ethyl, propyl, t-butyl, methoxy, ethoxy, fluorine, chlorine, trifluoromethyl, and benzoyl optionally having one to two substituents selected from among a group consisting of methyl, methoxy, fluorine, chlorine, and trifluoromethyl, (iv) benzyl optionally having in the phenyl portion one to two substituents selected from among a group consisting of methyl, ethyl, methoxy, ethoxy, propoxy, fluorine, chlorine, and trifluoromethyl, (v) methane, ethane, or propanesulfonyl, (vi) trifluoromethanesulfonyl, (vii) phenylsulfonyl optionally having one to two substituents selected from among a group consisting of methyl, methoxy, fluorine, chlorine, and trifluoromethyl, or (viii) benzylsulfonyl optionally having in the phenyl portion one to two substituents selected from among a group consisting of methyl, methoxy, fluorine, chlorine, and trifluoromethyl, W^{c1} is (i) oxygen, (ii) sulfur, or (iii) a group represented by the expression =N-R^{c3} (where R^{c3} is (i) hydrogen, (ii) methyl or ethyl, (iii) phenyl, (iv) benzyl, (v) acetyl, (vi) benzoyl optionally having one to two substituents selected from among a group consisting of methyl, methoxy, fluorine, chlorine, and trifluoromethyl, (vii) benzylcarbonyl optionally having in the phenyl portion one to two substituents selected from among a group consisting of methyl, methoxy, fluorine, chlorine, and trifluoromethyl, (ix) furylcarbonyl, thienylcarbonyl, or nicotinoyl, or (x) carbamoyl optionally having one to two substituents selected from among a group consisting of methyl and ethyl, phenyl optionally having one to two substituents selected from among a group consisting of methyl, methoxy, fluorine, chlorine, and trifluoromethyl, and benzyl optionally having in the phenyl portion one to two substituents selected from among a group consisting of methyl, methoxy, fluorine, chlorine, bromine, and trifluoromethyl), or
(d) a group represented by the expression -N(W^{c2})-CH₂COOH (where W^{c2} is (i) hydrogen, (ii) methyl, (iii) phenyl, (iv) benzyl optionally having in the phenyl portion one to two substituents selected from among a group consisting of methyl, methoxy, fluorine, chlorine, and trifluoromethyl, (v) acetyl, propionyl, butyryl, or valeryl, (vi) benzoyl optionally having one to two substituents selected from among a group consisting of methyl, methoxy, fluorine, chlorine, and trifluoromethyl, (vii) benzylcarbonyl optionally having in the phenyl portion one to two substituents selected from among a group consisting of methyl, methoxy, fluorine, chlorine, and trifluoromethyl, (ix) furylcarbonyl, thienylcarbonyl, or nicotinoyl, (x) carbamoyl optionally having one to two substituents selected from among a group consisting of methyl and ethyl, phenyl, and benzyl optionally having in the phenyl portion one to two substituents selected from among a group consisting of methyl, methoxy, fluorine, chlorine, bromine, and trifluoromethyl, (xi) methoxycarbonyl, (xii) phenoxycarbonyl optionally having one to two substituents selected from among a group consisting of methyl, methoxy, fluorine, chlorine, and trifluoromethyl, or (xiii) benzyloxycarbonyl optionally having in the phenyl portion one to two substituents selected from among a group consisting of methyl, methoxy, fluorine, chlorine, and trifluoromethyl).

14. The benzo- or pyrido-imidazole derivative, the pharmaceutically acceptable salt thereof, or the pharmaceutically acceptable ester or amide thereof according to claims 1 to 10, wherein C is
(a) carboxyl,
(b) thiazolidine-2,4-dion-5-yl, oxazolidine-2,4-dion-5-yl, or 1,2,4-oxadiazolidine-3,5-dion-2-yl,
(c) (ethoxy)(carboxy)methyl, (4-t-butylphenoxy)(carboxy)methyl, (4-chlorophenoxy)(carboxy)methyl, (benzyloxy)(carboxy)methyl, (4-propoxybenzyloxy)(carboxy)methyl, (3-chlorobenzyloxy)(carboxy)methyl, (4-chlorobenzyloxy)(carboxy)methyl, (4-methoxybenzyloxy)(carboxy)methyl, (N-benzoylamino)(carboxy)methyl, (N-methyl-N-benzoylamino)(carboxy)methyl, (propylsulfonylamino)(carboxy)methyl, (phenylsulfonylamino)(carboxy)methyl, (benzylsulfonylamino)(carboxy)methyl, (N-methyl-N-phenylsulfonylamino)(carboxy)methyl, (N-ethyl-N-benzylamino)(carboxy)methyl, (N-ethyl-N-nicotinoylamino)(carboxy)methyl, (3-phenylureido)(carboxy)methyl, [1-butyl-3-(3-bromobenzyl)ureido](carboxy)methyl, [N-ethyl-N-(4-methoxybenzylcarbonyl)amino](carboxy)methyl, or (2-benzoylphenylamino)(carboxy)methyl, or
(d) N-(4-methoxyphenoxycarbonyl)-N-carboxymethylamino, N-(4-chlorobenzyl)-N-carboxymethylamino, N-phenoxycarbonyl-N-carboxymethylamino, N-benzoyl-N-carboxymethylamino, N-(3-trifluoromethylbenzoyl)-N-carboxymethylamino, N-benzylcarbonyl-N-carboxymethylamino, N-valeryl-N-carboxymethylamino, N-(N-benzylcarbamoyl)-N-carboxymethylamino, N-(N-benzyl-N-methylcarbamoyl)-N-carboxymethylamino, or N-(N,N-diethylcarbamoyl)-N-carboxymethylamino.

15. The benzo- or pyrido-imidazole derivative, the pharmaceutically acceptable salt thereof, or the pharmaceutically acceptable ester or amide thereof according to claims 1 to 10, wherein C is
(a) carboxyl, (b) thiazolidine-2,4-dion-5-yl, (c) (4-chlorobenzyloxy)(carboxy)methyl, or (d) N-phenoxycarbonyl-N-carboxymethylamino.

16. The benzo- or pyrido-imidazole derivative, the pharmaceutically acceptable salt thereof, or the pharmaceutically acceptable ester or amide thereof according to claims 1 to 15, wherein E is
(1) a group represented by general formula (d-1) 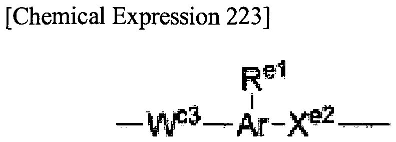 (where R^{e1} is the same or different zero or one to three groups, and R^{e1} is (i) a C₁-C₄ alkyl, (ii) a C₁-C₄ halogenoalkyl, (iii) a C₁-C₄ alkoxy, (iv) a halogen, (v) hydroxy, (vi) cyano, (vii) nitro, (viii) a C₃-C₆ cycloalkyl, (ix) phenyl or naphthyl optionally having one to three substituents selected from among a group consisting of C₁-C₄ alkyls, C₁-C₂ halogenoalkyls, C₁-C₄ alkoxys, and halogens, (x) a phenyl- or naphthyl-C₁-C₂ alkyl optionally having in the phenyl or naphthyl portion one to three selected from among a group consisting of C₁-C₄ alkyls, C₁-C₂ halogenoalkyls, C₁-C₄ alkoxys, and halogens, (xi) formyl or a C₁-C₄ alkylcarbonyl or C₂-C₃ alkenylcarbonyl, (xii) a C₃-C₆ cycloalkylcarbonyl, (xiii) benzoyl or naphthylcarbonyl optionally having one to three substituents selected from among a group consisting of C₁-C₄ alkyls, C₁-C₂ halogenoalkyls, C₁-C₄ alkoxys, and halogens, (xiv) a phenyl- or naphthyl-C₁-C₂ alkylcarbonyl optionally having in the aryl portion one to three substituents selected from among a group consisting of C₁-C₄ alkyls, C₁-C₂ halogenoalkyls, C₁-C₄ alkoxys, and halogens, (xv) furylcarbonyl, thienylcarbonyl, imidazolylcarbonyl, oxazolylcarbonyl, isooxazolylcarbonyl, thiazolylcarbonyl, isothiazolylcarbonyl, nicotinoyl, isonicotinoyl, pyridazinylcarbonyl, pyrimidinylcarbonyl, or pyrazinylcarbonyl optionally having one to two substituents selected from among a group consisting of C₁-C₄ alkyls, C₁-C₂ halogenoalkyls, C₁-C₄ alkoxys, and halogens, (xvi) carbamoyl, (xvii) phenylcarbamoyl or naphthylcarbonyl optionally having one to three substituents selected from among a group consisting of C₁-C₄ alkyls, C₁-C₂ halogenoalkyls, C₁-C₄ alkoxys, and halogens, (xviii) amino optionally substituted with one to two substituents selected from among a group consisting of C₁-C₄ alkyls, C₁-C₂ alkyls substituted with fluorine, and C₁-C₄ alkoxys; phenyl and naphthyl optionally having one to three substituents selected from among a group consisting of methyl, ethyl, methoxy, ethoxy, fluorine, chlorine, and trifluoromethyl; phenyl- and naphthyl-C₁-C₂ alkyls optionally having in the phenyl or naphthyl portion one to three substituents selected from among a group consisting of methyl, ethyl, methoxy, ethoxy, fluorine, chlorine, and trifluoromethyl; formyl, C₁-C₂ alkylcarbonyls, and acryloyl; benzoyl and naphthylcarbonyl optionally having one to three substituents selected from among a group consisting of methyl, ethyl, methoxy, ethoxy, fluorine, chlorine, and trifluoromethyl; phenyl- and naphthyl-C₁-C₂ alkylcarbonyls optionally having one to three substituents selected from among a group consisting of methyl, ethyl, methoxy, ethoxy, fluorine, chlorine, and trifluoromethyl; C₃-C₄ cycloalkylcarbonyls; furylcarbonyl, thienylcarbonyl, imidazolylcarbonyl, oxazolylcarbonyl, isooxazolylcarbonyl, thiazolylcarbonyl, isothiazolylcarbonyl, nicotinoyl, isonicotinoyl, pyridazinylcarbonyl, pyrimidinylcarbonyl, or pyrazinylcarbonyl optionally having one to two substituents selected from among a group consisting of methyl, ethyl, methoxy, ethoxy, fluorine, chlorine, and trifluoromethyl; carbamoyl, and phenylcarbamoyl and naphthylcarbomoyl optionally having one to three substituents selected from among a group consisting of methyl, ethyl, methoxy, ethoxy, fluorine, chlorine, and trifluoromethyl, (xix) a C₁-C₄ halogenoalkoxy, (xx) phenoxy or naphthyloxy optionally having one to two substituents selected from among a group consisting of C₁-C₄ alkyls, C₁-C₂ halogenoalkyls, C₁-C₄ alkoxys, and halogens, (xxi) a phenyl- or naphthyl-C₁-C₂ alkoxy optionally having in the phenyl or naphthyl portion one to three substituents selected from among a group consisting of C₁-C₄ alkyls, C₁-C₂ halogenoalkyls, C₁-C₄ alkoxys, and halogens, (xxii) a C₁-C₄ alkoxycarbonyl, (xxiii) phenoxycarbonyl or naphthyloxycarbonyl optionally having one to three substituents selected from among a group consisting of C₁-C₄ alkyls, C₁-C₂ halogenoalkyls, C₁-C₄ alkoxys, and halogens, or (xxiv) a phenyl- or naphthyl-C₁-C₂ alkoxycarbonyl optionally having in the phenyl or naphthyl portion one to three substituents selected from among a group consisting of C₁-C₄ alkyls, C₁-C₂ halogenoalkyls, C₁-C₄ alkoxys, and halogens,
W^{c3} is (a) oxygen, (b) a group represented by the expression -S(O)_{q}- (where q is 0 or an integer from 1 to 2), (c) a group represented by the expression -N(R^{e2})- (where R^{e2} is (i) hydrogen, (ii) a C₁-C₄ alkyl, (iii) phenyl or naphthyl optionally having one to three substituents selected from among a group consisting of C₁-C₄ alkyls, C₁-C₂ halogenoalkyls, C₁-C₄ alkoxys, and halogens, (iv) a phenyl- or naphthyl-C₁-C₂ alkyl optionally having in the phenyl or naphthyl portion one to three substituents selected from among a group consisting of C₁-C₄ alkyls, C₁-C₂ halogenoalkyls, C₁-C₄ alkoxys, and halogens, (v) a C₁-C₄ alkylsulfonyl, (vi) a C₁-C₄ halogenoalkylsulfonyl, (vii) phenylsulfonyl or naphthylsulfonyl optionally having in the phenyl or naphthyl portion one to three substituents selected from among a group consisting of C₁-C₄ alkyls, C₁-C₂ halogenoalkyls, C₁-C₄ alkoxys, and halogens, (viii) a phenyl- or naphthyl-C₁-C₂ alkylsulfonyl optionally having in the phenyl or naphthyl portion one to three substituents selected from among a group consisting of C₁-C₄ alkyls, C₁-C₂ halogenoalkyls, C₁-C₄ alkoxys, and halogens, (ix) formyl, a C₁-C₂ alkylcarbonyl, or acryloyl, (x) benzoyl or naphthylcarbonyl optionally having one to three substituents selected from among a group consisting of C₁-C₄ alkyls, C₁-C₂ halogenoalkyls, C₁-C₄ alkoxys, and halogens, (xi) a phenyl- or naphthyl-C₁-C₂ alkylcarbonyl optionally having in the phenyl or naphthyl portion one to three substituents selected from among a group consisting of C₁-C₄ alkyls, C₁-C₂ halogenoalkyls, C₁-C₄ alkoxys, and halogens, (xii) a C₃-C₆ cycloalkylcarbonyl, (xiii) furylcarbonyl, thienylcarbonyl, imidazolylcarbonyl, oxazolylcarbonyl, isooxazolylcarbonyl, thiazolylcarbonyl, isothiazolylcarbonyl, nicotinoyl, isonicotinoyl, pyridazinylcarbonyl, pyrimidinylcarbonyl, or pyrazinylcarbonyl optionally having one to two substituents selected from among a group consisting of C₁-C₄ alkyls, C₁-C₂ halogenoalkyls, C₁-C₄ alkoxys, and halogens, or (xiv) carbamoyl optionally substituted with one to two substituents selected from among a group consisting of C₁-C₄ alkyls, phenyl and naphthyl optionally having one to three substituents selected from among a group consisting of C₁-C₄ alkyls, C₁-C₂ halogenoalkyls, C₁-C₄ alkoxys, and halogens, and phenyl- and naphthyl-C₁-C₂ alkyls optionally having in the phenyl or naphthyl portion one to three substituents selected from among a group consisting of C₁-C₄ alkyls, C₁-C₂ halogenoalkyls, C₁-C₄ alkoxys, and halogens), (d) a group represented by the expression -CO-N(R^{e2})- (where R^{e2} is defined as described earlier), (e) a group represented by the expression -N(R^{e2})-CO- (where R^{e2} is defined as described earlier), (f) a group represented by the expression -CO-, (g) a C₁-C₆ alkylene optionally containing one to three atoms and/or groups selected from among a group consisting of (a) to (f), or (h) a dangling bond,
X^{e2} is (a) oxygen, (b) sulfur, (c) a group represented by the expression -N(R^{e2})- (where R^{e2} is defined as described earlier), (d) a group represented by the expression -CO-N(R^{e2})- (where R^{e2} is defined as described earlier), (e) a C₁-C₆ alkylene optionally containing one to three atoms and/or groups selected from among a group consisting of oxygen, sulfur, groups represented by the expression -N(R^{e2})- (where R^{e2} is defined as described earlier), and groups represented by the formula -CO-N (R^{e2})- (where R^{e2} is defined as described earlier), (f) a group represented by the expression -W^{c4}-Phe-X^{e3}- (where W^{c4} is oxygen, a group represented by the expression - S(O)_{q}- (where q is defined as described earlier) or the expression -N(R^{e2})- (where R^{e2} is defined as described earlier), a C₁-C₄ alkylene optionally containing one to two atoms and/or groups selected from among a group consisting of oxygen and groups represented by the expression - S(O)_{q}- (where q is defined as described earlier) and the expression -N(R^{e2})- (where R^{e2} is defined as described earlier), or a dangling bond, Phe is phenylene optionally having one to three substituents selected from among a group consisting of C₁-C₄ alkyls, C₁-C₂ halogenoalkyls, C₁-C₄ alkoxys, and halogens, and X^{e3} is oxygen, a group represented by the expression -S(O)_{q}-(where q is defined as described earlier) or the expression -N(R^{e2})- (where R^{e2} is defined as described earlier), a C₁-C₄ alkylene optionally containing one to two atoms and/or groups selected from among a group consisting of oxygen and groups represented by the expression - S(O)_{q}- (where q is defined as described earlier) and the expression -N(R^{e2})- (where R^{e2} is defined as described earlier), or a dangling bond), or (g) a dangling bond, and Ar is a divalent phenyl, naphthyl, pyrrole, furan, thiophen, pyrazole, imidazole, 1,2,4-triazole, tetrazole, isoxazole, oxazole, 1,3,4-oxadiazole, thiazole, isothiazole, 1,3,4-thiadiazole, pyridine, pyridazine, pyrimidine, pyrazine, indole, benzofuran, benzo[B]thiophen, benzimidazole, benzotriazole, benzisoxazole, benzoxazole, benzisothiazole, benzothiazole, oxazolo[4,5-B]pyridine, benzofurazan, pyrazolo[4,5-C]pyridine, pyrazolo[3,4-B]pyridine, 1,2,3-benzothiadiazole, pyrazolo-[3,4-D]pyrimidine, quinoline, isoquinoline, quinoxaline, pyrido[2,3-B]pyrazine, pyridoimidazole, quinazoline, phthalazine, or naphthylidine cyclic group); or
(2) the group represented by the expression -W^{c3}-W^{c3}-X^{e4}- is oxygen, a group represented by the expression -S(O)_{q}- (where q is defined as described earlier), a group represented by the expression -N(R^{e2})- (where R^{e2} is defined as described earlier), a group represented by the expression -CO-N(R^{e2})- (where R^{e2} is defined as described earlier), a group represented by the expression -N(R^{e2})-CO- (where R^{e2} is defined as described earlier), a C₁-C₁₀ alkylene optionally containing one to five atoms and/or groups selected from among a group consisting of oxygen, groups represented by the expression -S(O)_{q}- (where q is defined as described earlier), groups represented by the expression -N(R^{e2})- (where R^{e2} is defined as described earlier), groups represented by the expression -CO-N(R^{e2})- (where R^{e2} is defined as described earlier), and groups represented by the expression -N(R^{e2})-CO- (where R^{e2} is defined as described earlier) (advantageously a C₁-C₁₀ alkylene containing these atoms and/or groups), or a dangling bond).

17. The benzo- or pyrido-imidazole derivative, the pharmaceutically acceptable salt thereof, or the pharmaceutically acceptable ester or amide thereof according to claims 1 to 15, wherein
E is
(1) a group represented by general formula (d-1) 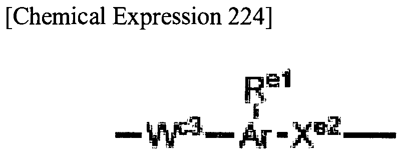 (where R^{e1} is the same or different zero or one to two groups, and R^{e1} is (i) methyl or ethyl, (ii) a C₁-C₂ alkyl substituted with fluorine, (iii) methoxy or ethoxy, (iv) fluorine or chlorine, (viii) cyclopropyl, (ix) phenyl, (x) benzyl, (xi) formyl or acetyl, (xii) cyclopropylcarbonyl, (xiii) benzoyl, (xiv) benzylcarbonyl, (xv) furylcarbonyl, thienylcarbonyl, or nicotinoyl, (xvi) carbamoyl, (xvii) phenylcarbamoyl, (xviii) amino, N-methylamino, N-ethylamino, N,N-dimethylamino, or N-methyl-N-ethylamino, (xix) fluoromethoxy or trifluoromethoxy, (xx) phenoxy, (xxi) benzyloxy, (xxiii) a C₁-C₂ alkoxycarbonyl, (xxiii) phenoxycarbonyl, or (xxiv) benzyloxycarbonyl,
W^{c3} is (a) oxygen, (b) a group represented by the expression -S(O)_{q}- (where q is an integer from 0 to 2), (c) a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, (v) methylsulfonyl, (vi) trifluoromethylsulfonyl, (vii) phenylsulfonyl, (viii) benzylsulfonyl, (ix) formyl or acetyl, (x) benzoyl, (xi) benzylcarbonyl optionally having in the phenyl portion one to two substituents selected from among a group consisting of methyl, methoxy, fluorine, chlorine, and trifluoromethyl, (xii) cyclopropylcarbonyl, (xiii) furylcarbonyl, thienylcarbonyl, or nicotinoyl, or (xiv) carbamoyl optionally substituted with one to two substituents selected from among a group consisting of methyl and ethyl, phenyl, and benzyl optionally having in the phenyl portion one to two substituents selected from among a group consisting of methyl, methoxy, fluorine, chlorine, and trifluoromethyl), (d) a group represented by the expression -CO-N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), (e) a group represented by the expression -N(R^{e2})-CO- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), (f) a group represented by the expression -CO-, (g) a C₁-C₄ alkylene optionally containing one to three atoms and/or groups selected from among a group consisting of (a) to (f), or (h) a dangling bond,
X^{e2} is (a) oxygen, (b) sulfur, (c) a group represented by the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; or acetyl), (d) a group represented by the expression -CO-N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), (e) a C₁-C₄ alkylene optionally containing one to three atoms and/or groups selected from among a group consisting of oxygen, sulfur, groups represented by the expression -N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), and groups represented by the formula -CO-N (R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), (f) a group represented by the expression -W⁴-Phe-X^{e3}- (where W^{c4} is oxygen, a group represented by the expression -S(O)_{q}- (where q is 0 or an integer from 1 to 2) or the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; formyl, or acetyl), a C₁-C₃ alkylene optionally containing one to two atoms and/or groups selected from among a group consisting of oxygen and groups represented by the expression -S(O)_{q}- (where q is 0 or an integer from 1 to 2) and the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; formyl, or acetyl), or a dangling bond, Phe is phenylene optionally having one to two substituents selected from among a group consisting of methyl, ethyl, trifluoromethyl, methoxy, ethoxy, fluorine, and chlorine, and X^{e3} is oxygen, a group represented by the expression -S(O)_{q}- (where q is defined as described earlier) or the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; formyl, or acetyl), a C₁-C₃ alkylene optionally containing one to two atoms and/or groups selected from among a group consisting of oxygen and groups represented by the expression -S(O)_{q}- (where q is defined as described earlier) and the expression -N(R^{e2})- (where R^{e2} is hydrogen, methyl, ethyl, phenyl; benzyl optionally having in the phenyl portion one to two substituents selected from among a group consisting of methyl, methoxy, fluorine, chlorine, and trifluoromethyl; formyl, or acetyl), or a dangling bond), or (g) a dangling bond, and Ar is a divalent phenyl, naphthyl, furan, thiophen, imidazole, tetrazole, oxazole, thiazole, pyridine, pyridazine, pyrimidine, pyrazine, indole, benzofuran, benzo[B]thiophen, benzimidazole, benzisoxazole, benzoxazole, benzisothiazole, benzothiazole, quinoline, isoquinoline, or pyridoimidazole cyclic group); or
(2) the group represented by the expression -W^{c3}-W^{c3}-X^{e4}- is oxygen, a group represented by the expression -S(O)_{q}- (where q is 0 or an integer from 1 to 2), a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, (v) methylsulfonyl, (vi) trifluoromethylsulfonyl, (vii) phenylsulfonyl, (viii) benzylsulfonyl, (ix) formyl or acetyl, (x) benzoyl, (xi) benzylcarbonyl optionally having in the phenyl portion one to two substituents selected from among a group consisting of methyl, methoxy, fluorine, chlorine, and trifluoromethyl, (xii) cyclopropylcarbonyl, (xiii) furylcarbonyl, thienylcarbonyl, or nicotinoyl, or (xiv) carbamoyl optionally substituted with one to two substituents selected from among a group consisting of methyl and ethyl, phenyl, and benzyl optionally having in the phenyl portion one to two substituents selected from among a group consisting of methyl, methoxy, fluorine, chlorine, and trifluoromethyl), a group represented by the expression -CO-N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), a group represented by the expression -N(R^{e2})-CO- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), a C₁-C₄ alkylene optionally containing one to three atoms and/or groups selected from among a group consisting of oxygen, groups represented by the expression - S(O)_{q}- (where q is defined as described earlier), groups represented by the expression -N(R²)-(where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, (v) methylsulfonyl, (vi) trifluoromethylsulfonyl, (vii) phenylsulfonyl, (viii) benzylsulfonyl, (ix) formyl or acetyl, (x) benzoyl, (xi) benzylcarbonyl optionally having in the phenyl portion one to two substituents selected from among a group consisting of methyl, methoxy, fluorine, chlorine, and trifluoromethyl, (xii) cyclopropylcarbonyl, , (xiii) furylcarbonyl, thienylcarbonyl, or nicotinoyl, or (xiv) carbamoyl optionally substituted with one to two substituents selected from among a group consisting of methyl and ethyl, phenyl, and benzyl optionally having in the phenyl portion one to two substituents selected from among a group consisting of methyl, methoxy, fluorine, chlorine, and trifluoromethyl), groups represented by the expression -CO-N(R^{e2})- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl), and groups represented by the expression N(R^{e2})-CO- (where R^{e2} is hydrogen, methyl, ethyl, phenyl, or benzyl optionally having in the phenyl portion one to two substituents selected from among a group consisting of methyl, methoxy, fluorine, chlorine, and trifluoromethyl) (advantageously a C₁-C₄ alkylene containing these atoms and/or groups), or a dangling bond.

18. The benzo- or pyrido-imidazole derivative, the pharmaceutically acceptable salt thereof, or the pharmaceutically acceptable ester or amide thereof according to claims 1 to 15, wherein E is
(1) a group represented by general formula (d-1) 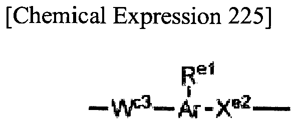 (where R^{e1} is the same or different zero or one to two groups, and R^{e1} is (i) methyl or ethyl, (ii) trifluoromethyl, (iii) methoxy or ethoxy, or (iv) fluorine or chlorine,
W^{c3} is (a) oxygen, (b) a group represented by the expression -S(O)q- (where q is 0 or an integer from 1 to 2), (c) a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, or (ix) formyl or acetyl), (d) a group represented by the expression -CON(R^{e2})- (where R^{e2} is hydrogen or methyl), (e) a group represented by the expression -N(R^{e2})-CO- (where R^{e2} is hydrogen or methyl), (g) a C₁-C₄ alkylene optionally containing one to three atoms and/or groups selected from among a group consisting of (a) to (e), or (h) a dangling bond, X^{e2} is (a) oxygen, (b) sulfur, (c) a group represented by the expression -N(R^{e2})- (where R^{e2} is hydrogen, methyl, benzyl, or acetyl), (e) a C₁-C₄ alkylene optionally containing one to two atoms and/or groups selected from among a group consisting of oxygen, sulfur, and groups represented by the expression -N(R^{e2})- (where R^{e2} is hydrogen, methyl, benzyl, or acetyl) or (g) a dangling bond, and
Ar is a divalent phenyl, pyridine, benzimidazole, or pyridoimidazole); or
(2) the group represented by the expression -W^{c3}-W^{c3}-X^{e4}- is oxygen, a group represented by the expression -S(O)_{q}- (where q is 0 or an integer from 1 to 2), a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, or (ix) formyl or acetyl), a group represented by the expression -CO-N(R^{e2})- (where R^{e2} is hydrogen or methyl), a group represented by the expression -N(R^{e2})-CO- (where R^{e2} is hydrogen or methyl), a C₁-C₄ alkylene optionally containing one to three atoms and/or groups selected from among a group consisting of oxygen, groups represented by the expression -S(O)_{q}- (where q is defined as described earlier), groups represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, or (ix) formyl or acetyl), groups represented by the expression -CO-N(R^{e2})-(where R^{e2} is hydrogen or methyl), and groups represented by the expression -N(R^{e2})-CO-(where R^{e2} is hydrogen or methyl) (advantageously a C₁-C₄ alkylene containing these atoms and/or groups), or a dangling bond.

19. The benzo- or pyrido-imidazole derivative, the pharmaceutically acceptable salt thereof, or the pharmaceutically acceptable ester or amide thereof according to claims 1 to 15, wherein E is
(1) a group represented by the expression -CH₂O-1,4-phenylene-S-, -CH₂S-1, 3-phenylene-O-, - CH₂S-1,4-phenylene-O-, -CH₂SO₂-1,4-phenylene-O-, -CH₂NH-1,4-phenylene-O-, -CH₂NH-1,4-(3,5-dimethylphenylene)-O-, -CH(CH₃)S-1,4-phenylene-O-, -CH₂SCH₂-1,4-phenylene-O-, - CH₂SCH₂CH₂-1,3-phenylene-O-, -SCH₂-1,4-phenylene-O-, -CONHCH₂-1,4-phenylene-O-, - CH₂S-3,5-pyridinylene-S-, -CH₂S-3,6-pyridinylene-O-, -CH(CH₃)S-3,6-pyridinylene-O-, -SCH₂-3,6-pyridinylene-O-, -CH₂SCH₂-2,5-pyridinylene-O-, -CH₂SCH₂-3,6-pyridinylene-O-, - CH₂SCH₂-2,5-pyridinylene-S-, 2,6-(1-methylbenzimidazolylene)-S-, 2,6-(1-methylbenzimidazolylene)-O-CH₂CH₂-O, or 2,6-(1-methylbenzimidazolylene)-N(CH₃)-CH₂CH₂-O-; or
(2) a group represented by the expression -CH₂O-, -CH₂S-, -CH(CH₃)S-, -CH₂SO-, -CH₂SO₂-, - CH₂N(CH₃)-, -CH₂CH₂O-, -CH₂SCH₂CH₂O-, -C(CH₃)₂SCH₂CH₂O-, -N(CH₃)-CH₂CH₂-O-, - CH₂SCH₂CH₂NHCOCH₂S-, or -SCH₂-, or a dangling bond.

20. The benzo- or pyrido-imidazole derivative, the pharmaceutically acceptable salt thereof, or the pharmaceutically acceptable ester or amide thereof according to claims 1 to 15, wherein E is
(1) a group represented by the expression -CH₂O-1,4-phenylene-S-, -CH₂S-1, 3-phenylene-O-, - CH₂S-1,4-phenylene-O-, -CH₂SO₂-1,4-phenylene-O-, -CH₂NH-1,4-phenylene-O-, -CH₂NH-1,4-(3,5-dimethylphenylene)-O-, -CH(CH₃)S-1,4-phenylene-O-, -CH₂SCH₂-1,4-phenylene-O-, - CH₂SCH₂CH₂-1,3-phenylene-O-, -SCH₂-1,4-phenylene-O-, -CONHCH₂-1,4-phenylene-O-, 2,6-(1-methylbenzimidazolylene)-S-, or 2,6-(1-methylbenzimidazolylene)-N(CH₃)-CH₂CH₂-O-; or
(2) a group represented by the expression -CH₂O-, -CH₂S-, -CH(-CH₃)S-, -CH₂SO-, -CH₂SO₂-, - CH₂N(-CH₃)-, -CH₂CH₂O-, -CH₂SCH₂CH₂O-, -C(-CH₃)₂SCH₂CH₂O-, -N(-CH₃)-CH₂CH₂-O-, - CH₂SCH₂CH₂NHCOCH₂S-, or -SCH₂-, or a dangling bond.

21. The benzo- or pyrido-imidazole derivative, the pharmaceutically acceptable salt thereof, or the pharmaceutically acceptable ester or amide thereof according to claims 1 to 20, wherein G is
a dangling bond, oxygen, sulfur, or a group represented by the expression -N(R^{e2})- (where R² is (i) hydrogen, (ii) a C₁-C₄ alkyl, (iii) phenyl or naphthyl optionally having one to three substituents selected from among a group consisting of C₁-C₄ alkyls, C₁-C₂ halogenoalkyls, C₁-C₄ alkoxys, and halogens, (iv) a phenyl- or naphthyl-C₁-C₂ alkyl optionally having in the phenyl or naphthyl portion one to three substituents selected from among a group consisting of C₁-C₄ alkyls, C₁-C₂ halogenoalkyls, C₁-C₄ alkoxys, and halogens, (v) a C₁-C₂ alkylsulfonyl, (vi) a C₁-C₂ alkylsulfonyl substituted with fluorine, (vii) phenylsulfonyl optionally having one to two substituents selected from among a group consisting of methyl, methoxy, fluorine, chlorine, and trifluoromethyl, (viii) benzylsulfonyl optionally having in the phenyl portion one to two substituents selected from among a group consisting of methyl, methoxy, fluorine, chlorine, and trifluoromethyl, (ix) formyl or acetyl, (x) benzyl optionally having one to two substituents selected from among a group consisting of methyl, methoxy, fluorine, chlorine, and trifluoromethyl, (xi) benzylcarbonyl optionally having in the phenyl portion one to two substituents selected from among a group consisting of methyl, methoxy, fluorine, chlorine, and trifluoromethyl, (xii) cyclopropylcarbonyl, (xiii) furylcarbonyl, thienylcarbonyl, or nicotinoyl, or (xiv) carbamoyl optionally having one to two substituents selected from among a group consisting of methyl, ethyl, phenyl, and benzyl optionally having one to two substituents selected from among a group consisting of methyl, methoxy, fluorine, chlorine, and trifluoromethyl).

22. The benzo- or pyrido-imidazole derivative, the pharmaceutically acceptable salt thereof, or the pharmaceutically acceptable ester or amide thereof according to claims 1 to 20, wherein G is
a dangling bond, oxygen, sulfur, or a group represented by the expression -N(R^{e2})- (where R^{e2} is (i) hydrogen, (ii) a C₁-C₃ alkyl, (iii) phenyl optionally substituted with methyl, methoxy, fluorine, chlorine, or trifluoromethyl, or (iv) benzyl optionally substituted in the phenyl portion with methyl, methoxy, fluorine, chlorine, or trifluoromethyl).

23. The benzo- or pyrido-imidazole derivative, the pharmaceutically acceptable salt thereof, or the pharmaceutically acceptable ester or amide thereof according to claims 1 to 20, wherein G is
a dangling bond, oxygen, sulfur, or a group represented by the expression -N(R^{e2})- (where R^{e2} is (i) hydrogen, or (ii) methyl or ethyl).

24. The benzo- or pyrido-imidazole derivative, the pharmaceutically acceptable salt thereof, or the pharmaceutically acceptable ester or amide thereof according to claims 1 to 20, wherein G is
a dangling bond or a group represented by the expression -N(R^{e2})- (where R^{e2} is methyl or ethyl).

25. The benzo- or pyrido-imidazole derivative, the pharmaceutically acceptable salt thereof, or the pharmaceutically acceptable ester or amide thereof according to claims 1 to 24, wherein in the group represented by the expression -V-R,
V is a dangling bond, oxygen, sulfur, or a group represented by the expression -N(R^{e2})- (where R^{e2} is (i) hydrogen, (ii) methyl or ethyl, (iii) phenyl, (iv) benzyl optionally having in the phenyl portion one to two substituents selected from among a group consisting of methyl, methoxy, fluorine, chlorine, and trifluoromethyl, or (ix) acetyl, and R is (i) hydrogen, (ii) a C₁-C₆ alkyl, (iii) phenyl or naphthyl optionally having one to two substituents selected from among a group consisting of methyl and ethyl; trifluoromethyl; methoxy and ethoxy; fluorine, chlorine, and bromine; phenyl; benzoyl optionally having one to two substituents selected from among a group consisting of methyl, methoxy, fluorine, chlorine, and trifluoromethyl; and trifluoromethoxy, or (iv) a phenyl- or naphthyl-C₁-C₃ alkyl optionally having in the phenyl or naphthyl portion one to two substituents selected from among a group consisting of methyl and ethyl; trifluoromethyl; methoxy and ethoxy; fluorine, chlorine, and bromine; phenyl; benzoyl optionally having one to two substituents selected from among a group consisting of methyl, methoxy, fluorine, chlorine, and trifluoromethyl; and trifluoromethoxy.

26. The benzo- or pyrido-imidazole derivative, the pharmaceutically acceptable salt thereof, or the pharmaceutically acceptable ester or amide thereof according to claims 1 to 24, wherein in the group represented by the expression -V-R,
V is a dangling bond, oxygen, or sulfur, and R is (i) hydrogen, (ii) methyl, ethyl, propyl, i-propyl, butyl, i-butyl, s-butyl, pentyl, or hexyl, (iii) phenyl optionally having one to two substituents selected from among a group consisting of methyl; trifluoromethyl; methoxy; fluorine, chlorine, and bromine; benzoyl optionally having one to two substituents selected from among a group consisting of methyl, methoxy, fluorine, chlorine, and trifluoromethyl; and trifluoromethoxy, or (iv) a phenyl-C₁-C₃ alkyl optionally having in the phenyl portion one to two substituents selected from among a group consisting of methyl; trifluoromethyl; methoxy; fluorine, chlorine, and bromine; phenyl; benzoyl optionally having one to two substituents selected from among a group consisting of methyl, methoxy, fluorine, chlorine, and trifluoromethyl; and trifluoromethoxy.

27. The benzo- or pyrido-imidazole derivative, the pharmaceutically acceptable salt thereof, or the pharmaceutically acceptable ester or amide thereof according to claims 1 to 24, wherein in the group represented by the expression -V-R,
V is a dangling bond or oxygen, and R is (i) hydrogen, (ii) methyl, i-propyl, or hexyl, (iii) phenyl, 2-, 3-, or 4-trifluoromethylphenyl, 4-methoxyphenyl, 4-chloro-3-methoxyphenyl, 3- or 4-bromophenyl, 4-methylbenzoylphenyl, 3-chlorobenzoylphenyl, or 3-trifluoromethoxyphenyl, or (iv) benzyl, 3- or 4-trifluoromethylbenzyl, 2-, 3-, or 4-methoxybenzyl, 4-chlorobenzyl, 3-bromobenzyl, 4-(4-methylbenzoyl)benzyl, 3-chlorobenzoylbenzyl, 3-trifluoromethoxybenzyl, α-methylbenzyl, 2-(4-methoxyphenyl)ethyl, or 3-phenylpropyl.

28. The benzo- or pyrido-imidazole derivative, the pharmaceutically acceptable salt thereof, or the pharmaceutically acceptable ester or amide thereof according to claims 1 to 27, wherein in the group represented by the expression -(CH₂)ₙ-Q-,
n is an integer of 1 to 4 (provided, however, that n is an integer of 2 to 4 when G is oxygen, sulfur, or a group represented by the expression -N(R^{e2})- (where R^{e2} is defined as described in claim 1)), and Q is oxygen or sulfur.

29. The benzo- or pyrido-imidazole derivative, the pharmaceutically acceptable salt thereof, or the pharmaceutically acceptable ester or amide thereof according to claims 1 to 27, wherein in the group represented by the expression -(CH₂)ₙ-Q-,
n is an integer of 1 to 4 (provided, however, that n is an integer of 2 to 4 when G is oxygen, sulfur, or a group represented by the expression -N(R^{e2})- (where R^{e2} is defined as described in claim 1)), and Q is oxygen.

30. The benzo- or pyrido-imidazole derivative, the pharmaceutically acceptable salt thereof, or the pharmaceutically acceptable ester or amide thereof according to claims 1 to 27, wherein in the group represented by the expression -(CH₂)ₙ-Q-,
n is an integer of 1 to 3 (provided, however, that n is an integer of 2 to 3 when G is oxygen, sulfur, or a group represented by the expression -N(R^{e2})- (where R^{e2} is defined as described in claim 1)), and Q is oxygen.

31. The benzo- or pyrido-imidazole derivative, the pharmaceutically acceptable salt thereof, or the pharmaceutically acceptable ester or amide thereof according to claims 1 to 27, wherein in the group represented by the expression -(CH₂)ₙ-Q-,
n is an integer of 1 to 2 (provided, however, that n is an integer of 2 when G is oxygen, sulfur, or a group represented by the expression -N(R^{e2})- (where R^{e2} is defined as described in claim 1)), and Q is oxygen.

32. The benzo- or pyrido-imidazole derivative, the pharmaceutically acceptable salt thereof, or the pharmaceutically acceptable ester or amide thereof according to claims 1 to 31, wherein p is an integer of 1 to 4.

33. The benzo- or pyrido-imidazole derivative, the pharmaceutically acceptable salt thereof, or the pharmaceutically acceptable ester or amide thereof according to claims 1 to 31, wherein p is an integer of 1 to 3.

34. The benzo- or pyrido-imidazole derivative, the pharmaceutically acceptable salt thereof, or the pharmaceutically acceptable ester or amide thereof according to claims 1 to 31, wherein p is an integer of 1 to 2.

35. The benzo- or pyrido-imidazole derivative, the pharmaceutically acceptable salt thereof, and the pharmaceutically acceptable ester or amide thereof according to claim 1, comprising
4-[4-{2-[4-(2,4-dioxothiazolidin-5-ylmethyl)phenoxymethyl]-1-methyl-1-H-benzimidazol-6-yloxy}phenylthiomethyl]-2-propyl-1-[2'-(1H-tetrazol-5-yl)biphenyl-4-ylmethyl]-1H-imidazole-5-carboxylic acid ethyl ester,
4-[4-{2-[4-(2,4-dioxothiazolidin-5-ylmethyl)phenoxymethyl]-1-methyl-1H-benzimidazol-6-yloxy}phenylthiomethyl]-2-propyl-1-[2'-(1H-tetrazol-5-yl)biphenyl-4-ylmethyl]-1H-imidazole-5-carboxylic acid methyl ester,
4-[4-{2-[4-(2,4-dioxothiazolidin-5-ylmethyl)phenoxymethyl]-1-methyl-1H-benzimidazol-6-yloxy}phenylthiomethyl]-2-propyl-1-[2'-(H-tetrazol-5-yl)biphenyl-4-ylmethyl]-1H-imidazole-5-carboxylic acid N-methylamide,
4-[3- {2-[4-(2,4-dioxothiazolidin-5-ylmethyl)phenoxymethyl]-1-methyl-1H-benzimidazol-6-yloxy}phenylthiomethyl]-2-propyl-1-[2'-(H-tetrazol-5-yl)biphenyl-4-ylmethyl]-1H-imidazole-5-carboxylic acid ethyl ester,
4-[4- {2-[4-(2,4-dioxothiazolidin-5-ylmethyl)phenoxymethyl]-1-methyl-1H-benzimidazol-6-ylthio}phenoxymethyl]-2-propyl-1-[2'-(1H-tetrazol-5-yl)biphenyl-4-ylmethyl]-1H-imidazole-5-carboxylic acid ethyl ester,
4-[2-[1-methyl-2-{4-(2,4-dioxothiazolidin-5-ylmethyl)phenoxymethyl}-1H-benzimidazol-6-yloxy]ethylthiomethyl]-2-propyl-1-[2'-(1H-tetrazol-5-yl)biphenyl-4-ylmethyl]-1H-imidazole-5-carboxylic acid ethyl ester,
4-[2-[1-methyl-2-{4-(2,4-dioxothiazolidin-5-ylmethyl)phenoxymethyl}-1H-benzimidazol-6-ylthioacetylamino]ethylthiomethyl]-2-propyl-1-[2'-(1H-tetrazol-5-yl)biphenyl-4-ylmethyl]-1H-imidazole-5-carboxylic acid ethyl ester,
4- {2-[4-(2,4-dioxothiazolidin-5-ylmethyl)phenoxymethyl]-1-methyl-1H-benzimidazol-6-yloxymethyl}-2-propyl-1-[2'-(1H-tetrazol-5-yl)biphenyl-4-ylmethyl]-1H-imidazole-5-carboxylic acid ethyl ester (or hydrochloric acid salt thereof),
4- {2-[4-(2,4-dioxothiazolidin-5-ylmethyl)phenoxymethyl]-1-methyl-1H-benzimidazol-6-yloxymethyl}-2-propyl-1-[2'-(1H-tetrazol-5-yl)biphenyl-4-ylmethyl]-1H-imidazole-5-carboxylic acid,
4-[4- {2-[4-(2,4-dioxothiazolidin-5-ylmethyl)phenoxymethyl]-1-methyl-1H-benzimidazol-6-yloxy}phenylsulfonylmethyl]-2-propyl-1-[2'-(1H-tetrazol-5-yl)biphenyl-4-ylmethyl]-1H-imidazole-5-carboxylic acid ethyl ester,
4- {2-[4-(2,4-dioxothiazolidin-5-ylmethyl)phenoxymethyl]-1-methyl-1H-benzimidazol-6-ylthiomethyl}-2-propyl-1-[2'-(1H-tetrazol-5-yl)biphenyl-4-ylmethyl]-1H-imidazole-5-carboxylic acid ethyl ester,
3-[4-[6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenyl]-2-(4-chlorobenzyloxy)propionic acid,
N-[4- {6-(5-ethoxycarbonyl-1-{2'-(1H-tetrazol-5-yl)biphenyl-4-ylmethyl}-2-propyl-1H-imidazol-4-ylmethylthio)-1-methyl-1H-benzimidazol-2-ylmethoxy]phenylmethyl]-N-phenoxycarbonylamino acetic acid,
4-[4-{2-[4-(2,4-dioxothiazolidin-5-ylmethyl)phenoxymethyl]-1-methyl-1H-benzimidazol-6-yloxy}benzylthiomethyl]-2-propyl-1-[2'-(1H-tetrazol-5-yl)biphenyl-4-ylmethyl]-1H-imidazole-5-carboxylic acid ethyl ester,
4-[2-{2-[4-(2,4-dioxothiazolidin-5-ylmethyl)phenoxymethyl]-1-methyl-1H-benzimidazol-6-yloxy}pyridin-5-ylmethylthiomethyl]-2-propyl-1-[2'-(1H-tetrazol-5-yl)biphenyl-4-ylmethyl]-1H-imidazole-5-carboxylic acid ethyl ester,
N-[4- {6-(5-ethoxycarbonyl-1-{2'-(1H-tetrazol-5-yl)biphenyl-4-ylmethyl} -2-propyl-1H-imidazol-4-ylmethylthio)-1-methyl-1H-benzimidazol-2-ylmethoxy]phenylmethyl]-N-(3-trifluoromethylbenzoyl)amino acetic acid,
5-(4- {6-[4-methyl-2-propyl-1-[2'-(1H-tetrazol-5-yl)biphenyl-4-ylmethyl]-1H-benzimidazol-6-ylmethoxy]-1-methyl-1H-benzimidazol-2-ylmethoxy}benzyl)thiazolidine-2,4-dione,
5-[4-[6- {2-(N-(1-methyl-2-[4-methyl-2-propyl-1-(2'-(1H-tetrazol-5-yl)biphenylmethyl)-1H-benzimidazol-6-yl]-1H-benzamidazol-6-yl)-N-methylamino)ethoxy}-1-methyl-1H-benzimidazol-2-ylmethoxy]benzyl]thiazolidine-2,4-dione,
4'-[6-(N-{2-[4-(2,4-dioxothiazolidin-5-ylmethyl)phenoxymethyl]-3-methyl-3H-imidazo[4,5-b]pyridin-5-yl}-N-methylaminomethyl)-4-methyl-2-propyl-1H-benzimidazol-1-ylmethyl]biphenyl-2-carboxylic acid,
4'-[6-{6-(N-2-[4-(2,4-dioxothiazolidin-5-ylmethyl)phenoxy]ethyl-N-methylamino)-1-methyl-1H-benzimidazol-2-yl}-4-methyl-2-propyl-1H-benzimidazol-1-ylmethyl]biphenyl-2-carboxylic acid,
5-[4-[6- {4-(4-methyl-2-propyl-1-<2'-(1H-tetrazol-5-yl)biphenyl-4-ylmethyl>-1H-benzimidazol-6-ylcarbonylaminomethyl)phenoxy}-1-methyl-1H-benzimidazol-2-ylmethoxy]benzyl]thiazolidin-2,4-dione, or
4'-[6-{6-(N-2-[4-(2,4-dioxothiazolidin-5-ylmethyl)phenoxy]ethy]-N-methylamino)-1-methyl-1H-benzimidazol-2-yl}-4-methyl-2-propyl-1H-benzimidazol-1-ylmethyl]biphenyl-2-carboxylic acid.

36. A pharmaceutical composition containing an active ingredient of the benzo- or pyrido-imidazole derivative, the pharmaceutically acceptable salt thereof, or the pharmaceutically acceptable ester or amide thereof according to claims 1 to 35.

37. The pharmaceutical composition according to claim 36, wherein the pharmaceutical has both PPAR activation activity and angiotensin receptor antagonistic activity.

38. The pharmaceutical composition according to claim 36, wherein the pharmaceutical comprises a prophylactic or therapeutic agent for diabetes, diabetes complications, cancer, glaucoma, dry eye, Alzheimer's disease, hypertension, or hyperlipidemia.

39. The pharmaceutical composition according to claim 36, wherein the pharmaceutical comprises a prophylactic or therapeutic agent of diabetes or diabetes complications.

40. A use of the benzo- or pyrido-imidazole derivative, the pharmaceutically acceptable salt thereof, or the pharmaceutically acceptable ester or amide thereof according to claims 1 to 35 for producing a pharmaceutical composition containing the benzo- or pyrido-imidazole derivative, the pharmaceutically acceptable salt thereof, or the pharmaceutically acceptable ester or amide thereof according to claims 1 to 35.

41. A use of the benzo- or pyrido-imidazole derivative, the pharmaceutically acceptable salt thereof, or the pharmaceutically acceptable ester or amide thereof according to claim 40, wherein the pharmaceutical comprises a prophylactic or therapeutic agent of diabetes or diabetes complications.

42. A use of the benzo- or pyrido-imidazole derivative, the pharmaceutically acceptable salt thereof, or the pharmaceutically acceptable ester or amide thereof according to claims 1 to 35 as a pharmaceutical composition.

43. A use of the the benzo- or pyrido-imidazole derivative, the pharmaceutically acceptable salt thereof, or the pharmaceutically acceptable ester or amide thereof according to claim 42, wherein the pharmaceutical comprises a prophylactic or therapeutic agent of diabetes or diabetes complications.

44. A method of prevention or treatment of diabetes or diabetes complications, comprising administering an effective dose of the benzo- or pyrido-imidazole derivative, the pharmaceutically acceptable salt thereof, or the pharmaceutically acceptable ester or amide thereof according to claims 1 to 35.
